(19) Europäisches Patentamt
European Patent Office
Office européen des brevets

(11) EP 4 081 521 B1

(12) EUROPEAN PATENT SPECIFICATION

(45) Date of publication and mention of the grant of the patent:
**10.12.2025 Bulletin 2025/50**

(21) Application number: **20842043.0**

(22) Date of filing: **27.12.2020**

(51) International Patent Classification (IPC):
***C07D 471/04*** (2006.01) ***C07D 487/04*** (2006.01)
***C07D 491/107*** (2006.01) ***C07D 498/04*** (2006.01)
***A61P 35/00*** (2006.01) ***A61K 31/519*** (2006.01)

(52) Cooperative Patent Classification (CPC):
**C07D 471/04; A61P 35/00; C07D 487/04; C07D 491/107; C07D 498/04**

(86) International application number:
**PCT/IB2020/062462**

(87) International publication number:
**WO 2021/130731 (01.07.2021 Gazette 2021/26)**

# (54) SUBSTITUTED TRICYCLIC COMPOUNDS

SUBSTITUIERTE TRICYCLISCHE VERBINDUNGEN

COMPOSÉS TRICYCLIQUES SUBSTITUÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority:
**27.12.2019 IN 201921054254**
**29.12.2019 IN 201921049099**
**29.05.2020 IN 202021022668**
**30.07.2020 IN 202021032769**
**14.08.2020 IN 202021035200**

(43) Date of publication of application:
**02.11.2022 Bulletin 2022/44**

(73) Proprietor: **Lupin Limited**
**Mumbai 400 055 (IN)**

(72) Inventors:
- **KURHADE, Sanjay, Pralhad**
  **Maharashtra Pune 412115 (IN)**
- **NAIR, Prathap, Sreedharan**
  **Maharashtra Pune 412115 (IN)**
- **SETHI, Sachin**
  **Maharashtra Pune 412115 (IN)**
- **SHUKLA, Manojkumar, Ramprasad**
  **Maharashtra Pune 412115 (IN)**
- **SINDKHEDKAR, Milind, Dattatraya**
  **Maharashtra Pune 412115 (IN)**
- **PALLE, Venkata, P.**
  **Maharashtra Pune 412115 (IN)**
- **KAMBOJ, Rajender, Kumar**
  **Maharashtra Pune 412115 (IN)**
- **PHUKAN, Samiron**
  **Maharashtra Pune 412115 (IN)**
- **PATIL, Pradeep, Rangrao**
  **Maharashtra Pune 412115 (IN)**
- **MAJID, Sayyed**
  **Maharashtra Pune 412115 (IN)**
- **PHADATARE, Ramesh**
  **Maharashtra Pune 412115 (IN)**
- **WALKE, Navnath**
  **Maharashtra Pune 412115 (IN)**
- **PACHPUTE, Vipul**
  **Maharashtra Pune 412115 (IN)**
- **GORE, Balasaheb**
  **Maharashtra Pune 412115 (IN)**
- **TAMBE, Vikas**
  **Maharashtra Pune 412115 (IN)**
- **LIMAYE, Rohan**
  **Maharashtra Pune 412115 (IN)**
- **BHOSALE, Avadhut**
  **Maharashtra Pune 412115 (IN)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **MAHANGARE, Sachin**
**Maharashtra Pune 412115 (IN)**

(74) Representative: **Meroni, Francesca et al**
**Jacobacci & Partners S.p.A.**
**Via Senato, 8**
**20121 Milano (IT)**

(56) References cited:
**EP-A1- 0 668 280**
**WO-A1-2018/115380**
**WO-A1-2019/122129**
**CN-A- 102 633 812**

## Description

FIELD OF THE INVENTION

The present invention is related to a compound of the general formula (I),

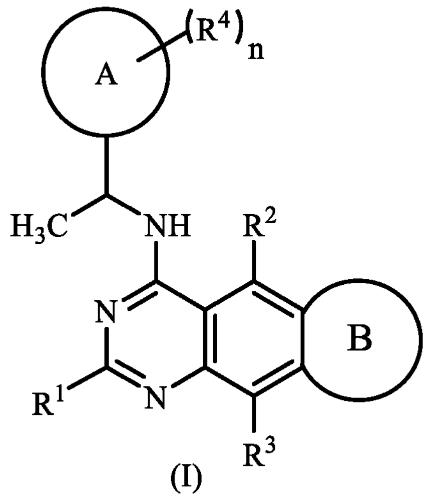

(I)

its pharmaceutically acceptable salt its combination with suitable medicament, its pharmaceutical composition, method of making of the compound, its use as SOS1 inhibitor, and its therapeutic utility in various pathological conditions.

BACKGROUND OF THE INVENTION

Multiple signaling pathways control the initiation, progression, spread, metastasis, immune evasion of cancer. Key signaling pathways include RTK/RAS pathway, PI3K pathway, Wnt pathway, Myc pathway and the cell cycle pathway (Francisco Sanchez-Vega et al., Cell, 2018, 173(2):321-337.e10). RAS-family proteins (KRAS, HRAS and NRAs and their respective mutants) are small GTPases that exist in cells in either GTP-bound (inactive) or GDP-bound (active) states (Siqi Li et al, Nat. Rev. Cancer, 2018, 18(12):767-777). The activity of RAS proteins is modulated by proteins known as GTPase Activating Proteins (GAPs) or Guanine Nucleotide Exchange Factors (GEFs). The GAP proteins belonging to the RAS family include members such as NF1, TSC2, IQGAP1, etc. which activate the GTPase function of the RAS proteins and thus terminate the signaling by catalyzing the hydrolysis of GTP to GDP. In contrast, the RAS family GEFs include proteins such as SOS1, SOS2, RASGRP, RASGRF2, etc. which activate the RAS proteins by exchanging GTP for GDP (Johannes L. Bos et al, Cell, 2007, 129(5):865-77).

Ras-GTP binds to effector proteins such as Raf and PI3K which in turn leads to activation of the RAF-MEK-ERK (MAPK) and PI3K-mTOR-AKT (PI3K) signaling pathways (Suzanne Schubbert et al., Nat. Rev. Cancer, 2007, 7(4):295-308). Triggering of one or more of these cellular signaling pathways leads to the initiation and maintenance of the oncogenic phenotype involving enhanced cell proliferation, increased cell survival, altered metabolism, angiogenesis, migratory potential and immune evasion eventually leading to establishment and metastasis of cancers (Yousef Ahmed Fouad et al., Am. J. Cancer Res., 2017, 1;7(5):1016-1036; Douglas Hanahan et al., Cell, 2011, 4;144(5):646-74). RAS proteins undergo point mutations at several amino acid residues - the key hot spots being positions G12, G13 and Q61. These mutations render the RAS proteins constitutively active since the proteins are predominantly in the active GTP-bound form (Ian A. Prior et al., Cancer Res. 2012, 15; 72(10): 2457-2467; Adrienne D. Cox, et al., Nat. Rev. Drug. Discov., 2014, 13(11):828-51). Interaction of RAS proteins with GEFs such as Son of Sevenless 1 (SOS1) plays a crucial role in relaying the signals to downstream effectors. The SOS1 protein harbors several domains such as the Dbl homology domain (DH), a Pleckstrin homology domain (PH), RAS exchanger motif (REM), CDC25 homology domain and a C-terminal proline rich domain (PxxP) (Pradeep Bandaru et al., Cold Spring Harb Perspect Med., 2019, 1;9(2). pii: a031534). SOS1 has been shown to have a catalytic site as well as an allosteric site. The catalytic site is preferentially bound by RAS-GDP whereas RAS-GTP binds with the allosteric site with better affinity than RAS-GDP (S. Mariana Margarit et al., Cell, 2003, 7;112(5):685-95; Hao-Hsuan Jeng et al., Nat. Commun., 2012; 3:1168). Furthermore, binding of oncogenic KRAS to SOS1 promotes the activation of wild type HRAS and NRAS (Hao-Hsuan Jeng et al., Nat. Commun., 2012;3:1168). The catalytic (guanine nucleotide exchange) function of SOS1 is critical for KRAS oncogenic activity in cancer cells (You X et al., Blood. 2018, 13;132(24):2575-2579; Erin Sheffels et al., Sci Signal. 2018, 4;11(546). pii: eaar8371). SOS1 plays a key role in signal transmission following cellular activation by Receptor Tyrosine Kinases (RTKs) (Frank McCormick et al.,

Nature, 1993, 6;363(6424):45-51; Stephane Pierre et al., Biochem Pharmacol. 2011, 1;82(9):1049-56). Additionally, receptors on lymphocytes (B cell and T cell receptor) (Mateusz Poltorak et al., Eur J Immunol. 2014, 44(5):1535-40; Stephen R. Brooks et al., J Immunol. 2000, 15;164(6):3123-31) and hematopoietic cells (Mario N. Lioubin et al., Mol Cell Biol., 1994, 14(9):5682-91).

The role of SOS1 in the RAS-mediated signaling pathways make it an attractive target for cancer therapy. Pharmacological intervention with SOS1 inhibitors has been shown to attenuate or eliminate the downstream effector events of the RAS-mediated pathways (Roman C. Hillig et al., Proc. Natl. Acad. Sci. U S A. 2019, 12;116(7):2551-2560; Chris R. Evelyn et al., J Biol Chem., 2015, 15; 290(20): 12879-98).

In addition to cancer, hereditary SOS1 mutations are implicated in the pathogenesis of RASopathies like *e.g.* Noonan syndrome (NS), cardio-facio-cutaneous syndrome (CFC) and hereditary gingival fibromatosis type 1 (Pierre et a/., Biochem. Pharmacol., 2011,82(9):1049-56).

In addition, the other diseases associated with *hSOS1* expression is significantly upregulated in whole blood cell extracts of pediatric patients with acute community-acquired *Staphylococcus aureus* infection and in patients with Acute Respiratory Distress Syndrome (ARDS)/Acute Lung Injury (ALI) and Sepsis (F.C. Baltanás, et al. BBA - Reviews on Cancer 1874 (2020) 188445).

In addition, several patent applications related to SOS1 are published which are as follows: WO2004003152, WO2014144148, WO2016077793, WO2018115380, WO2018172250, WO2019122129, WO2019201848, WO2020180768, and WO2020180770.

The foregoing shows that there exists an unmet need for SOS1 inhibitory compounds for treating diseases or disorders involving SOS1, particularly cancer that are dependent on the SOS1.

BRIEF SUMMARY OF THE INVENTION

The present invention provides compound of the general formula (I), its pharmaceutically acceptable salts its combinations with suitable other medicament or medicaments, its pharmaceutical compositions thereof, and its use thereof in treating various diseases or disorders including cancers,

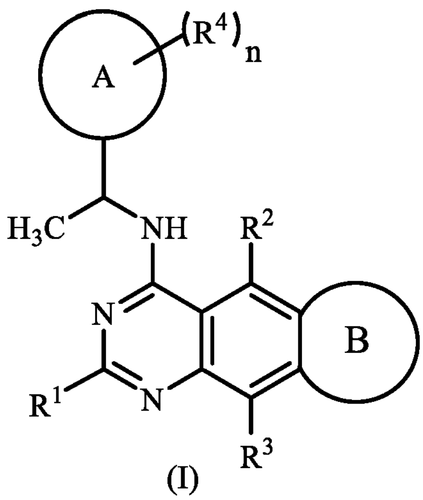

(I)

wherein, ring A, ring B, $R^1$ to $R^4$, and n are as described hereinbelow. The compounds of the present invention are potent inhibitors of SOS1.

According to one aspect of the present invention, there is provided a compound represented by the general formula (I), its pharmaceutically acceptable salt, its combinations with suitable medicament and its pharmaceutical compositions.

In other aspect the present invention provides a pharmaceutical composition, containing the compound of the general formula (I) as defined herein, or its pharmaceutically acceptable salt in combination with the usual pharmaceutically employed carriers, diluents, and the like are useful for the treatment of a disease or disorder mediated through SOS1.

In another aspect the present invention provides a pharmaceutical composition, containing the compound of the general formula (I) as defined herein or its pharmaceutically acceptable salt in combination with the usual pharmaceutically employed carriers, diluents, and the like are useful for the treatment of a disease or disorder such as cancer, infectious disease or disorder, or RASopathy disease or disorder.

In yet other aspect the present invention provides the compound of formula I its pharmaceutically acceptable salt,

its combination with suitable medicament, or its pharmaceutical composition for treating disease characterized by excessive or abnormal cell proliferation such as cancer.

In another aspect the present invention provides the compound of formula I, its pharmaceutically acceptable salt, its combination with suitable medicament, or its pharmaceutical composition for treating diseases like pancreatic cancer, lung cancer, colorectal cancer, class 3 BRAF-mutant cancers, hematological cancer, cholangiocarcinoma, multiple myeloma, melanoma, uterine cancer, endometrial cancer, thyroid cancer, acute myeloid leukaemia, bladder cancer, urothelial cancer, gastric cancer, cervical cancer, head and neck squamous cell carcinoma, diffuse large B cell lymphoma, esophageal cancer, chronic lymphocytic leukaemia, hepatocellular cancer, breast cancer, ovarian cancer, prostate cancer, glioblastoma, renal cancer, Pure mucosal neuroma syndrome, Fibrous Epulis, and sarcomas.

In another aspect the present invention provides the compound of formula I, its pharmaceutically acceptable salt, its combination with suitable medicament, or its pharmaceutical composition for treating diseases such as Neurofibromatosis type 1 (NF1), Noonan Syndrome with Multiple Lentigines (NSML), Noonan-like/multiple giant cell lesion syndrome, Hereditary Gingival Fibromatosis (HGF), Capillary Malformation-Arteriovenous Malformation Syndrome (CM-AVM), Legius Syndrome, Acute Staphylococcus aureus infection (Pediatric Patients), Pure mucosal neuroma syndrome, Fibrous Epulis, Acute Respiratory Distress syndrome/Acute Lung injury and Sepsis, Costello Syndrome (CS), and Cardio-Facio-cutaneous Syndrome (CFC Syndrome).

In another aspect the present invention provides the compound of formula I, its pharmaceutically acceptable salt, its combination with suitable medicament, or its pharmaceutical composition for use in therapeutic regimens in the context of first line, second line, or any further line of treatments.

In another aspect the present invention provides the compound of formula I, its pharmaceutically acceptable salt, its combination with suitable medicament, or its pharmaceutical composition for use in the prevention, short-term or long term treatment of the above-mentioned diseases optionally in combination with radiotherapy and/or surgery.

In yet another aspect the present invention provides the compound of formula I, its pharmaceutically acceptable salt, its combination with suitable medicament, or its pharmaceutical composition for treating various cancers mentioned above which harbor hyperactive or aberrantly activated signaling pathways involving RAS and or SOS1 proteins.

In another aspect the present invention provides use of the compound of formula I, its pharmaceutically acceptable salt, in combination with other agents such as radiation, chemotherapeutic agents and/or targeted agents in multiple cancers and their subtypes as mentioned above. The agents that can be used for combination therapy include targeted agents such as inhibitors of RTKs, cyclin-dependent kinase (CDK) inhibitors, Ser-Thr kinase inhibitors, non-receptor tyrosine kinase inhibitors, inhibitors of epigenetic mechanism such as histone methyltransferases (HMTs), DNA methyltransferases (DNMTs), protein arginine methyltransferases (PRMTs), RAS inhibitors, KRAS inhibitors, MEK inhibitors, ERK1/2 inhibitors, Focal Adhesion Kinase (FAK) inhibitors, PI3K inhibitors, AKT inhibitors, and mTOR inhibitors.

## DETAIL DESCRIPTION OF THE INVENTION

The present invention is related to a compound of the general formula (I), its pharmaceutically acceptable salt, its combination with suitable one or more other medicaments, its pharmaceutical composition, method of making of the compound, its use as SOS1 inhibitor, and its therapeutic utility in treating, or ameliorating various pathological conditions.

The references to methods of treatment in the subsequent paragraphs of this description are to be interpreted as references to the compounds, pharmaceutical compositions and medicaments of the present invention for use in a method for treatment and/or prevention of a disease in a subject.

The compound of formula (I) is as shown below:

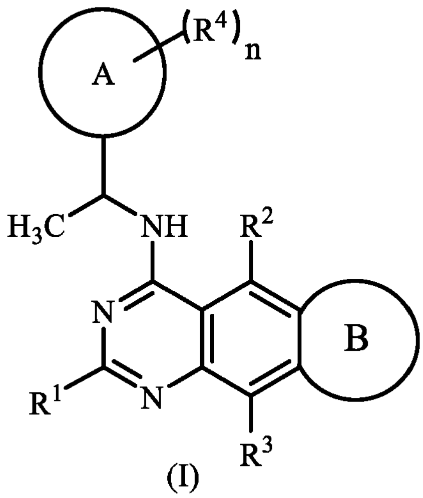

(I)

Wherein,

Ring A is selected from phenyl and

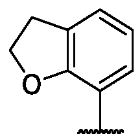;

Ring B is selected from

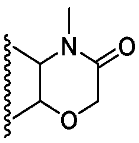, 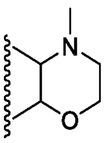, 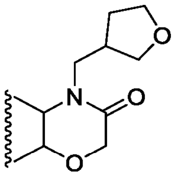, 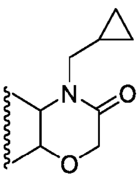, 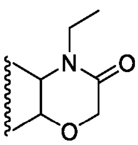, 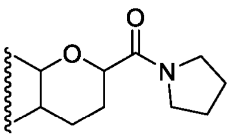, 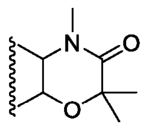,

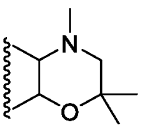, 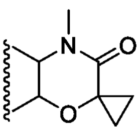, 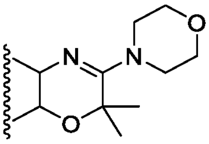, 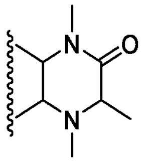, 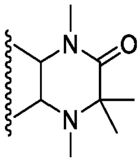, 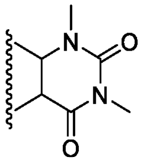, 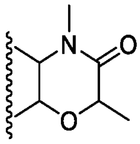,

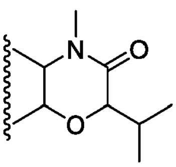, 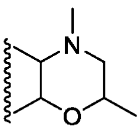, 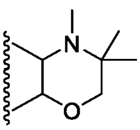, 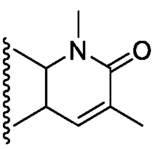, 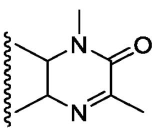, 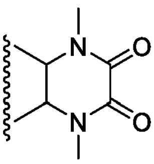, 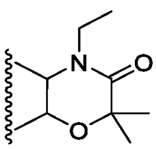,

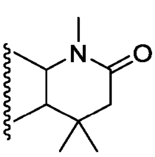, 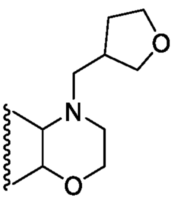, 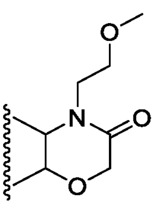, 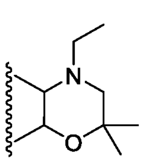, 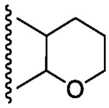, 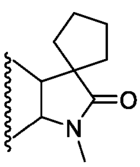, 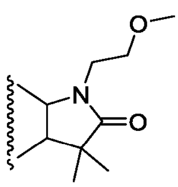,

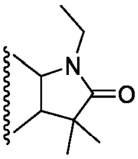, 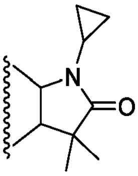, 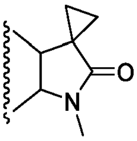, 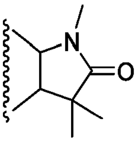, 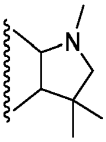, 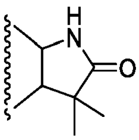, 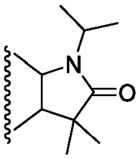, 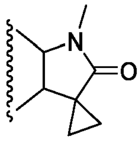,

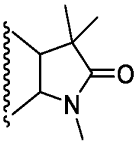, 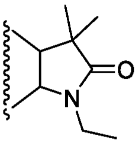, 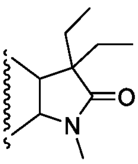, 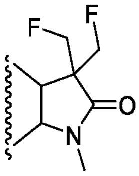, 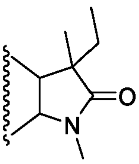, 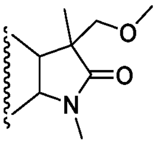, 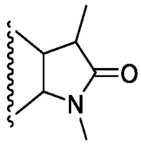,

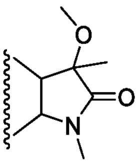, 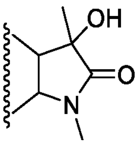, 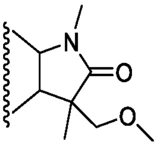, 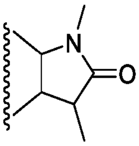, 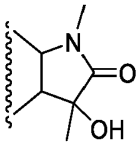, 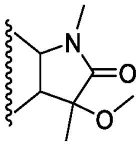, 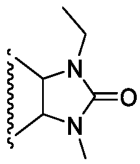,

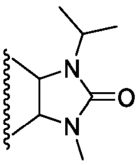, 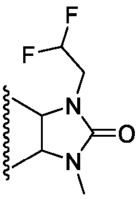, 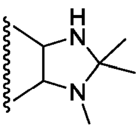, 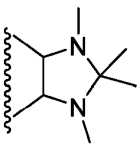, 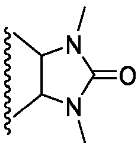, 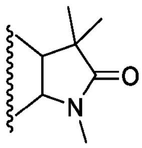, 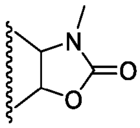,

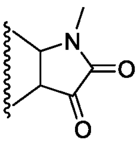, 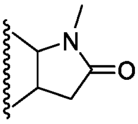, 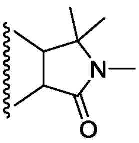, 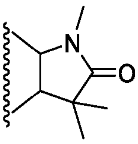, 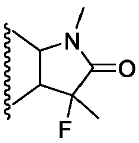, 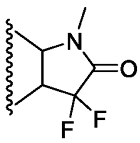, 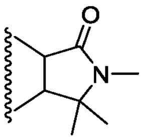,

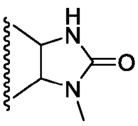, 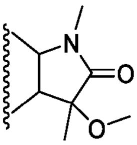, 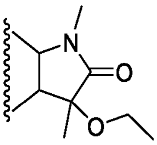, 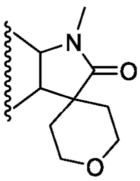, , , ,

, , and ;

$R^1$ is selected from methyl, ethyl, isopropyl, and cyclopropyl;
$R^2$ and $R^3$ are independently selected from hydrogen, fluorine, and methyl;
$R^4$ is selected from fluorine, $-NH_2$, $-CH_3$, $-CF_3$, $-CHF_2$,

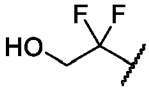, 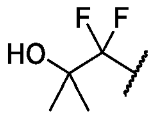, 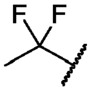, 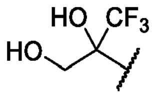,

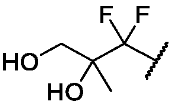, 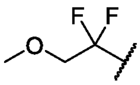, 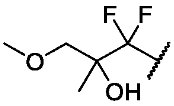, 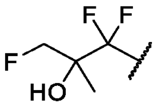, 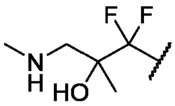, 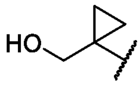, 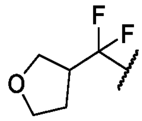,

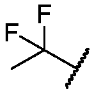, 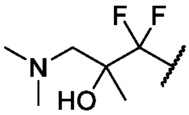, and 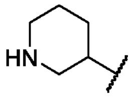;

"n" is an integer selected from 1, 2, and 3.

The invention provides the compound of the general formula (I), its pharmaceutically acceptable salt, wherein ring A is selected from phenyl and

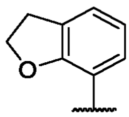;

wherein ring B is selected from

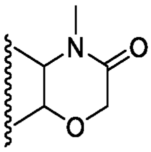, 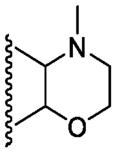,

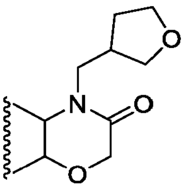, 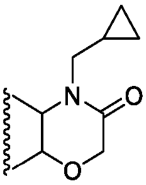, 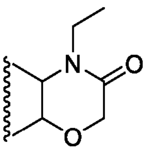, 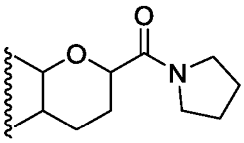, 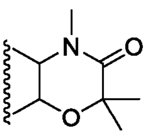, 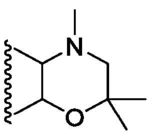, 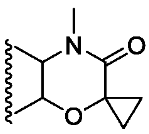,

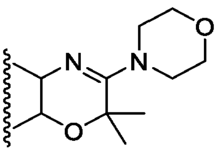, 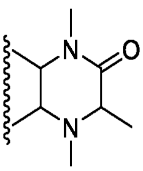, 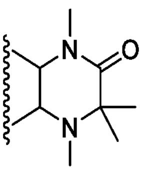, 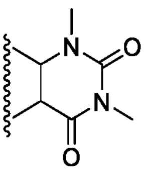, 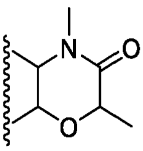, 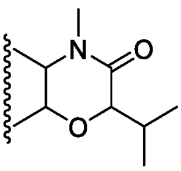, 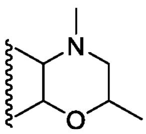,

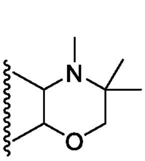, 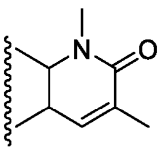, 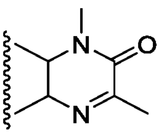, 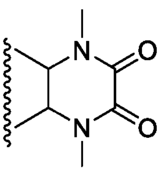, 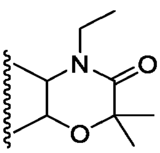, 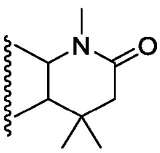, 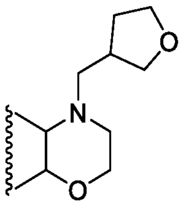,

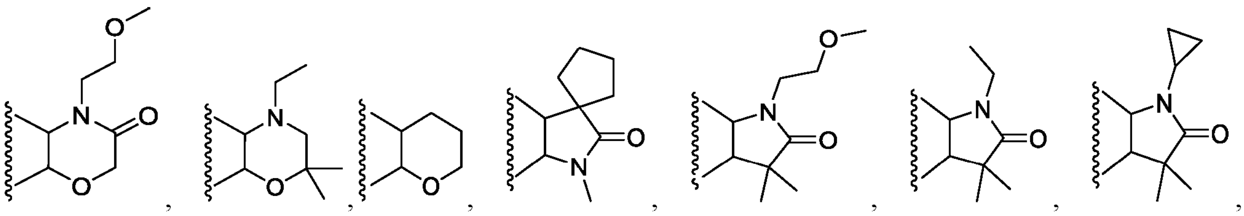
, , , , , , ,

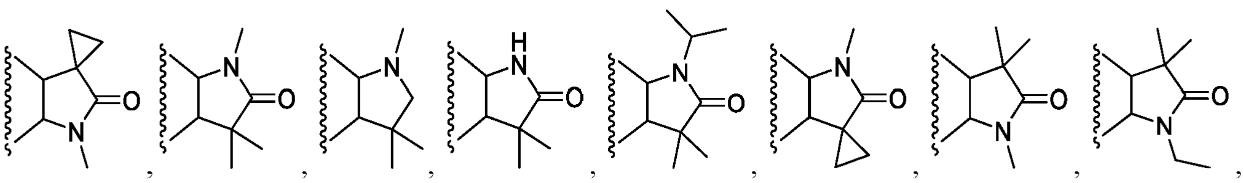
, , , , , , ,

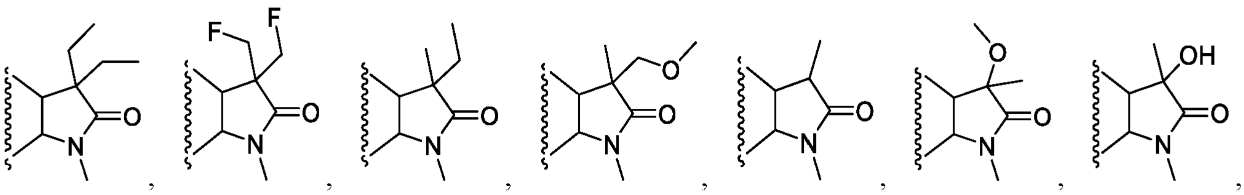
, , , , , , ,

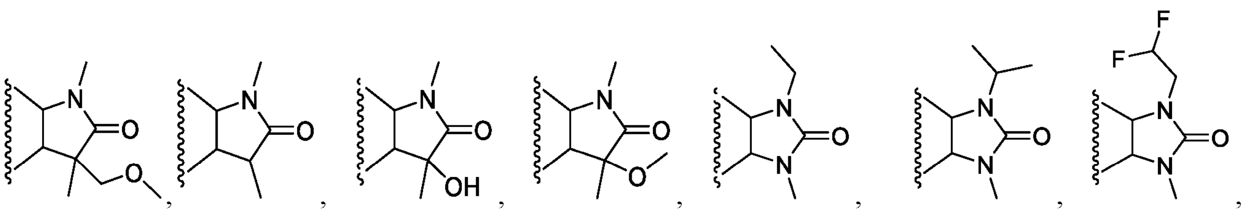
, , , , , , ,

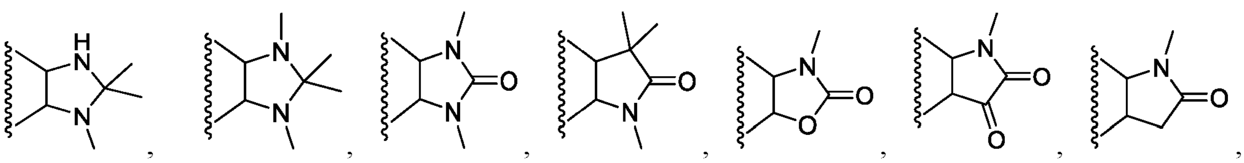
, , , , , , ,

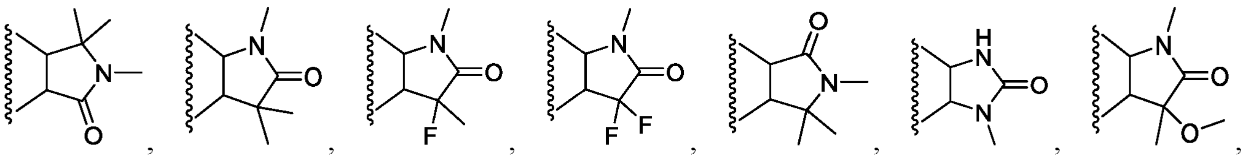
, , , , , , ,

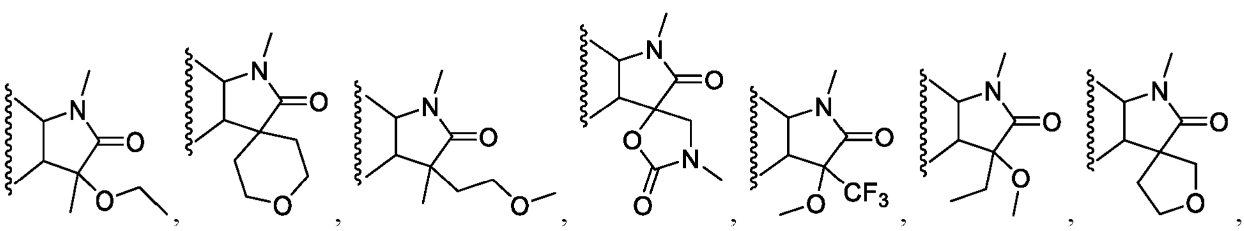
, , , , , , ,

and

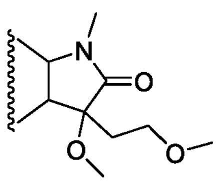;

$R^1$ is selected from methyl, ethyl, isopropyl, and cyclopropyl; $R^2$ and $R^3$ are independently selected from hydrogen, fluorine, and methyl; $R^4$ is selected from fluorine, $-NH_2$, $-CH_3$, $-CF_3$, $-CHF_2$,

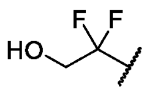, 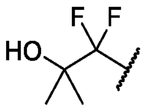, 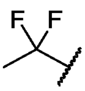, 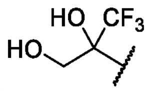, 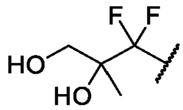, 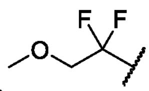, 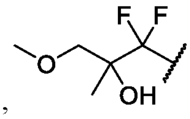,

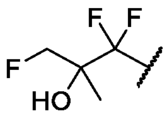 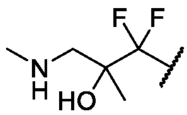, 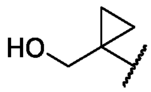, 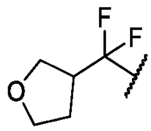, 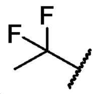, 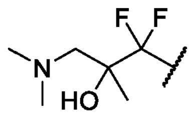, and 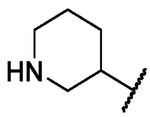.

In another aspects, the invention provides a pharmaceutical composition comprising a compound of the general formula (I), its pharmaceutically acceptable salt, and a pharmaceutically acceptable carrier.

In another aspects, the invention provides a method for the treatment and/or prevention of a disease, disorder, and/or a condition by inhibiting SOS1 in a subject, comprising administering to the subject a therapeutically effective amount of a compound of the general formula (I), its pharmaceutically acceptable salt.

In yet another aspects, the invention provides a method for the treatment and/or prevention of a disease, disorder, and/or a condition by inhibiting the interaction of SOS1 and RAS family protein in a subject, comprising administering to the subject a therapeutically effective amount of a compound of the general formula (I), its pharmaceutically acceptable salt.

In another aspects, the invention provides a method for the treatment and/or prevention of a disease, disorder, and/or a condition, wherein the said disease, disorder, and/or condition is a cancer.

In certain embodiments, a method for the treatment and/or prevention of a disease, disorder, and/or a condition is a cancer, wherein the cancer is selected from the group consisting of pancreatic cancer, lung cancer, colorectal cancer, class 3 BRAF-mutant cancers, hematological cancer, cholangiocarcinoma, multiple myeloma, melanoma, uterine cancer, endometrial cancer, thyroid cancer, acute myeloid leukaemia, bladder cancer, urothelial cancer, gastric cancer, cervical cancer, head and neck squamous cell carcinoma, diffuse large B cell lymphoma, esophageal cancer, chronic lymphocytic leukaemia, hepatocellular cancer, breast cancer, ovarian cancer, prostate cancer, glioblastoma, renal cancer, Pure mucosal neuroma syndrome, Fibrous Epulis, and sarcomas.

In another aspects, the invention provides a method for the treatment and/or prevention of a disease, disorder, and/or a condition, wherein the said disease is Acute Staphylococcus aureus infection (Pediatric Patients), Acute Respiratory Distress syndrome/Acute Lung injury, and Sepsis.

In another aspects, the invention provides a method for the treatment and/or prevention of a disease, disorder, and/or a condition, wherein the said disease, disorder, and/or condition is a RASopathy.

In certain embodiments, a method for the treatment and/or prevention of a disease, disorder, and/or a condition is a RASopathy, wherein the RASopathy is selected from the group consisting of Neurofibromatosis type 1 (NF1), Noonan Syndrome (NS), Noonan Syndrome with Multiple Lentigines (NSML), Capillary Malformation-Arteriovenous Malformation Syndrome (CM-AVM), Costello Syndrome (CS), Cardio-Facio-Cutaneous Syndrome (CFC), Legius Syndrome, Noonan-like/multiple giant cell lesion syndrome and Hereditary Gingival Fibromatosis (HGF).

In another aspects, the invention provides the method, wherein the compound of the general formula (I), its pharmaceutically acceptable salt, is administered before, after, or together with at least one or more pharmacologically active substance.

In another aspects, the invention provides use of a compound of the general formula (I), its pharmaceutically acceptable salt, for the treatment and/or prevention of a disease, disorder, and/or a condition by inhibiting SOS1 in a subject, comprising administering to the subject a therapeutically effective amount of a said compound.

In another aspects, the invention provides use of a compound of the general formula (I), its pharmaceutically acceptable salt, for the treatment and/or prevention of a disease, disorder, and/or condition by inhibiting the interaction of SOS1 and RAS family protein in a subject, comprising administering to the subject a therapeutically effective amount of a said compound.

In another aspects, the invention provides the use of a compound for the treatment and/or prevention of a disease, disorder, and/or condition, wherein the said disease, disorder, and/or condition is cancer.

In certain embodiments, the use of a compound for the treatment and/or prevention of a disease, disorder, and/or condition is cancer, wherein the cancer is selected from the group consisting of pancreatic cancer, lung cancer, colorectal cancer, class 3 BRAF-mutant cancers, hematological cancer, cholangiocarcinoma, multiple myeloma, melanoma, uterine cancer, endometrial cancer, thyroid cancer, acute myeloid leukaemia, bladder cancer, urothelial cancer, gastric cancer, cervical cancer, head and neck squamous cell carcinoma, diffuse large B cell lymphoma, esophageal cancer, chronic lymphocytic leukaemia, hepatocellular cancer, breast cancer, ovarian cancer, prostate cancer, glioblastoma, renal cancer, Pure mucosal neuroma syndrome, Fibrous Epulis, and sarcomas.

In another aspects, the invention provides the use of a compound for the treatment and/or prevention of a disease, disorder, and/or condition, wherein the said disease is Acute Staphylococcus aureus infection (Pediatric Patients), Acute Respiratory Distress syndrome/Acute Lung injury, and Sepsis.

In another aspects, the invention provides the use of a compound for the treatment and/or prevention of a disease, disorder, and/or condition, wherein the said the disease is a RASopathy.

In certain embodiments, the use of a compound for the treatment and/or prevention of a disease, disorder, and/or condition is a RASopathy, wherein the RASopathy is selected from the group consisting of Neurofibromatosis type 1 (NF1), Noonan Syndrome (NS), Noonan Syndrome with Multiple Lentigines (NSML), Capillary Malformation-Arteriovenous Malformation Syndrome (CM-AVM), Costello Syndrome (CS), Cardio-Facio-Cutaneous Syndrome (CFC), Legius Syndrome, Noonan-like/multiple giant cell lesion syndrome and Hereditary gingival fibromatosis (HGF).

In another aspects, the invention provides the compound of the general formula (I), its pharmaceutically acceptable salt, for treatment and/or prevention of cancer, wherein said compound is administered in combination with at least one more pharmacologically active substance.

In another aspects, the invention provides the compound of the general formula (I), its pharmaceutically acceptable salt, for treatment and/or prevention of cancer, wherein the compound is administered before, after, or together with at least one other pharmacologically active substance.

Whenever a range of the number of atoms in a structure is indicated (e.g., a $C_1$ to $C_{20}$ alkyl etc.), it is specifically contemplated that any sub-range or individual number of carbon atoms falling within the indicated range also can be used. Thus, for instance, the recitation of a range of 1-6 carbon atoms (e.g., $C_1$ to $C_6$), 2-6 carbon atoms (e.g., $C_2$ to $C_6$), 3-6 carbon atoms (e.g., $C_3$ to $C_6$), as used with respect to any chemical group (e.g., alkyl etc.) referenced herein encompasses and specifically describes 1, 2, 3, 4, 5, and/or 6 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 2-3 carbon atoms, 2-4 carbon atoms, 2-5 carbon atoms, 2-6 carbon atoms, 3-4 carbon atoms, 3-5 carbon atoms, 3-6 carbon atoms, 4-5 carbon atoms, 4-6 carbon atoms, as appropriate).

General terms used in formula can be defined as follows; however, the meaning stated should not be interpreted as limiting the scope of the term *per se.*

The term 'alkyl', as used herein, means a straight chain or branched hydrocarbon containing from 1 to 20 carbon atoms. Preferably, the alkyl chain may contain 1 to 10 carbon atoms. More preferably, alkyl chain may contain up to 6 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, and n-hexyl.

The term 'haloalkyl', as used herein means an alkyl group as defined hereinabove wherein at least one of the hydrogen atoms of the said alkyl group is substituted with halogen. The haloalkyl group is exemplified by chloromethyl, 1-chloroethyl, and the like.

The term 'perhaloalkyl', as used herein, means an alkyl group as defined hereinabove wherein all the hydrogen atoms of the said alkyl group are substituted with halogen. The perhaloalkyl group is exemplified by trifluoromethyl, pentafluoroethyl, and the like.

The term 'carbocycle' or 'carbocyclic ring' as used herein, means a monocyclic, bicyclic, or tricyclic saturated or unsaturated non-aromatic ring system containing from 3 to 14 carbon atoms, preferably monocyclic cycloalkyl ring containing 3 to 6 carbon atoms. Examples of monocyclic ring systems include cyclopropyl, cyclobutyl, cyclopentyl, cyclohexyl, cycloheptyl, and cyclooctyl. Bicyclic ring systems include monocyclic ring system fused across a bond with another cyclic system which may be an alicyclic ring or an aromatic ring. Bicyclic rings also include spirocyclic systems wherein the second ring gets annulated on a single carbon atom. Bicyclic ring systems are also exemplified by a bridged monocyclic ring system in which two non-adjacent carbon atoms of the monocyclic ring are linked by an alkylene bridge. Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane, bicyclo[3.3.2]decane, bicyclo[3.1.0]hexane, bicyclo[4.1.0]heptane, bicyclo[3.2.0]heptanes, octahydro-1H-indene, spiro[2.5]octane, spiro[4.5]decane, spiro[bicyclo[4.1.0]heptane-2,1'-cyclopentane], hexahydro-2'H-spiro[cyclopropane-1,1'-pentalene]. Tricyclic ring systems are the systems wherein the bicyclic systems as described above are further annulated with third ring, which may be an alicyclic ring or aromatic ring. Tricyclic ring systems are also exemplified by a bicyclic ring system in

which two non-adjacent carbon atoms of the bicyclic ring are linked by a bond or an alkylene bridge. Representative examples of tricyclic-ring systems include, but are not limited to, tricyclo[3.3.1.$0^{3.7}$]nonane, and tricyclo[3.3.1.$1^{3.7}$]decane (adamantane).

The term "cycloalkyl" as used herein, means a monovalent carbocyclic ring.

The term 'aryl', as used herein, refers to a monovalent monocyclic, bicyclic or tricyclic aromatic hydrocarbon ring system. Examples of aryl groups include phenyl, naphthyl, anthracenyl, fluorenyl, indenyl, azulenyl, and the like. Aryl group also include partially saturated bicyclic and tricyclic hydrocarbon ring systems with at least one aromatic ring, e.g. tetrahydro-naphthalene. Aryl group also include bicyclic systems like 2,3-dihydro-indene-5-yl, and 2,3-dihydro-1-indenone-5-yl.

The term 'heteroaryl', as used herein, refers to a 5-14 membered monocyclic, bicyclic, or tricyclic ring system having 1-4 ring heteroatoms selected from O, N, or S, and the remainder ring atoms being carbon (with appropriate hydrogen atoms unless otherwise indicated), wherein at least one ring in the ring system is aromatic. The term 'heteroaryl' as used herein, also include partially saturated bicyclic and tricyclic aromatic ring system, e.g. 2,3-dihydro-isobenzofuran-5-yl, 2,3-dihydro-1-isobenzofuranone-5-yl, 2,3-dihydro-1H-indol-4-yl, 2,3-dihydro-1H-indol-6-yl, and 2,3-dihydro-1-isoindolinone-5-yl. Heteroaryl groups may be optionally substituted with one or more substituents. In one embodiment, 0, 1, 2, 3, or 4 atoms of each ring of a heteroaryl group may be substituted by a substituent. Examples of heteroaryl groups include, but not limited to, 1H-1,2,3-triazolyl, 2H-1,2,3-triazolyl, pyridyl, 1-oxo-pyridyl, furanyl, thienyl, pyrrolyl, oxazolyl, oxadiazolyl, imidazolyl, thiazolyl, isoxazolyl, quinolinyl, pyrazolyl, isothiazolyl, pyridazinyl, pyrimidinyl, pyrazinyl, triazinyl, triazolyl, thiadiazolyl, isoquinolinyl, benzoxazolyl, benzofuranyl, indolizinyl, imidazopyridyl, imidazolyl, tetrazolyl, benzimidazolyl, benzothiazolyl, benzothiadiazolyl, benzoxadiazolyl, indolyl, azaindolyl, imidazopyridyl, quinazolinyl, purinyl, pyrrolo[2,3]pyrimidinyl, pyrazolo[3,4]pyrimidinyl, and benzo(b)thienyl, 2,3-thiadiazolyl, 1H-pyrazolo[5,1-c]-1,2,4-triazolyl, pyrrolo[3,4-d]-1,2,3-triazolyl, cyclopentatriazolyl, 3H-pyrrolo[3,4-c] isoxazolyl, 2,3-dihydro-benzo[1,4]dioxin-6-yl, 2,3-dihydro-benzo[1,4]dioxin-5-yl, 2,3-dihydro-benzofuran-5-yl, 2,3-dihydro-benzofuran-4-yl, 2,3-dihydro-benzofuran-6-yl, 2,3-dihydro-benzofuran-6-yl, 2,3-dihydro-isobenzofuran-5-yl, 2,3-dihydro-1-isobenzofuranone-5-yl, 2,3-dihydro-1H-indol-5-yl, 2,3-dihydro-1H-indol-4-yl, 2,3-dihydro-1H-indol-6-yl, 2,3-dihydro-1H-indol-7-yl, 2,3-dihydro-1-isoindolinone-5-yl, benzo[1,3]dioxol-4-yl, benzo[1,3]dioxol-5-yl, 1,2,3,4-tetrahydroquinolinyl, 1,2,3,4-tetrahydroisoquinolinyl, 2,3-dihydrobenzothien-4-yl, 2-oxoindolin-5-yl and the like.

The term 'heterocycle' or 'heterocyclic ring' or 'heterocyclyl' as used herein, means a 'carbocycle' or 'carbocyclic ring' or 'cycloalkyl' group wherein one or more of the carbon atoms are replaced by heteroatoms/groups selected from N, S, $SO_2$, and O. The heterocycle may be connected to the parent molecular moiety through any carbon atom or any nitrogen atom contained within the heterocycle. Representative examples of monocyclic heterocycle include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyranyl, pyrazolinyl, pyrazolidinyl, pyrrolinyl, pyrrolidinyl, tetrahydrofuranyl, tetrahydrothienyl, thiadiazolinyl, thiadiazolidinyl, thiazolinyl, thiazolidinyl, thiomorpholinyl, 1.1-dioxidothiomorpholinyl (thiomorpholine sulfone), thiopyranyl, and trithianyl. Representative examples of bicyclic heterocycle include, but are not limited to, 1,2,3,4-tetrahydroisoquinolin-2-yl, 1,2,3,4-tetrahydroquinolin-1-yl, 1,3-benzodioxolyl, 1,3-benzodithiolyl, 2,3-dihydro-1,4-benzodioxinyl, 2,3-dihydro-1-benzofuranyl, 2,3-dihydro-1-benzothienyl, 2,3-dihydro-1H-indolyl, and 1,2,3,4-tetrahydroquinolinyl. The term heterocycle also includes bridged and spiro heterocyclic systems such as azabicyclo[3.2.1]octane, azabicyclo[3.3.1]nonane, 8-oxa-3-azabicyclo[3.2.1]octan-3-yl, 3-oxa-8-azabicyclo[3.2.1]octan-8-yl, 6-oxa-3-azabicyclo[3.1.1]heptan-3-yl, 8-azabicyclo[3.2.1]octan-8-yl, 3-azabicyclo[3.2.1]octan-3-yl, 3-azabicyclo[3.1.0]hexan-3-yl, 6-azaspiro[2.5]octan-6-yl, 5-azaspiro[2.5]octan-5-yl, 4-azaspiro[2.4]heptan-4-yl, 2-oxa-6-azaspiro[3.3]heptan-6-yl, tetrahydrofuran-3-yl, oxetan-3-yl, 1-oxa-8-azaspiro[4.5]decan-8-yl, 8-oxa-2-azaspiro[4.5]decan-2-yl, tetrahydro-2H-pyran-4-yl, 2-azaspiro[3.3]heptan-6-ol-2-yl, morpholin-3-one-4-yl, 1-methylpyridin-2(1H)-one-5-yl, 1-methyl-1,2,3,6-tetrahydropyridin-4-yl, 3,6-dihydro-2H-pyran-4-yl, pyridin-2(1H)-one-5-yl, pyridin-2(1H)-one-4-yl, and the like.

The 'halogen' means fluorine, chlorine, bromine, or iodine.

The term 'oxo' means a divalent oxygen (=O) attached to the parent group. For example, oxo attached to carbon forms a carbonyl, oxo substituted on cyclohexane forms a cyclohexanone, and the like.

The term 'annulated' means the ring system under consideration is either annulated with another ring at a carbon atom of the cyclic system or across a bond of the cyclic system as in the case of fused or spiro ring systems.

The term 'bridged' means the ring system under consideration contain an alkylene bridge having 1 to 4 methylene units joining two non-adjacent ring atoms.

A compound, its tautomeric form, its stereoisomer, its pharmaceutically acceptable salt, its polymorph, its solvate, its combination with suitable medicament, its pharmaceutical composition thereof as described hereinabove wherein the compound of general formula (I), is selected from the group consisting of:

(R)-4-((1-(3-amino-5-(trifluoromethyl)phenyl)ethyl)amino)-2,6-dimethyl-6H-[1,4]oxazino[3,2-g]quinazolin-7(8H)-one (Compound 1);

(R)-4-((1-(3-(1,1-difluoro-2-hydroxyethyl)-2-fluorophenyl)ethyl)amino)-2,6-dimethyl-6H-[1,4]oxazino[3,2-g]quinazolin-7(8H)-one (Compound 2);

(R)-4-((1-(3-(1,1-difluoro-2-hydroxy-2-methylpropyl)-2-fluorophenyl)ethyl)amino)-2,6-dimethyl-6H-[1,4]oxazino[3,2-g]quinazolin-7(8H)-one (Compound 3);

4-((1-(3-(1,1-difluoroethyl)-2-fluorophenyl)ethyl)amino)-2,6-dimethyl-6H-[1,4]oxazino[3,2-g]quinazolin-7(8H)-one (Compound 4);

4-((1-(3-amino-5-(difluoromethyl)phenyl)ethyl)amino)-2,6-dimethyl-6H-[1,4]oxazino[3,2-g]quinazolin-7(8H)-one (Compound 5);

4-((1-(3-(1,1-difluoro-2-hydroxyethyl)-2-methylphenyl)ethyl)amino)-2,6-dimethyl-6H-[1,4]oxazino[3,2-g]quinazolin-7(8H)-one (Compound 6);

2,6-dimethyl-4-((1-(3-(trifluoromethyl)phenyl)ethyl)amino)-6H-[1,4]oxazino[3,2-g]quinazolin-7(8H)-one (Compound 7);

4-((1-(2-fluoro-3-(trifluoromethyl)phenyl)ethyl)amino)-2,6-dimethyl-6H-[1,4]oxazino[3,2-g]quinazolin-7(8H)-one (Compound 8);

4-((1-(3,3-difluoro-2,3-dihydrobenzofuran-7-yl)ethyl)amino)-2,6-dimethyl-6H-[1,4]oxazino[3,2-g]quinazolin-7(8H)-one (Compound 9);

(R)-N-(1-(3-amino-5-(trifluoromethyl)phenyl)ethyl)-2,6-dimethyl-7,8-dihydro-6H-[1,4]oxazino[3,2-g]quinazolin-4-amine (Compound 10);

4-(((R)-1-(3-amino-5-(trifluoromethyl)phenyl)ethyl)amino)-2-methyl-6-((tetrahydrofuran-3-yl)methyl)-6H-[1,4]oxazino[3,2-g]quinazolin-7(8H)-one (Compound 11);

(R)-4-((1-(3-amino-5-(trifluoromethyl)phenyl)ethyl)amino)-6-(cyclopropylmethyl)-2-methyl-6H-[1,4]oxazino[3,2-g]quinazolin-7(8H)-one (Compound 12);

(R)-4-((1-(3-(1,1-difluoro-2-hydroxyethyl)-2-fluorophenyl)ethyl)amino)-6-ethyl-2-methyl-6H-[1,4]oxazino[3,2-g]quinazolin-7(8H)-one (Compound 13);

(R)-4-((1-(3-amino-5-(trifluoromethyl)phenyl)ethyl)amino)-2-ethyl-6-methyl-6H-[1,4]oxazino[3,2-g]quinazolin-7(8H)-one. (Compound 14);

(R)-4-((1-(3-(1,1-difluoro-2-hydroxyethyl)-2-fluorophenyl)ethyl)amino)-2-ethyl-6-methyl-6H-[1,4]oxazino[3,2-g]quinazolin-7(8H)-one (Compound 15);

(R)-2-cyclopropyl-4-((1-(3-(1,1-difluoro-2-hydroxy-ethyl)-2-fluorophenyl)ethyl)amino)-6-methyl-6H-[1,4]oxazino[3,2-g]quinazolin-7(8H)-one (Compound 16);

(4-(((R)-1-(3-amino-5-(trifluoromethyl)phenyl)ethyl)amino)-2-methyl-8,9-dihydro-7H-pyrano[2,3-g]quinazolin-7-yl)(pyrrolidin-1-yl)methanone (Compound 17);

(R)-4-((1-(3-(1,1-difluoro-2-hydroxyethyl)-2-fluorophenyl)ethyl)amino)-2,6,8,8-tetramethyl-6H-[1,4]oxazino[3,2-g]quinazolin-7(8H)-one (Compound 18);

2,6,8,8-tetramethyl-4-((1-(3-(trifluoromethyl)phenyl)ethyl)amino)-6H-[1,4]oxazino[3,2-g]quinazolin-7(8H)-one (Compound 19);

4-((1-(2-fluoro-3-(trifluoromethyl)phenyl)ethyl)amino)-2,6,8,8-tetramethyl-6H-[1,4]oxazino[3,2-g]quinazolin-7(8H)-one (Compound 20);

(R)-4-((1-(3-amino-5-(trifluoromethyl)phenyl)ethyl)amino)-2,6,8,8-tetramethyl-6H-[1,4]oxazino[3,2-g]quinazolin-7(8H)-one (Compound 21);

4-((1-(2-fluoro-3-(1,1,1-trifluoro-2,3-dihydroxypropan-2-yl)phenyl)ethyl)amino)-2,6,8,8-tetramethyl-6H-[1,4]oxazino[3,2-g]quinazolin-7(8H)-one (Compound 22);

4-((1-(3-(1,1-difluoro-2,3-dihydroxy-2-methylpropyl)-2-fluorophenyl)ethyl)amino)-2,6,8,8-tetramethyl-6H-[1,4]oxazino[3,2-g]quinazolin-7(8H)-one (Compound 23);

(R)-N-(1-(3-amino-5-(trifluoromethyl)phenyl)ethyl)-2,6,8,8-tetramethyl-7,8-dihydro-6H-[1,4]oxazino[3,2-g]quinazolin-4-amine (Compound 24);

(R)-4'-((1-(3-(1,1-difluoro-2-hydroxyethyl)-2-fluorophenyl)ethyl)amino)-2',6'-dimethylspiro[cyclopropane-1,8'-[1,4]oxazino[3,2-g]quinazolin]-7'(6'H)-one (Compound 25);

(R)-1,1-Difluoro-1-(2-fluoro-3-(1-((2,8,8-trimethyl-7-morpholino-8H [1,4]oxazino[3,2-g]quinazolin-4-yl)amino)ethyl)phenyl)-2-methylpropan-2-ol (Compound 26);

4-(((R)-1-(3-(1,1-difluoro-2-hydroxy-2-methylpropyl)-2-fluorophenyl)ethyl)amino)-2,6,8,9-tetramethyl-8,9-dihydropyrazino[2,3-g]quinazolin-7(6H)-one (Compound 27);

(R)-4-((1-(3-(1,1-difluoro-2-hydroxy-2-methylpropyl)-2-fluorophenyl) ethyl)amino)-2,6,8,8,9-pentamethyl-8,9-dihydropyrazino[2,3-g]quinazolin-7(6H)-one (Compound 28);

(R)-9-((1-(3-(1,1-difluoro-2-hydroxy-2-methylpropyl)-2-fluorophenyl) ethyl)amino)-1,3,7-trimethylpyrimido[4,5-g]quinazoline-2,4(1H,3H)-dione (Compound 29);

4-(((R)-1-(3-(1,1-difluoro-2-hydroxyethyl)-2-fluorophenyl)ethyl)amino)-2,6,8-trimethyl-6H-[1,4]oxazino[3,2-g]quinazolin-7(8H)-one (Compound 30);

4-(((R)-1-(3-(1,1-difluoro-2-hydroxy-2-methylpropyl)-2-fluorophenyl)ethyl)amino)-2,6,8-trimethyl-6H-[1,4]oxazino[3,2-g]quinazolin-7(8H)-one (Compound 31);

4-(((R)-1-(3-(1,1-difluoro-2-hydroxy-2-methylpropyl)-2-fluorophenyl)ethyl)amino)-8-isopropyl-2,6-dimethyl-6H-[1,4]oxazino[3,2-g]quinazolin-7(8H)-one (Compound 32);

(R)-4-((1-(3-(1,1-Difluoro-2-hydroxy-2-methylpropyl)-2-fluorophenyl)ethyl) amino)-2,6,8,8-tetramethyl-6H-[1,4]oxazino[3,2-g]quinazolin-7(8H)-one (Compound 33);

(R)-1,1-Difluoro-1-(2-fluoro-3-(1-((2,6,8,8-tetramethyl-7,8-dihydro-6H-[1,4]oxazino [3,2-g]quinazolin-4-yl)amino)ethyl)phenyl)-2-methylpropan-2-ol (Compound 34);

(R)-2,2-Difluoro-2-(2-fluoro-3-(1-((2,6,8,8-tetramethyl-7,8-dihydro-6H-[1,4]oxazino[3,2-g]quinazolin-4-yl)amino)ethyl)phenyl)ethan-1-ol (Compound 35);

2,2-difluoro-2-(2-fluoro-3-((1R)-1-((2,6,8-trimethyl-7,8-dihydro-6H-[1,4]oxazino[3,2-g]quinazolin-4-yl)amino)ethyl)phenyl)ethan-1-ol (Compound 36);

(R)-1,1-Difluoro-1-(2-fluoro-3-(1-((2,6,7,7-tetramethyl-7,8-dihydro-6H-[1,4]oxazino[3,2-g]quinazolin-4-yl)amino)ethyl)phenyl)-2-methylpropan-2-ol (Compound 37);

(R)-4-((1-(3-(1,1-Difluoro-2-hydroxy-2-methylpropyl)-2-fluorophenyl)ethyl)amino)-2,6,8-trimethylpyrido[2,3-g]quinazolin-7(6H)-one (Compound 38);

(R)-4-((1-(3-(1,1-Difluoro-2-hydroxy-2-methylpropyl)-2-fluorophenyl)ethyl)amino)-2,6,8-trimethylpyrazino[2,3-g]quinazolin-7(6H)-one (Compound 39);

(R)-4-((1-(3-(1,1-Difluoro-2-hydroxy-2-methylpropyl)-2-fluorophenyl)ethyl) amino)-2,6,9-trimethyl-6,9-dihydropyrazino[2,3-g]quinazoline-7,8-dione (Compound 40);

(R)-4-((1-(3-(1,1-difluoro-2-hydroxy-2-methylpropyl)-2-fluorophenyl)ethyl)amino)-6-ethyl-2,8,8-trimethyl-6H-[1,4]oxazino[3,2-g]quinazolin-7(8H)-one (Compound 41);

(R)-4-((1-(3-(1,1-difluoro-2-hydroxy-2-methylpropyl)-2-fluorophenyl)ethyl)amino)-2,6,9,9-tetramethyl-8,9-dihydropyrido[2,3-g]quinazolin-7(6H)-one (Compound 42);

N-((R)-1-(3-amino-5-(trifluoromethyl)phenyl)ethyl)-2-methyl-6-(((R/S)-tetrahydrofuran-3-yl)methyl)-7,8-dihydro-6H-[1,4]oxazino[3,2-g]quinazolin-4-amine (Compound 43);

N-((R)-1-(3-amino-5-(trifluoromethyl)phenyl)ethyl)-2-methyl-6-(((S/R)-tetrahydrofuran-3-yl)methyl)-7,8-dihydro-6H-[1,4]oxazino[3,2-g]quinazolin-4-amine (Compound 44);

(R)-1-(3-(1-((2,6-dimethyl-7,8-dihydro-6H-[1,4]oxazino[3,2-g]quinazolin-4-yl)amino)ethyl)-2-fluorophenyl)-1,1-difluoro-2-methylpropan-2-ol (Compound 45);

4-(((R)-1-(3-amino-5-(trifluoromethyl)phenyl)ethyl)amino)-2,6,8-trimethyl-6H-[1,4]oxazino[3,2-g]quinazolin-7(8H)-one (Compound 46);

(R)-4-((1-(3-(1,1-difluoro-2-hydroxy-2-methylpropyl)-2-fluoro phenyl)ethyl)amino)-10-fluoro-2,6-dimethyl-6H-[1,4]oxazino[3,2-g]quinazolin-7(8H)-one (Compound 47);

(R)-4-((1-(3-(1,1-difluoro-2-hydroxyethyl)-2-fluorophenyl)ethyl)amino)-6-(2-methoxyethyl)-2-methyl-6H-[1,4]oxazino[3,2-g]quinazolin-7(8H)-one (Compound 48);

(R)-4-((1-(3-amino-5-(trifluoromethyl)phenyl)ethyl)amino)-6-ethyl-2-methyl-6H-[1,4]oxazino[3,2-g]quinazolin-7(8H)-one (Compound 49);

(R)-1-(3-(1-((6-ethyl-2,8,8-trimethyl-7,8-dihydro-6H-[1,4]oxazino[3,2-g]quinazolin-4-yl)amino)ethyl)-2-fluorophenyl)-1,1-difluoro-2-methylpropan-2-ol (Compound 50);

(R)-4-((1-(3-(1,1-difluoro-2-methoxyethyl)-2-fluorophenyl)ethyl)amino)-2,6,8,8-tetramethyl-6H-[1,4]oxazino[3,2-g]quinazolin-7(8H)-one (Compound 51);

(R)-N-(1-(3-(1,1-difluoro-2-methoxyethyl)-2-fluorophenyl)ethyl)-2,6,8,8-tetramethyl-7,8-dihydro-6H-[1,4]oxazino[3,2-g]quinazolin-4-amine (Compound 52);

4-(((1R)-1-(3-(1,1-difluoro-2-hydroxy-3-methoxy-2-methylpropyl)-2-fluorophenyl)ethyl)amino)-2,6,8,8-tetramethyl-6H-[1,4]oxazino[3,2-g]quinazolin-7(8H)-one (Compound 53);

4-(((1R)-1-(2-fluoro-3-(1,1,3-trifluoro-2-hydroxy-2-methylpropyl)phenyl)ethyl)amino)-2,6,8,8-tetramethyl-6H-[1,4]oxazino[3,2-g]quinazolin-7(8H)-one (Compound 54);

4-(((1R)-1-(3-(1,1-difluoro-2-hydroxy-2-methyl-3-(methylamino)propyl)-2-fluorophenyl)ethyl)amino)-2,6,8,8-tetramethyl-6H-[1,4]oxazino[3,2-g]quinazolin-7(8H)-one (Compound 55);

(R)-2,2-difluoro-2-(2-fluoro-3-(1-((2-methyl-7,8-dihydro-6H-pyrano[3,2-g]quinazolin-4-yl)amino)ethyl)phenyl)ethan-1-ol (Compound 56);

(R)-4-((1-(3-(1,1-difluoro-2-hydroxy-2-methylpropyl)phenyl)ethyl)amino)-2,6,8,8-tetramethyl-6H-[1,4]oxazino[3,2-g]quinazolin-7(8H)-one (Compound 57);

(R)-4'-((1-(3-(1,1-difluoro-2-hydroxy-2-methylpropyl)-2-fluorophenyl) ethyl)amino)-2',8'-dimethylspiro[cyclopentane-1,6'-pyrrolo[3,2-g]quinazolin]-7'(8'H)-one (Compound 58);

4'-((1-(2-fluoro-3-(trifluoromethyl)phenyl)ethyl)amino)-2',8'-dimethylspiro[cyclopentane-1,6'-pyrrolo[3,2-g]quinazolin]-7'(8'H)-one (Compound 59);

2',8'-dimethyl-4'-((1-(2-methyl-3-(trifluoromethyl)phenyl)ethyl)amino)spiro[cyclopentane-1,6'-pyrrolo[3,2-g]quinazolin]-7'(8'H)-one (Compound 60);

4'-((1-(3-(difluoromethyl)-2-methylphenyl)ethyl)amino)-2',8'-dimethylspiro[cyclopentane-1,6'-pyrrolo[3,2-g]quinazolin]-7'(8'H)-one (Compound 61);

(R)-4'-((1-(3-(1,1-difluoro-2-hydroxyethyl)-2-fluorophenyl)ethyl)amino)-2',8'-dimethylspiro[cyclopentane-1,6'-pyrrolo[3,2-g]quinazolin]-7'(8'H)-one (Compound 62);

(R)-4-((1-(3-(1,1-difluoro-2-hydroxy-2-methylpropyl)-2-fluorophenyl) ethyl)amino)-6-(2-methoxyethyl)-2,8,8-trimethyl-6,8-dihydro-7H-pyrrolo[2,3-g]quinazolin-7-one (Compound 63);

(R)-4-((1-(3-(1,1-difluoro-2-hydroxy-2-methylpropyl)-2-fluorophenyl)ethyl)amino)-6-ethyl-2,8,8-trimethyl-6,8-dihydro-7H-pyrrolo[2,3-g]quinazolin-7-one (Compound 64);

(R)-6-cyclopropyl-4-((1-(3-(1,1-difluoro-2-hydroxy-2-methylpropyl)-2-fluoro phenyl)ethyl)amino)-2,8,8-trimethyl-6,8-dihydro-7H-pyrrolo[2,3-g]quinazolin-7-one (Compound 65);

(R)-4'-((1-(3-(1,1-difluoro-2-hydroxyethyl)-2-fluorophenyl)ethyl)amino)-2',8'-dimethylspiro [cyclopropane-1,6'-pyrrolo[3,2-g]quinazolin]-7'(8'H)-one (Compound 66);

(R)-4'-((1-(3-(1,1-difluoro-2-hydroxy-2-methylpropyl)-2-fluorophenyl)ethyl)amino)-2',8'-dimethylspiro[cyclopropane-1,6'-pyrrolo[3,2-g]quinazolin]-7'(8'H)-one (Compound 67);

(R)-4-((1-(3-(1,1-Difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one (Compound 68);

4-((1-(3-amino-5-(difluoromethyl)phenyl)ethyl)amino)-2,6,8,8-tetramethyl-6,8-dihydro-7H-pyrrolo[2,3-g]quinazolin-7-one (Compound 69);

(R)-4-((1-(3-amino-5-(trifluoromethyl)phenyl)ethyl)amino)-2,6,8,8-tetramethyl-6H-pyrrolo[2,3-g]quinazolin-7(8H)-one (Compound 70);

(S)-4-((1-(3-(1,1-difluoro-2-hydroxy-2-methylpropyl)-2-fluorophenyl)ethyl)amino)-2,6,8,8-tetramethyl-6H-pyrrolo [2,3-g]quinazolin-7(8H)-one (Compound 71);

(R)-4-((1-(3-(1,1-difluoro-2-hydroxyethyl)-2-fluorophenyl)ethyl)amino)-2,6,8,8-tetramethyl-6,8-dihydro-7H-pyrrolo [2,3-g]quinazolin-7-one (Compound 72);

4-((1-(3-(1,1-difluoro-2-hydroxy-2-methylpropyl)phenyl)ethyl)amino)-2,6,8,8-tetramethyl-6,8-dihydro-7H-pyrrolo [2,3-g]quinazolin-7-one (Compound 73);

4-((1-(2-fluoro-3-(1,1,1-trifluoro-2,3-dihydroxypropan-2-yl)phenyl)ethyl)amino)-2,6,8,8-tetramethyl-6,8-dihydro-7H-pyrrolo[2,3-g]quinazolin-7-one (Compound 74);

(R)-1,1-difluoro-1-(2-fluoro-3-(1-((2,6,8,8-tetramethyl-7,8-dihydro-6H-pyrrolo[2,3-g]quinazolin-4-yl)amino)ethyl) phenyl)-2-methylpropan-2-ol (Compound 75);

4-(((1R)-1-(3-(1,1-difluoro-2,3-dihydroxy-2-methylpropyl)-2-fluorophenyl)ethyl)amino)-2,6,8,8-tetramethyl-6,8-dihydro-7H-pyrrolo[2,3-g]quinazolin-7-one (Compound 76);

(R)-4-((1-(3-(1,1-difluoro-2-hydroxy-2-methylpropyl)-2-fluoro phenyl)ethyl)amino)-2,8,8-trimethyl-6H-pyrrolo[2,3-g] quinazolin-7(8H)-one (Compound 77);

(R)-4-((1-(3-(1,1-difluoro-2-hydroxy-2-methylpropyl)-2-fluoro phenyl)ethyl)amino)-6-isopropyl-2,8,8-trimethyl-6H-pyrrolo[2,3-g]quinazolin-7(8H)-one (Compound 78);

(R)-4'-((1-(3-(1,1-difluoro-2-hydroxy-2-methylpropyl)-2-fluorophenyl)ethyl)amino)-2',6'-dimethylspiro[cyclopropane-1,8'-pyrrolo[2,3-g]quinazolin]-7'(6'H)-one (Compound 79);

(R)-4-((1-(3-(1,1-difluoro-2-hydroxyethyl)-2-fluorophenyl)ethyl)amino)-2,6,6,8-tetramethyl-6,8-dihydro-7H-pyrrolo [3,2-g]quinazolin-7-one (Compound 80);

(R)-4-((1-(3-(1,1-difluoro-2-hydroxy-2-methylpropyl)-2-fluorophenyl)ethyl)amino)-2,6,6,8-tetramethyl-6,8-dihydro-7H-pyrrolo[3,2-g]quinazolin-7-one (Compound 81);

4-((1-(2-fluoro-3-(trifluoromethyl)phenyl)ethyl)amino)-2,6,6,8-tetramethyl-6,8-dihydro-7H-pyrrolo[3,2-g]quinazolin-7-one (Compound 82);

2,6,6,8-tetramethyl-4-((1-(2-methyl-3-(trifluoromethyl)phenyl)ethyl)amino)-6,8-dihydro-7H-pyrrolo[3,2-g]quinazolin-7-one (Compound 83);

4-((1-(3-(1,1-difluoroethyl)-2-fluorophenyl)ethyl)amino)-2,6,6,8-tetramethyl-6,8-dihydro-7H-pyrrolo[3,2-g]quinazolin-7-one (Compound 84);

4-((1-(3-(difluoro(tetrahydrofuran-3-yl)methyl)-2-fluorophenyl)ethyl)amino)-2,6,6,8-tetramethyl-6,8-dihydro-7H-pyrrolo[3,2-g]quinazolin-7-one (Compound 85);

(R)-4-((1-(3-(1,1-difluoro-2-hydroxy-2-methylpropyl)-2-fluorophenyl) ethyl)amino)-2-ethyl-6,6,8-trimethyl-6,8-dihydro-7H-pyrrolo[3,2-g]quinazolin-7-one (Compound 86);

(R)-4-((1-(3-(1,1-difluoro-2-hydroxy-2-methylpropyl)-2-fluorophenyl)ethyl)amino)-2-isopropyl-6,6,8-trimethyl-6H-pyrrolo[3,2-g]quinazolin-7(8H)-one (Compound 87);

(R)-4-((1-(3-(1,1-difluoro-2-hydroxyethyl)-2-fluorophenyl)ethyl)amino)-8-ethyl-2,6,6-trimethyl-6,8-dihydro-7H-pyrrolo[3,2-g]quinazolin-7-one (Compound 88);

(R)-4-((1-(3-(1,1-difluoro-2-hydroxy-2-methylpropyl)-2-fluorophenyl) ethyl)amino)-8-ethyl-2,6,6-trimethyl-6H-pyrrolo[3,2-g]quinazolin-7(8H)-one (Compound 89);

(R)-4-((1-(3-(1,1-difluoro-2-hydroxy-2-methylpropyl)-2-fluorophenyl)ethyl)amino)-2,6,6,8,9-pentamethyl-6,8-dihydro-7H-pyrrolo[3,2-g]quinazolin-7-one (Compound 90);

(R)-4-((1-(3-(1,1-difluoro-2-hydroxy-2-methylpropyl)-2-fluorophenyl)ethyl)amino)-6,6-diethyl-2,8-dimethyl-6,8-dihydro-7H-pyrrolo[3,2-g]quinazolin-7-one (Compound 91);

(R)-4-((1-(3-(1,1-difluoro-2-hydroxy-2-methylpropyl)-2-fluorophenyl)ethyl)amino)-6,6-bis(fluoromethyl)-2,8-dimethyl-6,8-dihydro-7H-pyrrolo[3,2-g]quinazolin-7-one (Compound 92);

4-(((R)-1-(3-(1,1-difluoro-2-hydroxy-2-methylpropyl)-2-fluorophenyl)ethyl)amino)-6-ethyl-2,6,8-trimethyl-6,8-dihydro-7H-pyrrolo[3,2-g]quinazolin-7-one (Compound 93);

4-(((R)-1-(3-(1,1-difluoro-2-hydroxy-2-methylpropyl)-2-fluorophenyl)ethyl)amino)-6-(methoxymethyl)-2,6,8-trimethyl-6,8-dihydro-7H-pyrrolo[3,2-g]quinazolin-7-one (Compound 94);

4-(((R)-1-(3-(1,1-difluoro-2-hydroxy-2-methylpropyl)-2-fluorophenyl)ethyl)amino)-2,6,8-trimethyl-6,8-dihydro-7H-pyrrolo[3,2-g]quinazolin-7-one (Compound 95);

4-(((R)-1-(3-(1,1-difluoro-2-hydroxy-2-methylpropyl)-2-fluorophenyl)ethyl)amino)-6-methoxy-2,6,8-trimethyl-6,8-dihydro-7H-pyrrolo[3,2-g]quinazolin-7-one (Compound 96);

4-(((R)-1-(3-(1,1-difluoro-2-hydroxy-2-methylpropyl)-2-fluorophenyl)ethyl)amino)-6-hydroxy-2,6,8-trimethyl-6,8-dihydro-7H-pyrrolo[3,2-g]quinazolin-7-one (Compound 97);

4-(((R)-1-(3-(1,1-difluoro-2-hydroxy-2-methylpropyl)-2-fluorophenyl)ethyl)amino)-8-(methoxymethyl)-2,6,8-trimethyl-6,8-dihydro-7H-pyrrolo[2,3-g]quinazolin-7-one (Compound 98);

4-(((R)-1-(3-(1,1-difluoro-2-hydroxy-2-methylpropyl)-2-fluorophenyl)ethyl)amino)-2,6,8-trimethyl-6,8-dihydro-7H-pyrrolo[2,3-g]quinazolin-7-one (Compound 99);

4-(((R)-1-(3-(1,1-difluoro-2-hydroxy-2-methylpropyl)-2-fluorophenyl)ethyl)amino)-8-hydroxy-2,6,8-trimethyl-6,8-dihydro-7H-pyrrolo[2,3-g]quinazolin-7-one (Compound 100);

4-(((R)-1-(3-(1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 101);

(S/R)-4-(((R/S)-1-(3-((S/R)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 102);

(S/R)-4-(((R/S)-1-(3-((R/S)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 103);

(R)-8-((1-(3-(1,1-difluoro-2-hydroxy-2-methylpropyl)-2-fluorophenyl)ethyl)amino)-1-ethyl-3,6-dimethyl-1,3-dihydro-2H-imidazo[4,5-g]quinazolin-2-one (Compound 104);

(R)-8-((1-(3-(1,1-difluoro-2-hydroxy-2-methylpropyl)-2-fluoro phenyl)ethyl)amino)-1-isopropyl-3,6-dimethyl-1,3-dihydro-2H-imidazo[4,5-g]quinazolin-2-one (Compound 105);

(R)-8-((1-(3-(1,1-difluoro-2-hydroxy-2-methylpropyl)-2-fluorophenyl)ethyl)amino)-1-(2,2-difluoroethyl)-3,6-dimethyl-1H-imidazo[4,5-g]quinazolin-2(3H)-one (Compound 106);

(R)-1,1-difluoro-1-(2-fluoro-3-(1-((2,2,3,6-tetramethyl-2,3-dihydro-1H-imidazo[4,5-g]quinazolin-8-yl)amino)ethyl)phenyl)-2-methylpropan-2-ol (Compound 107);

(R)-1,1-difluoro-1-(2-fluoro-3-(1-((1,2,2,3,6-pentamethyl-2,3-dihydro-1H-imidazo[4,5-g]quinazolin-8-yl)amino)ethyl)phenyl)-2-methylpropan-2-ol (Compound 108);

(R)-8-((1-(3-(1,1-difluoro-2-hydroxy-2-methylpropyl)-2-fluorophenyl)ethyl)amino)-1,3,6-trimethyl-1,3-dihydro-2H-imidazo[4,5-g]quinazolin-2-one (Compound 109);

(R)-2-cyclopropyl-4-((1-(3-(1,1-difluoro-2-hydroxy-2-methylpropyl)-2-fluorophenyl)ethyl)amino)-6,6,8-trimethyl-6,8-dihydro-7H-pyrrolo[3,2-g]quinazolin-7-one (Compound 110);

(R)-8-((1-(3-(1,1-difluoro-2-hydroxy-2-methylpropyl)-2-fluorophenyl)ethyl)amino)-1,6-dimethyloxazolo[4,5-g]quinazolin-2(1H)-one (Compound 111);

(R)-4-((1-(3-(1,1-difluoro-2-hydroxy-2-methylpropyl)-2-fluorophenyl)ethyl)amino)-2,6-dimethyl-6H-pyrrolo[2,3-g]quinazoline-7,8-dione (compound 112);

(R)-4-((1-(3-(1,1-difluoro-2-hydroxy-2-methylpropyl)-2-fluorophenyl)ethyl)amino)-2,6-dimethyl-6,8-dihydro-7H-pyrrolo[2,3-g]quinazolin-7-one (Compound 113);

(R)-4-((1-(3-(1,1-difluoro-2-hydroxy-2-methylpropyl)-2-fluorophenyl)ethyl)amino)-2,6,6,7-tetramethyl-6,7-dihydro-8H-pyrrolo[3,4-g]quinazolin-8-one (Compound 114);

4-((1-(2-fluoro-3-(1-(hydroxymethyl)cyclopropyl)phenyl)ethyl)amino)-2,6,8,8-tetramethyl-6,8-dihydro-7H-pyrrolo[2,3-g]quinazolin-7-one (Compound 115);

4-(((R)-1-(3-(1,1-difluoro-2-hydroxy-2-methylpropyl)-2-fluorophenyl)ethyl)amino)-8-fluoro-2,6,8-trimethyl-6,8-dihydro-7H-pyrrolo[2,3-g]quinazolin-7-one (Compound 116);

(R)-4-((1-(3-(1,1-difluoro-2-hydroxy-2-methylpropyl)-2-fluorophenyl)ethyl)amino)-8,8-difluoro-2,6-dimethyl-6,8-dihydro-7H-pyrrolo[2,3-g]quinazolin-7-one (Compound 117);

(R)-4-((1-(3-(1,1-difluoro-2-hydroxy-2-methylpropyl)-2-fluorophenyl)ethyl)amino)-2,7,8,8-tetramethyl-7,8-dihy-

dro-6H-pyrrolo[3,4-g]quinazolin-6-one (Compound 118);

(R)-8-((1-(3-(1,1-difluoro-2-hydroxy-2-methylpropyl)-2-fluorophenyl)ethyl)amino)-3,6-dimethyl-1,3-dihydro-2H-imidazo[4,5-g]quinazolin-2-one (Compound 119);

(S)-4-(((S)-1-(3-(1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 120);

4-(((R)-1-(3-(1,1-difluoro-2-hydroxy-2-methylpropyl)-2-fluorophenyl)ethyl)amino)-8-ethoxy-2,6,8-trimethyl-6,8-dihydro-7H-pyrrolo[2,3-g]quinazolin-7-one (Compound 121);

(R)-4'-((1-(3-(1,1-difluoro-2-hydroxy-2-methylpropyl)-2-fluorophenyl)ethyl)amino)-2',6'-dimethyl-2,3,5,6-tetrahydrospiro[pyran-4,8'-pyrrolo[2,3-g]quinazolin]-7'(6'H)-one (Compound 122);

4-(((R)-1-(3-(1,1-difluoro-2-hydroxy-2-methylpropyl)-2-fluorophenyl)ethyl)amino)-8-(2-methoxyethyl)-2,6,8-trimethyl-6,8-dihydro-7H-pyrrolo[2,3-g]quinazolin-7-one (Compound 123);

4'-(((R)-1-(3-(1,1-difluoro-2-hydroxy-2-methylpropyl)-2-fluorophenyl)ethyl)amino)-2',3,6'-trimethylspiro[oxazolidine-5,8'-pyrrolo[2,3-g]quinazoline]-2,7'(6'H)-dione (Compound 124);

4-(((R)-1-(3-(1,1-difluoro-2-hydroxy-2-methylpropyl)-2-fluorophenyl)ethyl)amino)-8-methoxy-2,6-dimethyl-8-(trifluoromethyl)-6,8-dihydro-7H-pyrrolo[2,3-g]quinazolin-7-one (Compound 125);

4-(((R)-1-(3-(1,1-difluoro-2-hydroxy-2-methylpropyl)-2-fluorophenyl)ethyl)amino)-8-ethyl-8-methoxy-2,6-dimethyl-6,8-dihydro-7H-pyrrolo[2,3-g]quinazolin-7-one (compound 126);

4-(((1R)-1-(3-(1,1-difluoro-2-hydroxy-3-methoxy-2-methylpropyl)-2-fluorophenyl)ethyl)amino)-2,6,8,8-tetramethyl-6,8-dihydro-7H-pyrrolo[2,3-g]quinazolin-7-one (Compound 127);

4'-(((R)-1-(3-(1,1-difluoro-2-hydroxy-2-methylpropyl)-2-fluorophenyl)ethyl)amino)-2',6'-dimethyl-4,5-dihydro-2H-spiro[furan-3,8'-pyrrolo[2,3-g]quinazolin]-7'(6'H)-one (Compound 128);

4-(((1R)-1-(3-(3-(dimethylamino)-1,1-difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one (Compound 129);

4-(((1R)-1-(3-(1,1-difluoro-2-hydroxy-2-methyl-3-(methylamino)propyl)-2-fluorophenyl)ethyl)amino)-2,6,8,8-tetramethyl-6,8-dihydro-7H-pyrrolo[2,3-g]quinazolin-7-one (Compound 130);

4-(((R)-1-(3-amino-5-(trifluoromethyl)phenyl)ethyl)amino)-8-methoxy-2,6,8-trimethyl-6,8-dihydro-7H-pyrrolo[2,3-g]quinazolin-7-one (Compound 131);

4-(((R)-1-(3-(1,1-difluoro-2-hydroxy-2-methylpropyl)-2-fluorophenyl)ethyl)amino)-8-methoxy-8-(2-methoxyethyl)-2,6-dimethyl-6,8-dihydro-7H-pyrrolo[2,3-g]quinazolin-7-one (Compound 132);

4-(((1R)-1-(2-fluoro-3-(piperidin-3-yl)phenyl)ethyl)amino)-2,6,8,8-tetramethyl-6,8-dihydro-7H-pyrrolo[2,3-g]quinazolin-7-one (Compound 133);

(R)-4-((1-(2-fluoro-3-(trifluoromethyl)phenyl)ethyl)amino)-2,6,8,8-tetramethyl-6,8-dihydro-7H-pyrrolo[2,3-g]quinazolin-7-one (Compound 134);

4-(((1R)-1-(3-(3-(dimethylamino)-1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 135);

2,6,8,8-tetramethyl-4-((1-(3-(trifluoromethyl)phenyl)ethyl)amino)-6,8-dihydro-7H-pyrrolo[2,3-g]quinazolin-7-one (Compound 136);

4-(((R)-1-(3-amino-5-(trifluoromethyl)phenyl)ethyl)amino)-6-methoxy-2,6,8-trimethyl-6,8-dihydro-7H-pyrrolo[3,2-g]quinazolin-7-one (Compound 137);

(R)-4'-((1-(3-amino-5-(trifluoromethyl)phenyl)ethyl)amino)-2',6'-dimethyl-2,3,5,6-tetrahydrospiro[pyran-4,8'-pyrrolo [2,3-g]quinazolin]-7'(6'H)-one (Compound 138); and

4-(((R)-1-(3-amino-5-(trifluoromethyl)phenyl)ethyl)amino)-8-ethyl-8-methoxy-2,6-dimethyl-6,8-dihydro-7H-pyrrolo [2,3-g]quinazolin-7-one (Compound 139).

According to a feature of the present invention, the compounds of general formula (I) where all the symbols are as defined earlier, can be prepared by methods illustrated in the schemes and examples provided herein below. However, the disclosure should not be construed to limit the scope of the invention arriving at compound of formula (I) as disclosed hereinabove. Further, in the following schemes, where specific bases, acids, reagents, solvents, coupling agents, etc., are mentioned, it is understood that other bases, acids, reagents, solvents, coupling agents etc., known in the art may also be used and are therefore included within the scope of the present invention. Variations in reaction conditions, for example, temperature and/or duration of the reaction, which may be used as known in the art, are also within the scope of the present invention. All the isomers of the compound of formula in described in these schemes, unless otherwise specified, are also encompassed within the scope of this invention.

The corresponding $\alpha$-methyl amine derivatives represented as formula (A5) could be prepared by following the sequential transformations as depicted in Scheme - A herein below-

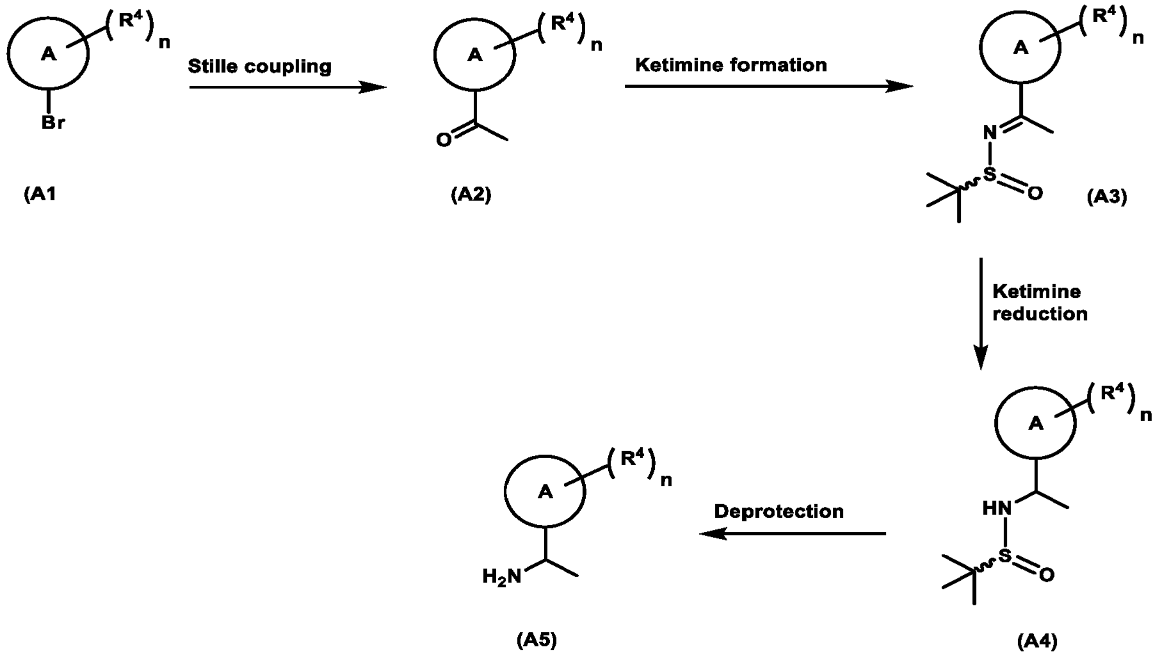


(A1 (A2) (A3) (A4) (A5)

**SCHEME - A**

The compound of formula (A1) undergoes a metal catalyzed cross coupling with alkoxy vinyl stannane, e.g. tributyl(1-ethoxyvinyl)tin in presence of palladium catalysts such as $Pd(Ph_3P)_2Cl_2$, $Pd_2(dba)_3$ and like; optionally using bases such as triethylamine, N,N-Diisopropylethylamine and like, in hydrocarbon solvents like toluene or ether solvents like 1,4-dioxane to furnish the alkoxy vinyl intermediate which in turn provide compound of formula (A2) in acidic condition by employing aqueous mineral acids such as hydrochloric acid in ether solvent such as THF, 1,4-dioxane and like. The similar transformation can be carried out by reaction of compound of formula (A1) with n-alkylvinyl ether using catalysts such as palladium (II) acetate and like, ligands such as 1,3-Bis(diphenylphosphino)propane and like, in presence of organic bases such as DIPEA, TEA and like in alcoholic solvents such as ethylene glycol and at elevated temperatures ,in solvents such as 1,4-dioxane, THF and mixtures thereof to give alkoxy vinyl intermediate which in turn provide compound of formula (A2) in acidic condition by employing aqueous mineral acids such as hydrochloric acid in ether solvent such as THF, 1,4-dioxane and like

The compound of formula (A2) was then reacted with corresponding chirally pure t-butanesulfinamide in presence of Lewis acid such as Titanium alkoxides e.g. titanium tetraethoxide, titanium isopropoxide and the like, in ether solvents such as 1,4-dioxane, THF and like, to obtain the compound of formula (A3).

The compound of formula (A3) reacted with reducing agent such as metal hydrides e.g. sodium borohydride, L-selectride and like, in solvents such as THF, 1,4- dioxane, methanol and the like, optionally in presence of water to provide sulfinamide of formula (A4). Major diastereoisomer in the compound of formula (A4) after reduction was separated or taken

ahead as such.

The compound of formula (A4) under acidic condition undergoes cleavage of reduced ketimine derivative to generate amine of formula (A5) as a free base or salt. The acids employed for the transformation may involve mineral acids such as hydrochloric acid, organic acids like trifluoroacetic acid and thereof.

The compounds of formula (I) was prepared by following the sequential transformations as depicted and described in Scheme-B herein below-

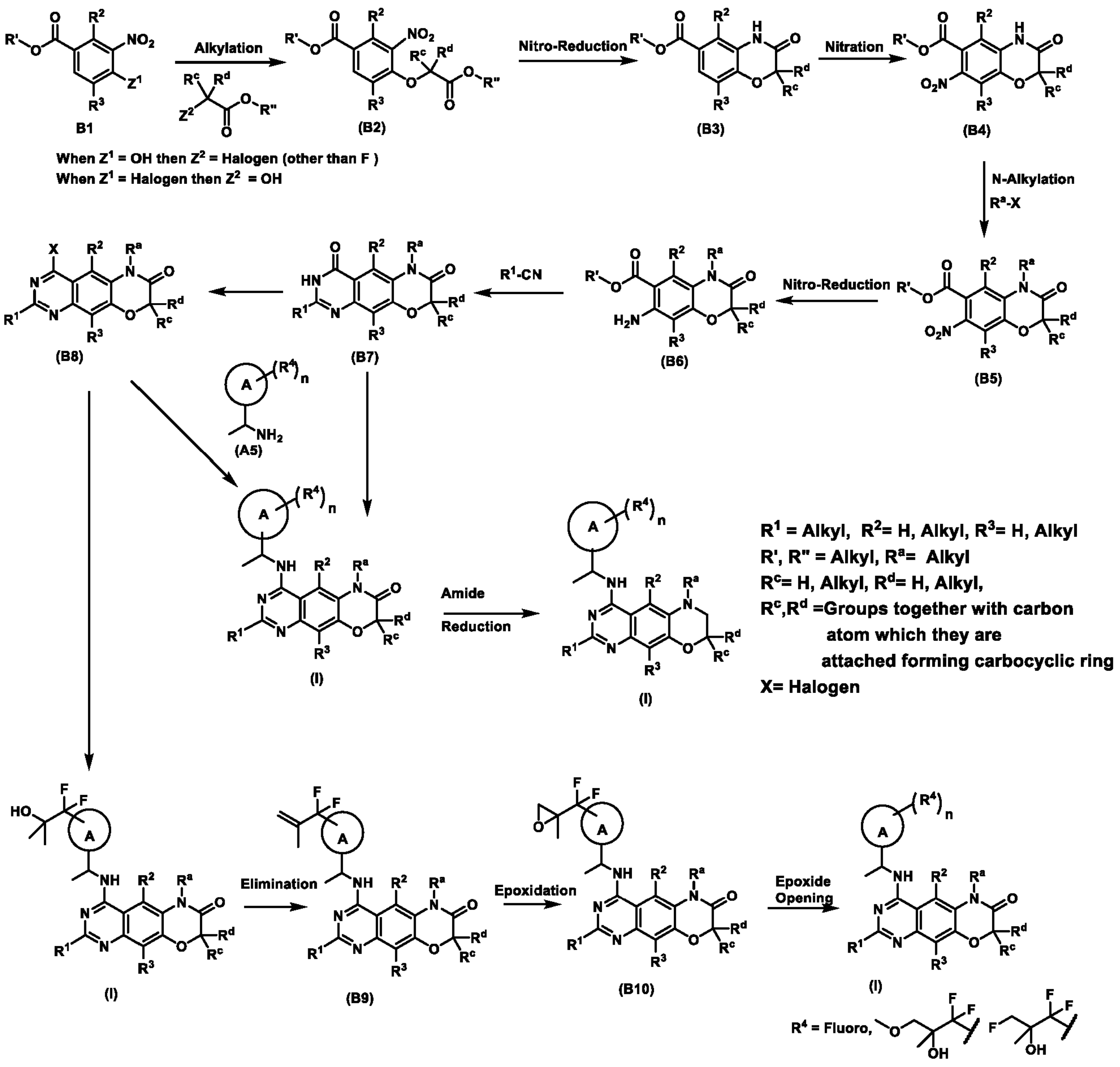


SCHEME - B

Compound of formula (B2) can be synthesized from compound of formula (B1) by following the reaction protocol as mentioned in EP2243779 ($R^a$= $R^b$ = $CH_3$) and WO2015164480 ($R^a$ and $R^b$ together forms a ring). Compound of formula (B2) was converted to corresponding cyclic amide of formula (B3) through selective reduction of nitro group by using different reducing agents. Although not limited, such reducing agents include hydrogenation with palladium on carbon, metal reductions like iron, tin or tin chloride and the like. Such reduction of the compound of formula (B2) can be carried out in one or more solvents, e.g., ethers such as THF, 1,4-dioxane, and the like; alcohol such as methanol, ethanol and the like; under acidic conditions involving ammonium chloride, acetic acid, hydrochloric acid and mixtures thereof. Nitration of

compound of formula (B3) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (B4). Compound of formula (B4) can be further alkylated by using corresponding alkyl halide in presence of bases such as $Na_2CO_3$, $K_2CO_3$, $Cs_2CO_3$ etc. in polar aprotic solvents like DMF, DMSO etc. at temperature 20°C - 60°C leading to compound of formula (B5). An alternative synthetic route towards the compound of formula (B5) is the transformation of intermediate of compound of formula (B4) via Mitsunobu reaction with corresponding alcohol, using different reagents such as but not limited to DEAD, DIAD etc. Such reactions can be carried out in aprotic solvents like, e.g., ethers such as THF, Dioxane and the like; hydrocarbons, e.g., toluene or mixtures thereof, at temperature 25°C - 90°C. Compound of formula (B5) was converted to corresponding aniline derivative compound of formula (B6) through selective reduction of nitro group by using different reducing agents. Although not limited, such reducing agents include hydrogenation with palladium on carbon, metal reductions like iron, tin or tin chloride and the like. Such reduction of the compound of formula (B5) can be carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof. Compound of formula (B6) upon treatment with corresponding alkylnitriles using acids such as but not limited to Methane sulfonic acid, HCl etc. at 25°C-120°C to afford compound of formula (B7), which could be further coupled with different chiral benzyl amine (A5) derivatives using different coupling reagents such as but not limited to BOP, PyBop etc. and organic bases such as DBU, DIPEA etc. in a polar aprotic solvent like DMF, DMSO etc. at 0°-120°C to afford a compound of formula (I).

Alternatively, compound of formula (I) can be prepared from compound of formula (B7) by reacting with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (B8).

Compound of formula (B8) undergoes a nucleophilic substitution reaction with different chiral benzylic amines (A5) leading to the final compound of formula (I) using organic basic reagents such as but not limited to DIPEA, TEA etc. optionally neat or in a polar aprotic solvents like dioxane, THF etc. at 0°C -130°C. Carbonyl functional group in Compound of formula (I) on further reduction using different reducing reagents such as but not limited to borane DMS, borane THF, $LiAlH_4$ in polar aprotic solvents like THF, dioxane etc. at temperature 70 - 90°C leading to final compound of formula (I).

Compound of formula (I) allowed to react with fluorinating reagent such as DAST, martin sulfurane in solvents such as DCM, chloroform, THF, ether, 1,4-dioxane to provide compound of formula (B9).

Compound of formula (B9) undergoes epoxidation reaction to provide compound of formula (B10). This reaction is effected by hydrogen peroxide in presence of acidic medium using organic acids such as formic acid and like.

Compound of formula (B10) on epoxide opening by nucleophilic reagent provide compound of formula (I). Such transformations can be effected by reaction of epoxide compound with various nucleophilic reagents such as sodium alkoxides, primary or secondary amines in alcohol solvents like ethanol, methanol, and like and at room temperature or elevated temperature.

The compounds of formula (I) was prepared by following the sequential transformations as depicted and described in **Scheme-C** herein below-

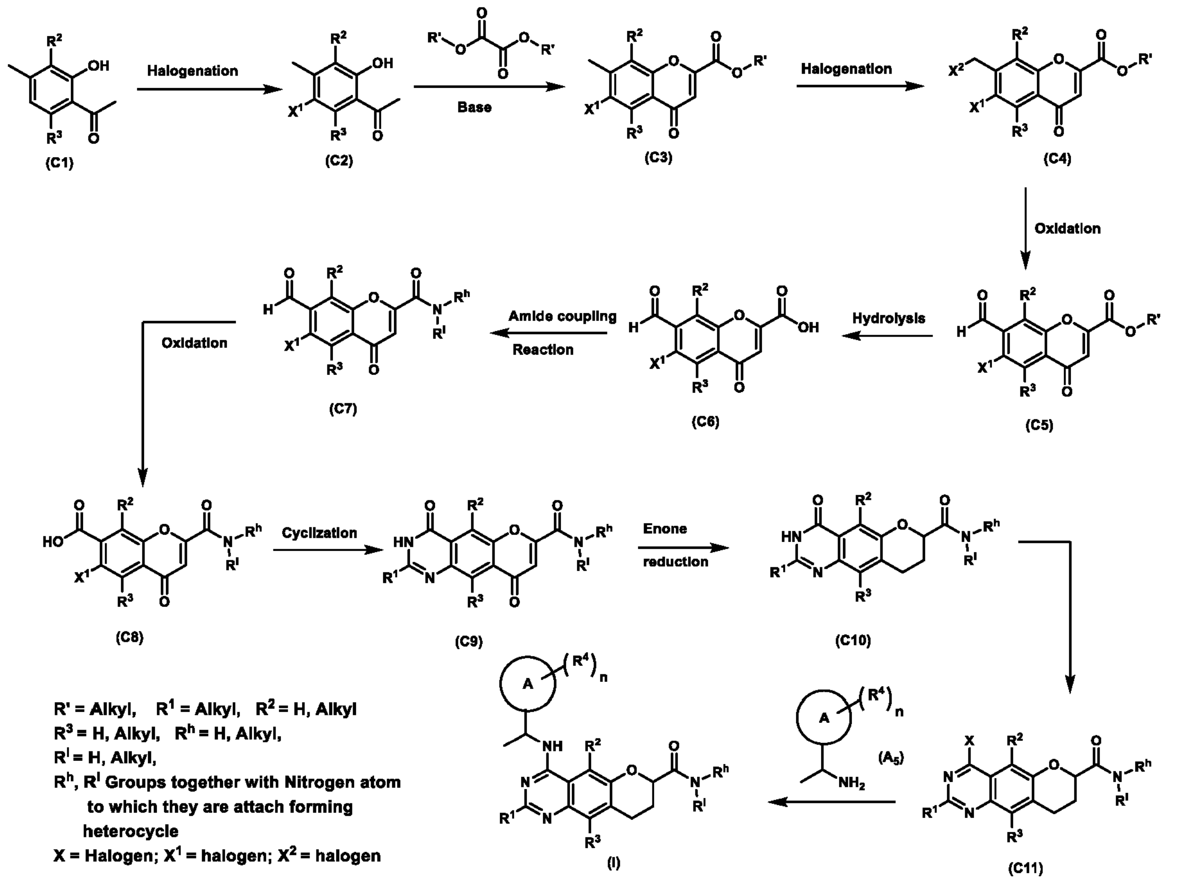


**SCHEME - C**

Compound of formula (C2) is prepared by following a procedure reported in Chemistry - A European Journal, 2015, vol. 21, # 4, p. 1482 - 1487. The compound of formula (C2) is converted to corresponding 4-oxo chromene carboxylic ester derivative of compound of formula (C3) using corresponding alpha diketo ester and basic reagents such as but not limited to NaOMe, NaOEt, $K^tOBu$ etc. in a polar aprotic solvents like DMF, DMA etc. at 0°C - 75°C. Halogenation of compound of formula (C3) using N-halosuccinamide reagent such as but not limited to NBS , NIS and NCS gives corresponding dihalo compound of formula (C4) via e.g. benzylic halogenation in a aprotic halogenated solvents like $CCl_4$, DCM etc. at 0°-80°C. The compound of formula (C5) aldehyde derivative can be synthesized by oxidation of compound of formula (C4). Compound of formula (C5) undergoes an acidic hydrolysis leading to compound of formula (C6), that can be further functionalized to corresponding amide of compound of formula (C7) using coupling reagent such as but not limited to PyBop in a polar aprotic solvents like DMF, DMSO etc. at temperature ranging from 0°C -30°C for about 1-16h. Compound of formula (C8) can be achieved by oxidation of compound of formula (C7) with suitable oxidizing reagent such as but not limited to sulphamic acid and sodium chlorite. Compound of formula (C8) when condensed with corresponding amidine by coupling reaction affords a quinazoline enone derivative of compound of formula (C9). Reduction of enone compound of formula (C9) using reagents such as but not limited to $H_2$-Pd/C leading to corresponding compound of formula (C10). The compound of formula (C10) can be transformed to the corresponding compound of formula (C11) via halogenation using reagents such as phosphorus oxyhalide, thionyl chloride and like, in aprotic solvents like chlorobenzene, toluene and mixtures thereof. Compound of formula (C11) undergoes a coupling with different chiral benzylic amines (A5 ) leading to the final compound of formula (I). This reaction can be effected by organic base such as DIPEA, TEA, DBU or the like, or using coupling reagents such as DCC, EDC, BOP, pyBOP, HBTU or the like; optionally neat or in etheral solvents such as THF, 1,4 dioxane and like or polar aprotic solvents like DMF, DMA, DMSO and thereof at temperature ranging from 20-130°C.

The compounds of formula (I) was prepared by following the sequential transformations as depicted and described in **Scheme** - **D** herein below.

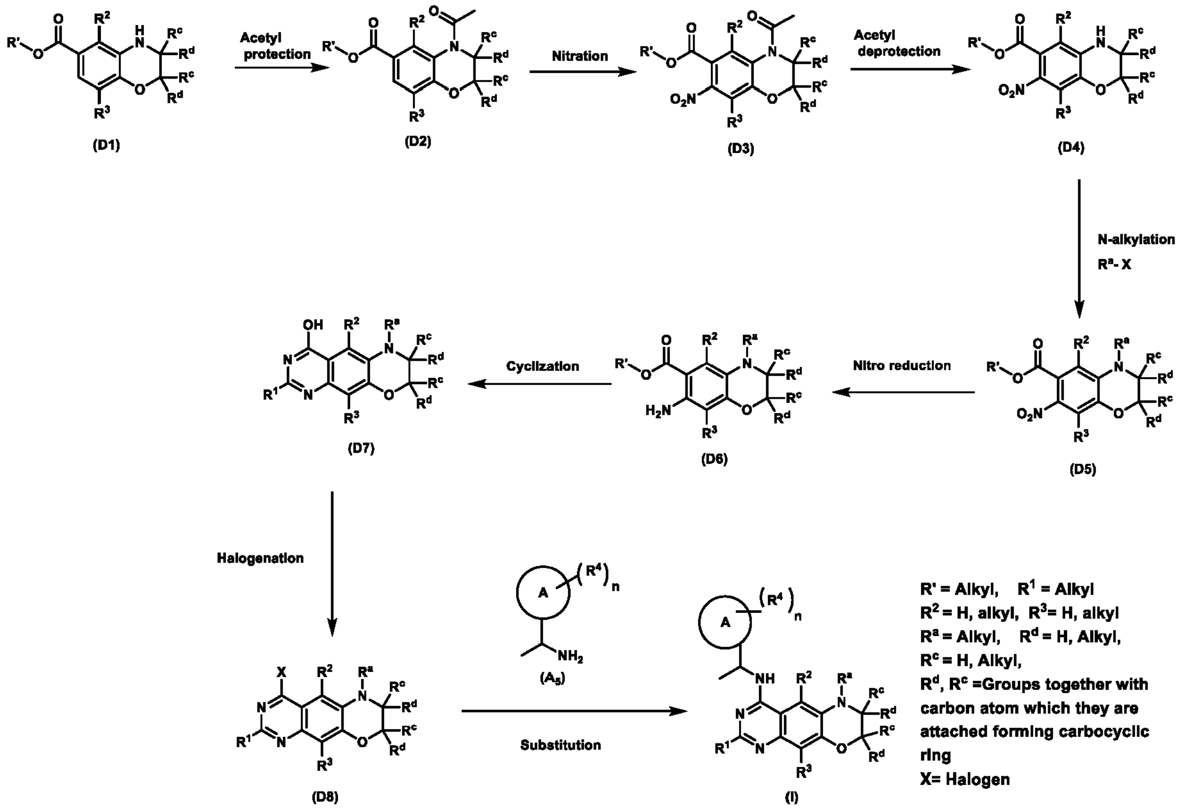


(D1) (D2) (D3) (D4) (D5) (D6) (D7) (D8) ($A_5$) (I)

SCHEME - D

The compound of formula (D1) is converted to corresponding acetyl derivative of compound of formula (D2) via N-acylation reaction using acetyl chloride & using organic basic reagents such as but not limited to pyridine, DIPEA, TEA etc in halogenated solvents such as, although not limited chloroform, dichloromethane, and the like mixtures thereof. Nitration of compound of formula (D2) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (D3).

Acetyl deprotection of compound of formula (D3) using inorganic bases such as $Na_2CO_3$, $K_2CO_3$, $Cs_2CO_3$, etc in polar protic solvents like methanol, ethanol etc at appropriate temperature afforded compound of formula (D4).

Compound of formula (D4) can be further alkylated by using alkyl halides and bases such as NaH, $Na_2CO_3$, $K_2CO_3$, $Cs_2CO_3$ etc. in polar aprotic solvents like THF, DMF, and DMSO etc. at temperature 20°C - 60°C leading to compound of formula (D5).

Compound of formula (D5) can be converted to corresponding aniline derivative, compound of formula (D6) through selective reduction of nitro group by using different reducing agents. Although not limited, such reducing agents include hydrogenation with palladium on carbon, metal reductions like iron, tin or tin chloride and the like. Such reduction of the compound of formula (D6) can be carried out in one or more solvents, such as methanol, ethanol and the like; under acidic conditions involving ammonium chloride, acetic acid, hydrochloric acid and the like mixtures thereof.

Compound of formula (D6) allowed to react with alkylnitrile in presence of the acidic reagents such as methane sulfonic acid, sulfuric acid, hydrochloric acid or the like to obtain compound of formula (D7).

Compound of formula (D7) was reacted with $POCl_3$ or $POBr_3$ optionally in solvents such as toluene, xylene or the like or the mixtures thereof, optionally using organic base such as triethylamine, diisopropylethylamine or the like to provide compound of formula (D8).

Compound of formula (D8) was reacted with compound of formula (A5) in the presence DIPEA, TEA, DBU or the like, or using coupling reagents such as DCC, EDC, BOP, pyBOP, HBTU or the like; optionally neat or in etheral solvents such as THF, 1,4 dioxane and like or polar aprotic solvents like DMF, DMA, DMSO and thereof at temperature ranging from 20-130°C. to provide compound of formula (I).

The compounds of formula (I) was prepared by following the sequential transformations as depicted and described in Scheme - E herein below.

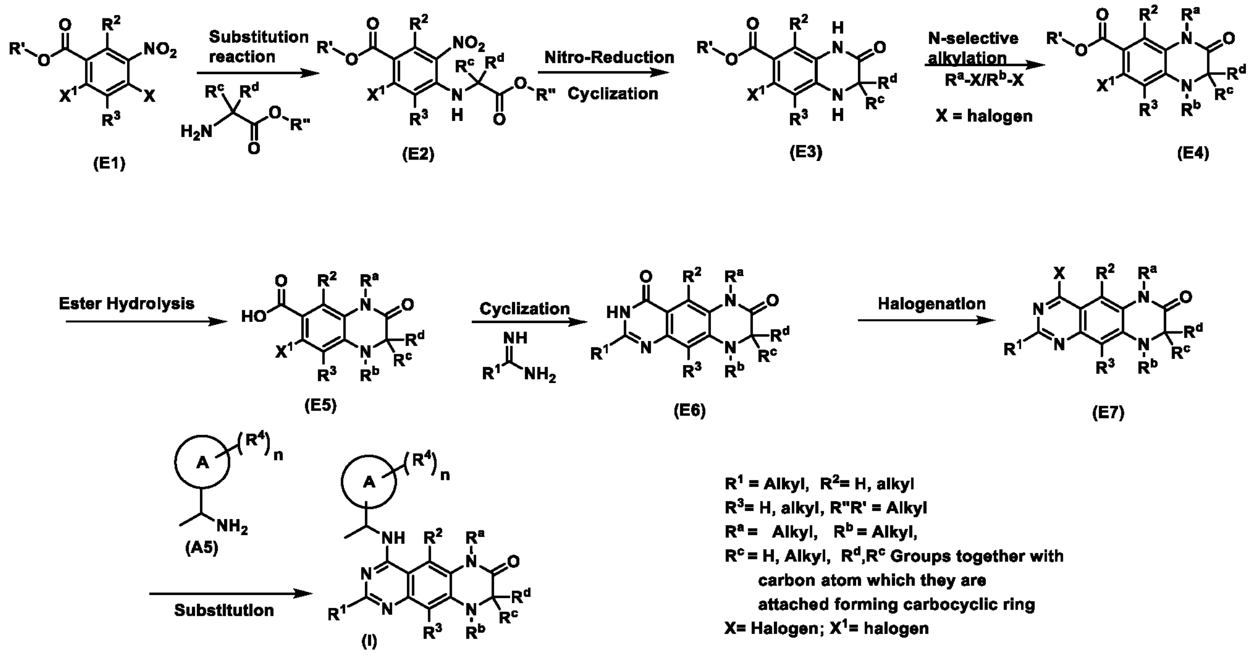


(E1) (E2) (E3) (E4) (E5) (E6) (E7) (A5) (I)

**SCHEME - E**

Compound of formula (E1) can be synthesized following a reaction protocol described in WO200879759.Compound of formula (E2) can be synthesized by appropriate displacement of aromatic halogen with corresponding alkyl amine using appropriate bases such as TEA, NaH, $Na_2CO_3$, $K_2CO_3$, $Cs_2CO_3$ etc. in polar aprotic solvents like THF, DMF, DMSO etc. at temperature 20°C - 120°C.

Compound of formula (E2) can be converted to corresponding cyclic amide of formula (E3) through selective reduction of nitro group by using different reducing agents. Although not limited, such reducing agents include hydrogenation with palladium on carbon, metal reductions like iron, tin or tin chloride and the like. Such reduction of the compound of formula (E2) can be carried out in one or more solvents, alcohol such as methanol, ethanol and the like; under acidic conditions involving ammonium chloride, acetic acid, hydrochloric acid and the like mixtures thereof. Compound of formula (E3) can be further alkylated by using bases such as NaH, $Na_2CO_3$, $K_2CO_3$, $Cs_2CO_3$ etc. in polar aprotic solvents like THF, DMF, and DMSO etc. at temperature 20°C - 60°C leading to compound of formula (E4). Compound of formula (E5) can be synthesized by ester hydrolysis of compound of formula (E4) using bases such as NaOH, LiOH and KOH etc. Compound of formula (E5) which on coupling with different amidines such as acetamidine, formamidine etc. in polar aprotic solvents like DMF, DMSO etc. at temperature 80°C - 100°C leading to compound of formula (E6).Compound of formula (E6) can be converted to the corresponding compound of formula (E7) by halogenation using reagents such as $POCl_3$, $POBr_3$, $SOCl_2$ etc.

Compound of formula (E7) undergoes a nucleophilic substitution reaction with different chiral benzyl amine (A5) leading to compound of formula (I) using aprotic solvents like dioxane, THF and like, at temperature 0°C -130°C and bases such as but limited to DIPEA, TEA and thereof.

The compounds of formula (I) was prepared by following the sequential transformations as depicted and described in Scheme -F herein below.

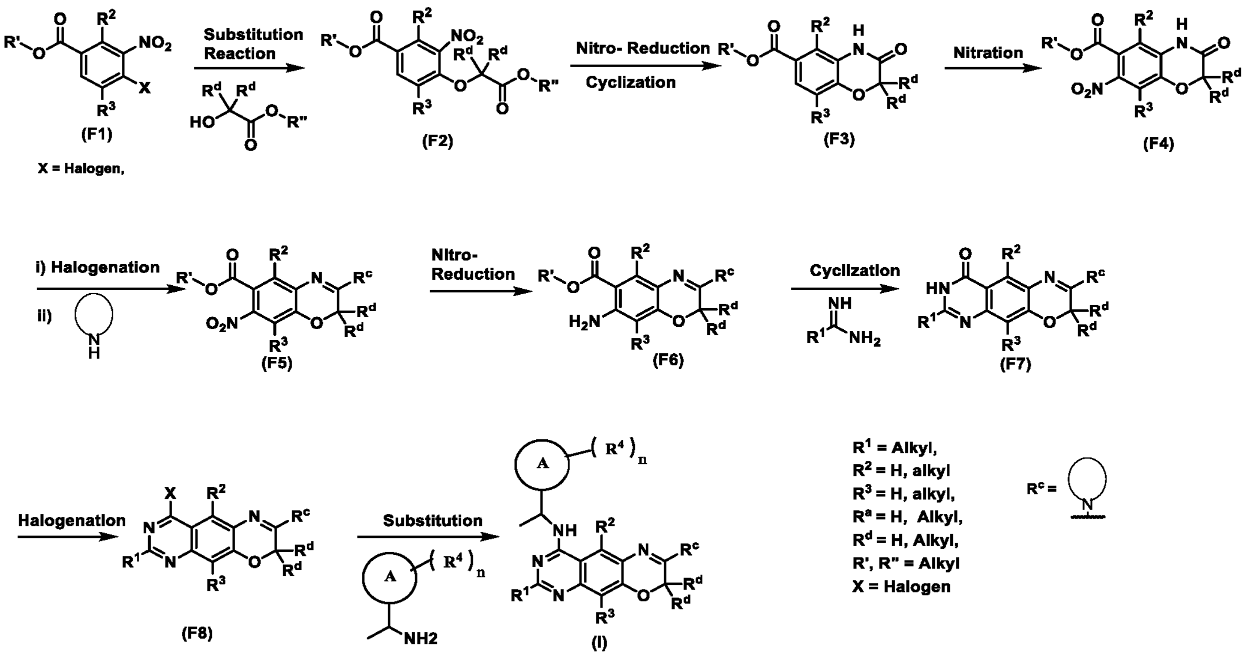


(F1) (F2) (F3) (F4) (F5) (F6) (F7) (F8) (I)

**SCHEME - F**

Compound of formula (F2) can be synthesized by following the reaction protocol as mentioned in EP2243779 ($R^c$ = $R^d$ = $CH_3$) and WO2015164480 ($R^c$ and $R^d$ together forms a ring). Compound of formula (F2) was converted to corresponding cyclic amide of formula (F3) through selective reduction of nitro group by using different reducing agents. Although not limited, such reducing agents include hydrogenation with palladium on carbon, metal reductions like iron, tin or tin chloride and the like. Such reduction of the compound of formula (F2) can be carried out in one or more solvents, such as methanol, ethanol and the like; under acidic conditions involving ammonium chloride, acetic acid, hydrochloric acid and the like mixtures thereof. Nitration of compound of formula (F3) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (F4).

Compound of formula (F4) can be treated with $SOCl_2$ , $POCl_3$, $POBr_3$ and thereof using DMF to give an intermediate (Halogenation reaction intermediate), which undergoes a nucleophilic substitution reaction with appropriate amines leading to the compound of formula (F5), using organic basic reagents such as but not limited to DIPEA, TEA etc. in a polar aprotic solvent like dioxane, THF etc. at appropriate temperature. Compound of formula (F5) can be converted to corresponding aniline derivative, compound of formula (F6) through selective reduction of nitro group by using different reducing agents. Although not limited, such reducing agents include hydrogenation with palladium on carbon, metal reductions like iron, tin or tin chloride and the like. Such reduction of the compound of formula (F5) can be carried out in one or more solvents, such as methanol, ethanol and the like; under acidic conditions involving ammonium chloride, acetic acid, hydrochloric acid and mixtures thereof. Compound of formula (F6) upon treatment with corresponding nitrile solvents such as but not limited to acetonitrile using acids such as but not limited to methane sulfonic acid, HCl etc. at 25°C-120°C to afford compound of formula (F7), which can be transformed to intermediate (F8), via e.g. triflate or halogenation etc. of the corresponding compound of formula (F7). Compound of formula (F8) undergoes a nucleophilic substitution reaction with different chiral benzyl amine (A5), using aprotic solvents like dioxane, THF etc., at temperature 0°C-130°C and bases such as but limited to DIPEA, TEA etc. leading to final compound of formula (I).

The compounds of formula (I) was prepared by following the sequential transformations as depicted and described in **Scheme** - **G** herein below.

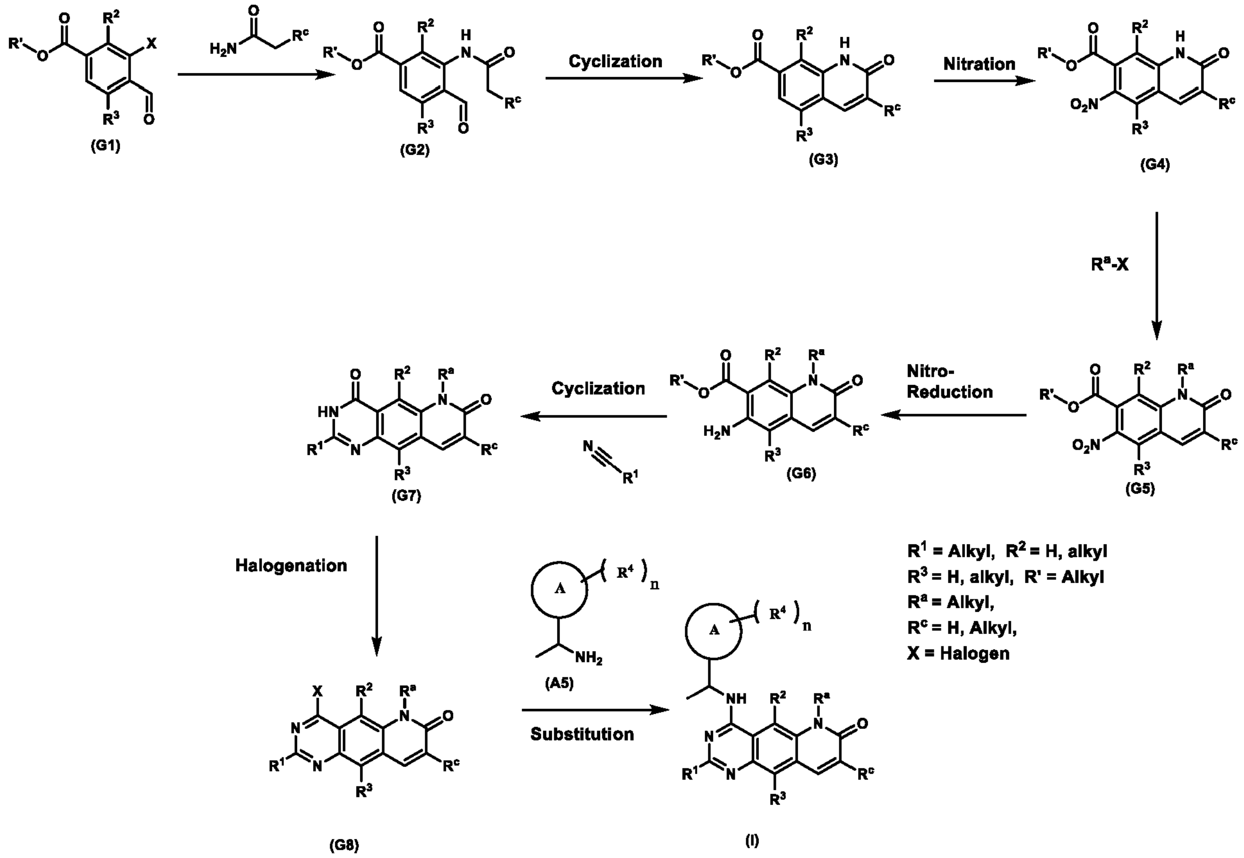


SCHEME - G

Compound of formula (G1) was allowed to react with corresponding carbamate in the presence of catalyst such as (tris(dibenzylideneacetone)dipalladium(0), palladium(II) acetate, Bis(dibenzylideneacetone)2 Pd(0), rac 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as sodium carbonate, cesium carbonate, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (G2)

Cyclization of compound of formula(G2) provided compound of formula (G3), in the presence of suitable base, preferably inorganic bases such as alkali metal carbonates, e.g., $Na_2CO_3$, $K_2CO_3$, $Cs_2CO_3$, $NaO^tBu$, Potassium phosphate, or mixture thereof. Such reactions can be carried out in solvents like, e.g., ethers such as THF, Dioxane and the like; hydrocarbons, e.g., toluene; amides such as DMF, DMA or mixtures thereof.

Nitration of compound of formula (G3) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (G4).

The compound of formula (G4) was alkylated to give compound of formula (G5). This conversion was effected in presence alkali hydrides like sodium hydride and like; or bases such as potassium carbonate and like; and alkylating reagents alkyl halides e.g. Methyl iodide and like; in presence of solvents such as THF, DMF or mixture(s) thereof.

Compound of the formula (G6) was obtained from compound of formula (G5) using by metal reductions using iron, tin or tin chloride or the like in solvents selected from THF, 1,4-dioxane methanol, ethanol or the like or mixtures thereof under acidic condition using ammonium chloride, acetic acid, hydrochloric acid or the like or mixture(s) thereof. This transformation can also be carried out by catalytic hydrogenation using Pd/C and thereof in solvents ethyl acetate, Methanol or mixture(s) thereof.

Compound of formula (G6) reacted with alkylnitriles in presence of the reagent such as methane sulfonic acid, sulfuric acid, hydrochloric acid or the like to obtain compound of formula (G7).

Compound of formula (G7) was reacted with $POCl_3$ or $POBr_3$ optionally in solvents such as toluene, xylene or the like or the mixtures thereof, optionally using organic base such as triethylamine, diisopropylethylamine or the like to provide compound of formula (G8).

Compound of formula (G8) was reacted with compound of formula (A5) in the presence of triethyl amine, N,N-ethyldiisopropyl amine, pyridine, DBU or the like in solvents such as THF, 1,4-Dioxane, toluene, DCM, DMSO or mixture(s) thereof to provide compound of formula (I).

The compounds of formula (I) was prepared by following the sequential transformations as depicted and described in Scheme - H herein below.

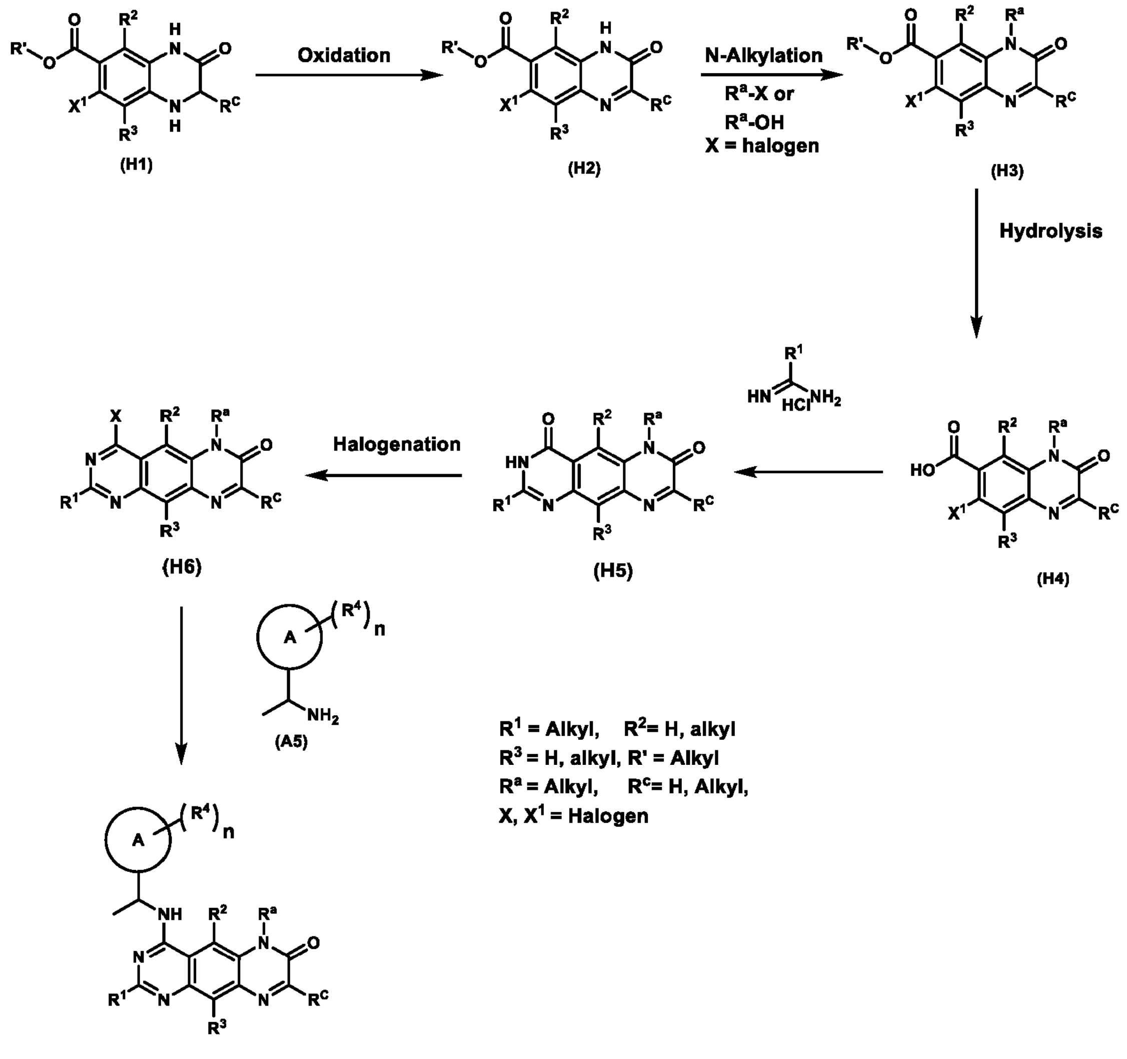


(I)

**SCHEME - H**

Compound of formula (H1) can be synthesized by reaction protocol as mentioned in (WO243823). Compound of formula (H2) can be synthesized from compound of formula (H1) by using oxidizing agents like $MnO_2$, $H_2O_2$, $AgNO_3$, DDQ and thereof.

Compound of formula (H2) undergoes alkylation reaction using alkyl halides in presence of bases such as $K_2CO_3$, $Na_2CO_3$, $Cs_2CO_3$ and like; in polar aprotic solvents like DMF, DMSO and thereof; at temperature 20°C - 60°C afforded compound of formula (H3).

An alternative synthetic route towards the compound of formula (H3) is the transformation of intermediate of compound of formula (H2) via Mitsunobu reaction with corresponding alcohol, using different reagents such as but not limited to DEAD, DIAD etc. Such reactions can be carried out in aprotic solvents like, e.g., ethers such as THF, Dioxane and the like; hydrocarbons, e.g., toluene or mixtures thereof, at temperature 25°C - 90°C.

Compound of formula (H4) can be synthesized by ester hydrolysis of formula (H3) using bases such as NaOH, LiOH, KOH and like; in polar protic solvents such as methanol, ethanol and like.

Compound of formula (H4) on reaction with acetamidine, formamidine and like; in polar aprotic solvents like DMF, DMSO and thereof at temperature elevated temperatures afforded compound of formula (H5).

Compound of formula (H7) was reacted with $POCl_3$ or $POBr_3$ optionally in solvents such as toluene, xylene or the like or the mixtures thereof, optionally using organic base such as triethylamine, diisopropylethylamine or the like to provide compound of formula (H6).

Compound of formula (H6) was reacted with compound of formula (A5) in the presence of triethyl amine, N,N-ethyldiisopropyl amine, pyridine, DBU or the like in solvents such as THF, 1,4-Dioxane, toluene, DCM, DMSO or mixture(s) thereof to provide compound of formula (I).

The compounds of formula (I) was prepared by following the sequential transformations as depicted and described in **Scheme** - **I** herein below.

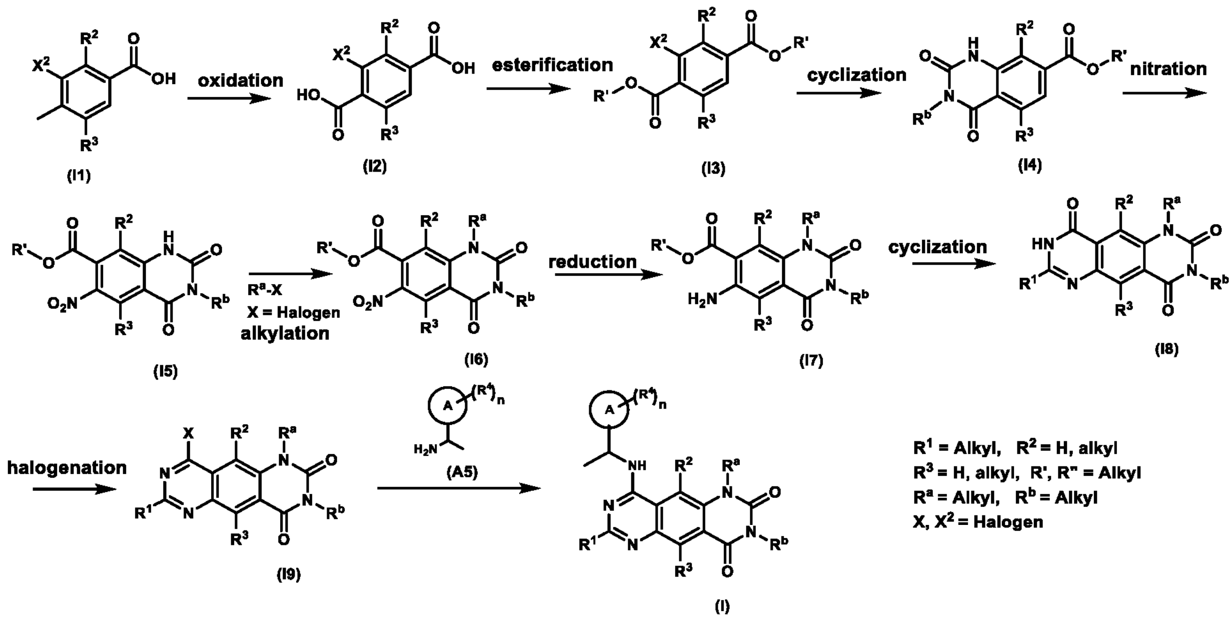


**SCHEME - I**

The compound of the formula (I2) obtained by treating compound of the formula (I1) with oxidizing agent potassium permanganate, potassium dichromate, sodium dichromate in presence of acids like sulphuric acid, acetic acid and like, in 1:1 mixture of t-butanol and Water as Solvent.

The compound of formula (I2) was subjected to esterification in alcoholic solvents like methanol ethanol and thereof in presence of chlorinating agents such as thionyl chloride, oxalyl chloride and thereof, or in presence of acidic reagents such as sulfuric and methane sulfonic acid thereof to provide the compound of formula (I3). The compound of formula (I3) was subjected to C-N coupling reaction e.g. Buchwald reaction with 1-methylurea provided compound of formula (I4). This reaction can mediated by a suitable catalyst such as, e.g., $Pd(PPh_3)_2Cl_2$, $Pd_2dba_3$, $Pd(PPh_3)_4$, $Pd(OAc)_2$ or mixtures thereof; a suitable ligand such as Xantphos, BINAP, Ru-Phos, XPhos, or mixtures thereof; in the presence of suitable base, preferably inorganic bases such as alkali metal carbonates, e.g., $K_2CO_3$, $Na_2CO_3$, $Cs_2CO_3$, $NaO^tBu$, Potassium phosphate, or mixture thereof. Such reactions can be carried out in solvents like, e.g., ethers such as THF, Dioxane and the like; hydrocarbons, e.g., toluene; amides such as DMF, DMA or mixtures thereof.

Nitration of compound of formula (I4) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (I5).

The compound of formula (I5) was alkylated to give compound of formula (I6). This conversion was effected in presence alkali hydrides like sodium hydride and like; or bases such as potassium carbonate and like; and alkylating reagents alkyl halides e.g. Methyl iodide and like; in presence of solvents such as THF, DMF or mixture(s) thereof.

Compound of the formula (I7) was obtained from compound of formula (I6) using by metal reductions using iron, tin or tin chloride or the like in solvents selected from THF, 1,4-dioxane methanol, ethanol or the like or mixtures thereof under acidic condition using ammonium chloride, acetic acid, hydrochloric acid or the like or mixture(s) thereof. This transformation can also be carried out by catalytic hydrogenation using Pd/C and thereof in solvents ethyl acetate, Methanol or mixture(s) thereof.

Compound of formula (I7) reacted with acetonitrile in presence of the reagent such as methane sulfonic acid, sulfuric acid, hydrochloric acid or the like to obtain compound of formula (I8).

Compound of formula (I8) was reacted with $POCl_3$ or $POBr_3$ optionally in solvents such as toluene, xylene or the like or the mixtures thereof, optionally using organic base such as triethylamine, diisopropylethylamine or the like to provide compound of formula (I9).

Compound of formula (I9) was reacted with compound of formula (A5) in the presence of triethyl amine, N,N-ethyldiisopropyl amine, pyridine, DBU or the like in solvents such as THF, 1,4-Dioxane, toluene, DCM, DMSO or mixture(s) thereof to provide compound of formula (I).

The compounds of formula (I) was prepared by following the sequential transformations as depicted and described in **Scheme** - **J** herein below.

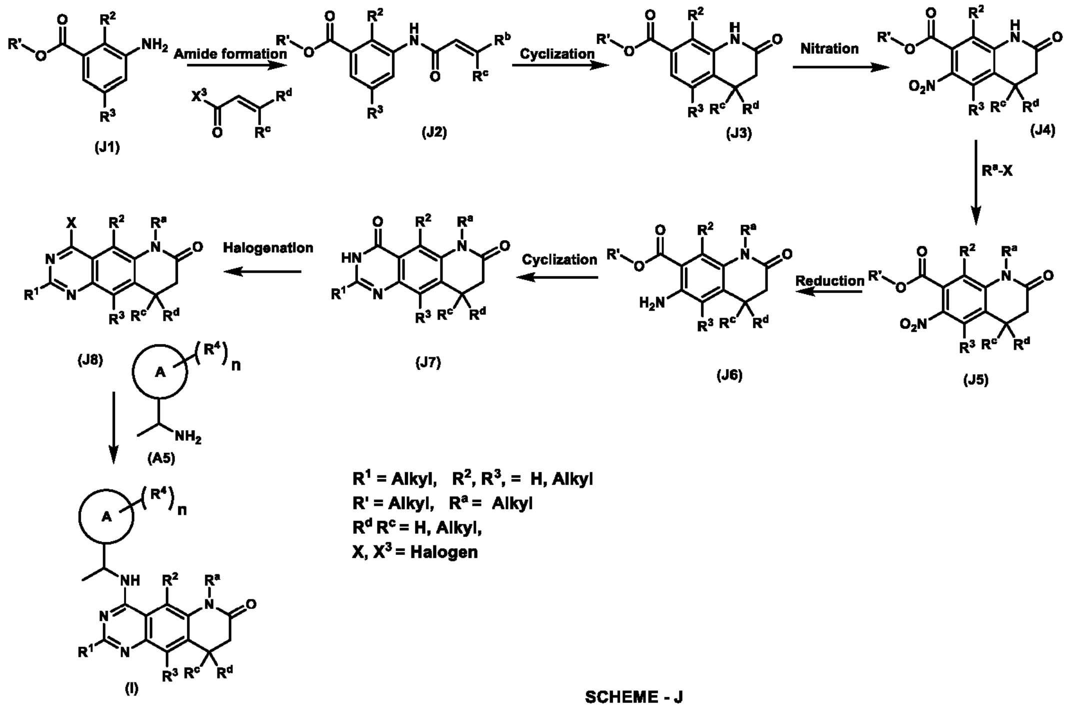


**SCHEME - J**

Compound of formula (J2) can be synthesized from compound of formula (J1) by following the reaction protocol as mentioned in ACS Medicinal Chemistry Letters, 2018, vol. 9, # 8, p. 827 - 831 ($R^b = R^c = CH_3$). Upon thermal cyclization at elevated temperature(s) the compound of the formula (J2) can undergo ring cyclization to produce compound of formula (J3). Such reaction can be carried out by using Lewis acids such as, although not limited to $AlCl_3$, $BF_3$, etc., either neat or by using solvents such as DCM, DCE, chlrobenzene, toluene, xylene, etc. and the like or mixture(s) thereof. Nitration of compound of formula (J3) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (J4). Compound of formula (J4) can be further alkylated by using bases such as NaH, $K_2CO_3$, $Na_2CO_3$, $Cs_2CO_3$ etc. in polar aprotic solvents like THF, DMF, DMSO etc. at appropriate temperature leading to compound of formula (J5). Compound of formula (J5) was converted to corresponding aniline derivative compound of formula (J6) through selective reduction of nitro group by using different reducing agents. Such reducing agents include hydrogenation with palladium on carbon, metal reductions like iron, tin or tin chloride and the like. Such reduction can be carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof. Compound of formula (J6) upon treatment with corresponding alkylnitriles using acids such as but not limited to Methane sulfonic acid, HCl etc. at appropriate temperature to afford compound of formula (J7). The halogenation of compound of formula (J7) to produce the compound of formula (J8). Such reaction can be carried out by using neat halogenating reagents, such as but not limited to $POCl_3$, $POBr_3$, $SOCl_2$ and the like at appropriate temperature. This reaction can also be caried out by using combination of halogenating reagents and organic bases such as $POCl_3$, $POBr_3$, $SOCl_2$ and the like; and organic bases like DIPEA, TEA, N,N-Dimethylaniline and the like; using solvents such as DCE, DCM, chlorobenzene, toluene and the like or mixture(s) thereof at appropriate temperature. The compound of formula (I) can be obtained by using nucleophilic substitution of benzyl amines (A5) with the compound of the formula (J8). Such reaction can be carried out at appropriate temperature in presence of bases like DIPEA, TEA and the like; in solvents such as THF, 1,4-Dioxane, DCE, ACN, DMSO, etc., and the like or mixture(s) thereof.

The compounds of formula (I) was prepared by following the sequential transformations as depicted and described in Scheme -K herein below.

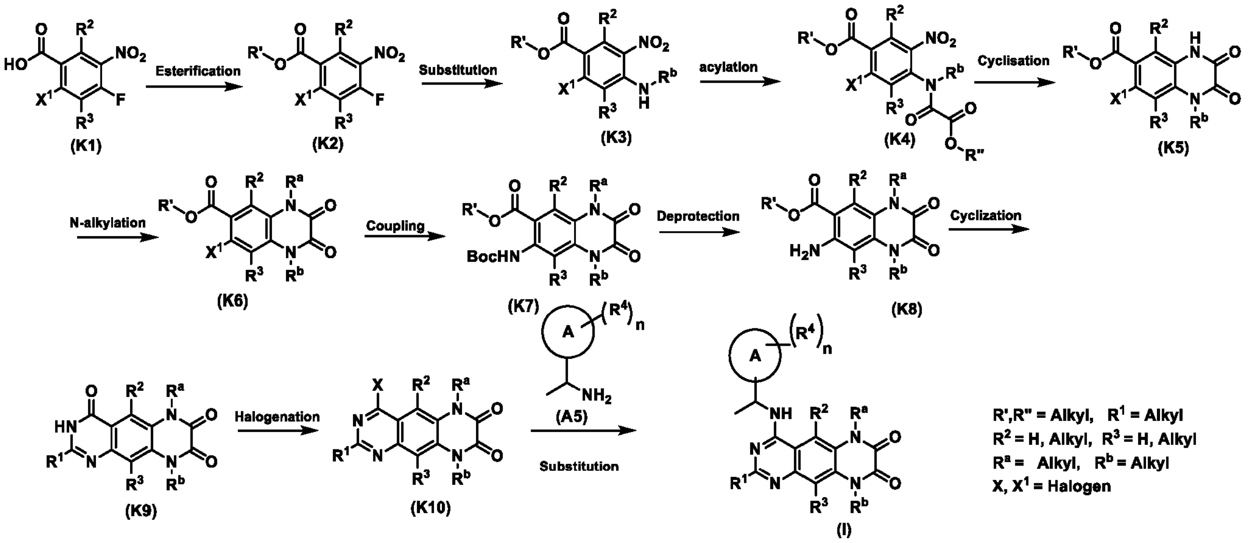


SCHEME - K

The compound of formula (K1) was subjected to esterification in alcoholic solvents like methanol ethanol and thereof in presence of chlorinating agents such as thionyl chloride, oxalyl chloride and thereof, or in presence of acidic reagents such as sulfuric and methane sulfonic acid thereof to provide the compound of formula (K2). Compound of formula (K3) can be synthesized by appropriate displacement of aromatic halogen with corresponding alkyl amine in alcoholic solvents like methanol ethanol and thereof.

Compound of formula (K3) was reacted with oxalyl chloride in the presence of bases like triethyl amine, N,N-ethyldiisopropyl amine, pyridine, DBU or the like in solvents such as THF , 1,4-Dioxane, toluene, DCM, or mixture(s) thereof to provide compound of formula (K4).

Compound of formula (K4) was subjected to cyclisation using dithionate salts in the presence of mixture of solvents such as THF , 1,4-Dioxane , in alcoholic solvents like methanol ethanol and water, mixture(s) thereof to provide compound of formula (K5).

The compound of formula (K5) was alkylated to give compound of formula (K6). This conversion was effected in presence alkali hydrides like sodium hydride and like; or bases such as potassium carbonate and like; and alkylating reagents alkyl halides e.g. Methyl iodide and like; in presence of solvents such as THF, DMF or mixture(s) thereof.

The compound of formula (K6) was subjected to C-N coupling reaction e.g. Buchwald reaction with tert-butyl carbamate provided compound of formula (K7). This reaction can mediated by a suitable catalyst such as, e.g., $Pd(PPh_3)_2Cl_2$, $Pd_2dba_3$, $Pd(PPh_3)_4$, $Pd(OAc)_2$ or mixtures thereof; a suitable ligand such as Xantphos, BINAP, Ru-Phos, XPhos, or mixtures thereof; in the presence of suitable base, preferably inorganic bases such as alkali metal carbonates, e.g., $K_2CO_3$, $Na_2CO_3$, $Cs_2CO_3$, NaOtBu, Potassium phosphate, or mixture thereof. Such reactions can be carried out in solvents like, e.g., ethers such as THF, Dioxane and the like; hydrocarbons, e.g., toluene; amides such as DMF, DMA or mixtures thereof.

Compound of formula (K7) undergoes deprotection using acids like organic acids such as trifluoroacetic acid, Methane sulfonic acid and like, mineral acids like hydrochloric acid, acetic acid (Aqueous or in etheral solvents), sulfuric acid and the like; using solvents like dichloromethane, dichloroethane, THF, 1,4-dioxane and like thereof to provide compound of formula (K8).

Compound of formula (K8) reacted with alkyl nitriles in presence of the reagent such as methane sulfonic acid, sulfuric acid, hydrochloric acid, or the like to obtain compound of formula (K9).

Compound of formula (K9) was reacted with $POCl_3$ or $POBr_3$ optionally in solvents such as toluene, xylene or the like or the mixtures thereof, optionally using organic base such as triethylamine, diisopropylethylamine or the like to provide compound of formula (K10).

Compound of formula (K10) was reacted with compound of formula (A5) in the presence of triethyl amine, N,N-ethyldiisopropyl amine, pyridine, DBU or the like in solvents such as THF, 1,4-Dioxane, toluene, DCM, DMSO or mixture(s) thereof to provide compound of formula (I).

The compounds of formula (I) was prepared by following the sequential transformations as depicted and described in **Scheme** - **L** herein below.

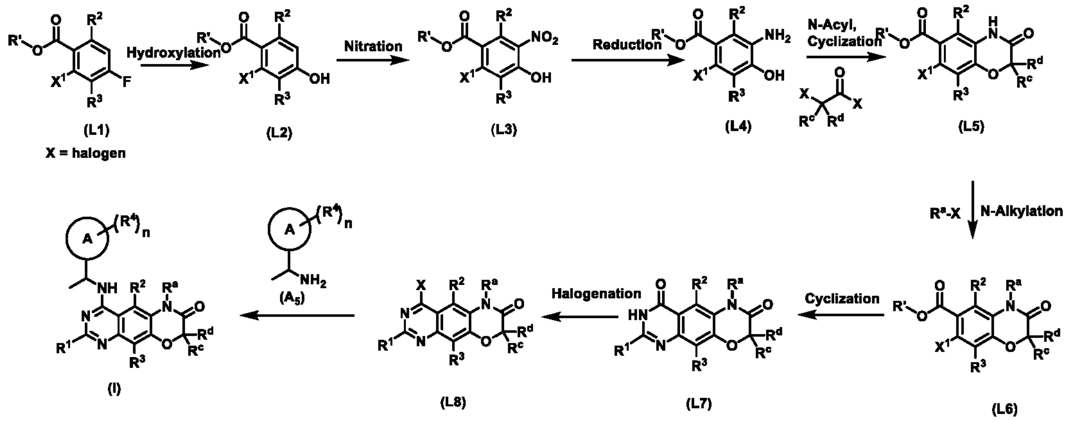


R' = Alkyl, $R^1$ = Alkyl
R' = Alkyl, $R^a$ = Alkyl
$R^d$ = H, Alkyl, $R^c$= Alkyl,
X, $X^1$= Halogen

$R^2$ and $R^3$= group consisting of hydrogen, halogen, cyano, substituted or unsubstituted alkyl, and substituted or unsubstituted cycloalkyl.

SCHEME - L

Compound of formula (L1) allowed to react with N-hydroxyacetamide in presence of the bases such as $K_2CO_3$, $Na_2CO_3$, $Cs_2CO_3$ etc. in polar aprotic solvents like DMF, DMSO etc. at temperature 20°C - 80°C leading to compound of formula (L2). Nitration of compound of formula (L2) with nitrating reagents such as, although not limited to fuming nitric acid, potassium nitrate, and the like in acids such as, although not limited to tin (IV) chloride, sulphuric acid, trifluroacetic acid, acetic acid and the like, anhydrides like acetic anhydride, trifluroacetic anhydride and the like, or mixture(s) thereof to provide compound of formula (L3). Compound of formula (L3) was converted to corresponding aniline derivative compound of formula (L4) through selective reduction of nitro group by using different reducing agents. Although not limited, such reducing agents include hydrogenation with palladium on carbon, metal reductions like iron, tin or tin chloride and the like. Such reduction of the compound of formula (L3) can be carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof. Compound of formula (L4) allowed to react with corresponding acyl halide in presence of the organic basic reagents such as but not limited to DIPEA, TEA etc. in polar aprotic solvents like DMF, DMSO etc. at temperature 20°C - 80°C leading to compound of formula (L5). Compound of formula (L5) can be further alkylated by using bases such as $K_2CO_3$, $Na_2CO_3$, $Cs_2CO_3$ etc. in polar aprotic solvents like DMF, DMSO etc. at temperature 20°C - 60°C leading to compound of formula (L6).Compound of formula (L6) which on coupling with different amidines such as acetamidine, formamidine etc. in polar aprotic solvents like DMF, DMSO etc. at temperature 80°C - 100°C leading to compound of formula (L7).

Compound of formula (L8) can be prepared from compound of formula (L7) by reacting with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (L8).

Compound of formula (L8) undergoes a nucleophilic substitution reaction with different chiral benzylic amines (A5) leading to the final compound of formula (I) using organic basic reagents such as but not limited to DIPEA, TEA etc. in a polar aprotic solvents like dioxane, THF etc. at 0°C -130°C.

The compounds of formula (I) was prepared by following the sequential transformations as depicted and described in Scheme - M herein below.

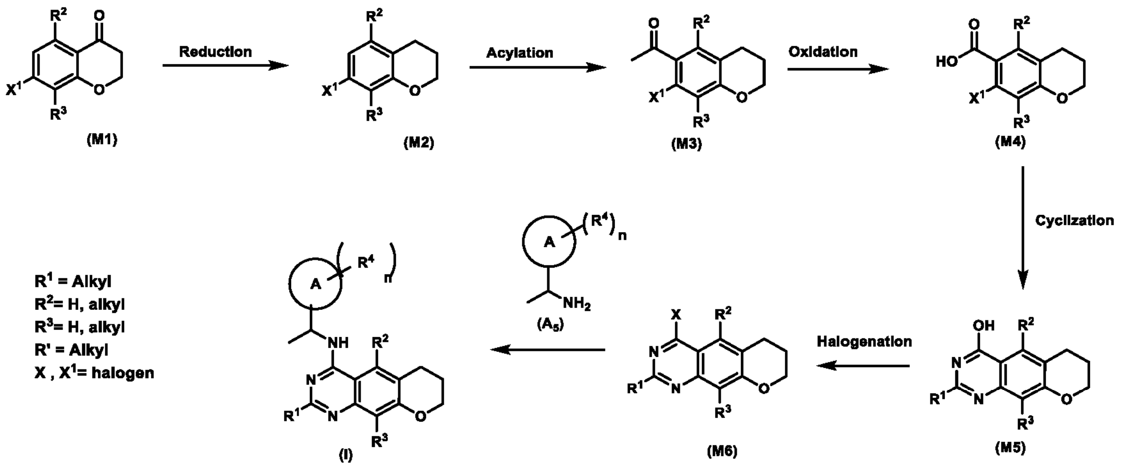


SCHEME - M

Carbonyl functional group in Compound of formula (M1) on further reduction using different reducing reagents such as but not limited to triethyl silane, borane DMS, borane THF, LiAlH4 in polar aprotic solvents like THF, dioxane etc or like in acids such as, although not limited to trifluroacetic acid, sulphuric acid, acetic acid and the like, or mixture(s) thereof to provide compound of formula (M2).

Compound of formula (M2) converted to compound of formula (M3) using Friedel craft acylation. This transformation was carried out by reaction of Compound of formula (M2) with corresponding acyl halide in presence of Lewis acids such as aluminum trichloride, zinc chloride, boron trifluoride etherate and like, in halogenated solvents like dichloromethane, dichloroethane and like.

Compound of formula (M3) was allowed to react with mixture of bromine & aqueous metal hydroxides like NaOH, KOH or the like or mixtures thereof to provide compound of formula (M4).

Compound of formula (M4) which on coupling with different amidines such as acetamidine, formamidine etc. in polar aprotic solvents like DMF, DMSO etc. at temperature 80°C - 100°C leading to compound of formula (M5).

Compound of formula (M6) can be prepared from compound of formula (M5) by reacting with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (M6).

Compound of formula (M6) undergoes a nucleophilic substitution reaction with different chiral benzylic amines (A5) leading to the final compound of formula (I) using organic basic reagents such as but not limited to DIPEA, TEA etc. in a polar aprotic solvents like dioxane, THF etc. at 0°C -130°C.

The compounds of formula (I) was prepared by following the sequential transformations as depicted and described in **Scheme** - **N** herein below.

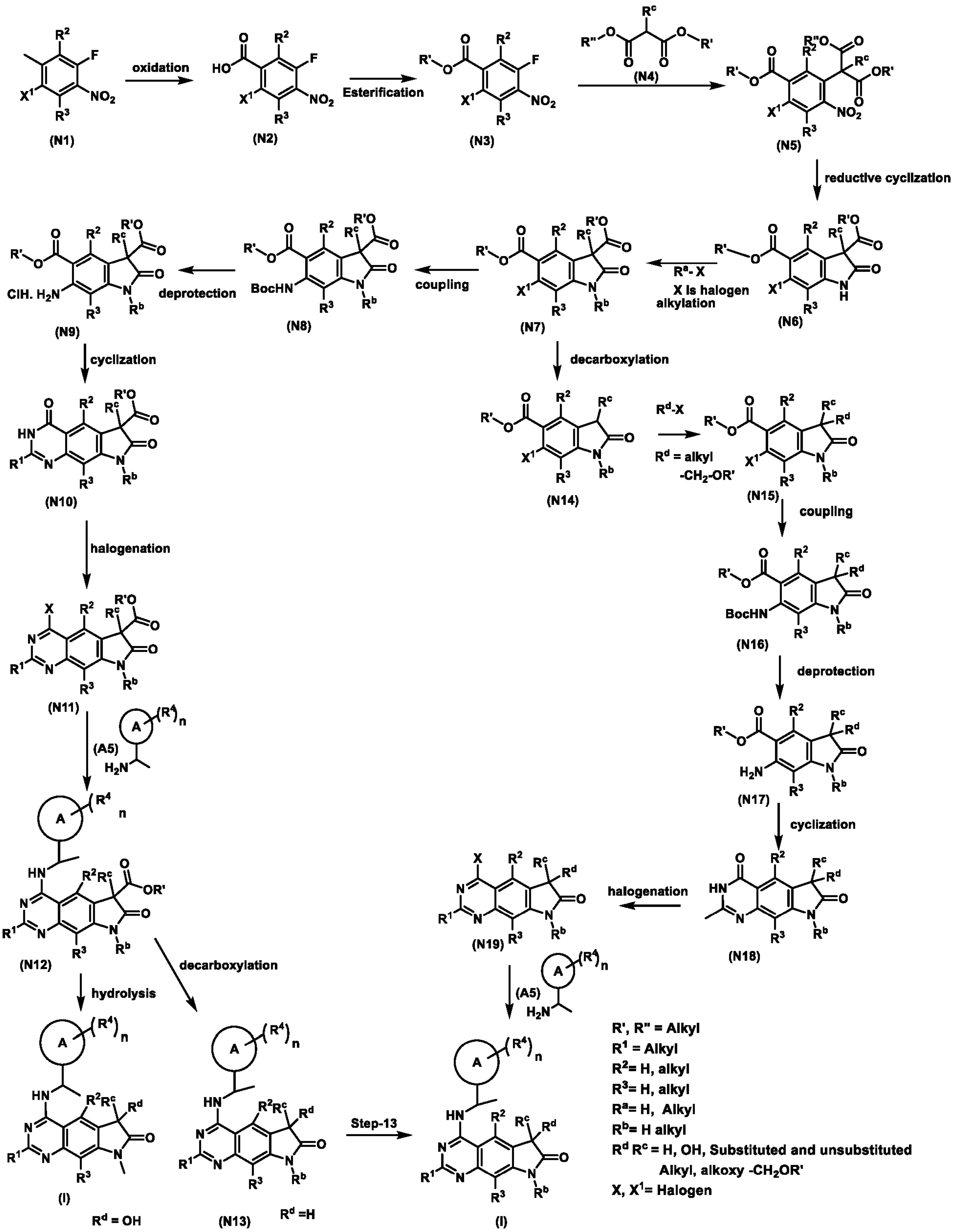


Compound of the formula (N2) was obtained by oxidation of compound of the formula (N1). This transformation can be effected by oxidizing reagents such as potassium permanganate, potassium dichromate, sodium dichromate and like; in presence of acids like $H_2SO_4$, acetic acid and like.

Compound of the formula (N3) was obtained from compound of the formula (N2) by esterification reaction. This transformation can be effected by reaction of alcohols such as methanol, ethanol and like; in presence of mineral acids like sulfuric acid, organic acids like methane sulfonic acid and like, or in presence of chloride reagents like thionyl chloride,

oxalyl chloride and thereof. This transformation can also be effected by Mitsonobu reaction between acid (N3) and corresponding alcohols in presence of Triaryl phosphines and azo carboxylates such as DEAD, DIAD and like.

The reaction between compound of formula (N3) and substituted dialkyl dicarboxylates (compound of the formula (N4)) in presence of base provided compound of the formula (N5). This type of transformations can be carried out either at room temperature or at elevated temperatures using alkali bases such as NaOH, KOH and like; carbonates such as potassium carbonate, cesium carbonate and like; or organic bases like Triethylamine, diisopropylethyl amine and thereof; in amidic solvents like DMF, DMA and like; etheral solvents like 1, 4-dioxane, THF and thereof.

Compound of formula (N5) undergo reductive cyclization to provide compound of formula (N6). The reduction of nitro group was carried out using different reagents; although not limited, such reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and mixtures thereof.

Compound of formula (N6) undergoes N-alkylation using alkyl halides and bases such as $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (N7).

Compound of formula (N7) allowed to react with tert-butyl carbamate in the presence of catalyst such as (tris(dibenzylideneacetone) dipalladium(0), palladium (II) acetate, Bis(dibenzylideneacetone)$_2$ Pd(0), racemic 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as sodium carbonate, cesium carbonate, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (N8).

Compound of formula (N8) undergoes deprotection using acids like organic acids such as trifluoroacetic acid, Methane sulfonic acid and like, mineral acids like hydrochloric acid, acetic acid (aqueous or in etheral solvents), sulfuric acid and the like; using solvents like dichloromethane, dichloroethane, THF, 1,4-dioxane and like thereof to provide compound of formula (N9).

Compound of formula (N9) allowed to react with alkylnitrile in presence of the acidic reagents such as methane sulfonic acid, sulfuric acid, hydrochloric acid or the like to obtain compound of formula (N10). The same transformation can be carried out using trialkyl orthoacetate in presence of ammonium acetate, in corresponding polar protic solvents like ethanol, methanol and thereof.

Alternatively, compound of formula (N8) on reaction with alkylnitrile in presence of the acidic reagents such as methane sulfonic acid, sulfuric acid, hydrochloric acid or the like can directly give compound of formula (N10)

Compound of formula (N10) can also be obtained directly from compound of formula (N8) by reaction alkylnitrile in presence of the acidic reagents such as methane sulfonic acid, sulfuric acid, hydrochloric acid and thereof.

Compound of formula (N10) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (N11). Compound of formula (N11) allowed to react with compound of formula (A5) in presence of suitable coupling reagent to provide compound of formula (N12). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof.

Compound of formula (N12) converted to compound of formula (I) in presence of alkali hydroxides such as NaOH, LiOH and thereof, in solvents like methanol, ethanol and thereof or using tetrabutyl ammonium halide in etheral solvents like THF, 1,4-dioxane and thereof.

Compound of formula (N12) undergoes decarboxylation reaction to furnish compound of the formula (N13). This transformation can be effected by acidic reagents such as mineral acids like sulfuric acid, organic acids like trifluoroacetic acid and thereof; similar transformation can be achieved using sodium chloride, lithium chloride and thereof, in solvents such as dimethyl sulfoxide and like; at elevated temperatures.

Compound of formula (N13) converted to compound of formula (I) using ceric ammonium nitrate, thallium nitrate and thereof in present of alcoholic solvents like methanol, ethanol and thereof.

Further, Compound of formula (N7) undergoes decarboxylation reaction to furnish compound of the formula (N14). This transformation can be achieved using sodium chloride, lithium chloride and thereof, in solvents such as dimethyl sulfoxide and like, at elevated temperatures. Similar transformation can be effected by acidic reagents such as mineral acids like sulfuric acid, organic acids like trifluoroacetic acid and thereof.

Compound of formula (N14) undergoes C-alkylation reaction with alkyl halides in presence of bases such as NaH, sodium/potassium alkoxides, $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1, 4-dioxane and like,

at room temperature or elevated temperatures provide compound of formula (N15)

Compound of formula (N15) can be converted to compound of formula (I) in five steps by employing analogous protocol mentioned above in scheme -N for the conversion of compound of formula (N7) to compound of formula (N12).

The compounds of formula (I) was prepared by following the sequential transformations as depicted and described in Scheme-O herein below.

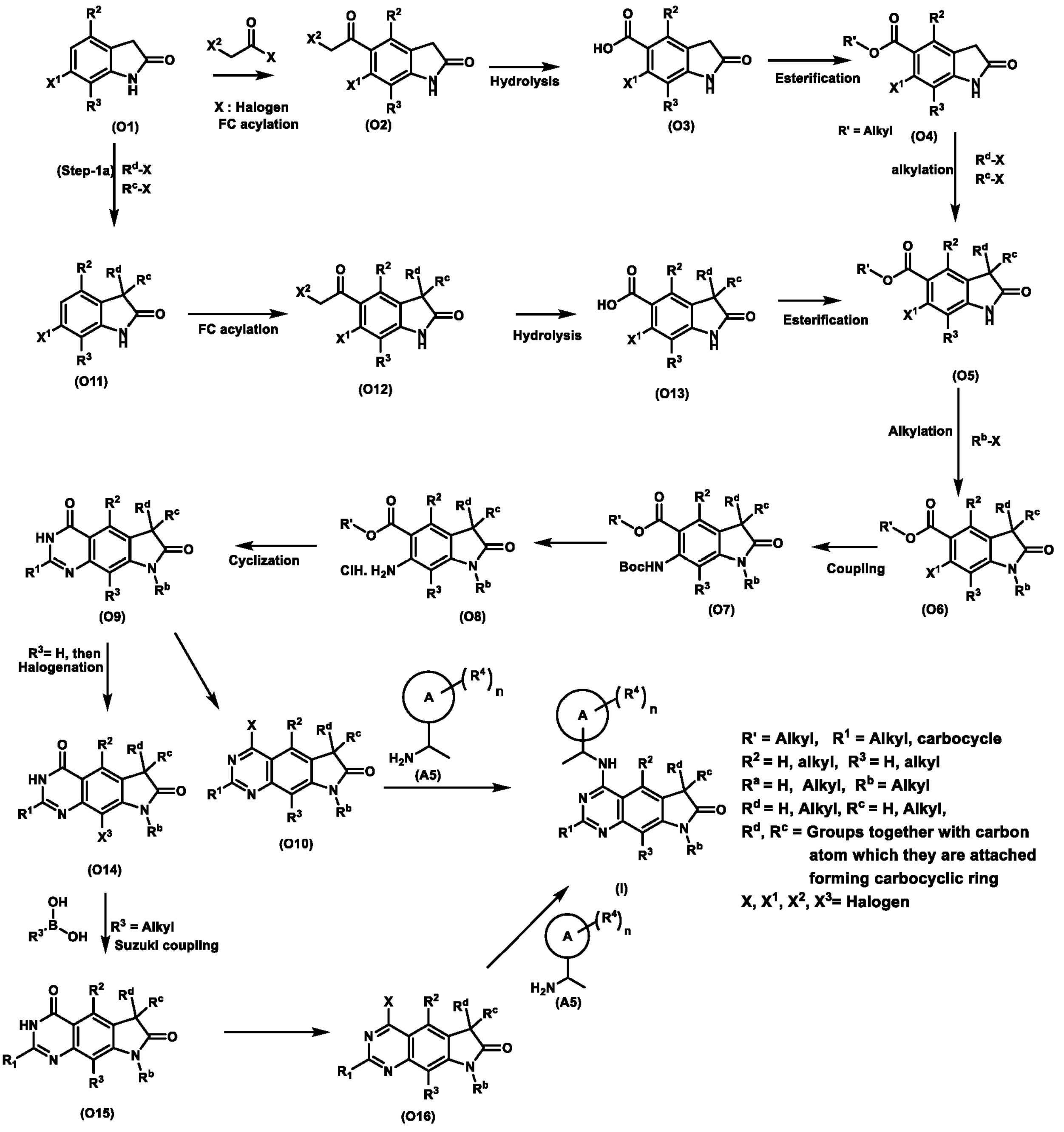


(O1) (O2) (O3) (O4) (O5) (O6) (O7) (O8) (O9) (O10) (O11) (O12) (O13) (O14) (O15) (O16) (A5) (I)

**SCHEME - O**

Compound of formula (O1) converted to compound of formula (O2) using Friedel craft acylation. This transformation was carried out by reaction of Compound of formula (O1) with corresponding acyl halide in presence of Lewis acids such as aluminum trichloride, zinc chloride, boron trifluoride etherate and like, in halogenated solvents like dichloromethane, dichloroethane and like.

Compound of formula (O2) was allowed to react with pyridine, optionally in solvents such as THF, toluene, xylene or the like or the mixtures thereof, followed by treatment of aqueous metal hydroxides like NaOH, KOH or the like or mixtures thereof to provide compound of formula (O3).

Compound of formula (O3) acid derivative undergoes esterification reaction to corresponding compound of formula (O4) using solvents such as methanol, ethanol, propanol, tert-butanol using acidic conditions like hydrochloric acid, sulfuric acid, thionyl chloride or the like or mixture(s) thereof.

Compound of formula (O4) was undergoes coupling with alkyl/substituted alkyl halide/dihalides to the corresponding formula (O5) using bases like Lithium diisopropylamide, butyl lithium, lithium bis(trimethylsilyl)amide, sodium bis(trimethylsilyl)amide, sodium tert-butoxide, potassium tertbutoxide, sodium ethoxide, sodium methoxide, cesium carbonate, potassium carbonate or the like possibly in the presence of additives such as N,N,N',N'-Tetramethylethane-1,2-diamine in solvents selected from THF, 1,4-dioxane, DMF and like.

Alternatively, the compound of formula (O1) undergoes alkylation/acylation reaction to give compound of formula (O11) the reaction was carried out using alkyl halides/ acyl halide and bases like Lithium diisopropylamide, butyl lithium, lithium bis(trimethylsilyl)amide, sodium bis(trimethylsilyl)amide, sodium tert-butoxide, potassium tertbutoxide, sodium ethoxide, sodium methoxide, cesium carbonate, potassium carbonate or the like possibly in the presence of additives such as N,N,N',N'-Tetramethylethane-1,2-diamine in solvents selected from THF, 1,4-dioxane, DMF and like

Compound of formula (O11) was converted to compound of formula (013) by employing similar protocol mentioned above for conversion of compound of formula (O1) to compound of formula (O3).

Compound of formula (013) undergoes esterification reaction to corresponding compound of formula (O5) using solvents such as methanol, ethanol, propanol, tert-butanol using acidic conditions like hydrochloric acid, sulfuric acid, thionyl chloride or the like or mixture(s) thereof.

Compound of formula (O5) can be further reacted with alkyl halide, acyl chlorides using bases such as $K_2CO_3$, $Na_2CO_3$, $Cs_2CO_3$ etc. in polar aprotic solvents like DMF, DMSO etc. at elevated temperatures leading to compound of formula (O6)

Compound of formula (O6) allowed to react with tert-butyl carbamate in the presence of catalyst such as (tris(dibenzylideneacetone) dipalladium(0), palladium (II) acetate, Bis(dibenzylideneacetone)2 Pd(0), racemic 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as sodium carbonate, cesium carbonate, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (O7).

Compound of formula (O7) undergoes deprotection using acids like organic acids such as trifluoroacetic acid, Methane sulfonic acid and like, mineral acids like hydrochloric acid, acetic acid (aqueous or in etheral solvents), sulfuric acid and the like; using solvents like dichloromethane, dichloroethane, THF, 1,4-dioxane and like, to provide compound of formula (O8).

Compound of formula (O8) allowed to react with alkylnitrile in presence of the acidic reagents such as methane sulfonic acid, sulfuric acid, hydrochloric acid and the like to obtain compound of formula (O9). The same transformation can be carried out using trialkyl orthoacetate in presence of ammonium acetate, in corresponding polar protic solvents like ethanol, methanol and thereof.

Further, compound of formula (O7) on reaction with alkylnitrile in presence of the acidic reagents such as methane sulfonic acid, sulfuric acid, hydrochloric acid or the like can directly give compound of formula (O9)

Compound of formula (O9) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (O10). Compound of formula (O10) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof.

Further, compound of formula (O10) converted to compound of formula (014) using halogenating reagents such as NBS, NCS, bromine and like, in polar solvents such as DMF, AcOH, DCM and like.

Compound of formula (015) was prepared from compound of formula (014) using C-C coupling reactions such as Suzuki coupling reaction using corresponding boronic acid in presence of Pd catalyst such as tris(dibenzylideneacetone) dipalladium(0), palladium(II)acetate, Bis(dibenzylideneacetone)2Pd(0), rac 2,2'-Bis(diphenylphosphino)-1,1'-binaphthyl, 2,5 bis(tri-t-butylphosphine) palladium (0), $Pd(PPh_3)_4$ and like in base such as $K_2CO_3$, $Na_2CO_3$, $Cs_2CO_3$, Potassium phosphate and like; in solvents such as toluene, 1,4-dioxane , DMA, DMF and like

The compound of formula (014) can be converted to compound of formula (I) using similar protocol used earlier for conversion of compound of formula (O9) to compound of formula (I) in two steps.

The compounds of formula (I) was prepared by following the sequential transformations as depicted and described in Scheme - P herein below.

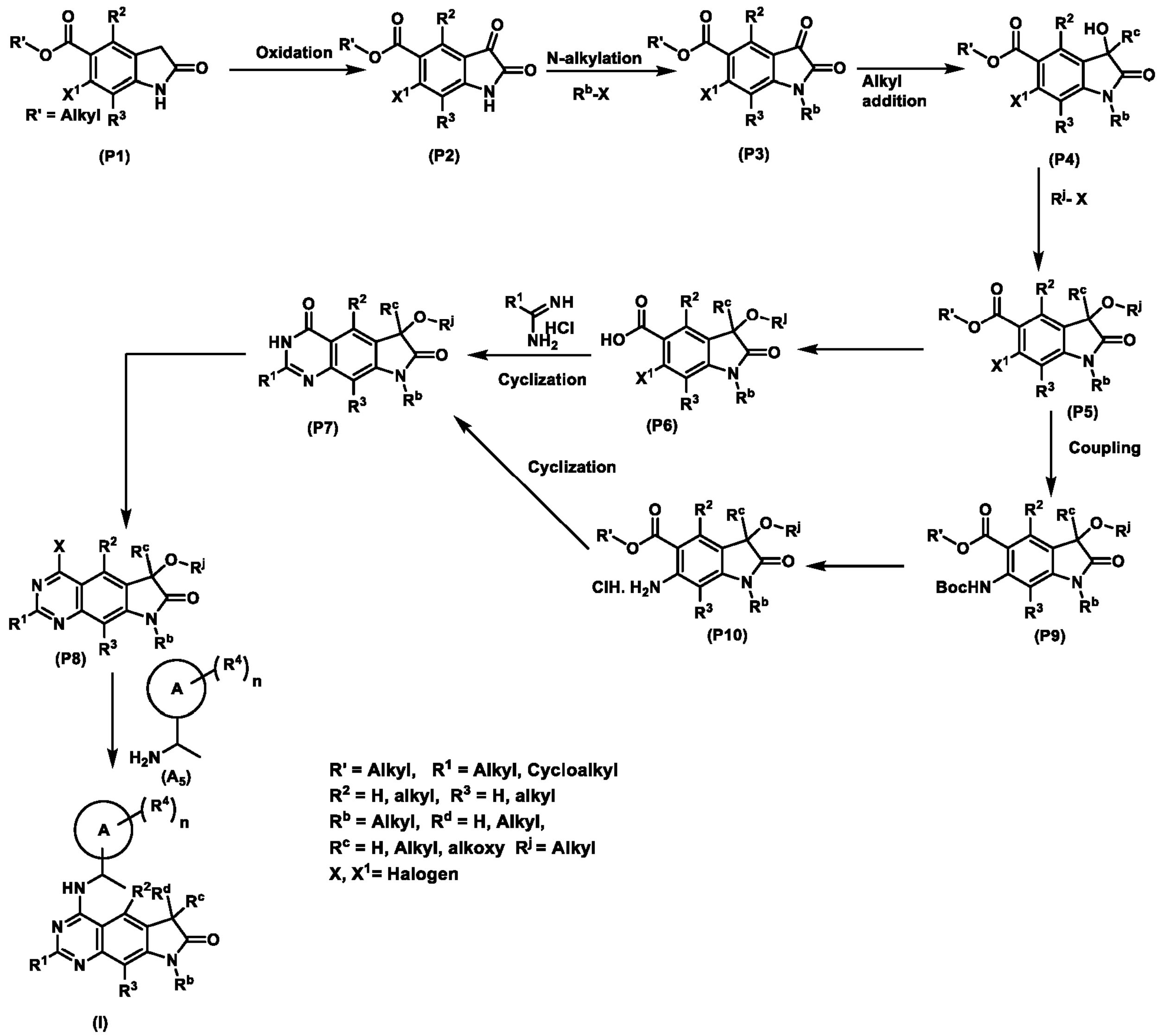


## SCHEME - P

Compound of the formula (P2) was obtained by oxidation of compound of the formula (P1). This transformation can be effected by oxidizing reagents such as potassium permanganate, potassium dichromate, sodium dichromate and like; in presence of acids like $H_2SO_4$, acetic acid and like.

Compound of formula (P2) undergoes N-alkylation using alkyl halides in presence of bases such as NaH, Potassium/sodium alkoxides, $K_2CO_3$, $Na_2CO_3$, $Cs_2CO_3$, organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (P3). Compound of formula (P3) undergoes reaction with organometallic reagents such as grignard reagent, dialkyl zinc , alkyl lithiums, and thereof; silane reagents such as trifluromethyl trimethyl silane and thereof; in etheral solvents such as THF, MTBE and like to provide compounds of formula (P4)

Compound of formula (P4)undergoes O-alkylation using alkyl halides in presence of bases such as sodium hydride, potassium /sodium alkoxide $K_2CO_3$, $Na_2CO_3$, $Cs_2CO_3$, NaH and thereof; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (P5).

Compound of formula (P5) converted to compound of formula (P6) in presence of alkali hydroxides such as NaOH, LiOH and thereof, in solvents like methanol, ethanol and thereof or using solvents like THF, 1,4-dioxane and thereof.

Compound of formula (P6) on reaction with acetamidine, formamidine and like; in polar aprotic solvents like DMF,

DMSO and metals like copper, thereof at temperature elevated temperatures afforded compound of formula (P7).

Alternatively, Compound of formula (P5) allowed to react with tert-butyl carbamate in the presence of catalyst such as (tris(dibenzylideneacetone) dipalladium(0), palladium (II) acetate, Bis(dibenzylideneacetone)2 Pd(0), racemic 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as sodium carbonate, cesium carbonate, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (P9).

Compound of formula (P9) undergoes deprotection using acids like organic acids such as trifluoroacetic acid, Methane sulfonic acid and like, mineral acids like hydrochloric acid, acetic acid (aqueous or in etheral solvents), sulfuric acid and the like; using solvents like dichloromethane, dichloroethane, THF, 1,4-dioxane and like thereof to provide compound of formula (P10).

Compound of formula (P10) allowed to react with alkylnitrile in presence of the acidic reagents such as methane sulfonic acid, sulfuric acid, hydrochloric acid or the like to obtain compound of formula (P7). The same transformation can be carried out using trialkyl orthoacetate in presence of ammonium acetate, in corresponding polar protic solvents like ethanol, methanol and thereof.

Alternatively, compound of formula (P9) on reaction with alkylnitrile in presence of the acidic reagents such as methane sulfonic acid, sulfuric acid, hydrochloric acid or the like can directly give compound of formula (P7)

Compound of formula (P7) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (P8). Compound of formula (P8) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof.

The compounds of formula (I) was prepared by following the sequential transformations as depicted and described in **Scheme-Q** herein below

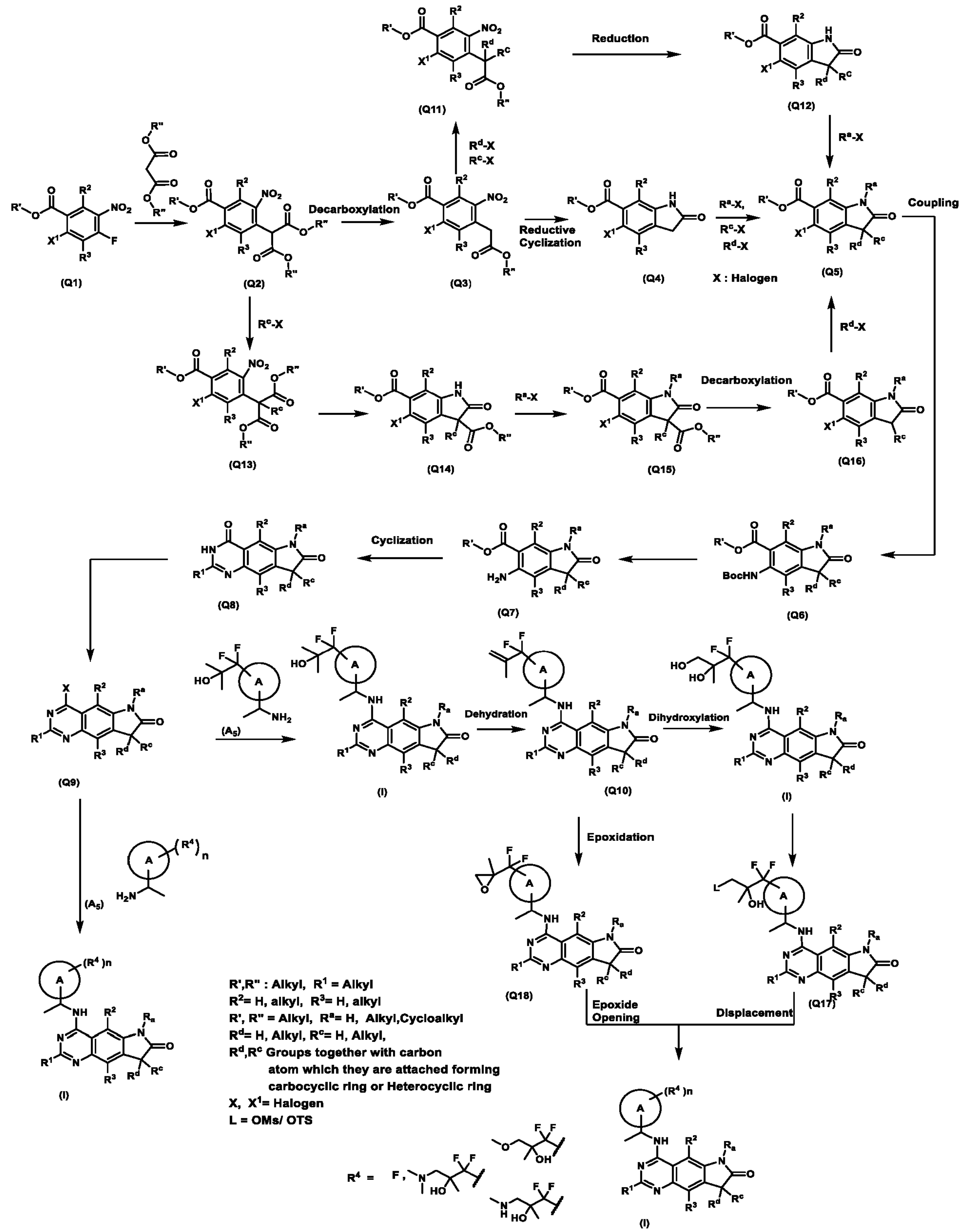


The reaction between compound of formula (Q1) and substituted dialkyl dicarboxylates in presence of base provided compound of the formula (Q2). This type of transformations can be carried out at appropriate temperature using alkali bases such as NaOH, KOH and like; carbonates such as potassium carbonate, cesium carbonate and like; or organic bases like Triethylamine, diisopropyl ethyl amine and the like; in amidic solvents like DMF, DMA and like; etheral solvents like 1, 4-dioxane, THF and mixtures thereof.

Compound of formula (Q2) undergoes decarboxylation reaction to furnish compound of formula (Q3). This

transformation was carried out in polar solvents like DMSO, DMF, and like, using sodium chloride, lithium chloride and like. Similar transformation can be done using acids such as sulfuric acid, trifluoroacetic acid and like, at appropriate temperature.

Reductive cyclization of compound of the formula (Q3) provide compound of formula (Q4). The reduction of nitro group was carried out using different reagents; although not limited, such reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof.

Compound of formula (Q4) undergoes alkylation reaction by reacting with corresponding alkyl halide in presence of bases such as sodium hydride, potassium tert butoxide, $K_2CO_3$, $Na_2CO_3$, $Cs_2CO_3$; organic bases like diisopropyl ethyl amine, DBU, DABCO and the like; in polar aprotic solvents like DMF, DMSO, acetone and like, etheral solvents such as THF, 1,4-dioxane and like, at appropriate temperature provided compound of formula (Q5).

Alternatively, Compound of formula (Q3) undergoes C-alkylation reaction by reacting with corresponding alkyl halide in presence of bases such as sodium hydride, potassium tert butoxide and like; in polar aprotic solvents like DMF, DMSO, acetone and like, etheral solvents such as THF, 1,4-dioxane and like, to provide compound of formula (Q11). Compound of formula (Q11) undergoes reductive cyclization similar to conversion of compound of formula (Q3) to compound of formula (Q4) to provide compound of formula (Q12). Compound of formula (Q12) undergoes N-alkylation reaction with alkyl halides in presence of bases such as NaH, Potassium/sodium alkoxides, $K_2CO_3$, $Na_2CO_3$, $Cs_2CO_3$, organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (Q5) Alternatively, Compound of formula (Q2) undergoes C-alkylation using corresponding alkyl halide in presence of bases such as sodium hydride, potassium tert butoxide , $K_2CO_3$, $Na_2CO_3$, $Cs_2CO_3$ and like; in polar aprotic solvents like DMF, DMSO, acetone and like, etheral solvents such as THF, 1,4-dioxane and like to provide compound of formula (Q13).

The compound of formula (Q13) was converted to compound of formula (Q15) in two steps viz. reductive cyclization and N-alkylation by following similar reactions employed for conversion of compound of formula (Q3) to compound of formula (Q5).

Compound of formula (Q15) undergoes decarboxylation reaction to furnish compound of formula (Q16). This transformation was carried out in polar solvents like DMSO, DMF, and like, using sodium chloride, lithium chloride and like. Similar transformation can be done using acids such as sulfuric acid, trifluoroacetic acid and like, at elevated temperatures.

Compound of formula (Q16) undergoes C-alkylation using corresponding alkyl halide in presence of bases such as sodium hydride, potassium tert butoxide , $K_2CO_3$, $Na_2CO_3$, $Cs_2CO_3$ and like; in polar aprotic solvents like DMF, DMSO, acetone and like, etheral solvents such as THF, 1,4-dioxane and like to provide compound of formula (Q5).

Compound of formula (Q5) allowed to react with tert-butyl carbamate in the presence of catalyst such as (tris(dibenzylideneacetone)dipalladium(0), palladium(II) acetate, Bis(dibenzylideneacetone)2 Pd(0), rac 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as sodium carbonate, cesium carbonate, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (Q6).

Compound of formula (Q6) undergoes deprotection using acids like organic acids such as trifluoroacetic acid, Methane sulfonic acid and like, mineral acids like hydrochloric acid, acetic acid (Aqueous or in etheral solvents), sulfuric acid and the like; using solvents like dichloromethane, dichloroethane, THF, 1,4-dioxane and like thereof to provide compound of formula (Q7).

Compound of formula (Q7) allowed to react with alkylnitrile in presence of the acidic reagents such as methane sulfonic acid, sulfuric acid, hydrochloric acid or the like to obtain compound of formula (Q8). The same transformation can be carried out using trialkyl orthoacetate in presence of ammonium acetate, in corresponding polar protic solvents like ethanol, methanol and thereof.

Alternatively, compound of formula (Q6) on reaction with alkylnitrile in presence of the acidic reagents such as methane sulfonic acid, sulfuric acid, hydrochloric acid or the like can directly give compound of formula (Q8)

Compound of formula (Q8) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (Q9). Compound of formula (Q9) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof.

Compound of formula (Q9) allowed to react with 1-(3-(1-aminoethyl)-2-fluorophenyl)-1,1-difluoro-2-methylpropan-2-ol hydrochloride in presence of suitable coupling reagent to provide compound of formula (I). The reaction can be

carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof. Compound of formula (I) allowed to react with fluorinating reagent such as DAST, martin sulfurane in solvents such as DCM, chloroform, THF, ether, 1,4-dioxane to provide compound of formula (Q10).

Compound of formula (Q10) allowed to react with osmium tetra oxide, potassium osmate dihydrate (Sharpless asymmetric dihydroxylation method) using potassium chlorate, hydrogen peroxide, potassium ferricyanide, N-methylmorpholine N-oxide, chiral quinine or the like, in solvents like acetone, tert butanol water system to provide compound of formula (I).

Compound of formula (I) undergoes mesylation, tosylation and thereof , reactions in presence of organic bases such as TEA, DIPEA, Pyridine and like, in solvents such as THF, DCM and mixtures thereof, to provide compound of formula (Q17)

Compound of formula (Q17) undergoes displacement reaction with primary or secondary amines in presence of alcohol solvents such as ethanol, IPA and mixtures thereof to provide compound of formula (I).

Compound of formula (Q10) undergoes epoxidation reaction to provide compound of formula (Q18). This reaction is effected by hydrogen peroxide in presence of acidic medium using organic acids such as formic acid and like.

Compound of formula (Q18) on epoxide opening by nucleophilic reagent provide compound of formula (I). Such transformations can be effected by reaction of epoxide compound with various nucleophilic reagents such as sodium alkoxides, primary or secondary amines in alcohol solvents like ethanol, methanol, and like and at room temperature or elevated temperature.

The compounds of formula (I) was prepared by following the sequential transformations as depicted and described in **Scheme-R** herein below

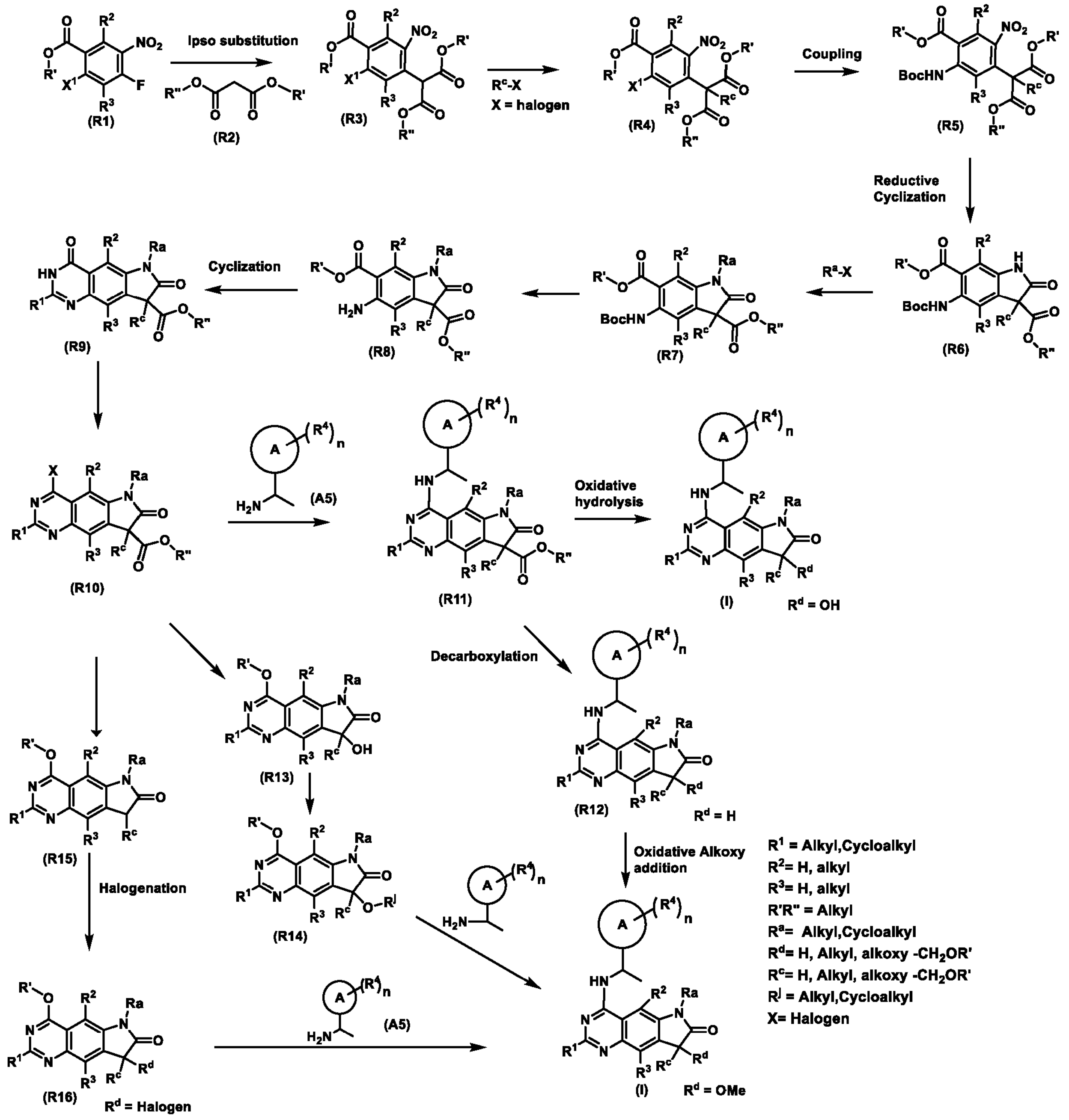


SCHEME - R

The reaction between compound of formula (R1) and substituted dialkyl dicarboxylates (compound of the formula (R2) in presence of base provided compound of the formula (R3). This type of transformations can be carried out either at room temperature or at elevated temperatures using alkali bases such as NaOH, KOH and like; carbonates such as potassium carbonate, cesium carbonate and like; or organic bases like triethylamine, diisopropylethyl amine and thereof; in amidic solvents like DMF, DMA and like; etheral solvents like dioxane, THF and thereof.

Compound of formula (R3) undergoes alkylation using alkyl halides in presence of bases such as sodium hydride, potassium /sodium alkoxide bases such as $K_2CO_3$, $Na_2CO_3$, $Cs_2CO_3$; organic bases like diisopropylethyl amine, DBU, DABCO and so on; in polar aprotic solvents like DMF, DMSO and like, at room temperature or elevated temperatures provide compound of formula (R4).

Compound of formula (R4) allowed to react with tert-butyl carbamate in the presence of catalyst such as (tris(dibenzylideneacetone) dipalladium(0), palladium (II) acetate, Bis(dibenzylideneacetone)2 Pd(0), rac 2,2'-Bis(diphenylphosphino)-1,1'-binaphthyl,2,5 bis(tri-t-butylphosphine) palladium (0) and the like; in presence of ligands such as RuPhos, Xanthphos, Davephos, BINAP, or the like; using a suitable base such as sodium carbonate, cesium carbonate,

sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (R5).

Compound of formula (R5) undergo reductive cyclization to provide compound of formula (R6). This nitro reduction can be achieved by reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof. Compound of formula (R6) undergoes N-alkylation using alkyl halides in presence of bases such as sodium hydride, potassium /sodium alkoxide bases such as $K_2CO_3$, $Na_2CO_3$, $Cs_2CO_3$; organic bases like diisopropylethyl amine, DBU, DABCO and so on; in polar aprotic solvents like DMF, DMSO and like, at room temperature or elevated temperatures provide compound of formula (R7).

Compound of formula (R7) can be converted to the compound of formula (R11) by employing 4 step protocol mentioned in conversion of compound of formula (N8) to compound of formula (N12)

Compound of formula (R11) undergoes decarboxylation reaction to furnish compound of the formula (R12). This transformation can be effected by acidic reagents such as mineral acids like sulfuric acid, organic acids like trifluoroacetic acid and thereof; similar transformation can be achieved using sodium chloride, lithium chloride and thereof, in solvents such as dimethyl sulfoxide and like; at elevated temperatures. Compound of formula (R12) converted to compound of formula (I) using ceric ammonium nitrate, thallium nitrate and thereof in present of alcoholic solvents like methanol, ethanol and thereof.

Further, Compound of formula (R11) on reaction with alkalis such as NaOH, LiOH and like, in alcoholic solvents like methanol ethanol and thereof, provide compound of formula (I) where ($R^d$= -OH)

Compound of formula (R10) undergoes nucleophilic substitution along with air oxidation in presence of bases like LiOH and like, in alcoholic solvent such as methanol in presence of air, provide compound of formula (R13).

Compound of formula (R13) on O-alkylation using corresponding alkyl halide in presence of bases such as sodium hydride, potassium tert butoxide , $K_2CO_3$, $Na_2CO_3$, $Cs_2CO_3$ and like; in polar aprotic solvents like DMF, DMSO, acetone and like, etheral solvents such as THF, 1,4-dioxane and like to provide compound of formula (R14). This reaction Yielded decarboxylation product viz. compound of formula (R15).

Compound of formula (R14) undergoes coupling reaction with compound of formula (A5) to furnish compound of formula (I). The reaction can be carried out in presence of organic base such as diisopropylethylamine, triethylamine, DBU or the like, or using coupling reagents such as DCC, EDC, BOP, pyBOP, HBTU or the like; either neat reaction in base or in etheral solvents such as THF, 1,4 dioxane and like or polar aprotic solvents like DMF, DMA, DMSO and thereof.

Compound of formula (R15) undergoes fluorination reaction by fluorinating reagents such as DAST, selectflour and thereof. or C-alkylation reaction with various alkyl halides in presence of bases such as sodium hydride, potassium tert butoxide , $K_2CO_3$, $Na_2CO_3$, $Cs_2CO_3$ and like; in polar aprotic solvents like DMF, DMSO, acetone and like, etheral solvents such as THF, 1,4-dioxane and like to give compound of formula (R16).

Compound of formula (R16) can be converted to compound of formula (I) by analogous protocol mentioned above for the conversion of (R14) to compound of formula (I).

The compounds of formula (I) was prepared by following the sequential transformations as depicted and described in **Scheme S:**

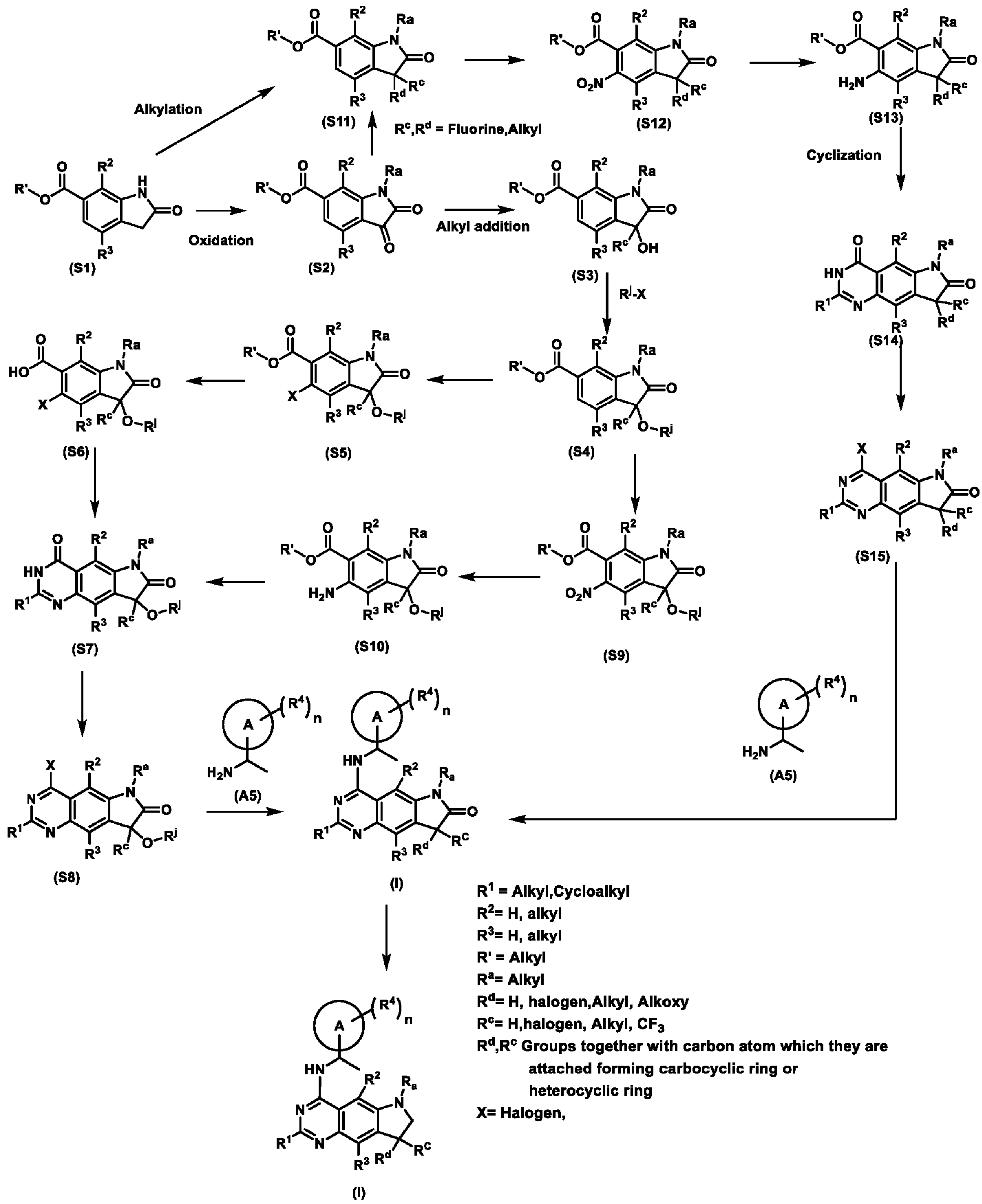


## SCHEME - S

Compound of formula (S2) was prepared from compound of formula (S1) by oxidation reaction followed by N-alkylation reaction. This oxidation was effected by reagents like tertiary butyl hydroperoxide, selenium dioxide, manganese dioxide and like; in presence of catalytic CuI, Cu(I) reagents and thereof. Further the N-alkylation was carried out by using alkyl halides in presence of bases such as sodium hydride, potassium /sodium alkoxide bases such as $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (S2) Compound of formula (S2) undergoes reaction with organometallic

reagents such as Grignard reagent, dialkyl zinc , alkyl lithiums, and thereof; silane reagents such as trifluoromethyl trimethyl silane and thereof; in etheral solvents such as THF, MTBE and like to provide compounds of formula (S3)

Compound of formula (S3) undergoes O-alkylation to provide compound of formula (S4). This transformation can be effected by using alkyl halides in presence of bases such as sodium hydride, potassium /sodium alkoxide $K_2CO_3$, $Na_2CO_3$, $Cs_2CO_3$, sodium hydride; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures.

Compound of formula (S5) can be prepared from compound of formula (S4) by employing halogenation reaction. Such reactions can be carried out in presence of halogenating reagents such as N-halo succinamide, hydrohaloic acid and likes; in solvents like DMF, Acetic acid and thereof; optionally in presence additives such as trifluoroacetic acid and like, in catalytic or molar proportions; and at room temperature or at elevated temperatures.

Compound of formula (S5) undergoes hydrolysis of ester group to provide compound of formula (S6). This transformation can be effected in presence of alkali hydroxides such as NaOH, LiOH and thereof, in solvents like methanol, ethanol and thereof or using solvents like THF, 1,4-dioxane and thereof

Compound of formula (S6) on reaction with acetamidine, formamidine and like; in polar aprotic solvents like DMF, DMSO and metals copper and like; optionally in presence of additives like proline and thereof, at room temperature or elevated temperatures afforded compound of formula (S7).

Alternatively compound of formula (S7) can be prepared in three steps. Compound of formula (S4) undergoes nitration reaction to provide compound of formula (S9). This reaction was carried out in presence of nitrating reagents such as potassium nitrate, sodium nitrate nitric acid and like; in acidic solvents such as sulfuric acid and thereof.

Compound of formula (S9) undergoes reduction reaction to provide compound of formula (S10). These transformations can be carried out using reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof

Compound of formula (S10) allowed to react with alkylnitrile in presence of the acidic reagents such as methane sulfonic acid, sulfuric acid, hydrochloric acid or the like to obtain compound of formula (S7). The same transformation can be carried out using trialkyl orthoacetate in presence of ammonium acetate, in corresponding polar protic solvents like ethanol, methanol and thereof.

Compound of formula (S7) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (S8). Compound of formula (S8) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; either neat or in etheral solvents such as THF, 1,4 dioxane and like or polar aprotic solvents like DMF, DMA, DMSO and thereof

Further, compound of formula (S2) undergoes difluorination reaction with reagents such as DAST, selectfluor and like, in chlorinated solvent like dichloromethane and like; provided compound of formula (S11) ( $R^c$, $R^d$ = F)

Also, compound of formula (S1) undergoes c-alkylation and N-alkylation simultaneously in presence of alkyl halides in presence of bases such as sodium hydride, potassium /sodium alkoxide, $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (S11) ( $R^c$, $R^d$ = alkyl)

Compound of formula (S11) can be converted to compound of formula (I) by employing analogous five step protocol as mentioned above for conversion of compound of formula (S4) to compound of formula (I). Further, Compound of formula (S11) can be converted to compound of formula (S14) by employing analogous three step protocol as mentioned above for conversion of compound of formula (S4) to compound of formula (S7) via compound of formula (S5) followed by compound of formula (S6).

Compound of formula (S14) can be converted to compound of formula (I) by employing analogous two step protocol as mentioned above for conversion of compound of formula (S7) to compound of formula (I). Compound of formula (I) further on reaction with various organometallic reagents like $LiAlH_4$,$BH_3$-DMS and like provide compound of formula (I) These reactions are carried out in one or more solvents, e.g., ethers such as THF, 1,4-dioxane, and the like

The compounds of formula (I) was prepared by following the sequential transformations as depicted and described in **Scheme T:**

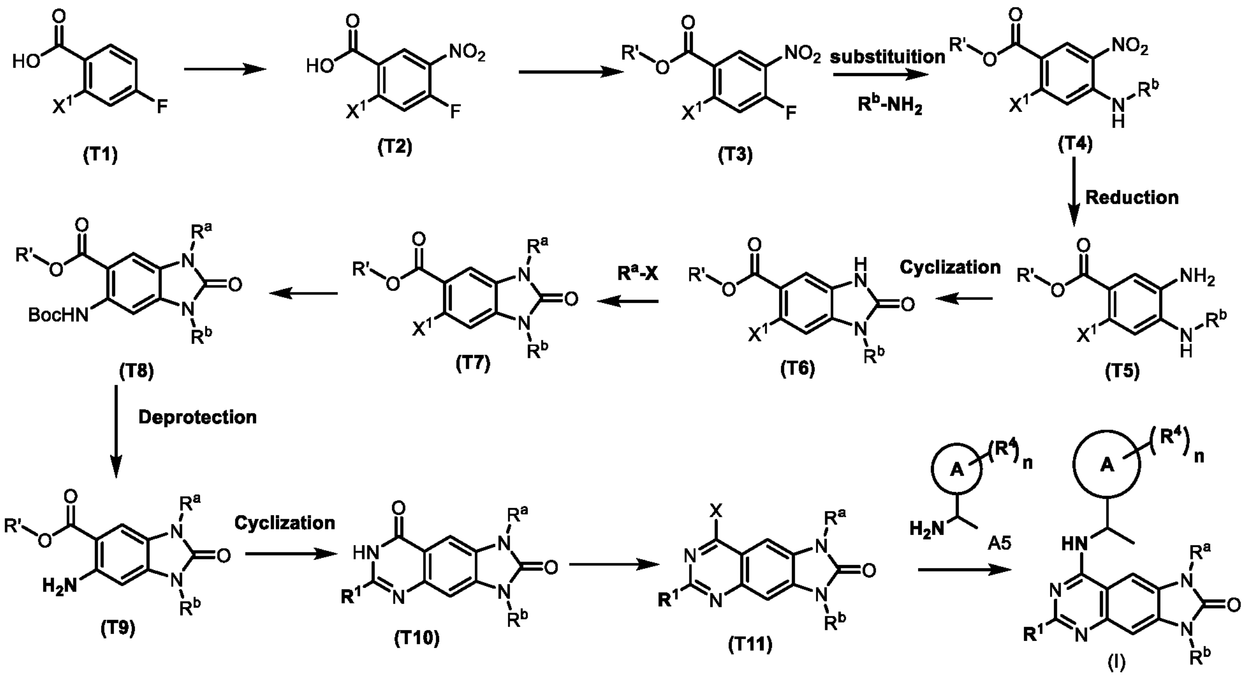


$R^1$ = Alkyl
R' = Alkyl
$R^b$= alkyl ,Cycloalkyl
$R^a$= Alkyl,Cycloalkyl
X, $X^1$= Halogen

SCHEME - T

Nitration of compound of formula (T1) with nitrating reagents such as, although not limited to fuming nitric acid, potassium nitrate, and the like in acids such as, although not limited to tin (IV) chloride, sulphuric acid, trifluoracetic acid, acetic acid and the like, anhydrides like acetic anhydride, trifluoracetic anhydride and the like, or mixture(s) thereof to provide compound of formula (T2).

Compound of formula (T2) undergoes esterification reaction to corresponding compound of formula (T3) using solvents such as methanol, ethanol, propanol, tert-butanol using acidic conditions like hydrochloric acid, sulfuric acid, thionyl chloride or the like or mixture(s) thereof.

Compound of formula (T3) derivative undergoes N-alkylation reaction to corresponding compound of formula (T4) using alkylamine and solvents such as methanol, ethanol, propanol, tert-butanol.

Compound of the formula (T4) on reduction of nitro group to corresponding anilinic compound of formula (T5). The reduction of nitro group was carried out using different reagents; although not limited, such reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof.

Cyclization of compound of the formula (T5) using CDI in polar aprotic solvents like DMF, DMSO, halogenated solvents like DCM, chloroform, ethereal solvents like THF, 1,4-dioxane, at room temperature or elevated temperatures provided compound of formula (T6).

Compound of formula (T6) undergoes N-alkylation using alkyl halides in presence of bases such as sodium hydride, potassium /sodium alkoxide $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (T7).

Compound of formula (T7) allowed to react with tert-butyl carbamate in the presence of catalyst such as (tris(dibenzylideneacetone)dipalladium(0), palladium(II) acetate, Bis(dibenzylideneacetone)2 Pd(0), rac 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as sodium carbonate, cesium carbonate, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (T8).

Compound of formula (T8) undergoes deprotection using acids like organic acids such as trifluoroacetic acid,

Methane sulfonic acid and like, mineral acids like hydrochloric acid, acetic acid (Aqueous or in etheral solvents), sulfuric acid and the like; using solvents like dichloromethane, dichloroethane, THF, 1,4-dioxane and like thereof to provide compound of formula (T9).

Compound of formula (T9) allowed to react with alkyl nitrile in presence of the acidic reagents such as methane sulfonic acid, sulfuric acid, hydrochloric acid or the like to obtain compound of formula (T10). The same transformation can be carried out using trialkyl orthoacetate in presence of ammonium acetate, in corresponding polar protic solvents like ethanol, methanol and thereof.

Compound of formula (T10) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (T11). Compound of formula (T11) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof.

The compounds of formula (I) was prepared by following the sequential transformations as depicted and described in **Scheme U:**

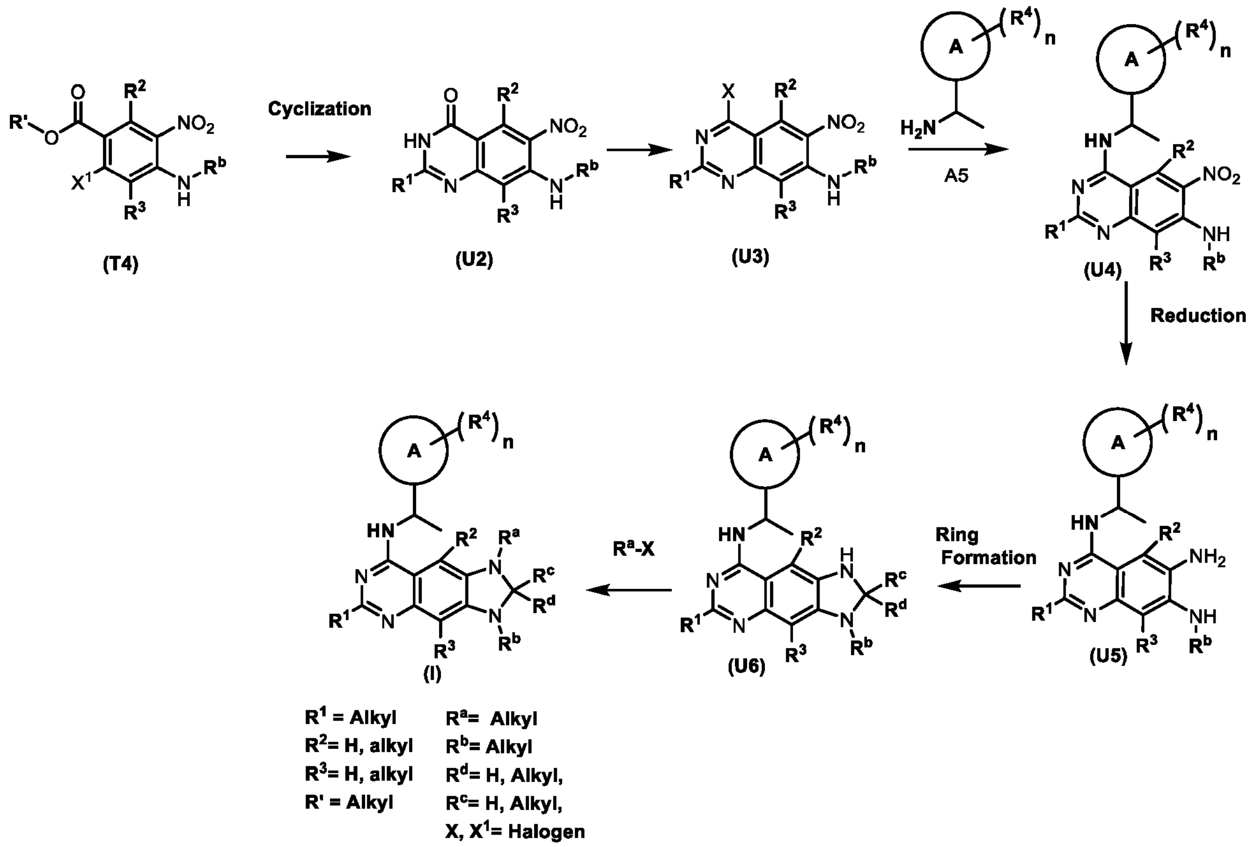


SCHEME - U

Compound of formula (T4) on reaction with acetamidine, formamidine and like; in polar aprotic solvents like DMF, DMSO and thereof at temperature elevated temperatures afforded compound of formula (U2). Compound of formula (U2) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (U3). Compound of formula (U3) allowed to react with compound of formula (A5) in presence of suitable coupling reagent to provide compound of formula (U4). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof.

Reduction of compound of the formula (U4) provide compound of formula (U5). The reduction of nitro group was carried out using different reagents; although not limited, such reducing agents include hydrogenation with palladium on

carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof.

Cyclization of compound of the formula (U5) using corresponding ketone in acid catalyst like pTsOH, Benzene sulphonic acid, sulfuric acid and acetic acid at room temperature or elevated temperatures provided compound of formula (U6).

Compound of formula (U6) undergoes N-alkylation using alkyl halides in presence of bases such as sodium hydride, potassium /sodium alkoxide $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (I). The compounds of formula (I) was prepared by following the sequential transformations as depicted and described in **Scheme V:**

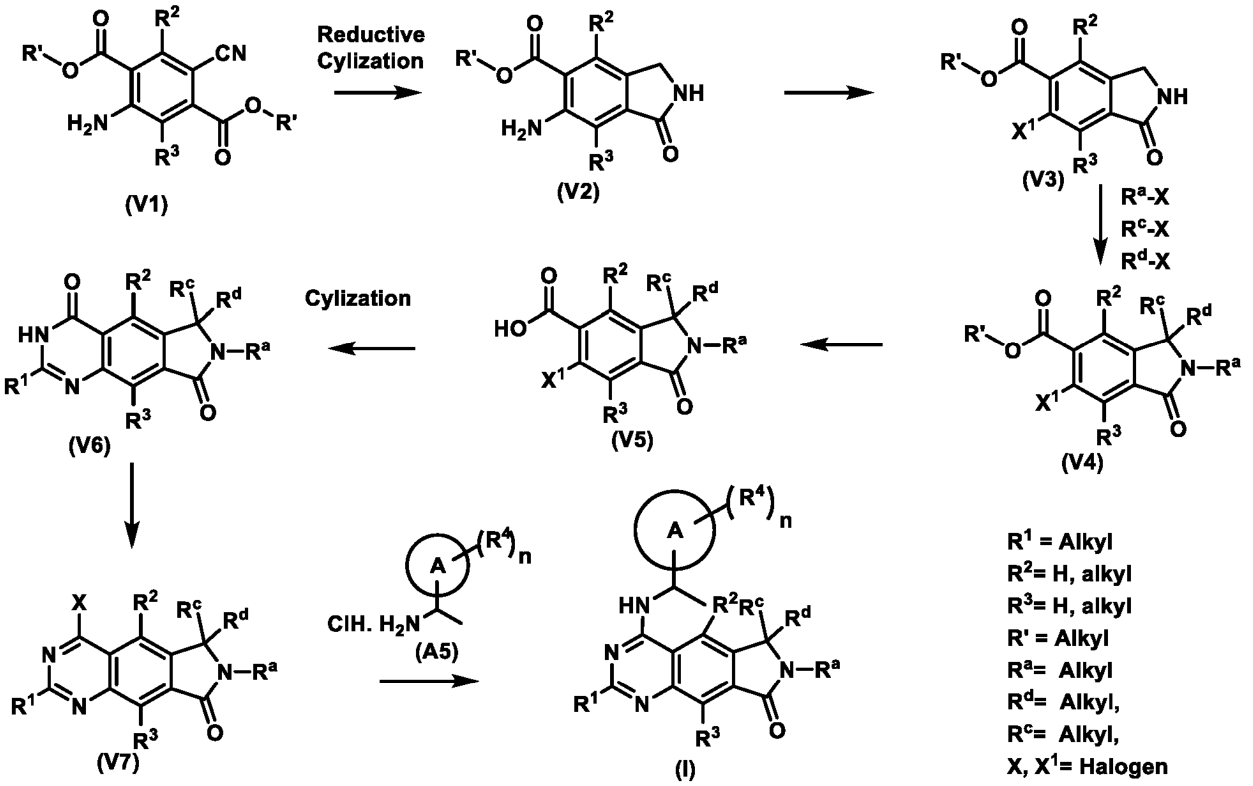


**SCHEME - V**

Compound of formula (V2) can be prepared from compound of formula (V1) via reductive cyclization reaction. This transformations can be carried out using reducing reagents such as contact hydrogenation in presence of Raney nickel, Pd/C, Pt/C and like; in etheral solvents such as 1,4-dioxane and like; optionally at room temperature or at elevated temperatures.

Compound of formula (V2) undergoes diazotization reaction using tert-butyl nitrite, isoamyl nitrite, sodium nitrite and like; followed by reaction with copper halides and like; can provide compound of formula (V3)

Compound of formula (V3) undergoes C-alkylation and N-alkylation simultaneously in presence of alkyl halides in presence of bases such as sodium hydride, potassium /sodium alkoxide $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (V4)

Compound of formula (V4) can be converted to compound of formula (I) by employing analogous 3 step protocol mentioned in scheme-P for conversion of compound of formula (P6) to compound of formula (I).

The compounds of formula (I) was prepared by following the sequential transformations as depicted and described in **Scheme W:**

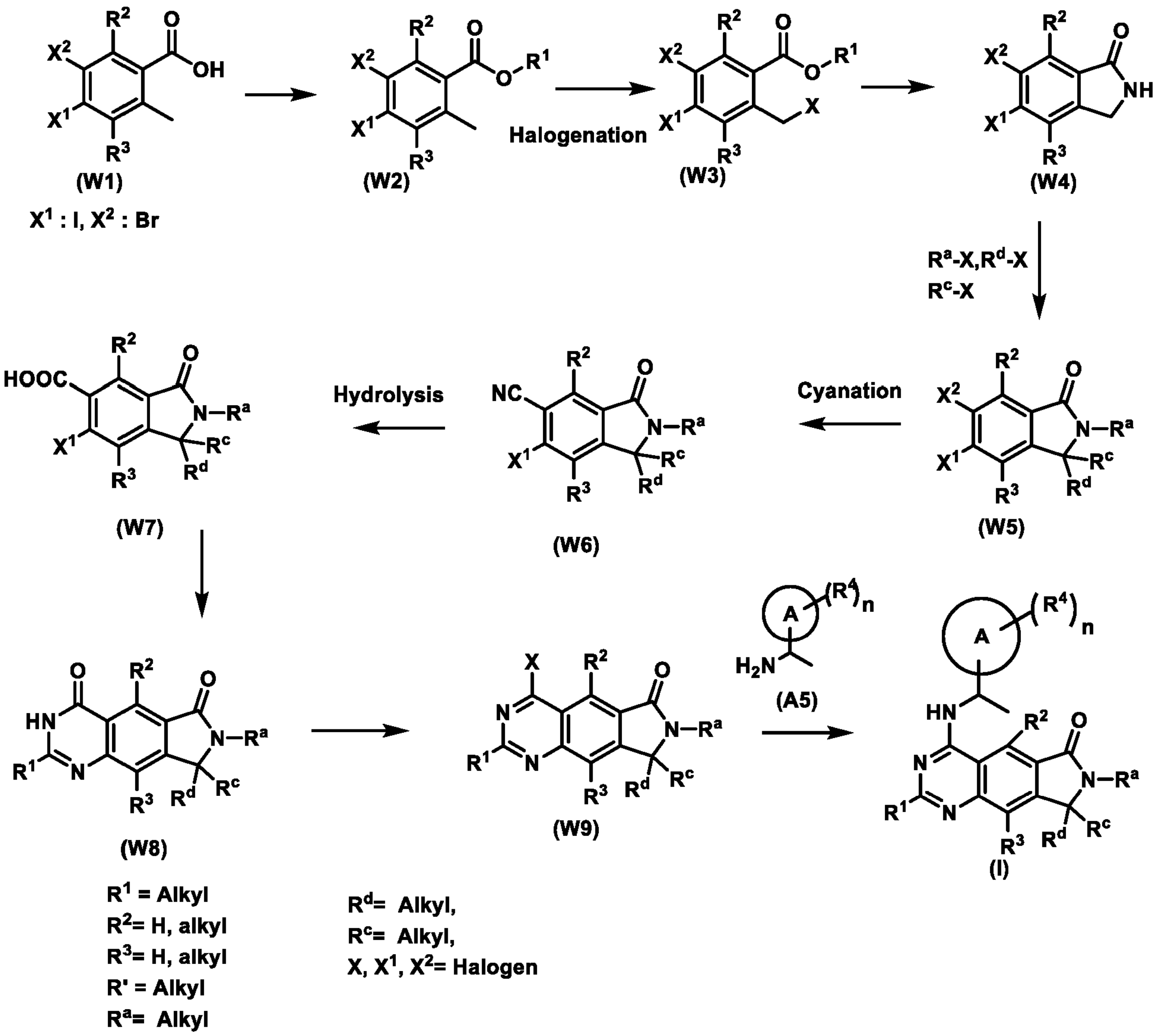


(W1) (W2) (W3) (W4) (W5) (W6) (W7) (W8) (W9) (A5) (I)

**SCHEME - W**

Compound of formula (W1) undergoes esterification reaction to provide compound of formula (W2). This transformation can be effected by reaction of alcohols such as methanol, ethanol and like; in presence of mineral acids like sulfuric acid, organic acids like methane sulfonic acid and like, or in presence of chloride reagents like thionyl chloride, oxalyl chloride and thereof. This transformation can also be effected by Mitsonobu reaction between acid (W1) and corresponding alcohols in presence of Triaryl phosphines and azocarboxylates such as DEAD, DIAD and like.

Compound of formula (W2) undergoes benzylic halogenation reaction using halogenating reagents like N-halo succinimide and thereof; in presence of initiators such as benzoyl peroxide, AIBN and like; in solvents such as carbon tetrachloride and thereof; at elevated temperature provide compound of formula (W3).

Compound of formula (W3) on reaction with ammonium hydroxide at room temperature or at elevated temperatures in alcoholic solvents like methanol, ethanol and like; undergoes cyclization reaction to provide compound of formula (W4).

Compound of formula (W4) undergoes C-alkylation and N-alkylation simultaneously in presence of alkyl halides in presence of bases such as sodium hydride, potassium /sodium alkoxide $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (W5).

Compound of formula (W5) on reaction with metal cyanides such as Copper(I) cyanide and like in polar aprotic solvent such as DMF and like at elevated temperatures afford compound of formula (W6).

Compound of formula (W6) undergoes hydrolysis reaction to furnish compound of formula (W7). This transformation can be carried out in presence of alkali hydroxides such as NaOH, LiOH and thereof, in solvents like methanol,

ethanol and thereof or using solvents like DMF, THF, 1,4-dioxane.

Compound of formula (W7) can be converted to compound of formula (I) by employing analogous 3 step protocol mentioned in scheme P for conversion of compound of formula (P6) to compound of formula (I). The compounds of formula (I) was prepared by following the sequential transformations as depicted and described in **Scheme-Y** herein below

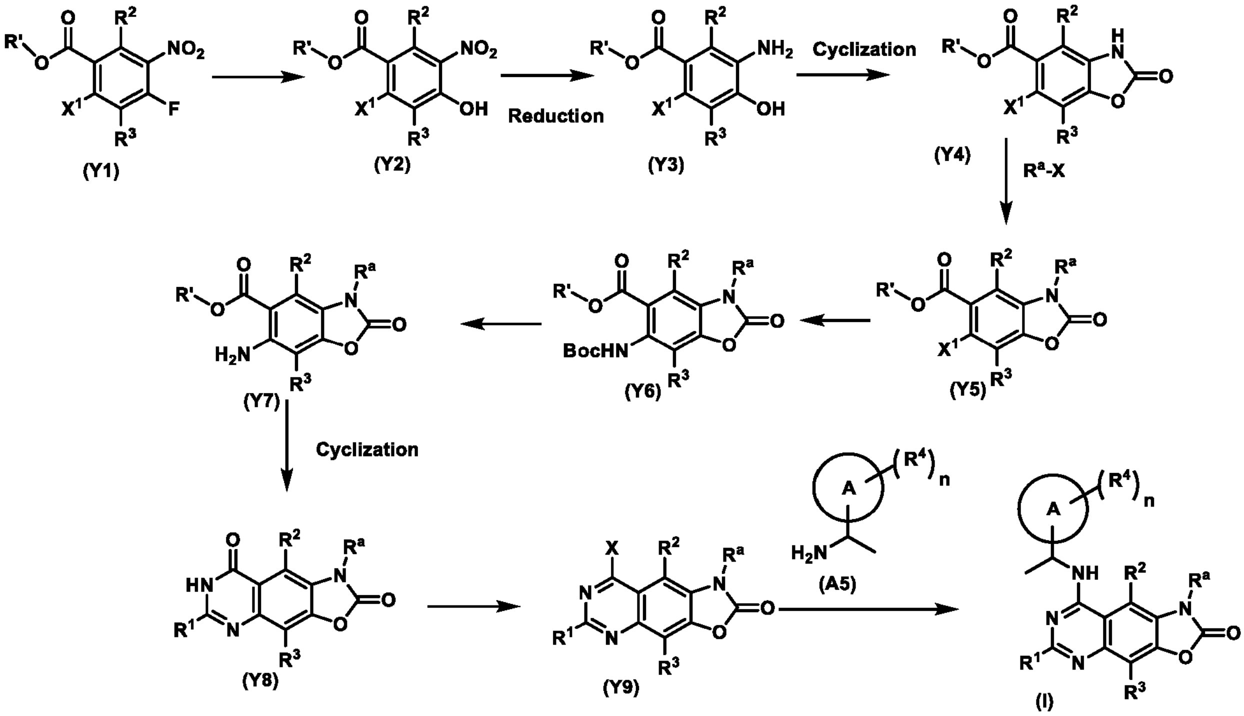


$R^1$ = Alkyl
$R^2$= H, alkyl
$R^3$= H, alkyl
R' = Alkyl
$R^a$= Alkyl
X, $X^1$= Halogen

**SCHEME - Y**

Compound of formula (Y1) undergoes reaction with bases such as $K_2CO_3$, $Na_2CO_3$, $Cs_2CO_3$ and like; in solvents such as DMF, DMSO and thereof, at elevated temperatures to afford compound of formula (Y2)

Compound of formula (Y2) undergoes reduction reaction to furnish compound of formula (Y3). Such reductions of nitro group were carried out using different reagents; although not limited, such reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof.

Cyclization of compound of the formula (Y3) using CDI in polar aprotic solvents like DMF, DMSO, halogenated solvents like DCM, chloroform, ethereal solvents like THF, 1,4-dioxane, at room temperature or elevated temperatures provided compound of formula (Y4).

Compound of formula (Y4) undergoes N-alkylation using alkyl halides in presence of bases such as NaH, Potassium/sodium alkoxides, $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (Y5). Compound of formula (Y5) allowed to react with tert-butyl carbamate in the presence of catalyst such as (tris(dibenzylideneacetone) dipalladium(0), palladium (II) acetate, Bis(dibenzylideneacetone)2 Pd(0), racemic 2,2'-Bis(diphenylphosphino)-1,1'-binaphthyl, 2,5 bis(tri-t-butylphosphine) palladium (0) and the like; in presence of ligands such as RuPhos, Xanthphos, Davephos, BINAP, or the like; using a suitable base such as $K_2CO_3$, $Na_2CO_3$, $Cs_2CO_3$, sodium tert-butoxide, potassium tert-butoxide, DIPEA, Potassium triphosphate and thereof; in a suitable solvent selected from THF, 1,4-dioxane, dimethoxyethane, DMF, DMA, toluene and the like to provide compound of formula (Y6).

Compound of formula (Y6) undergoes deprotection in acidic conditions using organic acids such as trifluoroacetic acid, Methane sulfonic acid and like, mineral acids like hydrochloric acid, acetic acid (aqueous or in etheral solvents), sulfuric acid and the like; using solvents like dichloromethane, dichloroethane, THF, 1,4-dioxane and like thereof

to provide compound of formula (Y7).

Compound of formula (Y7) allowed to react with alkylnitrile in presence of the acidic reagents such as methane sulfonic acid, sulfuric acid, hydrochloric acid or the like to obtain compound of formula (Y8). The same transformation can be carried out using trialkyl orthoacetate in presence of ammonium acetate, in corresponding polar protic solvents like ethanol, methanol and thereof.

Compound of formula (Y8) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like at room temperature or elevated temperatures to provide compound of formula (Y9).

Compound of formula (Y9) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and like, at elevated temperatures.

The compounds of formula (I) was prepared by following the sequential transformations as depicted and described in **Scheme-Z** herein below

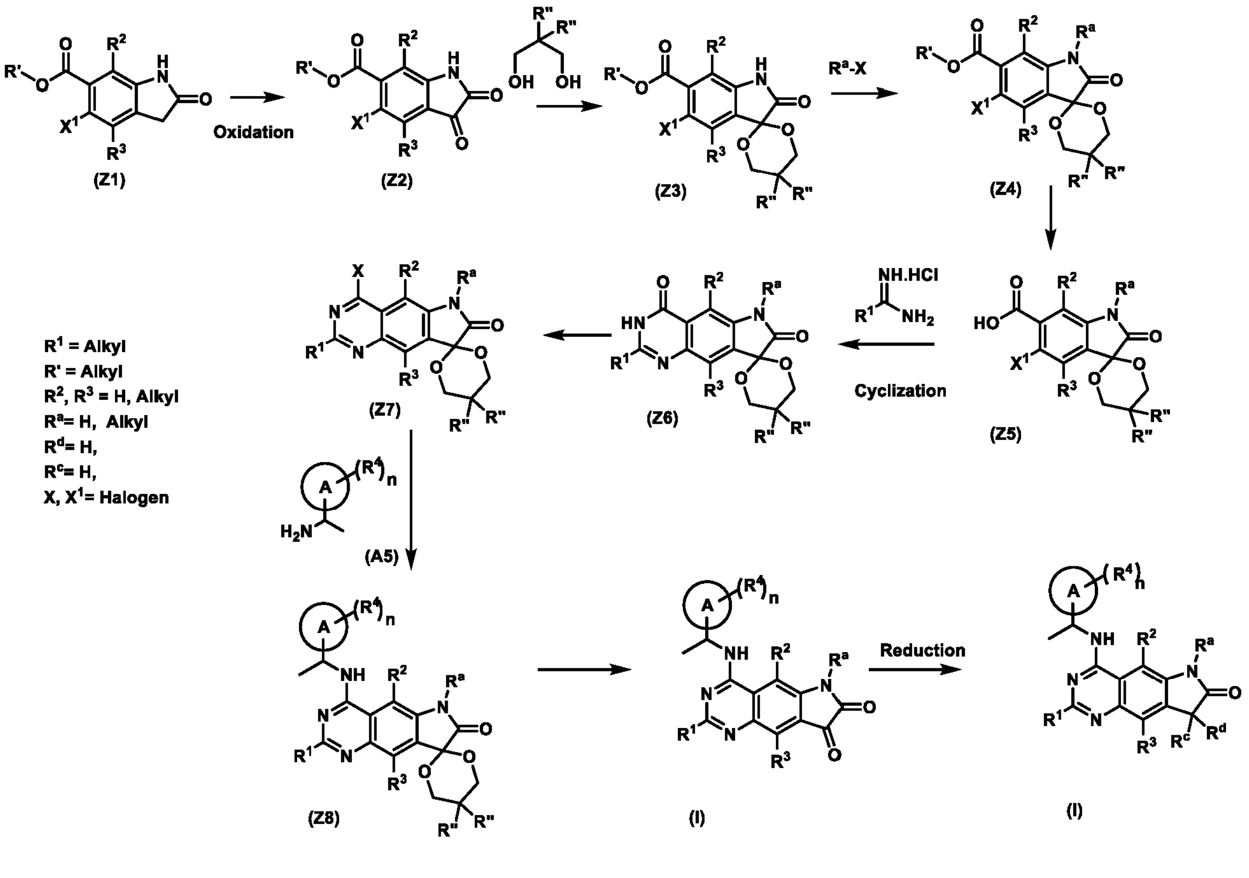


(Z1) (Z2) (Z3) (Z4) (Z5) (Z6) (Z7) (A5) (Z8) (I) (I)

SCHEME - Z

Compound of formula (Z1) undergoes oxidation reaction using oxidizing reagents such as tertiary butyl hydroperoxide, selenium dioxide, manganese dioxide and like; in presence of catalytic CuI , Cu(I) reagents and thereof; to provide compound of formula (Z2).

Compound of formula (Z3) can be obtained from compound of formula (Z2) by employing carbonyl protection reaction using diols such as 2,2-dimethylpropane-1.3-diol and like; in presence of mild acidic reagents such as PTSA and thereof; using hydrocarbon solvents like cyclohexane and like.

Compound of formula (Z3) on N-alkylation using alkyl halides and bases such as $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (Z4)

Compound of formula (Z4) undergoes hydrolysis of ester group in presence of alkali hydroxides such as NaOH,

LiOH and thereof, using solvents like methanol, ethanol and thereof or using solvents like THF, 1,4-dioxane and thereof; to afford compound of formula (Z5).

Compound of formula (Z5) can be converted to compound of formula (Z8) by employing analogous 3 step protocol mentioned in scheme P for conversion of compound of formula (P6) to compound of formula (I).

Compound of formula (Z8) on ketal deprotection in acidic medium provide compound of formula (I). This transformation was done by employing mineral acids such as HCl, $H_2SO_4$ and like; by employing solvents such as 1,4-dioxane, THF, acetic acid and like.

Further, Compound of formula (I) can be converted to compound of formula (I) by Wolff kishner reduction using hydroxyl amine hydrochloride reduction in alkaline medium. Such transformation can also be carried out by using Clemmensen reduction reaction in acidic medium.

The compounds of formula (I) was prepared by following the sequential transformations as depicted and described in **Scheme-AA** herein below

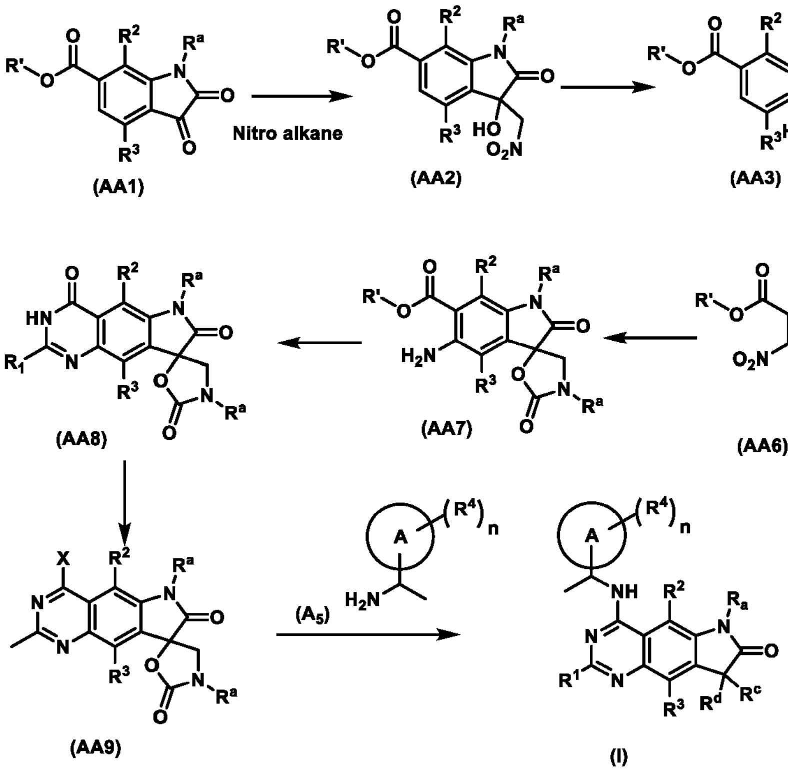


(AA1) (AA2) (AA3) (AA4) (AA5) (AA6) (AA7) (AA8) (AA9) (I)

**SCHEME AA1**

R' : Alkyl
$R^1$ = Alkyl
$R^2$= H, alkyl
$R^3$= H, alkyl
R', R" = Alkyl
$R^a$= H, Alkyl
$R^c$ and $R^d$ groups together with the carbon atom which they are attached forming heterocyclic ring
X, = Halogen

Compound of formula (AA1) on henry's reaction with nitroalkane in basic medium provided compound of formula (AA2). Such transformations can be carried out in presence of organic bases such as DIPEA, DABCO, and DBU and like, using nitroalkanes as solvent.

Compound of formula (AA2) on nitro reduction provided compound of formula (AA3). The reduction of nitro group was carried out using different reagents; although not limited, such reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and mixtures thereof.

Compound of formula (AA3) undergoes carbamate formation reaction mediated by reagents such as using CDI in polar aprotic solvents like DMF, DMSO, halogenated solvents like DCM, chloroform, ethereal solvents like THF, 1,4-dioxane, at room temperature or elevated temperatures provided compound of formula (AA4)

Compound of formula (AA4) undergoes N-alkylation using alkyl halides and bases such as $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1,4-dioxane and like, at room temperature or elevated temperatures provide compound of formula (AA5)

Compound of formula (AA5) can be transformed to compound of formula (I) in five steps analogous to protocol mentioned in scheme-S for conversion of compound of formula (S4) to compound of formula (I).

The compounds of formula (I) was prepared by following the sequential transformations as depicted and described in **Scheme-AB** herein below

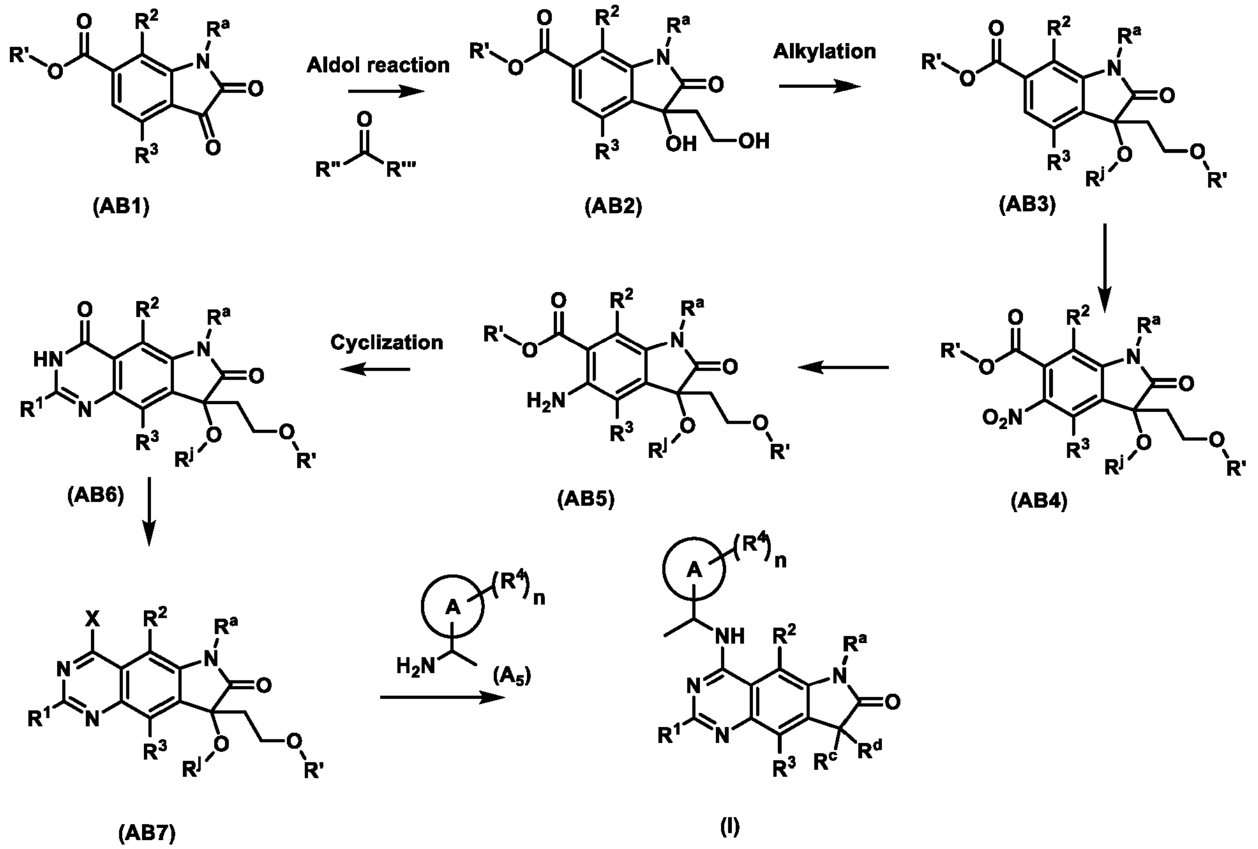


**R' = Alkyl**
**R"= Alkyl**
**R"' =H, Alkyl**
**$R^1$ = Alkyl**
**$R^2$= H, alkyl**
**$R^3$= H, alkyl**
**$R^a$= H, Alkyl**
**$R^j$= Alkyl**
**$R^c$,$R^d$ : Alkoxy, $-(CH_2)_n-OR'$**
**X, = Halogen**

Compound of formula (AB1) undergoes Aldol type reaction with aldehydes and ketones viz. acetaldehyde in presence of secondary amines such as diethyl amine, pyrrolidine and like, provide aldol intermediate which further on carbonyl reduction using $NaBH_4$ and like, in alcohol solvents such as methanol, ethanol and mixtures thereof provide diol compound of formula (AB2)

Compound of formula (AB2) undergoes O-alkylation reaction with alkyl halides in presence of bases such as NaH, sodium/potassium alkoxides, $K_2CO_3$, $Na_2CO_3$, $Cs_2CO_3$; organic bases like diisopropylethyl amine, DBU, DABCO and so on; in polar aprotic solvents like DMF, DMSO, acetone and like, etheral solvents such as THF, 1, 4-dioxane and like, at room temperature or elevated temperatures provide compound of formula (AB3)

Compound of formula (AB3) undergoes nitration reaction to provide compound of formula (AB4). This reaction was carried out in presence of nitrating reagents such as potassium nitrate, sodium nitrate nitric acid and like; in acidic solvents such as sulfuric acid and thereof.

Compound of formula (AB4) undergoes reduction reaction to provide compound of formula (AB5). These transformations can be carried out using reducing agents include hydrogenation with palladium on carbon, metal reductions like iron, tin or tin chloride and the like. These reactions are carried out in one or more solvents, e.g., ethers such as THF, 1,4-dioxane, and the like; alcohol such as methanol, ethanol and the like; under acidic conditions involving ammonium chloride, acetic acid, hydrochloric acid and the like mixtures thereof

Compound of formula (AB5) allowed to react with alkyl nitrile in presence of the acidic reagents such as methanesulfonic acid, sulfuric acid, hydrochloric acid, or the like to obtain compound of formula (AB6). The same transformation can be carried out using trialkyl orthoacetate in presence of ammonium acetate, in corresponding polar protic solvents like ethanol, methanol and thereof.

Compound of formula (AB6) allowed to react with phosporyl halides such as $POCl_3$ or $POBr_3$ optionally in solvents such as toluene, xylene, chlorobenzene or the like or the mixtures thereof, optionally using organic base such as triethylamine, diisopropylethylamine or the like to provide compound of formula (AB7).

Compound of formula (AB7) allowed to react with compound of formula (A5) in presence of suitable coupling reagent to provide compound of formula (I). The reaction can be carried out in presence of organic base such as diisopropylethylamine, triethylamine, DBU or the like, or using coupling reagents such as DCC, EDC, BOP, pyBOP, HBTU or the like; in etheral solvents such as THF, 1,4 dioxane and like or polar aprotic solvents like DMF, DMA, DMSO and thereof

The compounds of formula (I) was prepared by following the sequential transformations as depicted and described in **Scheme** - **AC** herein below

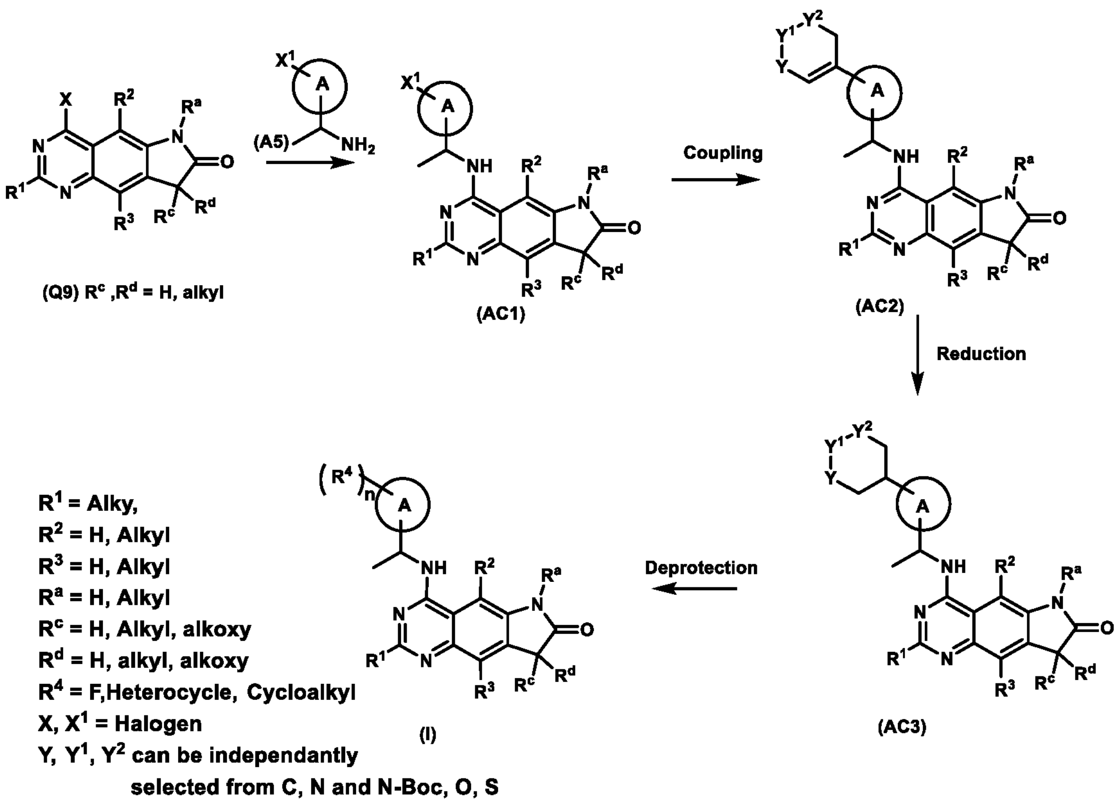


Compound of formula (Q9) undergoes coupling reaction with compound of formula (A5) to furnish compound of formula (I). The reaction can be carried out in presence of organic base such as diisopropylethylamine, triethylamine, DBU or the like, or using coupling reagents such as DCC, EDC, BOP, pyBOP, HBTU or the like; either neat reaction in base or in etheral solvents such as THF, 1,4 dioxane and like or polar aprotic solvents like DMF, DMA, DMSO and thereof

The compound of formula (AC1) was subjected to C-C coupling reaction e.g. suzuki coupling reaction with corresponding boronic acid or boronic ester to provide compound of formual (AC2). This reaction can mediated by a suitable catalyst such as, e.g., $Pd(PPh_3)_2Cl_2$, $Pd_2dba_3$, $Pd(PPh_3)_4$, $PdCl_2(dppf)$.DCM adduct or mixtures thereof; in the presence of suitable base, preferably inorganic bases such as $K_2CO_3$, $Na_2CO_3$, $Cs_2CO_3$, $NaO^tBu$, Potassium phosphate, or mixture thereof. Such reactions can be carried out in solvents like, e.g., ethers such as THF, 1,4-Dioxane and the like; hydrocarbons, e.g., toluene; amides such as DMF, DMA or mixtures thereof

Compound of formula (AC2) undergoes hydrogenation reaction in presence of catalyst such as $Pd(OH)_2$ on carbon, palladium on carbon, and the like; in one or more solvents e.g alcohol such as methanol, ethanol and the like; under acidic conditions involving ammonium chloride, acetic acid, hydrochloric acid and mixtures thereof, optionally in presence of water to provide compound of formula (AC3)

Compound of formula (AC3) undergoes deprotection reaction mediated by acids such as organic acids e,g trifluoroacetic acid, Methane sulfonic acid and the like, mineral acids e.g hydrochloric acid, acetic acid (Aqueous or in etheral solvents), sulfuric acid and the like; using solvents like dichloromethane, dichloroethane, THF, 1,4-dioxane and

mixtures thereof to provide compound of formula (I)

All intermediates used for the preparation of the compounds of the present invention, were prepared by approaches reported in the literature or by methods known to people skilled in the art of organic synthesis. Detailed experimental procedures for the synthesis of intermediates are given below.

The intermediates and the compounds of the present invention can be obtained in a pure form by any suitable method, for example, by distilling off the solvent in vacuum and/or re-crystallizing the residue obtained from a suitable solvent, such as pentane, diethyl ether, isopropyl ether, chloroform, dichloromethane, ethyl acetate, acetone or their combinations or subjecting it to one of the purification methods, such as column chromatography (e.g., flash chromatography) on a suitable support material such as alumina or silica gel using an eluent such as dichloromethane, ethyl acetate, hexane, methanol, acetone and/or their combinations. Preparative LC-MS method can also be used for the purification of the molecules described herein.

Unless otherwise stated, work-up includes distribution of the reaction mixture between the organic and aqueous phase indicated within parentheses, separation of the layers and drying of the organic layer over sodium sulphate, filtration, and evaporation of the solvent. Purification, unless otherwise mentioned, includes purification by silica gel chromatographic techniques, generally by using a mobile phase with suitable polarity, and purification using selective crystallization.

Salts of compound of formula (I) can be obtained by dissolving the compound in a suitable solvent, for example in a chlorinated hydrocarbon, such as methyl chloride or chloroform or a low molecular weight aliphatic alcohol, for example, ethanol or isopropanol, which is then treated with the desired acid or base as described in Berge S. M. et al., "Pharmaceutical Salts, a review article in Journal of Pharmaceutical sciences volume 66, page 1-19 (1977)" and in "Handbook of Pharmaceutical Salts - Properties, Selection, and Use," by P. Heinrich Stahland Camille G. Wermuth, Wiley-VCH (2002). Lists of suitable salts can also be found in Remington's Pharmaceutical Sciences, 18th ed., Mack Publishing Company, Easton, PA, 1990, p. 1445, and Journal of Pharmaceutical Science, 66, 2-19 (1977). For example, the salt can be of an alkali metal (e.g., sodium or potassium), alkaline earth metal (e.g., calcium), or ammonium.

The compound of the invention or a composition thereof can potentially be administered as a pharmaceutically acceptable acid-addition, base neutralized or addition salt, formed by reaction with an inorganic acid, such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base, such as sodium hydroxide or potassium hydroxide. The conversion to a salt is accomplished by treatment of the base compound with at least a stoichiometric amount of an appropriate acid. Typically, the free base is dissolved in an inert organic solvent such as diethyl ether, ethyl acetate, chloroform, ethanol, methanol, and the like, and the acid is added in a similar solvent. The mixture is maintained at a suitable temperature (e.g., between 0□C and 50□C). The resulting salt precipitates spontaneously or can be brought out of solution with a less polar solvent.

The stereoisomers of the compounds of formula (I) of the present invention can be prepared by stereospecific synthesis or resolution of racemic compound mixture by using an optically active amine, acid or complex forming agent, and separating the diastereomeric salt/complex by fractional crystallization or by column chromatography.

Prodrugs of the compounds of the invention can be prepared in situ during the isolation and purification of the compounds, or by separately reacting the purified compound with a suitable derivatizing agent. For example, hydroxy groups can be converted to ester groups via treatment with a carboxylic acid in the presence of a catalyst. Examples of cleavable alcohol prodrug moieties include substituted or unsubstituted, branched or unbranched lower alkyl ester moieties, e.g., ethyl esters, lower alkenyl esters, di-lower alkylamino lower-alkyl esters, e.g., dimethyl aminoethyl ester, acylamino lower alkyl esters, acyloxy lower alkyl esters (e.g., pivaloyloxymethyl ester), aryl esters, e.g., phenyl ester, aryl-lower alkyl esters, e.g., benzyl ester, optionally substituted, e.g., with methyl, halo, or methoxy substituents aryl and aryl-lower alkyl esters, amides, lower-alkyl amides, di-lower alkyl amides, and hydroxy amides.

The compounds of formula (I) of the present invention can exist in tautomeric forms, such as keto-enol tautomer. Such tautomeric forms are contemplated as an aspect of the present invention and such tautomer's may be in equilibrium or predominant in one of the forms.

The present invention also embraces isotopically-labelled compounds of the present invention which are identical to those recited herein, but for the fact that one or more atoms are replaced by an atom having an atomic mass or mass number different from the atomic mass or mass number usually found in abundance in nature. Examples of isotopes that can be incorporated into compounds of the invention include isotopes of hydrogen, carbon, nitrogen, oxygen, phosphorus, fluorine and chlorine and iodine, such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{36}Cl$, and $^{123}I$ respectively.

Thus the present invention further provides a pharmaceutical composition, containing the compounds of the general formula (I) as defined above, its tautomeric form, its stereoisomer, its polymorph, its solvate, its pharmaceutically acceptable salts in combination with pharmaceutically acceptable carriers, diluents, excipients, and the like.

The pharmaceutically acceptable carrier or excipient is preferably one that is chemically inert to the compound of

the invention and one that has no detrimental side effects or toxicity under the conditions of use. Such pharmaceutically acceptable carriers or excipients include saline (e.g., 0.9% saline), Cremophor EL® (which is a derivative of castor oil and ethylene oxide available from Sigma Chemical Co., St. Louis, MO) (e.g., 5% Cremophor EL/5% ethanol/90% saline, 10% Cremophor EL/90% saline, or 50% Cremophor EL/50% ethanol), propylene glycol (e.g., 40% propylene glycol/10% ethanol/50% water), polyethylene glycol (e.g., 40% PEG 400/60% saline), and alcohol (e.g., 40% ethanol/60% water). A preferred pharmaceutical carrier is polyethylene glycol, such as PEG 400, and particularly a composition comprising 40% PEG 400 and 60% water or saline. The choice of carrier will be determined in part by the particular compound chosen, as well as by the particular method used to administer the composition. Accordingly, there is a wide variety of suitable formulations of the pharmaceutical composition of the present invention.

Formulations for oral, aerosol, parenteral, subcutaneous, intravenous, intraarterial, intramuscular, intrathecal, intraperitoneal, rectal, and vaginal administration can be developed for the compound of formula (I), its tautomeric form, its stereoisomer, its polymorph, its solvate, and its pharmaceutically acceptable salt.

The pharmaceutical compositions can be administered parenterally, e.g., intravenously, intraarterially, subcutaneously, intradermally, intrathecally, or intramuscularly. Thus, the invention provides compositions for parenteral administration that comprise a solution of the compound of the invention dissolved or suspended in an acceptable carrier suitable for parenteral administration, including aqueous and non-aqueous, isotonic sterile injection solutions.

Overall, the requirements for effective pharmaceutical carriers for parenteral compositions are well known to those of ordinary skill in the art. See Pharmaceutics and Pharmacy Practice, J.B. Lippincott Company, Philadelphia, PA, Banker and Chalmers, eds., pages 238-250 (1982), and ASHP Handbook on Injectable Drugs, Toissel, 4th ed., pages 622-630 (1986). Such compositions include solutions containing anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol (for example in topical applications), or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride, with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils useful in parenteral formulations include petroleum, animal, vegetable, and synthetic oils. Specific examples of oils useful in such formulations include peanut, soybean, sesame, cottonseed, corn, olive, petrolatum, and mineral oil. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene polypropylene copolymers, (d) amphoteric detergents such as, for example, alkyl-β-aminopropionates, and 2-alkyl-imidazoline quaternary ammonium salts, and (e) mixtures thereof.

The parenteral formulations typically will contain from about 0.5% or less to about 25% or more by weight of a compound of the invention in solution. Preservatives and buffers can be used. In order to minimize or eliminate irritation at the site of injection, such compositions can contain one or more nonionic surfactants having a hydrophile-lipophile balance (HLB) of from about 12 to about 17. The quantity of surfactant in such formulations will typically range from about 5% to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets.

Topical formulations, including those that are useful for transdermal drug release, are well known to those of skill in the art and are suitable in the context of the present invention for application to skin.

Formulations suitable for oral administration can consist of (a) liquid solutions, such as an effective amount of a compound of the invention dissolved in diluents, such as water, saline, or orange juice; (b) capsules, sachets, tablets, lozenges, and troches, each containing a pre-determined amount of the compound of the invention, as solids or granules; (c) powders; (d) suspensions in an appropriate liquid; and (e) suitable emulsions. Liquid formulations can include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the

ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the compound ingredient in a flavor, usually sucrose and acacia or tragacanth, as well as pastilles comprising a compound of the invention in an inert base, such as gelatin and glycerin, or sucrose and acacia, emulsions, gels, and the like containing, in addition to the compound of the invention, such excipients as are known in the art.

A compound of the present invention, alone or in combination with other suitable components, can be made into aerosol formulations to be administered via inhalation. A compound of the invention is preferably supplied in finely divided form along with a surfactant and propellant. Typical percentages of the compounds of the invention can be about 0.01% to about 20% by weight, preferably about 1% to about 10% by weight. The surfactant must, of course, be nontoxic, and preferably soluble in the propellant. Representative of such surfactants are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides can be employed. The surfactant can constitute from about 0.1% to about 20% by weight of the composition, preferably from about 0.25% to about 5%. The balance of the composition is ordinarily propellant. A carrier can also be included as desired, e.g., lecithin, for intranasal delivery. These aerosol formulations can be placed into acceptable pressurized propellants, such as dichlorodifluoromethane, propane, nitrogen, and the like. They also can be formulated as pharmaceuticals for non-pressured preparations, such as in a nebulizer or an atomizer. Such spray formulations can be used to spray mucosa.

Additionally, the compound of the invention can be made into suppositories by mixing with a variety of bases, such as emulsifying bases or water-soluble bases. Formulations suitable for vaginal administration can be presented as pessaries, tampons, creams, gels, pastes, foams, or spray formulas containing, in addition to the compound ingredient, such carriers as are known in the art to be appropriate.

The concentration of the compound in the pharmaceutical formulations can vary, e.g., from less than about 1% to about 10%, to as much as about 20% to about 50% or more by weight, and can be selected primarily by fluid volumes, and viscosities, in accordance with the particular mode of administration selected.

For example, a typical pharmaceutical composition for intravenous infusion could be made up to contain 250 ml of sterile Ringer's solution, and 100 mg of at least one compound of the invention. Actual methods for preparing parenterally administrable compounds of the invention will be known or apparent to those skilled in the art and are described in more detail in, for example, Remington's Pharmaceutical Science (17th ed., Mack Publishing Company, Easton, PA, 1985).

It will be appreciated by one of ordinary skill in the art that, in addition to the aforesaid described pharmaceutical compositions, the compound of the invention can be formulated as inclusion complexes, such as cyclodextrin inclusion complexes, or liposomes. Liposomes can serve to target a compound of the invention to a particular tissue, such as lymphoid tissue or cancerous hepatic cells. Liposomes can also be used to increase the half-life of a compound of the invention. Many methods are available for preparing liposomes, as described in, for example, Szoka et al., Ann. Rev. Biophys. Bioeng., 9, 467 (1980) and U.S. Patents no. 4235871, 4501728, 4837028, and 5019369.

The compounds of the invention can be administered in a dose sufficient to treat the disease, condition or disorder. Such doses are known in the art (see, for example, the Physicians' Desk Reference (2004)). The compounds can be administered using techniques such as those described in, for example, Wasserman et al., Cancer, 36, pp. 1258-1268 (1975) and Physicians' Desk Reference, 58th ed., Thomson PDR (2004).

Suitable doses and dosage regimens can be determined by conventional range-finding techniques known to those of ordinary skill in the art. Generally, treatment is initiated with smaller dosages that are less than the optimum dose of the compound of the present invention. Thereafter, the dosage is increased by small increments until the optimum effect under the circumstances is reached. The present method can involve the administration of about 0.1 μg to about 50 mg of at least one compound of the invention per kg body weight of the individual. For a 70 kg patient, dosages of from about 10 μg to about 200 mg of the compound of the invention would be more commonly used, depending on a patient's physiological response.

By way of example and not intending to limit the invention, the dose of the pharmaceutically active agent(s) described herein for methods of treating a disease or condition as described above can be about 0.001 to about 1 mg/kg body weight of the subject per day, for example, about 0.001 mg, 0.002 mg, 0.005 mg, 0.010 mg, 0.015 mg, 0.020 mg, 0.025 mg, 0.050 mg, 0.075 mg, 0.1 mg, 0.15 mg, 0.2 mg, 0.25 mg, 0.5 mg, 0.75 mg, or 1 mg/kg body weight per day. The dose of the pharmaceutically active agent(s) described herein for the described methods can be about 1 to about 1000 mg/kg body weight of the subject being treated per day, for example, about 1 mg, 2 mg, 5 mg, 10 mg, 15 mg, 20 mg, 25 mg, 50 mg, 75 mg, 100 mg, 150 mg, 200 mg, 250 mg, 500 mg, 750 mg, or 1000 mg/kg body weight per day.

The terms "treat," "ameliorate," and "inhibit," as well as words stemming therefrom, as used herein, do not necessarily imply 100% or complete treatment, amelioration, or inhibition. Rather, there are varying degrees of treatment,

amelioration, and inhibition of which one of ordinary skill in the art recognizes as having a potential benefit or therapeutic effect. In this respect, the disclosed methods can provide any amount of any level of treatment, amelioration, or inhibition of the disorder in a mammal. For example, a disorder, including symptoms or conditions thereof, may be reduced by, for example, 100%, 90%, 80%, 70%, 60%, 50%, 40%, 30%, 20%, or 10%. Furthermore, the treatment, amelioration, or inhibition provided by the inventive method can include treatment, amelioration, or inhibition of one or more conditions or symptoms of the disorder, e.g., cancer. Also, for purposes herein, "treatment," "amelioration," or "inhibition" can encompass delaying the onset of the disorder, or a symptom or condition thereof.

In accordance with the invention, the term subject includes an "animal" which in turn includes a mammal such as, without limitation, the order Rodentia, such as mice, and the order Lagomorpha, such as rabbits. In one aspect, the mammals are from the order Carnivora, including Felines (cats) and Canines (dogs). In another aspect, the mammals are from the order Artiodactyla, including Bovines (cows) and Swine (pigs) or of the order Perssodactyla, including Equines (horses). In a further aspect, the mammals are of the order Primates, Ceboids, or Simoids (monkeys) or of the order Anthropoids (humans and apes). In yet another aspect, the mammal is human.

The present invention provides a pharmaceutical composition, containing the compound of the general formula (I) as defined herein, its tautomeric form, and its stereoisomer, its polymorph, its solvate, or its pharmaceutically acceptable salt in combination with the usual pharmaceutically employed carriers, diluents, and the like are useful for the treatment of a disease or disorder mediated through SOS1.

The present invention provides a pharmaceutical composition, containing the compound of the general formula (I) as defined herein, its tautomeric form, its stereoisomer, its polymorph, its solvate, or its pharmaceutically acceptable salt in combination with the usual pharmaceutically employed carriers, diluents, and the like are useful for the treatment of a disease or disorder such as cancer, infectious disease or disorder, or RASopathy disease or disorder.

The present invention provides the compound of formula I, its tautomeric form, its stereoisomer, its pharmaceutically acceptable salt, its polymorph, its solvate, its combination with suitable medicament, or its pharmaceutical composition for treating disease characterized by excessive or abnormal cell proliferation such as cancer.

The present invention provides the compound of formula I, its tautomeric form, its stereoisomer, its pharmaceutically acceptable salt, its polymorph, its solvate, its combination with suitable medicament, or its pharmaceutical composition for treating diseases like pancreatic cancer, lung cancer, colorectal cancer, class 3 BRAF-mutant cancers, hematological cancer, cholangiocarcinoma, multiple myeloma, melanoma, uterine cancer, endometrial cancer, thyroid cancer, acute myeloid leukaemia, bladder cancer, urothelial cancer, gastric cancer, cervical cancer, head and neck squamous cell carcinoma, diffuse large B cell lymphoma, esophageal cancer, chronic lymphocytic leukaemia, hepatocellular cancer, breast cancer, ovarian cancer, prostate cancer, glioblastoma, renal cancer, Pure mucosal neuroma syndrome, Fibrous Epulis, and sarcomas.

Compounds belonging to this invention can be used for the treatment of the various cancers mentioned below which harbor hyperactive or aberrantly activated signaling pathways involving SOS1 proteins.

Compounds belonging to this invention can be used for the treatment of the various cancers mentioned below which harbor hyperactive or aberrantly activated signaling pathways involving RAS and or SOS1 proteins.

The compounds, its tautomeric form, its stereoisomer, its pharmaceutically acceptable salt, its polymorph, or its solvate its combination with suitable medicament, its pharmaceutical composition thereof as described hereinbelow can be suitable for treating diseases characterized by excessive or abnormal cell proliferation such as cancer.

The cancer, tumor, and other proliferative diseases can be treated with the compounds of the present invention is but not limited to:

1. Cancers of the head and neck, e.g. cancers of nasal cavity, paranasal sinuses, nasopharynx, oral cavity (including lip, gum, alveolar ridge, retromolar trigone, floor of mouth, tongue, hard palate, buccal mucosa), oropharynx (including base of tongue, tonsil, tonsillar pillar, soft palate, tonsillar fossa, pharyngeal wall), middle ear, larynx (including supraglottis, glottis, subglottis, vocal cords, hypopharynx, salivary glands (including minor salivary glands).
2. Cancers of the lung, e.g. non-small cell lung cancer (NSCLC) (squamous cell carcinoma, spindle cell carcinoma, adenocarcinoma, large cell carcinoma, clear cell carcinoma, bronchioalveolar), small cell lung cancer (SCLC) (oat cell cancer, intermediate cell cancer, combined oat cell cancer), class 3 BRAF-mutant lung cancer.
3. Neoplasms of the mediastinum, e.g. neurogenic tumors (including neurofibroma, neurilemoma, malignant schwannoma, neurosarcoma, ganglioneuroblastoma, ganglioneuroma, neuroblastoma, pheochromocytoma, paraganglioma), germ cell tumors (including seminoma, teratoma, non-seminoma), thymic tumors (including thymoma, thymolipoma, thymic carcinoma, thymic carcinoid), mesenchymal tumors (including fibroma, fibrosarcoma, lipoma, liposarcoma, myxoma, mesothelioma, leiomyoma, leiomyosarcoma, rhabdomyosarcoma, xanthogranuloma, mesenchymoma, hemangioma, hemangioendothelioma, hemangiopericytoma, lymphangioma, lymphangiopericytoma, lymphangiomyoma).
4. Cancers of the gastrointestinal (GI) tract, e.g. cancers of the esophagus, stomach (gastric cancer), esophagiogastric adenocarcinoma pancreas, liver and biliary tree (including hepatocellular carcinoma (HCC), e.g. childhood

HCC, fibrolamellar HCC, combined HCC, spindle cell HCC, clear cell HCC, giant cell HCC, carcinosarcoma HCC, sclerosing HCC; hepatoblastoma; cholangiocarcinoma.

5. Cholangiocellular carcinoma; hepatic cystadenocarcinoma; angiosarcoma, hemangioendothelioma, leiomyosarcoma, malignant schwannoma, fibrosarcoma, Klatskin tumor), gall bladder, extrahepatic bile ducts, small intestine (including duodenum, jejunum, ileum), large intestine (including cecum, colon, rectum, anus; colorectal cancer, gastrointestinal stroma tumor (GIST)), genitourinary system (including kidney, e.g. renal pelvis, renal cell carcinoma (RCC), nephroblastoma (Wilms' tumor), hypernephroma, Grawitz tumor; ureter; urinary bladder, e.g. urachal cancer, urothelial cancer; urethra, e.g. distal, bulbomembranous, prostatic; prostate (androgen dependent, androgen independent, castration resistant, hormone independent, hormone refractory), penis).

6. Cancers of the testis, e.g. seminomas, non-seminomas.

7. Gynecologic cancers e.g. cancers of the ovary, fallopian tube, peritoneum, cervix, vulva, vagina, uterine body (including endometrium, fundus).

8. Cancers of the breast, e.g. mammary carcinoma (infiltrating ductal, colloid, lobular invasive, tubular, adenocystic, papillary, medullary, mucinous), hormone receptor positive breast cancer (estrogen receptor positive breast cancer, progesterone receptor positive breast cancer), Her2 positive breast cancer, triple negative breast cancer, Paget's disease of the breast.

9. Cancers of the endocrine system, e.g. cancers of the endocrine glands, thyroid gland (thyroid carcinomas/tumors; papillary, follicular, anaplastic, medullary), parathyroid gland (parathyroid carcinoma/tumor), adrenal cortex (adrenal cortical carcinoma/tumors), pituitary gland (including prolactinoma, craniopharyngioma), thymus, adrenal glands, pineal gland, carotid body, islet cell tumors, paraganglion, pancreatic endocrine tumors (PET; non-functional PET, PPoma, gastrinoma, insulinoma, VIPoma, glucagonoma, somatostatinoma, GRFoma, ACTHoma), carcinoid tumors.

10. Sarcomas of the soft tissues, e.g. fibrosarcoma, fibrous histiocytoma, liposarcoma, leiomyosarcoma, rhabdomyosarcoma, angiosarcoma, lymphangiosarcoma, Kaposi's sarcoma, glomus tumor, hemangiopericytoma, synovial sarcoma, giant cell tumor of tendon sheath, solitary fibrous tumor of pleura and peritoneum, diffuse mesothelioma, malignant peripheral nerve sheath tumor (MPNST), granular cell tumor, clear cell sarcoma, melanocytic schwannoma, plexosarcoma, neuroblastoma, ganglioneuroblastoma, neuroepithelioma, extraskeletal Ewing's sarcoma, paraganglioma, extraskeletal chondrosarcoma, extraskeletal osteosarcoma, mesenchymoma, alveolar soft part sarcoma, epithelioid sarcoma, extrarenal rhabdoid tumor, desmoplastic small cell tumor.

11. Sarcomas of the bone, e.g. myeloma, reticulum cell sarcoma, chondrosarcoma (including central, peripheral, clear cell, mesenchymal chondrosarcoma), osteosarcoma (including parosteal, periosteal, high-grade surface, small cell, radiation-induced osteosarcoma, Paget's sarcoma), Ewing's tumor, malignant giant cell tumor, adamantinoma, (fibrous) histiocytoma, fibrosarcoma, chordoma, small round cell sarcoma, hemangioendothelioma, hemangiopericytoma, osteochondroma, osteoid osteoma, osteoblastoma, eosinophilic granuloma, chondroblastoma;

12. Mesothelioma, e.g. pleural mesothelioma, peritoneal mesothelioma.

13. Cancers of the skin, e.g. basal cell carcinoma, squamous cell carcinoma, Merkel's cell carcinoma, melanoma (including cutaneous, superficial spreading, lentigo maligna, acral lentiginous, nodular, intraocular melanoma), actinic keratosis, eyelid cancer, class 3 BRAF-mutant melanoma.

14. Neoplasms of the central nervous system and brain, e.g. astrocytoma (cerebral, cerebellar, diffuse, fibrillary, anaplastic, pilocytic, protoplasmic, gemistocytary), glioblastoma, gliomas, oligodendrogliomas, oligoastrocytomas, ependymomas, ependymoblastomas, choroid plexus tumors, medulloblastomas, meningiomas, schwannomas, hemangioblastomas, hemangiomas, hemangiopericytomas, neuromas, ganglioneuromas, neuroblastomas, retinoblastomas, neurinomas (e.g. acoustic), spinal axis tumors.

15. Lymphomas and Leukemias, e.g. B-cell non-Hodgkin lymphomas (NHL) (including small lymphocytic lymphoma (SLL), lymphoplasmacytoid lymphoma (LPL), mantle cell lymphoma (MCL), follicular lymphoma (FL), diffuse large cell lymphoma (DLCL), Burkitt's lymphoma (BL)), T-cell non-Hodgkin lymphomas (including anaplastic large cell lymphoma (ALCL), adult T-cell leukemia/lymphoma (ATLL), cutaneous T-cell lymphoma (CTCL), peripheral T-cell lymphoma (PTCL)), lymphoblastic T-cell lymphoma (T-LBL), adult T-cell lymphoma, lymphoblastic B-cell lymphoma (B-LBL), immunocytoma, chronic B-cell lymphocytic leukemia (B-CLL), chronic T-cell lymphocytic leukemia (T-CLL) B-cell small lymphocytic lymphoma (B-SLL), cutaneous T-cell lymphoma (CTLC), primary central nervous system lymphoma (PCNSL), immunoblastoma, Hodgkin's disease (HD) (including nodular lymphocyte predominance HD (NLPHD), nodular sclerosis HD (NSHD), mixed-cellularity HD (MCHD), lymphocyte-rich classic HD, lymphocyte-depleted HD (LDHD)), large granular lymphocyte leukemia (LGL), chronic myelogenous leukemia (CML), acute myelogenous/myeloid leukemia (AML), acute lymphatic/lymphoblastic leukemia (ALL), acute promyelocytic leukemia (APL), chronic lymphocytic/lymphatic leukemia (CLL), prolymphocytic leukemia (PLL), hairy cell leukemia, chronic myelogenous/myeloid leukemia (CML), myeloma, plasmacytoma, multiple myeloma (MM), plasmacytoma, myelodysplastic syndromes (MDS), chronic myelomonocytic leukemia (CMML).

16. Cancers of unknown primary site (CUP).

17. Epithelial cancers, e.g. squamous cell carcinoma (SCC) (carcinoma in situ, superficially invasive, verrucous

carcinoma, pseudosarcoma, anaplastic, transitional cell, lymphoepithelial), adenocarcinoma (AC) (well-differentiated, mucinous, papillary, pleomorphic giant cell, ductal, small cell, signet-ring cell, spindle cell, clear cell, oat cell, colloid, adenosquamous, mucoepidermoid, adenoid cystic), mucinous cystadenocarcinoma, acinar cell carcinoma, large cell carcinoma, small cell carcinoma, neuroendocrine tumors (small cell carcinoma, paraganglioma, carcinoid), oncocytic carcinoma. and

18. Nonepithelial cancers, e.g. sarcomas (fibrosarcoma, chondrosarcoma, rhabdomyosarcoma, leiomyosarcoma, hemangiosarcoma, giant cell sarcoma, lymphosarcoma, fibrous histiocytoma, liposarcoma, angiosarcoma, lymphangiosarcoma, neurofibrosarcoma), lymphoma, melanoma, germ cell tumors, hematological neoplasms, mixed and undifferentiated carcinomas.

All cancers mentioned above which are characterized by their specific location or origin in the body are meant to include both the primary tumors and the metastatic tumors derived therefrom.

All cancers mentioned above may be further differentiated by their histopathological classification.

The compound of formula I, its tautomeric form, its stereoisomer, its pharmaceutically acceptable salt, its polymorph, its solvate, its combination with suitable medicament, or its pharmaceutical composition as described herein above can be suitable for treating diseases such as Neurofibromatosis type 1 (NF1), Noonan Syndrome with Multiple Lentigines (NSML), Noonan-like/multiple giant cell lesion syndrome, Hereditary Gingival Fibromatosis (HGF), Capillary Malformation-Arteriovenous Malformation Syndrome (CM-AVM), Legius Syndrome, Acute Staphylococcus aureus infection (Pediatric Patients), Pure mucosal neuroma syndrome, Fibrous Epulis, Acute Respiratory Distress syndrome/Acute Lung injury and Sepsis, Costello Syndrome (CS), and Cardio-Facio-cutaneous Syndrome (CFC Syndrome).

The compound of formula I, its tautomeric form, its stereoisomer, its pharmaceutically acceptable salt, its polymorph, its solvate, its combination with suitable medicament, or its pharmaceutical composition as described hereinabove may be used in therapeutic regimens in the context of first line, second line, or any further line of treatments.

The compound of formula I, its tautomeric form, its stereoisomer, its pharmaceutically acceptable salt, its polymorph, its solvate, its combination with suitable medicament, or its pharmaceutical composition as described hereinabove may be used for the prevention, short-term or long term treatment of the above-mentioned diseases, optionally also in combination with radiotherapy and/or surgery.

The compound of formula I, its tautomeric form, its stereoisomer, its pharmaceutically acceptable salt, its polymorph, its solvate, belonging to the present invention can be combined with other agents such as radiation, chemotherapeutic agents and/or targeted agents in multiple cancers and their subtypes as mentioned above. The agents that can be used for combination therapy include targeted agents such as inhibitors of RTKs, cyclin-dependent kinase (CDK) inhibitors, Ser-Thr kinase inhibitors, non-receptor tyrosine kinase inhibitors, inhibitors of epigenetic mechanism such as histone methyl transferases (HMTs), DNA methyl transferases (DNMTs), protein arginine methyl transferases (PRMTs), RAS inhibitors, KRAS inhibitors, MEK inhibitors, ERK1/2 inhibitors, Focal Adhesion Kinase (FAK) inhibitors, PI3K inhibitors, AKT inhibitors, and mTOR inhibitors.

The following examples are provided to further illustrate the present invention and should not be constructed in any way to limit the scope of the present invention.

All $^1$HNHR spectra were determined in the solvent indicated and chemical shifts are reported in $\delta$ units downfield from the internal standard tetramethylsilane (TMS) and interproton coupling constants are reported in Hertz (Hz).

Some of the representative examples of the present invention were prepared by following one or more reaction schemes as described above.

The invention is further illustrated by the following examples which are provided merely to be exemplary of the invention and do not limit the scope of the invention. The examples set forth below demonstrate the synthetic procedures for the preparation of the relative compounds. Certain modifications will be apparent to those skilled in the art and are intended to be included within the scope of the invention.

The notation "or1" "or2" and "or3" in structural formulae denote that chiral center is ascertained to be either R or S, herein absolute configuration is not determined.

Abbreviations:

The following abbreviations may be used herein:

ACN = Acetonitrile
AcOH = Acetic acid
AIBN = Azobisisobutyronitrile
BINAP = 2,2'-bis(diphenylphosphino)-1,1'-binaphthyl
BOP = Benzotriazol-1-yloxytris(dimethylamino)phosphonium hexafluorophosphate
CAN = Ceric ammonium nitrate
$CCl_4$ = Carbon tetrachloride
$CDCl_3$ = Deuterated chloroform

CDI = 1,1'-Carbonyldiimidazole
DABCO = 1,4-diazabicyclo[2.2. 2]octane
DAST = Diethylaminosulfur trifluoride
dba = Benzylideneacetone
DBU = 1,8-Diazabicyclo[5.4.0]undec-7-ene
DCC = Dicyclohexyl carbodimide
DCE = Dichlormethane
DCM = Dichloromethane
DCM = Dichloromethane
DDQ = 2,3-Dichloro-5,6-dicyano-1,4-benzoquinone
DEA = Diethyl amine
DEAD = Diethyl azodicarboxylate
DIAD = Diisopropyl azodicarboxylate
DIPEA = Diisopropylethylamine
DMA = Dimethyl Acetamide
DMF = N,N-Dimethylformamide
DMS = Dimethyl sulfide
DMSO = Dimethyl sulfoxide
DMSO-$d_6$ = Deuterated dimethyl sulfoxide
EDC = 1-Ethyl-3-(3-dimethylaminopropyl)carbodiimide
HBTU = 2-(1H-benzotriazol-1-yl)-1,1,3,3-tetramethyluronium hexafluorophosphate
HEX = n-Hexane
HOBt = Hydroxybenzotriazole
IPA = 2-Propanol
LCMS = Liquid chromatography mass spectrometry
LiOH = Lithium hydroxide
Me = Methyl
MTBE = Methyl tert-butyl ether
NaH = Sodium hydride
NBS = N-Bromo Succinimide
NCS = N-Chloro Succinimide
NIS = N-Iodo succinimide
NMO = N methyl morpholine n-oxide
NMP = N-methyl-2-pyrolidinone
NMR = Nuclear magnetic resonance
Pd/C = Palladium on carbon
$POCl_3$= Phosphorus oxychloride
Pt/C = Platinum on carbon
PyBop = Benzotriazol-1-yl-oxytripyrrolidinophosphonium hexafluorophosphate
pTsOH = p-Toluene sulphonic acid
RT = room temperature
Rt = retention time
TEA = Triethylamine
THF = Tetrahydrofuran
TPP = Triphenyl phosphene
TBAF = Tetra butyl ammonium fluoride

Example 1: Preparation of (R)-3-(1-aminoethyl)-5-(trifluoromethyl) aniline

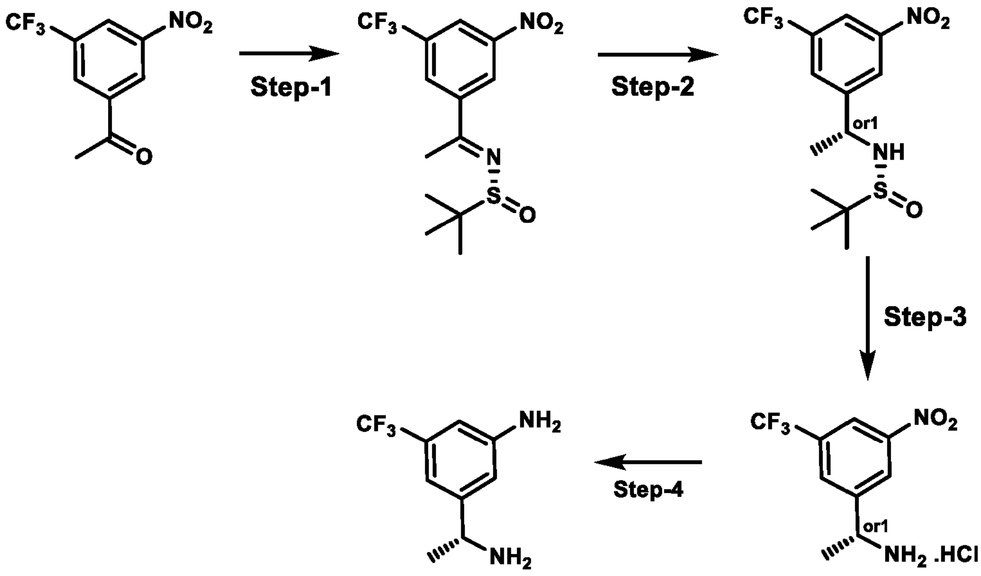


Step-1: (R)-2-methyl-N-(1-(3-nitro-5-(trifluoromethyl)phenyl)ethylidene)propane-2-sulfinamide

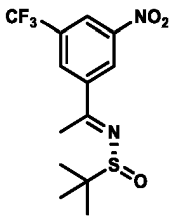

To a stirred solution of 1-(3-nitro-5-(trifluoromethyl)phenyl)ethan-1-one (60 g, 257 mmol) in THF (600.0 mL), (R)-2-methylpropane-2-sulfinamide (46.8 g, 386 mmol) and tetraethoxytitanium (135 mL , 643 mmol) were added at room temperature and the resulting reaction mixture was heated to 80 °C for 5 h. The reaction mixture was cooled to room temperature, quenched with cold water (100 mL) and diluted with ethyl acetate (600 mL). Resulting mixture was passed through celite bed and layers were separated. Organic layer was washed with brine (200 mL), dried over anhydrous $Na_2SO_4$ and evaporated. The crude product was purified by flash chromatography to provide the titled compound (61 g, 70.5 % yield).

MS(ES+) m/z = 337.2 (M+1)

Step-2: (R)-2-methyl-N-((R/S)-1-(3-nitro-5-(trifluoromethyl)phenyl)ethyl)propane-2-sulfinamide

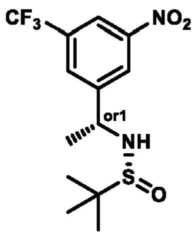

To a stirred solution of (R, E)-2-methyl-N-(1-(3-nitro-5-(trifluoromethyl)phenyl)ethylidene)propane-2-sulfinamide (60.0 g, 178 mmol) in THF (300.0 mL) and water (6.0 mL), $NaBH_4$ (13.50 g, 357 mmol) was added at -78 °C. The reaction was stirred at same temperature for 25 min, quenched with cold water and extracted with ethyl acetate (3 x 200.0 mL). Combined organic layer was washed with brine (100.0 mL), dried over anhydrous $Na_2SO_4$ and concentrated. The crude material (diastereomeric mixture) was purified using flash chromatography to yield titled compound as major

product (40 g, 66.3 % yield).

MS(ES+) m/z = 339.1 (M+1)

Step-3: (R/S)-1-(3-Nitro-5-(trifluoromethyl)phenyl)ethan-1-amine hydrochloride

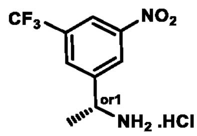

To a stirred solution of (R/S)-2-methyl-N-((R)-1-(3-nitro-5-(trifluoromethyl)phenyl)ethyl)propane-2-sulfinamide (30.0 g, 89 mmol) in DCM (100.0 mL ) was added 4M HCl in dioxane (222.0 mL, 887 mmol) and stirred at room temperature for 30 min. Solvent was removed under reduced pressure to get solid compound. Diethyl ether (200.0 mL) was added and stirred for 15 min, precipitated solid was filtered, dried under vacuum to afford titled compound (21.2 g, 88 % yield).

$^1$H NMR (400 MHz, DMSO-$d_6$) δ 8.92 (s, 2H), 8.80 (t, *J* = 1.9 *Hz,* 1H), 8.53 - 8.47 (m, 2H), 4.83 - 4.69 (m, 1H), 1.60 (d, *J* = 6.7 *Hz,* 3H).

Step-4: (R)-3-(1-Aminoethyl)-5-(trifluoromethyl)aniline

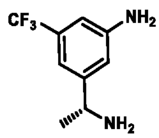

The (R/S)-1-(3-nitro-5-(trifluoromethyl)phenyl)ethan-1-amine hydrochloride (12 g, 44.3 mmol) was charged to Parr shaker containing MeOH (300.0 mL ) and Pd-C (0.944 g, 8.87 mmol) was added carefully. The reaction was stirred for 3h under hydrogen pressure (40 psi). Reaction mixture was filtered through a celite bed . Filtrate was concentrated under vacuum and residue was basified with a sat. sodium bicarbonate solution. The bicarbonate layer was extracted with DCM (150.0 mL x 3). The organic layer was dried over anhydrous Na2SO4 and concentrated under reduced pressure to afford titled compound (8.5g, 95% yield) Chirality of the compound was confirmed as 'R' by VCD experiment.

$^1$H NMR (400 MHz, DMSO-$d_6$) δ 6.85 - 6.77 (m, 2H), 6.70 - 6.65 (m, 1H), 5.46 (s, 2H), 3.92 - 3.83 (m, 1H), 1.20 (d, *J* = 6.6 *Hz,* 3H).

Example 2: Preparation of (R/S)-1-(2-fluoro-3-(trifluoromethyl)phenyl)ethan-1-amine hydrochloride

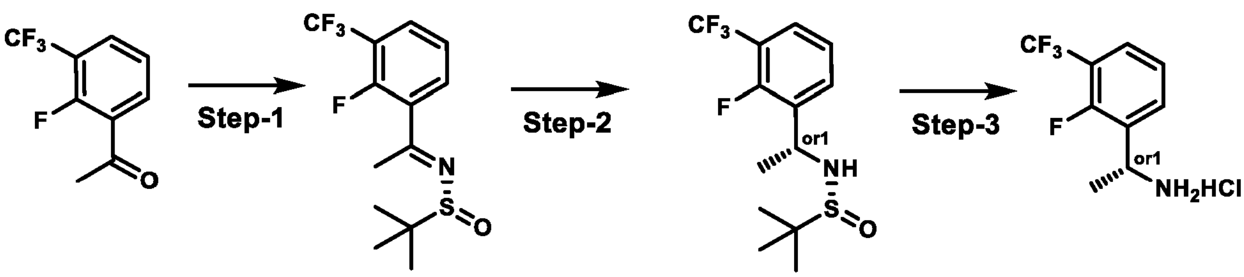


Step-1: (R)-N-(1-(2-fluoro-3-(trifluoromethyl)phenyl)ethylidene)-2-methylpropane-2-sulfinamide

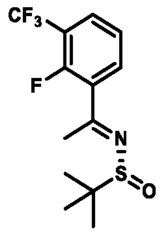

The titled compound was synthesized from 1-(2-fluoro-3-(trifluoromethyl)phenyl)ethan-1-one (commercial) by following analogous reaction protocol as described in Example - 1, Step - 1 (14.1 g, 85% yield)
$^{1}$H NMR (400 MHz, Chloroform-*d*) δ 7.90 - 7.84 (m, 1H), 7.76 - 7.71 (m, 1H), 7.34 - 7.29 (m, 1H), 2.82 (d, *J* = 3.6 *Hz,* 3H), 1.34 (s, 9H).

Step-2: (R/S)-N-((R)-1-(2-fluoro-3-(trifluoromethyl)phenyl)ethyl)-2-methylpropane-2-sulfinamide

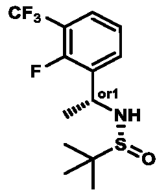

The titled compound was synthesized from Step - 1 intermediate by following analogous reaction protocol as described in Step- 2 of Example - 1 (5.30 g, 70% yield). It was obtained as major isomer.

MS(ES+) m/z = 312.34 (M+1)
($T_{ret}$(min) = 1.88, eluted as second peak)

Step - 3: (R/S)-1-(2-Fluoro-3-(trifluoromethyl)phenyl)ethan-1-amine hydrochloride

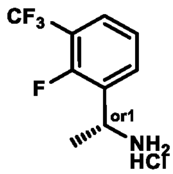

The titled compound was synthesized from Step - 2 intermediate by following analogous reaction protocol as described in Step -3 of Example - 1 (3.40 g, 82% yield).
$^{1}$H NMR (400 MHz, DMSO-$d_6$) δ 8.84 (s, 2H), 8.17 - 8.08 (m, 1H), 7.87 - 7.78 (m, 1H), 7.58 - 7.48 (m, 1H), 4.76 - 4.61 (m, 1H), 1.58 (d, *J* = 6.8 *Hz,* 3H).

**Example 3**: Preparation of (R/S)-1-(3-(1,1-Difluoroethyl)-2-fluorophenyl)ethan-1-amine.

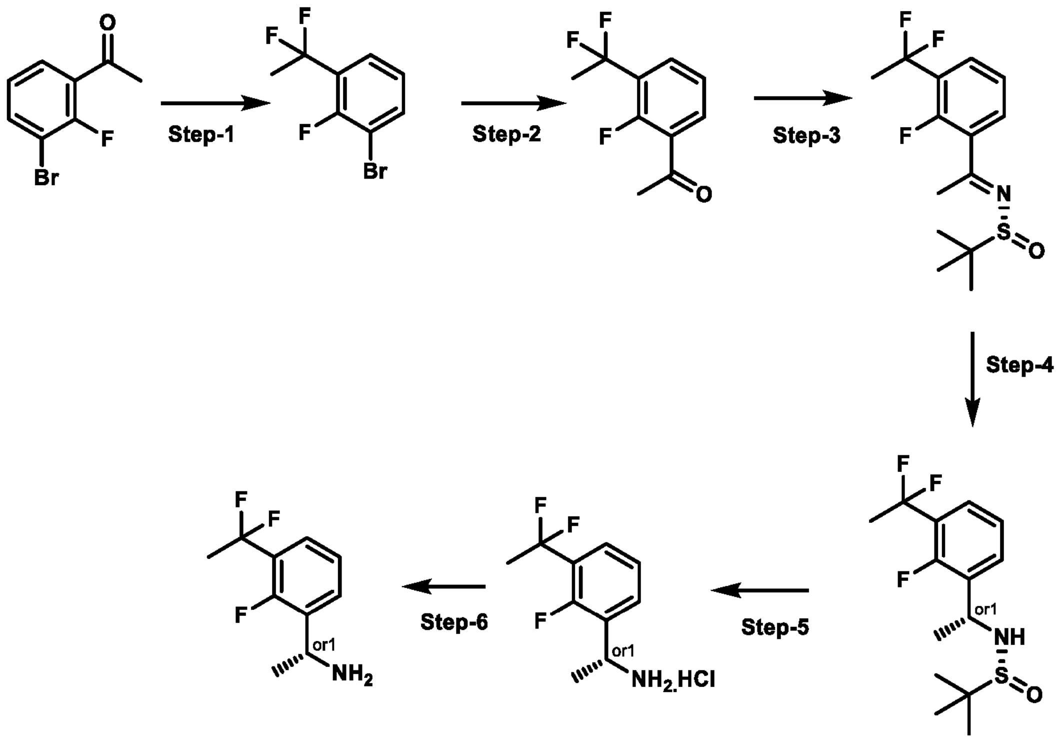


Step-1: 1-Bromo-3-(1,1-difluoroethyl)-2-fluorobenzene.

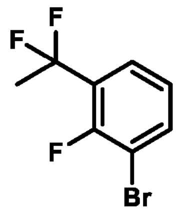

To a stirred solution of 1-(3-bromo-2-fluorophenyl)ethan-1-one (3.50 g, 16.13 mmol) in DCM (35.0 mL) was added DAST (17.0 mL, 129 mmol) and heated at 50 °C for 48 h. Reaction mixture was slowly quenched in ice water and then basified with sat. $NaHCO_3$ solution. The aqueous layer was extracted with Ethyl acetate (100.0 mL x 2). The combined organic layer was washed with water, brine, dried over anhydrous $Na_2SO_4$ and concentrated under the reduced pressure. The crude residue obtained was purified by flash chromatography in hexane-Ethyl acetate gradient to afford the titled compound (3.0 g, 78 % yield) as a colorless oil.

$^1H$ NMR (400 MHz, Chloroform-d) δ 7.69 - 7.60 (m, 1H), 7.55 - 7.47 (m, 1H), 7.15 - 7.07 (m, 1H), 2.03-2.08 (m, 3H).

Step-2: 1-(3-(1,1-Difluoroethyl)-2-fluorophenyl)ethan-1-one

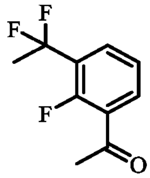

The titled compound was prepared from Step - 1 intermediate by following the analogous reaction protocol as described in Bulletin of the Chemical Society of Japan, 1987, vol. 60, # 2, p. 767 - 768 (2.0 g, 79% yield).

MS(ES+) m/z = 202.14 (M+)

Step-3: (R)-N-(1-(3-(1,1-difluoroethyl)-2-fluorophenyl)ethylidene)-2-methylpropane-2-sulfinamide

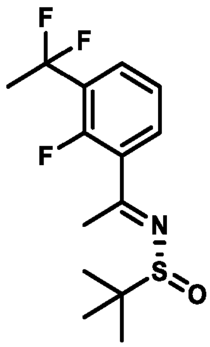

The titled compound was synthesized from Step - 2 intermediate by following analogous reaction protocol as described in Step - 1 of Example - 1 (2.10 g, 73% yield)

MS(ES+) m/z = 306.2 (M+1)

Step-4: (R/S)-N-((R)-1-(3-(1,1-Difluoroethyl)-2-fluorophenyl)ethyl)-2-methylpropane-2-sulfinamide

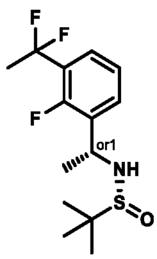

The titled compound was synthesized from Step - 3 intermediate by following analogous reaction protocol as described in Step - 2 of Example - 1 (1.60 g, 79% yield)

MS(ES+) m/z = 308.34 (M+1).

Step-5: (R/S)-1-(3-(1,1-Difluoroethyl)-2-fluorophenyl)ethan-1-amine hydrochloride

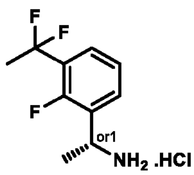

The titled compound was synthesized from Step - 4 intermediate by following analogous reaction protocol as described in Step - 3 of Example - 1 (1.2 g, 92% yield)

$^{1}$H NMR (400 MHz, DMSO-$d_6$) δ 8.80 (s, 2H), 7.92 - 7.80 (m, 1H), 7.68 - 7.58 (m, 1H), 7.42 (t, *J* = 7.8 *Hz,* 1H), 4.74 - 4.62 (m, 1H), 2.03 (t, *J* = 19.2 *Hz,* 3H), 1.56 (d, *J* = 6.8 *Hz,* 3H).

Step-6: (R/S)-1-(3-(1,1-Difluoroethyl)-2-fluorophenyl)ethan-1-amine.

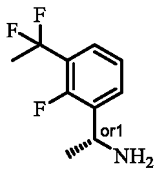

To a stirred solution of (R/S)-1-(3-(1,1-difluoroethyl)-2-fluorophenyl)ethan-1-amine hydrochloride (0.150 g, 0.626 mmol) in DCM (10.0 mL ) was added saturated solution of $NaHCO_3$ and stirred for 5 min at room temperature. The aqueous layer was extracted with DCM (20.0 mL x 2), organic layer dried over anhydrous $Na_2SO_4$ and concentrated to afford titled compound(crude) (0.110 g, 86% yield).

$^1$H NMR (400 MHz, DMSO-$d_6$) δ 7.78 - 7.69 (m, 1H), 7.46 - 7.37 (m, 1H), 7.27 (t, $J$ = 7.7 *Hz,* 1H), 4.31 (q, $J$ = 6.6 *Hz,* 1H), 2.05-1.98 (m, 4H), 1.26 (d, $J$ = 6.6 *Hz,* 3H).

**Example - 4:** Preparation of (R)-1-(3-(1-aminoethyl)-2-fluorophenyl)-1, 1-difluoro-2-methylpropan-2-ol hydrochloride & (S)-1-(3-(1-aminoethyl)-2-fluorophenyl)-1, 1-difluoro-2-methylpropan-2-ol hydrochloride

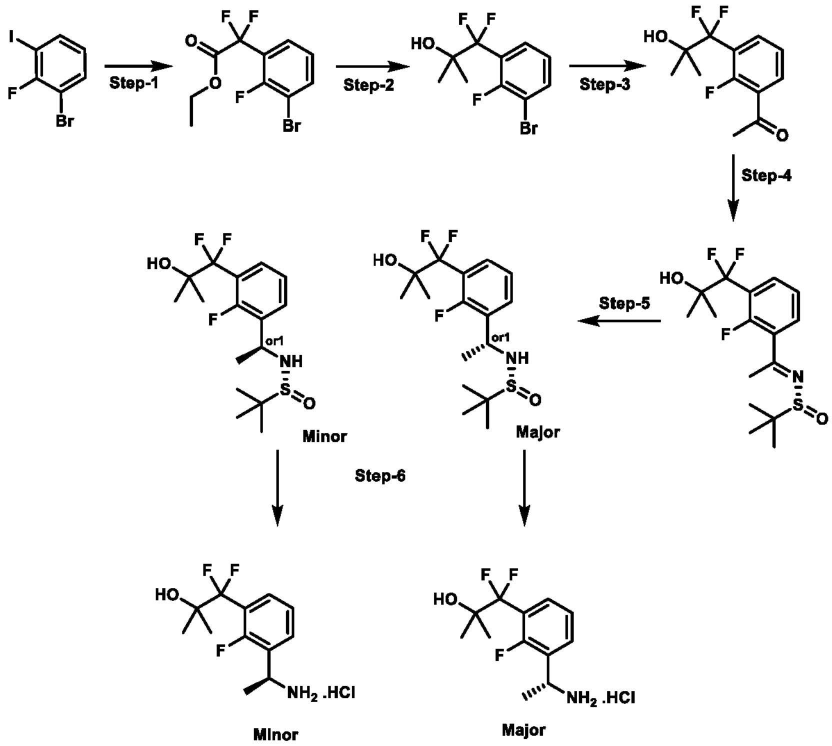


Step - 1: Ethyl 2-(3-bromo-2-fluorophenyl)-2, 2-difluoroacetate.

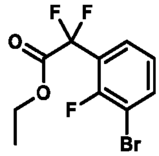

To a stirred solution of ethyl 2-bromo-2,2-difluoroacetate (69.1 g, 341 mmol) in DMSO (200.0 mL) was added copper powder (21.65 g, 341 mmol) and reaction was stirred for 30 min followed by addition of 1-bromo-2-fluoro-3-iodobenzene (41.0 g, 136 mmol). The reaction was stirred at 70 °C for 2 h. The reaction was cooled to room temperature, quenched with water (400.0 mL) and filtered through a celite bed. Celite bed was washed with diethyl ether (400.0 mL). The

Organic layer was separated, dried over anhydrous $Na_2SO_4$ and concentrated under reduced pressure. The crude residue was purified by flash chromatography hexane-Ethyl acetate gradient to afford the titled compound (24.1 g, 59.5 % yield) as a colorless liquid.

MS(ES+) m/z = 297.90 (M+1).

$^1H$ NMR (400 MHz, Chloroform-d) δ 7.77 - 7.70 (m, 1H), 7.65 - 7.59 (m, 1H), 7.21 - 7.15 (m, 1H), 4.39 (q, *J* = 7.1 Hz, 2H), 1.36 (t, *J* = 7.1 Hz, 3H).

Step - 2: 1-(3-Bromo-2-fluorophenyl)-1,1-difluoro-2-methylpropan-2-ol.

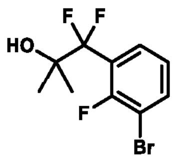

To a stirred solution of ethyl 2-(3-bromo-2-fluorophenyl)-2,2-difluoroacetate (10.0 g, 33.7 mmol) in THF (100.0 mL) was added methyl magnesium bromide in diethyl ether (3M, 33.7 mL, 101.0 mmol) in dropwise at 0 °C and the reaction was stirred at same temperature for 30 min. The reaction was quenched with saturated aqueous $NH_4Cl$ solution and extracted with diethyl ether (100.0 mL). The organic layer was separated, dried over anhydrous $Na_2SO_4$ and concentrated under reduced pressure. The crude product was purified by flash chromatography in hexane-Ethyl acetate gradient to afford the titled compound (9.2 g, 97 % yield) as a colorless liquid.

$^1H$ NMR (400 MHz, DMSO-$d_6$) δ 7.89 - 7.84 (m, 1H), 7.50 - 7.44 (m, 1H), 7.30 -7.23 (m, 1H), 5.43 (s, 1H), 1.21 (s, 3H), 1.20 (s, 3H)

Step - 3: 1-(3-(1,1-Difluoro-2-hydroxy-2-methylpropyl)-2-fluorophenyl) ethan-1-one

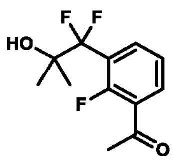

To a stirred solution of 1-(3-bromo-2-fluorophenyl)-1,1-difluoro-2-methylpropan-2-ol (12.5 g, 44.2 mmol) in toluene (150.0 mL), tributyl(1-ethoxyvinyl)stannane (19.14 g, 53.0 mmol), TEA (15.39 mL, 110 mmol) was added and reaction was purged with $N_2$ for 10 min. $PdCl_2(PPh_3)_2$ (1.24 g, 1.766 mmol) was added and reaction was stirred at 100 °C for 16 h. The reaction was cooled to room temperature and filtered through celite bed. The filtrate was evaporated under reduced pressure to afford 11.5 g crude product. The crude product as such was dissolved in THF (50.0 mL) and HCl: water (1:1) (3.0 mL) was added to it at 0 °C. The reaction mixture was warmed to room temperature and stirred for 15 min. The reaction mixture was neutralized with saturated $NaHCO_3$ (5.0 mL) and extracted with Ethyl acetate (100.0 mL x 3). The organic layer was separated, dried over anhydrous $Na_2SO_4$, and concentrated under reduced pressure. The crude product obtained was purified by flash chromatography in hexane-Ethyl acetate gradient to afford the titled compound (8.8 g, 81% yield) as an oily compound.

$^1H$ NMR (400 MHz, $CDCl_3$) δ 7.99 - 7.94 (m, 1H), 7.69 - 7.63 (m, 1H), 7.34 - 7.29 (m, 1H), 2.68 (d, *J* = 5.3 *Hz,* 3H), 1.39 (s, 3H), 1.38 (s , 3H).

Step - 4: (R)-N-(1-(3-(1,1-difluoro-2-hydroxy-2-methylpropyl)-2-fluorophenyl)ethylidene)-2-methylpropane-2-sulfinamide

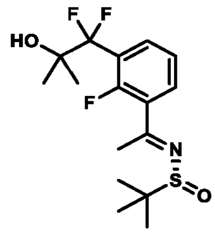

To a stirred solution of 1-(3-(1,1-difluoro-2-hydroxy-2-methylpropyl)-2-fluorophenyl)ethan-1-one (8.7 g, 35.3 mmol) in THF (100.0 mL), (R)-2-methylpropane-2-sulfinamide (6.42 g, 53.0 mmol) and Titanium (IV)isopropoxide (25.9 mL, 88 mmol) were added at room temperature. The resulting reaction mixture was heated at 100 °C for 16 h. Reaction was quenched with ice-cold water (100.0 mL) and diluted with Ethyl acetate(100.0 mL ). The mixture was filtered through celite bed. Organic layer of the filtrate was separated, dried over anhydrous $Na_2SO_4$, and concentrated under reduced pressure. The crude product was purified by flash chromatography using hexane-Ethyl acetate gradient to afford the titled compound (8.9 g, 72.1 % yield). MS(ES+) m/z = 350.28 (M+1)

$^1$H NMR (400 MHz, DMSO-$d_6$) δ 7.78 - 7.70 (m, 1H), 7.62 - 7.54 (m, 1H), 7.41 -7.34 (m, 1H), 5.40 (s, 1H), 2.82 - 2.75 (m, 3H) , 1.22 (s, 15H).

Step - 5: (R&S) -(R)-N-(1-(3-(1,1-difluoro-2-hydroxy-2-methylpropyl)-2-fluorophenyl)ethyl)-2-methylpropane-2-sulfinamide

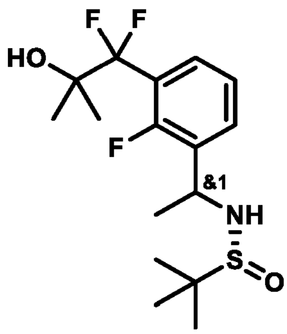

To a stirred solution of (R)-N-(1-(3-(1,1-difluoro-2-hydroxy-2-methylpropyl)-2-fluorophenyl)ethylidene)-2-methylpropane-2-sulfinamide (8.7 g, 24.90 mmol) in THF (90.0 mL) was added $NaBH_4$ (1.13 g, 29.9 mmol) at 0°C. The reaction mixture was stirred at room temperature for 1 h. Reaction was diluted with water (100 mL) and extracted with Ethyl acetate (100.0 mL x 3). The organic layer was separated, dried over anhydrous $Na_2SO_4$ and concentrated under reduced pressure. The crude product was purified by flash chromatography in hexane-Ethyl acetate gradient to afford titled compound as mixture of diastereomers. The two diastereomers were separated by preparative HPLC.

a. (R)-N-((R/S)-1-(3-(1,1-difluoro-2-hydroxy-2-methylpropyl)-2-fluorophenyl)ethyl)-2-methylpropane-2-sulfinamide (42.3 % yield, Major isomer).

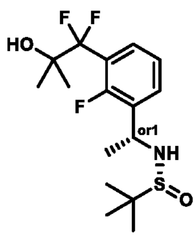

$^1$H NMR (400 MHz, DMSO-$d_6$) δ 7.70 - 7.64 (m, 1H), 7.37 - 7.31 (m, 1H), 7.30 - 7.24 (m, 1H), 5.84 (d, *J = 7.7 Hz,* 1H), 5.33 (s, 1H), 4.74 - 4.62 (m, 1H), 1.40 (d, *J* =6.8 *Hz,* 3H), 1.20 (bs, 6H), 1.10 (s, 9H).

b. (R)-N-((S/R)-1-(3-(1,1-difluoro-2-hydroxy-2-methylpropyl)-2-fluorophenyl)ethyl)-2-methylpropane-2-sulfinamide (15 % yield, Minor isomer).

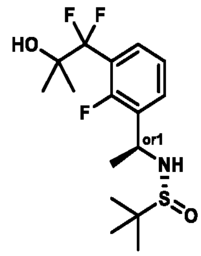

$^1H$ NMR (400 MHz, DMSO-$d_6$) δ 7.63 - 7.57 (m, 1H), 7.38 - 7.31 (m, 1H), 7.30 - 7.23 (m, 1H), 5.50 (d, *J* = 6.0 *Hz,* 1H), 5.34 (s, 1H), 4.78 - 4.64 (m, 1H), 1.49 (d, *J*= 6.8 *Hz,* 3H), 1.20 (bs, 6H), 1.10 (s, 9H).

Step - 6a: (R)-1-(3-(1-Aminoethyl)-2-fluorophenyl)-1,1-difluoro-2-methylpropan-2-ol hydrochloride.

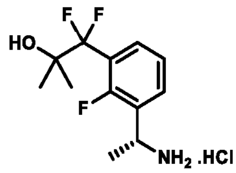

To a stirred solution of (R)-N-((R)-1-(3-(1,1-difluoro-2-hydroxy-2-methylpropyl)-2-fluorophenyl)ethyl)-2-methylpropane-2-sulfinamide (Step-5a, 3.65 g, 10.39 mmol) in DCM (30.0 mL) was added 4M HCl in dioxane (12.98 mL, 51.9 mmol) at 0 °C. The reaction mixture was stirred at room temperature for 30 min. The solvent was evaporated, and the residue was crystallized from diethyl ether to give titled compound. (2.7 g, 92.0 % yield) as a white solid. The chirality of the compound was confirmed as 'R' by X-ray crystallography.

$^1H$ NMR (400 MHz, DMSO-$d_6$) δ 8.73 - 8.67 (m, 2H), 7.87 - 7.80 (m, 1H), 7.51 -7.44 (m, 1H), 7.42 - 7.35 (m, 1H), 5.48 - 5.36 (m, 1H), 4.70 - 4.58 (m, 1H), 1.54 (d, *J*= 6.8 *Hz,* 3H), 1.22 (bs, 6H).

Step - 6b. (S)-1-(3-(1-Aminoethyl)-2-fluorophenyl)-1,1-difluoro-2-methylpropan-2-ol hydrochloride.

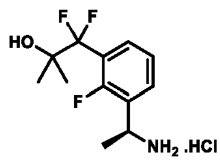

Titled compound was prepared from Step - 5 'b' intermediate using analogous protocol mentioned in Step-6a of Example 4 (90 % yield). The chirality of the compound was confirmed as 'S' by VCD experiment.

$^1H$ NMR (400 MHz, DMSO-$d_6$) δ 8.78 - 8.71 (m, 2H), 7.88 - 7.81 (m, 1H), 7.51 - 7.44 (m, 1H), 7.41 - 7.35 (m, 1H), 4.72 - 4.57 (m, 1H), 1.54 (d, *J*= 6.8 *Hz,* 3H), 1.22 (bs, 6H).

Example - 5: Preparation of (R)-2-(3-(1-aminoethyl)-2-fluorophenyl)-2,2-difluoroethan-1-ol hydrochloride

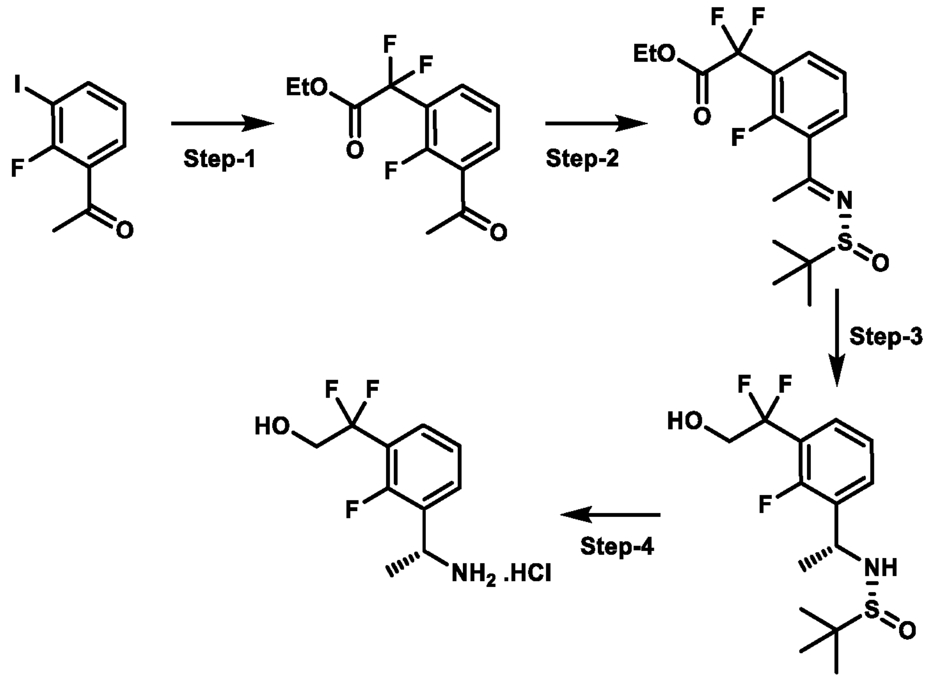


Step - 1: Ethyl 2-(3-acetyl-2-fluorophenyl)-2,2-difluoroacetate

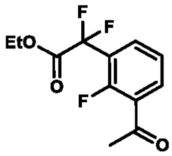

Titled compound was prepared from 1-(2-fluoro-3-iodophenyl)ethan-1-one using analogous protocol mentioned in Example 4-Step-1 (4.1 g, 69.3 % yield)

MS(ES+) m/z = 260.13

$^1$H NMR (400 MHz, Chloroform - d) δ 8.09 - 8.00 (m, 1H), 7.91 - 7.79 (m, 1H), 7.38 (t, *J* = 7.9 Hz, 1H), 4.40 (q, *J*= 7.0 Hz, 2H), 2.67 (d, *J*= 5.1 Hz, 3H), 1.37 (t, *J*= 7.1 Hz, 3H).

Step 2: ethyl (R)-2-(3-(1-((tert-butylsulfinyl)imino)ethyl)-2-fluorophenyl)-2,2-difluoroacetate

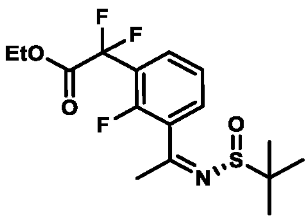

Titled compound was prepared from Step-1 intermediate using analogous protocol mentioned in Step - 4 of Example 4 (0.52 g, 74 % yield).

MS(ES+) m/z = 264.28

Step 3: (R)-N-((R/S)-1-(3-(1,1-difluoro-2-hydroxyethyl)-2-fluorophenyl)ethyl)-2-methylpropane-2-sulfinamide

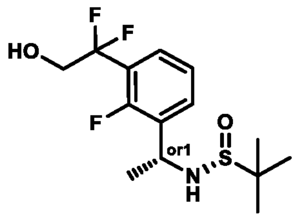

Titled compound as a major isomer was prepared from Step - 2 intermediate using analogous protocol mentioned in Step-5 of Example 4 (0.41 g, 92 % yield).

MS(ES+) m/z = 324.08 (M+1).

Step 4: (R)-2-(3-(1-Aminoethyl)-2-fluorophenyl)-2,2-difluoroethan-1-ol hydrochloride

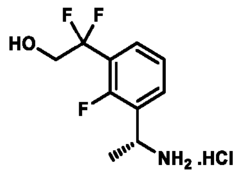

Titled compound was prepared from Step - 3 intermediate using analogous protocol mentioned in Step-3 of Example 1 (0.055 g, 81 % yield).

The compound chirality was confirmed as 'R' by VCD experiment.

$^1$H NMR (400 MHz, DMSO-$d_6$) δ 8.60 (bs, 2H), 7.86 - 7.80 (m, 1H), 7.63 - 7.56 (m, 1H ) 7.42 (t, *J* = 7.8 *Hz,* 1H), 4.73 - 4.62 (m, 1H), 3.94 (t, *J* = 14.3 *Hz,* 2H), 1.54 (d, *J* = 6.8 *Hz,* 3H).

**Example 6:** Preparation of (1R/S)-1-(3-(difluoro(tetrahydrofuran-2-yl)methyl)phenyl)ethan-1-amine hydrochloride

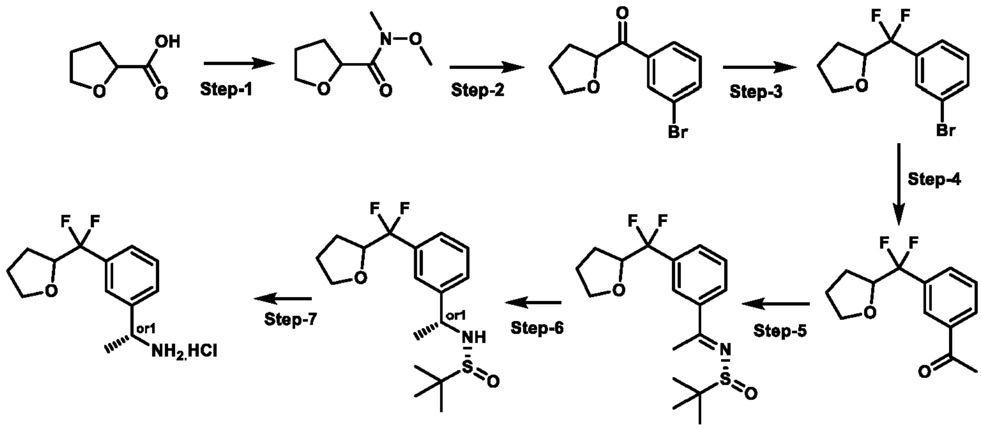


Step 1: N-methoxy-N-methyltetrahydrofuran-2-carboxamide

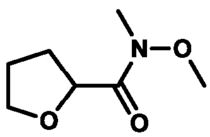

To a stirred solution of tetrahydrofuran-2-carboxylic acid (6.0 g, 51.7 mmol), N,O-dimethylhydroxylamine

hydrochloride (10.08 g, 103 mmol) and HATU (23.5 g, 62.0 mmol) in DMF (100.0 mL) was added DIPEA (22.5 mL, 129 mmol) and stirred reaction mixture for 16 h at room temperature. The reaction mixture was quenched with water and extracted with Ethyl acetate (2 x 50.0 mL). The combined organic layer was washed with water, dried over anhydrous $Na_2SO_4$ and concentrated under the reduced pressure to get a crude product. The crude compound was purified by flash chromatography to afford the titled compound as a colorless oil (6.0 g, 72.9 % yield).

$^1H$ NMR (400 MHz, DMSO-$d_6$) δ 4.73 - 4.66 (m, 1H), 3.84 - 3.74 (m, 2H), 3.67 (s, 3H), 3.66 - 3.59 (m, 1H), 3.34 (s, 3H), 3.18 - 3.12 (m, 1H), 2.13 - 2.06 (m, 1H), 1.88 - 1.82 (m, 1H).

Step 2: (3-Bromophenyl)(tetrahydrofuran-2-yl)methanone

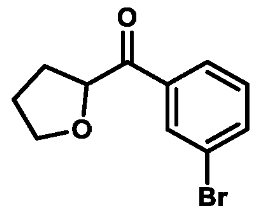

To a stirred solution of 1,3-dibromobenzene (6.4 mL , 53.4 mmol) in THF (50.0 mL) was slowly added nBuLi (21.3 mL , 53.4 mmol) at -78 °C and stirred for 30 min at -78 °C. A solution of N-methoxy-N-methyltetrahydrofuran-2-carboxamide (5.0 g, 31.4 mmol) in THF (30.0 mL) was added slowly at -78 °C. The reaction slowly warmed to room temperature over the period of 1 h. The reaction was quenched with saturated solution of ammonium chloride and was extracted with Ethyl acetate (2 x 50.0 mL). Combined organic layer was washed with brine, dried over anhydrous $Na_2SO_4$ and concentrated under reduced pressure to get a crude compound. The crude compound was purified by flash chromatography to afford the titled compound (2.0 g, 24.96 % yield).

MS(ES+) m/z = 159.13 (M+)

Step 3: 2-((3-Bromophenyl)difluoromethyl)tetrahydrofuran

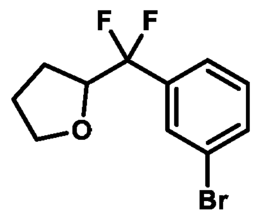

A stirred solution of (3-bromophenyl)(tetrahydrofuran-2-yl)methanone (2.0 g, 7.84 mmol) and DAST (10.36 ml, 78 mmol) was heated at 50 °C for 18 h. Reaction mixture was slowly quenched in ice water and then basified with NaHCO3. This was extracted with ethyl acetate (2 x 30.0 mL). Combined organic layer was washed with brine, dried over anhydrous $Na_2SO_4$ and concentrated under the reduced pressure. Crude product was purified by column chromatography in ethyl acetate -hexane gradient to titled compound (1.50 g, 69.0 % yield)

$^1H$ NMR (400 MHz, Chloroform-d) δ 7.70 (s, 1H), 7.62 - 7.57 (m, 1H), 7.51 - 7.46 (m, 1H), 7.36 - 7.30 (m, 1H), 4.38 - 4.27 (m, 1H), 3.89 - 3.82 (m, 2H), 2.12 - 2.02 (m, 2H), 1.94 - 1.82 (m, 2H).

Step 4: 1-(3-(Difluoro(tetrahydrofuran-2-yl)methyl)phenyl)ethan-1-one.

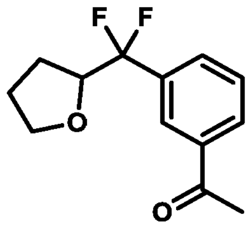

The titled compound was synthesized by using step 3 intermediate following analogous reaction protocol as described in Step - 3 of Example 4 (30 % yield)

$^1$H NMR (400 MHz, Chloroform-d) δ 8.15 - 8.12 (m, 1H), 8.08 - 8.04 (m, 1H), 7.79 - 7.74 (m, 1H), 7.60 - 7.54 (m, 1H), 4.42 - 4.31 (m, 1H), 3.88 - 3.81 (m, 2H), 2.66 (s, 3H), 2.14 - 2.05 (m, 2H), 1.93 - 1.83 (m, 2H).

Step 5: (R)-N-(1-(3-(difluoro(tetrahydrofuran-2-yl)methyl)phenyl)ethylidene)-2-methylpropane-2-sulfinamide.

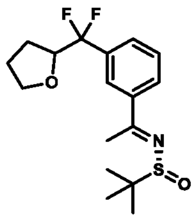

The titled compound was synthesized from Step - 4 intermediate by following analogous reaction protocol as described in Step - 4 of Example 4 (1.10 g, 64 % yield)

MS(ES+) m/z = 344.34 (M+1).

Step 6: (R)-N-((1R/S)-1-(3-(difluoro(tetrahydrofuran-2-yl)methyl)phenyl)ethyl)-2-methylpropane-2-sulfinamide

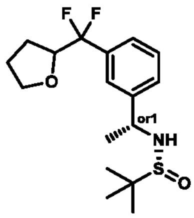

The titled compound was synthesized Step - 5 intermediate by following analogous reaction protocol as described in Step - 5 of Example 4 (0.9 g, 89 % yield)

MS(ES+) m/z =346.2 (M+1).

Step 7: (R/S)-1-(3-(difluoro(tetrahydrofuran-2-yl)methyl)phenyl)ethan-1-amine hydrochloride

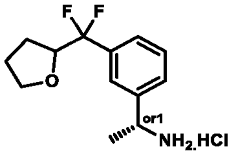

The titled compound was synthesized from Step - 6 intermediate by following analogous reaction protocol as described in Step - 6 of Example 4. (0.65 g, 90 % yield)

$^1$H NMR (400 MHz, DMSO-$d_6$) δ 8.55 (s, 2H), 7.73 - 7.63 (m, 2H), 7.58 - 7.51 (m, 2H), 4.53 - 4.37(m, 2H), 3.78 -

3.65 (m, 2H), 2.05 - 1.89 (m, 2H), 1.88 - 1.64 (m, 3H), 1.52 (d, *J*= 6.8 *Hz,* 3H).

Example 7 : Preparation of (R/S)-1-(3-(difluoromethyl)-2-methylphenyl)ethan-1-amine hydrochloride.

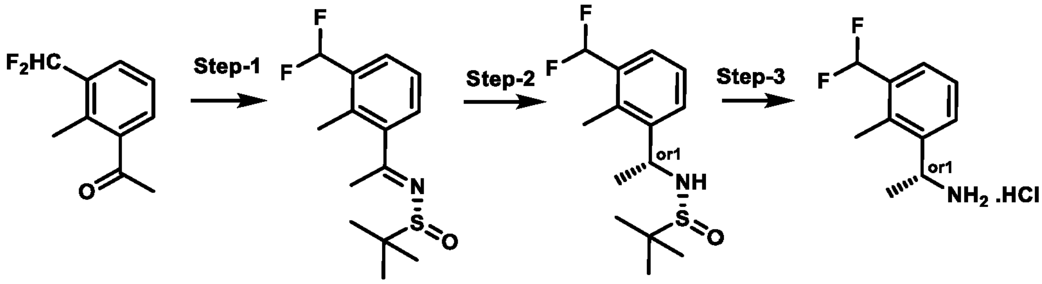


Step 1: (R)-N-(1-(3-(difluoromethyl)-2-methylphenyl)ethylidene)-2-methylpropane-2-sulfinamide

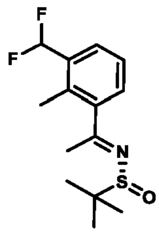

The titled compound was synthesized from 1-(3-(difluoromethyl)-2-methylphenyl)ethan-1-one ( prepared by using method mentioned in *Journal of Medicinal Chemistry* 2018, *vol 61,* #12, p.5235-5244.) by following analogous reaction protocol as described in Step - 4 of Example 4 (3.05 g, 67.4 % yield).

MS(ES+) m/z = 288.2 (M+1).

Step 2: (R)-N-((R/S)-1-(3-(difluoromethyl)-2-methylphenyl)ethyl)-2-methylpropane-2-sulfinamide

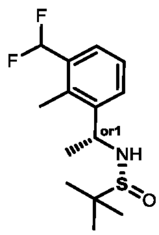

The titled compound was synthesized from Step - 1 intermediate by following analogous reaction protocol as described in step - 5 of Example 4 ((1.1 g, 36.4 % yield) req isomer $t_{ret}$ min = 1.77

$^1$H NMR (400 MHz, Chloroform-d) δ 7.57 (dd, *J*= 7.5, 1.5 *Hz,* 1H), 7.49 (d, *J*= 7.6 *Hz,* 1H), 7.34 (t, *J* = 7.8 *Hz,* 1H), 6.82 (t, *J* = 55.4 *Hz,* 1H), 4.91 - 4.89 (m, 1H), 2.45 (s, 3H), 1.52 (d, *J*= 6.5 *Hz,* 3H), 1.26 (s, 9H).

Step 3: (R/S)-1-(3-(difluoromethyl)-2-methylphenyl)ethan-1-amine hydrochloride.

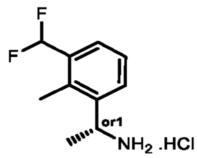

The titled compound was synthesized from Step - 2 intermediate by following analogous reaction protocol as described in step - 3 of Example 1 (0.45 g, 67.0 % yield).

$^1$H NMR (400 MHz, DMSO-$d_6$) δ 8.51 (s, 2H), 7.77 (d, *J*= 7.8 *Hz,* 1H), 7.55 (d, *J*= 7.6 *Hz,* 1H), 7.45 (t, *J = 7.8 Hz,* 1H), 7.32 (, *J* = 54.7 *Hz,* 1H), 4.73-4.62 (m ,1H), 2.40 (s, 3H), 1.49 (d, *J*= 6.7 *Hz,* 3H).

**Example 8:** Preparation of (R/S)-3-(1-aminoethyl)-5-(difluoromethyl)aniline

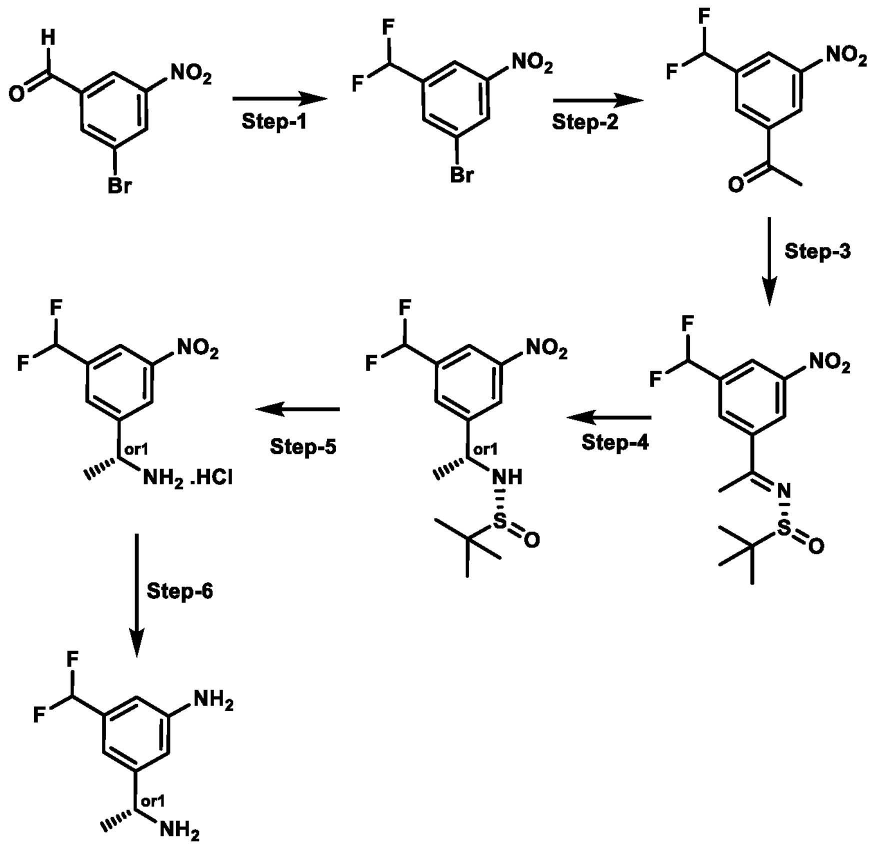


Step-1: 1-bromo-3-(difluoromethyl)-5-nitrobenzene

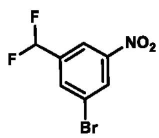

To a stirred solution of 3-bromo-5-nitrobenzaldehyde (35.0 g, 152.0 mmol) was dissolved in DCM (350.0 mL), DAST (101.0 mL, 761.0 mmol) was added dropwise at 0 °C and the reaction was allowed to warm room temperature and continued the stirring 18 h. Reaction poured over ice and extracted with DCM (400.0 mL). The combined organic layer was washed with water (500.0 mL x 2), brine and dried over anhydrous $Na_2SO_4$. Removal of solvent provided crude product. crude compound was purified by flash chromatography using hexane - Ethyl acetate gradient to afford the titled compound

(36.0 g, 94 % yield).

$^{1}H$ NMR (400 MHz, DMSO-$d_6$) δ 8.59 - 8.57 (m, 1H), 8.42 - 8.39 (m, 1H), 8.31 - 8.28 (m, 1H), 7.20 (t, *J = 55.0 Hz,* 1H).

Step-2: 1-(3-(difluoromethyl)-5-nitrophenyl)ethan-1-one

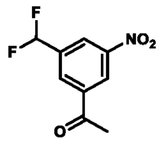

The titled compound was synthesized using step-1 intermediate by following analogous reaction protocol as described in Step-3 of Example 4. (Qty: 2.2 g 55 % yield) $^{1}H$ NMR (400 MHz, DMSO-$d_6$) δ 8.78 - 8.76 (m, 1H), 8.66 - 8.63 (m, 1H), 8.55 - 8.53 (m, 1H), 7.27 (t, *J* = 55 *Hz,* 1H), 2.74 (s, 3H).

Step-3: (R)-N-(1-(3-(difluoromethyl)-5-nitrophenyl)ethylidene)-2-methylpropane-2-sulfinamide

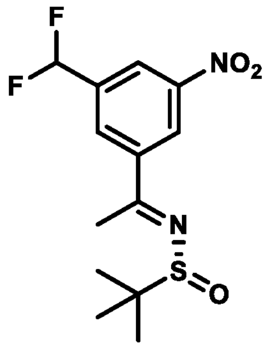

The title compound was synthesized by using step-2 intermediate and following analogous reaction protocol as described in Step - 4 of example 4.

$^{1}H$ NMR (400 MHz, DMSO-$d_6$) δ 8.76 - 8.74 (m, 1H), 8.57 - 8.54 (m, 1H), 8.49 - 8.46 (m, 1H), 7.30 (t, J = 55.1 Hz, 1H), 2.84 (s, 3H), 1.26 (s, 9H).

Step-4: (R)-N-((R/S)-1-(3-(difluoromethyl)-5-nitrophenyl)ethyl)-2-methylpropane-2-sulfinamide

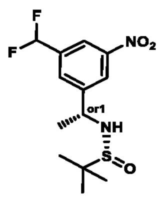

The titled compound was synthesized by using step-3 intermediate following analogous reaction protocol as described in Step-5 of Example 4. (1.85 g 55 % yield).

$^{1}H$ NMR (400 MHz, DMSO-$d_6$) δ 8.53 - 8.51 (m, 1H), 8.29 - 8.31 (m, 1H), 8.12 - 8.10 (m, 1H), 7.22 (t, J = 55.3 Hz, 1H), 6.05 (d, J = 8.3 Hz, 1H), 4.70 - 4.58 (m, 1H), 1.45 (d, J = 6.9 Hz, 3H), 1.14 (s, 9H).

Step-5: (R/S)-1-(3-(difluoromethyl)-5-nitrophenyl)ethan-1-amine hydrochloride

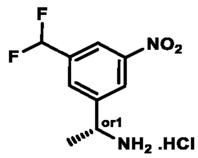

The titled compound was synthesized by using step-4 intermediate following analogous reaction protocol as described in Step-6 of Example 4.

$^1$H NMR (400 MHz, DMSO-$d_6$) δ 8.89 (s, 2H), 8.69 - 8.66 (m, 1H), 8.45 - 8.41 (m, 1H), 8.30 - 8.28 (m, 1H), 7.26 (t, *J* = 55.1 Hz, 1H), 4.75 - 4.66 (m, 1H), 1.58 (d, *J* = 6.8 Hz, 3H).

Step-6: (R/S)-3-(1-aminoethyl)-5-(difluoromethyl)aniline

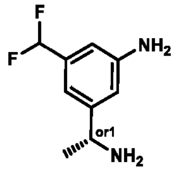

The titled compound was synthesized by using Step - 5 intermediate following analogous reaction protocol as described in Step - 6 of Example 3. (0.5 g, 98 % yield) as crude solid.

$^1$H NMR (400 MHz, DMSO - $d_6$) δ 6.80 (t, *J* = 55 Hz, 1H), 6.71 - 6.67 (m, 2H), 6.57 - 6.54 (m, 1H), 5.28 (s, 2H), 3.91 - 3.83 (m, 1H), 1.92 - 1.71 (m, 2H), 1.20 (d, *J* = 6.5 Hz, 3H).

**Example 9:** Synthesis of (R&S)-3-(3-((R&S)-1-aminoethyl)-2-fluorophenyl)-3,3-difluoro-2-methylpropane-1,2-diol hydrochloride

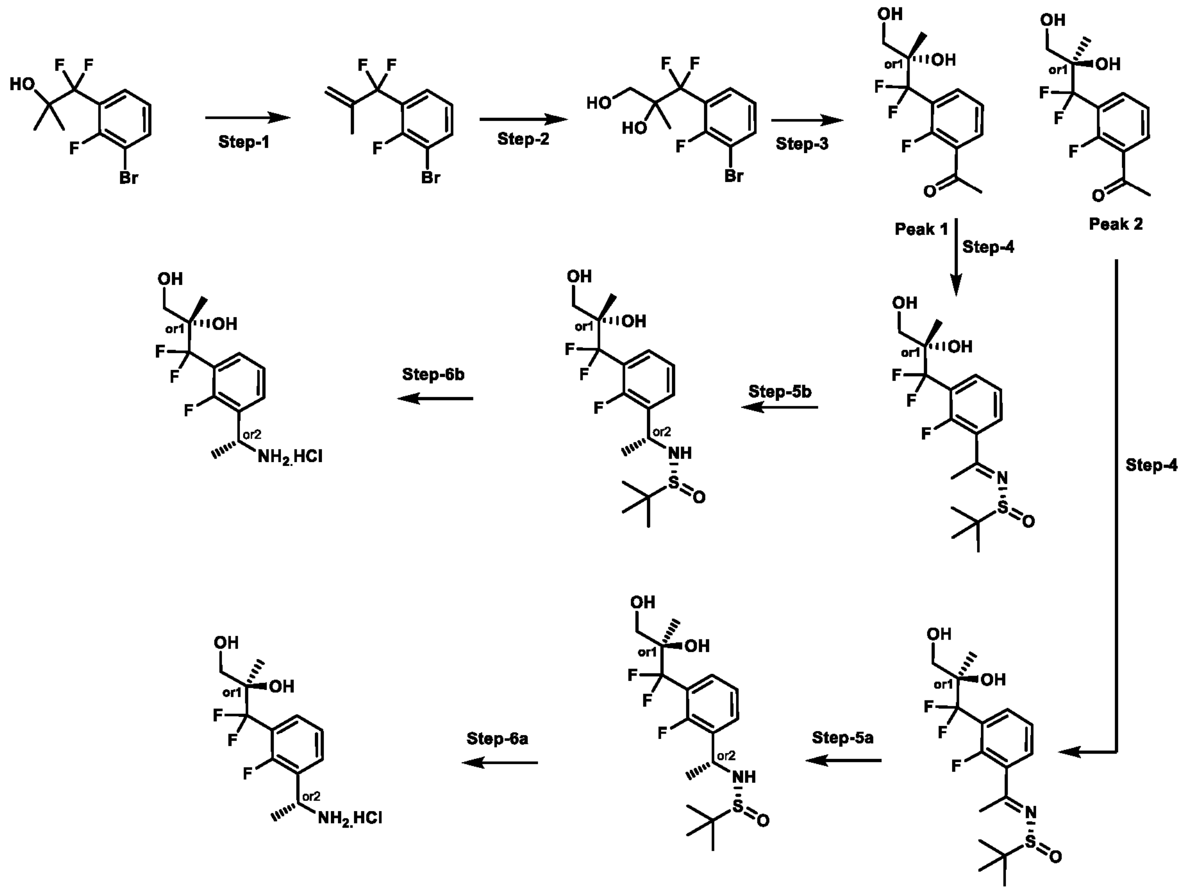


**Step- 1:** 1-bromo-3-(1, 1-difluoro-2-methylallyl)-2-fluorobenzene.

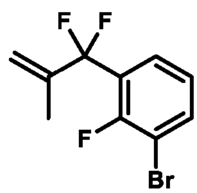

To a stirred solution of 1-(3-bromo-2-fluorophenyl)-1, 1-difluoro-2-methylpropan-2-ol (12.5 g, 44.2 mmol) in DCM (130.0 mL) was added Martin's Sulfurane (29.7 g, 44.2 mmol) at room temperature. The reaction mixture was stirred at same temperature for 30 min. The reaction mixture was concentrated under reduced pressure and crude product obtained was purified by flash chromatography with gradient elution (0-2%) of Ethyl acetate: n-hexane to afford titled compound (8.1 g, 69.2 % yield) as a colorless liquid.

MS(ES+) m/z = 264.10 (M+).

$^1H$ NMR (400 MHz, Chloroform-d) δ 7.72 - 7.64 (m, 1H), 7.55 - 7.47 (m, 1H), 7.17 - 7.08 (m, 1H), 5.36 - 5.21 (m, 2H), 1.91 - 1.84 (m, 3H).

**Step- 2:** 3-(3-bromo-2-fluorophenyl)-3, 3-difluoro-2-methylpropane-1, 2-diol

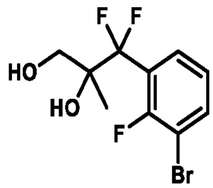

To a stirred solution of 1-bromo-3-(1,1-difluoro-2-methylallyl)-2-fluorobenzene (7.0 g, 26.4 mmol) in acetone (60.0 mL) and water (15.0 mL) was added N-methylmorpholine N-oxide (6.19 g, 52.8 mmol) and potassium osmate dihydrate (0.584 g, 1.584 mmol) at room temperature. The reaction mixture was stirred at same temperature for 24 h. Reaction mixture was diluted with cold water (70.0 mL) and compound was extracted with Ethyl acetate (70.0 mL x 3). The organic layer was separated, dried over anhydrous $Na_2SO_4$, and concentrated under reduced pressure. Crude obtained was purified by flash chromatography with gradient elution (0-60%) of Ethyl acetate in n-hexane to afford titled compound (6.7 g, 85 % yield) as brown liquid.

$^1$H NMR (400 MHz, DMSO-*d*$_6$) δ 7.90 - 7.79 (m, 1H), 7.54 - 7.42 (m, 1H), 7.31 - 7.20 (m, 1H), 4.07 - 3.98 (m, 1H), 3.57 - 3.55 (m, 1H), 3.46 - 3.40 (m, 1H), 3.38 - 3.30 (m, 1H), 1.18 (s, 3H).

**Step- 3:** (S/R)-1-(3-(1, 1-difluoro-2, 3-dihydroxy-2-methylpropyl)-2-fluorophenyl) ethan-1-one **(Peak-1) and** (R/S)-1-(3-(1, 1-difluoro-2, 3-dihydroxy-2-methylpropyl)-2-fluorophenyl) ethan-1-one **(Peak2)**

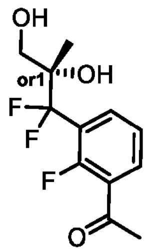

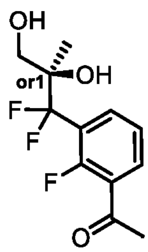

The titled compounds were synthesized by following analogous reaction protocol as described in Step-3 of Example-4 preparation using 3-(3-bromo-2-fluorophenyl)-3,3-difluoro-2-methylpropane-1,2-diol and further separated by chiral chromatography (Instrument Method: MEOH_0.1%DEA_100_B_1.0 ML _8MIN Flow Rate: 1.00 mL /min) to afford two isomers as Peak 1 (1.25g, 41% yield) & Peak 2 (1.35 g, 45% yield) respectively.

**Peak 1:** $^1$H NMR (400 MHz, DMSO-*d*$_6$) δ 7.98 - 7.83 (m, 1H), 7.73 - 7.62 (m, 1H), 7.45 - 7.34 (m, 1H), 5.35 (s, 1H), 4.74 (t, *J* = 6.0 Hz, 1H), 3.52 - 3.36 (m, 2H), 2.59 (d, *J* = 4.3 Hz, 3H), 1.21 (s, 3H). Chiral Purity-99.93%, (RT-3.75) **Peak 2:** $^1$H NMR (400 MHz, DMSO-*d*$_6$) δ 7.95 - 7.84 (m, 1H), 7.72 - 7.64 (m, 1H), 7.44 - 7.35 (m, 1H), 5.35 (s, 1H), 4.74 (t, *J* = 6.1 Hz, 1H), 3.50 - 3.33 (m, 2H), 2.59 (d, *J* = 4.3 Hz, 3H), 1.21 (s, 3H). Chiral Purity-93.76%, (RT-4.28).

**Step- 4:** (R)-N-(1-(3-((S/R)-1,1-difluoro-2,3-dihydroxy-2-methylpropyl)-2-fluorophenyl)ethylidene)-2-methylpropane-2-sulfinamide **(Peak 1)** and (R)-N-(1-(3-((R/S)-1,1-difluoro-2,3-dihydroxy-2-methylpropyl)-2-fluorophenyl)ethylidene)-2-methylpropane-2-sulfinamide **(Peak 2)**

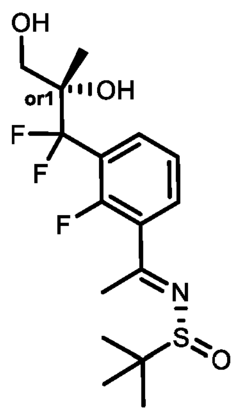

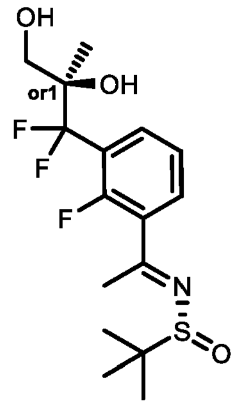

The titled compounds were synthesized from Step - 3 intermediate by following analogous reaction protocol as described in Step - 4 of Example 4 (Peak 1 & Peak 2 respectively).**(Peak-1)** : (1.60 g, 63% yield) MS(ES+) m/z = 366.35 (M+1).

$^1$H NMR (400 MHz, DMSO-$d_6$) δ 7.79 - 7.68 (m, 1H), 7.64 - 7.55 (m, 1H), 7.42 - 7.32 (m, 1H), 5.33 (s, 1H), 4.73 (t, *J*= 6.0 Hz, 1H), 3.48 - 3.36 (m, 2H), 2.68 (d, *J* = 2.5 Hz, 3H), 1.22 (s, 3H), 1.08 (s, 9H).

**(Peak-2):** (1.80 g, 71% yield)
MS(ES+) m/z = 366.35 (M+1).

$^1$H NMR (400 MHz, DMSO-$d_6$) δ 7.78 - 7.68 (m, 1H), 7.63 - 7.54 (m, 1H), 7.42 - 7.31 (m, 1H), 5.33 (s, 1H), 4.73 (t, *J*= 6.0 Hz, 1H), 3.50 - 3.38 (m, 2H), 2.68 (d, *J*= 2.6 Hz, 3H), 1.23 (s, 9H), 1.08 (s, 3H).

**Step- 5a:** (R)-N-((R/S)-1-(3-((R/S)-1,1-difluoro-2,3-dihydroxy-2-methylpropyl)-2-fluorophenyl)ethyl)-2-methylpropane-2-sulfinamide

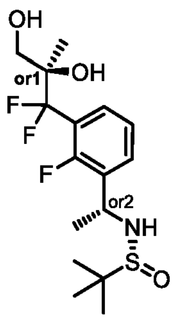

The titled compounds were synthesized from Step - 4 intermediate by following analogous reaction protocol mentioned in Step-5 of Example 4 (Peak 1) (0.45 g, 37% yield).

**(Major isomer of Peak 1):**

MS(ES+) m/z = 368.35 (M+1)

$^1$H NMR (400 MHz, DMSO-$d_6$) δ 7.69 - 7.63 (m, 1H), 7.38 - 7.31 (m, 1H), 7.31 - 7.23 (m, 1H), 5.85 (d, *J* = 7.7 Hz, 1H), 5.23 (s, 1H), 4.71 - 4.65 (m, 2H), 3.48 - 3.35 (m, 2H), 1.40 *(d, J*= 6.8 Hz, 3H), 1.19 (s, 3H), 1.11 (s, 9H).

Step 5b: (R)-N-((R/S)-1-(3-((S/R)-1,1-difluoro-2,3-dihydroxy-2-methylpropyl)-2-fluorophenyl)ethyl)-2-methylpropane-2-sulfinamide

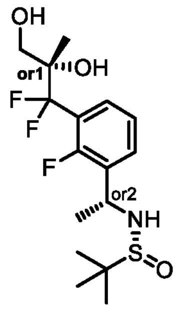

The titled compounds were synthesized from Step-4 intermediate by following analogous reaction protocol mentioned in Step-5 of Example 4 ( peak-2) (0.41 g, 34% yield) **(Major isomer of Peak 2):**
MS(ES+) m/z = 368.35 (M+1).

$^1$H NMR (400 MHz, DMSO-$d_6$) δ 7.69 - 7.63 (m, 1H), 7.38 - 7.31 (m, 1H), 7.30 - 7.23 (m, 1H), 5.85 (d, $J$ = 7.6 Hz, 1H), 5.24 (s, 1H), 4.76 - 4.61 (m, 2H), 3.44 - 3.38 (m, 2H), 1.40 (d, $J$ = 6.8 Hz, 3H), 1.19 (s, 3H), 1.11 (s, 9H).

**Step- 6a:** (R/S)-3-(3-((R/S)-1-aminoethyl)-2-fluorophenyl)-3,3-difluoro-2-methylpropane-1,2-diol hydrochloride

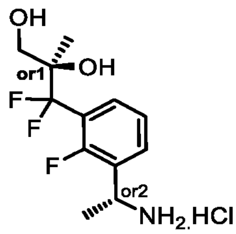

The titled compounds were synthesized from Step-5a intermediate by following analogous protocol as described in of Step-6 of Example 4 **(Major isomer of Peak 1)** (0.42 g, 94% yield)

$^1$H NMR (400 MHz, DMSO-$d_6$) δ 8.62 (s, 2H), 7.84 - 7.76 (m, 1H), 7.52 - 7.45 (m, 1H), 7.41 - 7.34 (m, 1H), 5.43 - 5.21 (m, 1H), 4.30 - 4.10 (m, 1H), 4.08 - 3.94 (m, 1H), 3.46 - 3.37 (m, 2H), 1.53 (d, $J$ = 6.8 Hz, 3H), 1.20 (s, 3H) MS(ES+) m/z = 264.15 (M+1).

Step 6b: (S/R)-3-(3-((S/R)-1-aminoethyl)-2-fluorophenyl)-3,3-difluoro-2-methylpropane-1,2-diol hydrochloride

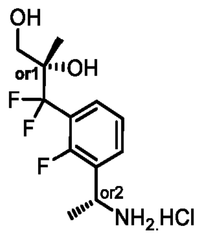

The titled compounds were synthesized from Step-5a intermediate by following analogous protocol as described in of Step-6 of Example-4 **(Major isomer of Peak 2** (0.52 g , 86% yield)
MS(ES+) m/z = 364.15 (M+1).

$^1$H NMR (400 MHz, DMSO-$d_6$) δ 8.60 (s, 2H), 7.81 - 7.74 (m, 1H), 7.53 - 7.45 (m, 1H), 7.41 - 7.33 (m, 1H), 5.37-5.28 (m, 1H), 4.76 (t, $J$= 6.1 Hz, 1H), 4.73 - 4.61 (m, 1H), 3.49 - 3.35 (m, 2H), 1.53 (d, $J$= 6.8 Hz, 3H), 1.20 (s, 3H)

**Example 10:** (R/S)-1-(3-(1-aminoethyl)phenyl)-1,1-difluoro-2-methylpropan-2-ol hydrochloride

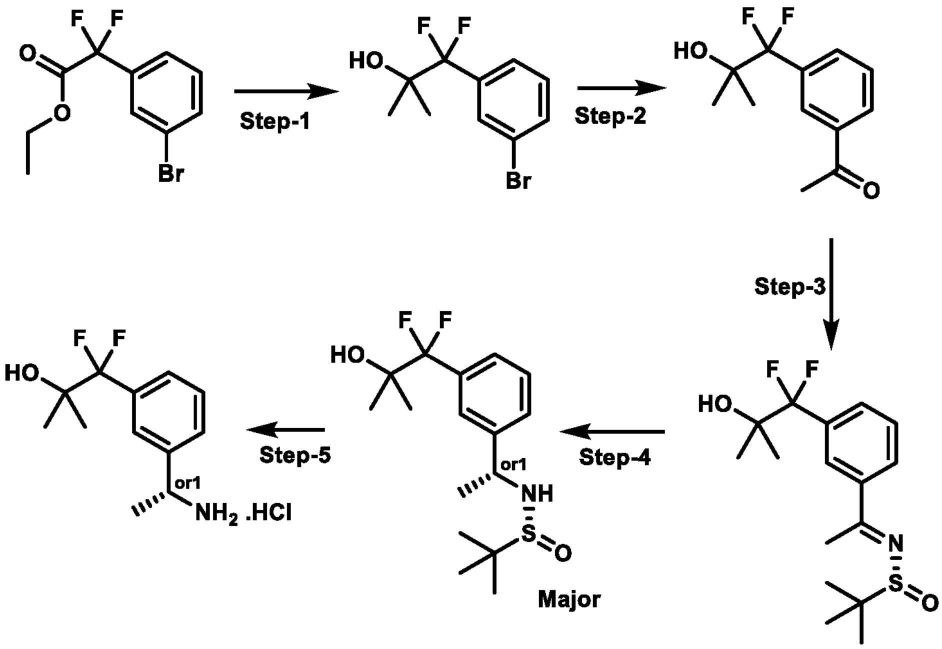


Step - 1: 1-(3-Bromophenyl)-1,1-difluoro-2-methylpropan-2-ol

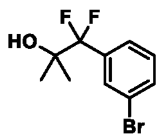

The titled compounds were synthesized from commercially available ethyl 2-(3-bromophenyl)-2,2-difluoroacetate (commercial) by following analogous reaction protocol as described in of Step - 2 of Example - 4 (65 % yield)

$^1H$ NMR (400 MHz, DMSO-$d_6$) δ 7.73 - 7.69 (m, 1H), 7.64 - 7.61 (m, 1H), 7.52 - 7.41 (m, 2H), 5.41 (s, 1H), 1.18 (s, 3H), 1.15 (s, 3H).

Step - 2: 1-(3-(1,1-Difluoro-2-hydroxy-2-methylpropyl)phenyl)ethan-1-one

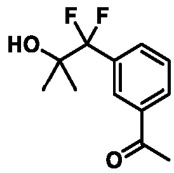

The titled compounds were synthesized from Step - 1 intermediate by following analogous reaction protocol as described in Step - 3 of Example 4 (3.80 g, 67% yield)

$^1H$ NMR (400 MHz, DMSO-$d_6$) δ 8.13 - 8.07 (m, 1H), 8.04 - 7.99 (m, 1H), 7.76 - 7.72 (m, 1H), 7.67 - 7.60 (m, 1H), 5.41 (s, 1H), 2.62 (s, 3H), 1.21 (s, 3H), 1.14 (s, 3H).

Step - 3: (R)-N-(1-(3-(1,1-Difluoro-2-hydroxy-2-methylpropyl)phenyl)ethylidene)-2-methylpropane-2-sulfinamide

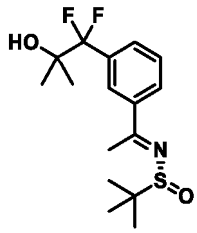

The titled compounds were synthesized from Step - 2 intermediate by following analogous reaction protocol as described in Step - 4 of Example 4. (4.0 g, 72.5% yield)
MS(ES+) m/z = 332.28 (M+1).

Step - 4: (R/S)-2-methyl-N-((R/S)-1-(3-(1,1,2,2-tetrafluoro-2-hydroxyethyl)phenyl)ethyl)propane-2-sulfinamide

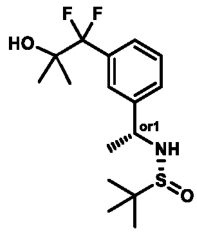

The titled compounds were synthesized from Step - 3 intermediate by following analogous reaction protocol as described in Step - 5 of Example 4 (1.6 g, 39.5% yield)
MS(ES+) m/z = 334.11 (M+1).

Step - 5: (R/S)-1-(3-(1-Aminoethyl)phenyl)-1,1-difluoro-2-methylpropan-2-ol hydrochloride

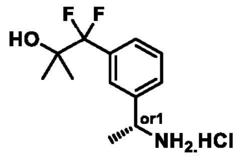

The titled compounds were synthesized from Step - 4 intermediate by following analogous reaction protocol as described in Step - 6 of Example 4 (1.1 g, 86% yield)

$^1$H NMR (400 MHz, DMSO-$d_6$) δ 8.68 - 8.53 (m, 3H), 7.69 - 7.64 (m, 1H), 7.63 - 7.59 (m, 1H), 7.55 - 7.45 (m, 2H), 5.32 (s, 1H), 4.57 - 4.38 (m, 1H), 1.53 (d, $J$ = 6.8 Hz, 3H), 1.22 - 1.13 (m, 6H).

**Example 11:** (R/S)-2-(3-(1-Aminoethyl)-2-methylphenyl)-2,2-difluoroethan-1-ol hydrochloride

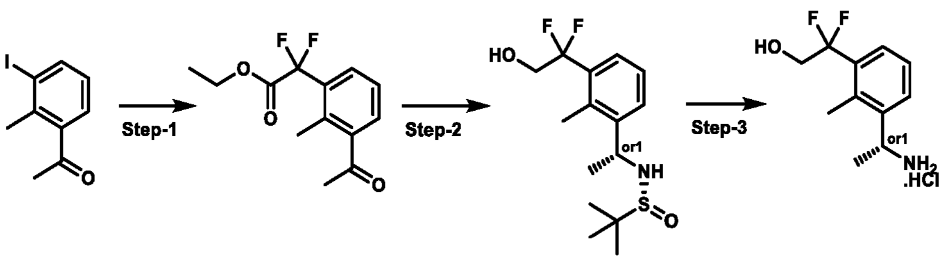

Step-1: Ethyl 2-(3-acetyl-2-methylphenyl)-2,2-difluoroacetate

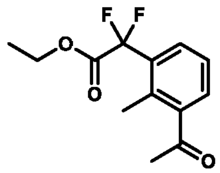

The title compound was synthesized by using 1-(3-iodo-2-methylphenyl)ethan-1-one (commercially available) and following analogous reaction protocol as described in Step - 3 of Example 4 (11.5 g, 90% yield).

MS(ES+) m/z = 257.2 (M+1)

Step-2: (R/S)-N-((R)-1-(3-(1,1-Difluoro-2-hydroxyethyl)-2-methylphenyl)ethyl)-2-methylpropane-2-sulfinamide

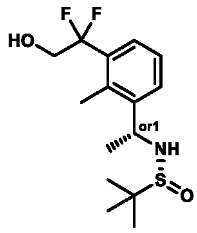

To a stirred solution of ethyl 2-(3-acetyl-2-methylphenyl)-2,2-difluoroacetate (Step - 1 product of Example 11) (10 g, 39.0 mmol) and (R)-2-methylpropane-2-sulfinamide (5.20 g, 42.9 mmol) in THF (100 mL) was added a under nitrogen atmosphere and reaction mass was heated to 80 °C for 16 h. After complete consumption of starting material, reaction mixture was allowed to cooled to -78 °C, followed by addition of sodium borohydride (5.17 g, 137 mmol) then temperature of the mixture was gradually raised to room temperature. The reaction was again monitored by TLC, it showed complete consumption of intermediate imine formed. Poured the reaction mass into ice cold water, then to it added Ethyl acetate (200 mL) and stirred for 30 min. Formed suspension was filtered, the residue was washed with ethyl acetate (50 mL x 3). The separated the two layers of filtrate and aqueous again washed with ethyl acetate (100 ml). The combined organic layers was dried over anhydrous $Na_2SO_4$ and concentrated under reduced pressure to get the crude product and it was purified by flash column chromatography using eluent 20% EtOAc in hexane to get the titled compound (R)-N-((R/S)-1-(3-(1,1-difluoro-2-hydroxyethyl)-2-methylphenyl)ethyl)-2-methylpropane-2-sulfinamide (2.5 g, 20.06 % yield).

MS(ES+) m/z = 320.03 (M+1).

Step-3: (R/S)-2-(3-(1-Aminoethyl)-2-methylphenyl)-2,2-difluoroethan-1-ol hydrochloride

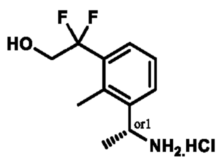

The title compound was synthesized by using Step - 2 product of Intermediate 11 and following analogous reaction protocol as described in Step - 6 of Example 4. (1.2 g, 70% yield)

$^1$H NMR (400 MHz, DMSO-$d_6$) δ 8.72 - 8.58 (m, 3H), 7.86 - 7.73 (m, 1H), 7.53 - 7.45 (m, 1H), 7.44 - 7.36 (m, 1H), 4.72 - 4.54 (m, 1H), 3.90 (t, *J* = 14.5 Hz, 2H), 2.43 - 2.39 (m, 3H), 1.49 - 1.46 (m, 3H).

**Example 12: -** (R/S)-1-(3,3-Difluoro-2,3-dihydrobenzofuran-7-yl)ethan-1-amine

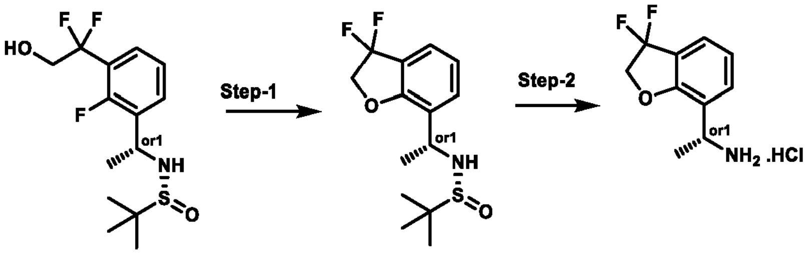


Step-1: (R)-N-((R/S)-1-(3,3-Difluoro-2,3-dihydrobenzofuran-7-yl)ethyl)-2-methylpropane-2-sulfinamide

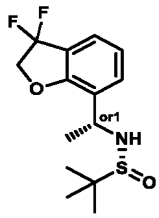

To a stirred solution of (R)-N-((R/S)-1-(3-(1,1-difluoro-2-hydroxyethyl)-2-fluorophenyl)ethyl)-2-methylpropane-2-sulfinamide (1.0 g, 3.09 mmol) ( Step - 3 product of Example 5) in THF (5.0 mL) was added 18-crown-6 (0.409 g, 1.546 mmol) and cesium carbonate (3.02 g, 9.28 mmol) under nitrogen atmosphere and heated the reaction mass at 80 °C for 16 h. Reaction was quenched with water and extracted with Ethyl acetate (30.0 mL). organic layer was separated, dried over anhydrous $Na_2SO_4$ and concentrated under reduced pressure to get crude residue. The crude residue was purified by flash chromatography using eluent 40-50% Ethyl acetate: n-hexane to afford titled compound (0.69 g, 76%)

(MS(ES+) m/z = 304.34 (M+1)

Step-2: (R/S)-1-(3,3-Difluoro-2,3-dihydrobenzofuran-7-yl)ethan-1-amine

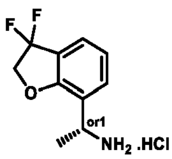

The titled compound was synthesized from Step-1 intermediate by following analogous reaction protocol as described in Step 6 of Example 4 (0.35 g, 90% yield).

MS(ES+) m/z = 198.18 (M+).

$^1$H NMR (400 MHz, DMSO-$d_6$) δ 8.60 (s, 2H), 7.80 - 7.73 (m, 1H), 7.73 - 7.63 (m, 1H), 7.22 (t, *J* = 7.6 Hz, 1H), 4.95 - 4.81 (m, 2H), 4.55 - 4.50 (m, 1H), 1.53 (d, *J*= 6.9 Hz, 3H).

**Example 13:** Synthesis of (R/S)-2-(3-((S/R)-1-aminoethyl)-2-fluorophenyl)-3,3,3-trifluoropropane-1,2-diol hydrochloride

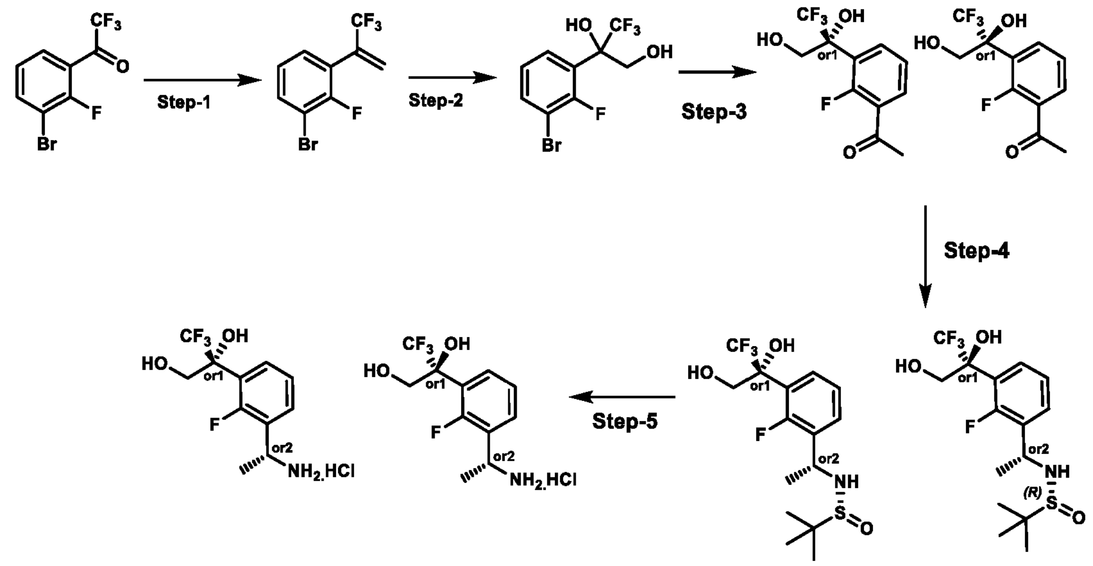


Step 1: 1-Bromo-2-fluoro-3-(3,3,3-trifluoroprop-1-en-2-yl)benzene

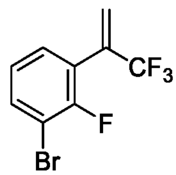

To a stirred solution of methyl triphenyl phosphonium bromide (16.81 g, 47.0 mmol) in THF (20.0 mL), n-butyllithium (18.07 mL, 45.2 mmol) was added at 0° C. The mixture was stirred at 0 °C for 10 min under a nitrogen atmosphere. The reaction mixture was cooled to -78°C and a solution of 1-(3-bromo-2-fluorophenyl)-2,2,2-trifluoroethan-1-one (10.2 g, 37.6 mmol) in THF (20.0 mL) was added in dropwise manner. The reaction was warmed to room temperature and stirred further for 1h. Reaction was quenched with Sat. $NH_4Cl$ solution (5.0 mL) and extracted with Ethyl acetate (10.0 mL x 3). The combined organic layer was washed with brine (5.0 mL), dried over anhydrous $Na_2SO_4$ and concentrated to give crude residue. The crude material was purified by flash chromatography using eluent 10 - 20% Ethyl acetate: n-hexane to afford titled compound (8.5 g, 84 % yield)

MS(ES+) m/z = 268.06, 270.06 (M, M+2)

Step 2: 2-(3-Bromo-2-fluorophenyl)-3,3,3-trifluoropropane-1,2-diol

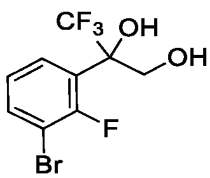

To a stirred solution of 1-bromo-2-fluoro-3-(3,3,3-trifluoroprop-1-en-2-yl)benzene (8.2 g, 30.5 mmol) in acetone (80.0 mL) and water (20.0 mL) was added 4-methylmorpholine N-oxide (7.14 g, 61.0 mmol) and potassium osmate dihydrate (0.674 g, 1.82 mmol) at room temperature. The resulting mixture was stirred at room temperature for 24 h. The reaction mixture was diluted with Ethyl acetate (100.0 mL) and washed with sat. $NaHCO_3$ solution. Organic layer was separated, dried over anhydrous $Na_2SO_4$ and concentrated to give crude product. The crude compound was purified by flash chromatography using eluent 0 - 60% of Ethyl acetate in n-hexane to afford titled compound (9.1 g, 99 % yield)

$^1H$ NMR (400 MHz, DMSO-$d_6$) δ 7.82 - 7.70 (m, 2H), 7.28 - 7.18 (m, 1H), 6.87 (s, 1H), 5.25 (t, $J$ = 5.7 Hz, 1H), 4.27 - 4.13 (m, 1H), 4.01 - 3.89 (m, 1H).

Step 3: (R&S)-1-(2-fluoro-3-(1,1,1-trifluoro-2,3-dihydroxypropan-2-yl)phenyl)ethan-1-one

The titled compounds were synthesized from Step - 2 intermediate by following analogous reaction protocol as described in Step - 3 of Example 4 and the enantiomers were separated by chiral preparative HPLC using HPLC method - Chiral HPLC Mobile phase A Hex+0.1% DEA Mobile phase B : IPA -MeOH(1:1) + 0.1% DEA

**a. Peak 1: (R/S)-1-(2-fluoro-3-(1,1,1-trifluoro-2,3-dihydroxypropan-2-yl)phenyl)ethan-1-one**

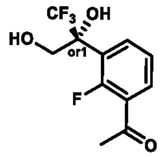

(Peak-1: 3.4 g, 41% yield, $T_{ret}$-3.75min, chiral purity 99.62%)
$^1$H NMR (400 MHz, DMSO-$d_6$) δ 8.05 - 7.94 (m, 1H), 7.86 - 7.78 (m, 1H), 7.38 (t, J = 7.8 Hz, 1H), 6.87 (s, 1H), 5.25 (s, 1H), 4.30 - 4.10 (m, 1H), 4.08 - 3.94 (m, 1H), 2.58 (d, J = 4.2 Hz, 3H).

**b. Peak 2: (S/R)-1-(2-fluoro-3-(1,1,1-trifluoro-2,3-dihydroxypropan-2-yl)phenyl)ethan-1-one**

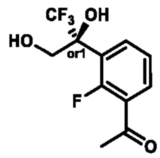

Peak-2: (3.4 g 41% yield, $T_{ret}$-4.73min, chiral purity: 99.85)
$^1$H NMR (400 MHz, DMSO-$d_6$) δ 8.05 - 7.94 (m, 1H), 7.86 - 7.75 (m, 1H), 7.38 (t, *J*= 7.8 Hz, 1H), 6.88 (s, 1H), 5.26 (s, 1H), 4.30 - 4.10 (m, 1H), 4.08 - 3.94 (m, 1H), 2.58 (d, *J*= 4.2 Hz, 3H).

Step 4a: (R)-N-((R/S)-1-(3-((R/S)-1,1-difluoro-2,3-dihydroxy-2-methylpropyl)-2-fluorophenyl)ethyl)-2-methylpropane-2-sulfinamide

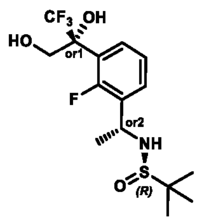

The titled compounds were synthesized from Step - 3 (peak - 1) intermediate by following analogous reaction protocol as described in Step - 2 Example 11 (0.60 g, 15.36% yield). Major isomer was taken forward. MS(ES+) m/z = 372.02 (M+1).

**(Major isomer of Peak 1) :** $^1$H NMR (400 MHz, DMSO-*d6*) δ 7.68 - 7.61 (m, 1H), 7.59 - 7.52 (m, 1H), 7.24 (t, *J*= 7.8 Hz, 1H), 6.61 (s, 1H), 5.84 (d, *J* = 7.2 Hz, 1H), 5.14 (t, *J*= 5.9 Hz, 1H), 4.71 - 4.62 (m, 1H), 4.20 - 4.11 (m, 1H), 4.06 - 3.92 (m, 1H), 1.40 (d, *J* = 6.8 Hz, 3H), 1.09 (s, 9H).

HPLC acetonitrile-water (9:1) +0.1 %formic acid $T_{ret}$: 1.47min

Step 4b: (R)-N-((R/S)-1-(3-((S/R)-1,1-difluoro-2,3-dihydroxy-2-methylpropyl)-2-fluorophenyl)ethyl)-2-methylpropane-2-sulfinamide

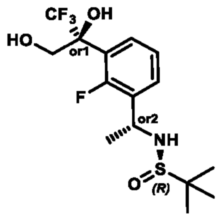

The titled compounds were synthesized from peak-2 of Step - 3 intermediate by following analogous reaction protocol as described in Step - 2 of Example 11 (0.90 g, 23.04% yield). Major isomer was taken forward. MS(ES+) m/z = 372.04(M+1).

**(Major isomer of Peak 2) :** $^{1}$H NMR (400 MHz, DMSO-$d_6$) δ 7.67 - 7.61 (m, 1H), 7.59 - 7.53 (m, 1H), 7.24 (t, *J* = 7.8 Hz, 1H), 6.63 (s, 1H), 5.79 (d, *J* = 7.8 Hz, 1H), 5.15 (t, *J* = 5.9 Hz, 1H), 4.71 - 4.63 (m, 1H), 4.19 - 4.09 (m, 1H), 4.05 - 3.98 (m, 1H), 1.39 (d, *J* = 6.8 Hz, 3H), 1.11 (s, 9H).

HPLC acetonitrile-water (9:1) +0.1 %formic acid $T_{ret}$: 1.56min

Step 5a: (R/S)-3-(3-((R/S)-1-aminoethyl)-2-fluorophenyl)-3,3-difluoro-2-methylpropane-1,2-diol hydrochloride

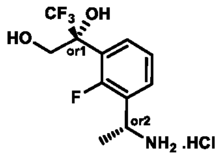

The titled compounds were synthesized by using step 4a intermediate following analogous reaction protocol as described in Step - 6 of Example 4 (0.55 g , 96% yield)
MS(ES+) m/z = 268.92 (M+1)

**(Major isomer of Step-5a)** : $^{1}$H NMR (400 MHz, DMSO-$d_6$) δ 8.66 (bs, 3H), 7.81 - 7.75 (m, 1H), 7.74 -7.69 (m, 1H), 7.35 (t, *J* = 7.8 Hz, 1H), 4.69 - 4.55 (m, 1H), 4.25 - 4.16 (m, 1H), 4.05 - 3.95 (m, 1H), 3.57 (s, 2H), 1.52 *(d, J* = 6.8 Hz, 3H).

Step 5b: (R/S)-3-(3-((S/R)-1-aminoethyl)-2-fluorophenyl)-3,3-difluoro-2-methylpropane-1,2-diol hydrochloride

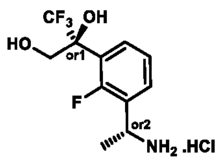

The titled compounds were synthesized by following analogous reaction protocol as described in Step - 6 of Example 4 preparation using Step - 4b. (0.70 g, 95% yield)
MS(ES+) m/z = 268.91 (M+1)

**(Major isomer of Step-5b)** : $^{1}$H NMR (400 MHz, DMSO-$d_6$) δ 7.82 - 7.73 (m, 1H), 7.68 - 7.62 (m, 1H), 7.38 - 7.31 (m, 1H), 4.68 - 4.57 (m, 1H), 4.21 - 4.15 (m, 1H), 4.04 - 3.98 (m, 1H), 3.57 (s, 2H), 1.51 (d, *J* = 6.8 Hz, 3H).

**Example 14:** Preparation of (R)-4-((1-(3-amino-5-(trifluoromethyl)phenyl)ethyl)amino)-2,6-dimethyl-6H-[1,4]oxazino [3,2-g]quinazolin-7(8H)-one **(Compound 1)**

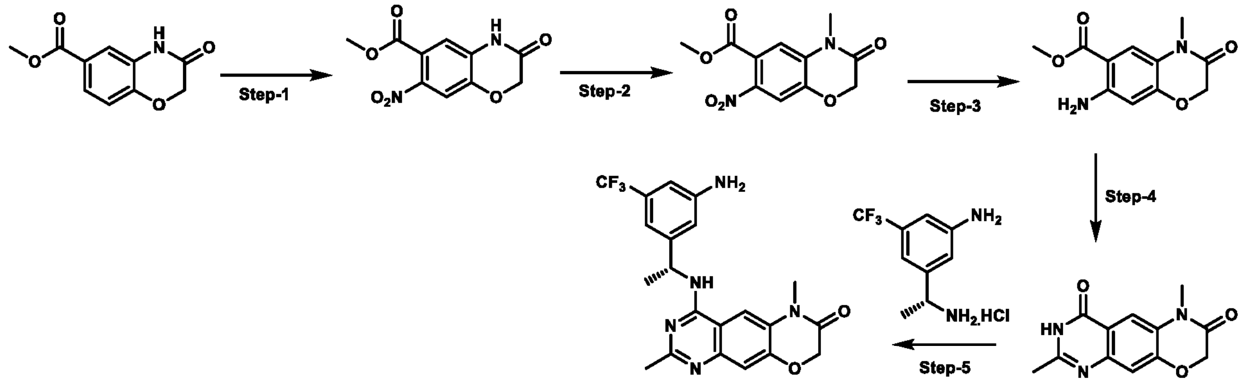


**Step** 1: Methyl 7-nitro-3-oxo-3,4-dihydro-2H-benzo[b][1,4]oxazine-6-carboxylate

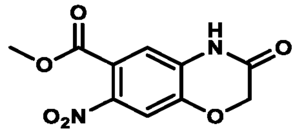

To a stirred solution of methyl 3-oxo-3,4-dihydro-2H-benzo[b][1,4]oxazine-6-carboxylate (10.0 g, 48.3 mmol) (Bioorganic and Medicinal Chemistry Letters, 2016, vol. 26, # 6, p. 1571 - 1575) in acetic acid (200.0 mL), fuming nitric acid (88.0 mL, 1931.0 mmol) was added slowly at 0 °C under inert atmosphere. The reaction was stirred for 2 h at same temperature. The reaction was poured in ice-cold water (500.0 mL) and stirred for 30 min. The resulting precipitate was filtered off, washed with ice-cold water (500.0 mL) and dried under reduced pressure to afford the titled compound (12.0 g, 99.0 %) as a white solid.
GCMS m/z = 252.14 (M+).
$^1$H NMR (400 MHz, DMSO-$d_6$) δ 11.32 (s, 1H), 7.68 (s, 1H), 7.23 (s, 1H), 4.79 (s, 2H), 3.82 (s, 3H).

**Step 2:** Methyl 4-methyl-7-nitro-3-oxo-3, 4-dihydro-2H-benzo[b][1,4]oxazine-6-carboxylate

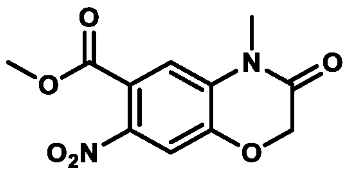

To a stirred solution of methyl 7-nitro-3-oxo-3,4-dihydro-2H-benzo[b][1,4]oxazine-6-carboxylate (2.0 g, 7.93 mmol) in DMF (20.0 mL) was added $K_2CO_3$ (4.38 g, 31.7 mmol), potassium iodide (0.26 g, 1.586 mmol) and iodomethane (2.48 mL, 39.7 mmol) and reaction mass were heated at 50 °C for 16 h. After completion of reaction, reaction mixture was quenched in water (100.0 mL) and extracted with ethyl acetate (150.0 mL), the organic layer was washed with water (100.0 ml), brine (100.0 mL) and dried over anhydrous $Na_2SO_4$, filtered and concentrated in vacuo to give crude compound. This crude residue was purified by flash chromatography using eluent 0 - 30% ethyl acetate in n-hexane to afford the title compound methyl 4-methyl-7-nitro-3-oxo-3,4-dihydro-2H-benzo[b][1,4] oxazine-6-carboxylate (1.6 g, 76.0 % yield) as an off white solid.
GCMS m/z = 266.05 (M+).
$^1$H NMR (400 MHz, DMSO-$d_6$) δ 7.74 (s, 1H), 7.52 (s, 1H), 4.87 (s, 2H), 3.85 (s, 3H), 3.34 (s, 3H).

**Step 3:** Methyl 7-amino-4-methyl-3-oxo-3,4-dihydro-2H-benzo[b][1,4]oxazine-6-carboxylate

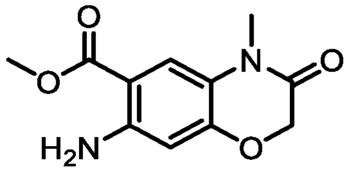

To a suspension of methyl 4-methyl-7-nitro-3-oxo-3,4-dihydro-2H-benzo[b][1,4]oxazine-6-carboxylate (1.5 g, 5.63 mmol) in acetic acid (30.0 mL) was added iron (0.94 g, 16.90 mmol) with stirring at 90 °C for 30 min and then the mixture was filtered. The resulting filtrate was washed with aqueous $K_2CO_3$ solution (100.0 mL), then aqueous layer was extracted with ethyl acetate (100.0 mL x 2), dried over anhydrous $Na_2SO_4$, filtered and concentrated in vacuo to give methyl 7-amino-4-methyl-3-oxo-3,4-dihydro-2H-benzo[b][1,4]oxazine-6-carboxylate (1.2 g, 90 % yield) as an off white solid.

GCMS m/z = 236.14 (M+)

$^1$H NMR (400 MHz, DMSO-$d_6$) $\delta$ 7.33 (s, 1H), 6.63 (s, 2H), 6.40 (s, 1H), 4.64 (s, 2H), 3.79 (s, 3H), 3.22 (s, 3H).

**Step 4:** 2,6-Dimethyl-3,6-dihydro-4H-[1,4]oxazino[3,2-g]quinazoline-4,7(8H)-dione

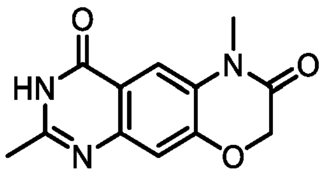

To a stirred solution of methyl 7-amino-4-methyl-3-oxo-3,4-dihydro-2H-benzo[b][1,4]oxazine-6-carboxylate (1.2 g, 5.08 mmol) in acetonitrile (20.0 mL) was added methane sulfonic acid (2.31 mL, 35.6 mmol) at 25°C and the reaction was stirred at 115 °C for 16 h in a sealed tube. After completion of reaction, the reaction mass was evaporated, and the residue was diluted with 30.0 mL water and neutralized with saturated aqueous sodium hydroxide solution to get the white precipitate. Precipitate was filtered, washed with water (15.0 mL) and the residue was dried to afford the titled compound 4-hydroxy-2,6-dimethyl-6H-[1,4]oxazino[3,2-g]quinazolin-7(8H)-one (0.780 g, 62.6 % yield) as an off white solid.

MS (ES+) m/z = 246.33 (M+1)

$^1$H NMR (400 MHz, DMSO-$d_6$) $\delta$ 12.17 (s, 1H), 7.63 (s, 1H), 7.10 (s, 1H), 4.80 (s, 2H), 3.36 (s, 3H), 2.32 (s, 3H).

**Step 5:** (R)-4-((1-(3-amino-5-(trifluoromethyl)phenyl)ethyl)amino)-2,6-dimethyl-6H-[1,4]oxazino[3,2-g]quinazolin-7(8H)-one

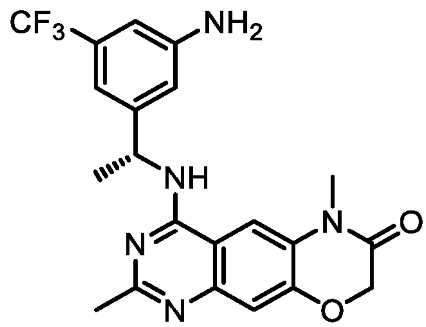

To a stirred solution of 2,6-Dimethyl-3,6-dihydro-4H-[1,4]oxazino[3,2-g]quinazoline-4,7(8H)-dione (0.40 g, 1.63 mmol) in DMF (15.0 mL) was added DBU (1.23 mL, 8.16 mmol), BOP (0.72 g, 1.63 mmol) followed by addition of (R)-3-(1-aminoethyl)-5-(trifluoromethyl)aniline hydrochloride (0.59 g, 2.447 mmol) and reaction mass heated to 100 °C for 16 h. After completion of reaction, reaction mass was concentrated in vacuo to give crude compound. This crude residue was purified by flash chromatography using eluent 0 - 4% methanol in DCM to afford the title compound (R)-4-((1-(3-amino-5-(trifluoromethyl)phenyl)ethyl)amino)-2,6-dimethyl-6H-[1,4]oxazino[3,2-g]quinazolin-7(8H)-one (0.36 g, 51.2

% yield) as an off white solid.

MS (ES+) m/z = 432.17 (M+1).

$^1$H NMR (400 MHz, DMSO-*$d_6$*) δ 8.25 (d, *J* = 8.0 Hz, 1H), 7.95 (s, 1H), 7.09 (s, 1H), 6.91 - 6.83 (m, 2H), 6.71 (d, *J* = 1.9 Hz, 1H), 5.63-5.53 (m , 2H), 5.56 (s, 1H), 4.78 (s, 2H), 3.43 (s, 3H), 2.37 (s, 3H), 1.57 (d, *J* = 7.0 Hz, 3H).

**Examples 15 - 21** in **Table-1** were synthesized by following analogous reaction protocol as was used for the preparation of **Example 14** using appropriate chiral amines and commercially available chiral amine for example 20.

**Table-1**

| Example | Chemical structure | LCMS and $^1$H NMR data |
|---|---|---|
| **15** | 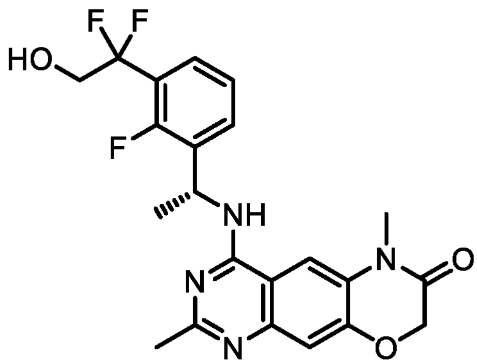 (R)-4-((1-(3-(1,1-difluoro-2-hydroxyethyl)-2-fluorophenyl)ethyl)amino)-2,6-dimethyl-6H-[1,4]oxazino[3,2-g]quinazolin-7(8H)-one **(Compound 2)** | MS (ES+) m/z = 447.17(M+1) $^1$H NMR (400 MHz, DMSO-*$d_6$*) δ 8.34 (d, *J* = 7.3 Hz, 1H), 7.99 (s, 1H), 7.68 - 7.62 (m, 1H), 7.46 - 7.39 (m, 1H), 7.29 - 7.23 (m, 1H), 7.09 (s, 1H), 5.84 - 5.76 (m, 1H), 5.72 (t, *J* = 6.5 Hz, 1H), 4.79 (s, 2H), 3.98 - 3.84 (m, 2H), 3.45 (s, 3H), 2.32 (s, 3H), 1.62 (d, *J* = 7.1 Hz, 3H). |
| **16** | 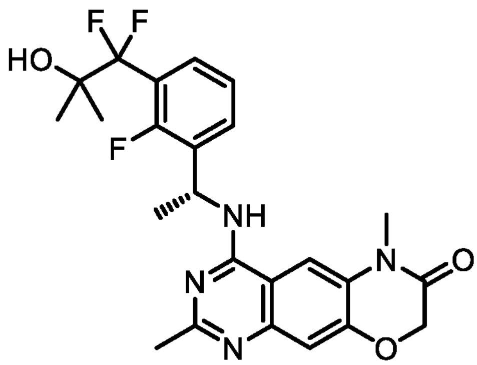 (R)-4-((1-(3-(1,1-difluoro-2-hydroxy-2-methylpropyl)-2-fluorophenyl)ethyl)amino)-2,6-dimethyl-6H-[1,4]oxazino[3,2-g]quinazolin-7(8H)-one **(Compound 3)** | MS (ES+) m/z = 475.20 (M+1) $^1$H NMR (400 MHz, DMSO-*$d_6$*) δ 8.34 (d, *J* = 7.3 Hz, 1H), 8.00 (s, 1H), 7.62 - 7.56 (m, 1H), 7.34 - 7.28 (m, 1H), 7.25 - 7.18 (m, 1H), 7.09 (s, 1H), 5.85 - 5.76 (m, 1H), 5.34 (s, 1H), 4.79 (s, 2H), 3.46 (s, 3H), 2.30 (s, 3H), 1.61 (d, *J* = 7.0 Hz, 3H), 1.24 (s, 3H), 1.21 (s, 3H) |
| **17** | 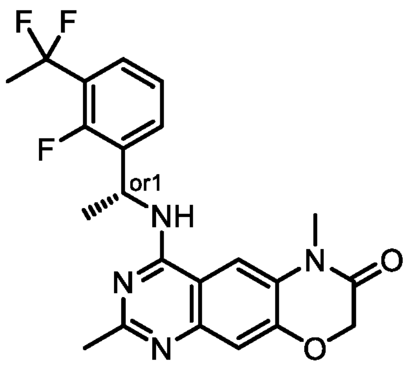 (R/S)-4-((1-(3-(1,1-Difluoroethyl)-2-fluorophenyl)ethyl)amino)-2,6-dimethyl-6H-[1,4]oxazino[3,2-g]quinazolin-7(8H)-one **(Compound 4)** | MS (ES+) m/z = 431.10 (M+1) $^1$H NMR (400 MHz, DMSO-*$d_6$*) δ 8.39 (d, *J* = 7.1 Hz, 1H), 7.99 (s, 1H), 7.65 - 7.58 (m, 1H), 7.46 - 7.42 (m, 1H), 7.29 - 7.21 (m, 1H), 7.09 (s, 1H), 5.85 - 5.78 (m, 1H), 4.79 (s, 2H), 3.45 (s, 3H), 2.32 (s, 3H), 2.04 (t, *J* = 19.1 Hz, 3H), 1.63 (d, *J* = 7.0 Hz, 3H). |

(continued)

| Example | Chemical structure | LCMS and $^1$H NMR data |
|---|---|---|
| **18** | 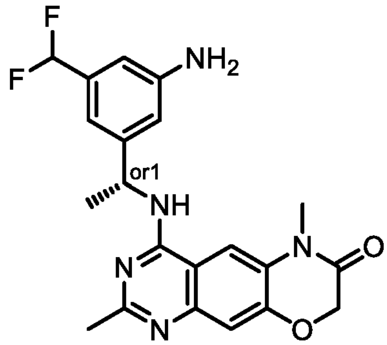 (R/S)-4-((1-(3-Amino-5-(difluoromethyl)phenyl)ethyl)amino)-2,6-dimethyl-6H-[1,4]oxazino[3,2-g]quinazolin-7(8H)-one **(Compound 5)** | MS (ES+) m/z = 414.16 (M+1) $^1$H NMR (400 MHz, DMSO-$d_6$) δ 8.24 (d, *J* = 8.0 Hz, 1H), 7.97 (s, 1H), 7.09 (s, 1H), 6.83 (t, *J* = 56 Hz, 1H), 6.79 - 6.75 (m, 2H), 6.59 (bs, 1H), 5.62 - 5.57 (m, 1H), 5.36 (s, 2H), 4.78 (s, 2H), 3.43 (s, 3H), 2.37 (s, 3H), 1.56 (d, *J* = 7.1 Hz, 3H). |
| **19** | 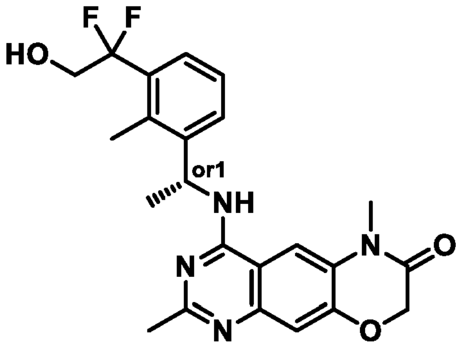 (R/S)-4-((1-(3-(1,1-Difluoro-2-hydroxyethyl)-2-methylphenyl)ethyl) amino)-2,6-dimethyl-6H-[1,4]oxazino[3,2-g]quinazolin-7(8H)-one **(Compound 6)** | MS (ES+) m/z = 443.17 (M+1) $^1$H NMR (400 MHz, DMSO-$d_6$) δ 8.39 (d, J = 7.2 Hz, 1H), 7.98 (s, 1H), 7.62 (d, J = 7.7 Hz, 1H), 7.37 - 7.31 (m, 1H), 7.29 - 7.22 (m, 1H), 7.07 (s, 1H), 5.79 - 5.74 (m, 1H), 5.67 (t, *J* = 6.4 Hz, 1H), 4.77 (s, 2H), 3.95 - 3.86 (m, 2H), 3.45 (s, 3H), 2.59 (s, 3H), 2.32 (s, 3H), 1.56 (d, *J* = 7.0 Hz, 3H). |
| **20** | 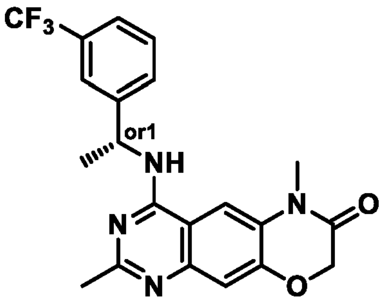 (R/S)-2,6-dimethyl-4-((1-(3-(trifluoromethyl)phenyl)ethyl)amino)-6H-[1,4]oxazino[3,2-g]quinazolin-7(8H)-one **(Compound 7)** | MS (ES+) m/z = 417.2 (M +1) $^1$H NMR (400 MHz, DMSO-$d_6$) δ 8.34 (d, *J* = 7.7 Hz, 1H), 7.95 (s, 1H), 7.81 (s, 1H), 7.78 - 7.73 (m, 1H), 7.63 - 7.53 (m, 2H), 7.10 (s, 1H), 5.73 - 5.63 (m, 1H), 4.78 (s, 2H), 3.44 (s, 3H), 2.34 (s, 3H), 1.64 (d, *J* = 7.1 Hz, 3H). |

(continued)

| Example | Chemical structure | LCMS and $^1$H NMR data |
|---|---|---|
| **21** | 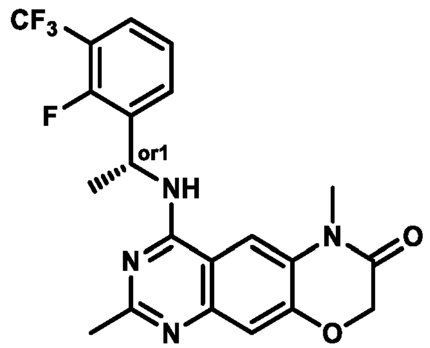 (R/S)-4-((1-(2-fluoro-3-(trifluoromethyl)phenyl)ethyl)amino)-2,6-dimethyl-6H-[1,4]oxazino[3,2-g]quinazolin-7(8H)-one.**(Compound 8)** | MS (ES+) m/z = 435.10 (M+1) $^1$H NMR (400 MHz, DMSO-$d_6$) δ 8.39 (d, *J* = 6.9 Hz, 1H), 7.98 (s, 1H), 7.82 - 7.77 (m, 1H), 7.69 - 7.61 (m, 1H), 7.40 - 7.33 (m, 1H), 7.09 (s, 1H), 5.81 - 5.72 (m, 1H), 4.79 (s, 2H), 3.46 (s, 3H), 2.29 (s, 3H), 1.65 (d, *J* = 7.1 Hz, 3H). |

Example 22: Preparation of (R/S)-4-((1-(3,3-Difluoro-2,3-dihydrobenzofuran-7-yl)ethyl)amino)-2,6-dimethyl-6H-[1,4]oxazino[3,2-g]quinazolin-7(8H)-one **(Compound 9)**

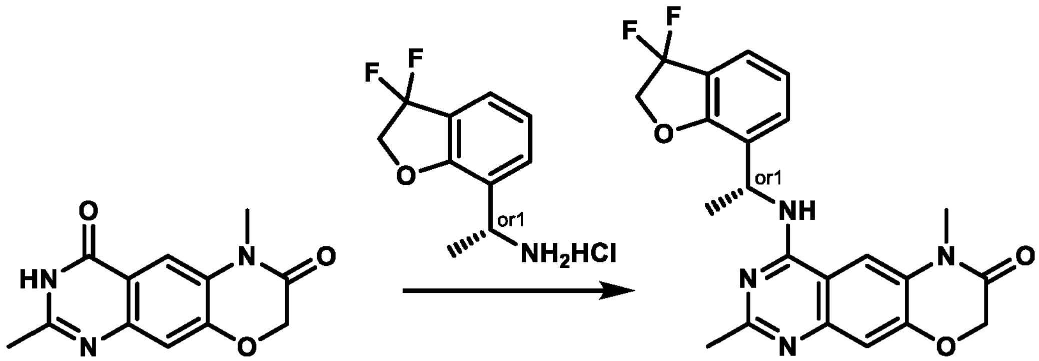

To a suspension of 2,6-dimethyl-3,6-dihydro-4H-[1,4]oxazino[3,2-g]quinazoline-4,7(8H)-dione (0.17 g, 0.69 mmol) (Example 14 step 4 intermediate), potassium carbonate (0.283 g, 2.08 mmol) in acetonitrile (3.0 mL) was added phosphonitrilic chloride trimer (0.241 g, 0.69 mmol) and reaction mass was stirred at 25 °C for 1 h, (R/S)-1-(3,3-difluoro-2,3-dihydrobenzofuran-7-yl)ethan-1-amine (0.097 g, 0.485 mmol) was added and reaction mass stirred for 1 h, aqueous ammonia (0.45 mL, 20.80 mmol) was added and stirred for 1 h, After that added sat aqueous $K_2CO_3$ solution (5.0 mL) and stirred reaction mass for 16 h at room temp, organic layer separated and concentrate to get crude compound. This crude residue was purified by flash chromatography using eluent 0 - 4% methanol in DCM to afford the title compound (R/S)-4-((1-(3,3-difluoro-2,3-dihydrobenzofuran-7-yl)ethyl)amino)-2,6-dimethyl-6H-[1,4]oxazino[3,2-g]quinazolin-7(8H)-one (0.012 g, 4.06 % yield) as an off white solid.

MS (ES+) m/z = 427.10 (M+1).

$^1$H NMR (400 MHz, DMSO-$d_6$) δ 8.27 (d, *J* = 7.6 Hz, 1H), 8.00 (s, 1H), 7.61 - 7.47 (m, 2H), 7.16 -7.03 (m, 2H), 5.84-5.76 (m , 1H), 4.88 (t, *J* = 16.7 Hz, 2H), 4.79 (s, 2H), 3.45 (s, 3H), 2.32 (s, 3H), 1.61 (d, J=7.0 Hz, 3H).

**Examples 23:** Preparation of (R)-N-(1-(3-amino-5-(trifluoromethyl)phenyl)ethyl)-2,6-dimethyl-7,8-dihydro-6H-[1,4]oxazino[3,2-g]quinazolin-4-amine **(Compound 10)**

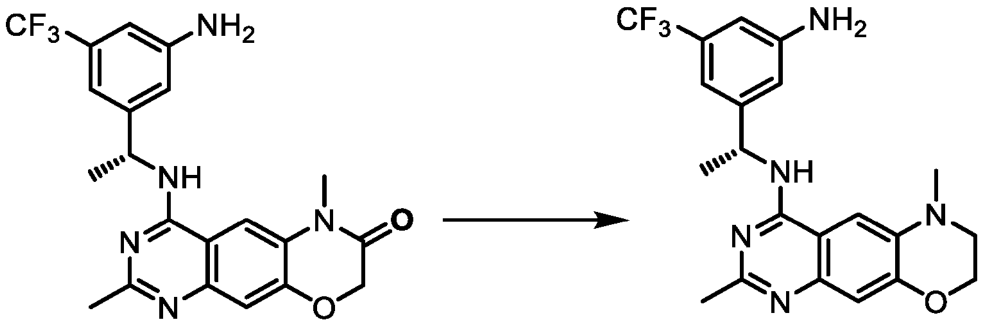

To a stirred solution of (R)-4-((1-(3-amino-5-(trifluoromethyl)phenyl)ethyl)amino)-2,6-dimethyl-6H-[1,4]oxazino [3,2-g]quinazolin-7(8H)-one **(Compound 1)** in THF (5.0 mL) was added borane tetrahydrofuran complex (3.0 mL, 3.01 mmol) at 0 °C and then reaction mass was heated to 70 °C for 2 h. After completion of reaction, cooled reaction mass at 0 °C, quenched by adding methanol and filtered through celite, filtrate was extracted with ethyl acetate (50.0 mL x 2 ) and water (25.0 mL), dried over anhydrous $Na_2SO_4$, filtered and concentrated in vacuo to give crude compound. The crude compound was purified by reverse prep to afford the titled compound (R)-N-(1-(3-amino-5-(trifluoromethyl)phenyl) ethyl)-2,6-dimethyl-7,8-dihydro-6H-[1,4]oxazino[3,2-g]quinazolin-4-amine (0.07 g, 27.8 % yield) as an off white solid.

MS (ES+) m/z = 417.10 (M+).

$^1$H NMR (400 MHz, DMSO-$d_6$) δ 7.85 (d, *J* = 8.0 Hz, 1H), 7.33 (s, 1H), 6.89 - 6.83 (m, 2H), 6.80 (s, 1H), 6.69 (s, 1H), 5.60 - 5.54 (m, 3H), 4.38 - 4.30 (m, 2H), 3.32 - 3.27 (m, 2H), 3.00 (s, 3H), 2.31 (s, 3H), 1.54 (d, *J*= 7.1 Hz, 3H).

Example 24: Preparation of (R&S)-4-(((R)-1-(3-amino-5(trifluoromethyl)phenyl)ethyl)amino)-2-methyl-6-((tetrahydrofuran-3-yl)methyl)-6H-[1,4]oxazino[3,2-g]quinazolin-7(8H)-one (**Compound 11)**

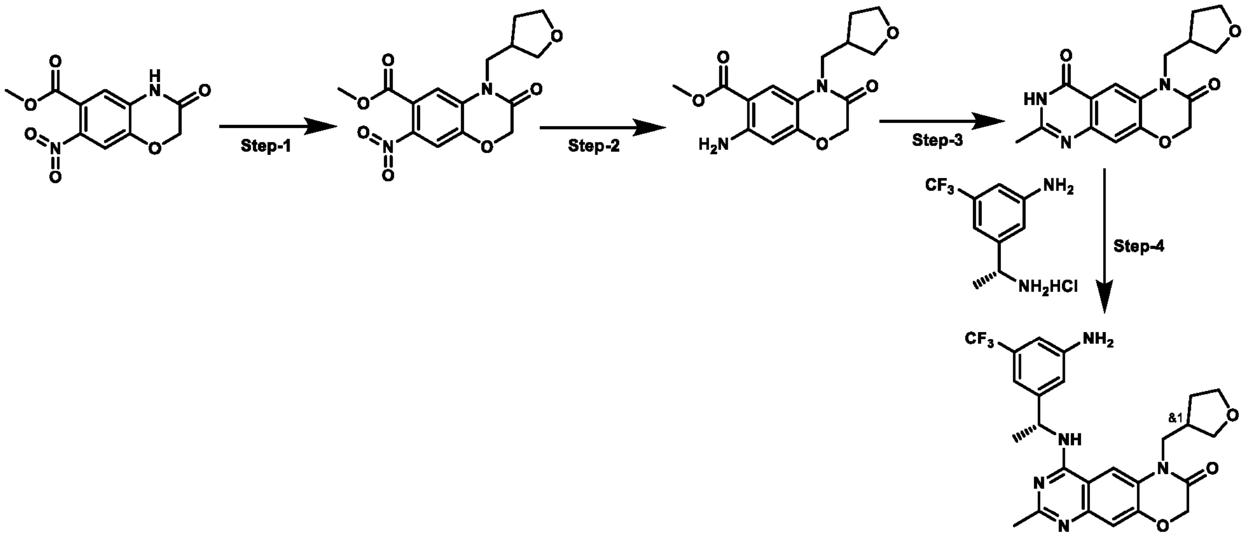


(11a) & (11b)

**Step 1**: (R&S)-Methyl 7-nitro-3-oxo-4-((tetrahydrofuran-3-yl) methyl)-3,4-dihydro-2H benzo[b][1,4]oxazine-6-carboxylate

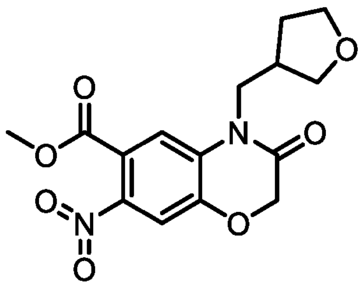

The titled compound was prepared from **Example 14** Step 1 intermediate & 3-(Bromomethyl) tetrahydrofuran by employing similar protocol mentioned in Example 14 Step 2 (1.1 g, 68.70 % yield).

MS (ES+) m/z = 337.1 (M+1).

$^1$H NMR (400 MHz, DMSO-$d_6$) δ 7.77 (s, 1H), 7.66 (s, 1H), 4.87 (s, 2H), 4.12 - 3.94 (m, 2H), 3.85 (s, 3H), 3.82 - 3.74 (m, 1H), 3.73 - 3.55 (m, 2H), 3.49 - 3.41 (m, 1H), 2.63 - 2.55 (m, 1H), 1.98 - 1.86 (m, 1H), 1.65 - 1.52 (m, 1H).

**Step 2:** (R&S)-Methyl 7-amino-3-oxo-4-((tetrahydrofuran-3-yl)methyl)-3,4-dihydro-2H-benzo[b] [1,4]oxazine-6-carboxylate

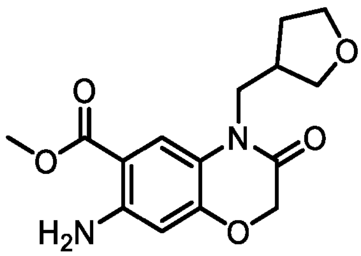

The titled compound was prepared from **Step 1** intermediate by employing similar protocol mentioned in **Example 14 Step 3** (0.85 g, 85.00 % yield).

MS (ES+) m/z = 307.2 (M+1).

$^1$H NMR (400 MHz, DMSO-$d_6$) δ 7.43 (s, 1H), 6.63 (s, 2H), 6.42 (s, 1H), 4.64 (s, 2H), 3.92 - 3.83 (m, 2H), 3.81 - 3.75 (m, 4H), 3.66 - 3.53 (m, 2H), 3.49 - 3.44 (m, 1H), 2.60 - 2.53 (m, 1H), 1.98 - 1.86 (m, 1H), 1.65 - 1.52 (m, 1H).

**Step 3:** (R&S)-2-Methyl-6-((tetrahydrofuran-3-yl) methyl)-3,6-dihydro-4H-[1,4]oxazino[3,2-g]quinazoline-4,7(8H)-dione

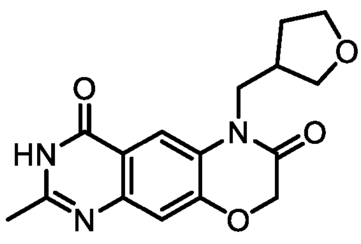

The titled compound was prepared from Step 2 intermediate by employing similar protocol mentioned in **Example 14 Step 4** (0.6 g, 72.90 % yield).

MS (ES+) m/z = 316.2 (M+1).

$^1$H NMR (400 MHz, DMSO-$d_6$) δ 12.21 (s, 1H), 7.74 (s, 1H), 7.13 (s, 1H), 4.81 (s, 2H), 4.07 - 4.03 (m, 2H), 3.85 - 3.75 (m, 1H), 3.67 - 3.53 (m, 2H), 3.55 - 3.47 (m, 1H), 2.65 - 2.55 (m, 1H), 2.32 (s, 3H), 1.97 - 1.85 (m, 1H), 1.73 - 1.52 (m, 1H).

**Step 4:** (R&S)- 4-(((R)-1-(3-amino-5(trifluoromethyl)phenyl)ethyl)amino)-2-methyl-6-((tetrahydrofuran-3-yl) methyl)-6H-[1,4]oxazino[3,2-g]quinazolin-7(8H)-one. **(Compound 11)**

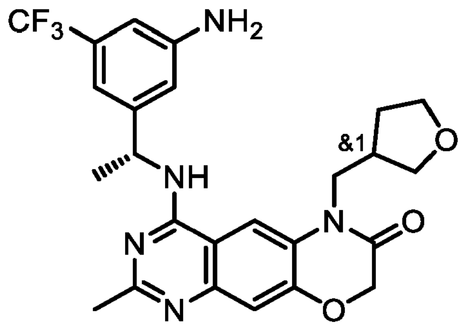

The titled compound was prepared from **Step 3** intermediate by employing similar protocol mentioned in

**Example 14 Step 5**

Chiral separation of the above compound gave two stereoisomers

**Peak1** 4-(((R)-1-(3-amino-5-(trifluoromethyl)phenyl)ethyl)amino)-2-methyl-6-(((R/S)-tetrahydrofuran-3-yl) methyl)-6H-[1,4]oxazino[3,2-g]quinazolin-7(8H)-one **(Compound 11a)**

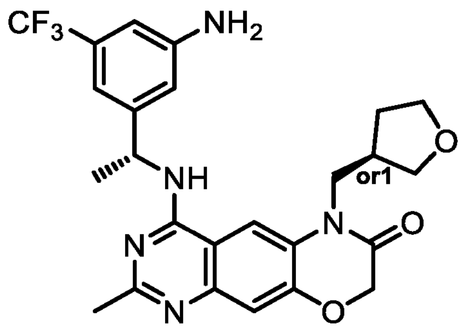

MS (ES+) m/z = 502.19 (M + 1).

$^1$H NMR (400 MHz, DMSO-$d_6$) δ 8.23 (d, $J$ = 8.1 Hz, 1H), 8.04 (bs, 1H), 7.11 (bs, 1H), 6.90 - 6.82 (m, 2H), 6.71 (bs, 1H), 5.69 - 5.53 (m, 3H), 4.78 (s, 2H), 4.30 - 4.08 (m, 2H), 3.85 - 3.75 (m, 1H), 3.72 - 3.55 (m, 2H), 3.53 - 3.40 (m, 1H), 2.67 - 2.57 (m, 1H), 2.37 (s, 3H), 1.96 - 1.81 (m, 1H), 1.68 - 1.54 (m, 4H).

Chiral HPLC: HEX 0.1%DEA:IPA-DCM(1:1)0.1% DEA(10:90) $t_{ret}$: 3.68 min

**Peak 2:** 4-(((R)-1-(3-amino-5-(trifluoromethyl)phenyl)ethyl)amino)-2-methyl-6-(((S/R)-tetrahydrofuran-3-yl) methyl)-6H-[1,4]oxazino[3,2-g]quinazolin-7(8H)-one **(Compound 11b)**

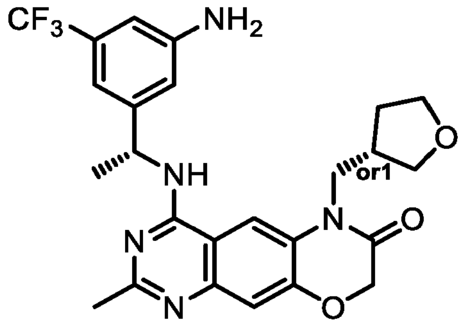

MS (ES+) m/z = 502.19 (M + 1).

$^1$H NMR (400 MHz, DMSO-$d_6$) δ 8.23 (d, *J* = 8.0 Hz, 1H), 8.04 (bs, 1H), 7.13 (s, 1H), 6.89 - 6.83 (m, 2H), 6.71 (bs, 1H), 5.69 - 5.54 (m, 3H), 4.78 (s, 2H), 4.31 - 4.08 (m, 2H), 3.85 - 3.75 (m, 1H), 3.72 - 3.55 (m, 2H), 3.53 - 3.40 (m, 1H), 2.67 - 2.57 (m, 1H), 2.37 (s, 3H), 1.96 - 1.81 (m, 1H), 1.67 - 1.54 (m, 4H).

Chiral HPLC: HEX 0.1%DEA:IPA-DCM(1:1)0.1% DEA(10:90) $t_{ret}$: 4.59 min

**Example 25:** Preparation of (R)-4-((1-(3-amino-5-(trifluoromethyl)phenyl)ethyl)amino)-6-(cyclopropylmethyl)-2-methyl-6H-[1,4]oxazino[3,2-g]quinazolin-7(8H)-one **(Compound 12)**

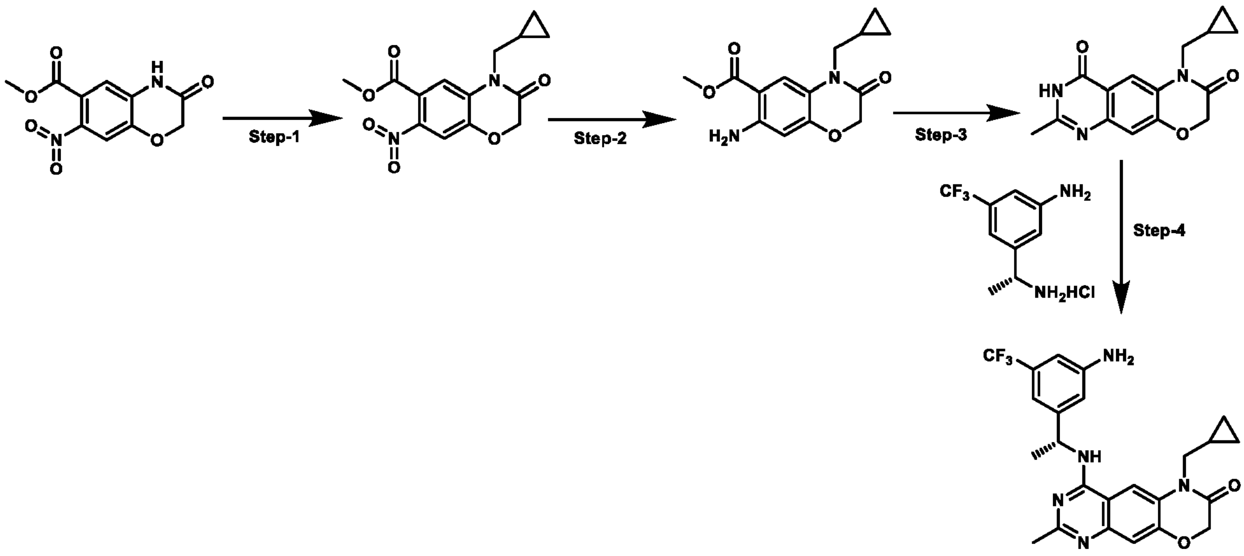


**Step** 1: Methyl 4-(cyclopropylmethyl)-7-nitro-3-oxo-3,4-dihydro-2H-benzo[b][1,4]oxazine-6-carboxylate

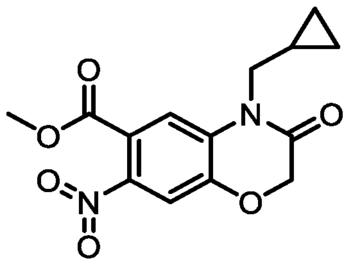

The titled compound was prepared from Example 14 Step 1 intermediate & (bromomethyl) cyclopropane by employing similar protocol mentioned in Example 14 Step 2 (1.69 g, 93.0 % yield)

$^1$H NMR (400 MHz, Chloroform-d) δ 7.58 (s, 1H), 7.42 (s, 1H), 4.77 (s, 2H), 3.95 (s, 3H), 3.92 (d, *J* = 7.0 Hz, 2H), 1.31- 1.25 (m, 1H), 0.64 - 0.57 (m, 2H), 0.52 - 0.46 (m, 2H).

Step 2: Methyl 7-amino-4-(cyclopropylmethyl)-3-oxo-3,4-dihydro-2H-benzo[b][1,4]oxazine-6-carboxylate

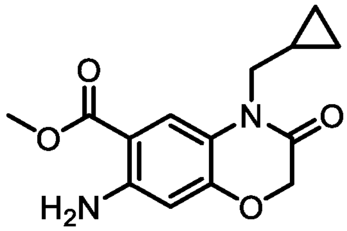

The titled compound was prepared from **Step 1** intermediate by employing similar protocol mentioned in **Example 14 Step 3** (1.61 g, 100%) crude yield.
MS (ES+) m/z = 277.2 (M+1).
$^1$H NMR (400 MHz, DMSO-$d_6$) δ 7.49 (s, 1H), 6.62 (s, 2H), 6.42 (s, 1H), 4.64 (s, 2H), 3.79 (s, 3H), 3.75 (d, J = 6.8 Hz, 2H), 1.12 - 0.92 (m, 1H), 0.53 - 0.44 (m, 2H), 0.40 - 0.29 (m, 2H).

**Step** 3: 6-(Cyclopropylmethyl)-2-methyl-3,6-dihydro-4H-[1,4]oxazino[3,2-g]quinazoline-4,7(8H)-dione

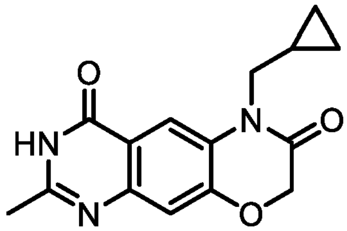

The titled compound was prepared from **Step 2** intermediate by employing similar protocol mentioned in **Example 14 Step 4** (0.89 g, 57.50 % yield).
MS (ES+) m/z = 286.27 (M + 1).
$^1$H NMR (400 MHz, DMSO-$d_6$) δ 12.18 (s, 1H), 7.78 (s, 1H), 7.13 (s, 1H), 4.81 (s, 2H), 3.92 (d, *J* = 6.8 Hz, 2H), 2.33 (s, 3H), 1.18 - 1.07 (m, 1H), 0.55 - 0.47 (m, 2H), 0.42 - 0.34 (m, 2H).

Step 4: (R)-4-((1-(3-amino-5-(trifluoromethyl)phenyl)ethyl)amino)-6-(cyclopropylmethyl)-2-methyl-6H-[1,4]oxazino[3,2-g]quinazolin-7(8H)-one **(Compound 12 )**

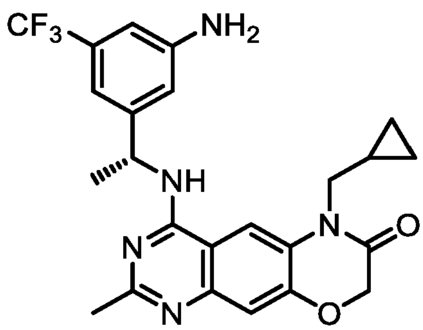

The titled compound was prepared from **Step** 3 intermediate by employing similar protocol mentioned in **Example 14 Step** 5 (0.51 g, 78.0 % yield).

MS (ES+) m/z = 472.23 (M+1).
$^1$H NMR (400 MHz, DMSO-$d_6$) δ 8.30 (d, *J* = 8.1 Hz, 1H), 8.09 (s, 1H), 7.12 (s, 1H), 6.90 - 6.83 (m, 2H), 6.71 (bs, 1H), 5.69 - 5.49 (m, 3H), 4.77 (s, 2H), 4.09 - 4.00 (m, 2H), 2.37 (s, 3H), 1.58 (d, *J*= 7.1 Hz, 3H), 1.23 - 1.20 (m, 1H), 0.60 - 0.19 (m, 4H).

**Example 26:** Preparation of (R)-4-((1-(3-(1,1-difluoro-2-hydroxyethyl)-2-fluorophenyl)ethyl)amino)-6-ethyl-2-methyl-6H-[1,4]oxazino[3,2-g]quinazolin-7(8H)-one **(Compound 13** )

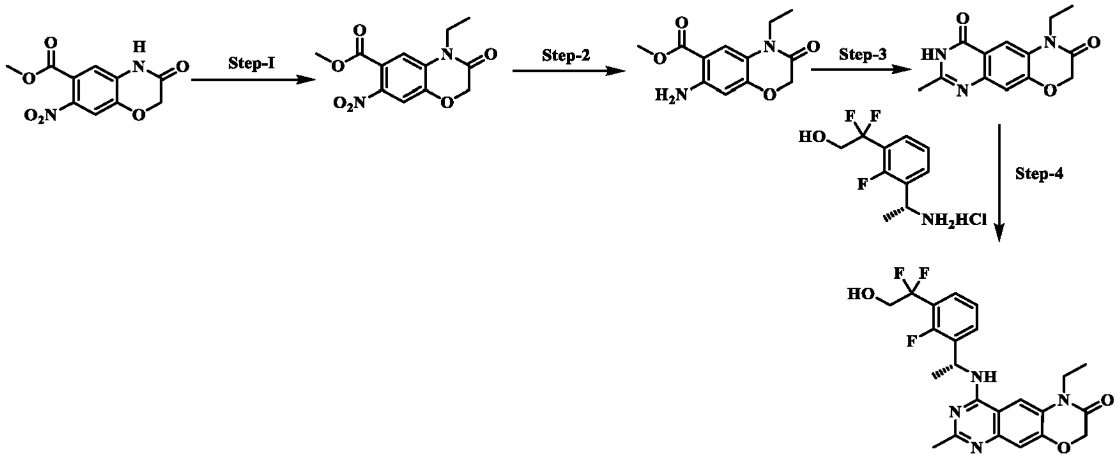


**Step** 1: Methyl 4-ethyl-7-nitro-3-oxo-3,4-dihydro-2H-benzo[b][1,4]oxazine-6-carboxylate

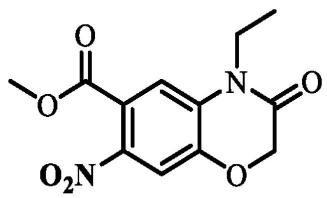

The titled compound was prepared from **Example 14 Step 1** intermediate by employing similar protocol mentioned in **Example 14 Step 2** (4.36 g, 78.0 % yield).
$^1$H NMR (400 MHz, DMSO-$d_6$) δ 7.75 (s, 1H), 7.56 (s, 1H), 4.85 (s, 2H), 4.01 (q, *J* = 7.1 Hz, 2H), 3.85 (s, 3H), 1.16 (t, J = 7.1 Hz, 3H).

**Step** 2: Methyl 7-amino-4-ethyl-3-oxo-3,4-dihydro-2H-benzo[b][1,4]oxazine-6-carboxylate.

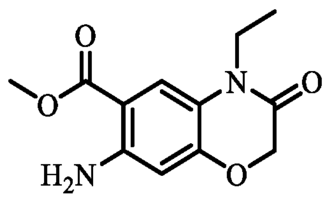

The titled compound was prepared from Step 1 intermediate by employing similar protocol mentioned in **Example 14 Step** 3 (3.2 g, 90.0% yield).
MS (ES+) m/z = 251.1 (M+1).
$^1$H NMR (400 MHz, DMSO-$d_6$) δ 7.37 (s, 1H), 6.62 (bs, 2H), 6.41 (s, 1H), 4.62 (s, 2H), 3.86 (q, *J* = 6.8 Hz, 2H), 3.79

(s, 3H), 1.14 (t, *J* = 7.1 Hz, 3H).

**Step 3:** 6-Ethyl-2-methyl-3,6-dihydro-4H-[1,4]oxazino[3,2-g]quinazoline-4,7(8H)-dione

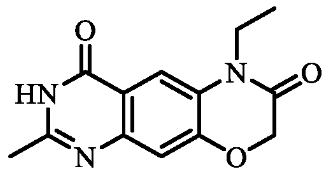

The titled compound was prepared from **Step 2** intermediate by employing similar protocol mentioned in **Example 14 Step 4** (2.71 g, 84.0 % yield).

MS (ES+) m/z = 260.1 (M +1).

$^1$H NMR (400 MHz, DMSO-$d_6$) δ 12.20 (s, 1H), 7.66 (s, 1H), 7.11 (s, 1H), 4.79 (s, 2H), 4.17 - 3.88 (m, 2H), 2.32 (s, 3H), 1.28 - 1.01 (m, 3H).

**Step 4:** (R)-4-((1-(3-(1,1-difluoro-2-hydroxyethyl)-2-fluorophenyl)ethyl)amino)-6-ethyl-2-methyl-6H-[1,4]oxazino[3,2-g]quinazolin-7(8H)-one. **(Compound 13)**

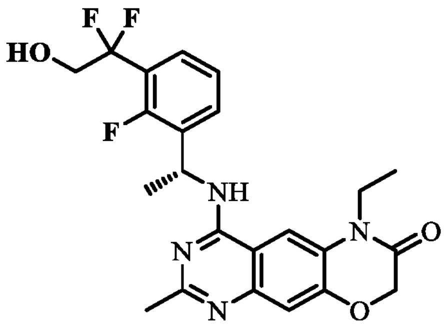

The titled compound was prepared by reaction of **Step 3** intermediate and appropriate amine using similar protocol mentioned in **Example 14 Step 5** (0.028 g, 8.0% yield).

MS (ES+) m/z = 461.2 (M+1).

$^1$H NMR (400 MHz, DMSO-$d_6$) δ 8.35 *(d, J=* 7.3 Hz, 1H), 8.00 (s, 1H), 7.64 - 7.61 (t, *J* = 7.0 Hz, 1H), 7.44 - 7.40 (m, 1H), 7.28 - 7.24 (m, 1H), 7.10 (s, 1H), 5.87 - 5.78 (m, 1H), 5.77 - 5.68 (m, 1H), 4.76 (s, 2H), 4.24 - 4.09 (m, 2H), 3.99 - 3.90 (m, 2H), 2.31 (s, 3H), 1.63 (d, *J*= 7.1 Hz, 3H), 1.28 - 1.22 (m, 3H).

**Example 27:** Preparation of (R)-4-((1-(3-amino-5-(trifluoromethyl)phenyl)ethyl)amino)-2-ethyl-6-methyl-6H-[1,4]oxazino[3,2-g]quinazolin-7(8H)-one. **(Compound-14)**

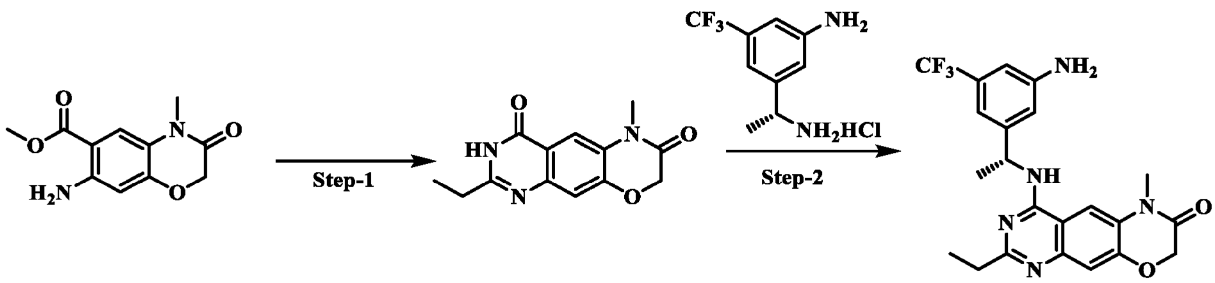

**Step** 1: 2-Ethyl-6-methyl-3,6-dihydro-4H-[1,4]oxazino[3,2-g]quinazoline-4,7(8H)-dione

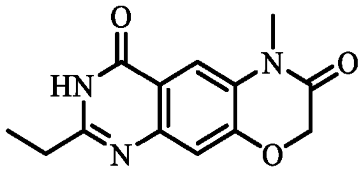

The titled compound was prepared from **Example 14 Step 3** intermediate & propionitrile by employing similar protocol mentioned in **Example 14 Step 4** (1.4 g, 85.0% yield).

$^1H$ NMR (400 MHz, DMSO-$d_6$) δ 12.14 (s, 1H), 7.63 (s, 1H), 7.12 (s, 1H), 4.80 (s, 2H), 3.36 (s, 3H), 2.6 (q, $J$ = 7.5 Hz, 2H), 1.23 (t, $J$ = 7.5 Hz, 3H).

**Step 2:** (R)-4-((1-(3-amino-5-(trifluoromethyl)phenyl)ethyl)amino)-2-ethyl-6-methyl-6H-[1,4]oxazino[3,2-g]quinazolin-7(8H)-one. **(Compound-14)**

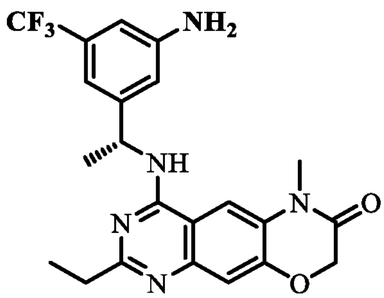

The titled compound was prepared from **Step 1** intermediate by employing similar protocol mentioned in Example 14 Step 5 (0.09 g, 18.5% yield).

MS (ES+) m/z = 446.11 (M+1).

$^1H$ NMR (400 MHz, DMSO-$d_6$) δ 8.25 (d, $J$ = 7.6 Hz, 1H), 7.95 (s, 1H), 7.11 (s, 1H), 6.92 - 6.83 (m, 2H), 6.70 (d, $J$= 1.9 Hz, 1H), 5.61 - 5.47 (m, 3H), 4.78 (s, 2H), 3.43 (s, 3H), 2.62 (q, $J$= 7.5 Hz , 2H), 1.58 (d, $J$ = 7.1 Hz, 3H), 1.17 (t, $J$ = 7.6 Hz, 3H).

**Example 28:** Preparation of (R)-4-((1-(3-(1,1-difluoro-2-hydroxyethyl)-2-fluorophenyl)ethyl)amino)-2-ethyl-6-methyl-6H-[1,4]oxazino[3,2-g]quinazolin-7(8H)-one **(Compound 15)**

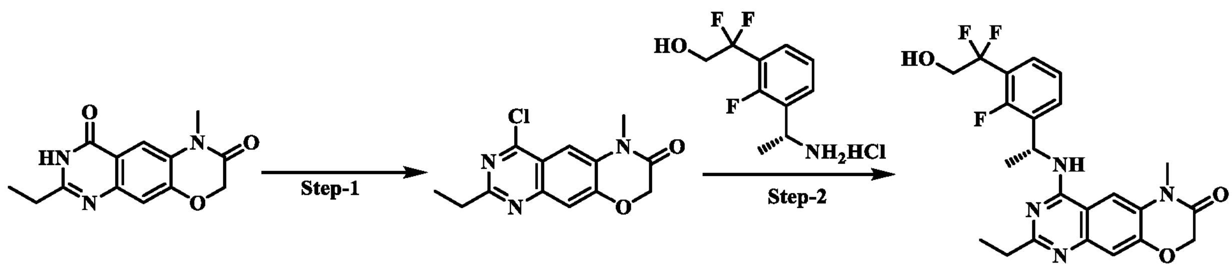


**Step** 1: 4-Chloro-2-ethyl-6-methyl-6H-[1,4]oxazino[3,2-g]quinazolin-7(8H)-one

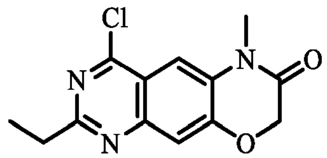

To a stirred solution of 2-Ethyl-4-hydroxy-6-methyl-6H-[1,4]oxazino[3,2-g]quinazolin-7(8H)-one (0.9 g, 3.47 mmol) **(Example 27 Step** 1) in chlorobenzene (50.0 mL) was added DIPEA (6.06 mL, 34.7 mmol) followed by $POCl_3$ (1.94 mL, 20.83 mmol) at 0 °C and stirred at room temperature for 0.5 h. After that reaction mixture was heated at 92 °C for 16 h. After completion, reaction mixture was diluted with cold water (50.0 mL) and extracted with dichloromethane (30.0 mL x 2). The combined organic layer was dried over sodium sulphate and concentrated in vacuo to get crude residue. This crude residue was purified by flash chromatography using eluent 0 - 30% ethyl acetate in n-hexane to afford the titled compound 4-chloro-2-ethyl-6-methyl-6H-[1,4]oxazino[3,2-g]quinazolin-7(8H)-one (0.68 g, 70.5 % yield).

GCMS m/z = 277.0 (M+).

$^1$H NMR (400 MHz, DMSO-$d_6$) δ 7.67 (s, 1H), 7.38 (s, 1H), 4.88 (s, 2H), 3.38 (s, 3H), 2.82 (q, *J*= 7.6 Hz, 2H), 1.33 (t, *J*= 7.6 Hz, 3H).

**Step 2:** (R)-4-((1-(3-(1,1-difluoro-2-hydroxyethyl)-2-fluorophenyl)ethyl)amino)-2-ethyl-6-methyl-6H-[1,4]oxazino[3,2-g]quinazolin-7(8H)-one. (Compound 15)

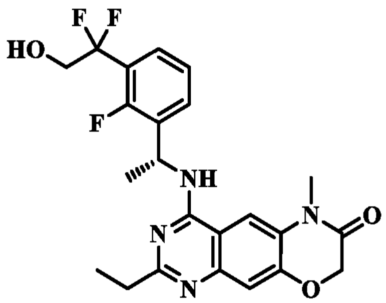

To a stirred solution of 4-chloro-2-ethyl-6-methyl-6H-[1,4]oxazino[3,2-g]quinazolin-7(8H)-one (0.06 g, 0.216 mmol) in 1,4 dioxane (5.0 mL) was added (R)-2-(3-(1-aminoethyl)-2-fluorophenyl)-2,2-difluoroethan-1-ol hydrochloride and Hunig's base (0.19 mL, 1.08 mmol) in microwave vial and reaction mass was heated at 120 °C for 18 h . After completion of reaction mixture was concentrated in vacuo to get crude residue. The crude residue was purified by using eluent 60 - 70% ethyl acetate in n-hexane to afford the titled compound (R)-4-((1-(3-(1,1-difluoro-2-hydroxyethyl)-2-fluorophenyl)ethyl)amino)-2-ethyl-6-methyl-6H-[1,4]oxazino[3,2-g]quinazolin-7(8H)-one (0.048 g, 48.0 % yield).

MS (ES+) m/z = 461.0 (M+1).

$^1$H NMR (400 MHz, DMSO-$d_6$) δ 8.38 (d, *J*= 7.1 Hz, 1H), 8.00 (s, 1H), 7.64 - 7.56 (m, 1H), 7.46 - 7.35 (m, 1H), 7.27 - 7.20 (m, 1H), 7.10 (s, 1H), 5.83 - 5.76 (m, 1H), 5.72 (t, *J* = 6.5 Hz, 1H), 4.79 (s, 2H), 3.98-3.92 (m, 2H), 3.46 (s, 3H), 2.59 - 2.52 (m, 2H), 1.63 *(d, J*= 7.1 Hz, 3H), 1.11 - 1.06 (m, 3H).

**Example 29:** Preparation of (R)-2-cyclopropyl-4-((1-(3-(1,1-difluoro-2-hydroxyethyl)-2-fluorophenyl)ethyl)amino)-6-methyl-6H-[1,4]oxazino[3,2-g]quinazolin-7(8H)-one **(Compound-16)**

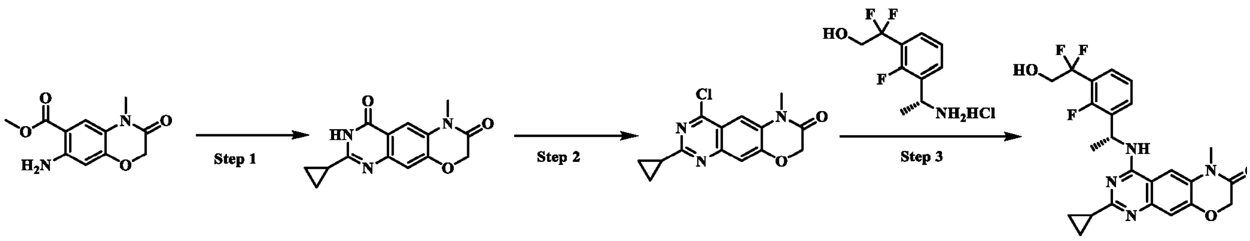

**Step 1:** 2-Cyclopropyl-6-methyl-3,6-dihydro-4H-[1,4]oxazino[3,2-g]quinazoline-4,7(8H)-dione

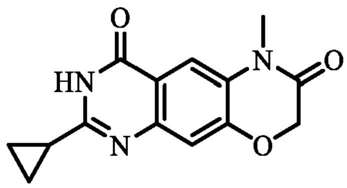

The titled compound was prepared from **Example 14 Step 3** intermediate & cyclopropane carbonitrile by employing similar protocol mentioned in **Example 14 Step 4** (1.02 g, 59.2% yield).

MS (ES+) m/z = 272.2 (M+1).

$^1$H NMR (400 MHz, DMSO-$d_6$) δ 12.39 (s, 1H), 7.60 (s, 1H), 6.99 (s, 1H), 4.78 (s, 2H), 3.33 (s, 3H), 1.97 - 1.87 (m, 1H), 1.10 - 0.96 (m, 4H).

**Step 2:** 4-Chloro-2-cyclopropyl-6-methyl-6H-[1,4]oxazino[3,2-g]quinazolin-7(8H)-one

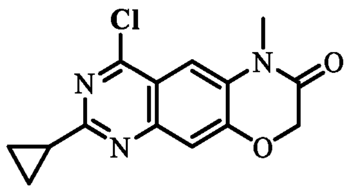

The titled compound was prepared from **Step 1** intermediate by employing similar protocol mentioned in **Example 28 Step 1** (0.62 g, 58.0% yield).

MS (ES+) m/z = 290.1 (M+1).

$^1$H NMR (400 MHz, DMSO-$d_6$) δ 7.55 (s, 1H), 7.39 (s, 1H), 4.92 (s, 2H), 3.43 (s, 3H), 2.31 - 2.21 (m, 1H), 1.17 - 1.03 (m, 4H).

Step 3: (R)-2-cyclopropyl-4-((1-(3-(1,1-difluoro-2-hydroxyethyl)-2-fluorophenyl)ethyl)amino)-6-methyl-6H-[1,4]oxazino[3,2-g]quinazolin-7(8H)-one **(Compound-16)**

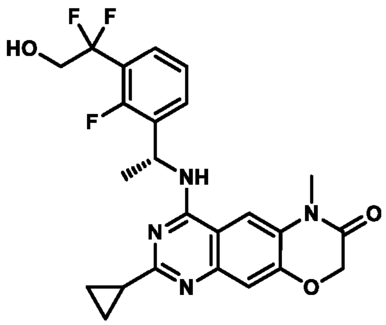

The titled compound was prepared by reaction of **Step 2** intermediate and appropriate amine using similar protocol mentioned in **Example 28 Step 2** (0.021 g, 13.0% yield).

MS (ES+) m/z = 473.1 (M+1).

$^1$H NMR (400 MHz, DMSO-$d_6$) δ 8.35 (d, *J*= 6.5 Hz, 1H), 7.97 (s, 1H), 7.57 - 7.50 (m, 1H), 7.43 - 7.37 (m, 1H), 7.25 - 7.20 (m, 1H), 7.06 (s, 1H), 5.76 - 5.71 (m, 1H), 5.63 - 5.60 (m, 1H), 4.78 (s, 2H), 3.95 - 3.90 (m, 2H), 3.45 (s, 3H), 1.86 - 1.82 (m, 1H), 1.60 (d, *J*= 7.1 Hz, 3H), 1.04 - 0.94 (m, 1H), 0.86 - 0.82 (m, 1H), 0.73 - 0.62 (m, 1H), 0.51 - 0.40 (m, 1H).

Example 30 : Preparation of (R&S)-(4-(((R)-1-(3-amino-5-(trifluoromethyl)phenyl)ethyl)amino)-2-methyl-8,9-dihydro-7H-pyrano[2,3-g]quinazolin-7-yl)(pyrrolidin-1-yl)methanone (Compound 17a and 17b)

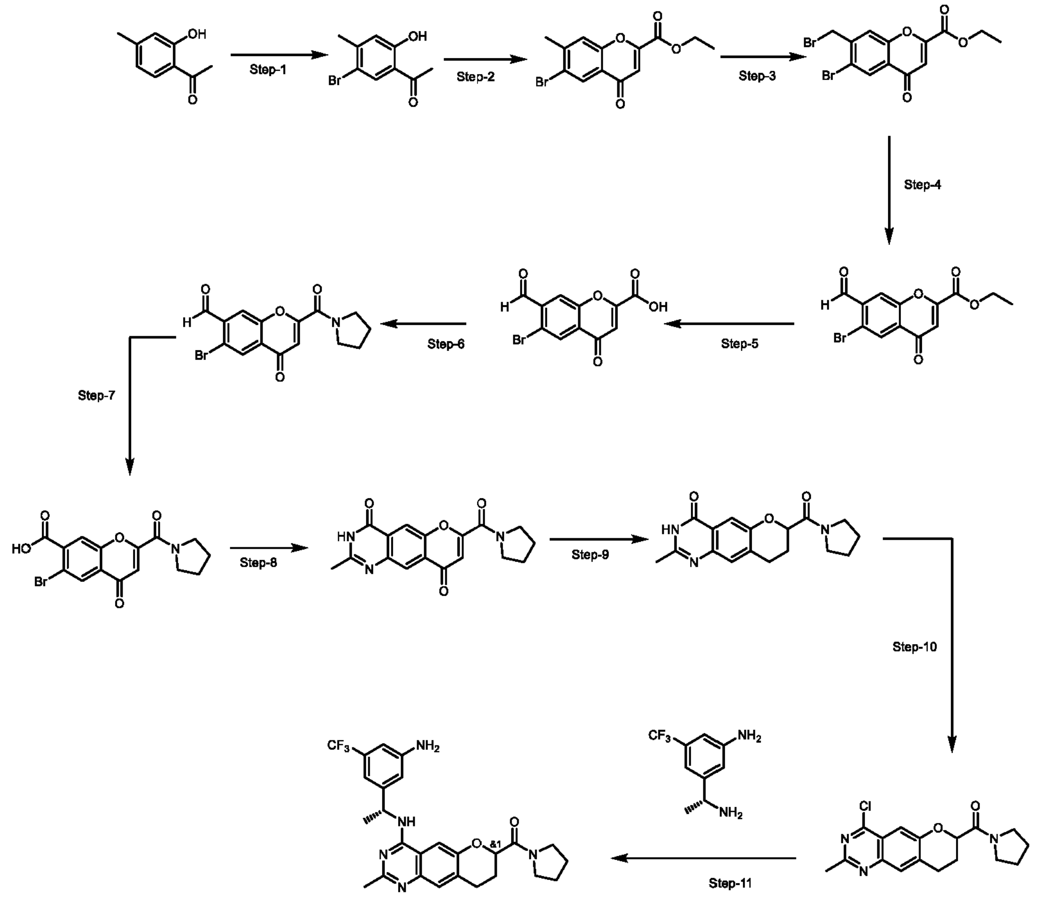


Compound 17a and 17b

**Step** 1: 1-(5-Bromo-2-hydroxy-4-methylphenyl)ethan-1-one.

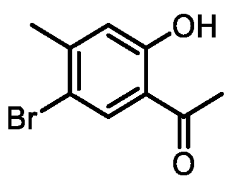

To a stirred solution of 1-(2-Hydroxy-4-methylphenyl) ethan-1-one (30.0 g, 200.0 mmol) in chloroform (300.0 mL) at -12°C was added bromine (10.50 mL, 204.0 mmol) in chloroform (80.0 mL) over 10 min under nitrogen atmosphere. The reaction mixture was stirred at -12°C for 1h, then poured into water (300.0 mL) and diluted with 300.0 mL of DCM. The organic layer was washed with water (200.0 mL), 10% sodium thiosulfate (2 x 200.0 mL), and brine (100.0 mL), organic layer was dried over on anhydrous $Na_2SO_4$ then concentrated in vacuo to give crude compound. This crude compound was purified by flash chromatography using eluent 0-10% ethyl acetate in petroleum ether to afford the titled compound 1-(5-bromo-2-hydroxy-4-methylphenyl)ethan-1-one (39.0 g, 85.0 % yield) as an off white solid.

GCMS m/z = 230.10 (M+1).

$^1H$ NMR (400 MHz, DMSO-$d_6$) δ 11.80 (s, 1H), 7.99 (s, 1H), 6.99 (d, $J$ = 0.9 Hz, 1H), 2.62 (s, 3H), 2.33 (d, $J$ = 0.7 Hz, 3H).

**Step 2:** Ethyl 6-bromo-7-methyl-4-oxo-4H-chromene-2-carboxylate

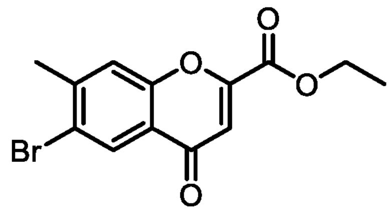

To a stirred solution of 1-(5-bromo-2-hydroxy-4-methylphenyl)ethan-1-one (1.1 g, 4.80 mmol) and diethyl oxalate (1.97 mL, 14.41 mmol) in DMF (5.0 mL) was added potassium tert butoxide (39.2 g, 349.0 mmol) portion wise at 0 - 5 °C and reaction mixture was stirred for 3 h at 0 - 5 °C under nitrogen atmosphere. Then conc. HCl solution (0.5 mL) in 10 ml of water was added at 0°C, and the mixture was extracted with ethyl acetate (3 × 20.0 mL). The combined organic layers were dried over anhydrous $Na_2SO_4$ and concentrated under reduced pressure to get residue. The residue was dissolved in ethanol (5.0 mL) and conc. HCl (1.60 mL, 19.21 mmol) was subsequently added. The reaction mixture was stirred at 80 °C under nitrogen atmosphere for 16 h. After completion of reaction, reaction mixture was concentrated under reduced pressure to get crude compound. The crude compound was purified by flash chromatography using eluent 0 -15% ethyl acetate n-hexane to afford the titled compound ethyl 6-bromo-7-methyl-4-oxo-4H-chromene-2-carboxylate (0.8 g, 53.5 % yield) as an off white solid.

MS (ES+) m/z = 311.21 (M+1).

$^1H$ NMR (400 MHz, Chloroform-d) δ 8.36 (s, 1H), 7.54 (d, *J* = 1.0 Hz, 1H), 7.12 (s, 1H), 4.48 (q, *J* = 7.1 Hz, 2H), 2.56 (d, *J* = 0.8 Hz, 3H), 1.45 (t, *J* = 7.1 Hz, 3H).

**Step 3:** Ethyl 6-bromo-7-(bromomethyl)-4-oxo-4H-chromene-2-carboxylate

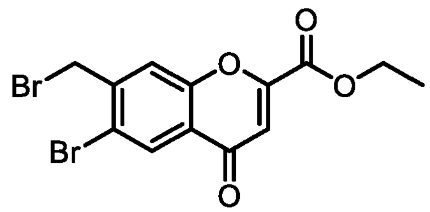

To a stirred solution of ethyl 6-bromo-7-methyl-4-oxo-4H-chromene-2-carboxylate (13.0 g, 41.8 mmol) in carbon tetrachloride (130.0 mL) were added NBS (7.81 g, 43.9 mmol) and AIBN (0.69 g, 4.18 mmol) under nitrogen atmosphere. The reaction mixture was heated at 85 °C for 16 h. After completion of reaction, the reaction mass was cooled to room temperature and concentrated under reduced pressure to get crude residue. The crude residue was purified by flash chromatography using eluent 0 - 5% ethyl acetate in n-hexane to afford the titled compound ethyl 6-bromo-7-(bromomethyl)-4-oxo-4H-chromene-2-carboxylate (12.0 g, 73.6 % yield) as an of white solid .

$^1H$ NMR (400 MHz, DMSO-$d_6$) δ 8.19 (s, 1H), 8.17 (s, 1H), 7.00 (s, 1H), 4.85 (s, 2H), 4.41 (q, J = 7.1 Hz, 2H), 1.36 (t, J = 7.1 Hz, 3H).

Step 4: Ethyl 6-bromo-7-formyl-4-oxo-4H-chromene-2-carboxylate

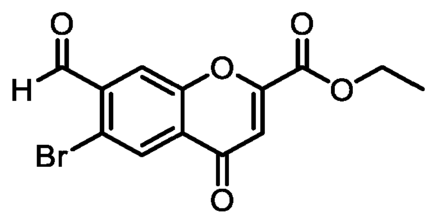

To a mixture of ethyl 6-bromo-7-(bromomethyl)-4-oxo-4H-chromene-2-carboxylate (10.0 g, 25.6 mmol) and molecular sieves 30.0 g in acetonitrile (100.0 mL) at room temperature was added 4-methyl-4-oxidomorpholin-4-ium (10.51 g, 90 mmol). The resulting mixture was stirred under nitrogen atmosphere for 1 h at room temperature. After completion of reaction, the reaction mixture was diluted with 150.0 mL of ethyl acetate and filtered through celite bed. The filtrate was washed with water (50.0 mL), 1N HCl (15.0 mL) and brine (25.0 mL). The organic layer was dried over anhydrous sodium sulfate and concentrated under reduced pressure to afford the titled compound ethyl 6-bromo-7-formyl-4-oxo-4H-chromene-2-carboxylate (8.1 g, 97.0 % yield) as off white solid.

$^1$H NMR (400 MHz, DMSO-$d_6$) δ 10.26 (s, 1H), 8.29 (s, 1H), 8.11 (s, 1H), 7.06 (s, 1H), 4.42 (q, $J$ = 7.1 Hz, 2H), 1.36 (t, $J$ = 7.1 Hz, 3H).

**Step** 5: 6-Bromo-7-formyl-4-oxo-4H-chromene-2-carboxylic acid

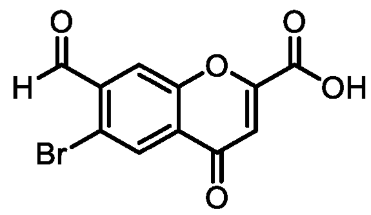

To a mixture of ethyl 6-bromo-7-formyl-4-oxo-4H-chromene-2-carboxylate (8.0 g, 24.61 mmol) in acetic acid (70.0 mL) was added conc. HCl (7.48 mL, 246.0 mmol). The resulting mixture was stirred for 1 h at 85 °C. The mixture was diluted with 100.0 mL of water. The reaction mixture was extracted in ethyl acetate (80.0 mL x 3), organic was washed with water (35.0 mL), and brine (50.0 mL). The organic layer was dried over anhydrous sodium sulfate and concentrated under reduced pressure to afford the title compound 6-bromo-7-formyl-4-oxo-4H-chromene-2-carboxylic acid (7.2 g, 98.49%) as an off white solid.

$^1$H NMR (400 MHz, DMSO-$d_6$) δ 10.27 (s, 1H), 8.29 (s, 1H), 8.09 (s, 1H), 7.00 (s, 1H).

**Step 6:** 6-Bromo-4-oxo-2-(pyrrolidine-1-carbonyl)-4H-chromene-7-carbaldehyde

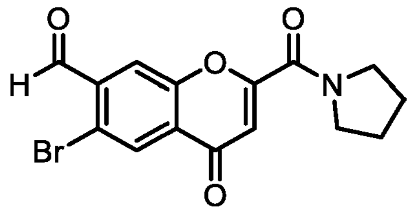

To a stirred solution of 6-bromo-7-formyl-4-oxo-4H-chromene-2-carboxylic acid (7.1 g, 23.90 mmol) in DMF (60.0 mL) under nitrogen atmosphere at room temperature was added bromo(tripyrrolidin-1-yl) phosphanium hexafluorophosphate (13.37 g, 28.7 mmol), pyrrolidine (2.04 g, 28.7 mmol) followed by addition of hunig's base (8.35 mL, 47.8 mmol). The reaction was stirred at room temperature for 4 h, then poured into water (100.0 mL) and diluted with 100.0 mL of dichloromethane. The organic layer was washed with water (50.0 mL), and brine (50.0 mL), organic layer was dried over anhydrous $Na_2SO_4$ and concentrated under reduced pressure to give crude compound. The crude residue was purified by flash chromatography using eluent 0-50% ethyl acetate in n-hexane to afford the titled compound 6-bromo-4-oxo-2-(pyrrolidine-1-carbonyl)-4H-chromene-7-carbaldehyde (4.8 g, 57.4 % yield) as off white solid.

MS (ES+) m/z = 350.22 (M+1).

$^1$H NMR (400 MHz, DMSO-$d_6$) δ 10.28 (s, 1H), 8.30 (s, 1H), 8.16 (s, 1H), 6.79 (s, 1H), 3.80 - 3.75 (m, 2H), 3.52 - 3.48 (m, 2H), 1.91 - 1.88 (m, 4H).

**Step 7:** 6-Bromo-4-oxo-2-(pyrrolidine-1-carbonyl)-4H-chromene-7-carboxylic acid

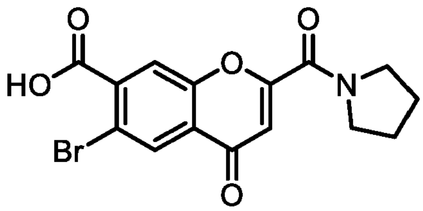

To a stirred suspension of 6-bromo-4-oxo-2-(pyrrolidine-1-carbonyl)-4H-chromene-7-carbaldehyde (4.7 g, 13.42 mmol) and sulfamic acid (1.69 g, 17.45 mmol) in acetone (40.0 mL) was added a solution of sodium chlorite (1.7 g, 18.79 mmol) in water (10.0 mL). The reaction mixture was stirred at room temperature for 5 h. After completion of reaction, distilled the reaction mixture under reduced pressure to get crude residue and it was extracted with ethyl acetate (50.0 ml x 3). The combined organic layer was washed by water (50.0 ml), brine (20.0 ml) and dried over anhydrous $Na_2SO_4$ and concentrated under reduced pressure to afford the title compound 6-bromo-4-oxo-2-(pyrrolidine-1-carbonyl)-4H-chromene-7-carboxylic acid (4.7 g, 95.72 % yield) as an off white solid.

MS (ES+) m/z = 366.16 (M+).

$^1$H NMR (400 MHz, DMSO-*d*$_6$) δ 8.21 (s, 1H), 8.09 (s, 1H), 6.75 (s, 1H), 3.77 - 3.73 (m, 2H), 3.51 - 3.48 (m, 2H), 2.18 - 1.85 (m, 4H).

**Step 8:** 2-Methyl-7-(pyrrolidine-1-carbonyl)-3H-pyrano[2,3-g]quinazoline-4,9-dione

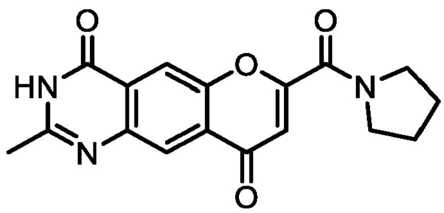

To a stirred suspension of 6-bromo-4-oxo-2-(pyrrolidine-1-carbonyl)-4H-chromene-7-carboxylic acid (4.6 g, 12.56 mmol) and acetamidine hydrochloride (1.78 g, 18.84 mmol) in DMF (50.0 mL) was added cesium carbonate (8.19 g, 25.1 mmol). The mixture was thoroughly deoxygenated by purging nitrogen for 15 min and then copper (I) iodide (0.48 g, 2.51 mmol) was added, the resulting mixture was stirred at 70°C for 16 h. The hot reaction mass was filtered through celite bed and was washed with DMF (20.0 mL x 2) and filtrate was concentrated to get crude residue. The crude residue was purified by flash chromatography using eluent 0-6% methanol in DCM to afford the title compound 2-methyl-7-(pyrrolidine-1-carbonyl)-3H-pyrano[2,3-g]quinazoline-4,9-dione (1.7 g, 41.6 % yield) as off white solid.

MS (ES+) m/z = 326.13 (M+1).

$^1$H NMR (400 MHz, DMSO-*d*$_6$) δ 12.46 (s, 1H), 8.31 (s, 1H), 8.10 (s, 1H), 6.71 (s, 1H), 3.86 - 3.73 (m, 2H), 3.59 - 3.41 (m, 2H), 2.39 (s, 3H), 1.93-1.87 (m, 4H).

Step 9: (R&S) -(4-Hydroxy-2-methyl-8,9-dihydro-7H-pyrano[2,3-g]quinazolin-7-yl)(pyrrolidin-1-yl) methanone

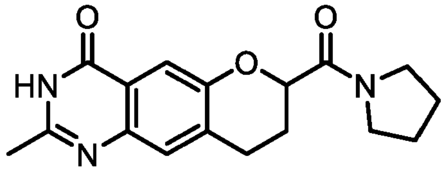

To a stirred solution of 4-hydroxy-2-methyl-7-(pyrrolidine-1-carbonyl)-9H-pyrano[2,3-g]quinazolin-9-one (1.0 g, 3.07 mmol) in methanol (5.0 mL) was added Pd/C (3.27 g, 30.7 mmol) and subjected for reduction under hydrogen atmosphere in parr shaker for 3 days. The reaction was filtered through celite bed and solvent was evaporated to get crude compound. This crude compound was purified by flash chromatography using eluent 0-5% methanol in DCM to afford the titled compound (4-hydroxy-2-methyl-8,9-dihydro-7H-pyrano[2,3-g]quinazolin-7-yl)(pyrrolidin-1-yl)methanone (0.4 g , 41.5 % yield) as off white solid.

MS (ES+) m/z = 314.40 (M+1).

$^1$H NMR (400 MHz, DMSO-$d_6$) δ 12.00 (s, 1H), 7.33 (d, *J* = 2.9 Hz, 2H), 5.03 - 4.98 (m, 1H), 3.70 - 3.60 (m, 1H), 3.58 - 3.49 (m, 1H), 3.38 - 3.30 (m, 2H), 3.00 - 2.86 (m, 2H), 2.30 (s, 3H), 2.14 - 1.97 (m, 2H), 1.93 - 1.86 (m, 2H), 1.83 - 1.76 (m, 2H).

**Step 10:** (R&S)-(4-Chloro-2-methyl-8,9-dihydro-7H-pyrano[2,3-g]quinazolin-7-yl)(pyrrolidin-1-yl) methanone

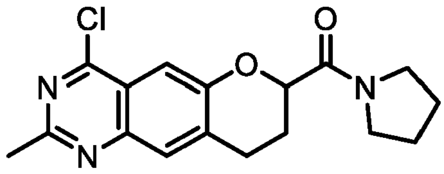

The titled compound was prepared from Step 9 intermediate by employing similar protocol mentioned in **Example 28 Step** 1 (0.13 g, 79.0% yield).

MS (ES+) m/z = 332.34 (M+1).

$^1$H NMR (400 MHz, DMSO-$d_6$) δ 7.76 (s, 1H), 7.46 - 7.36 (m, 1H), 5.18 (dd, *J*= 6.7, 3.9 Hz, 1H), 3.70- 3.62 (m, 1H), 3.53 (dt, *J*= 10.3, 6.1 Hz, 1H), 3.40-3.29 (m, 2H), 3.06 (dt, *J*= 12.5, 6.8 Hz, 1H), 3.01 -2.89 (m, 1H), 2.69 (s, 3H), 2.19 - 2.05 (m, 2H), 1.96-1.88 (m, 2H), 1.84-1.76 (m, 2H).

**Step 11:** Preparation of (R&S)-(4-(((R)-1-(3-amino-5-(trifluoromethyl)phenyl)ethyl)amino)-2-methyl-8,9-dihydro-7H-pyrano[2,3-g]quinazolin-7-yl)(pyrrolidin-1-yl)methanone.

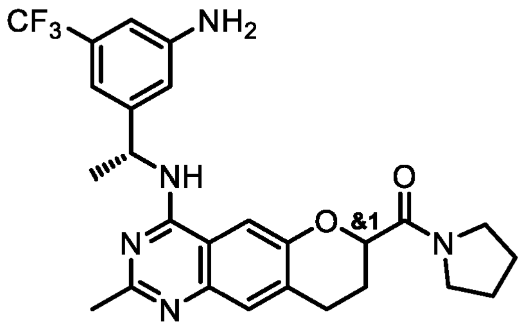

The titled compound was prepared by reaction of Step 10 intermediate and appropriate chiral amine using analogous protocol mentioned in Example 28 Step 2 (0.15g, 13.0% yield).

Diastereomer formed in this product was separated by reverse phase preparative HPLC

Peak 1: ((R/S)-4-(((R)-1-(3-amino-5-(trifluoromethyl)phenyl)ethyl)amino)-2-methyl-8,9-dihydro-7H-pyrano[2,3-g]quinazolin-7-yl)(pyrrolidin-1-yl)methanone.

**(Compound 17a)**

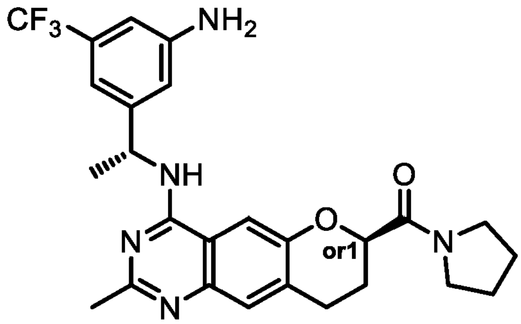

MS (ES+) m/z = 500.02 (M+1).

HPLC RT: 1.39 min

$^1$H NMR (400 MHz, DMSO-$d_6$) δ 8.03 (d, *J* = 8.0 Hz, 1H), 7.80 (s, 1H), 7.33 (s, 1H), 6.89 (s, 1H), 6.84 (s, 1H), 6.69 (s, 1H), 5.61 - 5.45 (m, 3H), 5.07 - 4.97 (m, 1H), 3.74 - 3.63 (m, 1H), 3.62 - 3.52 (m, 1H), 3.40 - 3.35 (m, 2H), 3.03 - 2.89 (m, 2H), 2.35 (s, 3H), 2.15 - 1.99 (m, 2H), 1.97 - 1.88 (m, 2H), 1.87 - 1.75 (m, 2H), 1.52 (d, *J*= 7.0 Hz, 3H).

Peak 2: (S/R)-(4-(((R)-1-(3-amino-5-(trifluoromethyl)phenyl)ethyl)amino)-2-methyl-8,9-dihydro-7H-pyrano[2,3-g]quinazolin-7-yl)(pyrrolidin-1-yl)methanone (Compound 17b)

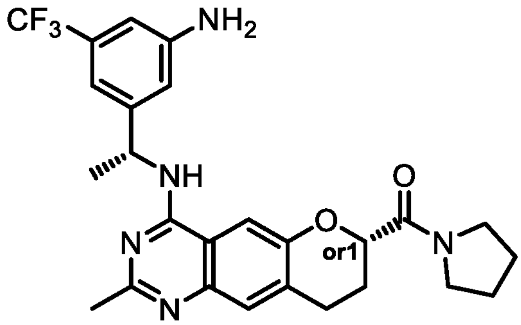

MS (ES+) m/z = 500.30 (M+1).

HPLC RT: 1.58 min

$^1$H NMR (400 MHz, DMSO-$d_6$) δ 8.04 (d, J = 8.1 Hz, 1H), 7.81 (s, 1H), 7.34 (s, 1H), 6.88 (s, 1H), 6.83 (s, 1H), 6.68 (bs, 1H), 5.60 - 5.45 (m, 3H), 5.05 - 4.98 (m, 1H), 3.74 - 3.65 (m, 1H), 3.62 - 3.51 (m, 1H), 3.39 - 3.35 (m, 2H) 3.04 - 2.89 (m, 2H), 2.34 (s, 3H), 2.15 - 2.09 (m, 1H), 2.07 - 2.00 (m, 1H), 1.96 - 1.90 (m, 2H), 1.85 - 1.78 (m, 2H), 1.52 (d, J = 7.1 Hz, 3H).

**Example 31:** Preparation of (R)-4-((1-(3-(1,1-difluoro-2-hydroxyethyl)-2-fluorophenyl)ethyl)amino)-2,6,8,8-tetramethyl-6H-[1,4]oxazino[3,2-g]quinazolin-7(8H)-one **(Compound 18)**

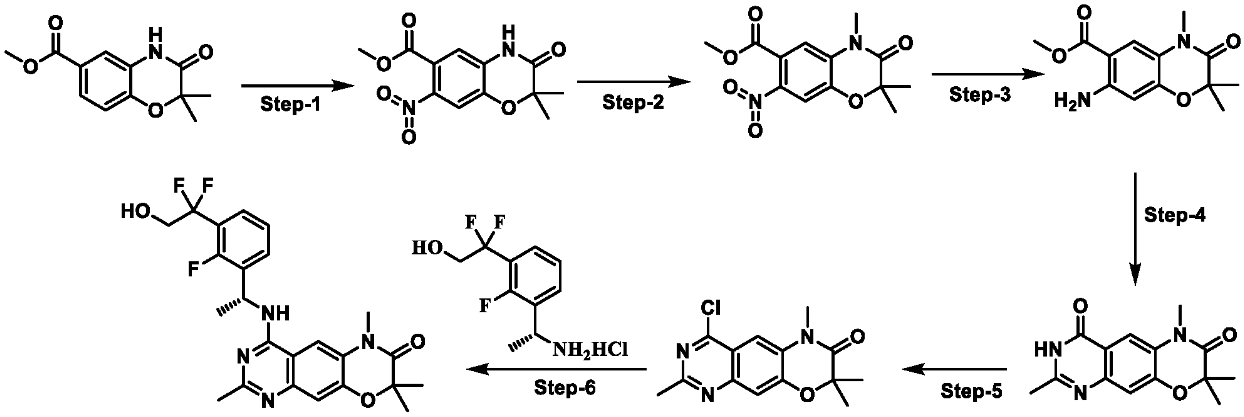


**Step** 1: Methyl 2,2-dimethyl-7-nitro-3-oxo-3,4-dihydro-2H-benzo[b][1,4]oxazine-6-carboxylate.

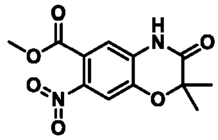

The titled compound was prepared from methyl 2,2-dimethyl-3-oxo-3,4-dihydro-2H-benzo[b][1,4]oxazine-6-carboxylate (prepared by method described in EP2243779) by employing similar protocol mentioned in **Example 14 Step 1** (8.24 g, 99.0 % yield).
MS (ES+) m/z = 281.27 (M+1).

**Step** 2: Methyl 2,2,4-trimethyl-7-nitro-3-oxo-3,4-dihydro-2H-benzo[b][1,4]oxazine-6-carboxylate

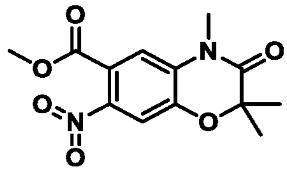

The titled compound was prepared from Step **1** intermediate by employing similar protocol mentioned in Example **14** Step **2** (4.13 g, 98 % yield).
GCMS m/z = 294.07 (M+).
$^{1}$H NMR (400 MHz, DMSO-$d_6$) δ 7.72 (s, 1H), 7.52 (s, 1H), 3.85 (s, 3H), 3.36 (s, 3H), 1.48 (s, 6H).

Step 3: Methyl 7-amino-2,2,4-trimethyl-3-oxo-3,4-dihydro-2H-benzo[b][1,4]oxazine-6-carboxylate

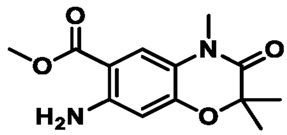

The titled compound was prepared from Step 2 intermediate by employing similar protocol mentioned in **Example 14 Step 3** (3.57 g, 99.0 % yield)
$^{1}$H NMR (400 MHz, DMSO-$d_6$) δ 7.33 (s, 1H), 6.62 (s, 2H), 6.38 (s, 1H), 3.79 (s, 3H), 3.23 (s, 3H), 1.40 (s, 6H).

Step 4: 2,6,8,8-Tetramethyl-3,6-dihydro-4H-[1,4]oxazino[3,2-g]quinazoline-4,7(8H)-dione

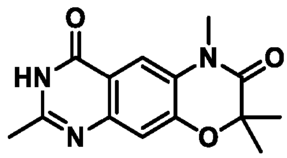

The titled compound was prepared from Step 3 intermediate by employing similar protocol mentioned in **Example 14 Step 4** (3.1 g, 86 % yield).

MS (ES+) m/z = 274.27 (M+1).

$^1$H NMR (400 MHz, DMSO-$d_6$) δ 12.17 (s, 1H), 7.64 (s, 1H), 7.09 (s, 1H), 3.38 (s, 3H), 2.33 (s, 3H), 1.46 (s, 6H).

Step 5: 4-Chloro-2,6,8,8-tetramethyl-6H-[1,4]oxazino[3,2-g]quinazolin-7(8H)-one

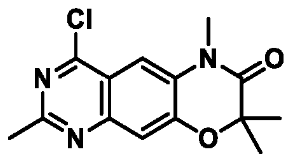

The titled compound was prepared from Step 4 intermediate by employing similar protocol mentioned in **Example 28 Step 1** (0.35 g, 65.6 % yield).

MS (ES+) m/z = 292.2 (M+1).

$^1$H NMR (400 MHz, DMSO-$d_6$) δ 7.63 (s, 1H), 7.47 (s, 1H), 3.47 (s, 3H), 2.70 (s, 3H), 1.52 (s, 6H).

Step 6: (R)-4-((1-(3-(1,1-difluoro-2-hydroxyethyl)-2-fluorophenyl)ethyl)amino)-2,6,8,8-tetramethyl-6H-[1,4]oxazino[3,2-g]quinazolin-7(8H)-one (Compound 18)

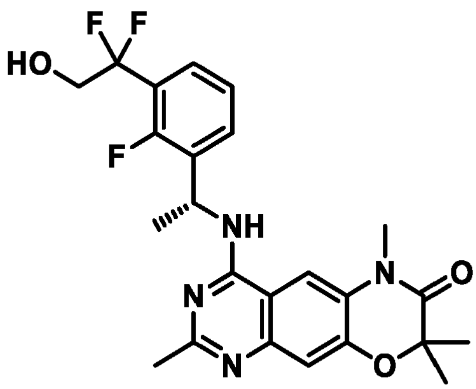

The titled compound was prepared from Step 5 intermediate and appropriate chiral amine by employing similar protocol mentioned in **Example 28 Step 2** (0.05 g, 21.96% yield).

MS (ES+) m/z = 475.17 (M+1).

$^1$H NMR (400 MHz, DMSO-$d_6$) δ 8.35 (d, $J$= 7.3 Hz, 1H), 8.00 (s, 1H), 7.74 - 7.60 (m, 1H), 7.52 - 7.37 (m, 1H), 7.32 - 7.20 (m, 1H), 7.07 (s, 1H), 5.92 - 5.77 (m, 1H), 5.77 - 5.64 (m, 1H), 4.12 - 3.81 (m, 2H), 3.47 (s, 3H), 2.32 (s, 3H), 1.62 (d, $J$ = 7.1 Hz, 3H), 1.47 (s, 3H), 1.46 (s, 3H).

Following **Examples 32-34** disclosed in **Table-2** were prepared using the similar procedure described in **Example 31** by using appropriate chiral amines and commercial chiral amine for compound 19

**Table-2**

| Example | Chemical structure | LCMS and $^1$H NMR data |
|---|---|---|
| **32** | 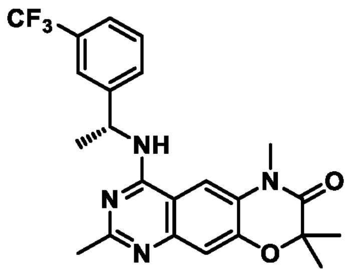 (R)-2,6,8,8-tetramethyl-4-((1-(3-(trifluoromethyl)phenyl)ethyl)amino)-6H-[1,4]oxazino[3,2-g]quinazolin-7(8H)-one **(Compound 19)** | MS (ES+) m/z = 445.10 (M+1) $^1$H NMR (400 MHz, DMSO-$d_6$) δ 8.35 (d, *J* = 7.6 Hz, 1H), 7.95 (s, 1H), 7.82 (s, 1H), 7.79 - 7.74 (m, 1H), 7.63 - 7.55 (m, 2H), 7.08 (s, 1H), 5.74 - 5.65 (m, 1H), 3.45 (s, 3H), 2.35 (s, 3H), 1.64 (d, *J* = 7.1 Hz, 3H), 1.47 (s, 3H), 1.45 (s, 3H). |
| **33** | 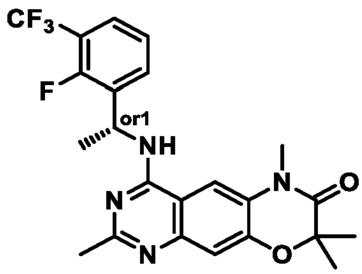 (R/S)-4-((1-(2-fluoro-3-(trifluoromethyl)phenyl)ethyl)amino)-2,6,8,8-tetramethyl-6H-[1,4]oxazino[3,2-g]quinazolin-7(8H)-one **(Compound 20)** | MS (ES+) m/z = 463.17 (M+1) $^1$H NMR (400 MHz, DMSO-$d_6$) δ 8.41 (d, *J* = 6.9 Hz, 1H), 7.99 (s, 1H), 7.85-7.78 (m, 1H), 7.69-7.61 (m, 1H), 7.40 - 7.33 (m, 1H), 7.07 (s, 1H), 5.82 - 5.77 (m, 1H), 3.47 (s, 3H), 2.30 (s, 3H), 1.65 (d, *J* = 7.1 Hz, 3H), 1.47 (s, 3H), 1.45 (s, 3H). |
| **34** | 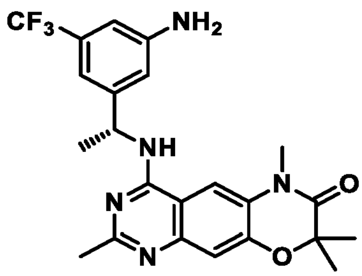 (R)-4-((1-(3-amino-5-(trifluoromethyl)phenyl)ethyl)amino)-2,6,8,8-tetramethyl-6H-[1,4]oxazino[3,2-g]quinazolin-7(8H)-one **(Compound 21)** | MS (ES+) m/z =460.10 (M+1) $^1$H NMR (400 MHz, DMSO-$d_6$) δ 8.31 - 8.23 (m, 1H), 7.96 (s, 1H), 7.08 (s, 1H), 6.98 - 6.85 (m, 2H), 6.73 - 6.69 (m, 1H), 5.70 - 5.46 (m, 3H), 3.44 (s, 3H), 2.38 (s, 3H), 1.57 (d, *J* = 7.0 Hz, 3H), 1.47 (s, 3H), 1.45 (s, 3H). |

**Example 35:** 4-(((R&S)-1-(2-Fluoro-3-((R&S)-1,1,1-trifluoro-2,3-dihydroxypropan-2-yl)phenyl)ethyl)amino)-2,6,8,8-tetramethyl-6H-[1,4]oxazino[3,2-g]quinazolin-7(8H)-one

**(Compound 22)**

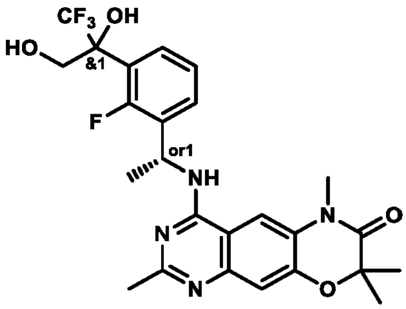

The titled compound as a diastereomeric mixture was prepared from **Example 31 Step 5** intermediate by employing similar protocol mentioned in **Example 28 Step 2.**

MS (ES+) m/z = 523.32 (M+1).

$^1$H NMR (400 MHz, DMSO-$d_6$) δ 8.31 (d, *J* = 7.6 Hz, 1H), 8.01 (s, 1H), 7.67 - 7.58 (m, 1H), 7.61 - 7.48 (m, 1H), 7.20 (t, *J* = 7.7 Hz, 1H), 7.07 (s, 1H), 6.61 (s, 1H), 5.91 - 5.82 (m, 1H), 5.23 (s, 1H), 4.24 - 4.03 (m, 2H), 3.47 (s, 3H), 2.34 (s, 3H), 1.58 (d, *J* = 7.0 Hz, 3H), 1.47 (s, 3H), 1.46 (s, 3H).

Chirally pure diastereomers prepared separately using chirally pure amine as follows.

**Peak 1:** 4-(((R/S)-1-(2-fluoro-3-((S/R)-1,1,1-trifluoro-2,3-dihydroxypropan-2-yl)phenyl)ethyl)amino)-2,6,8,8-tetramethyl-6H-[1,4]oxazino[3,2-g]quinazolin-7(8H)-one (**Compound 22a**)

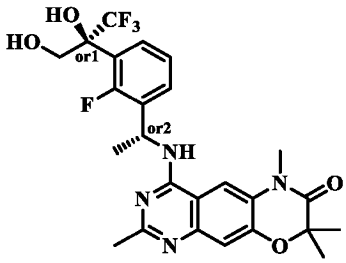

The titled compound was prepared from **Example 31 Step 5** & Example **13 Step 5a** intermediate by employing similar protocol mentioned in **Example 28 Step 2.**

MS (ES+) m/z = 523.32 (M+1).

$^1$H NMR (400 MHz, DMSO-$d_6$) δ 8.34 (d, *J* = 7.3 Hz, 1H), 8.00 (s, 1H),7.64 - 7.60 (m, 1H), 7.57 - 7.47 (m, 1H), 7.18 *(t, J*= 7.8 Hz, 1H), 7.06 (s, 1H), 6.60 (s, 1H), 5.85 - 5.78 (m, 1H), 5.19 (t, *J*= 5.8 Hz, 1H), 4.07-4.01 (m, 2H), 3.47 (s, 3H), 2.29 (s, 3H), 1.59 (d, *J*= 7.0 Hz, 3H), 1.47 (s, 3H), 1.46 (s, 3H).

**Peak 2:** 4-(((R/S)-1-(2-fluoro-3-((R/S)-1,1,1-trifluoro-2,3-dihydroxypropan-2-yl)phenyl)ethyl)amino)-2,6,8,8-tetramethyl-6H-[1,4]oxazino[3,2-g]quinazolin-7(8H)-one (**Compound 22b**)

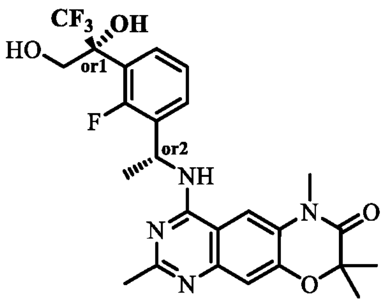

The titled compound was prepared from **Example 31 Step 5 & Example 13 Step** 5b intermediate by employing similar protocol mentioned in **Example 28 Step 2.**

MS (ES+) m/z = 523.19 (M+1).

$^1H$ NMR (400 MHz, DMSO-$d_6$) δ 8.31 (d, *J* = 7.6 Hz, 1H), 8.01 (s, 1H), 7.65 - 7.61 (m, 1H), 7.53 (t, *J* = 6.9 Hz, 1H), 7.20 (t, *J* = 7.8 Hz, 1H), 7.07 (s, 1H), 6.62 (s, 1H), 5.89 - 5.82 (m, 1H), 5.24 (t, *J* = 5.6 Hz, 1H), 4.12 (d, *J* = 5.9 Hz, 2H), 3.47 (s, 3H), 2.34 (s, 3H), 1.58 (d, *J = 7.0* Hz, 3H), 1.47 (s, 3H), 1.46 (s, 3H).

**Example 36:** 4-(((R&S)-1-(3-((R&S)-1,1-difluoro-2,3-dihydroxy-2-methylpropyl)-2-fluorophenyl)ethyl)amino)-2,6,8,8-tetramethyl-6H-[1,4]oxazino[3,2-g]quinazolin-7(8H)-one **(Compound 23)**

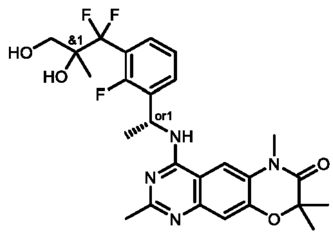

The titled compound as a diastereomeric mixture was prepared from **Example 31 Step 5** intermediate by employing similar protocol mentioned in **Example 28 Step 2**

Chirally pure diastereomers prepared separately using chirally pure amine as follows.

Peak 1: 4-(((R/S)-1-(3-((S/R)-1,1-difluoro-2,3-dihydroxy-2-methylpropyl)-2-fluorophenyl)ethyl)amino)-2,6,8,8-tetramethyl-6H-[1,4]oxazino[3,2-g]quinazolin-7(8H)-one **(Compound 23a)**

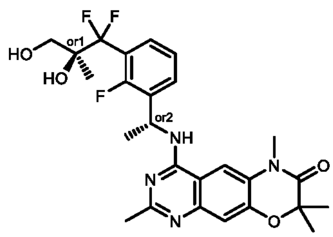

The titled compound was prepared from **Example 31 Step 5 & Example 9 Step 6a** intermediate by employing similar protocol mentioned in **Example 28 Step 2.**

MS (ES+) m/z = 519.32 (M+1).

$^1H$ NMR (400 MHz, DMSO-$d_6$) δ 8.35 (d, *J* = 7.4 Hz, 1H), 8.00 (s, 1H), 7.64 - 7.57 (m, 1H), 7.36 - 7.29 (m, 1H), 7.25 - 7.19 (m, 1H), 7.07 (s, 1H), 5.88 - 5.80 (m, 1H), 5.24 (s, 1H), 4.70 (t, *J* = 6.1 Hz, 1H), 3.52 - 3.41 (m, 5H), 2.31 (s, 3H), 1.60 (d, *J*= 7.0 Hz, 3H), 1.47 (s, 3H), 1.46 (s, 3H), 1.20 (s, 3H).

**Peak 2:** 4-(((R/S)-1-(3-((R/S)-1,1-difluoro-2,3-dihydroxy-2-methylpropyl)-2-fluorophenyl)ethyl)amino)-2,6,8,8-tetramethyl-6H-[1,4]oxazino[3,2-g]quinazolin-7(8H)-one. **(Compound 23b)**

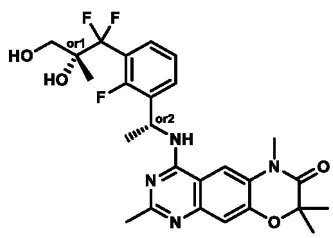

The titled compound was prepared from **Example 31 Step 5** & Example **9 Step 6b** intermediate by employing similar protocol mentioned in **Example 28 Step 2.**

MS (ES+) m/z = 519.32 (M+).

$^1$H NMR (400 MHz, DMSO-$d_6$) δ 8.34 (d, *J* = 7.4 Hz, 1H), 8.00 (s, 1H), 7.66 - 7.56 (m, 1H), 7.36 - 7.29 (m, 1H), 7.29 - 7.16 (m, 1H), 7.07 (s, 1H), 5.91 - 5.79 (m, 1H), 5.27 (s, 1H), 4.73 - 4.64 (m, 1H), 3.47 (s, 3H), 3.45 - 3.34 (m, 2H), 2.32 (s, 3H), 1.60 (d, *J* = 7.0 Hz, 3H), 1.47 (s, 3H), 1.46 (s, 3H), 1.23 (s, 3H).

**Example 37:** (R)-N-(1-(3-amino-5-(trifluoromethyl)phenyl)ethyl)-2,6,8,8-tetramethyl-7,8-dihydro-6H-[1,4]oxazino[3,2-g]quinazolin-4-amine **(Compound 24)**

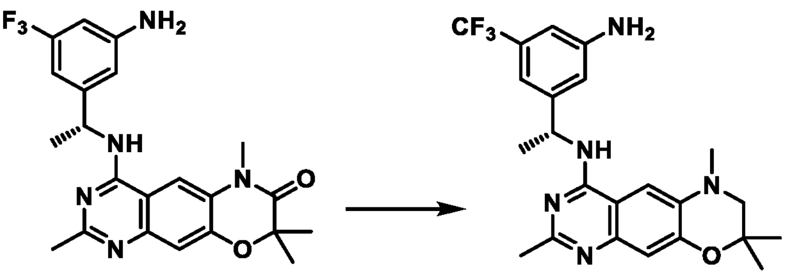

The titled compound was prepared from **Example 34** by employing similar protocol mentioned in **Example 23** (0.017 g, 11.69 % yield).

MS (ES+) m/z = 446.3 (M+1).

$^1$H NMR (400 MHz, DMSO-$d_6$) δ 7.86 (d, *J* = 8.1 Hz, 1H), 7.35 (s, 1H), 6.96 - 6.79 (m, 2H), 6.76 (s, 1H), 6.69 (s, 1H), 5.74 - 5.42 (m, 3H), 3.11 (s, 2H), 3.04 (s, 3H), 2.31 (s, 3H), 1.55 (d, *J* = *7.1* Hz, 3H), 1.33 (s, 6H). **Example 38:** Preparation of (R)-4'-((1-(3-(1,1-difluoro-2-hydroxyethyl)-2-fluorophenyl)ethyl)amino)-2',6'-dimethylspiro[cyclopropane-1,8'-[1,4]oxazino[3,2-g]quinazolin]-7'(6'H)-one **(Compound 25)**

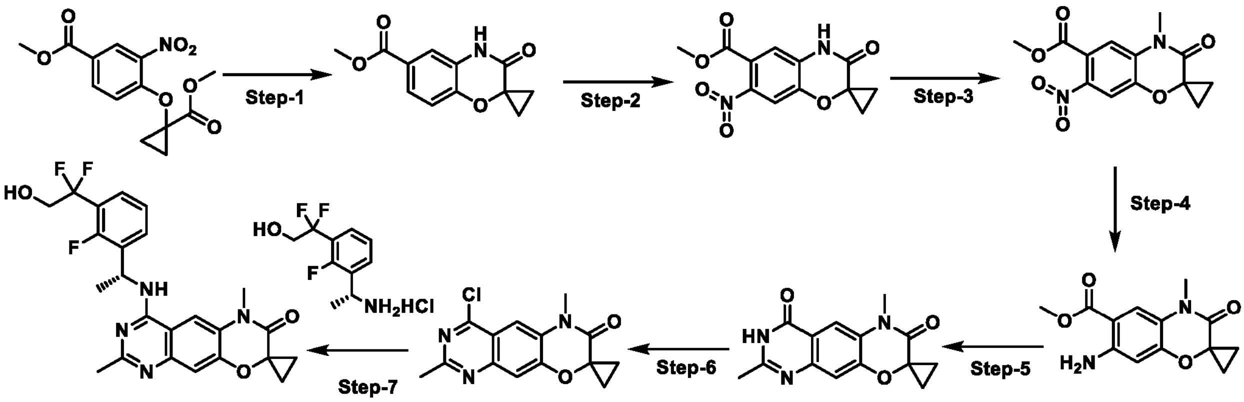


**Step 1:** Methyl 3-oxo-3,4-dihydrospiro[benzo[b][1,4]oxazine-2,1'-cyclopropane]-6-carboxylate

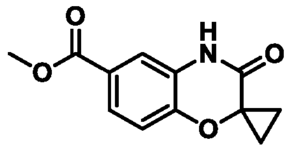

To a stirred solution of methyl 4-(1-(ethoxycarbonyl)cyclopropoxy)-3-nitrobenzoate (2.8 g, 9.05 mmol) (prepared by method described in WO2009/106599) in acetic acid (15.0 mL) was added iron (1.52 g, 27.2 mmol) and heated the reaction mixture at 90°C for 30 min. After completion of reaction, the reaction mixture was filtered and concentrated under reduced pressure to get a residue, the residue was diluted with water (20.0 mL) and basified with a saturated $K_2CO_3$ solution. The aqueous layer was extracted with ethyl acetate (50.0 mL x 2), washed the organic by water (50.0 mL), brine (50.0 mL), dried over anhydrous $Na_2SO_4$ and concentrated under reduced pressure to afford the titled compound methyl

3-oxo-3,4-dihydrospiro[benzo[b][1,4]oxazine-2,1'-cyclopropane]-6-carboxylate (1.4 g, 66.3 % yield) as an off white solid.

GCMS m/z = 233.05 (M+).

$^1H$ NMR (400 MHz, DMSO-$d_6$) δ 10.96 (s, 1H), 7.58 - 7.51 (m, 2H), 7.01 - 6.95 (m, 1H), 3.83 (s, 3H), 1.31 - 1.09 (m, 4H).

**Step 2:** Methyl 7-nitro-3-oxo-3,4-dihydrospiro[benzo[b][1,4]oxazine-2,1'-cyclopropane]-6-carboxylate

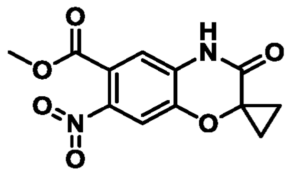

The titled compound was prepared from **Step 1** intermediate by employing similar protocol mentioned in **Example 14 Step 1** (1.1 g, 65.9 % yield).

GCMS m/z = 278.01 (M+).

$^1H$ NMR (400 MHz, DMSO-$d_6$) δ 11.41 (s, 1H), 7.61 (s, 1H), 7.26 (s, 1H), 3.83 (s, 3H), 1.45 - 1.27 (m, 4H).

**Step 3:** Methyl 4-methyl-7-nitro-3-oxo-3,4-dihydrospiro[benzo[b][1,4]oxazine-2,1'-cyclopropane]-6-carboxylate

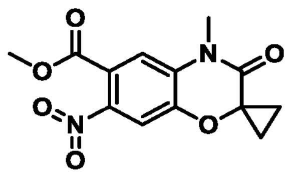

The titled compound was prepared from **Step 2** intermediate by employing similar protocol mentioned in **Example 14 Step 2** (0.91 g, 61.9 % yield).

MS (ES+) m/z = 293.09 (M+1).

$^1H$ NMR (400 MHz, DMSO-$d_6$) δ 7.67 (s, 1H), 7.53 (s, 1H), 3.86 (s, 3H), 3.37 (s, 3H), 1.39 - 1.30 (m, 4H).

**Step 4:** Methyl 7-amino-4-methyl-3-oxo-3,4-dihydrospiro[benzo[b][1,4]oxazine-2,1'-cyclopropane]-6-carboxylate

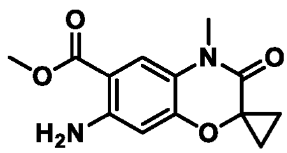

The titled compound was prepared from **Step 3** intermediate by employing similar protocol mentioned in **Example 14 Step 3** (0.8 g, 99.0 % yield).

GCMS m/z = 262.02 (M+).

$^1H$ NMR (400 MHz, DMSO-$d_6$, $D_2O$ exchange) δ 7.34 (s, 1H), 6.33 (s, 1H), 3.79 (s, 3H), 3.24 (s, 3H), 1.25 - 1.05 (m, 4H).

**Step 5:** 2',6'-Dimethyl-3',6'-dihydrospiro[cyclopropane-1,8'-[1,4]oxazino[3,2-g]quinazoline]-4',7'-dione

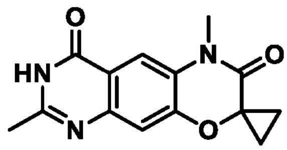

The titled compound was prepared from **Step 4** intermediate by employing similar protocol mentioned in **Example 14 Step 4** (0.19 g, 22.96 % yield).

MS (ES+) m/z = 272.1(M+1).

$^1$H NMR (400 MHz, DMSO-$d_6$) δ 12.20 (s, 1H), 7.64 (s, 1H), 7.04 (s, 1H), 3.39 (s, 3H), 2.33 (s, 3H), 1.35 - 1.27 (m, 4H).

**Step 6:** 4'-Chloro-2',6'-dimethylspiro[cyclopropane-1,8'-[1,4]oxazino[3,2-g]quinazolin]-7'(6'H)-one

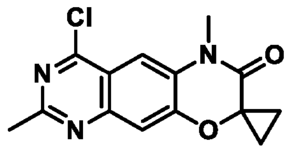

The titled compound was prepared from **Step 5** intermediate by employing similar protocol mentioned in **Example 28 Step 1** (0.12 g, 65.0 % yield).

MS (ES+) m/z = 289.21 (M+).

$^1$H NMR (400 MHz, DMSO-$d_6$) δ 7.61 (s, 1H), 7.40 (s, 1H), 3.47 (s, 3H), 2.69 (s, 3H), 1.46 - 1.31 (m, 4H).

**Step 7:** (R)-4'-((1-(3-(1,1-difluoro-2-hydroxyethyl)-2-fluorophenyl)ethyl)amino)-2',6'-dimethylspiro[cyclopropane-1,8'-[1,4]oxazino[3,2-g]quinazolin]-7'(6'H)-one (Compound 25)

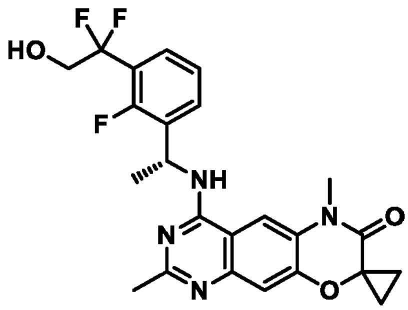

The titled compound was prepared from **Step 6** intermediate and appropriate chiral amine by employing similar protocol mentioned in **Example 28 Step 2** (0.05g, 21.96% yield).

MS (ES+) m/z = 472.96 (M+1).

$^1$H NMR (400 MHz, DMSO-$d_6$) δ 8.38 (d, $J$ = 7.3 Hz, 1H), 8.01 (s, 1H), 7.69 - 7.60 (m, 1H), 7.47 - 7.38 (m, 1H), 7.30 - 7.22 (m, 1H), 7.03 (s, 1H), 5.89 - 5.79 (m, 1H), 5.74 - 5.71 (m, 1H), 4.11 - 3.90 (m, 2H), 3.48 (s, 3H), 2.32 (s, 3H), 1.62 (d, $J$ = 7.0 Hz, 3H), 1.41 - 1.23 (m, 4H).

**Example 39:** Preparation of (R)-1,1-Difluoro-1-(2-fluoro-3-(1-((2,8,8-trimethyl-7-morpholino-8H [1,4]oxazino[3,2-g]quinazolin-4-yl)amino)ethyl)phenyl)-2-methylpropan-2-ol **(Compound 26)**

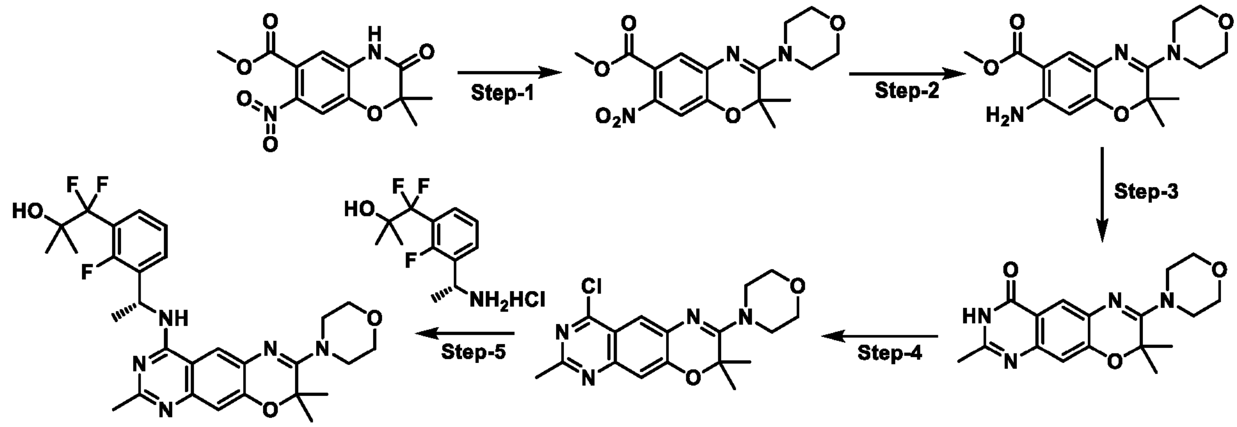


**Step 1:** Methyl 2,2-dimethyl-3-morpholino-7-nitro-2H-benzo[b] [1,4]oxazine-6-carboxylate.

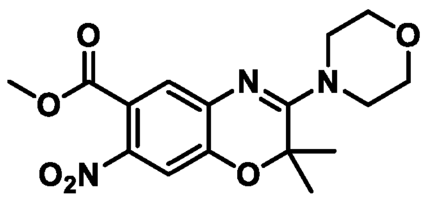

To a stirred solution of methyl 2,2-dimethyl-7-nitro-3-oxo-3,4-dihydro-2H-benzo[b][1,4]oxazine-6-carboxylate (2.5 g, 8.92 mmol) (**Example 31 Step 1 intermediate**) in $SOCl_2$ (13.02 mL, 178.0 mmol) was added DMF (0.14 ml, 1.784 mmol) dropwise at room temperature. The reaction mixture was heated at 70 °C for 2 h. After completion of the reaction, the reaction mixture was cooled to room temperature. The obtained residue was dissolved in 1,4-dioxane (10.0 mL) and TEA (1.24 mL, 8.92 mmol) followed by addition of morpholine (2.33 mL, 26.8 mmol) and stirred the reaction mass at room temperature for 1 h. After completion of the reaction, the reaction mixture was quenched with ice-cold water (30.0 mL) and extracted with ethyl acetate (30.0 mL x 2). The organic layer was dried over anhydrous $Na_2SO_4$ and concentrated under reduced pressure to get crude residue. The crude residue was purified by flash chromatography using eluent (0 - 30%) of ethyl acetate in n-hexane to afford the titled compound methyl 2,2-dimethyl-3-morpholino-7-nitro-2H-benzo[b][1,4] oxazine-6-carboxylate (2.5 g, 80 % yield) as an off white solid.

MS (ES+) m/z = 350.41 (M+1).

$^1H$ NMR (400 MHz, DMSO-$d_6$) δ 7.54 (s, 1H), 7.27 (s, 1H), 3.70 -3.66 (m, 4H), 3.61- 3.57 (m, 4H), 3.33 (s, 3H), 1.57 (s, 6H).

**Step 2:** Methyl 7-amino-2,2-dimethyl-3-morpholino-2H-benzo[b][1,4]oxazine-6-carboxylate

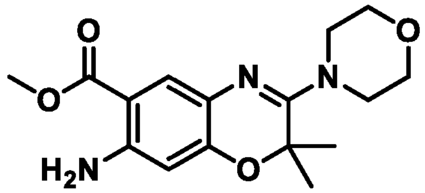

The titled compound was prepared from **Step 1** intermediate by employing similar protocol mentioned in **Example 14 Step 3** (1.8 g, 82.0% yield).

MS (ES+) m/z = 320.22 (M+1).

$^1H$ NMR (400 MHz, DMSO-$d_6$) δ 7.41 (s, 1H), 6.56 (s, 2H), 6.21 (s, 1H), 3.75 (s, 3H), 3.67 - 3.62 (m, 4H), 3.26 - 3.20 (m, 4H), 1.46 (s, 6H).

**Step 3:** 2,8,8-Trimethyl-7-morpholino-3,8-dihydro-4H-[1,4]oxazino[3,2-g]quinazolin-4-one

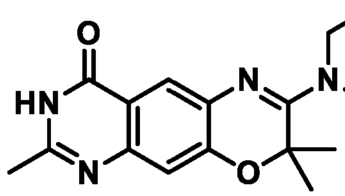

The titled compound was prepared from Step 2 intermediate by employing similar protocol mentioned in **Example 14 Step 4** (0.8 g, 51.9 % yield).

MS (ES+) m/z = 329.47 (M+1).

$^1$H NMR (400 MHz, DMSO-$d_6$) δ 11.95 (s, 1H), 7.59 (s, 1H), 6.92 (s, 1H), 3.72 - 3.65 (m, 4H), 3.46 - 3.40 (m, 4H), 2.30 (s, 3H), 1.54 (s, 6H).

**Step 4:** 4-Chloro-2,8,8-trimethyl-7-morpholino-8H-[1,4]oxazino[3,2-g]quinazoline

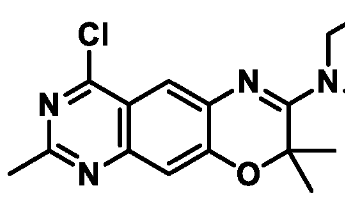

The titled compound was prepared from **Step 3** intermediate by employing similar protocol mentioned in **Example 28 Step 1** (0.21 g, 39.8% yield).

MS (ES+) m/z = 347.34 (M+1).

$^1$H NMR (400 MHz, DMSO-$d_6$) δ 7.61 (s, 1H), 7.27 (s, 1H), 3.71- 3.68 (m, 4H), 3.58 - 3.56 (m, 4H), 2.65 (s, 3H), 1.61 (s, 6H).

**Step 5:** (R)-1,1-Difluoro-1-(2-fluoro-3-(1-((2,8,8-trimethyl-7-morpholino-8H [1,4]oxazino[3,2-g] quinazolin-4-yl)amino) ethyl)phenyl)-2-methylpropan-2-ol. **(Compound 26)**

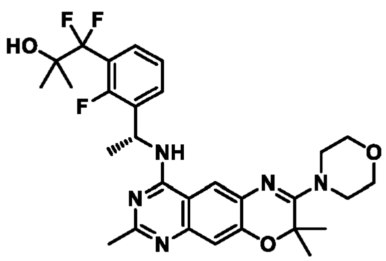

The titled compound was prepared by reaction of **Step 4** intermediate and (R)-1-(3-(1-Aminoethyl)-2-fluorophenyl)-1,1-difluoro-2-methylpropan-2-ol hydrochloride (Example 4-Step 6a) using similar protocol mentioned in **Example 28 Step 2** (0.081g, 33.6 % yield).

MS (ES+) m/z = 558.32 (M+1).

$^1$H NMR (400 MHz, DMSO-$d6$) δ 8.26 (d, $J$ = 7.6 Hz, 1H), 8.12 (s, 1H), 7.67 - 7.58 (m, 1H), 7.33 - 7.26 (m, 1H), 7.25 - 7.14 (m, 1H), 6.91 (s, 1H), 5.85 - 5.74 (m, 1H), 5.32 (s, 1H), 3.73 - 3.67 (m, 4H), 3.47 - 3.42 (m, 4H), 2.28 (s, 3H), 1.58 - 1.51 (m, 9H), 1.24 (s, 3H), 1.21 (s, 3H).

**Example 40:** Preparation of (R&S)-4-(((R)-1-(3-(1,1-difluoro-2-hydroxy-2-methylpropyl)-2-fluoro phenyl)ethyl) amino)-2,6,8,9-tetramethyl-8,9-dihydropyrazino[2,3-g]quinazolin-7(6H)-one.

**(Compound 27)**

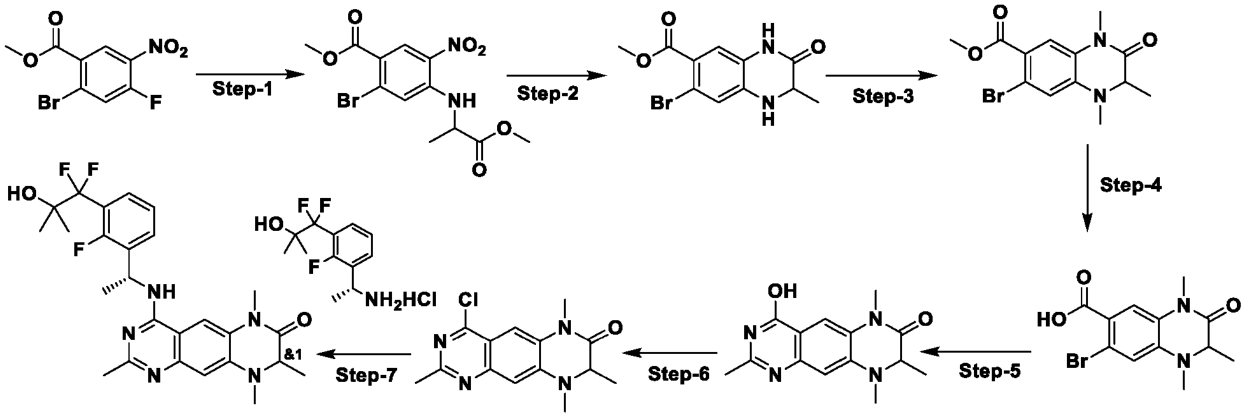


**Step 1:** Methyl 2-bromo-4-((1-methoxy-1-oxopropan-2-yl)amino)-5-nitrobenzoate.

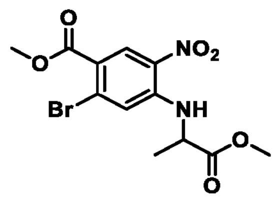

To the stirred solution of methyl 2-bromo-4-fluoro-5-nitrobenzoate (10.0 g, 36.0 mmol) (WO200879759) and methyl alaninate (5.56 g, 54.0 mmol) in DMF (100.0 mL) was added TEA (12.53 mL, 90.0 mmol). The reaction mixture was heated at 80°C for 2 h. After completion of the reaction, the reaction mixture was allowed to cool to room temperature, diluted with ethyl acetate (100.0 mL) and washed with $H_2O$ (50.0 mL x 3). The organic layer was dried over anhydrous $Na_2SO_4$ and concentrated under reduced pressure to get a crude residue. The crude residue was purified by flash chromatography using eluent (0 to 20%) of ethyl acetate in n-hexane to afford the titled compound methyl 2-bromo-4-((1-methoxy-1-oxopropan-2-yl)amino)-5-nitrobenzoate (10.0 g, 77.0% yield) as a yellow solid.

MS (ES+) m/z = 361.10 (M+1).

$^1H$ NMR (400 MHz, DMSO-$d_6$) δ 8.57 (d, $J$ = 7.6 Hz, 1H), 7.95 (s, 1H), 7.42 (s, 1H), 4.93 - 4.79 (m, 1H), 3.83 (s, 3H), 3.74 (s, 3H), 1.49 (d, $J$ = 6.9 Hz, 3H).

**Step 2:** Methyl 7-bromo-2-methyl-3-oxo-1,2,3,4-tetrahydroquinoxaline-6-carboxylate

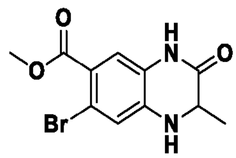

To a stirred solution of methyl 2-bromo-4-((1-methoxy-1-oxopropan-2-yl)amino)-5-nitrobenzoate (10.0 g, 27.7 mmol) in anhydrous ethanol (100.0 mL) was added tin(II) chloride (26.3 g, 138.0 mmol). The reaction mixture was heated at 95°C for 5 h. After completion of reaction, mixture was allowed to cool to room temperature. The reaction mixture was then neutralized with aqueous $NaHCO_3$ (10% 100.0 mL) and added ethyl acetate (100.0 mL) then filtered on a celite bed. After the filtrate was partitioned, the water layer was extracted with ethyl acetate (200.0 mL x 2). The combined organic layer was washed with water (100.0 mL x 2) and saturated brine (100.0 mL), dried over anhydrous $Na_2SO_4$, and concentrated under reduced pressure to get methyl 7-bromo-2-methyl-3-oxo-1,2,3,4-tetrahydroquinoxaline-6-carboxylate (6.5 g, 78.0%

yield) as a pale yellow solid.

MS (ES+) m/z = 299.21 (M+1).

$^{1}$H NMR (400 MHz, DMSO-$d_6$) δ 10.47 (s, 1H), 7.32 (s, 1H), 6.97 (s, 1H), 6.92 (s, 1H), 4.07 - 3.93 (m, 1H), 3.76 (s, 3H), 1.29 (d, *J* = 6.7 Hz, 3H).

**Step 3:** Methyl 7-bromo-1,2,4-trimethyl-3-oxo-1,2,3,4-tetrahydroquinoxaline-6-carboxylate.

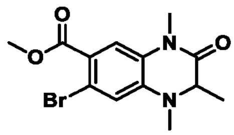

To a stirred solution of methyl 7-bromo-2-methyl-3-oxo-1,2,3,4-tetrahydroquinoxaline-6-carboxylate (3.0 g, 10.03 mmol) in DMF (20.0 mL) was added sodium hydride (60%) (1.44 g, 30.1 mmol) and methyl iodide (1.57 mL, 25.07 mmol) and stirred reaction mass for 30 min at 0 °C. After completion of the reaction, the reaction mixture was quenched with water (50.0 mL). The aqueous layer was extracted with ethyl acetate (100.0 mL), washed the organic layer with water (100.0 mL) and brine (100.0 mL), dried over anhydrous $Na_2SO_4$, and concentrated under reduced pressure to afford the titled compound methyl 7-bromo-1,2,4-trimethyl-3-oxo-1,2,3,4-tetrahydroquinoxaline-6-carboxylate (1.9 g, 57.9 % yield) as an off white solid.

MS (ES+) m/z = 327.22 (M+1), 329.22 (M+2).

$^{1}$H NMR (400 MHz, DMSO-$d_6$) δ 7.44 (s, 1H), 6.92 (s, 1H), 4.15 (q, *J* = 6.8 Hz, 1H), 3.81 (s, 3H), 3.29 (s, 3H), 2.91 (s, 3H), 1.08 (d, *J* = 6.8 Hz, 3H).

**Step 4:** 7-Bromo-1,2,4-trimethyl-3-oxo-1,2,3,4-tetrahydroquinoxaline-6-carboxylic acid

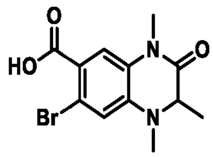

To a stirred solution of methyl 7-bromo-1,2,4-trimethyl-3-oxo-1,2,3,4-tetrahydroquinoxaline-6-carboxylate (4.1 g, 12.53 mmol) in methanol (20.0 mL), THF (20.0 mL), and water (20.0 mL) was added lithium hydroxide (3.0 g, 125 mmol). The reaction mixture was then stirred at 25 °C for 16 h. After completion of reaction, the reaction mass was concentrated under reduced pressure to get a crude compound. The crude compound was basified with 50% $NaHCO_3$ (50.0 mL) and extracted with ethyl acetate (100.0 mL x 2). The combined organic layer was washed with water (50.0 mL x 2) and saturated brine (100.0 mL), dried over anhydrous $Na_2SO_4$, and concentrated under reduced pressure to afford the titled compound 7-bromo-1,2,4-trimethyl-3-oxo-1,2,3,4-tetrahydroquinoxaline-6-carboxylic acid (3.2 g, 82.0 % yield) as a brown solid.

MS (ES+) m/z = 313.21 (M+1), 315.15 (M+2).

$^{1}$H NMR (400 MHz, DMSO-$d_6$) δ 12.84 (s, 1H), 7.46 (s, 1H), 6.89 (s, 1H), 4.14 (q, *J* = 6.8 Hz, 1H), 3.29 (s, 3H), 2.90 (s, 3H), 1.07 (d, *J* = 6.8 Hz, 3H).

**Step 5:** 2,6,8,9-Tetramethyl-3,6,8,9-tetrahydropyrazino[2,3-g]quinazoline-4,7-dione.

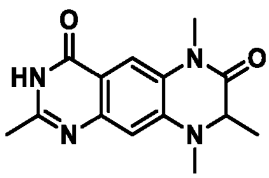

The titled compound was prepared from **Step 4** intermediate by employing similar protocol mentioned in

**Example 30 Step 8** (2.3 g, 83.0% yield).

MS (ES+) m/z = 273.15 (M+1).

$^1$H NMR (400 MHz, DMSO-$d_6$) δ 11.93 (s, 1H), 7.49 (s, 1H), 6.71 (s, 1H), 4.17 (q, *J* = 6.8 Hz, 1H), 3.36 (s, 3H), 2.94 (s, 3H), 2.30 (s, 3H), 1.09 (d, *J*= 6.8 Hz, 3H).

Step 6: 4-Chloro-2,6,8,9-tetramethyl-8,9-dihydropyrazino[2,3-g]quinazolin-7(6H)-one

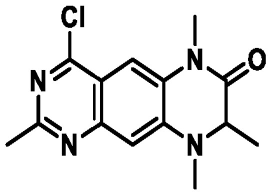

The titled compound was prepared from **Step 5** intermediate by employing similar protocol mentioned in **Example 28 Step 1** (0.38 g, 35.60 % yield).

MS (ES+) m/z = 291.21 (M+1), 293.27 (M+2).

$^1$H NMR (400 MHz, DMSO-$d_6$) δ 7.43 (s, 1H), 6.96 (s, 1H), 4.38 - 4.27 (m, 1H), 3.45 (s, 3H), 3.04 (s, 3H), 2.63 (s, 3H), 1.18 (d, *J* = 6.8 Hz, 3H).

**Step 7:** (R&S)-4-(((R)-1-(3-(1,1-difluoro-2-hydroxy-2-methylpropyl)-2-fluorophenyl)ethyl)amino)-2,6,8,9-tetramethyl-8,9-dihydropyrazino[2,3-g]quinazolin-7(6H)-one

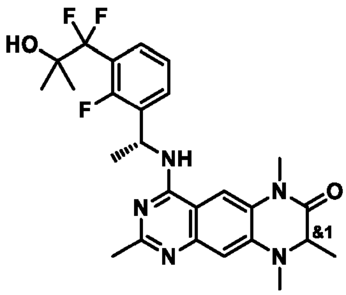

The titled compound was prepared by the reaction of **Step 6** intermediate and (R)-1-(3-(1-Aminoethyl)-2-fluorophenyl)-1,1-difluoro-2-methylpropan-2-ol hydrochloride (Example 4-Step 6a) using similar protocol mentioned in **Example 28 Step 2** (0.35 g, 54.80% yield). Chiral separation of the above compound gave two stereoisomers as

**Peak 1:** (S/R)-4-(((R)-1-(3-(1,1-difluoro-2-hydroxy-2-methylpropyl)-2-fluorophenyl)ethyl)amino)-2,6,8,9-tetramethyl-8,9-dihydropyrazino[2,3-g]quinazolin-7(6H)-one. **(Compound 27a)**

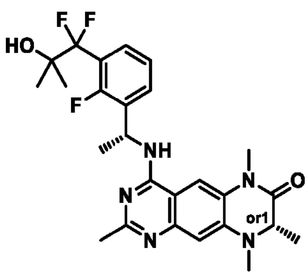

MS (ES+) m/z = 502.31 (M+1).

Chiral HPLC : ACN_0.1%DEA-MEOH-0.1% DEA (90:10) $t_{ret}$: 4.26 min

$^1H$ NMR (400 MHz, DMSO-$d_6$) δ 8.52 (s, 1H), 7.90 (s, 1H), 7.67 - 7.60 (m, 1H), 7.36 - 7.31 (m, 1H), 7.26 - 7.22 (m, 1H), 6.70 (s, 1H), 5.94 - 5.80 (m, 1H), 5.34 (s, 1H), 4.25 - 4.11 (m, 1H), 3.47 (s, 3H), 2.94 (s, 3H), 2.33 (s, 3H), 1.60 (d, *J* = 7.0 Hz, 3H), 1.24 (s, 3H), 1.21 (s, 3H), 1.11 (d, *J* = 6.9 Hz, 3H).

**Peak 2:** (R/S)-4-(((R)-1-(3-(1,1-difluoro-2-hydroxy-2-methylpropyl)-2-fluorophenyl)ethyl)amino)-2,6,8,9-tetramethyl-8,9-dihydropyrazino[2,3-g]quinazolin-7(6H)-one. **(Compound 27b)**

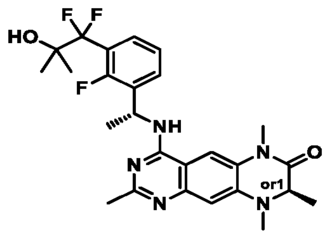

MS (ES+) m/z = 502.31 (M+1).

Chiral HPLC: ACN_0.1%DEA-MEOH-0.1% DEA (90:10) $t_{ret}$: 4.64 min

$^1H$ NMR (400 MHz, DMSO-$d_6$) δ 8.33 (s, 1H), 7.92 (s, 1H), 7.65 - 7.61 (m, 1H), 7.35 - 7.31 (m, 1H), 7.25 - 7.20 (m, 1H), 6.69 (s, 1H), 5.89 - 5.84 (m, 1H), 5.34 (s, 1H), 4.28 - 4.13 (m, 1H), 3.47 (s, 3H), 2.95 (s, 3H), 2.35 (s, 3H), 1.62 (d, *J* = 7.1 Hz, 3H), 1.24 (s, 3H), 1.21 (s, 3H), 1.13 - 1.09 (m, 3H).

**Example 41:** Preparation of (R)-4-((1-(3-(1,1-difluoro-2-hydroxy-2-methylpropyl)-2-fluorophenyl) ethyl)amino)-2,6,8,8,9-pentamethyl-8,9-dihydropyrazino[2,3-g]quinazolin-7(6H)-one **(Compound 28)**

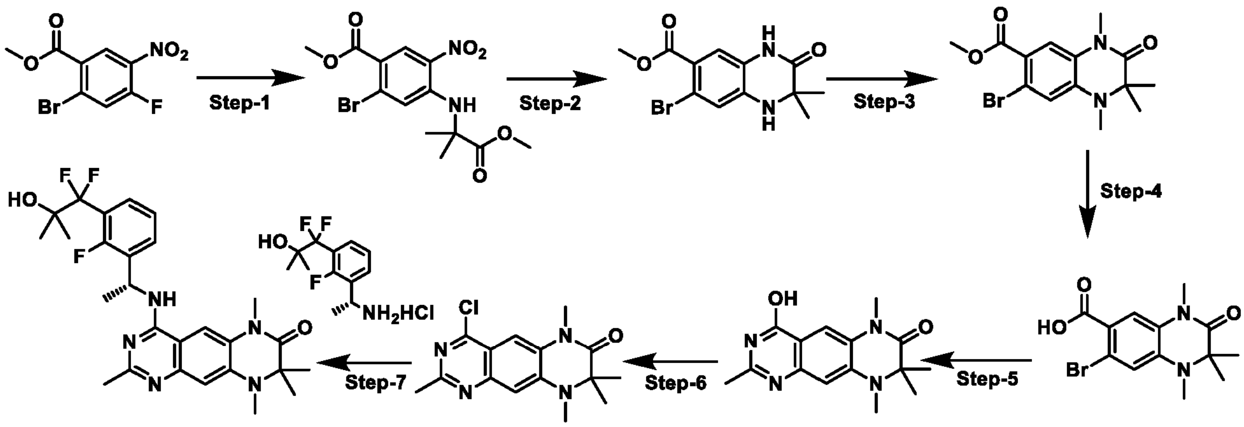


**Step 1:** Methyl 2-bromo-4-((1-methoxy-2-methyl-1-oxopropan-2-yl)amino)-5-nitrobenzoate.

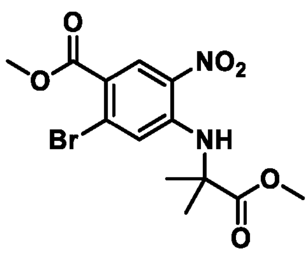

The titled compound was prepared from 2-bromo-4-fluoro-5-nitrobenzoate (WO200879759) intermediate by employing similar protocol mentioned in **Example 40 Step 1** (2.1 g, 78.0% yield)

$^1$H NMR (400 MHz, DMSO-$d_6$) δ 8.64 (s, 1H), 8.47 (s, 1H), 6.85 (s, 1H), 3.84 (s, 3H), 3.66 (d, *J*= 4.3 Hz, 3H), 1.65 (s, 6H).

**Step 2:** Methyl 7-bromo-2,2-dimethyl-3-oxo-1,2,3,4-tetrahydroquinoxaline-6-carboxylate

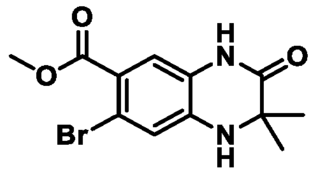

The titled compound was prepared from **Step 1** intermediate by employing similar protocol mentioned in **Example 40 Step 2** (1.40, 80.0% yield).
MS (ES+) m/z = 313.28 (M+1).
$^1$H NMR (400 MHz, Chloroform-d) δ 7.95 (s, 1H), 7.36 (s, 1H), 6.95 (s,1H), 3.93 (s, 1H), 3.91 (s, 3H), 1.47 (s, 6H).

**Step 3:** Methyl 7-bromo-1,2,2,4-tetramethyl-3-oxo-1,2,3,4-tetrahydroquinoxaline-6-carboxylate.

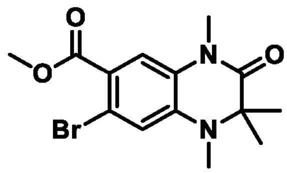

The titled compound was prepared from **Step 2** intermediate by employing similar protocol mentioned in **Example 40 Step 3** (1.2 g, 79.0% yield).
MS (ES+) m/z = 341.34 (M+1), 343.34 (M+2).
$^1$H NMR (400 MHz, DMSO-$d_6$) δ 7.44 (s, 1H), 7.02 (s, 1H), 3.82 (s, 3H), 3.31 (s, 3H), 2.87 (s, 3H), 1.31 (s, 6H).

**Step 4:** 7-Bromo-1,2,2,4-tetramethyl-3-oxo-1,2,3,4-tetrahydroquinoxaline-6-carboxylic acid.

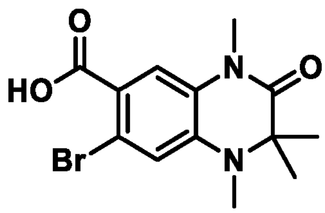

The titled compound was prepared from **Step 3** intermediate by employing similar protocol mentioned in **Example 40 Step 4** (1.1 g, 96.0% yield).
MS (ES+) m/z = 329.0 (M+2).
$^1$H NMR (400 MHz, DMSO-$d_6$) δ 12.92 (s, 1H), 7.46 (s, 1H), 6.99 (s, 1H), 3.31 (s, 3H), 2.86 (s, 3H), 1.30 (s, 6H).

**Step 5:** 2,6,8,8,9-Pentamethyl-3,6,8,9-tetrahydropyrazino[2,3-g]quinazoline-4,7-dione.

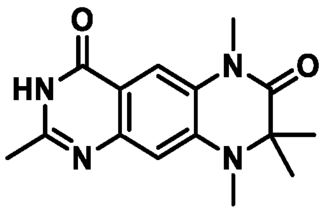

The titled compound was prepared from **Step 4** intermediate by employing similar protocol mentioned in **Example 30 Step 8** (0.70 g, 72.70% yield).

MS (ES+) m/z = 287.34 (M+1).

$^1H$ NMR (400 MHz, DMSO-$d_6$) δ 11.94 (s, 1H), 7.49 (s, 1H), 6.83 (s, 1H), 3.38 (s, 3H), 2.90 (s, 3H), 2.31 (s, 3H), 1.32 (s, 6H).

**Step 6:** 4-Chloro-2,6,8,8,9-pentamethyl-8,9-dihydropyrazino[2,3-g]quinazolin-7(6H)-one

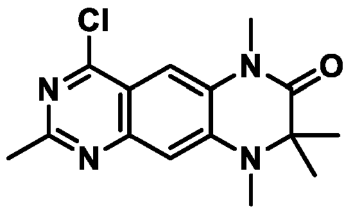

The titled compound was prepared from **Step 5** intermediate by employing similar protocol mentioned in **Example 28 Step 1** (0.15g, 28.20% yield).

MS (ES+) m/z = 305.28 (M+1).

$^1H$ NMR (400 MHz, DMSO-$d_6$) δ 7.44 (s, 1H), 7.08 (s, 1H), 3.47 (s, 3H), 3.01 (s, 3H), 2.64 (s, 3H), 1.41 (s, 6H).

**Step 7:** (R)-4-((1-(3-(1,1-Difluoro-2-hydroxy-2-methylpropyl)-2-fluorophenyl)ethyl)amino)-2,6,8,8,9-pentamethyl-8,9-dihydropyrazino[2,3-g]quinazolin-7(6H)-one

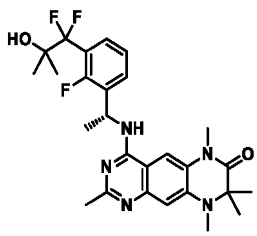

The titled compound was prepared from **Step 6** intermediate using appropriate chiral amine by employing similar protocol mentioned in **Example 28 Step 2** (0.065g, 29.60% yield).

MS (ES+) m/z = 516.28 (M+1).

$^1H$ NMR (400 MHz, DMSO-$d_6$) δ 9.96 (s, 1H), 8.15 (s, 1H), 7.75 - 7.71 (m, 1H), 7.45 - 7.34 (m, 1H), 7.32 - 7.27 (m, 1H), 6.85 (s, 1H), 5.99 - 5.92 (m, 1H), 5.36 (s, 1H), 3.50 (s, 3H), 3.38 (s, 3H), 2.96 (s, 3H), 1.69 (d, *J* = 7.0 Hz, 3H), 1.43 (s, 3H), 1.40 (s, 3H), 1.23 (s, 3H), 1.21 (s, 3H),

**Example 42:** Preparation of (R)-9-((1-(3-(1,1-difluoro-2-hydroxy-2-methylpropyl)-2-fluorophenyl) ethyl)amino)-1,3,7-trimethylpyrimido[4,5-g]quinazoline-2,4(1H,3H)-dione **(Compound 29)**

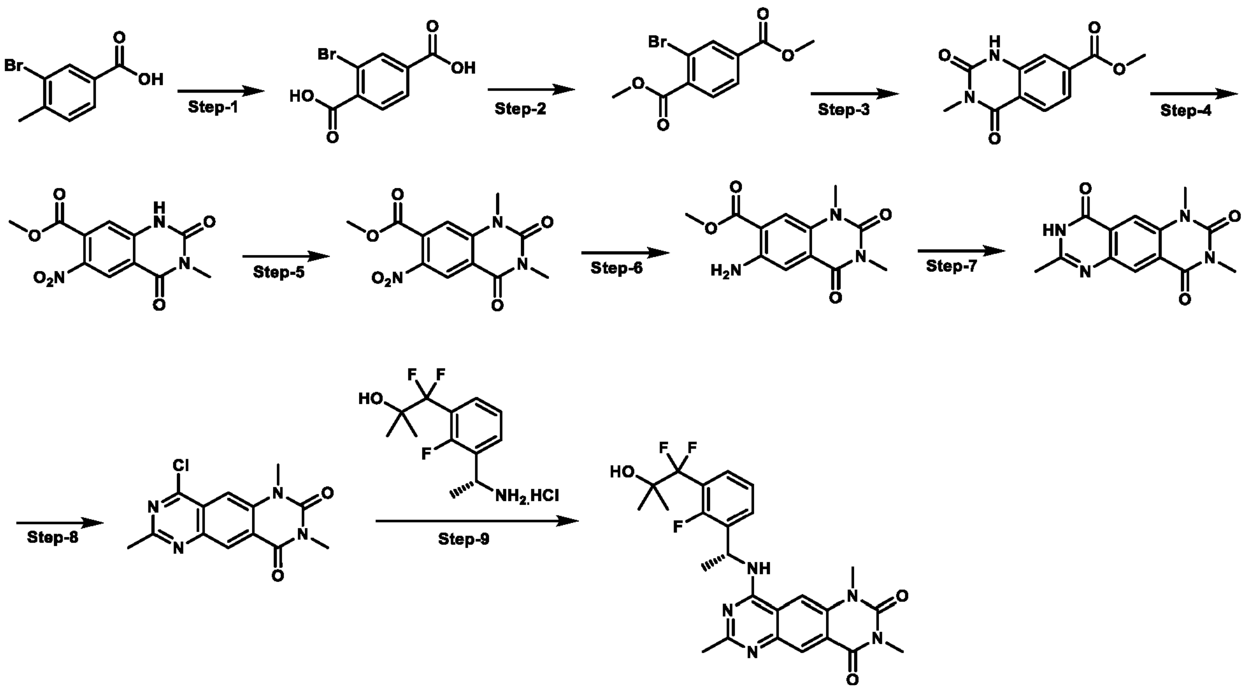


**Step 1:** 2-Bromoterephthalic acid

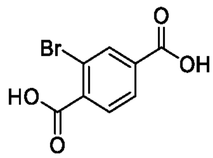

To a solution of 3-bromo-4-methylbenzoic acid (15.0 g, 69.8 mmol) in 200.0 mL of 1:1 mixture of t-butanol and water, was added potassium permanganate (62.8 g, 398 mmol). The reaction mixture was heated at 90°C for 16 h. Thereafter, reaction mixture was cooled to room temperature and filtered. The filtrate was concentrated, and residue was further acidified with concentrated hydrochloric acid. The resultant solution was further diluted with water (10.0 mL) and the resulting precipitates were collected via filtration. The precipitates were further dried to provide 2-bromoterephthalic acid as a crude white solid. The crude residue was purified by column chromatography using eluent 60-70% ethyl acetate in n-hexane to afford the titled compound 2-bromoterephthalic acid (15.1 g, 88.0 % yield) as white solid.

$^1$H NMR (400 MHz, DMSO-$d_6$) δ 13.60 (s, 2H), 8.15 (d, *J* = 1.6 Hz, 1H), 7.98 (dd, *J* = 8.0, 1.6 Hz, 1H), 7.83 (d, *J* = 8.0 Hz, 1H).

**Step 2:** Dimethyl 2-bromoterephthalate

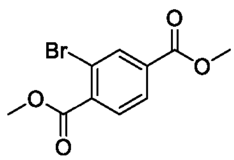

To a stirred solution of 2-bromoterephthalic acid (15.0 g, 61.2 mmol) in methanol (150.0 mL) was added dropwise thionyl chloride (4.91 mL, 67.3 mmol) at 0°C. The reaction mixture was allowed to warm to room temperature and stirred further for 16 h. The rection was monitored by TLC, the reaction mixture was concentrated. Added saturated solution of sodium bicarbonate to the residue and extracted using dichloromethane (3 x 150.0 mL). The combined organic phase was washed with water (200.0 mL), brine (100.0 mL) and dried over anhydrous sodium sulphate. Concentrate this reaction

mixture to afford 26.7 g crude, which was purified by column chromatography using eluent 0-30% of ethyl acetate in n-hexane to afford the titled compound dimethyl 2-bromoterephthalate (15.5 g, 93 % yield) as off white solid.

MS (ES+) m/z = 274.04 (M+1).

1H NMR (400 MHz, Chloroform-d) δ 8.33 (d, J = 1.6 Hz, 1H), 8.02 (dd, J = 8.1, 1.6 Hz, 1H), 7.83 (d, J = 8.2 Hz, 1H), 3.98 (s, 3H), 3.97 (s, 3H).

**Step 3:** Methyl 3-methyl-2,4-dioxo-1,2,3,4-tetrahydroquinazoline-7-carboxylate

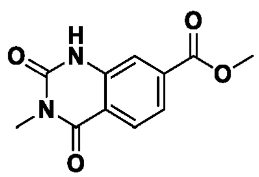

To a solution of dimethyl 2-bromoterephthalate (3.00 g, 10.99 mmol) and 1-methylurea (1.06 g, 14.28 mmol) in 1,4-dioxane (10.0 mL) was added cesium carbonate (7.16 g, 21.97 mmol), xantphos (0.64 g, 1.099 mmol) and tris(dibenzylideneacetone)dipalladium(0) (1.00 g, 1.099 mmol). The reaction mixture was heated to 120°C for 16 h. The reaction mixture was then cooled to room temperature and concentrated to afford crude solid. To the crude solid add 60.0 mL dichloromethane and stirred it for 5 minutes. Filtered this reaction mixture to get crude solid which was purified by column chromatography using eluent 0-10% of methanol in dichloromethane to afford the titled compound methyl 3-methyl-2,4-dioxo-1,2,3,4-tetrahydroquinazoline-7-carboxylate 1.5 g, 58.3 % yield as a light yellow solid.

MS (ES+) m/z = 235.27 (M+1).

$^1$H NMR (400 MHz, DMSO-$d_6$) δ 11.63 (s, 1H), 8.06 (d, *J* = 8.2 Hz, 1H), 7.80 - 7.67 (m, 2H), 3.90 (s, 3H), 3.27 (s, 3H).

**Step 4:** Methyl 3-methyl-6-nitro-2,4-dioxo-1,2,3,4-tetrahydroquinazoline-7-carboxylate

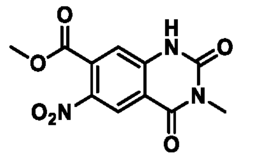

To a solution of methyl 3-methyl-2,4-dioxo-1,2,3,4-tetrahydroquinazoline-7-carboxylate (0.5 g, 2.135 mmol) in sulphuric acid (10.0 mL) at 0°C was slowly added potassium nitrate (0.22 g, 2.13 mmol). This reaction mixture was stirred at room temperature for 2 h and poured this into ice water (10.0 mL). The resulting solution was extracted with 3 x 10.0 mL of dichloromethane, washed with brine, dried over anhydrous sodium sulfate. Concentrate this reaction mixture to afford crude residue, which was purified by column chromatography using eluent 0 - 50% of ethyl acetate in n-hexane to afford the titled compound methyl 3-methyl-6-nitro-2,4-dioxo-1,2,3,4-tetrahydroquinazoline-7-carboxylate (0.5 g, 84.0 % yield) as a light yellow solid.

MS (ES+) m/z = 278.21 (M+).

$^1$H NMR (400 MHz, DMSO-$d_6$) δ 12.18 (s, 1H), 8.53 (s, 1H), 7.39 (s, 1H), 3.90 (s, 3H), 3.27 (s, 3H).

**Step 5:** Methyl 1,3-dimethyl-6-nitro-2,4-dioxo-1,2,3,4-tetrahydroquinazoline-7-carboxylate

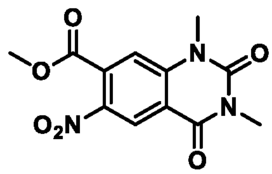

The titled compound was prepared from **Step 4** intermediate by employing similar protocol mentioned in **Example 40 Step 3** (1.6 g, 95.0% yield).
MS (ES+) m/z = 294.02 (M+1).
$^1$H NMR (400 MHz, DMSO-$d_6$) δ 8.64 (s, 1H), 7.83 (s, 1H), 3.93 (s, 3H), 3.58 (s, 3H), 3.33 (s, 3H).

**Step 6:** Methyl 6-amino-1, 3-dimethyl-2,4-dioxo-1,2,3,4-tetrahydroquinazoline-7-carboxylate

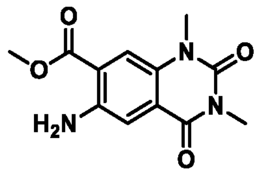

The titled compound was prepared from **Step 5** intermediate by employing similar protocol mentioned in **Example 14 Step 3** (1.35 g, 94.0 % yield) off white solid.
MS (ES+) m/z = 264.15 (M+1).
$^1$H NMR (400 MHz, DMSO-$d_6$) δ 7.63 (d, $J$ = 2.5 Hz, 1H), 7.54 (d, $J$ = 1.8 Hz, 1H), 6.60 (s, 2H), 3.88 (s, 3H), 3.46 (s, 3H), 3.29 (s, 3H).

**Step 7:** 1, 3, 7-Trimethyl-1, 8-dihydropyrimido[4,5-g]quinazoline-2,4,9(3H)-trione

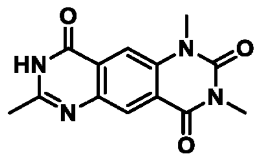

The titled compound was prepared from **Step 6** intermediate by employing similar protocol mentioned in **Example 14 Step 4** (1.1 g, 82.0% yield).
MS (ES+) m/z = 273.15 (M+1).

**Step 8:** 9-Chloro-1, 3, 7-trimethylpyrimido[4,5-g]quinazoline-2,4(1H,3H)-dione

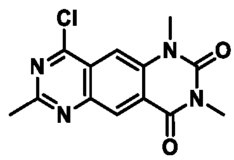

The titled compound was prepared from **Step 7** intermediate by employing similar protocol mentioned in **Example 28 Step 1** (0.35 g, 43.70% yield).
MS (ES+) m/z = 291.21 (M+1).
$^1$H NMR (400 MHz, DMSO-$d_6$) δ 8.21 (s, 1H), 7.92 (s, 1H), 3.61 (s, 3H), 3.34 (s, 3H), 2.43 (s, 3H).

**Step 9:** (R)-9-((1-(3-(1,1-difluoro-2-hydroxy-2-methylpropyl)-2-fluorophenyl) ethyl)amino)-1,3,7-trimethylpyrimido[4,5-g]quinazoline-2,4(1H,3H)-dione (6) **(Compound 29)**

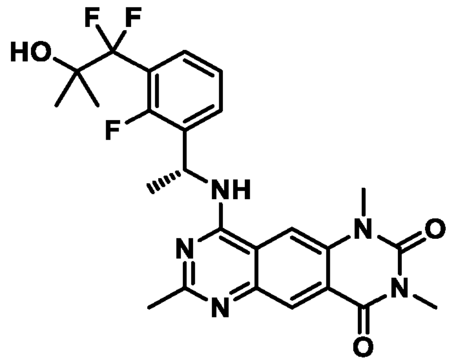

The titled compound was prepared by reaction of **Step 8** intermediate and (R)-1-(3-(1-Aminoethyl)-2-fluorophenyl)-1,1-difluoro-2-methylpropan-2-ol hydrochloride (Example 4-Step 6a) using similar protocol mentioned in **Example 28 Step 2** (0.04 g, 23.19% yield).

MS (ES+) m/z = 502.43 (M+1).

$^1$H NMR (400 MHz, DMSO- $d_6$) δ 8.81 (s, 1H), 8.24 - 8.18 (m, 2H), 7.65 - 7.58 (m, 1H), 7.39 - 7.29 (m, 1H), 7.25 - 7.20 (m, 1H), 5.88 - 5.79 (m, 1H), 5.35 (s, 1H), 3.66 (s, 3H), 3.37 (s, 3H), 2.37 (s, 3H), 1.65 (d, $J$ = 7.0 Hz, 3H), 1.24 (s, 3H), 1.21 (s, 3H),

**Example 43:** (R&S)-4-(((R)-1-(3-(1,1-Difluoro-2-hydroxyethyl)-2-fluorophenyl)ethyl)amino)-2,6,8-trimethyl-6H-[1,4]oxazino[3,2-g]quinazolin-7(8H)-one. **(Compound 30)**

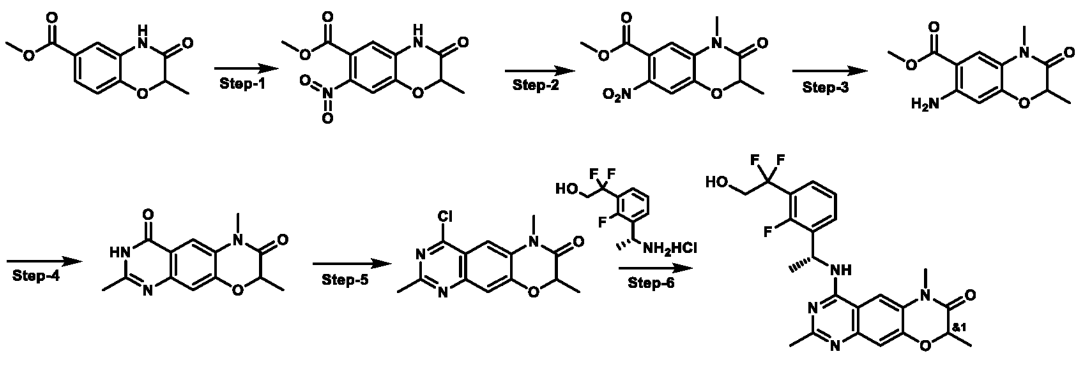


(30a & 30b)

**Step 1:** Methyl 2-methyl-7-nitro-3-oxo-3,4-dihydro-2H-benzo[b][1,4]oxazine-6-carboxylate.

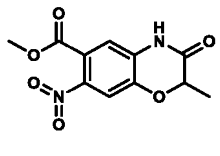

The titled compound was by employing similar protocol mentioned in **Example 14 Step 1** using methyl 2-methyl-3-oxo-3,4-dihydro-2H-benzo[b][1,4]oxazine-6-carboxylate (Reference: US5597820). (12.2 g, 91 % yield).

$^1$H NMR (400 MHz, DMSO-$d_6$) δ 11.30 (s, 1H), 7.69 (s, 1H), 7.24 (s, 1H), 4.93 (q, $J$ = 6.8 Hz, 1H), 3.82 (s, 3H), 1.48 (d, $J$ = 6.8 Hz, 3H).

**Step 2:** Methyl 2,4-dimethyl-7-nitro-3-oxo-3,4-dihydro-2H-benzo[b][1,4]oxazine-6-carboxylate.

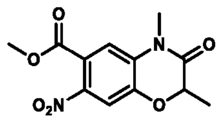

The titled compound was prepared from Step 1 intermediate by employing similar protocol mentioned in **Example 14 Step 2** (11.0 g, 87.0% yield).

$^1H$ NMR (400 MHz, DMSO-$d_6$) δ 7.75 (s, 1H), 7.53 (s, 1H), 4.98 (q, *J* = 6.8 Hz, 1H), 3.85 (s, 3H), 3.35 (s, 3H), 1.50 (d, *J* = 6.8 Hz, 3H).

**Step 3:** Methyl 7-amino-2,4-dimethyl-3-oxo-3,4-dihydro-2H-benzo[b][1,4]oxazine-6-carboxylate.

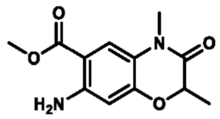

The titled compound was prepared from **Step 2** intermediate by employing similar protocol mentioned in **Example 14 Step 3** (7.2 g, 73.3% yield).

$^1H$ NMR (400 MHz, DMSO-$d_6$) δ 7.33 (s, 1H), 6.63 (s, 2H), 6.41 (s, 1H), 4.73 (q, *J* = 6.7 Hz, 1H), 3.79 (s, 3H), 3.22 (s, 3H), 1.41 (d, *J* = 6.8 Hz, 3H).

**Step 4:** 2,6,8-Trimethyl-3,6-dihydro-4H-[1,4]oxazino[3,2-g]quinazoline-4,7(8H)-dione.

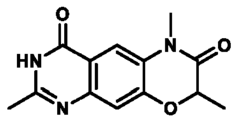

The titled compound was prepared from **Step 3** intermediate by employing similar protocol mentioned in **Example 14 Step 4** (5.2 g, 71.7 % yield).

$^1H$ NMR (400 MHz, DMSO-$d_6$) δ 12.38 (s, 1H), 7.64 (s, 1H), 7.13 (s, 1H), 4.91 (q, *J* = 6.8 Hz, 1H), 3.37 (s, 3H), 2.36 (s, 3H), 1.49 (d, *J* = 6.8 Hz, 3H).

**Step 5:** 4-Chloro-2,6,8-trimethyl-6H-[1,4]oxazino[3,2-g]quinazolin-7(8H)-one.

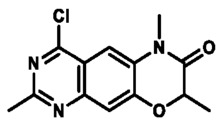

The titled compound was prepared from **Step 4** intermediate by employing similar protocol mentioned in **Example 28 Step 1** (1.3 g, 60.7 % yield).

$^1H$ NMR (400 MHz, DMSO-$d_6$) δ 7.61 (s, 1H), 7.48 (s, 1H), 5.05 (q, *J* = 6.8 Hz, 1H), 3.45 (s, 3H), 2.69 (s, 3H), 1.54 (d, *J* = 6.8 Hz, 3H).

**Step 6:** (R&S)-4-(((R)-1-(3-(1,1-difluoro-2-hydroxyethyl)-2-fluorophenyl)ethyl)amino)-2,6,8-trimethyl-6H-[1,4]oxazino [3,2-g]quinazolin-7(8H)-one . **(Compound 30)**

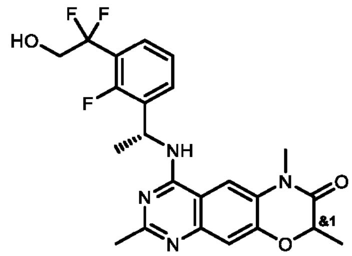

The titled compound was prepared from **Step 5** intermediate by using appropriate chiral amine employing similar protocol mentioned in **Example 28 Step 2** (0.22 g, 53.10% yield).

This compound was separated by chiral preparative HPLC to give two stereoisomers as-

**Peak 1:** (S/R)-4-(((R)-1-(3-(1,1-difluoro-2-hydroxyethyl)-2-fluorophenyl)ethyl)amino)-2,6,8-trimethyl-6H-[1,4]oxazino[3,2-g]quinazolin-7(8H)-one. **(Compound 30a)**

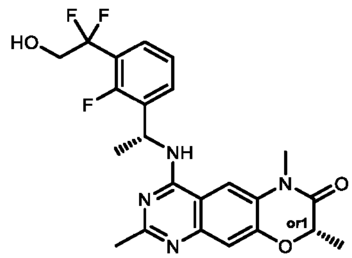

MS (ES+) m/z = 461.17 (M+1).

$^1$H NMR (400 MHz, DMSO-$d_6$) δ 8.35 (d, *J* = 7.3 Hz, 1H), 8.00 (s, 1H), 7.65 - 7.60 (m, 1H), 7.45 - 7.40 (m, 1H), 7.29 - 7.23 (m, 1H), 7.10 (s, 1H), 5.87 - 5.78 (m, 1H), 5.73 (t, *J* = 6.4 Hz, 1H), 4.92 - 4.82 (m, 1H), 4.01 - 3.88 (m, 2H), 3.46 (s, 3H), 2.32 (s, 3H), 1.62 (d, *J* = 7.1 Hz, 3H), 1.49 (d, *J* = 6.7 Hz, 3H).

Chiral HPLC: HEX 0.1%DEA:IPA-MeOH(1:1)(60:40) $t_{ret}$: 4.72 min

**Peak 2:** (R/S)-4-(((R)-1-(3-(1,1-difluoro-2-hydroxyethyl)-2-fluorophenyl)ethyl)amino)-2,6,8-trimethyl-6H-[1,4]oxazino[3,2-g]quinazolin-7(8H)-one. **(Compound 30b)**

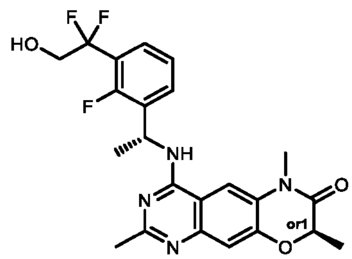

MS (ES+) m/z = 461.17 (M+1).

$^1$H NMR (400 MHz, DMSO-$d_6$) δ 8.35 (d, *J* = 7.3 Hz, 1H), 8.00 (s, 1H), 7.70 - 7.61 (m, 1H), 7.47 - 7.38 (m, 1H), 7.26 (t, *J* = 7.7 Hz, 1H), 7.10 (s, 1H), 5.89 - 5.77 (m, 1H), 5.77 - 5.71 (m, 1H), 4.94 - 4.84 (m, 1H), 4.01 - 3.89 (m, 2H), 3.46 (s, 3H), 2.32 (s, 3H), 1.62 (d, *J* = 7.0 Hz, 3H), 1.48 (d, *J* = 6.8 Hz, 3H).

Chiral HPLC: HEX 0.1%DEA:IPA-MeOH(1:1) (60:40) $t_{ret}$: 5.41 min

**Example 44:** (R&S)-4-(((R)-1-(3-(1,1-Difluoro-2-hydroxy-2-methylpropyl)-2-fluorophenyl) ethyl) amino)-2,6,8-trimethyl-6H-[1,4]oxazino[3,2-g]quinazolin-7(8H)-one. **(Compound 31)**

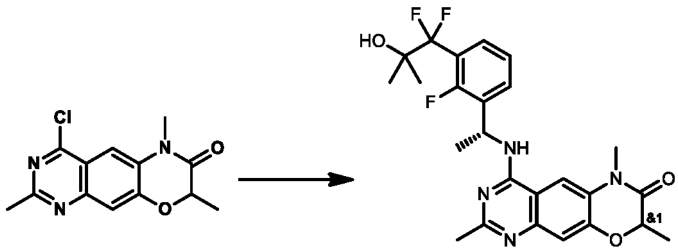
(31a) & (31b)

The titled compound was prepared by reaction of **Example 43 Step 5** intermediate and (R)-1-(3-(1-Aminoethyl)-2-fluorophenyl)-1,1-difluoro-2-methylpropan-2-ol hydrochloride (Example 4-Step 6a) by using similar protocol mentioned in **Example 28 Step 2** (0.3 g, 54.80% yield).

The two stereoisomers were separated by chiral preparative HPLC

**Peak 1:** (S/R)-4-(((R)-1-(3-(1,1-Difluoro-2-hydroxy-2-methylpropyl)-2-fluorophenyl)ethyl) amino)-2,6,8-trimethyl-6H-[1,4]oxazino[3,2-g]quinazolin-7(8H)-one. **(Compound 31a)**

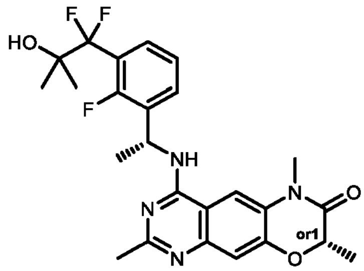

MS (ES+) m/z = 489.1 (M+1).

Chiral HPLC: HEX 0.1%DEA:IPA-MeOH(1:1) (60:40) $t_{ret}$: 4.23 min

$^1$H NMR (400 MHz, DMSO-$d_6$) δ 8.51 (s, 1H), 8.05 (s, 1H), 7.63 - 7.58 (m, 1H), 7.38 - 7.26 (m, 1H), 7.25 - 7.18 (m, 1H), 7.11 (s, 1H), 5.91 - 5.78 (m, 1H), 5.34 (s, 1H), 4.95 - 4.79 (m, 1H), 3.46 (s, 3H), 2.32 (s, 3H), 1.62 (d, *J* = 7.0 Hz, 3H), 1.50 (d, *J* = 6.8 Hz, 3H), 1.24 (s, 3H), 1.21 (s, 3H) .

**Peak 2:** (R/S)-4-(((R)-1-(3-(1,1-difluoro-2-hydroxy-2-methylpropyl)-2-fluorophenyl)ethyl) amino)-2,6,8-trimethyl-6H-[1,4]oxazino[3,2-g]quinazolin-7(8H)-one. **(Compound 31b)**

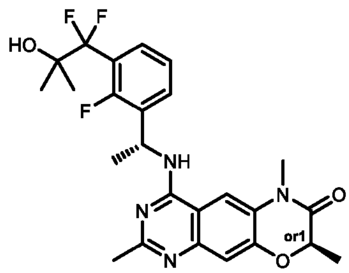

MS (ES+) m/z = 489.2 (M+1).

Chiral HPLC: HEX 0.1%DEA:IPA-MeoH(1:1) (60:40) $t_{ret}$: 4.69 min

$^1$HNMR (400 MHz, DMSO-$d_6$) δ 8.57 (s, 1H), 8.06 (s, 1H), 7.65 - 7.60 (m, 1H), 7.41 - 7.27 (m, 1H), 7.25 - 7.20 (m, 1H), 7.12 (s, 1H), 5.91 - 5.77 (m, 1H), 5.34 (s, 1H), 4.97 - 4.85 (m, 1H), 3.46 (s, 3H), 2.33 (s, 3H), 1.62 (d, *J* = 7.0 Hz, 3H), 1.49 (d, *J* = 6.8 Hz, 3H), 1.24 (s, 3H), 1.21 (s, 3H) .

**Example 45:** (R&S)-4-(((R)-1-(3-(1,1-difluoro-2-hydroxy-2-methylpropyl)-2-fluorophenyl) ethyl)amino)-8-isopropyl-2,6-dimethyl-6H-[1,4]oxazino[3,2-g]quinazolin-7(8H)-one. **(Compound 32)**

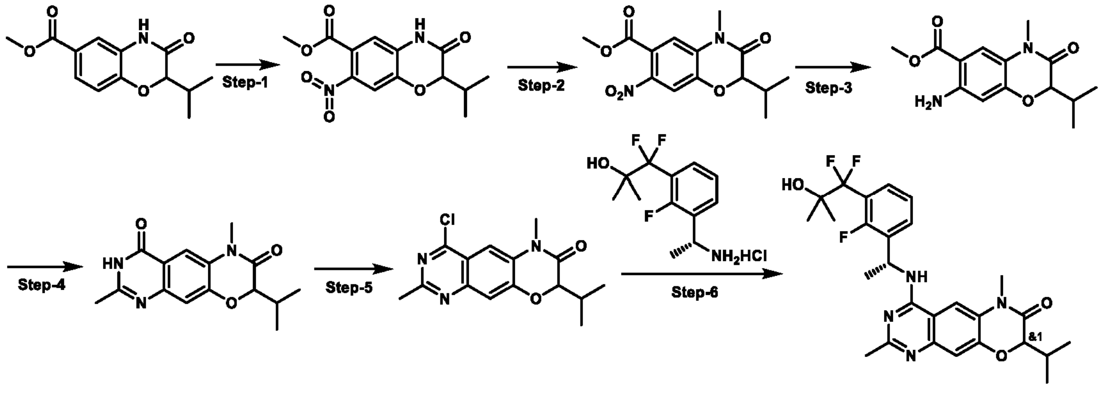


(32a) and (32b)

**Step 1:** Methyl 2-isopropyl-7-nitro-3-oxo-3,4-dihydro-2H-benzo[b][1,4]oxazine-6-carboxylate

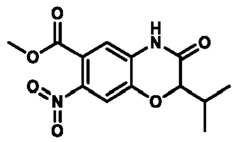

The titled compound was prepared from methyl 2-isopropyl-3-oxo-3,4-dihydro-2H-benzo[b][1,4]oxazine-6-carboxylate (prepared by method described in WO2017/40963) by employing similar protocol mentioned in **Example 14 Step 1** (5.10 g, 90.0% yield)

$^1$H NMR (400 MHz, DMSO-$d_6$) δ 11.33 (s, 1H), 7.71 (s, 1H), 7.22 (s, 1H), 4.67 (d, *J* = 4.9 Hz, 1H), 3.82 (s, 3H), 2.31-2.19 (m, 1H), 1.04 (d, *J* = 6.9 Hz, 3H), 0.94 (d, *J* = 6.7 Hz, 3H).

**Step 2:** Methyl 2-isopropyl-4-methyl-7-nitro-3-oxo-3,4-dihydro-2H-benzo[b][1,4]oxazine-6-carboxylate.

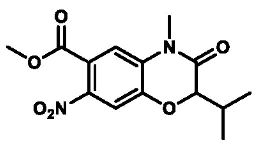

The titled compound was prepared from **Step 1** intermediate by employing similar protocol mentioned in **Example 14 Step 2** (4.10 g, 78.0 % yield).

$^1$H NMR (400 MHz, DMSO-$d_6$) δ 7.77 (s, 1H), 7.50 (s, 1H), 4.69 *(d, J* = 5.3 Hz, 1H), 3.85 (s, 3H), 3.36 (s, 3H), 2.27-2.19 (m, 1H), 1.14 - 0.99 (m, 3H), 0.94 (d, *J* = 6.7 Hz, 3H).

**Step 3:** Methyl 7-amino-2-isopropyl-4-methyl-3-oxo-3,4-dihydro-2H-benzo[b][1,4]oxazine-6-carboxylate.

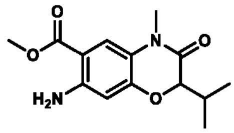

The titled compound was prepared from **Step 2** intermediate by employing similar protocol mentioned in **Example 14 Step 3** (2.81 g, 78.0 % yield).

$^1$H NMR (400 MHz, DMSO-$d_6$) δ 7.32 (s, 1H), 6.61 (s, 2H), 6.41 (s, 1H), 4.43 (d, $J$ = 5.7 Hz, 1H), 3.78 (s, 3H), 3.23 (s, 3H), 2.23-2.17 (m, 1H), 0.98 (d, $J$ = 6.9 Hz, 3H), 0.90 (d, $J$ = 6.7 Hz, 3H).

**Step 4:** 8-Isopropyl-2,6-dimethyl-3,6-dihydro-4H-[1,4]oxazino[3,2-g]quinazoline-4,7(8H)-dione.

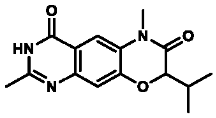

The titled compound was prepared from **Step 3** intermediate by employing similar protocol mentioned in **Example 14 Step 4** (1.75 g, 60.5% yield).

MS (ES+) m/z = 288.0(M+1).

**Step 5:** 4-Chloro-8-isopropyl-2,6-dimethyl-6H-[1,4]oxazino[3,2-g]quinazolin-7(8H)-one.

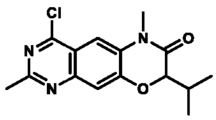

The titled compound was prepared from **Step 4** intermediate by employing similar protocol mentioned in **Example 28 Step 1** (0.95 g, 59.5% yield).

MS (ES+) m/z = 306.34 (M+), 308.40 (M+2).

**Step 6:** (R&S)-4-(((R)-1-(3-(1,1-Difluoro-2-hydroxy-2-methylpropyl)-2-fluorophenyl)ethyl)amino)-8-isopropyl-2,6-dimethyl-6H-[1,4]oxazino[3,2-g]quinazolin-7(8H)-one. **(Compound 32)**

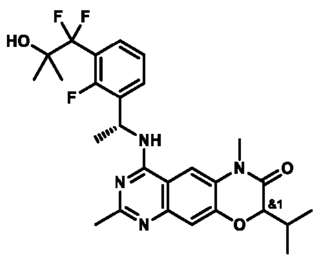

The titled compound was prepared by reaction of **Step 5** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.3 g, 50.76 % yield).

Chiral separation of the above compound gave two stereoisomers as -

**Peak 1:** (S/R)-4-(((R)-1-(3-(1,1-Difluoro-2-hydroxy-2-methylpropyl)-2-fluorophenyl) ethyl)amino)-8-isopropyl-2,6-dimethyl-6H-[1,4]oxazino[3,2-g]quinazolin-7(8H)-one **(Compound 32a)**

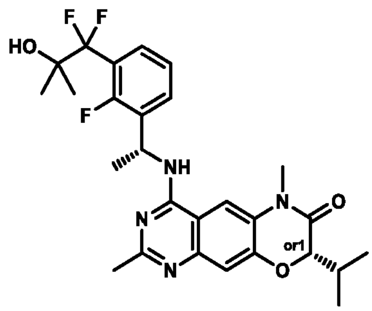

MS (ES+) m/z = 516.82 (M+).

Chiral HPLC: HEX 0.1%DEA:IPA-MeOH(1:1) (60:40) $t_{ret}$: 3.95 min

$^1$HNMR (400 MHz, DMSO-$d_6$) δ 8.48 (s, 1H), 8.02 (s, 1H), 7.66 - 7.57 (m, 1H), 7.38 - 7.27 (m, 1H), 7.23 (t, *J* = 7.7 Hz, 1H), 7.11 (s, 1H), 5.89 - 5.75 (m, 1H), 5.34 (s, 1H), 4.58 (d, *J* = 5.7 Hz, 1H), 3.47 (s, 3H), 2.32 (s, 3H), 2.22 - 2.07 (m, 1H), 1.61 (d, *J* = 7.0 Hz, 3H), 1.24 (s, 3H), 1.21 (s, 3H), 1.03 (d, *J* = 6.8 Hz, 3H), 0.94 (d, *J* = 6.7 Hz, 3H).

**Peak 2:** (R/S)-4-(((R)-1-(3-(1,1-Difluoro-2-hydroxy-2-methylpropyl)-2-fluorophenyl)ethyl) amino)-8-isopropyl-2,6-dimethyl-6H-[1,4]oxazino[3,2-g]quinazolin-7(8H)-one **(Compound 32b)**

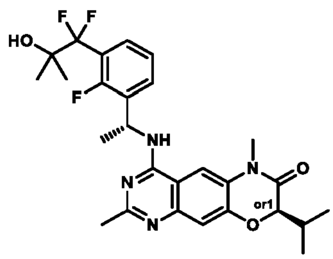

MS (ES+) m/z = 516.82 (M+).

Chiral HPLC: HEX 0.1%DEA:IPA-MeOH(1:1) (60:40) $t_{ret}$: 4.49 min

$^1$H NMR (400 MHz, DMSO-*d*6) δ 8.53 (s, 1H), 8.03 (s, 1H), 7.72 - 7.57 (m, 1H), 7.41 - 7.28 (m, 1H), 7.22 (t, *J* = 7.7 Hz, 1H), 7.11 (s, 1H), 5.89 - 5.75 (m, 1H), 5.34 (s, 1H), 4.58 (d, *J* = 6.1 Hz, 1H), 3.47 (s, 3H), 2.32 (s, 3H), 2.22 -2.07 (m, 1H), 1.62 (d, *J* = 7.1 Hz, 3H), 1.24 (s, 3H), 1.21 (s, 3H), 1.01 (d, *J* = 6.8 Hz, 3H), 0.93 (d, *J* = 6.7 Hz, 3H).

**Example 46:** (R)-4-((1-(3-(1,1-Difluoro-2-hydroxy-2-methylpropyl)-2-fluorophenyl)ethyl) amino)-2,6,8,8-tetramethyl-6H-[1,4]oxazino[3,2-g]quinazolin-7(8H)-one. **(Compound 33)**

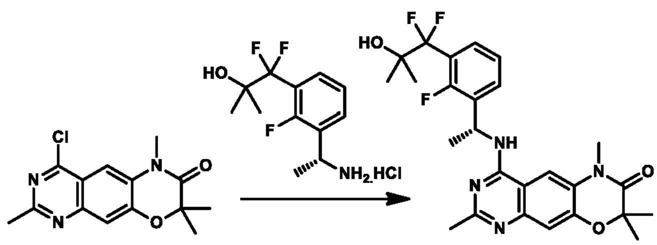

The titled compound was prepared by reaction of **Example 31 Step 5** intermediate and (R)-1-(3-(1-Aminoethyl)-2-fluorophenyl)-1,1-difluoro-2-methylpropan-2-ol hydrochloride (Example 4-Step 6a) by using similar protocol

mentioned in **Example 28 Step 2** (0.070 g, 27.1% yield). **(Compound 33).**

MS (ES+) m/z = 503.4 (M+1).

$^1$H NMR (400 MHz, DMSO-$d_6$) δ 8.38 (s, 1H), 8.01 (s, 1H), 7.64 - 7.59 (m, 1H), 7.39 - 7.27 (m, 1H), 7.23 (t, *J* = 7.7 Hz, 1H), 7.07 (s, 1H), 5.92 - 5.75 (m, 1H), 5.34 (s, 1H), 3.40 (s, 3H), 2.31 (s, 3H), 1.61 (d, *J* = 7.1 Hz, 3H), 1.47 (s, 3H), 1.46 (s, 3H), 1.24 (s, 3H), 1.21 (s, 3H).

**Example 47:** (R)-1,1-Difluoro-1-(2-fluoro-3-(1-((2,6,8,8-tetramethyl-7,8-dihydro-6H-[1,4]oxazino [3,2-g]quinazolin-4-yl) amino)ethyl)phenyl)-2-methylpropan-2-ol. **(Compound 34)**

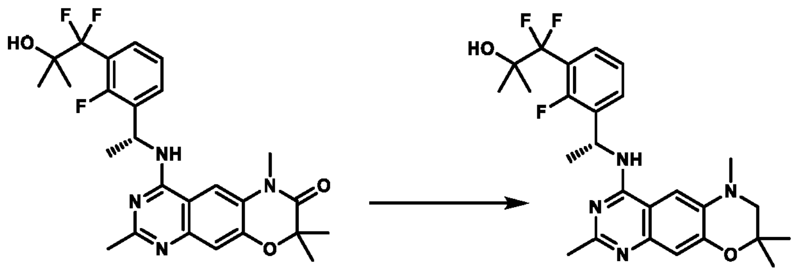

The titled compound was prepared from **Example 46 by** employing similar protocol mentioned in **Example 23** (0.037g, 29.30 % yield).

MS (ES+) m/z = 489.24 (M+1).

$^1$H NMR (400 MHz, DMSO-$d_6$) δ 7.96 (d, *J* = 7.6 Hz, 1H), 7.61 - 7.57 (m, 1H), 7.40 (s, 1H), 7.32 - 7.28 (m, 1H), 7.23 - 7.17 (m, 1H), 6.76 (s, 1H), 5.89 - 5.73 (m, 1H), 5.34 (s, 1H), 3.12 (s, 2H), 3.07 (s, 3H), 2.24 (s, 3H), 1.58 (d, *J* = 7.0 Hz, 3H), 1.34 (s, 3H), 1.33 (s, 3H), 1.24 (s, 3H), 1.21 (s, 3H).

**Example 48:** (R)-2,2-Difluoro-2-(2-fluoro-3-(1-((2,6,8,8-tetramethyl-7,8-dihydro-6H-[1,4]oxazino[3,2-g]quinazolin-4-yl) amino)ethyl)phenyl)ethan-1-ol. **(Compound 35)**

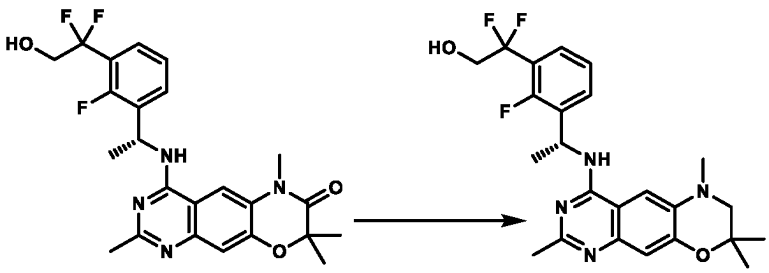

The titled compound was prepared from **Example 31** by employing similar protocol mentioned in **Example 23.** (0.015g, 17.17% yield).

MS (ES+) m/z = 461.23 (M+1).

$^1$H NMR (400 MHz, DMSO-$d_6$) δ 8.09 (s, 1H), 7.67 - 7.63 (m, 1H), 7.45 - 7.36 (m, 2H), 7.27 - 7.23 (m, 1H), 6.77 (s, 1H), 5.88 - 5.78 (m, 1H), 5.72 (t, *J* = 6.4 Hz, 1H), 3.99 - 3.88 (m, 2H), 3.13 (s, 2H), 3.07 (s, 3H), 2.28 (s, 3H), 1.60 (d, *J* = 7.1 Hz, 3H), 1.34 (s, 3H), 1.33 (s, 3H).

**Example 49:** (R&S)-2,2-Difluoro-2-(2-fluoro-3-((R)-1-((-2,6,8-trimethyl-7,8-dihydro-6H-[1,4]oxazino [3,2-g]quinazolin-4-yl)amino)ethyl)phenyl)ethan-1-ol. **(Compound 36)**

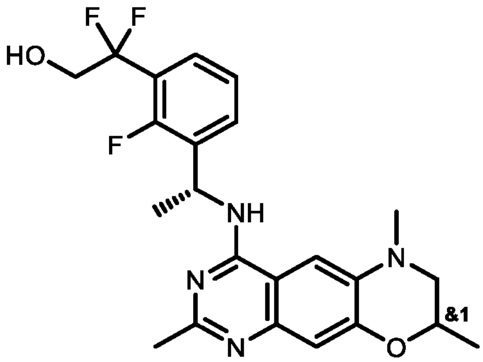

Two stereoisomers synthesized separately as mentioned below.

**Peak 1:** 2,2-difluoro-2-(2-fluoro-3-((R)-1-(((S/R)-2,6,8-trimethyl-7,8-dihydro-6H-[1,4]oxazino[3,2-g]quinazolin-4-yl)amino)ethyl)phenyl)ethan-1-ol **(Compound 36a)**

The titled compound was prepared from **Compound 30a** by employing similar protocol mentioned in **Example 23 Step 1.**

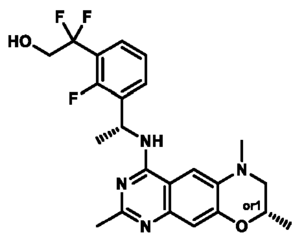

MS (ES+) m/z = 447.17 (M+1).
$^1$H NMR (400 MHz, DMSO-$d_6$) δ 7.93 (d, *J* = 7.5 Hz, 1H), 7.64 - 7.60 (m, 1H), 7.49 - 7.32 (m, 2H), 7.26 - 7.22 (m, 1H), 6.78 (s, 1H), 5.85 - 5.78 (m, 1H), 5.78 - 5.71 (m, 1H), 4.44 - 4.38 (m, 1H), 3.99 - 3.90 (m, 2H), 3.45 - 3.32 (m, 2H), 3.02 (s, 3H), 2.26 (s, 3H), 1.59 (d, *J = 7.1* Hz, 3H), 1.34 (d, *J* = 6.2 Hz, 3H).

**Peak 2:** 2,2-difluoro-2-(2-fluoro-3-((R)-1-(((R/S)-2,6,8-trimethyl-7,8-dihydro-6H-[1,4]oxazino[3,2-g]quinazolin-4-yl)amino)ethyl)phenyl)ethan-1-ol

**(Compound 36b).**

The titled compound was prepared from **Compound 30b** by employing similar protocol mentioned in **Example 23 Step 1.**

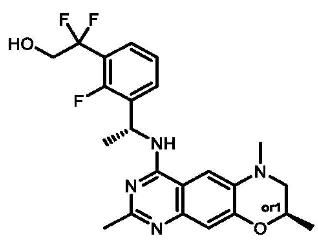

MS (ES+) m/z = 447.17 (M+1).
$^1$H NMR (400 MHz, DMSO-$d_6$) δ 8.16 (s, 1H), 8.03 (d, *J* = 7.4 Hz, 1H), 7.68 - 7.60 (m, 1H), 7.43 - 7.38 (m, 1H), 7.37 (s, 1H), 7.28 - 7.21 (m, 1H), 6.79 (s, 1H), 5.87 - 5.81 (m, 1H), 4.46 - 4.39 (m, 1H), 3.98 - 3.87 (m, 2H), 3.45 - 3.32 (m, 2H), 3.03 (s, 3H), 2.27 (s, 3H), 1.59 (d, *J = 7.1* Hz, 3H), 1.34 (d, *J* = 6.3 Hz, 3H).

**Example 50:** (R)-1,1-Difluoro-1-(2-fluoro-3-(1-((2,6,7,7-tetramethyl-7,8-dihydro-6H-[1,4]oxazino[3,2-g]quinazolin-4-yl) amino)ethyl)phenyl)-2-methylpropan-2-ol **(Compound 37)**

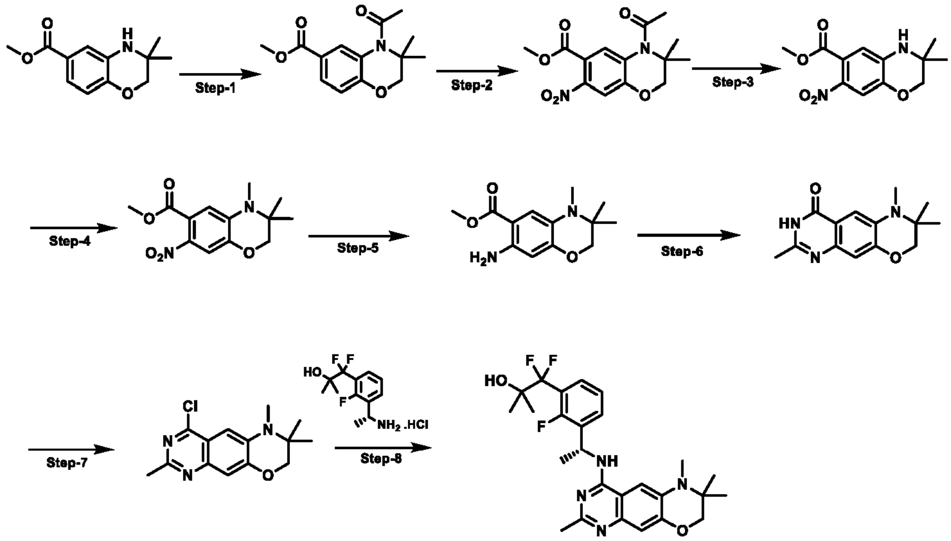


**Step 1:** Methyl 4-acetyl-3,3-dimethyl-3,4-dihydro-2H-benzo[b][1,4]oxazine-6-carboxylate.

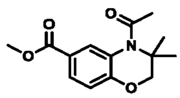

To a solution of methyl 3,3-dimethyl-3,4-dihydro-2H-benzo[b][1,4]oxazine-6-carboxylate (8.1 g, 36.6 mmol) (Reference: WO40963) and pyridine (29.6 mL, 366 mmol) in DCM (80.0 mL) was added acetyl chloride (3.90 mL, 54.9 mmol) at 0°C and reaction mixture was stirred at room temperature under nitrogen atmosphere for 16 h. After completion of the reaction, poured the reaction mixture into water and extracted with DCM (50.0 mL x 3), organic layer was washed with 30.0 mL water, 20.0 mL brine and dried over anhydrous $Na_2SO_4$ and concentrated under reduced pressure and crude product was purified by flash chromatography ethyl acetate- n-hexane gradient to afford the titled compound (8.9 g, 92.0 %) as a colorless semisolid.

$^1$H NMR (400 MHz, DMSO-$d_6$) δ 7.70 - 7.61 (m, 2H), 7.05 (d, *J* = 8.4 Hz, 1H), 4.00 (s, 2H), 3.33 (s, 3H), 2.19 (s, 3H), 1.44 (s, 6H).

**Step 2:** Methyl 4-acetyl-3,3-dimethyl-7-nitro-3,4-dihydro-2H-benzo[b][1,4]oxazine-6-carboxylate.

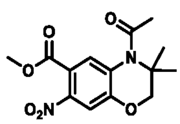

The titled compound was prepared from **Step 1** intermediate by employing similar protocol mentioned in **Example 14 Step 1** (5.1 g, 87.0% yield).

GCMS m/z 308.18 (M+).

$^1$H NMR (400 MHz, DMSO-$d_6$) δ 7.67 (s, 1H), 7.60 (s, 1H), 4.08 (s, 2H), 3.81 (s, 3H), 2.28 (s, 3H), 1.43 (s, 6H).

**Step 3:** Methyl 3,3-dimethyl-7-nitro-3,4-dihydro-2H-benzo[b][1,4]oxazine-6-carboxylate.

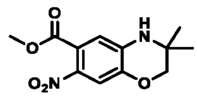

To a solution of methyl 4-acetyl-3,3-dimethyl-7-nitro-3,4-dihydro-2H-benzo[b][1,4]oxazine-6-carboxylate (5.1 g, 16.54 mmol) in MeOH (50.0 ml) was added $K_2CO_3$ (9.15 g, 66.2 mmol) at room temperature & reaction was stirred at room temperature for 1h. Solvent was evaporated & residue was diluted with water (200.0 mL) and extracted with DCM (100.0 mL x 3), organic layer was washed with brine (50.0 mL) dried over anhydrous $Na_2SO_4$, concentrated under reduced pressure and crude product was purified by flash chromatography in ethyl acetate : n-hexane gradient to afford titled compound (4.1 g, 93.0 %) as a yellow solid.

MS (ES+) m/z = 266.18 (M+).

**Step 4:** Methyl 3,3,4-trimethyl-7-nitro-3,4-dihydro-2H-benzo[b][1,4]oxazine-6-carboxylate.

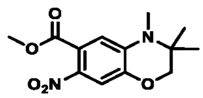

The titled compound was prepared from **Step 3** intermediate by employing similar protocol mentioned in **Example 14 Step 2** (3.5 g, 83.0 % yield).

MS (ES+) m/z = 280.09 (M+).

**Step 5:** Methyl7-amino-3,3,4-trimethyl-3,4-dihydro-2H-benzo[b][1,4]oxazine-6-carboxylate.

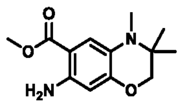

The titled compound was prepared from **Step 4** intermediate by employing similar protocol mentioned in **Example 14 Step 3** (2.95 g, 97.0 % yield).

MS (ES+) m/z = 250.12 (M+).

**Step 6:** 2,6,7,7-Tetramethyl-3,6,7,8-tetrahydro-4H-[1,4]oxazino[3,2-g]quinazolin-4-one.

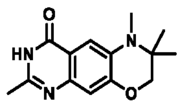

The titled compound was prepared from **Step 5** intermediate by employing similar protocol mentioned in **Example 14 Step 4** (2.7 g, 90.0 % yield).

MS (ES+) m/z = 260.0(M+1).

**Step 7:** 4-Chloro-2,6,7,7-tetramethyl-7,8-dihydro-6H-[1,4]oxazino[3,2-g]quinazoline.

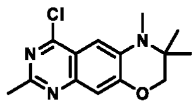

The titled compound was prepared from **Step 6** intermediate by employing similar protocol mentioned in **Example 28 Step 1** (0.35 g, 40.8 % yield).

MS (ES+) m/z = 278.27(M+1).

**Step 8:** (R)-1,1-difluoro-1-(2-fluoro-3-(1-((2,6,7,7-tetramethyl-7,8-dihydro-6H-[1,4]oxazino[3,2-g] quinazolin-4-yl)amino)ethyl)phenyl)-2-methylpropan-2-ol. **(Compound 37)**

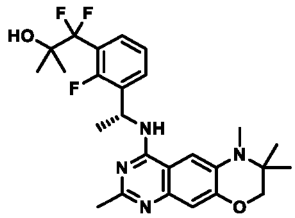

The titled compound was prepared by reaction of **Step 7** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.094 g, 21.38% yield).

MS (ES+) m/z = 489.12(M+1).

$^1$H NMR (400 MHz, DMSO-$d_6$) δ 8.00 (d, *J* = 7.6 Hz, 1H), 7.61 - 7.57 (m, 1H), 7.33 - 7.27 (m, 2H), 7.22 - 7.18 (m, 1H), 6.83 (s, 1H), 5.86 - 5.76 (m, 1H), 5.33 (s, 1H), 4.01 (s, 2H), 2.92 (s, 3H), 2.25 (s, 3H), 1.58 (d, *J* = 7.1 Hz, 3H), 1.24 (s, 3H), 1.21 (s, 3H), 1.20 (s, 6H).

**Example 51:** (R)-4-((1-(3-(1,1-Difluoro-2-hydroxy-2-methylpropyl)-2-fluorophenyl)ethyl)amino)-2,6,8-trimethylpyrido [2,3-g]quinazolin-7(6H)-one **(Compound 38)**

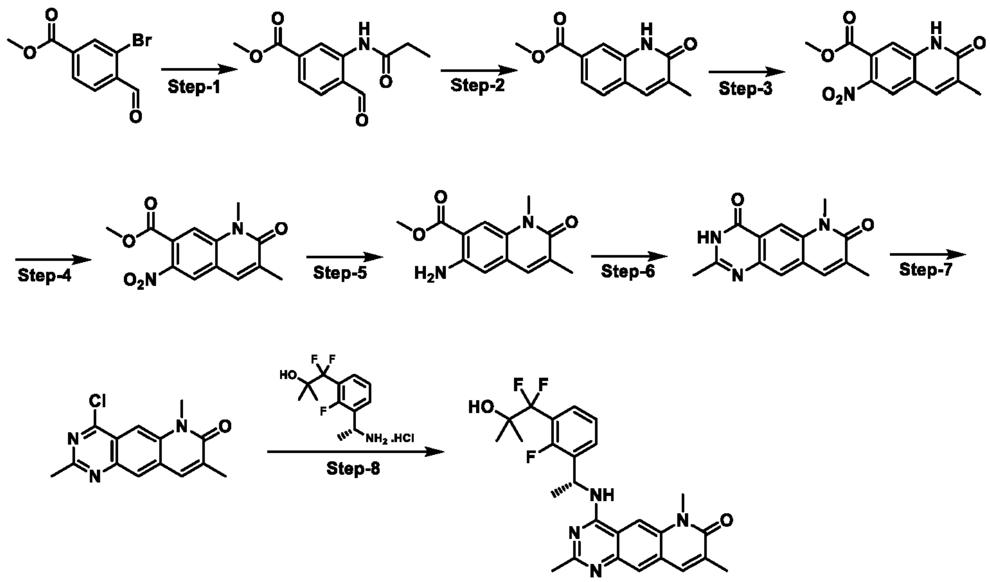


**Step 1:** Methyl 4-formyl-3-propionamidobenzoate.

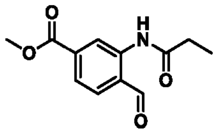

To a stirred solution of methyl 3-bromo-4-formylbenzoate (3.0 g, 12.34 mmol) (Tetrahedron, 2008, vol. 64, # 4, p. 688 - 695, European Journal of Medicinal Chemistry, 2015, vol. 92, p. 818 - 838), propionamide (1.35 g, 18.51 mmol), $Pd_2(dba)_3$ (1.13 g, 1.234 mmol), xantphos (1.43 g, 2.47 mmol) and $Cs_2CO_3$ (10.05 g, 30.9 mmol) under inert atmosphere. The reaction was stirred at 75°C for 12 h. The reaction was cooled to room temperature. The reaction mixture was diluted with water (50.0 mL) and extracted with ethyl acetate (100.0 mL). Organic layers was washed with brine (30.0 mL) and concentrated. the residue obtained was purified by flash chromatography using eluent 0-50% ethyl acetate in n- hexane to afford the titled compound as a white solid (1.8 g, 62.0 % yield).
MS (ES+) m/z = 235.14 (M+).

**Step 2:** Methyl 3-methyl-2-oxo-1,2-dihydroquinoline-7-carboxylate.

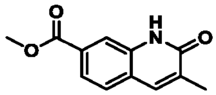

To a solution of methyl 4-formyl-3-propionamidobenzoate (2.9 g, 12.33 mmol) and $K_2CO_3$ (2.56 g, 18.49 mmol) in DMF (10.0 mL) was stirred at 100 °C for 2 h under inert atmosphere. The reaction mixture was diluted with water (30.0 mL) and extracted with ethyl acetate (50.0 mL x 2). The combined organic layer was separated, dried over anhydrous $Na_2SO_4$, concentrated under reduced pressure and crude product was purified by flash chromatography using eluent ethyl acetate : n-hexane gradient to afford the titled compound 1.5 g, 56.0 % yield as a yellow solid.
$^1H$ NMR (400 MHz, DMSO-$d_6$) δ 11.96 (s, 1H), 7.92 (s, 1H), 7.86 (s, 1H), 7.69 (s, 2H), 3.88 (s, 3H), 2.12 (s, 3H).

**Step 3:** Methyl 3-methyl-6-nitro-2-oxo-1,2-dihydroquinoline-7-carboxylate.

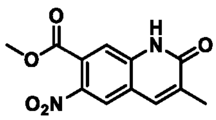

The titled compound was prepared from **Step 2** intermediate by employing similar protocol mentioned in **Example 14 Step 1** (1.0 g, 92 % yield).
MS (ES+) m/z = 260.96 (M-1)

Step 4: Methyl 1,3-dimethyl-6-nitro-2-oxo-1,2-dihydroquinoline-7-carboxylate.

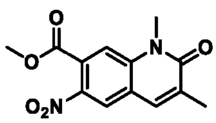

The titled compound was prepared from **Step 3** intermediate by employing similar protocol mentioned in **Example 14 Step 2** (0.90 g, 85% yield).
MS (ES+) m/z = 277.08 (M+1).

**Step 5:** Methyl 6-amino-1,3-dimethyl-2-oxo-1,2-dihydroquinoline-7-carboxylate.

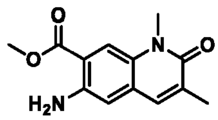

The titled compound was prepared from **Step 4** intermediate by employing similar protocol mentioned in **Example 14 Step 3** (0.65 g, 81.0% yield).

MS (ES+) m/z = 247.21 (M+1).

**Step 6:** 2,6,8-Trimethyl-3,6-dihydropyrido[2,3-g]quinazoline-4,7-dione.

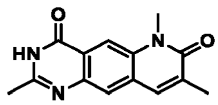

The titled compound was prepared from **Step 5** intermediate by employing similar protocol mentioned in **Example 14 Step 4** (0.20 g, 38.6% yield).

MS (ES+) m/z = 256.02 (M+1).

**Step 7:** 4-Chloro-2,6,8-trimethylpyrido[2,3-g]quinazolin-7(6H)-one.

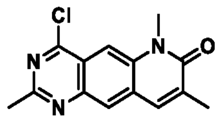

The titled compound was prepared from **Step 6** intermediate by employing similar protocol mentioned in **Example 28 Step 1** (0.19 g, 89.0 % yield).

MS (ES+) m/z = 274.08 (M+1).

**Step 8:** (R)-4-((1-(3-(1,1-Difluoro-2-hydroxy-2-methylpropyl)-2-fluorophenyl)ethyl)amino)-2,6,8-trimethylpyrido[2,3-g] quinazolin-7(6H)-one **(Compound 38)**

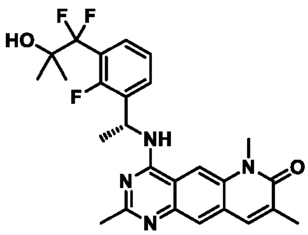

The titled compound was prepared by reaction of **Step 7** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.03 g, 8.92 % yield).

MS (ES+) m/z = 485.24 (M+1).

$^1$H NMR (400 MHz, DMSO-$d_6$) δ 8.64 (d, *J* = 7.3 Hz, 1H), 8.28 (s, 1H), 7.94 - 7.88 (m, 1H), 7.87 (s, 1H), 7.71 - 7.45 (m, 1H), 7.41 - 7.29 (m, 1H), 7.27 - 7.18 (m, 1H), 5.92 - 5.83 (m, 1H), 5.34 (s, 1H), 3.79 (s, 3H), 2.35 (s, 3H), 2.17 (s, 3H), 1.65 (d, *J* = 7.0 Hz, 3H), 1.25 (s, 3H), 1.22 (s, 3H).

**Example 52:** (R)-4-((1-(3-(1,1-Difluoro-2-hydroxy-2-methylpropyl)-2-fluorophenyl)ethyl)amino)-2,6,8-trimethylpyrazino [2,3-g]quinazolin-7(6H)-one. **(Compound 39)**

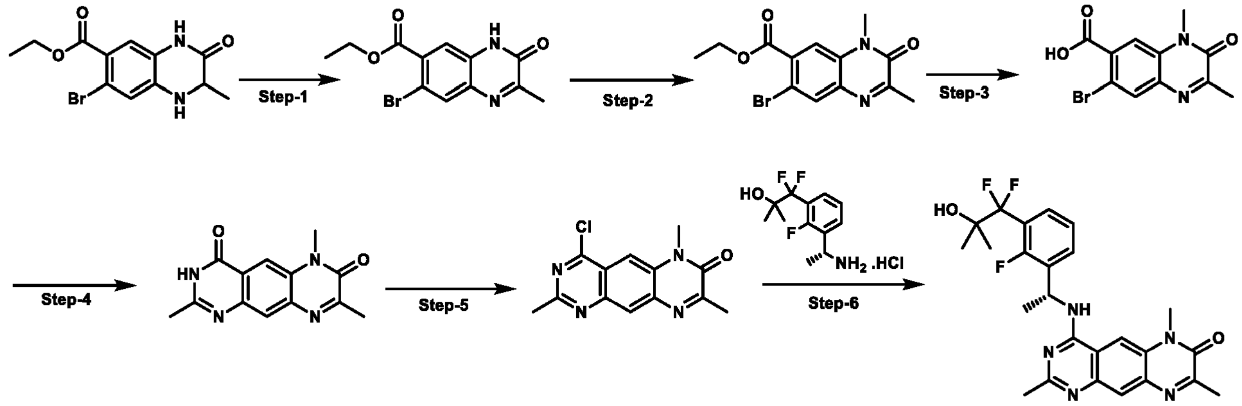


**Step 1:** Ethyl 7-bromo-2-methyl-3-oxo-3,4-dihydroquinoxaline-6-carboxylate.

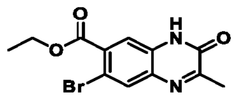

To a stirred solution of ethyl 7-bromo-2-methyl-3-oxo-1,2,3,4-tetrahydroquinoxaline-6-carboxylate (4.6 g, 14.69 mmol) (WO2019/243823) in THF (50.0 mL) was added manganese dioxide (12.77 g, 147.0 mmol) and the reaction mixture was stirred at 25°C for 16 h. After completion of reaction, reaction mixture was filtered through celite bed, washed with ethyl acetate (100.0 mL) and filtrate was evaporated under vacuo to afford the titled compound as a off white solid (3.5 g, 77.0 %).
MS (ES+) m/z =311.21 (M+1).

**Step 2:** Ethyl 7-bromo-2,4-dimethyl-3-oxo-3,4-dihydroquinoxaline-6-carboxylate.

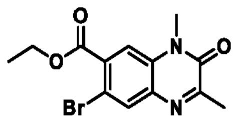

The titled compound was prepared from **Step 1** intermediate by employing similar protocol mentioned in **Example 40 Step 3** (2.8 g, 77.0% yield).
$^1$H NMR (400 MHz, DMSO-$d_6$) δ 8.04 (s, 1H), 7.81 (s, 1H), 4.39 (q, $J$ = 7.1 Hz, 2H), 3.61 (s, 3H), 2.47 (s, 3H), 1.36 (t, $J$ = 7.1 Hz, 3H).

**Step 3:** 7-Bromo-2,4-dimethyl-3-oxo-3,4-dihydroquinoxaline-6-carboxylic acid.

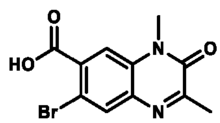

The titled compound was prepared from **Step 2** intermediate by employing similar protocol mentioned in **Example 40 Step 4** (1.8 g, 70.4 % yield).

$^1$H NMR (400 MHz, DMSO-$d_6$) δ 13.78 (s, 1H), 8.02 (s, 1H), 7.81 (s, 1H), 3.62 (s, 3H), 2.47 (s, 3H).

**Step 4:** 2,6,8-Trimethyl-3,6-dihydropyrazino[2,3-g]quinazoline-4,7-dione.

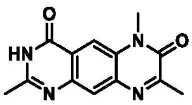

The titled compound was prepared from **Step 3** intermediate by employing similar protocol mentioned in **Example 30 Step 8** (1.49 g, 90.0 % yield).
MS (ES+) m/z = 257.14(M+1).

**Step 5:** 4-Chloro-2,6,8-trimethylpyrazino[2,3-g]quinazolin-7(6H)-one.

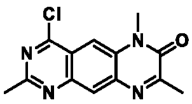

The titled compound was prepared from **Step 4** intermediate by employing similar protocol mentioned in **Example 28 Step 1** (0.05 g, 11.66% yield).
$^1$H NMR (400 MHz, $CDCl_3$) δ 8.43 (s, 1H), 7.96 (s, 1H), 3.84 (s, 3H), 2.91 (s, 3H), 2.71 (s, 3H).

**Step 6:** (R)-4-((1-(3-(1,1-difluoro-2-hydroxy-2-methylpropyl)-2fluorophenyl)ethyl)amino)-2,6,8-trimethylpyrazino[2,3-g] quinazolin-7(6H)-one **(Compound 39)**

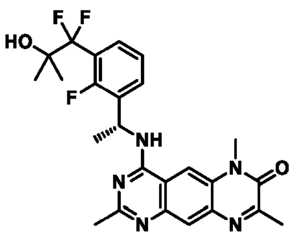

The titled compound was prepared by reaction of **Step 5** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.023 g, 15.31 % yield).
MS (ES+) m/z = 486.36 (M+1).
$^1$H NMR (400 MHz, DMSO-$d_6$) δ 8.67 (d, $J$ = 7.2 Hz, 1H), 8.33 (s, 1H), 7.90 (s, 1H), 7.65 - 7.60 (m, 1H), 7.35 - 7.31 (m, 1H), 7.25 - 7.20 (m, 1H), 5.90 - 5.82 (m, 1H), 5.34 (s, 1H), 3.75 (s, 3H), 2.46 (s, 3H), 2.37 (s, 3H), 1.65 (d, $J$ = 7.0 Hz, 3H), 1.25 (s, 3H), 1.22 (s, 3H).

**Example 53:** (R)-4-((1-(3-(1,1-Difluoro-2-hydroxy-2-methylpropyl)-2-fluorophenyl)ethyl) amino)-2,6,9-trimethyl-6,9-dihydropyrazino[2,3-g]quinazoline-7,8-dione.**(Compound 40)**

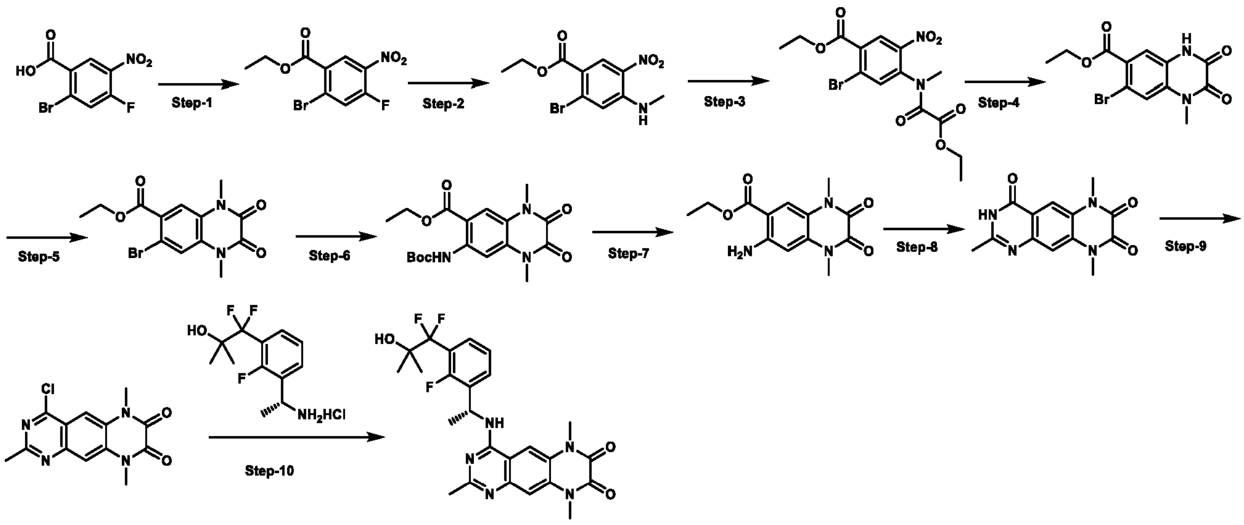


**Step 1:** Ethyl 2-bromo-4-fluoro-5-nitrobenzoate

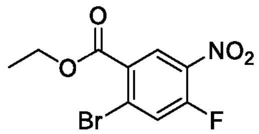

To a stirred solution of 2-bromo-4-fluoro-5-nitrobenzoic acid (40.5 g, 153.0 mmol) (US2018/291002, 2018, A1) in ethanol (300.0 mL) at 0°C, $H_2SO_4$ (16.35 mL, 307.0 mmol) was added dropwise for 30 min. The reaction mixture was heated at 100 °C for 16 h. The progress of the reaction was monitored by TLC. The reaction mixture was concentrated and was quenched with a sat. solution of $NaHCO_3$ (100.0 mL) and extracted with Ethyl acetate (2 x 200.0 mL).The combined organic layer was washed with brine (100.0 mL) and dried over anhydrous sodium sulphate and solvent was evaporated under reduced pressure to afford the titled compound ethyl 2-bromo-4-fluoro-5-nitrobenzoate (36.5 g, 81.0 % yield) as off white solid.

$^1H$ NMR (400 MHz, DMSO-$d_6$) δ 8.53 (d, *J* = 7.9 Hz, 1H), 8.22 (d, *J* = 10.8 Hz, 1H), 4.38 (q, *J* = 7.1 Hz, 2H), 1.35 (t, *J* = 7.1 Hz, 3H).

**Step 2:** Ethyl 2-bromo-4-(methylamino)-5-nitrobenzoate.

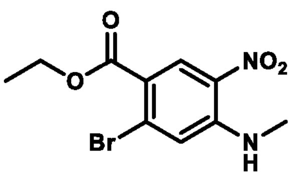

To a stirred solution of ethyl 2-bromo-4-fluoro-5-nitrobenzoate (6.0 g, 20.54 mmol) in ethanol (60.0 mL) and 40% methenamine (7.97 mL, 41.1 mmol) in ethanol was added at 0°C. After addition, the resulting mixture was stirred for 1h at 0°C. The progress of the reaction was monitored by TLC. To the reaction mixture, diethyl ether (100.0 mL) was added and the precipitate was separated by filtration. The product was dried under reduced pressure to afford the titled compound ethyl 2-bromo-4-(methylamino)-5-nitrobenzoate (5.4 g, 87.0 % yield) as a yellow powder.

$^1H$ NMR (400 MHz, DMSO-$d_6$) δ 8.70 - 8.46 (m, 2H), 7.27 (s, 1H), 4.28 (q, *J* = 7.1 Hz, 2H), 3.00 (d, *J* = 4.5 Hz, 3H), 1.32 (t, *J* = 7.1 Hz, 3H).

**Step 3:** Ethyl 2-bromo-4-(2-ethoxy-N-methyl-2-oxoacetamido)-5-nitrobenzoate.

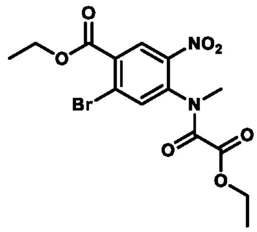

To a stirred solution of ethyl 2-bromo-4-(methylamino)-5-nitrobenzoate (5.3 g, 17.49 mmol) in THF (50.0 mL) was added TEA (24.37 mL, 175 mmol) and ethyl oxalyl chloride (19.57 mL, 175 mmol) slowly at 0° C. After the addition was completed, the reaction mixture was stirred at 75 °C for 30 min. The progress of the reaction was monitored by TLC. The reaction mixture was brought to room temp, quenched with a sat. solution of $NaHCO_3$ (100.0 mL) and extracted with ethyl acetate (2 x 200.0 mL). The combined organic layer was washed with brine (100.0 mL) and dried over anhydrous sodium sulphate and solvent was evaporated under reduced pressure to afford the crude product (6.0 g) as sticky solid, which was used as such for the next reaction.

MS (ES+) m/z = 403.1 (M+1).

**Step 4:** Ethyl 7-bromo-1-methyl-2,3-dioxo-1,2,3,4-tetrahydroquinoxaline-6-carboxylate.

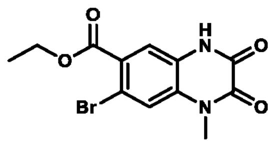

To a stirred solution of ethyl 2-bromo-4-(2-ethoxy-N-methyl-2-oxoacetamido)-5-nitrobenzoate (6.0 g, 14.88 mmol) in THF (30.0 mL), ethanol (30.0 mL) and water (30.0 mL) was added sodium dithionate (9.20 g, 44.6 mmol) at room temperature and heated reaction mass at 100 °C for 3 h. The progress of reaction was monitored by TLC. After completion of reaction, cooled to room temp and quenched with water. The reaction mass was extracted with ethyl acetate (2 x 100.0 mL). The organic layer was washed with water (100.0 mL), dried over anhydrous sodium sulphate and evaporated under reduced pressure to get crude residue which was purified by flash chromatography using eluent (1-5%) of methanol in DCM to afford the titled compound ethyl 7-bromo-1-methyl-2,3-dioxo-1,2,3,4-tetrahydroquinoxaline-6-carboxylate (1.9 g, 39.0 % yield) as white solid.

MS (ES+) m/z = 327.22 (M+1).

$^1H$ NMR (400 MHz, DMSO-$d_6$) δ 12.15 (s, 1H), 7.65 (s, 1H), 7.63 (s, 1H), 4.33 (q, $J$ = 7.1 Hz, 2H), 3.50 (s, 3H), 1.33 (t, $J$ = 7.1 Hz, 3H).

**Step 5:** Ethyl 7-bromo-1,4-dimethyl-2,3-dioxo-1,2,3,4-tetrahydroquinoxaline-6-carboxylate.

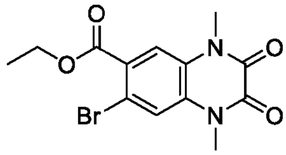

To a stirred solution of ethyl 7-bromo-1-methyl-2,3-dioxo-1,2,3,4-tetrahydroquinoxaline-6-carboxylate (1.9 g, 5.81 mmol) in DMF (50.0 mL) was added $K_2CO_3$ (2.41 g, 17.42 mmol) followed by dropwise addition of methyl iodide (1.09 mL, 17.42 mmol) and stirred reaction mass at 25 °C for 3 h. The progress of the reaction was monitored by TLC. After completion of reaction, quenched with water, solid precipitated out was filtered. The solid obtained was dried under reduced pressure to afford the titled compound ethyl 7-bromo-1,4-dimethyl-2,3-dioxo-1,2,3,4-tetrahydroquinoxaline-6-carboxylate (1.7 g, 86.0 % yield) as off white solid.

MS (ES+) m/z = 341.22 (M+1).

1\H NMR (400 MHz, DMSO-$d_6$) δ 7.71 (s, 1H), 7.70 (s, 1H), 4.36 (q, *J* = 7.1 Hz, 2H), 3.55 (s, 3H), 3.54 (s, 3H), 1.36 (t, *J* = 7.1 Hz, 3H).

**Step 6:** Ethyl 7-((tert-butoxycarbonyl)amino)-1,4-dimethyl-2,3-dioxo-1,2,3,4-tetrahydroquinoxaline-6-carboxylate.

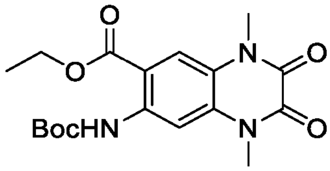

To a stirred solution of ethyl 7-bromo-1,4-dimethyl-2,3-dioxo-1,2,3,4-tetrahydroquinoxaline-6-carboxylate (0.6 g, 1.76 mmol) in 1,4-dioxane (15.0 mL) was added tert-butyl carbamate (0.31 g, 2.64 mmol), $Cs_2CO_3$ (0.688 g, 2.110 mmol) and the suspension was degassed with nitrogen for 10 min. To this were added xantphos (0.122 g, 0.211 mmol) and tris(dibenzylideneacetone) dipalladium (0) (0.081 g, 0.088 mmol). The reaction mixture was heated at 110 °C for 3 h. The progress of reaction was monitored by TLC. The reaction was cooled to RT and solvent was removed under reduced pressure to get crude compound. The crude compound was purified by flash chromatography using eluent (0 - 10%) of methanol in DCM to afford the titled compound ethyl 7-((tert-butoxycarbonyl)amino)-1,4-dimethyl-2,3-dioxo-1,2,3,4-tetrahydroquinoxaline-6-carboxylate (0.6 g, 90.0 % yield) as a brown solid.

MS (ES+) m/z = 378.35 (M+1).

$^1$H NMR (400 MHz, DMSO-$d_6$) δ 10.22 (s, 1H), 8.23 (s, 1H), 7.78 (s, 1H), 4.37 (q, *J* = 7.1 Hz, 2H), 3.54 (s, 3H), 3.52 (s, 3H), 1.51 (s, 9H), 1.37 (s, 3H).

**Step 7:** Ethyl 7-amino-1,4-dimethyl-2,3-dioxo-1,2,3,4-tetrahydroquinoxaline-6-carboxylate hydrochloride

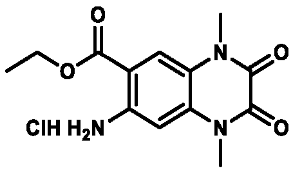

To a stirred solution of ethyl 7-((tert-butoxycarbonyl)amino)-1,4-dimethyl-2,3-dioxo-1,2,3,4-tetrahydroquinoxaline-6-carboxylate (0.6 g, 1.59 mmol) in 1,4-dioxane (5.0 mL) was added 4M HCl in dioxane (9.94 mL, 39.7 mmol) and heated the reaction mass at 60 °C for 1.5 h. The progress of the reaction was monitored by TLC, after completion of reaction, evaporated the reaction mass under reduced pressure to afford the crude ethyl 7-amino-1,4-dimethyl-2,3-dioxo-1,2,3,4-tetrahydroquinoxaline-6-carboxylate hydrochloride (0.48 g, 96.0 % yield) as off white solid.

MS (ES+) m/z = 278.21 (M+1)

**Step 8:** 2,6,9-Trimethyl-6,9-dihydropyrazino[2,3-g]quinazoline-4,7,8(3H)-trione.

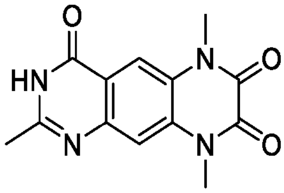

The titled compound was prepared from **Step 7** intermediate by employing similar protocol mentioned in **Example 14 Step 4** (0.31 g, 74.4 % yield).

MS (ES+) m/z = 273.15 (M+1).

$^1H$ NMR (400 MHz, DMSO-$d_6$) δ 12.26 (s, 1H), 7.84 (s, 1H), 7.51 (s, 1H), 3.61 (s, 3H), 3.59 (s, 3H), 2.37 (s, 3H).

**Step 9:** 4-Chloro-2,6,9-trimethyl-6,9-dihydropyrazino[2,3-g]quinazoline-7,8-dione.

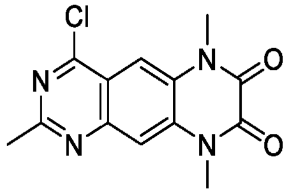

The titled compound was prepared from **Step 8** intermediate by employing similar protocol mentioned in **Example 28 Step1** (0.22 g, 74.10 % yield).

MS (ES+) m/z = 291.21 (M+1)

$^1H$ NMR (400 MHz, DMSO-$d_6$) δ 7.87 (s, 1H), 7.82 (s, 1H), 3.68 (s, 3H), 3.65 (s, 3H), 2.75 (s, 3H).

**Step 10:** (R)-4-((1-(3-(1,1-Difluoro-2-hydroxy-2-methylpropyl)-2-fluorophenyl)ethyl)amino)-2,6,9-trimethyl-6,9-dihydropyrazino[2,3-g]quinazoline-7,8-dione. **(Compound-41)**

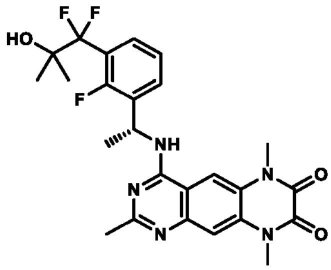

The titled compound was prepared by reaction of **Step 9** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.2 g, 55.2 % yield).

MS (ES+) m/z = 502.31 (M+1).

$^1H$ NMR (400 MHz, DMSO-$d_6$) δ 8.54 (d, $J$ = 7.3 Hz, 1H), 8.18 (s, 1H), 7.63 - 7.58 (m, 1H), 7.51 (s, 1H), 7.37 - 7.28 (m, 1H), 7.27 - 7.19 (m, 1H), 5.92 - 5.78 (m, 1H), 5.35 (s, 1H), 3.69 (s, 3H), 3.59 (s, 3H), 2.35 (s, 3H), 1.63 (d, $J$ = 7.1 Hz, 3H), 1.24 (s, 3H), 1.22 (s, 3H).

**Example 54:** Preparation of (R)-4-((1-(3-(1,1-difluoro-2-hydroxy-2-methylpropyl)-2-fluorophenyl)ethyl)amino)-6-ethyl-2,8,8-trimethyl-6H-[1,4]oxazino[3,2-g]quinazolin-7(8H)-one

**(Compound 41)**

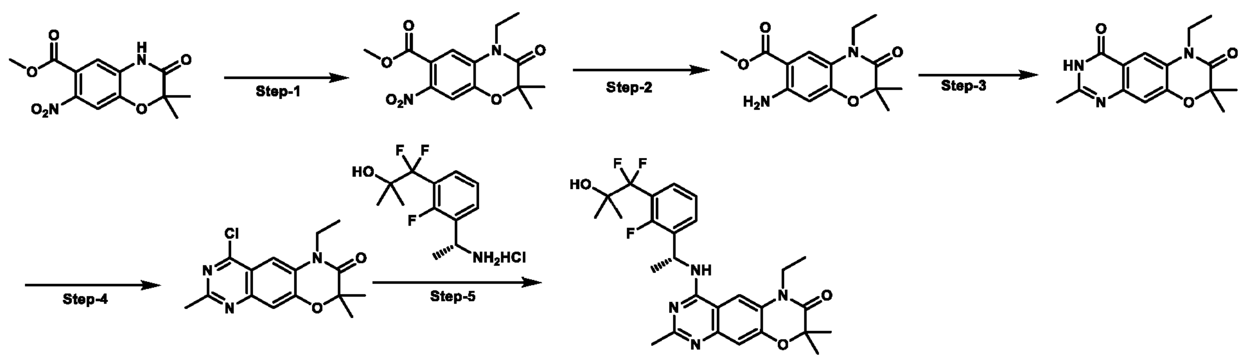


**Step 1:** Methyl 4-ethyl-2,2-dimethyl-7-nitro-3-oxo-3,4-dihydro-2H-benzo[b][1,4]oxazine-6-carboxylate

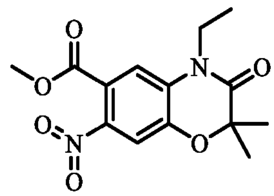

The titled compound was prepared from **Example 18 Step 1** intermediate by employing similar protocol mentioned in **Example 14 Step 2** (5.7 g, 74.0 % yield).

MS (ES+) m/z = 309.34 (M+1)

$^1$H NMR (400 MHz, DMSO-$d_6$) δ 7.72 (s, 1H), 7.57 (s, 1H), 4.09 - 3.94 (m, 2H), 3.85 (s, 3H), 1.47 (s, 6H), 1.20- 1.05 (m, 3H).

**Step 2:** Methyl 7-amino-4-ethyl-2,2-dimethyl-3-oxo-3,4-dihydro-2H-benzo[b][1,4]oxazine-6-carboxylate

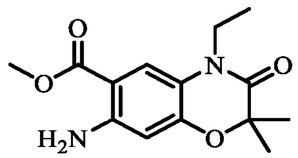

The titled compound was prepared from **Step 1** intermediate by employing similar protocol mentioned in **Example 14 Step 3** (3.48 g, 70.0 % yield).

$^1$H NMR (400 MHz, DMSO-$d_6$) δ 7.35 (s, 1H), 6.61 (s, 2H), 6.39 (s, 1H), 3.84 (q, $J$ = 7.1 Hz, 2H), 3.79 (s, 3H), 1.39 (s, 6H), 1.12 (t, $J$ = 7.1 Hz, 3H).

**Step 3:** 6-Ethyl-2,8,8-trimethyl-3,6-dihydro-4H-[1,4]oxazino[3,2-g]quinazoline-4,7(8H)-dione

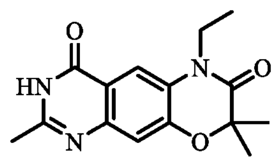

The titled compound was prepared from **Step 2** intermediate by employing similar protocol mentioned in **Example 14 Step 4** (3.2 g, 89.0 % yield)

$^1$H NMR (400 MHz, DMSO-$d_6$) δ 12.20 (s, 1H), 7.66 (s, 1H), 7.10 (s, 1H), 4.01 (q, $J$ = 7.0 Hz, 2H), 2.33 (s, 3H), 1.46

(s, 6H), 1.19 (t, $J$ =7.1 Hz, 3H).

**Step 4:** 4-Chloro-6-ethyl-2,8,8-trimethyl-6H-[1,4]oxazino[3,2-g]quinazolin-7(8H)-one

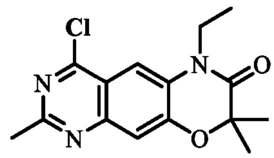

The titled compound was prepared from **Step 3** intermediate by employing similar protocol mentioned in **Example 28 Step 1** (1.8 g, 53.7 % yield).
MS (ES+) m/z = 306.1 (M+1).

**Step 5:** (R)-4-((1-(3-(1,1-difluoro-2-hydroxy-2-methylpropyl)-2-fluorophenyl)ethyl)amino)-6-ethyl-2,8,8-tnmethyl-6H-[1,4]oxazino[3,2-g]quinazolin-7(8H)-one

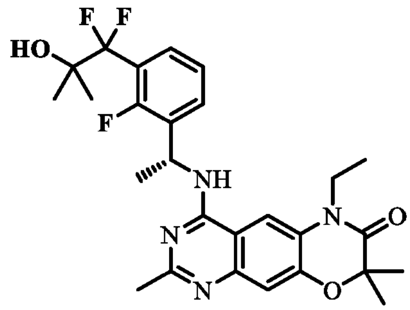

The titled compound was prepared by reaction of **Step 4** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.52 g, 77.0 % yield).
MS (ES+) m/z = 517.3 (M+1).
$^1$H NMR (400 MHz, DMSO-$d$6) δ 8.34 (d, $J$ = 7.3 Hz, 1H), 7.99 (s, 1H), 7.62 - 7.58 (m, 1H), 7.36 - 7.27 (m, 1H), 7.25 - 7.21 (m, 1H), 7.08 (s, 1H), 5.86 - 5.79 (m, 1H), 5.33 (s, 1H), 4.17 - 4.11 (m, 2H), 2.30 (s, 3H), 1.62 (d, $J$ = 7.1 Hz, 3H), 1.46 (s, 3H), 1.45 (s, 3H), 1.32 - 1.19 (m, 9H).

**Example 55:** Preparation of (R)-4-((1-(3-(1,1-difluoro-2-hydroxy-2-methylpropyl)-2-fluorophenyl)ethyl)amino)-2,6,9,9-tetramethyl-8,9-dihydropyrido[2,3-g]quinazolin-7(6H)-one **(Compound-42)**

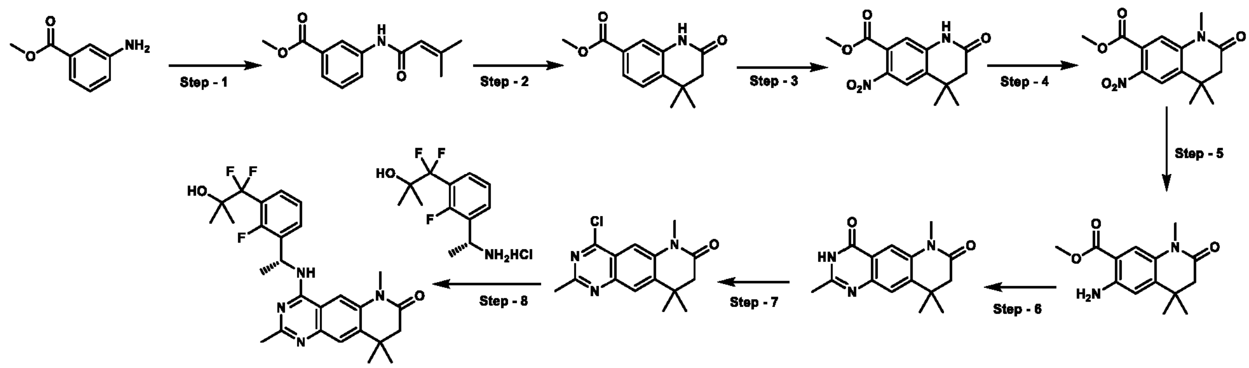

**Step 1:** Methyl 3-(3-methylbut-2-enamido)benzoate.

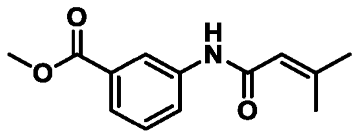

To a stirred solution of methyl 3-aminobenzoate (10.0 g, 66.2 mmol) in DCM (100.0 mL) were added N,N-diisopropylethylamine (57.8 mL, 331.0 mmol), 3-methylbut-2-enoyl chloride (8.63 g, 72.8 mmol) was added dropwise for 10 min and then 4-dimethylaminopyridine (0.81 g, 6.62 mmol) at room temperature. The resulting reaction mixture was stirred for 16 h. The progress of the reaction was monitored by TLC. After completion of reaction, reaction mixture was diluted with DCM (100.0 mL). The organic layer was separated and washed with water (100.0 ml) and brine (100.0 mL). The separated organic layer was dried over anhydrous $Na_2SO_4$, filtered and concentrated. The crude mass was purified by flash column chromatography by using eluent 0 - 70% ethyl acetate in n-hexane to afford the titled compound methyl 3-(3-methylbut-2-enamido)benzoate (14.0 g, 91.0 % yield) as off white solid.
MS (ES+) m/z = 234.17 (M+1).
$^1$H NMR (400 MHz, DMSO-$d_6$) δ 10.06 (s, 1H), 8.45 - 8.26 (m, 1H), 7.99 - 7.73 (m, 1H), 7.71 - 7.53 (m, 1H), 7.47 - 7.29 (m, 1H), 5.97 - 5.66 (m, 1H), 3.86 (s, 3H), 2.17 - 2.10 (m, 3H), 1.91 - 1.85 (m, 3H).

**Step 2:** Methyl 4,4-dimethyl-2-oxo-1,2,3,4-tetrahydroquinoline-7-carboxylate.

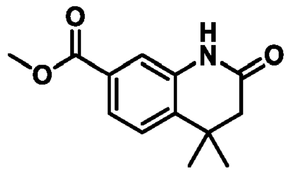

To a stirred mixture of methyl 3-(3-methylbut-2-enamido)benzoate (5.0 g, 21.43 mmol) and aluminum chloride (5.72 g, 42.9 mmol) was heated at 150 °C for 1 h. The progress of the reaction was monitored by TLC. The reaction mixture was cooled to room temperature and dissolved in with ethyl acetate (100.0 mL). The organic layer was washed with water (30.0 mL), brine (30.0 mL). The separated organic layer was dried over $Na_2SO_4$, filtered and concentrated under reduced pressure, and obtained solid was purified from diethyl ether to afford the titled compound methyl 3-(3-methylbut-2-enamido)benzoate (1.33 g, 26.6%) as off white solid MS (ES+) m/z = 234.25 (M+1).
$^1$H NMR (400 MHz, DMSO-$d_6$) δ 10.34 (s, 1H), 7.58 (dd, *J* = 8.0, 1.8 Hz, 1H), 7.50 (d, *J* = 1.8 Hz, 1H), 7.44 (d, *J* = 8.1 Hz, 1H), 3.84 (s, 3H), 1.25 (s, 6H).

**Step 3:** Methyl 4,4-dimethyl-6-nitro-2-oxo-1,2,3,4-tetrahydroquinoline-7-carboxylate

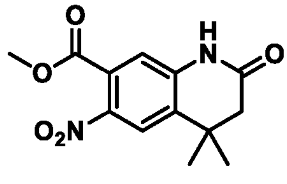

The titled compound was prepared from **Step 2** intermediate by employing similar protocol mentioned in **Example 42 Step 4** (0.23 g, 96.0 % yield).
MS (ES+) m/z = 278.91 (M+1).
$^1$H NMR (400 MHz, DMSO-$d_6$) δ 10.84 (s, 1H), 8.00 (s, 1H), 7.16 (s, 1H), 3.84 (s, 3H), 3.39 (s, 2H), 1.29 (s, 6H).

**Step 4:** Methyl 1,4,4-trimethyl-6-nitro-2-oxo-1,2,3,4-tetrahydroquinoline-7-carboxylate

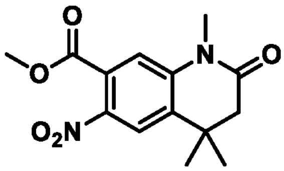

The titled compound was prepared from **Step 3** intermediate by employing similar protocol mentioned in **Example 40 Step 3** (0.21 g, 87.0 % yield).

MS (ES+) m/z = 292.94 (M+1).

$^1$H NMR (400 MHz, DMSO-$d_6$) δ 8.03 (s, 1H), 7.45 (s, 1H), 3.87 (s, 3H), 3.35 (s, 3H), 2.59 (s, 2H), 1.29 (s, 6H).

**Step 5:** Methyl 6-amino-1,4,4-trimethyl-2-oxo-1,2,3,4-tetrahydroquinoline-7-carboxylate

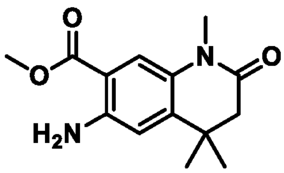

The titled compound was prepared from **Step 4** intermediate by employing similar protocol mentioned in **Example 14 Step 3** (0.17 g, 88.0 % yield).

MS (ES+) m/z = 263.33 (M+1).

$^1$H NMR (400 MHz, DMSO-$d_6$) δ 7.31 (s, 1H), 6.82 (s, 1H), 6.53 (s, 2H), 3.80 (s, 3H), 3.23 (s, 3H), 2.39 (s, 2H), 1.19 (s, 6H).

**Step 6:** 4-Chloro-2,6,9,9-tetramethyl-8,9-dihydropyrido[2,3-g]quinazolin-7(6H)-one

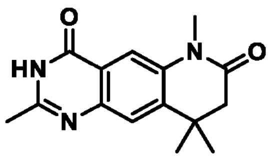

The titled compound was prepared from **Step 5** intermediate by employing similar protocol mentioned in **Example 14 Step 4** (0.13 g, 79.0 % yield).

MS (ES+) m/z = 272.25 (M+1).

$^1$H NMR (400 MHz, DMSO-$d_6$) δ 12.20 (s, 1H), 7.59 (s, 1H), 7.52 (s, 1H), 3.37 (s, 3H), 2.52 (s, 2H), 2.34 (s, 3H), 1.29 (s, 6H).

**Step 7:** 4-Chloro-2,6,9,9-tetramethyl-8,9-dihydropyrido[2,3-g]quinazolin-7(6H)-one

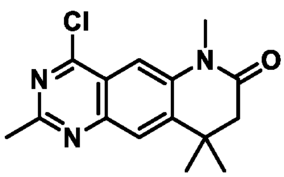

The titled compound was prepared from **Step 6** intermediate by employing similar protocol mentioned in **Example 28 Step 1** (0.07 g, 61.8 % yield).

MS (ES+) m/z = 289.21 (M+).

$^1H$ NMR (400 MHz, DMSO-$d_6$) δ 7.64 (s, 1H), 7.56 (s, 1H), 3.38 (s, 3H), 2.54 (s, 2H), 2.47 (s, 3H), 1.30 (s, 6H).

**Step 8:** (R)-4-((1-(3-(1,1-difluoro-2-hydroxy-2-methylpropyl)-2-fluorophenyl)ethyl)amino)-2,6,9,9-tetramethyl-8,9-dihydropyrido[2,3-g]quinazolin-7(6H)-one.

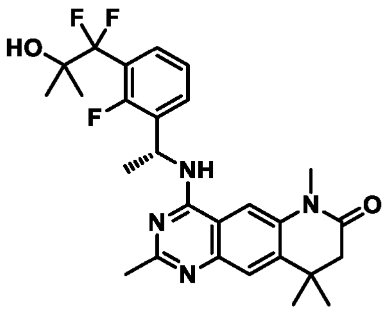

The titled compound was prepared by reaction of **Step 7** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.025 g, 24.12 % yield).

MS (ES+) m/z = 501.50 (M+1).

$^1H$ NMR (400 MHz, DMSO-$d_6$) δ 8.40 (d, $J$ = 7.4 Hz, 1H), 7.92 (s, 1H), 7.63 - 7.56 (m, 1H), 7.53 (s, 1H), 7.35 - 7.27 (m, 1H), 7.24 - 7.17 (m, 1H), 5.89 - 5.77 (m, 1H), 5.35 (s, 1H), 3.48 (s, 3H), 2.53 (s, 2H), 2.32 (s, 3H), 1.61 (d, $J$ = 7.0 Hz, 3H), 1.30 (s, 3H), 1.29 (s, 3H), 1.24 (s, 3H), 1.21 (s, 3H).

**Example 56:** Preparation of N-((R)-1-(3-amino-5-(trifluoromethyl)phenyl)ethyl)-2-methyl-6-(((R/S)-tetrahydrofuran-3-yl)methyl)-7,8-dihydro-6H-[1,4]oxazino[3,2-g]quinazolin-4-amine (**Compound-43**)

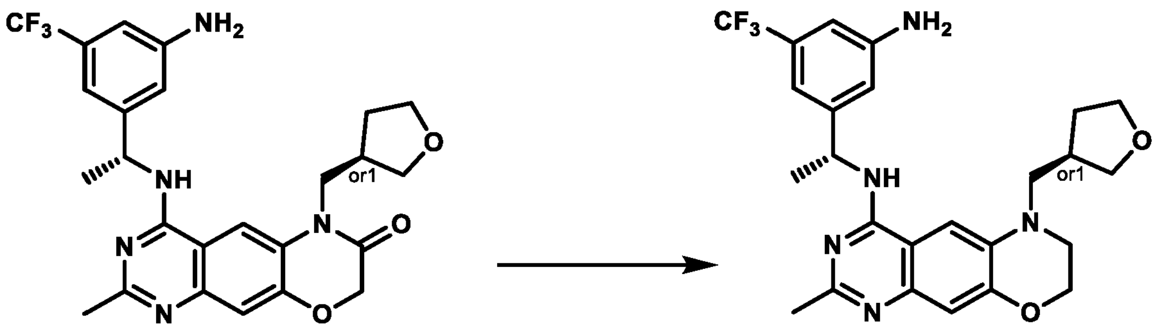

The titled compound was prepared from **Example 24a** by employing similar protocol mentioned in **Example 23 Step 1** (0.005 g, 17.14 % yield).

MS (ES+) m/z = 488.20 (M+1)

$^1H$ NMR (400 MHz, DMSO-$d_6$) δ 8.20 (s, 1H), 7.86 (d, $J$ = 8.1 Hz, 1H), 7.35 - 7.32 (m, 1H), 6.91 - 6.74 (m, 2H), 6.69 (s, 1H), 5.69 - 5.46 (m, 3H), 4.41 - 4.22 (m, 2H), 3.90 - 3.77 (m, 1H), 3.77 - 3.69 (m, 1H), 3.54 - 3.37 (m, 3H), 3.35 - 3.26 (m, 2H), 3.00 (s, 2H), 2.31 (s, 3H), 2.08 - 1.96 (m, 1H), 1.72 - 1.66 (m, 1H), 1.60 - 1.52 (m, 3H).

**Example 57:** Preparation of N-((R)-1-(3-amino-5-(trifluoromethyl)phenyl)ethyl)-2-methyl-6-(((S/R)-tetrahydrofuran-3-yl)methyl)-7,8-dihydro-6H-[1,4]oxazino[3,2-g]quinazolin-4-amine **(Compound 44)**

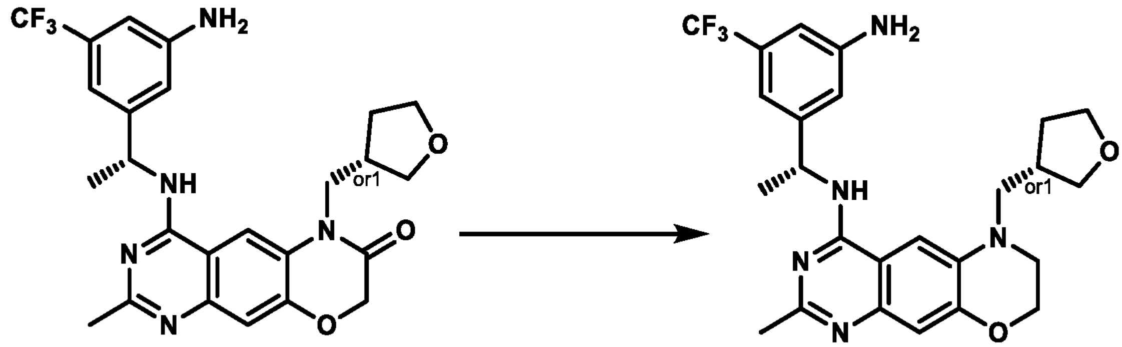

The titled compound was prepared from **Example 24b** by employing similar protocol mentioned in **Example 23 Step 1** (0.005 g, 12.86 % yield). **(Compound-44)**

MS (ES+) m/z = 488.24 (M+1)

$^{1}$H NMR (400 MHz, DMSO-$d_6$) δ 8.22 (s, 1H), 7.82 (d, *J* = 8.2 Hz, 1H), 7.34 (s, 1H), 6.94 - 6.79 (m, 2H), 6.69 (d, *J* = 1.9 Hz, 1H), 5.75 - 5.35 (m, 3H), 4.37 - 4.19 (m, 2H), 3.88 - 3.78 (m, 2H), 3.70 - 3.67 (m, 2H), 3.54 - 3.45 (m, 3H), 2.80 - 2.62 (m, 2H), 2.30 (s, 3H), 2.10 - 1.94 (m, 1H), 1.71 - 1.61 (m, 1H), 1.56 (d, *J* = 7.0 Hz, 3H).

**Example 58:** Preparation of (R)-1-(3-(1-((2,6-dimethyl-7,8-dihydro-6H-[1,4]oxazino[3,2-g]quinazolin-4-yl)amino) ethyl)-2-fluorophenyl)-1,1-difluoro-2-methylpropan-2-ol. **(Compound-45)**

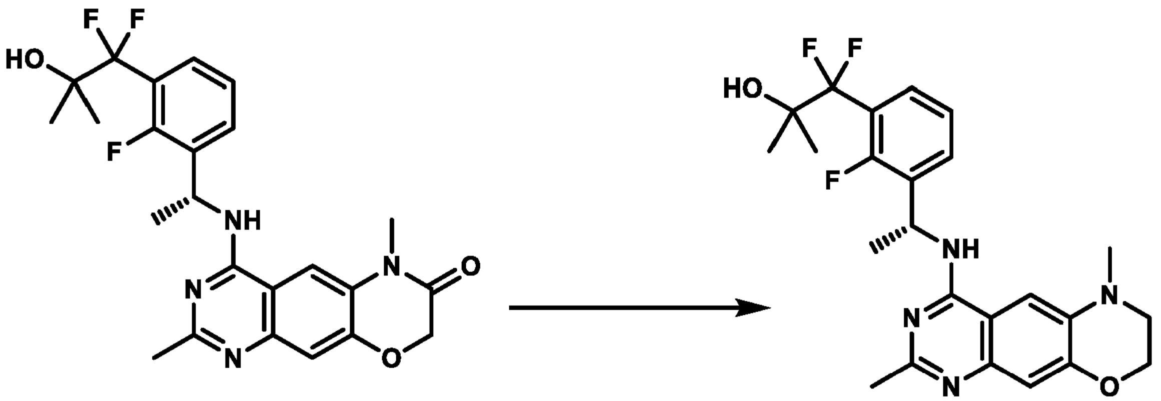

The titled compound was prepared from **Example 16** by employing similar protocol mentioned in **Example 23** (0.008 g, 27.4 % yield). **(Compound-45)**

MS (ES+) m/z = 461.20 (M+1).

$^{1}$H NMR (400 MHz, DMSO-$d_6$) δ 7.96 (d, *J* = 7.5 Hz, 1H), 7.64 - 7.51 (m, 1H), 7.37 (s, 1H), 7.36 - 7.24 (m, 1H), 7.22 - 7.18 (m, 1H), 6.79 (s, 1H), 5.92 - 5.73 (m, 1H), 5.33 (s, 1H), 4.39 - 4.25 (m, 2H), 3.43 - 3.36 (m, 2H), 3.03 (s, 3H), 2.24 (s, 3H), 1.58 (d, *J* = 7.1 Hz, 3H), 1.24 (s, 3H), 1.21 (s, 3H).

**Example 59:** (R&S)-4-(((R)-1-(3-amino-5-(trifluoromethyl)phenyl)ethyl)amino)-2,6,8-trimethyl-6H-[1,4]oxazino[3,2-g] quinazolin-7(8H)-one **(Compound 46)**

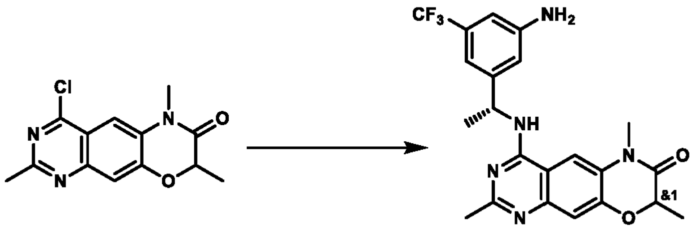

Compound (46a) & (46b)

The titled compound was prepared from **Example 43 Step 5** intermediate and using appropriate chiral amine by employing similar protocol mentioned in **Example 28 Step 2** (0.2 g, 40.20% yield).

The Two stereoisomers were separated by chiral preparative HPLC

**Peak 1:** (S/R)-4-(((R)-1-(3-amino-5-(trifluoromethyl)phenyl)ethyl)amino)-2,6,8-trimethyl-6H-[1,4]oxazino[3,2-g]quinazolin-7(8H)-one **(Compound 46a)**

MS (ES+) m/z = 446.17 (M+1).

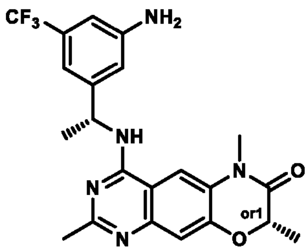

Chiral HPLC: HEX 0.1%DEA:IPA-DCM(1:1) (70:30) $t_{ret}$: 5.16 min.

$^1$H NMR (400 MHz, DMSO-$d_6$) δ 8.26 (d, *J* = 7.9 Hz, 1H), 7.97 (s, 1H), 7.10 (s, 1H), 6.88 (s, 1H), 6.86 (s, 1H), 6.71 (bs, 1H), 5.66 - 5.58 (m, 1H), 5.56 (s, 2H), 4.91 - 4.81 (m, 1H), 3.43 (s, 3H), 2.37 (s, 3H), 1.57 (d, *J*= 7.0 Hz, 3H), 1.49 (d, *J* = 6.8 Hz, 3H).

**Peak 2:** (R/S)-4-(((R)-1-(3-amino-5-(trifluoromethyl)phenyl)ethyl)amino)-2,6,8-trimethyl-6H-[1,4]oxazino[3,2-g]quinazolin-7(8H)-one. **(Compound 46b)**

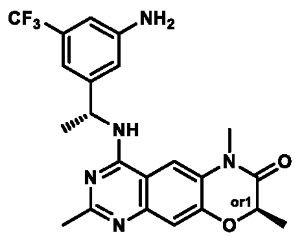

MS (ES+) m/z = 446.11 (M+1)

Chiral HPLC: HEX 0.1%DEA:IPA-MeOH(1:1)0.1% DEA(70:30) $t_{ret}$: 6.11 min

$^1$H NMR (400 MHz, DMSO-$d_6$) δ 8.25 (d, *J* = 8.0 Hz, 1H), 7.96 (s, 1H), 7.10 (s, 1H), 6.91 - 6.86 (m, 2H), 6.72 - 6.70 (m, 1H), 5.64 - 5.58 (m, 1H), 5.58 - 5.54 (m, 2H), 4.93 - 4.83 (m, 1H), 3.43 (s, 3H), 2.37 (s, 3H), 1.57 (d, *J* = 7.0 Hz, 3H), 1.48 (d, *J* = 6.8 Hz, 3H).

**Example 60:** Preparation of (R)-4-((1-(3-(1,1-difluoro-2-hydroxy-2-methylpropyl)-2-fluoro phenyl)ethyl)amino)-10-fluoro-2,6-dimethyl-6H-[1,4]oxazino[3,2-g]quinazolin-7(8H)-one

**(Compound-47)**

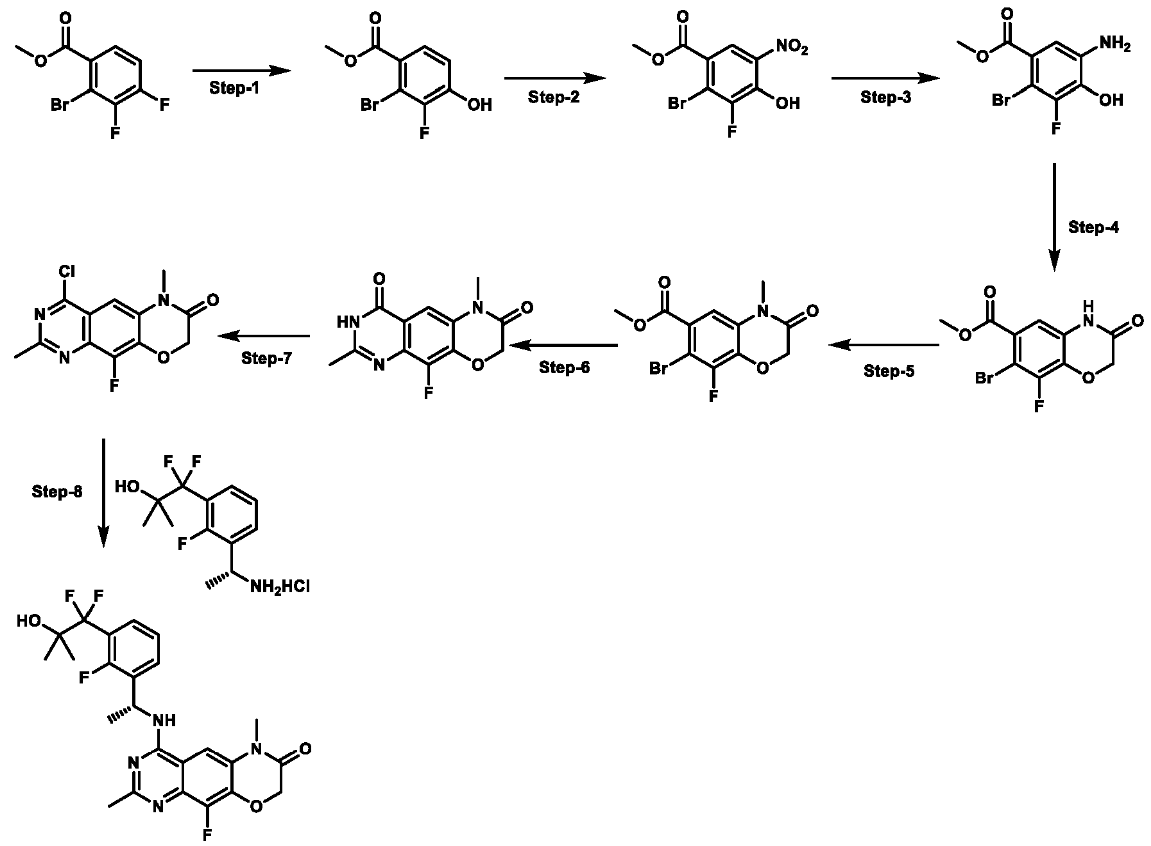


**Step 1:** Methyl 2-bromo-3-fluoro-4-hydroxybenzoate.

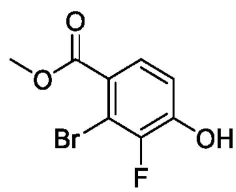

To a screw-cap vial were added N-hydroxyacetamide (5.38 g, 71.7 mmol), $K_2CO_3$ (16.52 g, 120.0 mmol), methyl 2-bromo-3,4-difluorobenzoate (6.0 g, 23.90 mmol) and DMSO (20.0 mL). The vessel was sealed and heated at 80 °C for 2 h and monitored for conversion by TLC. The reaction mixture was cooled to rt and quenched with approximately 9 equiv. of 2M HCl to a final pH of approximately 3-6. The aqueous phase was extracted with ethyl acetate (2 x 100.0 mL). The combined organic layer was washed with water (20.0 mL), brine (20.0 mL), dried over sodium sulphate and evaporated under reduced pressure to get the crude product. The crude product was purified by flash chromatography with 40% ethyl acetate in n-hexane as the eluent to afford the titled compound (5.5 g, 92.0 % yield) as white solid.
MS (ES+) m/z = 247.14 (M -2) (-ve mode)

**Step 2:** Methyl 2-bromo-3-fluoro-4-hydroxy-5-nitrobenzoate

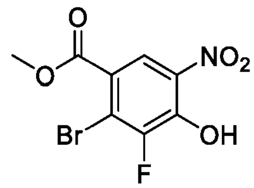

To a stirred solution of methyl 2-bromo-3-fluoro-4-hydroxybenzoate (5.5 g, 22.09 mmol) in $H_2SO_4$ (10.0 mL) at 0 °C was added potassium nitroperoxous acid (2.46 g, 24.29 mmol) portion wise for over 10 mins, the reaction mixture was stirred at 0°C for 3 h. The reaction mixture was poured on ice water, the precipitated solid was filtered and dried under reduced pressure to get crude compound. The crude compound was purified by flash chromatography with 40% ethyl acetate in n-hexane as the eluent to afford the titled compound (6.0 g, 92.0 % yield).

$^1H$ NMR (400 MHz, DMSO-$d_6$) $\delta$ 10.00 (s, 1H), 8.21-8.40 (m, 1H), 3.86 (s, 3H).

**Step 3:** Methyl 5-amino-2-bromo-3-fluoro-4-hydroxybenzoate

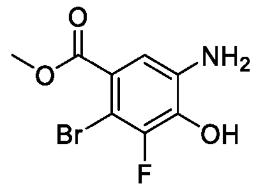

The titled compound was prepared from **Step 2** intermediate and by employing similar protocol mentioned in **Example 14 Step 3** (5.0 g, 93.0 % yield).

MS (ES+) m/z = 264.02 (M+).

**Step 4:** Methyl 7-bromo-8-fluoro-3-oxo-3,4-dihydro-2H-benzo[b][1,4]oxazine-6-carboxylate

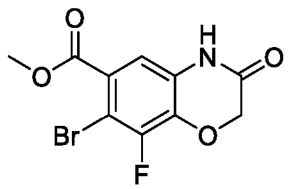

To a mixture of methyl 5-amino-2-bromo-3-fluoro-4-hydroxybenzoate (3.5 g, 13.26 mmol) in DMF (40.0 mL) was added $Et_3N$ (5.54 mL, 39.8 mmol) and 2-bromoacetyl bromide (3.21 g, 15.91 mmol) was added dropwise for 10 min and heated at 80 °C for 2 h. The progress of the reaction was monitored by TLC. The reaction mixture was allowed to cool to room temperature, diluted with ethyl acetate (100.0 mL) and washed with $H_2O$ (20.0 mL). The organic layer was dried over sodium sulfate and concentrated to get crude product. The crude product was purified by flash chromatography with 10% ethyl acetate in n-hexane as the eluent to afford the titled compound (3.7 g, 92.0 % yield).

MS (ES+) m/z = 302.21 (M -2)(-ve mode)

**Step 5:** Methyl 7-bromo-8-fluoro-4-methyl-3-oxo-3,4-dihydro-2H-benzo[b][1,4]oxazine-6-carboxylate.

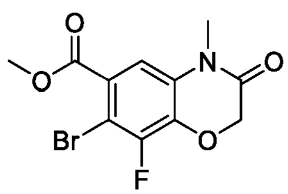

The titled compound was prepared from **Step 4** intermediate and by employing similar protocol mentioned in **Example 14 Step 2** (1.9 g, 79.0 % yield).

MS (ES+) m/z = 319.34 (M + 1)

**Step 6:** 10-Fluoro-2,6-dimethyl-3,6-dihydro-4H-[1,4]oxazino[3,2-g]quinazoline-4,7(8H)-dione

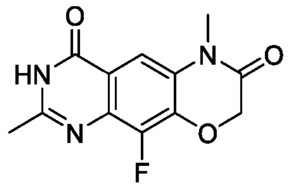

To the mixture of methyl 7-bromo-8-fluoro-4-methyl-3-oxo-3,4-dihydro-2H-benzo[b][1,4]oxazine-6-carboxylate (0.5 g, 1.57 mmol), acetimidamide hydrochloride (0.223 g, 2.36 mmol), copper(I) iodide (0.06 g, 0.314 mmol) and cesium carbonate (1.02 g, 3.14 mmol) in DMF (10.0 mL) was heated at 120 °C for 12 h. The progress of the reaction was monitored by TLC. The reaction mass was poured in water and extracted with ethyl acetate (2 x 100.0 mL). The combined organic layer was washed with water, brine and dried over anhydrous sodium sulphate and concentrated under reduced pressure to afford the titled compound (0.35 g, 85.0 % yield) as a yellow solid.

MS (ES+) m/z = 264.15 (M +1).

**Step 7:** 4-Chloro-10-fluoro-2,6-dimethyl-6H-[1,4]oxazino[3,2-g]quinazolin-7(8H)-one

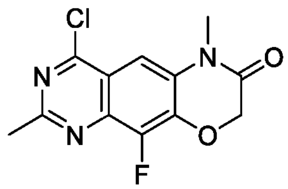

The titled compound was prepared from **Step 6** intermediate and by employing similar protocol mentioned in **Example 28 Step 1** (0.25 g, 78.0 % yield).

MS (ES+) m/z = 282.15 (M +1).

**Step 8:** (R)-4-((1-(3-(1,1-Difluoro-2-hydroxy-2-methylpropyl)-2-fluorophenyl)ethyl)amino)-10-fluoro-2,6-dimethyl-6H-[1,4]oxazino[3,2-g]quinazolin-7(8H)-one **(Compound 47)**

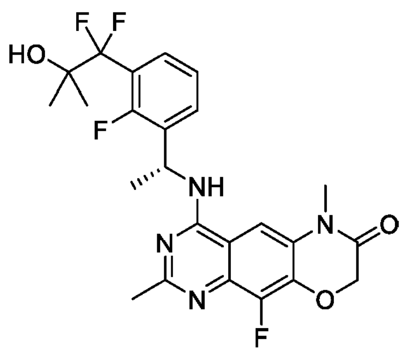

The titled compound was prepared by reaction of **Step 7** intermediate and (R)-1-(3-(1-Aminoethyl)-2-fluorophenyl)-1,1-difluoro-2-methylpropan-2-ol hydrochloride (Example 4-Step 6a) by employing similar protocol mentioned in **Example 28 Step 2** (0.025 g, 5.72 % yield).

MS (ES+) m/z = 493.36 (M + 1)

$^1$H NMR (400 MHz, DMSO-$d_6$) δ 8.49 (d, $J$ = 7.3 Hz, 1H), 7.84 (s, 1H), 7.52 - 7.65 (m, 1H), 7.35 - 7.31 (m, 1H), 7.25 - 7.20 (m, 1H), 5.89 - 5.73 (m, 1H), 5.35 (s, 1H), 4.88 (s, 2H), 3.45 (s, 3H), 2.34 (s, 3H), 1.61 (d, $J$ = 7.1 Hz, 3H), 1.24 (s, 3H), 1.21 (s, 3H).

**Example 61:** Preparation of (R)-4-((1-(3-(1,1-difluoro-2-hydroxyethyl)-2-fluorophenyl)ethyl)amino)-6-(2-methoxyethyl)-2-methyl-6H-[1,4]oxazino[3,2-g]quinazolin-7(8H)-one **(Compound 48)**

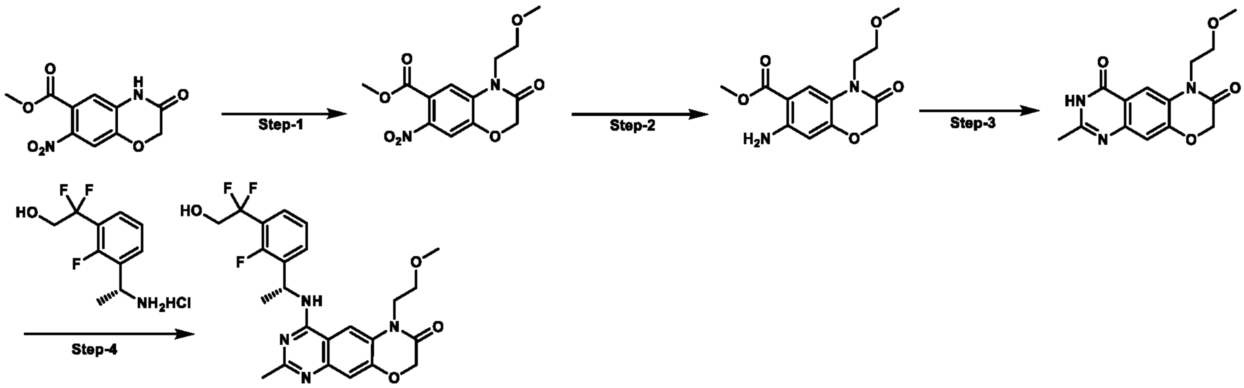


**Step 1:** Methyl 4-(2-methoxyethyl)-7-nitro-3-oxo-3,4-dihydro-2H-benzo[b][1,4]oxazine-6-carboxylate

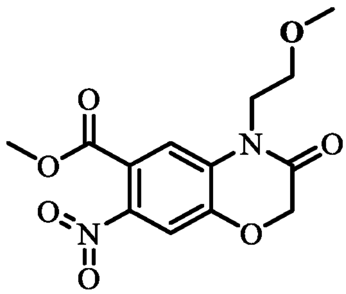

The titled compound was prepared from **Example 14 Step 1** intermediate and 1-bromo-2-methoxyethane by employing similar protocol mentioned in **Example 14 Step 2** (1.6 g, 87.0 % yield).

MS (ES+) m/z = 311.34 (M+1)

$^1$H NMR (400 MHz, Chloroform-$d$) δ 7.61 (s, 1H), 7.55 (s, 1H), 4.76 (s, 2H), 4.17 (t, $J$ = 5.3 Hz, 2H), 3.94 (s, 3H), 3.69 (t, $J$ = 5.3 Hz, 2H), 3.36 (s, 3H).

**Step 2:** Methyl 7-amino-4-(2-methoxyethyl)-3-oxo-3,4-dihydro-2H-benzo[b][1,4]oxazine-6-carboxylate

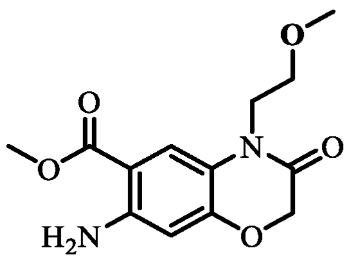

The titled compound was prepared from **Step 1** intermediate and by employing similar protocol mentioned in

**Example 14 Step 3.** (1.4 g, 97.0 % yield).
MS (ES+) m/z = 281.1 (M+1).

**Step 3:** 4-Hydroxy-6-(2-methoxyethyl)-2-methyl-6H-[1,4]oxazino[3,2-g]quinazolin-7(8H)-one

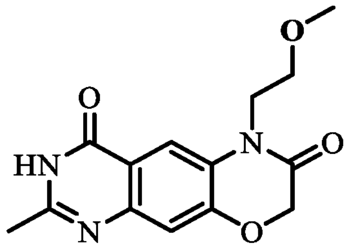

The titled compound was prepared from **Step 2** intermediate and by employing similar protocol mentioned in **Example 14 Step 4** (0.3 g, 20.76 % yield).
$^1$H NMR (400 MHz, DMSO-$d_6$) δ 12.17 (s, 1H), 7.81 (s, 1H), 7.11 (s, 1H), 4.80 (s, 2H), 4.17 (t, *J* = 5.6 Hz, 2H), 3.59 (t, *J*= 5.6 Hz, 2H), 3.25 (s, 3H), 2.32 (s, 3H).

**Step 4 :** (R)-4-((1-(3-(1,1-difluoro-2-hydroxyethyl)-2-fluorophenyl)ethyl)amino)-6-(2-methoxyethyl)-2-methyl-6H-[1,4] oxazino[3,2-g]quinazolin-7(8H)-one. **(Compound 48)**

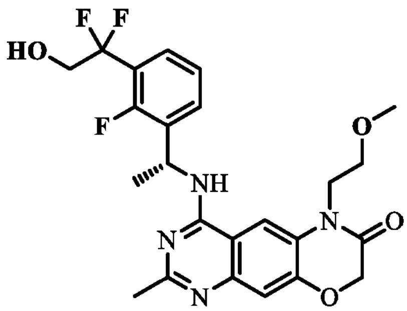

The titled compound was prepared from **Step 3** intermediate and using appropriate chiral amine by employing similar protocol mentioned in **Example 14 Step 5.** (0.02 g, 4.01 % yield).
MS (ES+) m/z = 491.17 (M+1)
$^1$H NMR (400 MHz, DMSO-$d_6$) δ 8.35 (d, *J* = 7.3 Hz, 1H), 8.08 (s, 1H), 7.64 - 7.61 (m, 1H), 7.45 - 7.41 (m, 1H), 7.28 - 7.25 (m, 1H), 7.11 (s, 1H), 5.85 - 5.77 (m, 1H), 4.77 (s, 2H), 4.47 - 4.20 (m, 3H), 3.94 (t, *J* = 14.4 Hz, 2H), 3.60 (s, 2H), 3.26 (s, 3H), 2.32 (s, 3H), 1.63 (d, *J* = 7.1 Hz, 3H).

**Example 62:** Preparation of (R)-4-((1-(3-amino-5-(trifluoromethyl)phenyl)ethyl)amino)-6-ethyl-2-methyl-6H-[1,4]oxazino[3,2-g]quinazolin-7(8H)-one. **(Compound 49)**

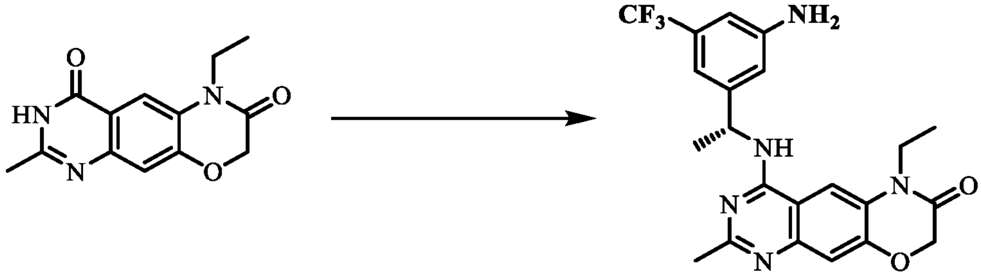

The titled compound was prepared from **Example 26 Step 3** intermediate and by employing similar protocol mentioned in **Example 14 Step 5** (0.089 g, 17.27% yield). **(Compound-49)**

MS (ES+) m/z = 446.2 (M+1).

$^{1}$H NMR (400 MHz, DMSO-$d_6$) δ 8.27 (d, *J* = 8.0 Hz, 1H), 7.96 (s, 1H), 7.11 (s, 1H), 6.93 - 6.83 (m, 2H), 6.73 - 6.69 (m, 1H), 5.68 - 5.58 (m, 1H), 5.57 (s, 2H), 4.76 (s, 2H), 4.26 - 3.99 (m, 2H), 2.36 (s, 3H), 1.58 (d, *J*= 7.1 Hz, 3H), 1.22 (t, *J* = 7.0 Hz, 3H).

**Example 63:** Preparation of (R)-1-(3-(1-((6-ethyl-2,8,8-trimethyl-7,8-dihydro-6H-[1,4]oxazino[3,2-g]quinazolin-4-yl) amino)ethyl)-2-fluorophenyl)-1,1-difluoro-2-methylpropan-2-ol. **(Compound 50)**

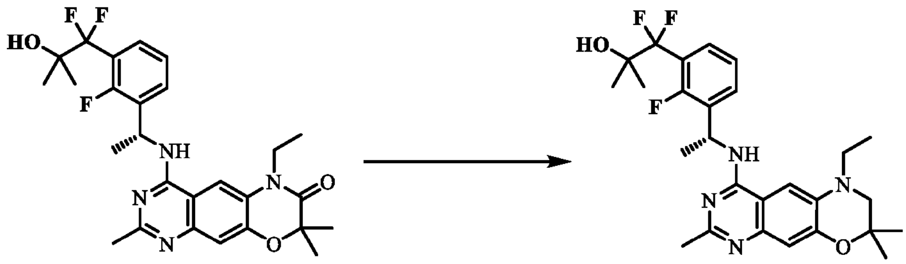

The titled compound was prepared from **compound 41** by employing similar protocol mentioned in **Example 23 Step 1** (0.165 g, 42.4 % yield)

MS (ES+) m/z = 503.2 (M+1)

$^{1}$H NMR (400 MHz, DMSO-$d_6$) δ 7.92 (d, *J* = 7.5 Hz, 1H), 7.62 - 7.57 (m, 1H), 7.42 (s, 1H), 7.34 - 7.25 (m, 1H), 7.22 - 7.18 (m, 1H), 6.76 (s, 1H), 5.85 - 5.78(m, 1H), 5.33 (s, 1H), 3.65 - 3.54 (m, 2H), 3.14 (s, 2H), 2.23 (s, 3H), 1.58 (d, *J*= 7.1 Hz, 3H), 1.32 (s, 3H), 1.31 (s, 3H), 1.24 (s, 3H), 1.21 (s, 3H), 1.18 (m, 3H). **Example 64:** Preparation of (R)-4-((1-(3-(1,1-difluoro-2-methoxyethyl)-2-fluorophenyl)ethyl)amino)-2,6,8,8-tetramethyl-6H-[1,4]oxazino[3,2-g]quinazolin-7(8H)-one **(Compound 51)**

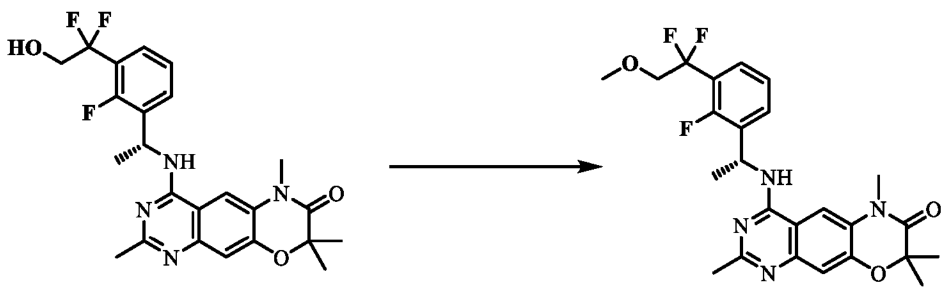

To a stirred solution of (R)-4-((1-(3-(1,1-difluoro-2-hydroxyethyl)-2-fluorophenyl) ethyl)amino)-2,6,8,8-tetramethyl-6H-[1,4]oxazino[3,2-g]quinazolin-7(8H)-one **Compound 18** (0.25 g, 0.53 mmol) in DMF (5.0 mL) was degassed with $N_2$ and was added NaH (0.023 g, 0.58 mmol) at 0°C and stirred for 5 min and then iodomethane (0.036 mL, 0.58 mmol) was added dropwise at 0°C for 30 min. The progress of the reaction was monitored by TLC. After completion of reaction, mixture was quenched in 10.0 mL ice cold water, solid precipitated was filtered and dried to get crude solid compound. The crude solid was purified by flash chromatography using (5-6% methanol in DCM) as a eluent to afford the titled compound (R)-4-((1-(3-(1,1-difluoro-2-methoxyethyl)-2-fluorophenyl)ethyl)amino)-2,6,8,8-tetramethyl-6H-[1,4]oxazino[3,2-g]quinazolin-7(8H)-one (0.140 g, 54.4 % yield) as an off white solid.

MS (ES+) m/z = 489.2 (M+1).

$^{1}$H NMR (400 MHz, DMSO-$d_6$) δ 8.38 (d, *J* = 7.1 Hz, 1H), 8.00 (s, 1H), 7.68 - 7.64 (m, 1H), 7.46 - 7.42 (m, 1H), 7.30 - 7.26 (m, 1H), 7.07 (s, 1H), 5.85 - 5.78 (m, 1H), 4.00 (t, *J* = 14.0 Hz, 2H), 3.47 (s, 3H), 3.34 (s, 3H), 2.31 (s, 3H), 1.62 (d, *J* = 7.1 Hz, 3H), 1.47 (s, 3H), 1.46 (s, 3H).

**Example 65:** Preparation of (R)-N-(1-(3-(1,1-difluoro-2-methoxyethyl)-2-fluorophenyl)ethyl)-2,6,8,8-tetramethyl-7,8-dihydro-6H-[1,4]oxazino[3,2-g]quinazolin-4-amine **(Compound 52)**

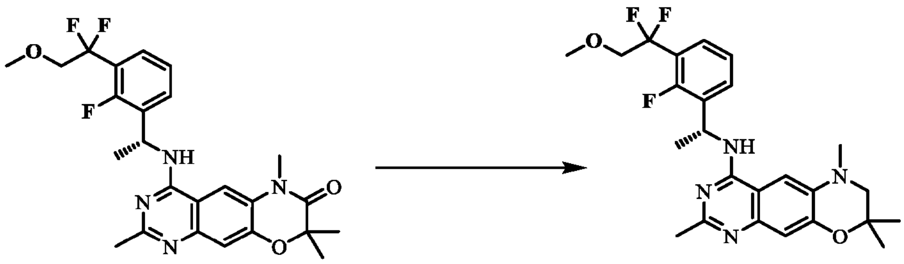

The titled compound was prepared from **compound 51** by employing similar protocol mentioned in **Example 23 Step 1** (0.036 g, 37.1 % yield).
MS (ES+) m/z = 475.17 (M+1)
$^1$H NMR (400 MHz, DMSO-d6) δ 7.95 (d, *J* = 7.4 Hz, 1H), 7.67 - 7.63 (m, 1H), 7.45 - 7.36 (m, 2H), 7.27 - 7.23 (m, 1H), 6.76 (s, 1H), 5.85 - 5.78 (m, 1H), 3.99 (t, *J* = 14.0 Hz, 2H), 3.34 (s, 3H), 3.12 (s, 2H), 3.07 (s, 3H), 2.25 (s, 3H), 1.59 (d, *J* = 7.1 Hz, 3H), 1.34 (s, 3H), 1.33 (s, 3H).

**Example 66:** Preparation of (R&S)-4-(((1R)-1-(3-(1,1-difluoro-2-hydroxy-3-methoxy-2-methylpropyl)-2-fluorophenyl) ethyl)amino)-2,6,8,8-tetramethyl-6H-[1,4]oxazino[3,2-g]quinazolin-7(8H)-one. **(Compound 53)**

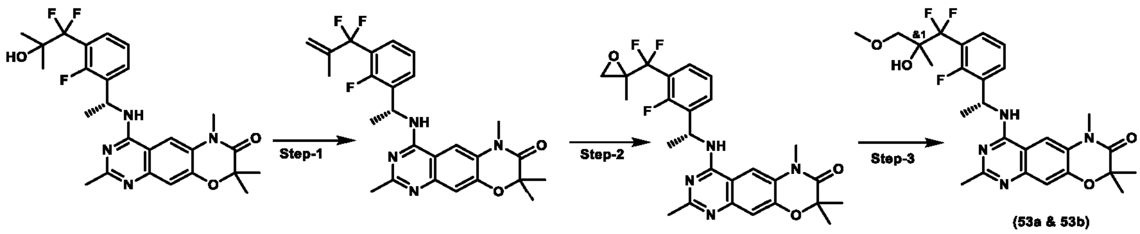


(53a & 53b)

**Step 1** : (R)-4-((1-(3-(1,1-difluoro-2-methylallyl)-2-fluorophenyl)ethyl)amino)-2,6,8,8-tetramethyl-6H-[1,4]oxazino[3,2-g]quinazolin-7(8H)-one

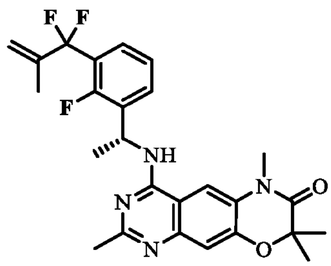

To a stirred solution of (R)-4-((1-(3-(1,1-difluoro-2-hydroxy-2-methylpropyl)-2-fluorophenyl)ethyl)amino)-2,6,8,8-tetramethyl-6H-[1,4]oxazino[3,2-g]quinazolin-7(8H)-one (0.4 g, 0.796 mmol) in DCM (2.0 mL) was added dimethylamino)sulfur trifluoride (0.315 mL, 2.388 mmol) at -78°C and stirred for 30 min. The reaction progress was monitored by TLC. The reaction mixture was diluted with 20.0 mL dichloromethane, washed with 10.0 mL 10% HCl, 5.0 mL water and brine solution. The organic layer was separated dried over sodium sulfate and evaporated under reduced pressure to get crude compound. The crude compound was purified by flash chromatography using eluent (0-10%) of methanol in dichloromethane to afford the titled compound (R)-4-((1-(3-(1,1-difluoro-2-methylallyl)-2-fluorophenyl)ethyl) amino)-2,6,8,8-tetramethyl-6H-[1,4]oxazino [3,2-g]quinazolin-7(8H)-one (0.37g, 96.0 % yield) as off white solid.
MS (ES+) m/z = 485.33 (M+1).

**Step 2:** 4-(((1R)-1-(3-(difluoro(2-methyloxiran-2-yl)methyl)-2-fluorophenyl)ethyl)amino)-2,6,8,8-tetramethyl-6H-[1,4]oxazino[3,2-g]quinazolin-7(8H)-one

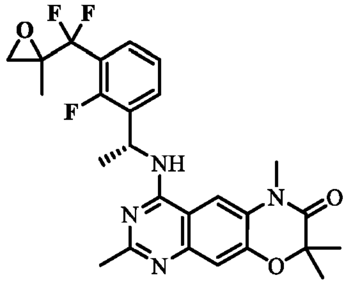

To a stirred solution of (R)-4-((1-(3-(1,1-difluoro-2-methylallyl)-2-fluorophenyl)ethyl)amino)-2,6,8,8-tetramethyl-6H-[1,4]oxazino[3,2-g]quinazolin-7(8H)-one (0.32 g, 0.660 mmol) was dissolved in formic acid (5.0 mL) and 50% hydrogen peroxide (0.02 mL, 0.660 mmol) was added and reaction was stirred at room temperature for 16 h. The progress of the reaction was monitored by TLC. The reaction was diluted with dichloromethane and basified with saturated sodium bicarbonate. The organic layer separated dried over sodium sulfate and evaporated under reduced pressure to get crude compound. The crude compound was purified by flash chromatography using eluent (0-10%) of methanol in dichloromethane to afford the titled compound 4-(((1R)-1-(3-(difluoro(2-methyloxiran-2-yl)methyl)-2-fluorophenyl)ethyl)amino)-2,6,8,8-tetramethyl-6H-[1,4]oxazino[3,2-g]quinazolin-7(8H)-one (0.33 g, 100.0% yield) as off white solid.
MS (ES+) m/z = 501.09 (M+1).

**Step 3: (R&S)**-4-(((1R)-1-(3-(1,1-difluoro-2-hydroxy-3-methoxy-2-methylpropyl)-2-fluorophenyl) ethyl)amino)-2,6,8,8-tetramethyl-6H-[1,4]oxazino[3,2-g]quinazolin-7(8H)-one.

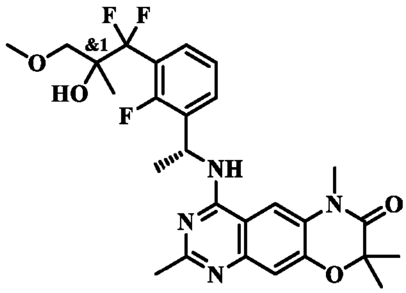

To a stirred solution of the 4-(((1R)-1-(3-(difluoro(2-methyloxiran-2-yl)methyl)-2-fluorophenyl)ethyl)amino)-2,6,8,8-tetramethyl-6H-[1,4]oxazino[3,2-g]quinazolin-7(8H)-one (0.13 g, 0.26 mmol) in methanol (2.0 mL) was added the sodium methoxide 30% in methanol (0.234 g, 1.299 mmol). The reaction mixture was stirred at 80°C for 24 h. The progress of the reaction was monitored by TLC. The reaction was cooled to room temperature and solvent was removed in vacuo. The crude reaction mixture was diluted with 20.0 mL dichloromethane and washed with 5.0 mL water and 5.0 mL brine solution. The organic layer separated was dried over sodium sulfate and was evaporated to get crude compound. The crude compound was purified by flash chromatography using eluent (0-10%) of methanol in dichloromethane to afford the titled compound 0.14 g solid compound was submitted for chiral prep purification to get both isomers as an off white solid. Chiral separation of the above compound gave two stereoisomers

**Peak 1:** 4-(((R)-1-(3-((S/R)-1,1-difluoro-2-hydroxy-3-methoxy-2-methylpropyl)-2-fluorophenyl)ethyl)amino)-2,6,8,8-tetramethyl-6H-[1,4]oxazino[3,2-g]quinazolin-7(8H)-one

**(Compound 53a)**

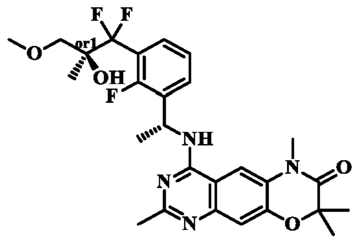

MS (ES+) m/z = 533.50 (M+1).

Chiral HPLC: HEX 0.1% DEA: IPA - MeOH (1:1) (85:15) $t_{ret}$: 9.78 min

**Peak 1:** $^{1}$H NMR (400 MHz, DMSO-d6) δ 8.42 (bs, 1H), 8.02 (s, 1H), 7.64 - 7.60 (m, 1H), 7.34 - 7.27 (m, 1H), 7.25 - 7.20 (m, 1H), 7.08 (s, 1H), 5.87 - 5.81 (m, 1H), 5.48 (s, 1H), 3.47 (s, 3H), 3.33 - 3.28 (m, 2H), 3.25 (s, 3H), 2.32 (s, 3H), 1.61 (d, *J* = 7.0 Hz, 3H), 1.48 (s, 3H), 1.46 (s, 3H), 1.22 (s, 3H).

**Peak 2:** 4-(((R)-1-(3-((R/S)-1,1-difluoro-2-hydroxy-3-methoxy-2-methylpropyl)-2-fluoro phenyl)ethyl) amino)-2,6,8,8-tetramethyl-6H-[1,4]oxazino[3,2-g]quinazolin-7(8H)-one **(Compound 53b)**

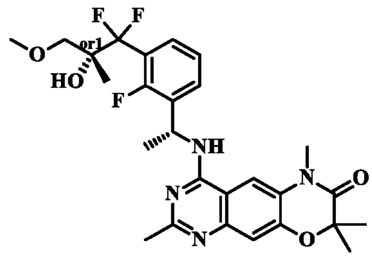

MS (ES+) m/z = 533.5 (M+1)

Chiral HPLC: HEX 0.1% DEA: IPA - MeOH (1:1) (85:15) $t_{ret}$: 10.64 min

**Peak 2:** $^{1}$H NMR (400 MHz, DMSO-$d_6$) δ 8.35 (d, *J* = 7.4 Hz, 1H), 8.01 (s, 1H), 7.63 - 7.59 (m, 1H), 7.34 - 7.28 (m, 1H), 7.25 - 7.20 (m, 1H), 7.07 (s, 1H), 5.87 - 5.81 (m, 1H), 5.50 (s, 1H), 3.47 (s, 3H), 3.33 -3.25 (m, 2H), 3.24 (s, 3H), 2.32 (s, 3H), 1.60 (d, *J*= 7.1 Hz, 3H), 1.47 (s, 3H), 1.46 (s, 3H), 1.24 (s, 3H).

**Example 67:** Preparation of (R&S)- 4-(((1R)-1-(2-fluoro-3-(1,1,3-trifluoro-2-hydroxy-2-methyl propyl)phenyl)ethyl)amino)-2,6,8,8-tetramethyl-6H-[1,4]oxazino[3,2-g]quinazolin-7(8H)-one **(Compound 54)**

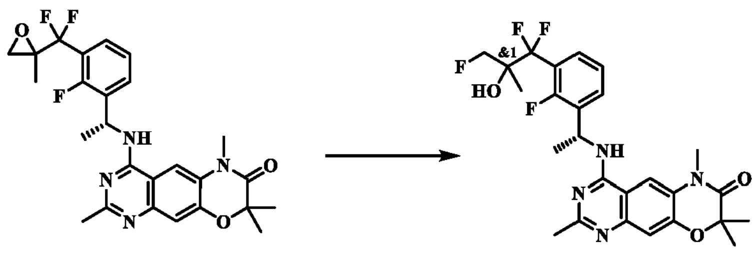

To a stirred solution of the 4-(((1R)-1-(3-(difluoro(2-methyloxiran-2-yl)methyl)-2-fluorophenyl)ethyl)amino)-2,6,8,8-tetramethyl-6H-[1,4]oxazino[3,2-g]quinazolin-7(8H)-one (0.20 g, 0.40 mmol) **(Example 66 Step 2)** in dichloromethane (10.0 mL) was added the HF-pyridine (0.28 g, 1.998 mmol) at 0°C. The reaction mixture was stirred at room temperature for 16 h. The progress of the reaction was monitored by TLC. The crude rection mixture was diluted with 20.0 mL dichloromethane and washed with 10.0 mL saturated sodium bicarbonate solution, 5.0 mL water and 5.0 mL brine solution. The organic layer was separated, dried over sodium sulfate evaporated under reduced pressure to get crude compound. The crude compound was purified by flash chromatography using eluent (0-10%) of methanol in dichloromethane to get the crude compound 0. 280 g which was submitted for RP-prep purification to afford the titled compound (R&S)-4-(((1R)-1-(2-fluoro-3-(1,1,3-trifluoro-2-hydroxy-2-methylpropyl)phenyl)ethyl)amino)-2,6,8,8-tetramethyl-6H-[1,4]oxazino[3,2-g]quinazolin-7(8H)-one (0.099 g, 47.6 % yield) as off white solid.

MS (ES+) m/z = 521.42 (M+1).

$^1$H NMR (400 MHz, DMSO-$d_6$) δ 8.39 - 8.34 (m, 1H), 8.00 (s, 1H), 7.65 - 7.61 (m, 1H), 7.38 - 7.29 (m, 1H), 7.27 - 7.23 (m, 1H), 7.07 (s, 1H), 5.91 (d, *J*= 8.2 Hz, 1H), 5.88 - 5.78 (m, 1H), 4.58 - 4.22 (m, 2H), 3.47 (s, 3H), 2.31 (d, *J* = 3.8 Hz, 3H), 1.61 (d, *J* = 7.0 Hz, 3H), 1.47 (s, 3H), 1.46 (s, 3H), 1.27 (d, *J* = 8.0 Hz, 3H).

**Example 68:** Preparation of (R&S)-4-(((1R)-1-(3-(1,1-difluoro-2-hydroxy-2-methyl-3-(methylamino)propyl)-2-fluorophenyl)ethyl)amino)-2,6,8,8-tetramethyl-6H-[1,4]oxazino[3,2-g]quinazolin-7(8H)-one **(Compound 55)**

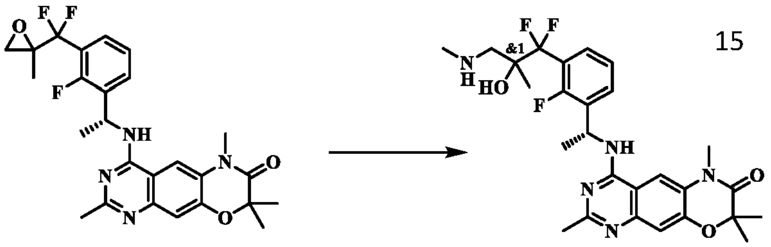

To a solution of the 4-(((1R)-1-(3-(difluoro(2-methyloxiran-2-yl)methyl)-2-fluorophenyl)ethyl)amino)-2,6,8,8-tetramethyl-6H-[1,4]oxazino[3,2-g]quinazolin-7(8H)-one (0.16 g, 0.320 mmol) **(Example 66 Step 2)** in IPA (3.0 mL) was added the methanamine in ethanol (301.0 mg, 3.20 mmol) followed by water (1.0 mL) and TEA (0.089 mL, 0.64 mmol). The reaction was stirred at 80°C for 16 h. The progress of the reaction was monitored by TLC. The reaction was cooled to room temperature and solvent was removed in vacuo. The crude reaction mixture was diluted with 20.0 mL dichloromethane and washed with 5.0 mL water, 5.0 mL brine solution. The organic layer was separated dried over sodium sulfate and evaporated under reduced pressure to get crude compound. The crude compound obtained was purified by flash chromatography using eluent (0-10%) of methanol in dichloromethane to afford 0.3 g solid compound which was submitted for RP-prep purification to afford the titled compound (R&S)- 4-(((1R)-1-(3-(1,1-difluoro-2-hydroxy-2-methyl-3-(methylamino)propyl)-2-fluorophenyl)ethyl)amino)-2,6,8,8-tetramethyl-6H-[1,4]oxazino[3,2-g]quinazolin-7(8H)-one (0.13 g, 77.0 % yield) as off white solid.

MS (ES+) m/z = 532.13 (M+1).

$^1$H NMR (400 MHz, DMSO-$d_6$) δ 8.39 - 8.34 (m, 1H), 8.01 (s, 1H), 7.63 - 7.59 (m, 1H), 7.33 - 7.30 (m, 1H), 7.25 - 7.21 (m, 1H), 7.08 - 7.06 (m, 1H), 5.87 - 5.79 (m, 1H), 5.36 (s, 1H), 3.47 (s, 3H), 2.71 - 2.55 (m, 2H), 2.35 - 2.27 (m, 6H), 1.64 - 1.56 (m, 3H), 1.47 (s, 3H), 1.46 (s, 3H), 1.26 (s, 3H).

**Example 69:** Preparation of (R)-2,2-difluoro-2-(2-fluoro-3-(1-((2-methyl-7,8-dihydro-6H-pyrano[3,2-g]quinazolin-4-yl)amino)ethyl)phenyl)ethan-1-ol **(Compound 56)**

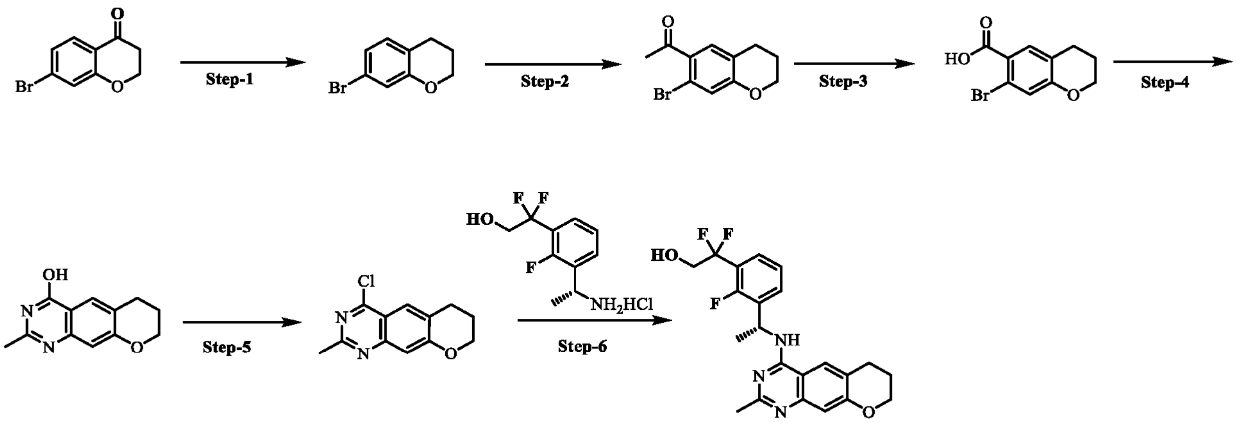

**Step 1:** 7-Bromochromane

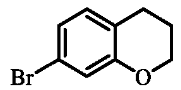

To a mixture of 7-bromochroman-4-one (8.0 g, 35.2 mmol) in TFA (70.0 mL) was added triethylsilane (28.1 mL, 176.0 mmol) at room temperature and reaction was stirred for 16 h at room temperature. The solvent was evaporated under reduced pressure and diluted 90.0 mL water and 90.0 mL aqueous solution of sodium bicarbonate was added to the mixture followed by extraction with ethyl acetate (90.0 mL x 3). The organic layer was washed with a aqueous solution of sodium bicarbonate, saturated brine and dried over sodium sulfate, and the solvent was distilled off under reduced pressure to get crude product. The crude product was purified by column chromatography using eluent 10% ethyl acetate: n-hexane to afford the titled compound 7-bromochromane (5.5 g, 73.3 % yield).

$^1$H NMR (400 MHz, Chloroform-*d*) δ 7.02 - 6.95 (m, 2H), 6.92-6.87 (m, 1H), 4.29 - 4.10 (m, 2H), 2.75 (t, *J* = 6.5 Hz, 2H), 2.11 - 1.91 (m, 2H).

**Step 2:** 1-(7-Bromochroman-6-yl)ethan-1-one

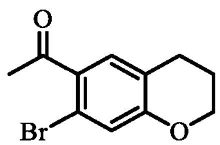

To the stirred solution of acetyl chloride (2.39 mL, 33.6 mmol) in dry DCM (50.0 mL) in a -10°C bath was added aluminum chloride (3.79 g, 28.4 mmol) in small portions. The mixture was stirred for 15 min until the solution became homogeneous. This solution was added to a solution of 7-bromochromane (5.5 g, 25.8 mmol) in dichloromethane (10.0 mL) at -10°C dropwise for 10 min. After complete addition, the solution was stirred at -10°C. for 30 min and at room temperature for 1 h. The solution was poured over a mixture of 50.0 g crushed ice and 10.0 mL conc HCl. The mixture was extracted with dichloromethane (50.0 mL x 3). The combined organic layers were washed with brine, dried over $Na_2SO_4$ and concentrated under reduced pressure to get crude residue. The crude residue was purified by flash chromatography using eluent 10-15% ethyl acetate in n-hexane to afford the titled compound 1-(7-bromochroman-6-yl)ethan-1-one (6.1 g, 93.0 % yield) of solid compound. $^1$H NMR (400 MHz, Chloroform-*d*) δ 7.37 (s, 1H), 7.06 (s, 1H), 4.26 - 4.16 (m, 2H), 2.80 - 2.72 (m, 2H), 2.63 (s, 3H), 2.05-2.02 (m, 2H).

**Step 3:** 7-Bromochromane-6-carboxylic acid

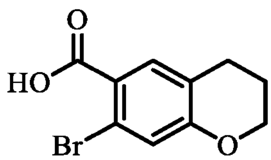

To the stirred solution of potassium hydroxide (13.72 g, 245.0 mmol) in water (50.0 mL) at 0 °C bromine (4.20 mL, 82.0 mmol) was added dropwise for 10 min. To the resulting solution was added 1-(7-bromochroman-6-yl)ethan-1-one (5.2 g, 20.38 mmol) dropwise at 0°C for 10 min. The reaction mixture was heated at 55°C and maintained for 16 h. The progress of the reaction was monitored by TLC. The reaction was cooled to room temperature and quenched with 40.0 mL of sodium thiosulfate. The mixture was acidified to pH 2-3 with cone. HCl (30.0 mL). The precipitated solid was collected by filtration and washed with water and dried under reduced pressure to afford the titled compound 7-bromochromane-6-carboxylic acid (5.21 g, 99 % yield).

$^1$H NMR (400 MHz, DMSO-$d_6$) δ 7.62 (s, 1H), 7.05 (s, 1H), 4.19 (t, *J* = 5.1 Hz, 2H), 2.72 (t, *J* = 6.4 Hz, 2H), 1.96 - 1.86 (m, 2H).

**Step 4:** 2-Methyl-3,6,7,8-tetrahydro-4H-pyrano[3,2-g]quinazolin-4-one

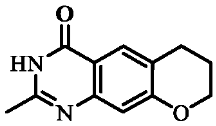

The titled compound was prepared from **Step 3** intermediate by employing similar protocol mentioned in **Example 30 Step 8** (1.80 g, 41.2% yield).

MS (ES+) m/z = 217.51 (M+1).

$^1$H NMR (400 MHz, DMSO-$d_6$) δ 12.04 - 11.77 (m, 1H), 7.78 (d, *J* = 1.2 Hz, 1H), 6.81 (s, 1H), 4.45 - 4.09 (m, 2H), 2.87 (t, *J* = 6.4 Hz, 2H), 2.29 (s, 3H), 2.14 - 1.86 (m, 2H).

**Step 5:** 4-Chloro-2-methyl-7,8-dihydro-6H-pyrano[3,2-g]quinazoline

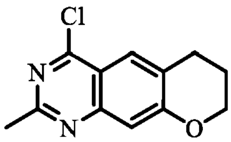

The titled compound was prepared from **Step 4** intermediate by employing similar protocol mentioned in **Example 28 Step 1** (0.35 g, 64.5 % yield).

MS (ES+) m/z = 235.33 (M+1).

$^1$H NMR (400 MHz, DMSO-$d_6$) δ 7.95 (d, *J* = 1.4 Hz, 1H), 7.17 (s, 1H), 4.51 - 4.26 (m, 2H), 3.05 (t, *J* = 6.1 Hz, 2H), 2.66 (s, 3H), 2.10 - 1.95 (m, 2H).

**Step 6:** (R)-2,2-difluoro-2-(2-fluoro-3-(1-((2-methyl-7,8-dihydro-6H-pyrano[3,2-g]quinazolin-4-yl)amino)ethyl)phenyl) ethan-1-ol **(Compound-56)**

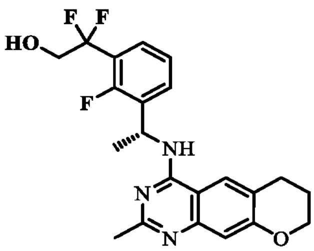

The titled compound was prepared from **Step 5** intermediate using appropriate chiral amine by employing similar protocol mentioned in **Example 28 Step 2** (0.03 g, 10.87 % yield).

MS (ES+) m/z = 417.04 (M+).

$^1$H NMR (400 MHz, DMSO- $d_6$) δ 8.23 - 8.12 (m, 2H), 7.64 (t, *J* = 7.0 Hz, 1H), 7.42-7.38 (m, 1H), 7.24 (t, *J*= 7.7 Hz, 1H), 6.81 (s, 1H), 5.84 - 5.65 (m, 2H), 4.28 - 4.20 (m, 2H), 3.94 (t, *J* = 14.4 Hz, 2H), 2.95 (t, *J* = 6.4 Hz, 2H), 2.29 (s, 3H), 2.02-1.97 (m, 2H), 1.57 (d, *J* = 7.0 Hz, 3H).

**Example 70:** Preparation of (R)-4-((1-(3-(1,1-difluoro-2-hydroxy-2-methylpropyl)phenyl)ethyl)amino)-2,6,8,8-tetramethyl-6H-[1,4]oxazino[3,2-g]quinazolin-7(8H)-one. **(Compound 57)**

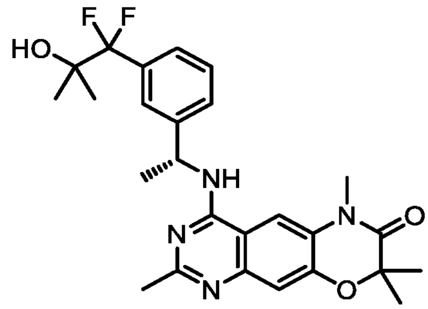

The titled compound was prepared from **Example 31 Step 5** intermediate using appropriate chiral amine by employing similar protocol mentioned in **Example 28 Step 2** (0.072 g, 43.30% yield).

MS (ES+) m/z = 485.42 (M+1).

$^{1}$H NMR (400 MHz, DMSO- $d_6$) d 8.32 (d, $J$ = 7.9 Hz, 1H), 7.95 (s, 1H), 7.59 - 7.56 (m, 1H), 7.46 - 7.38 (m, 2H), 7.36 - 7.32 (m, 1H), 7.07 (s, 1H), 5.71 - 5.62 (m, 1H), 5.25 (s, 1H), 3.44 (s, 3H), 2.35 (s, 3H), 1.63 (d, J = 7.1 Hz, 3H), 1.47 (s, 3H), 1.45 (s, 3H), 1.14 (s, 6H).

**Example 71:** Preparation of (R)-4'-((1-(3-(1,1-difluoro-2-hydroxy-2-methylpropyl)-2-fluorophenyl) ethyl)amino)-2',8'-dimethylspiro[cyclopentane-1,6'-pyrrolo[3,2-g]quinazolin]-7'(8'H)-one

**(Compound 58)**

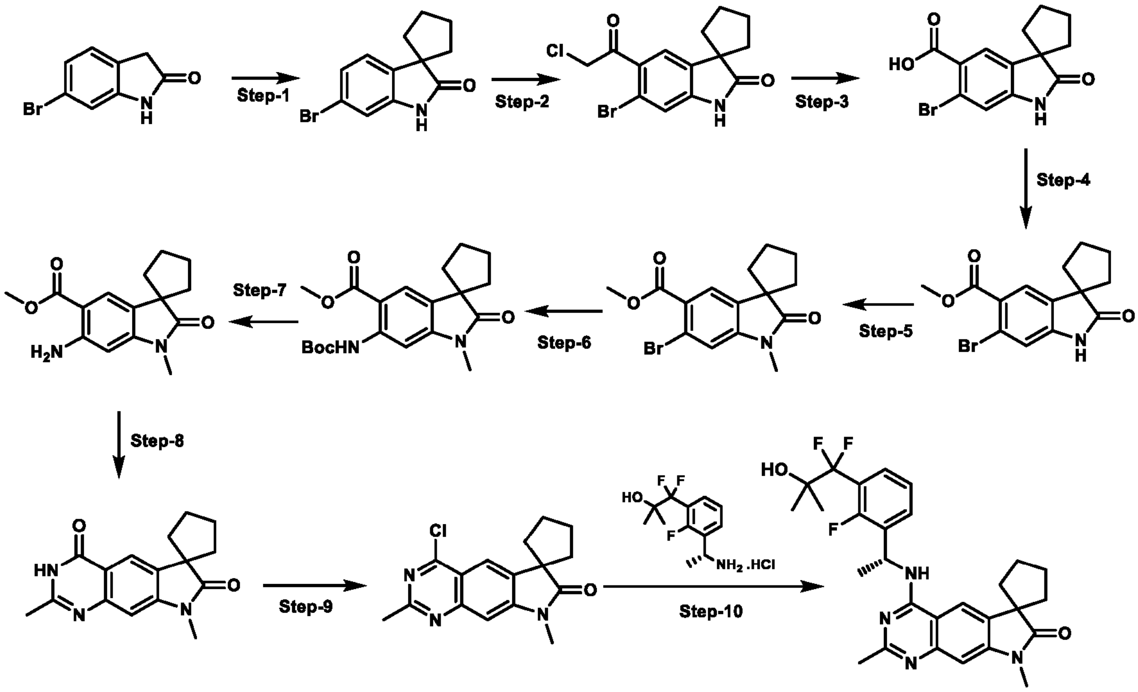


Step-1: 6'-Bromospiro[cyclopentane-1,3'-indolin]-2'-one

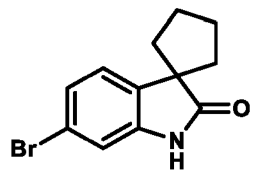

To a stirred suspension of 6-bromoindolin-2-one (30.0 g, 141 mmol) and TMEDA(42.4 mL , 283.0 mmol) in THF (500.0 mL), n-BuLi (113.0 mL , 283 mmol) was added dropwise over 30 minutes at - 78°C. The reaction was stirred for 1h, followed by addition of 1,4-dibromobutane (84.0 mL, 707 mmol) in drop wise manner over 10 min. The reaction was warmed to -20°C over the period of 1 h, further to room temperature and stirred for 3 h. The reaction was quenched with saturated ammonium chloride solution and extracted with ethyl acetate (2 x 500.0 mL). The organic layer was washed with water, brine, dried over anhydrous $Na_2SO_4$ and evaporated. The residue was purified by flash chromatography in 10% ethyl acetate-n-hexane to afford the titled compound (14.0 g, 37.2 %)

MS(ES+) m/z = 266.0 (M+1).

Step-2: 6'-Bromo-5'-(2-chloroacetyl)spiro[cyclopentane-1,3'-indolin]-2'-one

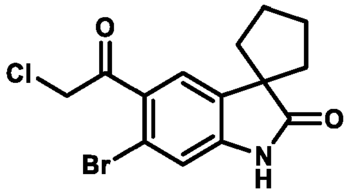

To a suspension of 6'-bromospiro[cyclopentane-1,3'-indolin]-2'-one (4.4 g, 16.53 mmol) and aluminum chloride (7.94 g, 59.5 mmol) in 1,2-dichloroethane (50.0 mL), 2-chloroacetyl chloride (2.76 mL , 34.7 mmol) was added in drop wise manner at 0°C. The resulting mixture was stirred at 0°C for 20 min and at 50°C for 17 h. Reaction mixture was cooled to room temperature and poured on ice. The solid was filtered off and washed with water. The solid was dried under vacuum to afford crude product. The crude product was purified by flash chromatography in 20% ethyl acetate -n-hexane as eluent to afford titled compound (5.0 g, 88.0 % yield).

MS(ES+) m/z = 344.22 (M+1).

Step-3: 6'-Bromo-2'-oxospiro[cyclopentane-1,3'-indoline]-5'-carboxylic acid

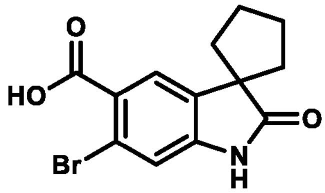

a mixture of 6'-bromo-5'-(2-chloroacetyl)spiro[cyclopentane-1,3'-indolin]-2'-one (5.0 g, 14.59 mmol) and pyridine (34.2 mL, 423 mmol) was heated at 90°C for 2.5 h. The formed precipitate was filtered off and washed with ethanol (25.0 mL). To this solid, aqueous NaOH (50.0 mL, 2.5 M) was added and the resulting mixture was heated at 80°C for 2 h to get a dark red solution. The reaction mixture was acidified using hydrochloric acid (5.0 M) to pH = 2-3 to get yellow solid. The solid obtained was filtered, washed with water (30.0 mL) and dried under vacuum to get titled compound (1.5 g, 33.1 % yield)

MS(ES+) m/z = 310.28 (M+1).

Step-4: Methyl 6'-bromo-2'-oxospiro[cyclopentane-1,3'-indoline]-5'-carboxylate

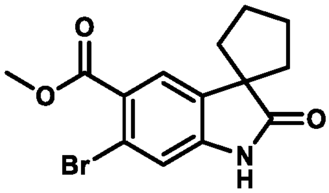

To a suspension of 6'-bromo-2'-oxospiro[cyclopentane-1,3'-indoline]-5'-carboxylic acid (1.5 g, 4.84 mmol) in MeOH (20.0 mL), $H_2SO_4$ (0.258 mL , 4.84 mmol) was added at 0°C and the reaction was stirred at 90 °C for 48 h. The mixture was cooled to room temperature, MeOH was evaporated, and residue was neutralized using aq. sodium bicarbonate solution. The precipitated solid was filtered, washed with water and dried in vacuum to afford the titled compound (1.5 g, 96 % yield)

MS(ES+) m/z = 323.90(M), 326.15 (M+2).

Step-5: Methyl 6'-bromo-1'-methyl-2'-oxospiro[cyclopentane-1,3'-indoline]-5'-carboxylate

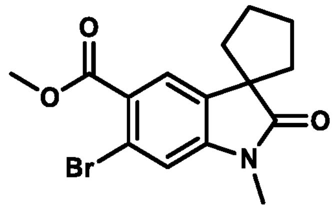

To an ice cooled solution of methyl 6'-bromo-2'-oxospiro[cyclopentane-1,3'-indoline]-5'-carboxylate (1.5 g, 4.63 mmol) in DMF (10.0 mL), $K_2CO_3$ (0.799 g, 5.78 mmol) and MeI (0.318 mL , 5.09 mmol) were added over 10 min. The reaction mixture was stirred at room temperature for 6h. The reaction was poured in ice water, the solid was filtered and dried to afford the titled compound (1.4 g, 89 % yield).

MS(ES+) m/z = 338.40 (M), 340.22 (M+2).

Step-6: Methyl 6'-((tert-butoxycarbonyl)amino)-1'-methyl-2'-oxospiro[cyclopentane-1,3'-indoline]-5'-carboxylate

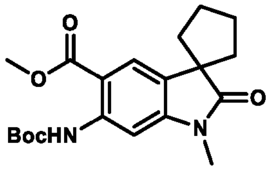

To a stirred solution of methyl 6'-bromo-1'-methyl-2'-oxospiro[cyclopentane-1,3'-indoline]-5'-carboxylate (0.90 g, 2.66 mmol) in dry 1,4-dioxane (15.0 mL), was added *tert*-butyl carbamate (0.34 g, 2.93 mmol). The reaction mixture was degassed with nitrogen for 10 min followed by addition of $Cs_2CO_3$ (1.56 g, 4.79 mmol), xantphos (0.185 g, 0.319 mmol) and $Pd_2(dba)_3$ (0.122 g, 0.133 mmol). The reaction was stirred at 120°C for 1h. The reaction was cooled to room temperature and concentrated in vacuum to give crude product. The crude product was purified by flash chromatography using ethyl acetate - n-hexane gradient to yield titled compound (0.90 g, 90 % yield).

MS(ES+) m/z = 375.3 (M+1).

Step-7: Methyl 6'-amino-1'-methyl-2'-oxospiro[cyclopentane-1,3'-indoline]-5'-carboxylate

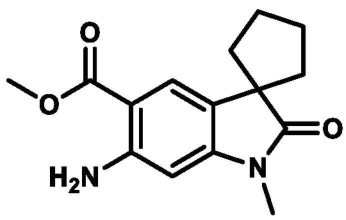

To a stirred solution of methyl 6'-((tert-butoxycarbonyl)amino)-1'-methyl-2'-oxospiro[cyclopentane-1,3'-indoline]-5'-carboxylate (1.8 g, 4.81 mmol) in 1,4-dioxane (10.0 mL), was added HCl in dioxane (4M, 8.0 mL) at 25°C and the reaction was stirred at 55°C for 2h. The reaction mixture was concentrated in vacuum to get sticky residue. The residue

was triturated with diethyl ether to afford the titled compound (1.0 g, 66.9 % yield). It was used as such for the next reaction.
MS(ES+) m/z = 275.27 (M+1).

Step-8: 2',8'-Dimethylspiro[cyclopentane-1,6'-pyrrolo[3,2-g]quinazoline]-4',7'(3'H,8'H)-dione

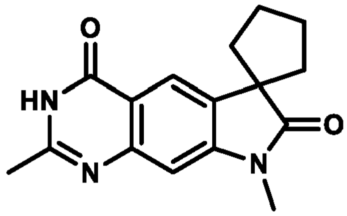

To a stirred solution of methyl 6'-amino-1'-methyl-2'-oxospiro[cyclopentane-1,3'-indoline]-5'-carboxylate (0.6 g, 2.187 mmol) in acetonitrile (15.0 mL), MSA (1.420 mL, 21.87 mmol) was added and the reaction was stirred at 100 °C for 36 h. The reaction was cooled to room temperature, solvent was evaporated, and the residue obtained was dissolved in ethyl acetate (25.0 mL). The organic layer was washed it with aq. $NaHCO_3$ (2 x 10.0 mL) and water (10.0 mL). The organic layer was separated, dried over anhydrous $Na_2SO_4$ and evaporated to afford the titled compound. (0.25 g, 40.3 % yield).
MS(ES+) m/z = 284.40 (M+1).

Step-9: 4'-Chloro-2',8'-dimethylspiro[cyclopentane-1,6'-pyrrolo[3,2-g]quinazolin]-7'(8'H)-one

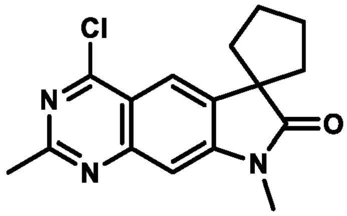

To a suspension of 2',8'-dimethylspiro[cyclopentane-1,6'-pyrrolo[3,2-g]quinazoline]-4',7'(3'H,8'H)-dione (0.030 g, 0.106 mmol) in chlorobenzene (3.0 mL), DIPEA (0.050 mL , 0.286 mmol) was added followed by drop wise addition of $POCl_3$ (0.025 mL , 0.265 mmol) at room temperature. The reaction was stirred at 90°C for the 2.5 h. The reaction was cooled to room temperature and solvent was evaporated. The residue was poured into ice cold water and extracted with ethyl acetate (2 x 10.0 mL). The organic layer was separated, washed with brine (25.0 mL), dried over $Na_2SO_4$ and concentrated under vacuum to afford the titled compound. (0.030 g, 94 % yield).
MS(ES+) m/z = 302.34 (M+1).

Step-10: (R)-4'-((1-(3-(1,1-difluoro-2-hydroxy-2-methylpropyl)-2-fluorophenyl)ethyl)amino)-2',8'-dimethylspiro[cyclopentane-1,6'-pyrrolo[3,2-g]quinazolin]-7'(8'H)-one **(Compound 58)**

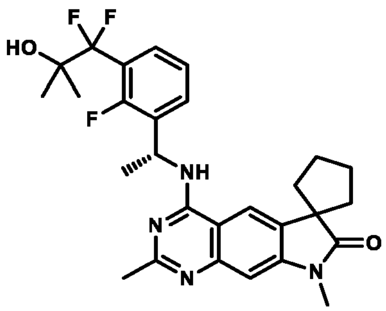

To a stirred solution of 4'-chloro-2',8'-dimethylspiro[cyclopentane-1,6'-pyrrolo[3,2-g]quinazolin]-7'(8'H)-one (0.060 g, 0.199 mmol) in 1,4-dioxane (3.0 mL), added (R)-1-(3-(1-aminoethyl)-2-fluorophenyl)-1,1-difluoro-2-methylpropan-2-ol hydrochloride (0.068 g, 0.239 mmol) and DIPEA (0.174 mL , 0.994 mmol) at room température. The reaction was stirred at 120°C for 16h. The reaction mixture was evaporated and dried under high vacuum. The crude compound was purified using preparative HPLC to afford the titled compound. (0.035 g, 34.3 % yield)

MS(ES+) m/z = 512.36 (M+1).

$^1$H NMR (400 MHz, DMSO-$d_6$) δ 8.24 (s, 1H), 8.20 (d, *J* = 7.4 Hz, 1H), 7.62 - 7.58 (m, 1H), 7.35 - 7.27 (m, 1H), 7.25 - 7.19 (m, 1H), 7.05 (s, 1H), 5.86 - 5.78 (m, 1H), 5.34 (s, 1H), 3.19 (s, 3H), 2.31 (s, 3H), 2.17 - 1.95 (m, 6H), 1.95 - 1.82 (m, 2H), 1.59 (d, *J* = 7.1 Hz, 3H), 1.24 (s, 3H), 1.21 (s, 3H).

**Examples 72-75** in **Table-3** were synthesized by following the analogous procedure as described in Example **71** using corresponding intermediate and appropriate chiral amine and commercially available chiral amine for compound 73.

**Table-3**

| Example | Chemical structure | LCMS and NMR Data |
|---|---|---|
| <u>**72**</u> | 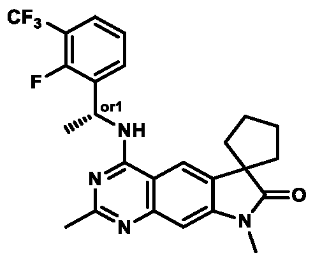 (R/S)-4'-((1-(2-fluoro-3-(trifluoromethyl)phenyl)ethyl)amino)-2', 8'-dimethylspiro[cyclopentane- 1,6'-pyrrolo[3,2-g]quinazolin]-7'(8'H)-one **(Compound 59)** | MS(ES+) m/z= 473.23 (M+1). $^1$H NMR (400 MHz, DMSO-$d_6$) δ 8.26 (d, *J* = 7.0 Hz, 1H), 8.23 (s, 1H), 7.82 - 7.79 (m, 1H), 7.66 - 7.62 (m, 1H), 7.38 - 7.34 (m, 1H), 7.06 (s, 1H), 5.82 - 5.70 (m, 1H), 3.19 (s, 3H), 2.30 (s, 3H), 2.19 - 1.98 (m, 6H), 1.98 - 1.83 (m, 2H), 1.64 (d, *J* = 7.1 Hz, 3H). |
| <u>**73**</u> | 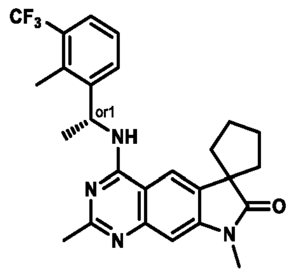 (R/S)-2',8'-dimethyl-4'-((1-(2-methyl-3-(trifluoromethyl)phenyl)ethyl)amino) spiro [cyclopentane-1,6'-pyrrolo[3,2-g]quinazolin]-7'(8'H)-one | MS(ES+) m/z = 469.23 (M+1). $^1$H NMR (400 MHz, DMSO-$d_6$) δ 8.36 - 8.30 (m, 1H), 8.23 (s, 1H), 7.82 - 7.75 (m, 1H), 7.57 - 7.50 (m, 1H), 7.41 - 7.32 (m, 1H), 7.04 (s, 1H), 5.76 - 5.69 (m, 1H), 3.19 (s, 3H), 2.64 (s, 3H), 2.32 (s, 3H), 2.13 - 1.98 (m, 6H), 1.92 - 1.85 (m, 2H), 1.57 (d, *J* = 7.0 Hz, 3H). |

(continued)

| | | |
|---|---|---|
| | **(Compound 60)** | |
| <u>74</u> | 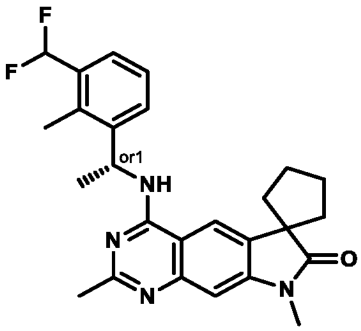<br>(R/S)-4'-((1-(3-(difluoromethyl)-2-methylphenyl)ethyl)amino)-2',8'-dimethylspiro[cyclopentane-1,6'-pyrrolo[3,2-g]quinazolin]-7'(8'H)-one **(Compound 61)** | MS(ES+) m/z = 449.93 (M+1)<br>$^1$H NMR (400 MHz, DMSO-$d_6$) δ 8.26 - 8.21 (m, 2H), 7.67 (d, *J* = 7.7 Hz, 1H), 7.41 - 7.34 (m, 1H), 7.34 - 7.26 (m, 1H), 7.22 (t, *J* = 55 Hz, 1H), (s, 1H), 7.04 (s, 1H), 5.82 - 5.73 (m, 1H), 3.19 (s, 3H), 2.56 (s, 3H), 2.33 (s, 3H), 2.14 - 1.98 (m, 6H), 1.94 - 1.83 (m, 2H), 1.55 (d, *J* = 7.0 Hz, 3H). |
| <u>75</u> | 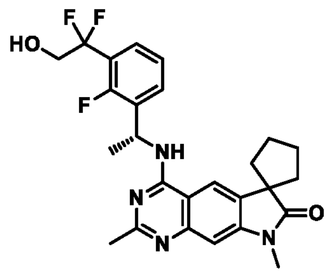<br>(R)-4'-((1-(3-(1,1-difluoro-2-hydroxyethyl)-2-fluorophenyl)ethyl)amino)-2',8'-dimethylspiro[cyclopentane-1,6'-pyrrolo[3,2-g]quinazolin]-7'(8'H)-one **(Compound 62)** | MS(ES+) m/z = 485.06 (M+1)<br>$^1$H NMR (400 MHz, DMSO-$d_6$) δ 8.24 (s, 1H), 8.20 (d, *J* = 7.3 Hz, 1H), 7.68 - 7.62 (m, 1H), 7.45 - 7.38 (m, 1H), 7.30 - 7.22 (m, 1H), 7.06 (s, 1H), 5.86 - 5.79 (m, 1H), 5.73 (t, *J* = 6.4 Hz, 1H), 4.03 - 3.87 (m, 2H), 3.20 (s, 3H), 2.33 (s, 3H), 2.16 - 1.96 (m, 6H), 1.94 - 1.82 (m, 2H), 1.61 (d, *J* = 7.1 Hz, 3H). |

**Example 76:** Preparation of (R)-4-((1-(3-(1,1-difluoro-2-hydroxy-2-methylpropyl)-2-fluorophenyl) ethyl)amino)-6-(2-methoxyethyl)-2,8,8-trimethyl-6,8-dihydro-7H-pyrrolo[2,3-g]quinazolin-7-one

**(Compound 63)**

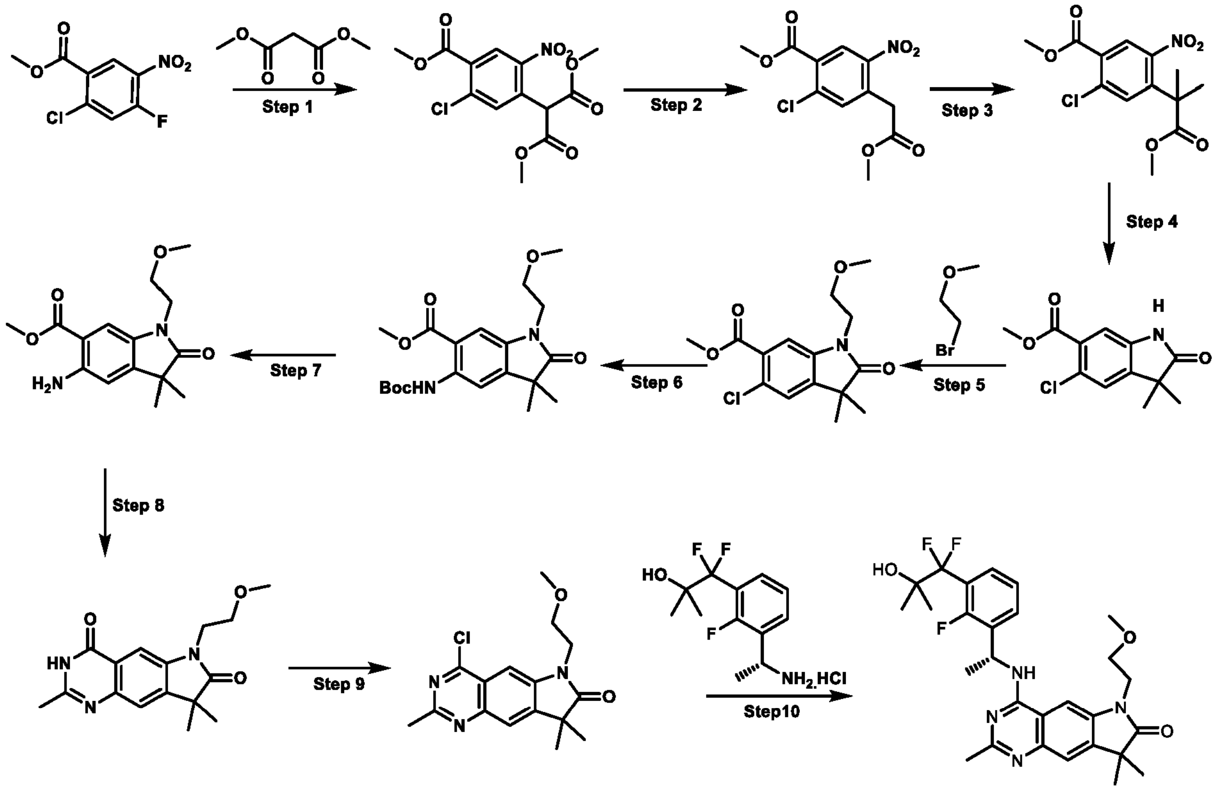


Step 1: Dimethyl 2-(5-chloro-4-(methoxycarbonyl)-2-nitrophenyl)malonate

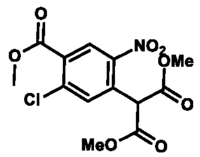

To a cooled (0 °C) solution of dimethyl malonate (12.17 mL , 106.0 mmol) in DMF (165.0 mL) were added methyl 2-chloro-4-fluoro-5-nitrobenzoate (16.5 g, 70.6 mmol) and $K_2CO_3$ (29.3 g, 212.0 mmol). The reaction mixture was stirred overnight at room temperature. The reaction was poured in ice cold 2 M aqueous HCl and extracted with ethyl acetate (2 x 200.0 mL), combined organic layer was washed with water (200.0 mL), brine (150.0 mL), and dried over anhydrous $Na_2SO_4$. Removal of solvent under reduced pressure and the crude material obtained was purified by flash chromatography in 10% ethyl acetate - n-hexane to afford titled compound (18.2 g, 74.5 % yield).

MS(ES+) m/z = 346.14 (M+1).

Step 2: Methyl 2-chloro-4-(2-methoxy-2-oxoethyl)-5-nitrobenzoate

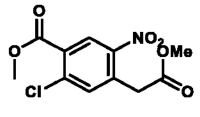

A solution of dimethyl 2-(5-chloro-4-(methoxycarbonyl)-2-nitrophenyl)malonate (10.0 g, 28.9 mmol), LiCl (2.453 g, 57.9 mmol) in DMSO (100.0 mL) and water (1.042 mL , 57.9 mmol) was heated at 90 $^0$C for 5 h. the reaction was cooled to room temperature and poured on ice water (200.0 mL). The aqueous layer was extracted with ethyl acetate (3 x 100.0 mL). The combined organic layers were washed with brine (100.0 mL), dried over anhydrous $Na_2SO_4$, and concentrated in vacuum. The crude product was purified by flash chromatography in ethyl acetate- n- hexane gradient to afford the titled

compound (5.6 g, 67.3 %).

$^1H$ NMR (400 MHz, $CDCl_3$) δ 8.68 (s, 1H), 7.51 (s, 1H), 4.07 (s, 2H), 4.01 (s, 3H), 3.75 (s, 3H).

Step 3: Methyl 2-chloro-4-(1-methoxy-2-methyl-1-oxopropan-2-yl)-5-nitrobenzoate

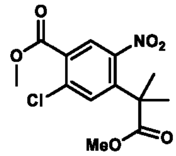

To a stirred solution of methyl 2-chloro-4-(2-methoxy-2-oxoethyl)-5-nitrobenzoate (1.0 g, 3.48 mmol) and methyl iodide (0.543 mL , 8.69 mmol) in DMF (10.0 mL), was added sodium hydride (0.417 g, 10.43 mmol) over 30 min at 0 °C. The reaction was stirred at room temperature for 16 h. The reaction was quenched with Aq. ammonium chloride, extracted with ethyl acetate (2 x 20.0 mL). The organic layer was separated, dried over anhydrous $Na_2SO_4$ and concentrated to give oily mass. The crude product purified by flash chromatography in 30% ethyl acetate- n-hexane to afford the titled compound (0.8 g, 72.9 %).

$^1H$ NMR (400 MHz, $CDCl_3$) δ 8.50 (s, 1H), 7.69 (s, 1H), 3.99 (s, 3H), 3.68 (s, 3H), 1.70 (s, 6H).

Step 4: Methyl 5-chloro-3,3-dimethyl-2-oxoindoline-6-carboxylate

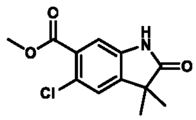

To a stirred solution of methyl 2-chloro-4-(1-methoxy-2-methyl-1-oxopropan-2-yl)-5-nitrobenzoate (0.8 g, 2.53 mmol) in acetic acid : ethanol (1:1, 20.0 mL), iron (0.388 g, 6.95 mmol) was added and resulting mixture was stirred at 100°C for 1h. The reaction was diluted with water (20.0 mL) and ethyl acetate (50.0 mL). The organic layer was separated, dried over anhydrous $Na_2SO_4$ and concentrated in vacuum to give crude product. The crude product was purified by flash chromatography in MeOH-DCM to afford the titled compound (0.6g, 93 %).

MS(ES+) m/z = 254.27 (M+1).

Step 5: Methyl 5-chloro-1-(2-methoxyethyl)-3,3-dimethyl-2-oxoindoline-6-carboxylate

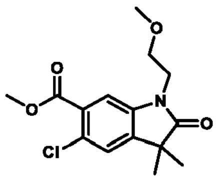

To a stirred solution of methyl 5-chloro-3,3-dimethyl-2-oxoindoline-6-carboxylate (Step-4, 2 g, 7.88 mmol) in acetonitrile (30.0 mL), $K_2CO_3$, (1.63g, 11.83 mmol) was added at 0°C followed by addition of 1-bromo-2-methoxyethane (1.315 g, 9.46 mmol) in drop wise manner at same temperature. Reaction was heated and stirred at 90 °C for 48 h. The reaction was quenched with Aq. ammonium chloride and extracted with ethyl acetate (2 x 20.0 mL). The organic layer was separated, dried over anhydrous $Na_2SO_4$ and evaporated. Crude mass obtained was purified by flash chromatography in ethyl acetate-n- hexane eluent to afford the titled compound (1.6 g, 65.1 % yield)

MS(ES+) m/z = 311.97 (M+1).

Step 6: Methyl 5-((tert-butoxycarbonyl)amino)-1-(2-methoxyethyl)-3,3-dimethyl-2-oxoindoline-6-carboxylate

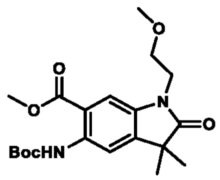

The titled compound was prepared from **Step-5** intermediate by employing analogous protocol mentioned in **Example 71-Step-6** (0.3 g, 95 % yield).

MS(ES+) m/z = 393.22 (M+1).

Step 7: Methyl-5-amino-1-(2-methoxyethyl)-3,3-dimethyl-2-oxoindoline-6-carboxylate

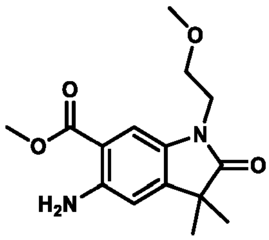

The titled compound was prepared from Step-6 intermediate by employing analogous protocol mentioned in **Example 71-Step-7** (0.22 g, 88 % yield).

MS(ES+) m/z = 293.15 (M+1)

Step 8: 6-(2-Methoxyethyl)-2,8,8-trimethyl-6,8-dihydro-3H-pyrrolo[2,3-g]quinazoline-4,7-dione

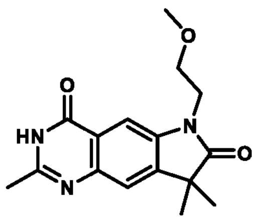

The titled compound was prepared from **Step-7** intermediate by employing analogous protocol mentioned in **Example 71-Step-8** (0.2 g, 87 %).

MS(ES+) m/z = 302.34 (M+1).

Step 9: 4-Chloro-6-(2-methoxyethyl)-2,8,8-trimethyl-6,8-dihydro-7H-pyrrolo[2,3-g]quinazolin-7-one

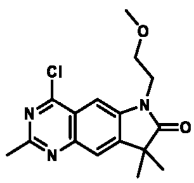

The titled compound was prepared from **Step-8** intermediate by employing analogous protocol mentioned in **Example 71-Step-9** (0.1 g, 47.1 % yield)

MS(ES+) m/z = 319.96 (M+1).

Step 10: (R)-4-((1-(3-(1,1-difluoro-2-hydroxy-2-methylpropyl)-2-fluorophenyl)ethyl)amino)-6-(2-methoxyethyl)-2,8,8-trimethyl-6,8-dihydro-7H-pyrrolo[2,3-g]quinazolin-7-one **(Compound 63)**

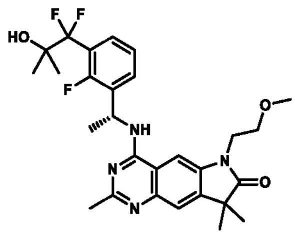

The titled compound was prepared by reaction of **Step-9** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71- Step-10** (0.020 g, 17.22 %).

MS(ES+) m/z = 531.57 (M+1).

$^1$H NMR (400 MHz, DMSO-$d_6$) δ 8.19 (d, *J* = 7.4 Hz, 1H), 7.93 (s, 1H), 7.62 - 7.56 (m, 2H), 7.33 - 7.28 (m, 1H), 7.25 - 7.20 (m, 1H), 5.86 - 5.79 (m, 1H), 5.34 (s, 1H), 3.97 (t, *J* = 6.2 Hz, 2H), 3.69 (t, *J*= 6.0 Hz, 2H), 3.29 (s, 3H), 2.32 (s, 3H), 1.61 (d, *J* = 7.1 Hz, 3H), 1.34 (s, 3H), 1.33 (s, 3H), 1.24 (s, 3H),1.21 (s, 3H). **Example 77** : Preparation of (R)-4-((1-(3-(1,1-difluoro-2-hydroxy-2-methylpropyl)-2-fluorophenyl)ethyl)amino)-6-ethyl-2,8,8-trimethyl-6,8-dihydro-7H-pyrrolo[2,3-g]quinazolin-7-one

**(Compound 64)**

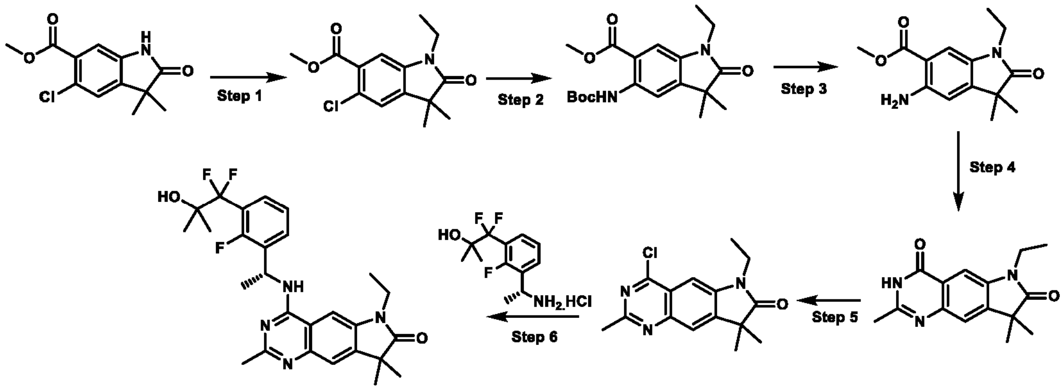


Step 1: Methyl 5-chloro-1-ethyl-3,3-dimethyl-2-oxoindoline-6-carboxylate

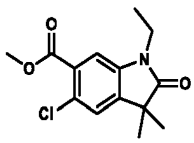

The titled compound was prepared from **Example 76 Step-4** intermediate by employing analogous protocol mentioned in **Example 76-Step-5** treated with Ethyl bromide to afford the titled compound. (0.40 g, 90 % yield)

MS(ES+) m/z = 282.34 (M+1).

**Step 2:** Methyl 5-((tert-butoxycarbonyl)amino)-1-ethyl-3,3-dimethyl-2-oxoindoline-6-carboxylate

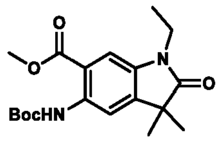

The titled compound was prepared from **Step-1** intermediate by employing analogous protocol mentioned in **Example 71 Step-6** (0.4 g, 78 % yield).

MS(ES+) m/z = 363.41 (M+1).

**Step 3:** Methyl 5-amino-1-ethyl-3,3-dimethyl-2-oxoindoline-6-carboxylate

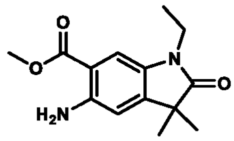

The titled compound was prepared from **Step-2** intermediate by employing analogous protocol mentioned in **Example 71 Step-7** (0.65 g, 99 % yield).

MS(ES+) m/z = 263.15 (M+1).

**Step 4:** 6-Ethyl-2,8,8-trimethyl-6,8-dihydro-3H-pyrrolo[2,3-g]quinazoline-4,7-dione

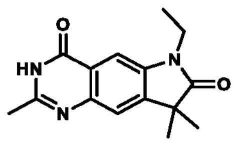

The titled compound was prepared from **Step-3** intermediate by employing analogous protocol mentioned in **Example 71-Step-8** (0.45 g, 83 % yield)

MS(ES+) 272.27 (M+).

**Step 5:** 4-Chloro-6-ethyl-2,8,8-trimethyl-6,8-dihydro-7H-pyrrolo[2,3-g]quinazolin-7-one

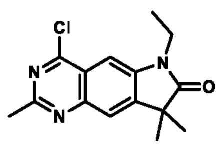

The titled compound was prepared from **Step-4** intermediate by employing analogous protocol mentioned in **Example 71 Step-9.** It was used as such for next Step

MS(ES+) m/z =289.15 (M+).

Step 6: (R)-4-((1-(3-(1,1-difluoro-2-hydroxy-2-methylpropyl)-2-fluorophenyl)ethyl)amino)-6-ethyl-2,8,8-trimethyl-6,8-dihydro-7H-pyrrolo[2,3-g]quinazolin-7-one **(Compound 64)**

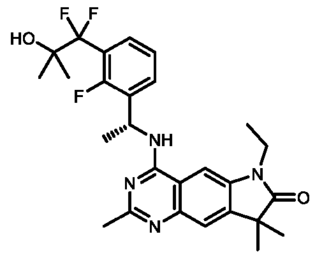

The titled compound was prepared by reaction of **Step-5** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71 Step-10** (0.030 g, 17.37% yield)

MS(ES+) m/z = 501.36 (M+1).

$^1$H NMR (400 MHz, DMSO- $d_6$) δ 8.21 (d, J = 7.4 Hz, 1H), 7.91 (s, 1H), 7.64 - 7.56 (m, 2H), 7.34 - 7.28 (m, 1H), 7.25 - 7.17 (m, 1H), 5.87 - 5.78 (m, 1H), 5.34 (s, 1H), 3.83 (q, *J* = 7.1 Hz, 2H), 2.31 (s, 3H), 1.62 (d, *J* = 7.0 Hz, 3H), 1.34 (s, 3H), 1.33 (s, 3H), 1.29 (t, *J* = 7.1 Hz, 3H), 1.24 (s, 1H), 1.21 (s, 3H)

**Example 78** : Preparation of (R)-6-cyclopropyl-4-((1-(3-(1,1-difluoro-2-hydroxy-2-methylpropyl)-2-fluoro phenyl)ethyl)amino)-2,8,8-trimethyl-6,8-dihydro-7H-pyrrolo[2,3-g]quinazolin-7-one

**(Compound 65)**

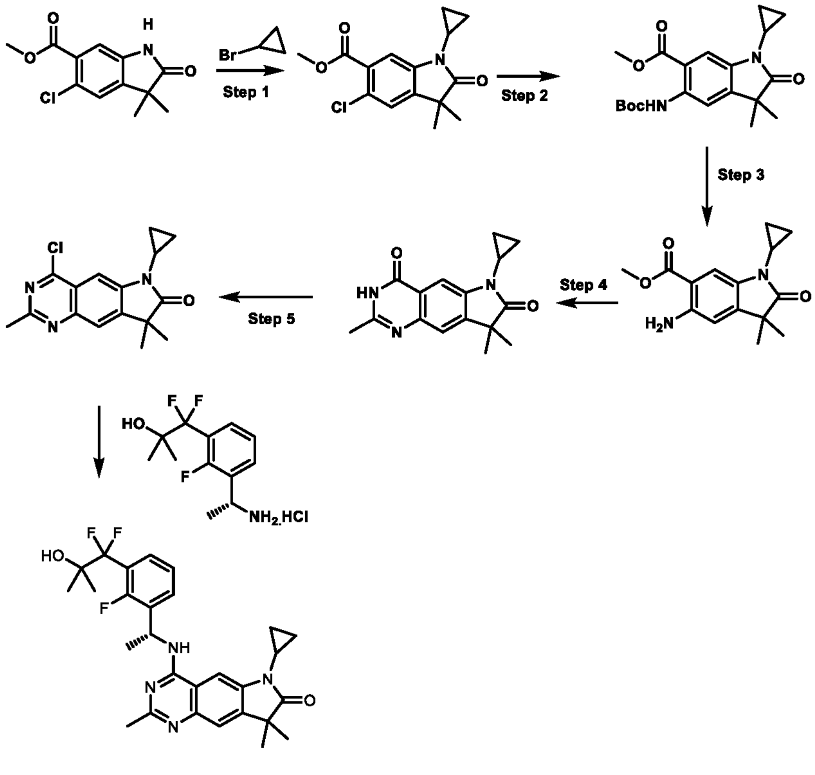


Step 1. Methyl 5-chloro-1-cyclopropyl-3,3-dimethyl-2-oxoindoline-6-carboxylate

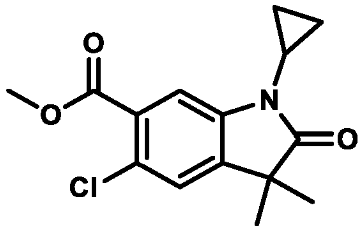

The titled compound was prepared from **Example 76 Step-4** intermediate by employing analogous protocol mentioned in **Example 76-Step-5** treated with cyclopropyl bromide (0.15 g, 59 % yield).

MS(ES+) m/z = 294.15 (M+1).

Step 2. Methyl 5-((tert-butoxycarbonyl)amino)-1-cyclopropyl-3,3-dimethyl-2-oxoindoline-6-carboxylate

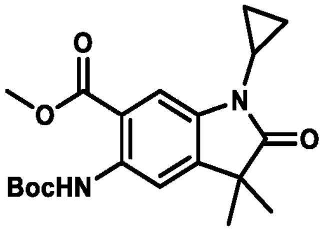

The titled compound was prepared from **Step 1** intermediate by employing analogous protocol mentioned in **Example 71 Step-6** (0.40 g, 44.8 % yield).

MS(ES+) m/z = 375.28 (M+1).

Step 3. Methyl 5-amino-1-cyclopropyl-3,3-dimethyl-2-oxoindoline-6-carboxylate

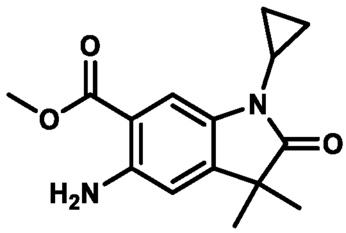

The titled compound was prepared from **Step-2** intermediate by employing analogous protocol mentioned in **Example 71 Step-**7 (0.30 g, 90 % yield).

MS(ES+) m/z =275.08 (M+1)

Step 4. 6-Cyclopropyl-2,8,8-trimethyl-6,8-dihydro-3H-pyrrolo[2,3-g]quinazoline-4,7-dione

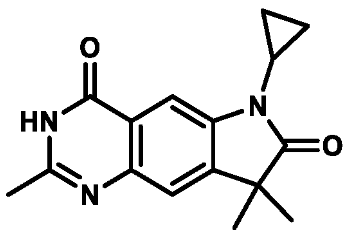

The titled compound was prepared from Step-3 intermediate by employing analogous protocol mentioned in **Example 71-Step-8** (0.25 g, 91 % yield).
MS(ES+) m/z = 284.27

Step 5. 4-Chloro-6-cyclopropyl-2,8,8-trimethyl-6,8-dihydro-7H-pyrrolo[2,3-g]quinazolin-7-one

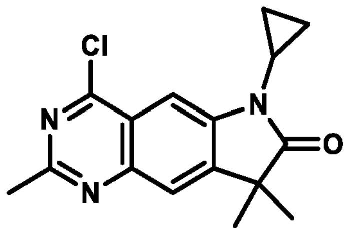

The titled compound was prepared from **Step-4** intermediate by employing analogous protocol mentioned in **Example 71 Step-9** (0.24 g, 90% yield)
MS(ES+) m/z = 302.28

Step 6: (R)-6-cyclopropyl-4-((1-(3-(1,1-difluoro-2-hydroxy-2-methylpropyl)-2-fluorophenyl)ethyl)amino)-2,8,8-trimethyl-6,8-dihydro-7H-pyrrolo[2,3-g]quinazolin-7-one

**(Compound 65)**

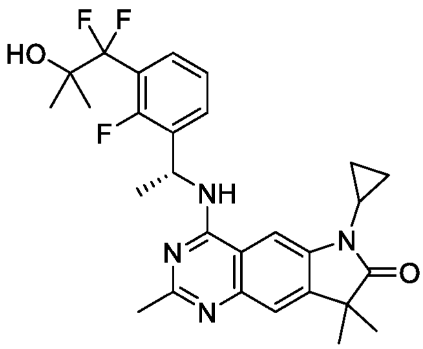

The titled compound was prepared from by reaction of **Step 5** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71 Step-10**
MS(ES+) m/z = 512.43 (M+).
$^1$H NMR (400 MHz, DMSO-$d_6$) d 8.33 (d, $J$= 7.3 Hz, 1H), 7.93 (s, 1H), 7.65 - 7.58 (m, 1H), 7.57 (s, 1H), 7.35 - 7.27 (m, 1H), 7.25 - 7.17 (m, 1H), 5.87 - 5.60 (m, 1H), 5.34 (s, 1H), 2.86 - 2.78 (m, 1H), 2.31 (s, 3H), 1.62 (d, $J$= 7.1 Hz, 3H), 1.31 (s, 3H), 1.30 (s, 3H), 1.24 (s, 3H), 1.22 (s, 3H), 1.17 - 1.09 (m, 2H), 0.98 - 0.84 (m, 2H).

**Example 79:** Preparation of (R)-4'-((1-(3-(1,1-difluoro-2-hydroxyethyl)-2-fluorophenyl)ethyl)amino)-2',8'-dimethylspiro [cyclopropane-1,6'-pyrrolo[3,2-g]quinazolin]-7'(8'H)-one **(Compound 66)**

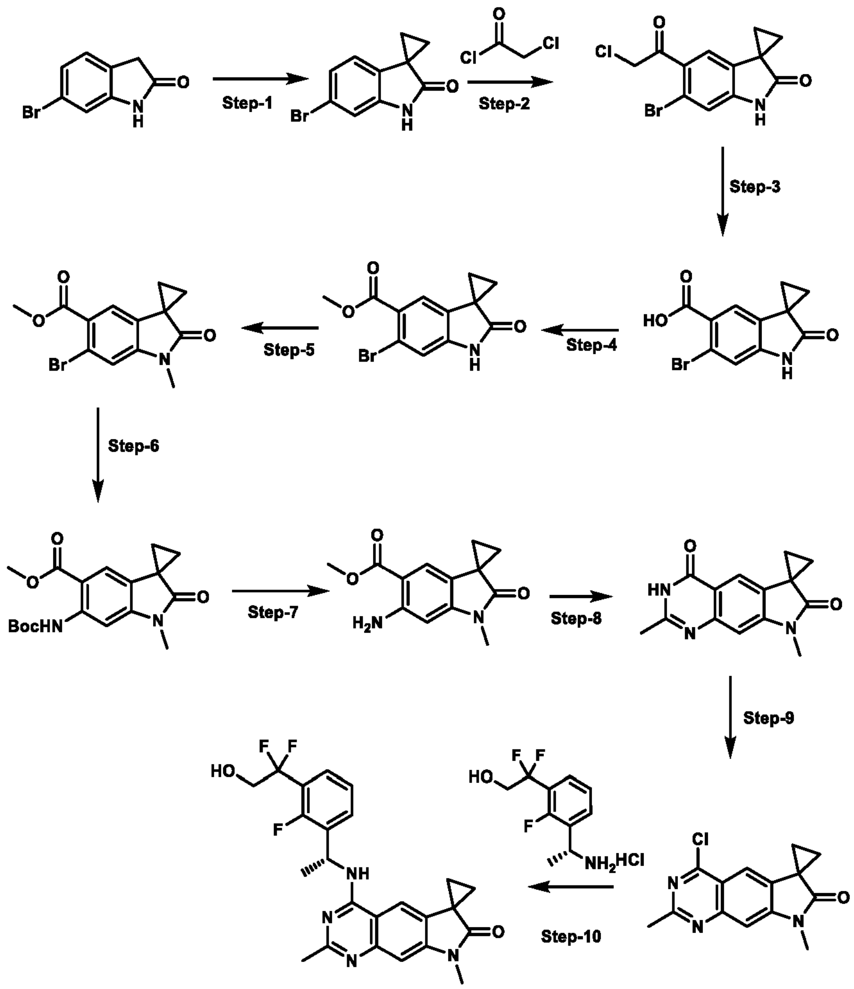


Step 1: 6'-Bromospiro[cyclopropane-1,3'-indolin]-2'-one

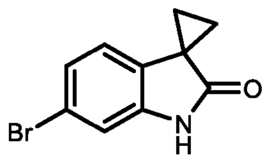

The titled compound was prepared from commercially available 6-bromoindolin-2-one and 1,2-dibromoethane by employing analogous protocol mentioned in **Example 71-step-1** (2.0 g, 35.6%).

1H NMR (400 MHz, DMSO-d6) δ 10.68 (s, 1H), 7.17 - 7.07 (m, 1H), 7.03 (d, J = 1.8 Hz, 1H), 6.94 (d, J = 7.9 Hz, 1H), 1.61 - 1.57 (m, 2H),, 1.54 - 1.44 (m, 2H).

Step 2: 6'-Bromo-5'-(2-chloroacetyl)spiro[cyclopropane-1,3'-indolin]-2'-one

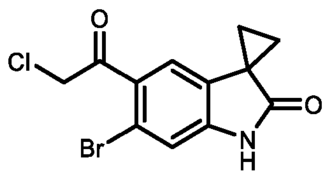

The titled compound was prepared from **Step-1** intermediate by employing analogous protocol mentioned in **Example 71 Step-2** (11.2 g, 85 % yield).

1H NMR (400 MHz, DMSO-d6) δ 10.99 (s, 1H), 7.54 (s, 1H), 7.19 (s, 1H), 5.01 (s, 2H), 1.73 - 1.66 (m, 2H), 1.67 - 1.48 (m, 2H).

Step 3: 6'-Bromo-2'-oxospiro[cyclopropane-1,3'-indoline]-5'-carboxylic acid

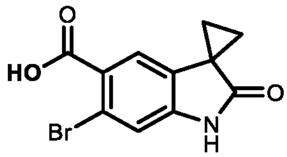

The titled compound was prepared from **Step 2** intermediate by employing analogous protocol mentioned in **Example 71 Step-3** (7.5 g, 76 % yield).

MS(ES+) m/z = 282.21, 284.09 (M, M+2).

Step 4: Methyl 6'-bromo-2'-oxospiro[cyclopropane-1,3'-indoline]-5'-carboxylate

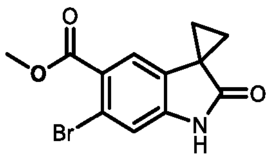

The titled compound was prepared from **Step-3** intermediate by employing analogous protocol mentioned in **Example 71 Step-4** (7.4 g, 97 %).

MS(ES+) m/z = 296.21(M+), 298.21 (M+2).

Step 5: Methyl 6'-bromo-1'-methyl-2'-oxospiro[cyclopropane-1,3'-indoline]-5'-carboxylate

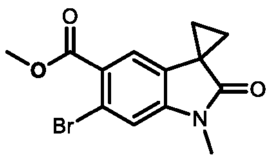

The titled compound was prepared from **Step-4** intermediate by employing analogous protocol mentioned in **Example 71 Step-5** (1.9 g, 91 %).

MS(ES+) m/z = 310.1 (M)

Step 6: 6'-((Tert-butoxycarbonyl)amino)-1'-methyl-2'-oxospiro[cyclopropane-1,3'-indoline]-5'-carboxylate

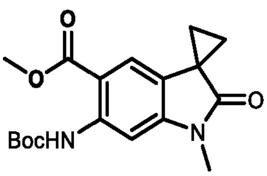

The titled compound was prepared from **Step-5** intermediate by employing analogous protocol mentioned in **Example 71 Step-6** (1.95 g, Yield: 83%)

MS(ES+) m/z = 347.51(M+1)/ 247.51 (M-100)

Step 7: methyl 6'-amino-1'-methyl-2'-oxospiro[cyclopropane-1,3'-indoline]-5'-carboxylate

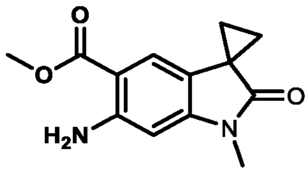

The titled compound was prepared from **Step-6** intermediate by employing analogous protocol mentioned in **Example 71 Step-7** (1.5 g Yield: 97%)

MS(ES+) m/z = 247.2(M+1)

Step 8: 2',8'-Dimethylspiro[cyclopropane-1,6'-pyrrolo[3,2-g]quinazoline]-4',7'(3'H,8'H)-dione

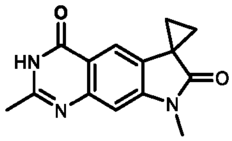

The titled compound was prepared from **Step-7** intermediate by employing analogous protocol mentioned in **Example 71 Step-8** (1.1g, 87% yield)

MS(ES+) m/z = 256.27(M+1).

Step 9: 4'-Chloro-2',8'-dimethylspiro[cyclopropane-1,6'-pyrrolo[3,2-g]quinazolin]-7'(8'H)-one

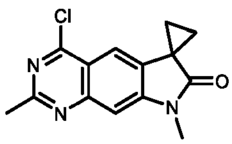

The titled compound was prepared from **Step-8** intermediate by employing analogous protocol mentioned in **Example 71 Step-9** (0.065 g, Yield: 76%)

MS(ES+) m/z = 274.21 (M+1).

Step-10: (R)-4'-((1-(3-(1,1-difluoro-2-hydroxyethyl)-2-fluorophenyl)ethyl)amino)-2',8'-dimethylspiro [cyclopropane-1,6'-pyrrolo[3,2-g]quinazolin]-7'(8'H)-one **(Compound 66)**

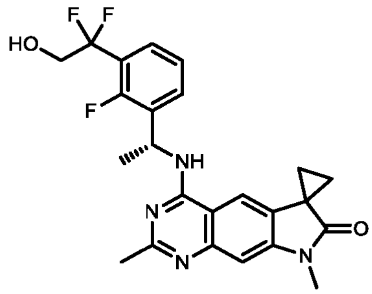

The titled compound was prepared from **Step-9** intermediate by employing analogous protocol mentioned in **Example 71 Step-10** (0.025 g, 25% yield).

MS(ES+) m/z = 457.17(M+1).

$^1$H NMR (400 MHz, DMSO-$d_6$) d 8.06 (d, *J* = 7.4 Hz, 1H), 8.00 (s, 1H), 7.68 - 7.61 (m, 1H), 7.45 - 7.37 (m, 1H), 7.28 - 7.21 (m, 1H), 7.16 (s, 1H), 5.84 - 5.76 (m, 1H), 5.73 (s, 1H), 3.94 (t, *J* =14.3 Hz, 2H), 3.28 (s, 3H), 2.34 (s, 3H), 1.73 - 1.64 (m, 4H), 1.58 (d, *J*= 7.1 Hz, 3H).

**Example 80** in **Table-4** were synthesized by following the analogous procedure as described in Example **71-Step-10** using corresponding intermediate and appropriate chiral amines.

**Table-4**

| Example | Chemical structure | LCMS and $^1$HNMR data |
|---|---|---|
| 80 | 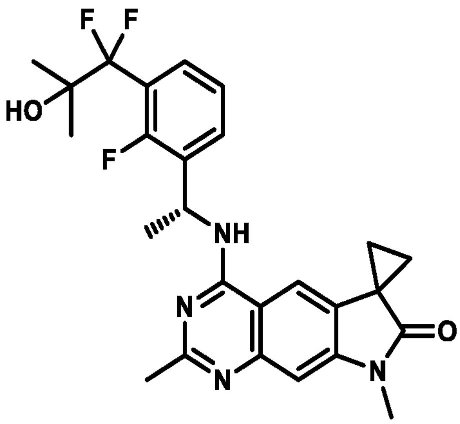 (R)-4'-((1-(3-(1,1-difluoro-2-hydroxy-2-methylpropyl)-2-fluorophenyl)ethyl)amino)-2',8'-dimethylspiro[cyclopropane-1,6'-pyrrolo[3,2-g]quinazolin]-7'(8'H)-one **(Compound 67)** | MS(ES+) m/z = 485.1 (M+1) $^1$H NMR (400 MHz, DMSO-$d_6$) δ 8.06 (d, *J* = 7.4 Hz, 1H), 8.01 (s, 1H), 7.63 - 7.55 (m, 1H), 7.34 - 7.27 (m, 1H), 7.24 - 7.18 (m, 1H), 7.16 (s, 1H), 5.83 - 5.75 (m, 1H), 5.33 (s, 1H), 3.28 (s, 3H), 2.32 (s, 3H), 1.72 - 1.66 (m, 4H), 1.57 (d, *J* = 7.0 Hz, 3H), 1.24 (s, 3H), 1.21 (s, 3H). |

**Example 81** : Preparation of (R)-4-((1-(3-(1,1-Difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one **(Compound 68)**

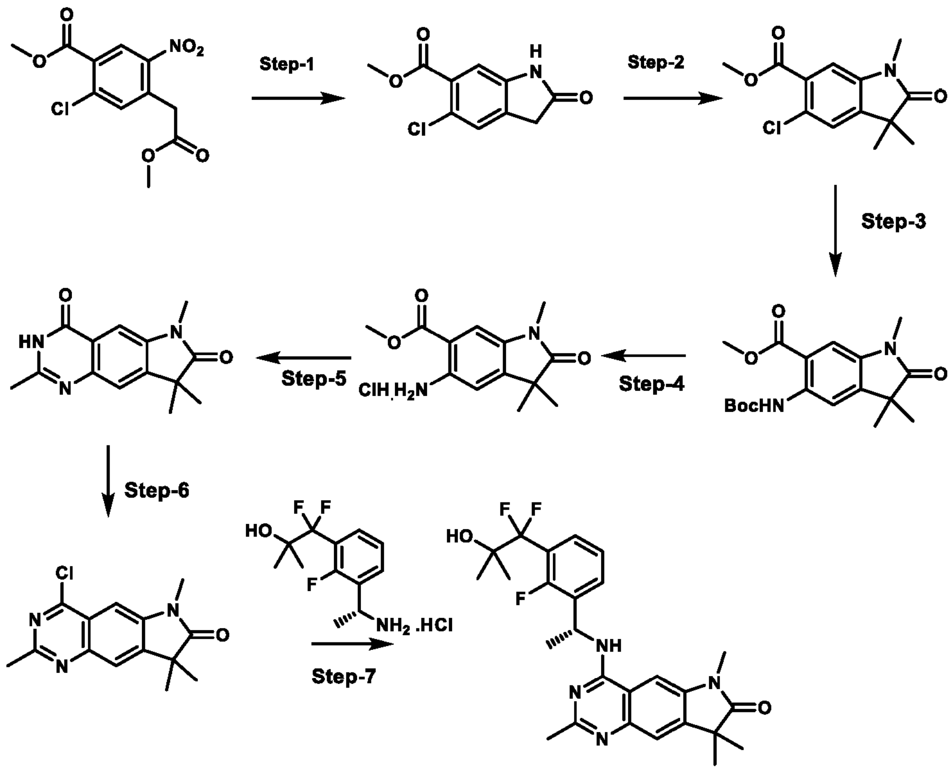


Step 1: Methyl 5-chloro-2-oxoindoline-6-carboxylate

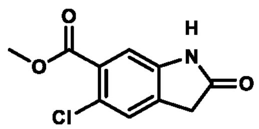

To a stirred solution of methyl 2-chloro-4-(2-methoxy-2-oxoethyl)-5-nitrobenzoate ( **Example 76-Step-2,** 5.6 g, 19.47 mmol) in ethanol-acetic acid (60.0 mL , ratio 1: 1), iron (2.19 g, 38.9 mmol) was added at 25 °C and the reaction was stirred at 100°C for 2 h. The reaction was cooled to room temperature, solvent was removed under vacuum and the residue was neutralized with aq. $NaHCO_3$ (30.0 mL). ethyl acetate (60.0 mL) was added and resulting mixture was filtered through celite bed. Separated aqueous layer from filtrate was extracted with ethyl acetate (50.0 mL). The combined organic layers were washed with brine (100.0 mL), dried over anhydrous $Na_2SO_4$ and concentrated in vacuum to give crude product. The crude product was purified by flash chromatography using 0-50% ethyl acetate - n-hexane as eluent to yield the titled compound (1.2 g, 27.3 % yield) as a white solid.

MS(ES+) m/z = 225.19 (M+), 227.14(M+2).

Step 2: Methyl 5-chloro-1,3,3-trimethyl-2-oxoindoline-6-carboxylate

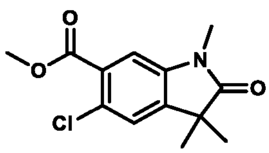

To a stirred solution of methyl 5-chloro-2-oxoindoline-6-carboxylate (1.2 g, 5.32 mmol) in DMF (20.0 mL), methyl iodide (0.998 mL, 15.96 mmol) was added. The reaction was cooled to -10°C and portion wise NaH (0.64 g, 15.96 mmol) was added. The reaction was stirred at -10°C for 1 h. The reaction was quenched with aq. ammonium chloride solution

(20.0 mL) extracted with ethyl acetate (3 x 30.0 mL). The combined organic layers were washed with water (30.0 mL), brine (30.0 mL), dried over anhydrous $Na_2SO_4$, and concentrated in vacuum to give crude product. The crude product was purified by flash chromatography using 0-20% ethyl acetate - n-hexane as eluent to yield titled compound (1.2 g, 84 % yield).

MS(ES+) m/z = 268.40 (M+1).

Step 3: Methyl 5-((tert-butoxycarbonyl)amino)-1,3,3-trimethyl-2-oxoindoline-6-carboxylate

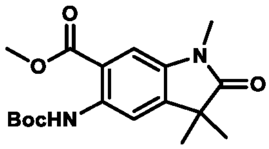

To a solution of methyl 5-chloro-1,3,3-trimethyl-2-oxoindoline-6-carboxylate (1.2 g, 4.48 mmol) in dry 1,4-dioxane (15.0 mL), were added tert-butyl carbamate (0.683 g, 5.83 mmol) and $Cs_2CO_3$ (2.63 g, 8.07 mmol). The suspension was degassed with nitrogen for 10 min. Xantphos (0.311 g, 0.538 mmol) and $Pd_2(dba)_3$ (0.205 g, 0.224 mmol) were added and resulting reaction mixture was heated at 120 °C for 16 h. The reaction was cooled to room temperature and solvent was removed under reduced pressure. The crude product was purified by flash chromatography using ethyl acetate - hexane gradient to afford titled compound (1.1 g, 70.4 % yield). MS(ES+) m/z = 349.2 (M+1).

Step 4: Methyl 5-amino-1,3,3-trimethyl-2-oxoindoline-6-carboxylate hydrochloride

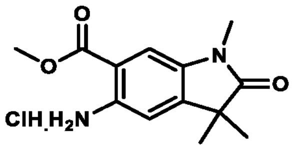

To a solution of methyl 5-((tert-butoxycarbonyl)amino)-1,3,3-trimethyl-2-oxoindoline-6-carboxylate (1.1 g, 3.16 mmol) in 1,4-dioxane (10.0 mL), was added HCl (4M in 1,4-dioxane, 8.0 mL) at 0°C and the reaction was warmed to 70 °C for 2 h. The reaction mixture was concentrated in vacuum to get sticky residue. The residue was triturated with diethyl ether to afford the titled compound (0.85 g, 95 % yield). The crude material was used as such for the next reaction.

MS(ES+) m/z = 249.27 (free amine)

Step-5: 2,6,8,8-Tetramethyl-6,8-dihydro-3H-pyrrolo[2,3-g]quinazoline-4,7-dione

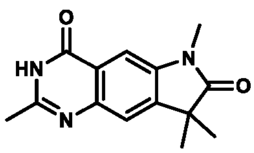

To a solution of methyl 5-amino-1,3,3-trimethyl-2-oxoindoline-6-carboxylate hydrochloride (0.45 g, 1.580 mmol) in acetonitrile (10.0 mL), methanesulfonic acid (1.026 ml, 15.80 mmol) was added and the resulting reaction mixture was stirred at 100 °C for 16 h. Solvent was evaporated under vacuo and obtained residue was dissolved in ethyl acetate ( 25.0 mL). Organic layer was washed with aq. Sodium bicarbonate (2 x 10.0 mL) and water (10.0 mL). The separated organic layer was dried over anhydrous $Na_2SO_4$, and concentrated under reduced pressure to afford titled compound (0.4 g, 198 % yield). Crude material was used as such for the next step.

MS(ES+) m/z = 258.1 (M+1).

**Example 81** - **step 5** intermediate could also be synthesized by following pathways

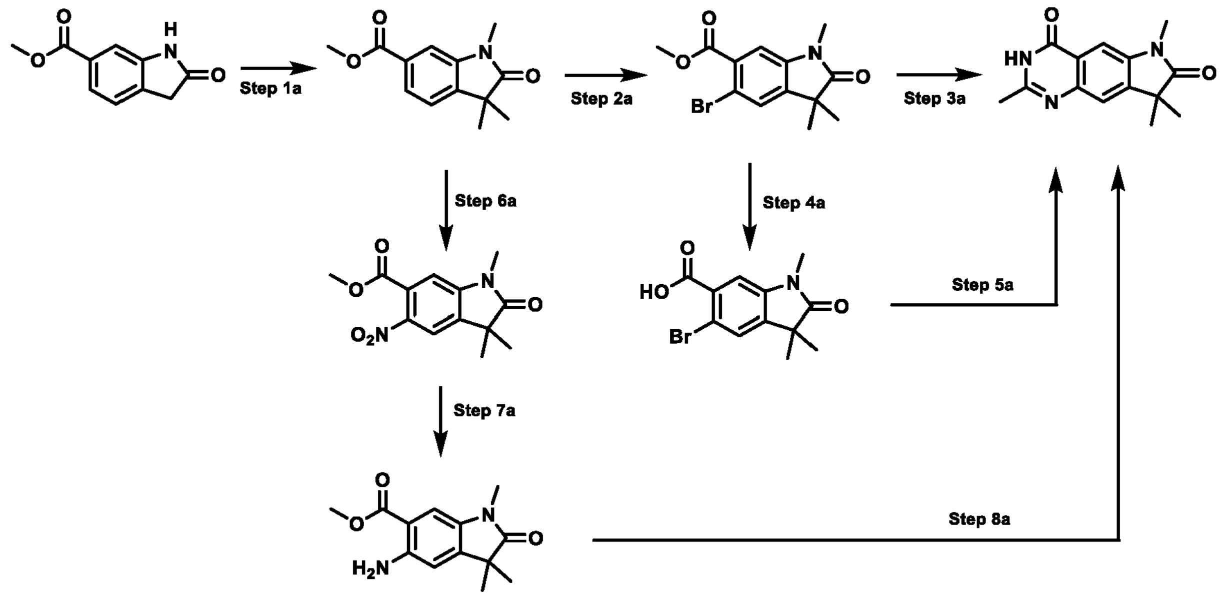


Step 1a: Methyl 1,3,3-trimethyl-2-oxoindoline-6-carboxylate

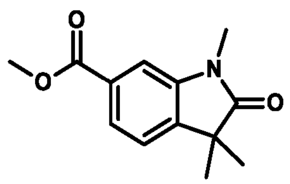

To a stirred solution of methyl 2-oxoindoline-6-carboxylate (10.0 g, 52.3 mmol) in DMF 100.0 mL was added methyl iodide (11.45 mL , 183 mmol) followed by portion wise addition of sodium hydride (7.99 g, 183 mmol) at -10 °C. The reaction mixture was stirred at -10 °C for 1 h. The reaction mixture was quenched with water (250.0 mL), precipitate obtained was filtered and washed with water to afford the titled compound (8.2 g, 69.7%).

MS(ES+) m/z = 234.27 (M+1).

Step 2a: Methyl 5-bromo-1,3,3-trimethyl-2-oxoindoline-6-carboxylate

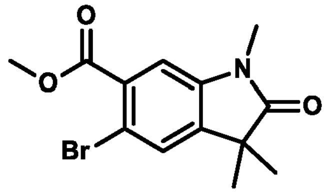

To a stirred solution of methyl 1,3,3-trimethyl-2-oxoindoline-6-carboxylate (1.0 g, 4.29 mmol) in DMF (20.0 mL) was added n-Bromo succinimide (0.76 g, 4.29 mmol) at -10°C. The reaction mixture was stirred initially at -10 °C for 30 min, warmed to room temperature for 1 h and further heated and stirred at 100°C for 30 min. The reaction mixture was cooled to room temperature and quenched with cold water (50.0 mL), precipitated solid was filtered and washed with water and dried under reduced pressure to afford the titled compound (0.55 g, 60% yield).

MS(ES+) m/z = 312.15 (M+1).

Step 3a: 2,6,8,8-Tetramethyl-3H-pyrrolo[2,3-g]quinazoline-4,7(6H,8H)-dione

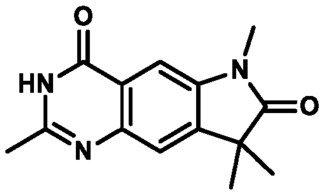

To a stirred solution of methyl 5-bromo-1,3,3-trimethyl-2-oxoindoline-6-carboxylate (0.50 g, 1.602 mmol) in DMF (30.0 mL), acetimidamide hydrochloride (0.182 g, 1.922 mmol), L-proline (0.037 g, 0.320 mmol), $Cs_2CO_3$ (1.56 g, 4.81 mmol), the mixture was stirred for 30 min under nitrogen atmosphere at room temperature, and copper(I) iodide (0.030 g, 0.160 mmol) was added and heated under $N_2$ atm at 80 °C for 18 h. Reaction mass was concentrated under reduced pressure and crude material was purified by flash chromatography in MeOH-DCM as eluent to afford the titled compound (0.40 g, 80% yield)

MS(ES+) m/z = 257.02 (M+)

Step 4a: 5-Bromo-1,3,3-trimethyl-2-oxoindoline-6-carboxylic acid

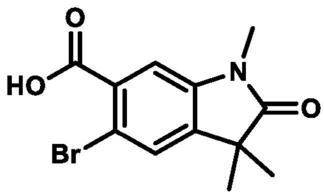

To a stirred solution of methyl 5-bromo-1,3,3-trimethyl-2-oxoindoline-6-carboxylate (0.80 g, 2.56 mmol) in THF (2.0 mL), MeOH (2.0 mL) and water (2.0 mL) was added LiOH (0.30 g, 12.81 mmol). The resulting reaction mixture was heated at 80 °C for 2 h. The reaction mixture was concentrated in vacuum, ice was added, and reaction was acidified with saturated citric acid solution. The precipitated solid was filtered and dried under reduced pressure to afford the titled compound (0.74 g, 97 % yield).

MS(ES+) m/z = 300.15 (M+2)

Step 5a: 2,6,8,8-Tetramethyl-3H-pyrrolo[2,3-g]quinazoline-4,7(6H,8H)-dione

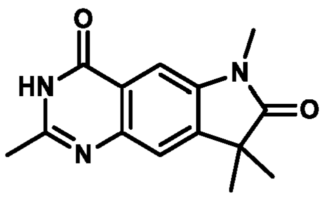

To a mixture of 5-bromo-1,3,3-trimethyl-2-oxoindoline-6-carboxylic acid (0.740 g, 2.482 mmol), acetimidamide hydrochloride (0.352 g, 3.72 mmol), cesium carbonate (2.426 g, 7.45 mmol) and copper(I) iodide (0.095 g, 0.496 mmol) were heated in DMF (10.0 mL) under $N_2$ atm at 85 °C for 3h. The reaction was cooled to room temperature and poured into ice water. The precipitated solid was filtered and dried to afford the titled compound (0.49g, 77 % yield).

MS(ES+) m/z = 257.02 ($M^+$)

Step 6a: Methyl 1,3,3-trimethyl-5-nitro-2-oxoindoline-6-carboxylate

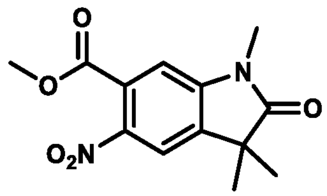

To a stirred solution of methyl 1,3,3-trimethyl-2-oxoindoline-6-carboxylate (0.5 g, 2.143 mmol) in $H_2SO_4$ (10.0 mL) at 0 °C was added $KNO_3$ (2.14 g, 0.22 mmol) portion wise for over 10 min, the reaction mixture was stirred at 0°C for 3 h. The reaction mixture was poured on ice water, the precipitated solid was filtered and dried over reduced pressure to afford the titled compound (0.45 g, 75 % yield).

$^1H$ NMR (400 MHz, DMSO-$d_6$) $\delta$ 8.28 (s, 1H), 7.42 (s, 1H), 3.88 (s, 3H), 3.22 (s, 3H), 1.36 (s, 6H).

Step 7a: Methyl 5-amino-1,3,3-trimethyl-2-oxoindoline-6-carboxylate

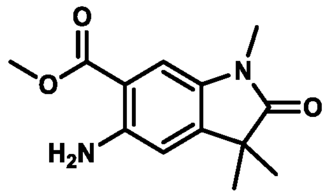

To a stirred solution of methyl 1,3,3-trimethyl-5-nitro-2-oxoindoline-6-carboxylate (0.5 g, 1.797 mmol) in acetic acid (10.0 mL), iron (0.291 g, 5.21 mmol) was added at 25 °C and the reaction was stirred at 90 °C for 1 h. The reaction was cooled to room temperature and solvent was removed under vacuum, and residue neutralized with aq. $NaHCO_3$ (30.0 mL). ethyl acetate (100 mL) was added and the resulting mixture was filtered through celite bed. The filtrate was extracted with ethyl acetate (3 x 50.0 mL). The combined organic layers were washed with brine (100.0 mL), dried over anhydrous $Na_2SO_4$ and concentrated in vacuum to give crude product. The crude product was purified by flash chromatography using MeOH-DCM gradient to afford the titled compound. (0.250 g, 56.0 % yield).

$^1H$ NMR (400 MHz, DMSO-$d_6$) $\delta$ 7.16 (s, 1H), 6.80 (s, 1H), 6.57 (s, 2H), 3.80 (s, 3H), 3.08 (s, 3H), 1.24 (s, 6H).

Step 8a: 2,6,8,8-Tetramethyl-6,8-dihydro-3H-pyrrolo[2,3-g]quinazoline-4,7-dione

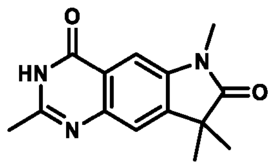

Titled compound was prepared from Step-7a intermediate by employing analogous protocol used in **Step 5 of Example 81** (0.2 g, 77% yield).

MS(ES+) m/z = 258.17 (M+1).

Step 6: 4-Chloro-2,6,8,8-tetramethyl-6,8-dihydro-7H-pyrrolo[2,3-g]quinazolin-7-one

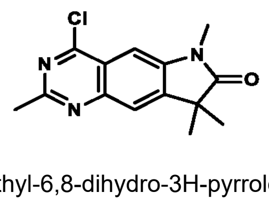

To a suspension of 2,6,8,8-tetramethyl-6,8-dihydro-3H-pyrrolo[2,3-g]quinazoline-4,7-dione (0.380 g, 1.477

mmol) in chlorobenzene (6.0 mL) was added DIPEA (0.696 ml, 3.99 mmol) followed by addition of $POCl_3$ (0.344 ml, 3.69 mmol) drop wise at room temperature. Resulting reaction mixture was heated at 90°C for the 2.5 h. Reaction mixture was poured in cold water and extracted with ethyl acetate (2 x 20.0 mL). Combined organic layer was washed with brine (25.0 mL), dried over anhydrous $Na_2SO_4$, concentrated under high vacuo to afford titled compound(0.4 g, 98 % yield).

MS(ES+) m/z = 276.2 (M+1).

Step-7: (R)-4-((1-(3-(1,1-Difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one **(Compound 68)**

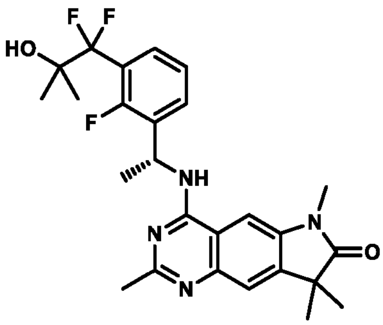

To the stirred solution of 4-chloro-2,6,8,8-tetramethyl-6,8-dihydro-7H-pyrrolo[2,3-g]quinazolin-7-one (100 mg, 0.363 mmol) in 1,4-Dioxane (3.0 mL), (R)-1-(3-(1-aminoethyl)-2-fluorophenyl)-1,1-difluoro-2-methylpropan-2-ol hydrochloride (86 mg, 0.302 mmol) and DIPEA (0.264 ml, 1.511 mmol) were added at room temperature. The resulting mixture was stirred at 120 °C for 30 h. Reaction mixture was concentrated under vacuum to get crude product. The crude product was purified by RP HPLC to afford titled compound (25 mg, 17.00 % yield)

MS(ES+) m/z = 487.2 (M+1).

$^1$H NMR (400 MHz, DMSO-$d_6$) δ 8.21 (d, *J* = 7.4 Hz, 1H), 7.89 (s, 1H), 7.62 - 7.58 (m, 2H), 7.34 - 7.28 (m, 1H), 7.25 - 7.17 (m, 1H), 5.85 - 5.79 (m, 1H), 5.34 (s, 1H), 3.28 (s, 3H), 2.32 (s, 3H), 1.60 (d, *J* = 7.0 Hz, 3H), 1.35 (s, 3H), 1.34 (s, 3H), 1.24 (s, 3H), 1.22 (s, 3H).

**Examples 82-87b** in **Table-5** were synthesized by following the analogous procedure as described in Example **81** using corresponding intermediate and appropriate chiral amines.

**Table-5**

| Example | Chemical structure | LCMS and $^1$H NMR data |
|---|---|---|
| 82 | 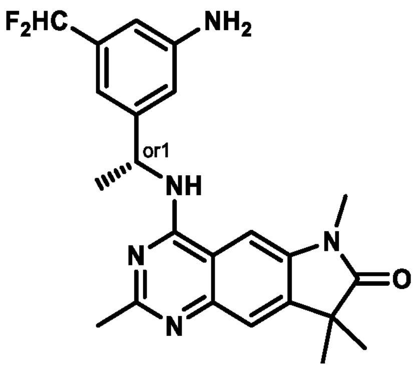 (R/S)-4-((1-(3-amino-5-(difluoromethyl)phenyl)ethyl)amino)-2,6,8,8-tetramethyl-6H-pyrrolo[2,3-g]quinazolin-7(8H)-one **(Compound 69)** | MS(ES+) m/z = 426.33 (M+1) $^1$H NMR (400 MHz, DMSO-$d_6$) δ 8.10 (d, *J*= 8.0 Hz, 1H), 7.88 (s, 1H), 7.60 (s, 1H), 6.83 (t, *J*= 56.3 Hz, 1H), 6.81 - 6.73 (m, 2H), 6.58 (s, 1H), 5.67 - 5.48 (m, 1H), 5.36 (s, 2H), 3.26 (s, 3H), 2.39 (s, 3H), 1.56 (d, *J*= 7.0 Hz, 3H), 1.35 (s, 3H), 1.34 (s, 3H). |

(continued)

| Example | Chemical structure | LCMS and $^1$H NMR data |
|---|---|---|
| 83 | 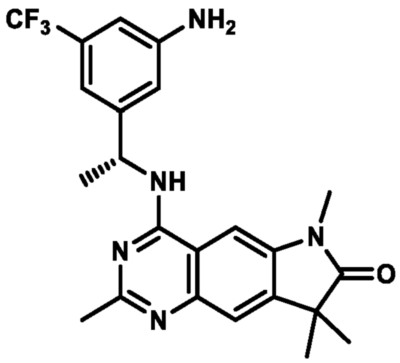 (R)-4-((1-(3-amino-5-(trifluoromethyl)phenyl)ethyl)amino)-2,6,8,8-tetramethyl-6H-pyrrolo[2,3-g]quinazolin-7(8H)-one **(Compound 70)** | MS(ES+) m/z = 444.33 (M+1) $^1$H NMR (400 MHz, DMSO-$d_6$) δ 8.11 (d, *J* = 7.9 Hz, 1H), 7.86 (s, 1H), 7.61 (s, 1H), 6.92 - 6.84 (m, 2H), 6.73 - 6.67 (m, 1H), 5.61 - 5.56 (m, 1H), 5.56 - 5.52 (m, 2H), 3.26 (s, 3H), 2.39 (s, 3H), 1.57 (d, *J* = 7.0 Hz, 3H), 1.35 (s, 3H), 1.34 (s, 3H). |
| 84 | 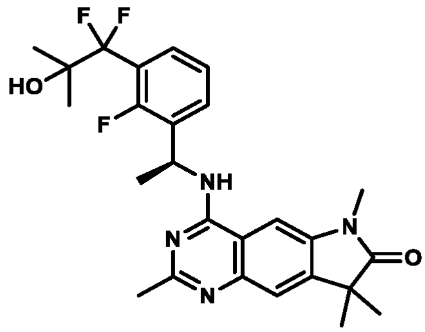 (S)-4-((1-(3-(1,1-difluoro-2-hydroxy-2-methylpropyl)-2-fluorophenyl)ethyl)amino)-2,6,8,8-tetramethyl-6H-pyrrolo[2,3-g]quinazolin-7(8H)-one **(Compound 71)** | MS(ES+) m/z = 487.42 (M+1) $^1$H NMR (400 MHz, DMSO-$d_6$) δ 8.22 (d, *J* = 7.4 Hz, 1H), 7.89 (s, 1H), 7.70 - 7.56 (m, 2H), 7.35 - 7.27 (m, 1H), 7.25 - 7.19 (m, 1H), 5.88 - 5.77 (m, 1H), 5.35 (s, 1H), 3.29 (s, 3H), 2.32 (s, 3H), 1.61 (d, *J* = 7.0 Hz, 3H), 1.35 (s, 3H), 1.34 (s, 3H),1.25 (s, 3H), 1.22 (s, 3H). |
| 85 | 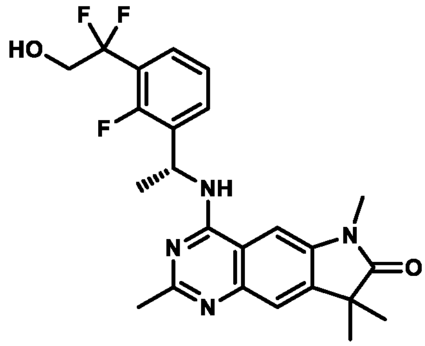 (R)-4-((1-(3-(1,1-difluoro-2-hydroxyethyl)-2-fluorophenyl)ethyl)amino)-2,6,8,8-tetramethyl-6,8-dihydro-7H-pyrrolo[2,3-g]quinazolin-7-one **(Compound 72)** | MS(ES+) m/z = 459.36 (M+1) $^1$H NMR (400 MHz, DMSO-$d_6$) δ 8.22 (d, *J* = 7.4 Hz, 1H), 7.88 (s, 1H), 7.69 - 7.62 (m, 1H), 7.61 (s, 1H), 7.44 - 7.40 (m, 1H), 7.32 - 7.21 (m, 1H), 5.87 - 5.79 (m, 1H), 5.76 - 5.68 (m, 1H), 4.00 - 3.90 (m, 2H), 3.28 (s, 3H), 2.34 (s, 3H), 1.62 (d, *J* = 7.0 Hz, 3H), 1.35 (s, 3H), 1.34 (s, 3H). |

(continued)

| Example | Chemical structure | LCMS and $^{1}H$ NMR data |
|---|---|---|
| 86 | 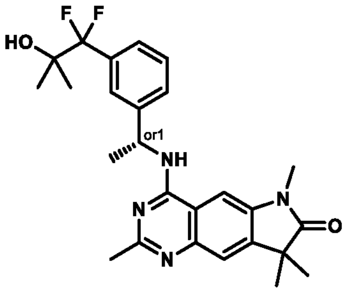<br>(R/S)-4-((1-(3-(1,1-difluoro-2-hydroxy-2-methylpropyl)phenyl)ethyl)amino)-2,6,8,8-tetramethyl-6,8-dihydro-7H-pyrrolo[2,3-g]quinazolin-7-one **(Compound 73)** | MS(ES+) m/z = 469.42 (M+1)<br>$^{1}H$ NMR (400 MHz, DMSO-$d_6$) δ 8.18 (d, *J* = 7.8 Hz, 1H), 7.85 (s, 1H), 7.61 - 7.53 (m, 3H), 7.43 - 7.37 (m, 1H), 7.35 - 7.31 (m, 1H), 5.68 - 5.59 (m, 1H), 5.25 (s, 1H), 3.26 (s, 3H), 2.36 (s, 3H), 1.63 (d, *J* = 7.0 Hz, 3H), 1.34 (s, 3H), 1.33 (s, 3H), 1.18 - 1.09 (m, 6H). |
| 87a | 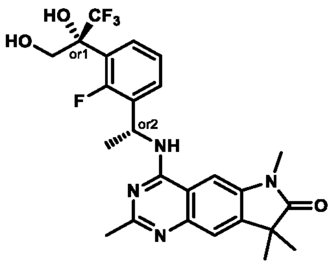<br>4-(((S/R)-1-(2-fluoro-3-((S/R)-1,1,1-trifluoro-2,3-dihydroxypropan-2-yl)phenyl) ethyl)amino)-2,6,8,8-tetramethyl-6,8-dihydro-7H-pyrrolo[2,3-g]quinazolin-7-one **(Compound 74a)** | MS(ES+) m/z = 507.42 (M+1)<br>$^{1}H$ NMR (400 MHz, DMSO-$d_6$) δ 8.17 (d, *J* = 7.7 Hz, 1H), 7.90 (s, 1H), 7.69 - 7.58 (m, 2H), 7.56 - 7.47 (m, 1H), 7.23 - 7.16 (m, 1H), 6.59 (s, 1H), 5.89 - 5.79 (m, 1H), 5.22 (t, *J* = 5.6 Hz, 1H), 4.20 - 4.01 (m, 2H), 3.28 (s, 3H), 2.35 (s, 3H), 1.58 (d, *J* = 7.0 Hz, 3H), 1.35 (s, 3H), 1.34 (s, 3H). |
| 87b | 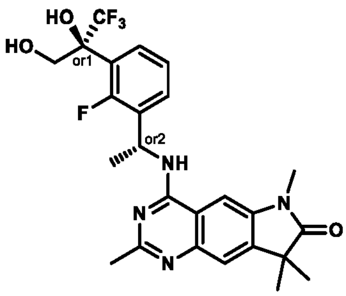<br>4-(((R/S)-1-(2-fluoro-3-((R/S)-1,1,1-trifluoro-2,3-dihydroxypropan-2-yl)phenyl) ethyl)amino)-2,6,8,8-tetramethyl-6,8-dihydro-7H-pyrrolo[2,3-g]quinazolin-7-one **(Compound 74b)** | MS(ES+) m/z = 507.42 (M+1)<br>$^{1}H$ NMR (400 MHz, DMSO-$d_6$) d 8.17 (d, *J* = 7.7 Hz, 1H), 7.90 (s, 1H), 7.69 - 7.58 (m, 2H), 7.56 - 7.47 (m, 1H), 7.23 - 7.17 (m, 1H), 6.59 (s, 1H), 5.89 - 5.79 (m, 1H), 5.22 (t, *J* = 5.6 Hz, 1H), 4.20 - 4.01 (m, 2H), 3.28 (s, 3H), 2.35 (s, 3H), 1.58 (d, *J* = 7.0 Hz, 3H), 1.35 (s, 3H), 1.34 (s, 3H). |

**Example 88** : - (R)-1,1-difluoro-1-(2-fluoro-3-(1-((2,6,8,8-tetramethyl-7,8-dihydro-6H-pyrrolo[2,3-g]quinazolin-4-yl)amino)ethyl)phenyl)-2-methylpropan-2-ol **(Compound 75)**

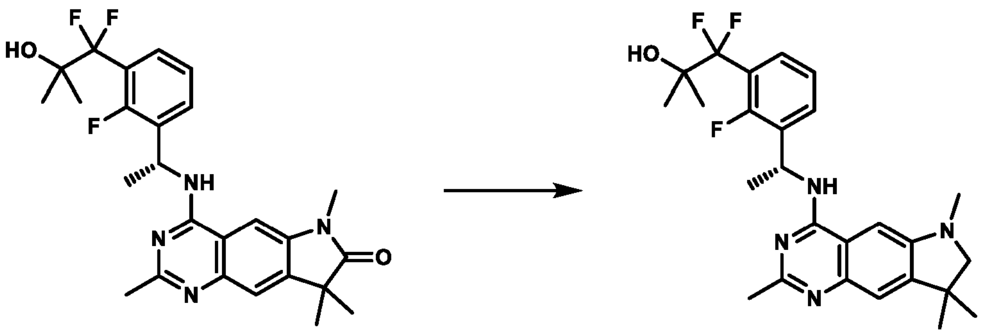

To a stirred solution of the (R)-4-((1-(3-(1,1-difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one (0.1 g, 0.206 mmol) **(Example 81, Compound 68)** in THF (2.0 mL) was added $BH_3$.DMS (0.154 mL , 0.308 mmol) at 0°C and heated to 70 °C for 1.5 h. The reaction mass was cooled to room temperature, solvent was evaporated, and residue was stirred in 1M HCl (10.0 mL) at room temperature for 30 minutes. The reaction mass was extracted with ethyl acetate, dried over anhydrous $Na_2SO_4$ and concentrated to obtain crude product. Crude was purified by Reverse Phase preparative HPLC to obtain the titled compound (0.020 g, 20.59 % yield)

MS(ES+) m/z = 473.42, 474.33

$^1H$ NMR (400 MHz, DMSO-$d_6$) δ 8.15 (s, 1H), 7.92 (d, *J* = 7.6 Hz, 1H), 7.61 - 7.55 (m, 1H), 7.33 - 7.27 (m, 1H), 7.23 - 7.16 (m, 2H), 5.84 - 5.76 (m, 1H), 5.34 (s, 1H), 3.17 (d, *J*= 1.5 Hz, 2H), 2.89 (s, 3H), 2.26 (s, 3H), 1.58 (d, *J*= 7.0 Hz, 3H), 1.32 (s, 3H), 1.30 (s, 3H), 1.24 (s, 3H), 1.21 (s, 3H).

**Example 89:** 4-(((R)-1-(3-((R&S)-1,1-Difluoro-2,3-dihydroxy-2-methylpropyl)-2-fluorophenyl) ethyl)amino)-2,6,8,8-tetramethyl-6,8-dihydro-7H-pyrrolo[2,3-g]quinazolin-7-one. **(Compound 76)**

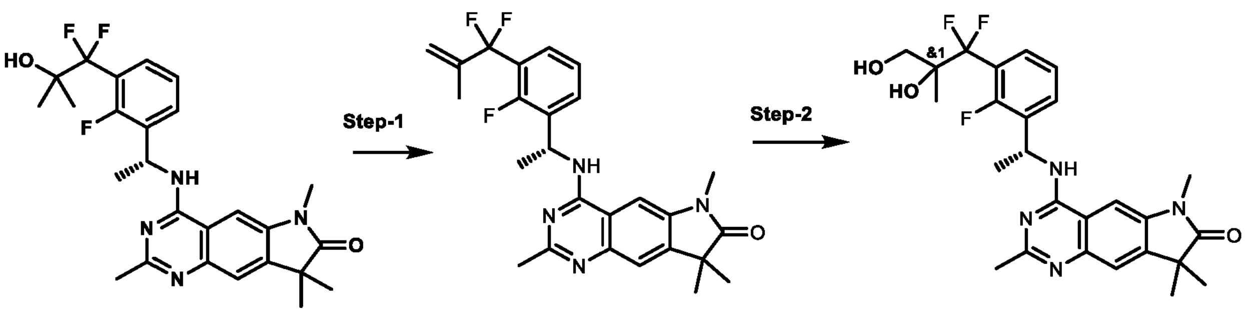


Step 1: (R)-4-((1-(3-(1,1-Difluoro-2-methylallyl)-2-fluorophenyl)ethyl)amino)-2,6,8,8-tetramethyl-6,8-dihydro-7H-pyrrolo[2,3-g]quinazolin-7-one

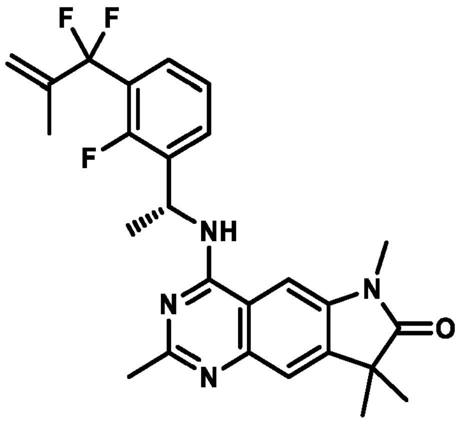

To a stirred solution of (R)-4-((1-(3-(1,1-difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one **(Example 81,Compound 68)** (0.25 g, 0.514 mmol) in DCM (5.0 mL) was added DAST (1.82 g, 11.3 mmol, 1.49 mL) at -70 °C. The reaction was stirred at -70 °C for 0.5 h , and gradually warmed to 0 °C for another 0.5 h under $N_2$ atmosphere. The reaction mixture was quenched with saturated $NaHCO_3$ (30.0 mL), extracted with DCM (50.0 mL × 2). The organic phase was dried over anhydrous $Na_2SO_4$ and concentrated to give a residue. The residue was purified by flash chromatography in 5% MeOH in DCM to afford the titled compound (0.2 g, 83 % yield).

MS(ES+) m/z = 469.53 (M+1).

Step 2: 4-(((R)-1-(3-((R&S)-1,1-Difluoro-2,3-dihydroxy-2-methylpropyl)-2-fluorophenyl) ethyl)amino)-2,6,8,8-tetramethyl-6,8-dihydro-7H-pyrrolo[2,3-g]quinazolin-7-one.

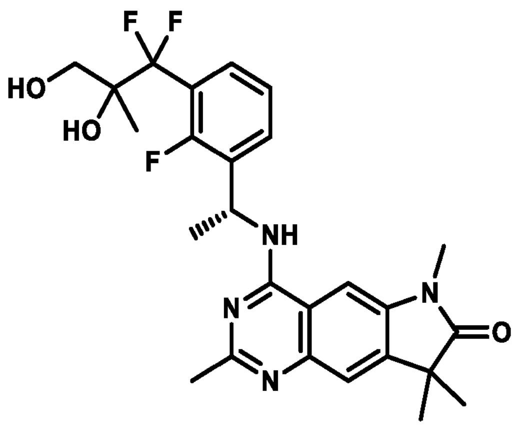

To a stirred solution of (R)-4-((1-(3-(1,1-difluoro-2-methylallyl)-2-fluorophenyl)ethyl)amino)-2,6,8,8-tetramethyl-6,8-dihydro-7H-pyrrolo[2,3-g]quinazolin-7-one (0.20 g, 0.427 mmol)**(Step 1)** in acetone (2.0 mL), tert-butanol (0.800 mL) and water (0.800 mL) , NMO (0.125 g, 1.067 mmol) and osmium tetroxide (6.70 µl, 0.021 mmol) was added at 0°C. The reaction was stirred at room temperature for 18 h. The reaction was quenched with sodium thiosulfate solution extracted with DCM (2 x 25.0 mL) and concentrated under reduced pressure to get crude compound. Crude product was purified by flash chromatography to get titled compound (0.17 g). The diastereomers were separated by chiral chromatography.

Chiral separation method: CHIRALPAK IE CRL-005 HEX_IPA_50_50_A_B_1.0 ML _8MIN_241NM; 1.0 mL /min

Peak 1: 4-(((R)-1-(3-((R/S)-1,1-difluoro-2,3-dihydroxy-2-methylpropyl)-2-fluorophenyl)ethyl)amino)-2,6,8,8-tetramethyl-6,8-dihydro-7H-pyrrolo[2,3-g]quinazolin-7-one **(Compound 76a)**

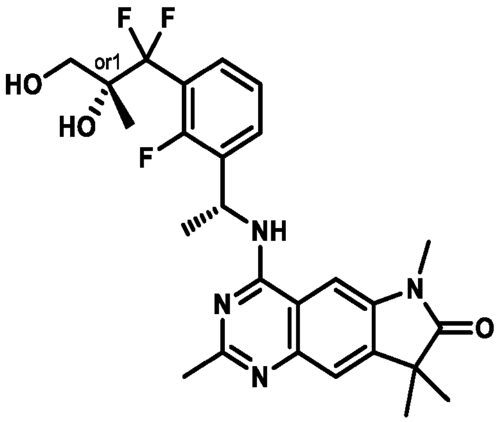

$t_{ret}$(min) = 4.45

MS(ES+) m/z = 503.42 (M+1).

$^1$H NMR (400 MHz, DMSO-$d_6$) δ 8.23 (d, *J* = 7.4 *Hz,* 1H), 7.90 (s, 1H), 7.64 - 7.56 (m, 2H), 7.32 (m, 1H), 7.24 - 7.18 (m, 1H), 5.84 - 5.80 (m, 1H), 5.24 (s, 1H), 4.70 (t, *J* = 6.1 Hz, 1H), 3.54 - 3.39 (m, 2H), 3.28 (s, 3H), 2.33 (s, 3H), 1.60 (d, *J* = 7.0 *Hz,* 3H), 1.35 (s, 3H), 1.34 (s, 3H), 1.21 (s, 3H).

Peak 2: 4-(((R)-1-(3-((S/R)-1,1-difluoro-2,3-dihydroxy-2-methylpropyl)-2-fluorophenyl)ethyl)amino)-2,6,8,8-tetramethyl-6,8-dihydro-7H-pyrrolo[2,3-g]quinazolin-7-one **(Compound 76b)**

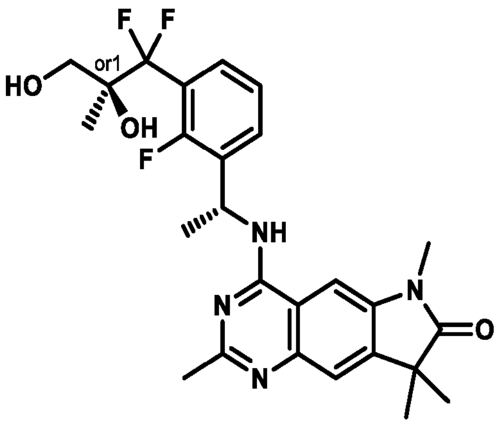

$t_{ret}$(min) = 5.18

MS(ES+) m/z = 503.42 (M+1)

$^1$H NMR (400 MHz, DMSO-$d_6$) δ 8.21 (d, *J* = 7.5 *Hz,* 1H), 7.89 (s, 1H), 7.64 - 7.56 (m, 2H), 7.33 - 7.30 (m, 1H), 7.22 - 7.20 (m, 1H), 5.84 - 5.81 (m, 1H), 5.27 (s, 1H), 4.71 - 4.69 (m, 1H), 3.50 - 3.36 (m, 2H), 3.28 (s, 3H), 2.34 (s, 3H), 1.60 (d, *J* = 7.0 *Hz,* 3H), 1.35 (s, 3H), 1.34 (s, 3H), 1.23 (s, 3H).

**Example 90:** Preparation of (R)-4-((1-(3-(1,1-difluoro-2-hydroxy-2-methylpropyl)-2-fluoro phenyl)ethyl)amino)-2,8,8-trimethyl-6H-pyrrolo[2,3-g]quinazolin-7(8H)-one **(Compound 77)**

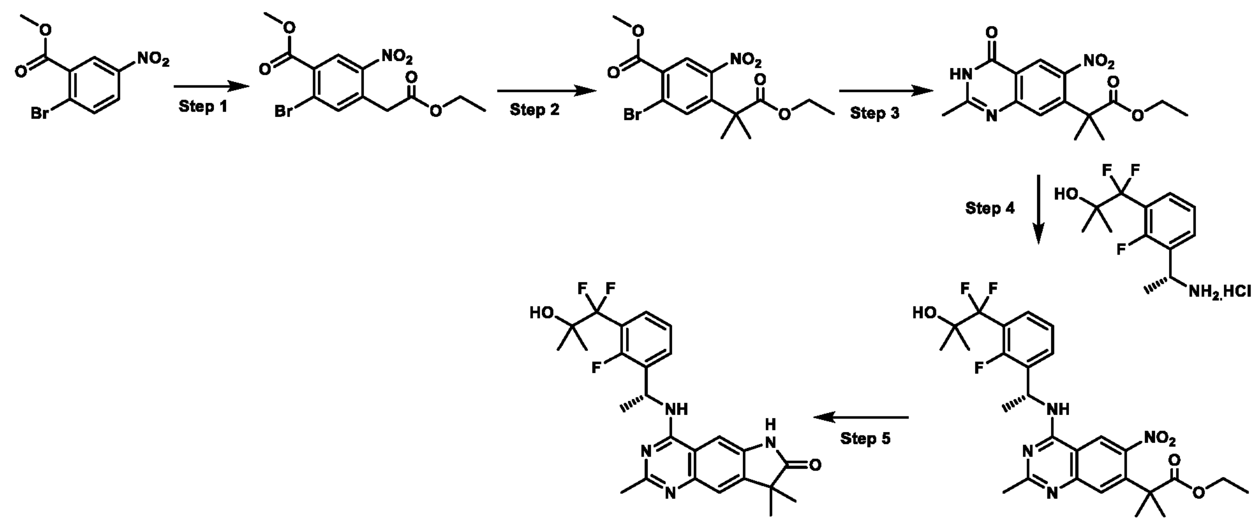


Step 1: Methyl 2-bromo-4-(2-ethoxy-2-oxoethyl)-5-nitrobenzoate

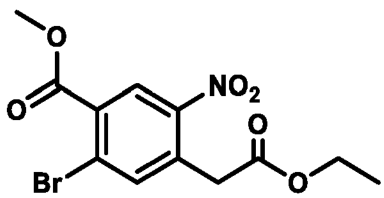

To a stirred solution of potassium tert-butoxide (16.18 g, 144 mmol) in DMF (20.0 mL), ethyl 2-chloroacetate (11.78 g, 96 mmol) was added in dropwise manner over 10 min at -78°C followed by the addition of methyl 2-bromo-5-nitrobenzoate in DMF (150.0 mL) at -78°C. The reaction was warmed -60 °C for 15 min. The reaction was poured into saturated ammonium chloride solution (700.0 mL) and extracted with MTBE (3 x 300.0 mL). The combined organic layer was washed with water (500.0 mL), brine (300.0 mL), dried over anhydrous $Na_2SO_4$ and concentrated in vacuum to give crude product. The crude product was purified by flash chromatography using ethyl acetate - n-hexane gradient to afford titled compound (8.30 g, 24.94 % yield).

$^1$H NMR (400 MHz, DMSO-*d*$_6$) δ 8.49 (s, 1H), 8.11 (s, 1H), 4.15 (s, 2H), 4.10 (q, *J* = 7.1 *Hz,* 2H), 3.92 (s, 3H), 1.18 (t, *J* = 7.1 *Hz,* 3H).

Step 2: Methyl 2-bromo-4-(1-ethoxy-2-methyl-1-oxopropan-2-yl)-5-nitrobenzoate

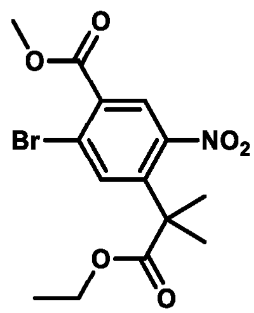

The titled compound was prepared from **Step-1** intermediate by employing analogous protocol mentioned in **Example 76 Step-3** (1.0 g, 61.7 % yield).

$^1$H NMR (400 MHz, DMSO-*d*$_6$) δ 8.36 (s, 1H), 8.09 (s, 1H), 4.01 (q, *J* = 7.1 *Hz,* 2H), 3.91 (s, 3H), 1.63 (s, 6H), 1.10 (t, *J* = 7.1 *Hz,* 3H).

Step 3: Ethyl 2-methyl-2-(2-methyl-6-nitro-4-oxo-3,4-dihydroquinazolin-7-yl)propanoate

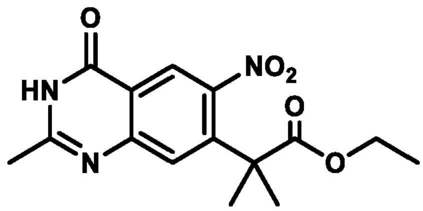

The titled compound was prepared from Step-2 intermediate by employing analogous protocol as **Example 81-Step-3a.** (0.334 g, 50.8 % yield).

MS(ES+) m/z = 320.22 (M+1).

Step 4: (R)-Ethyl 2-(4-((1-(3-(1,1-difluoro-2-hydroxy-2-methylpropyl)-2-fluorophenyl)ethyl)amino)-2-methyl-6-nitroquinazolin-7-yl)-2-methylpropanoate.

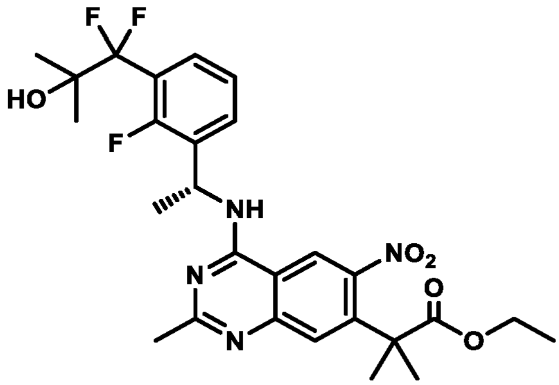

To a suspension of ethyl 2-methyl-2-(2-methyl-6-nitro-4-oxo-3,4-dihydroquinazolin-7-yl)propanoate (0.05 g, 0.157 mmol) in acetonitrile (2.0 mL), (R)-1-(3-(1-aminoethyl)-2-fluorophenyl)-1,1-difluoro-2-methylpropan-2-ol (0.04 g, 0.157 mmol) and 1,8-diazabicyclo[5.4.0]undec-7-ene (0.12 mL , 0.783 mmol) were added at 0°C followed by the addition of BOP (0.083 g, 0.188 mmol) in portion wise manner over 10 min. The reaction was warmed to room temperature and stirred for the 16 h. The reaction was poured in ice water (30.0 mL) and extracted with ethyl acetate (3 x 20.0 mL). The combined organic layer was washed with brine (50.0 mL), dried over anhydrous $Na_2SO_4$ and concentrated. The crude residue was purified by flash chromatography in ethyl acetate-hexane gradient to afford titled compound (0.030 g, 34.9 % yield).

MS(ES+) m/z = 549.33 (M+1).

Step 5: (R)-4-((1-(3-(1,1-difluoro-2-hydroxy-2-methylpropyl)-2-fluorophenyl)ethyl)amino)-2,8,8-trimethyl-6H-pyrrolo [2,3-g]quinazolin-7(8H)-one **Compound 77**

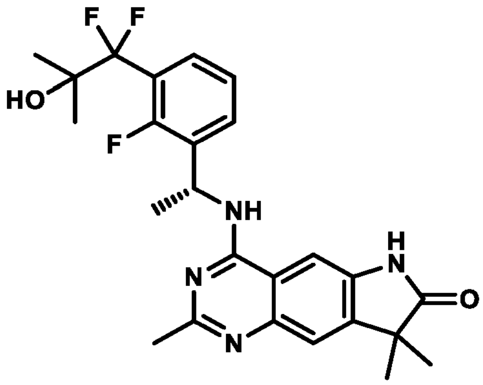

The titled compound was prepared from **Step-4** intermediate by employing analogous protocol mentioned in **Example-76 Step-4** (0.045 g, 52.2% yield)

MS(ES+) m/z = 473.33 (M+1).

$^1$H NMR (400 MHz, DMSO-$d_6$) δ 11.06 (s, 1H), 8.98 (bs, 1H), 7.86 (s, 1H), 7.65 - 7.58 (m, 1H), 7.57 (s, 1H), 7.37 - 7.33 (m, 1H), 7.27 - 7.18 (m, 1H), 5.91 - 5.79 (m, 1H), 5.34 (s, 1H), 2.39 (s, 3H), 1.60 (d, J = 7.0 Hz, 3H), 1.35 (s, 3H), 1.34 (s, 3H), 1.24 (s, 3H), 1.21 (s, 3H).

**Example 91:** Preparation of (R)-4-((1-(3-(1,1-difluoro-2-hydroxy-2-methylpropyl)-2-fluoro phenyl)ethyl)amino)-6-isopropyl-2,8,8-trimethyl-6H-pyrrolo[2,3-g]quinazolin-7(8H)-one **(Compound 78)**

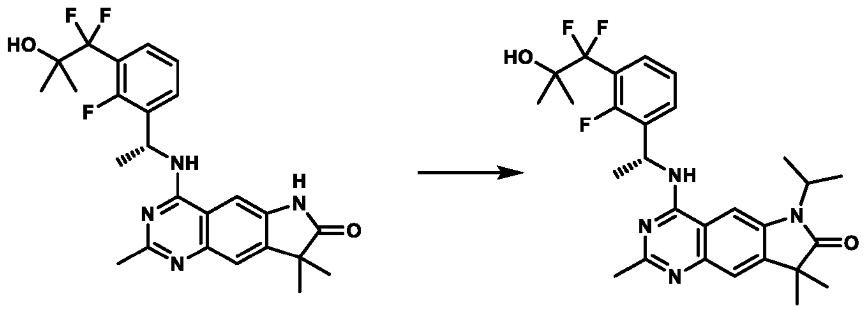

To a stirred solution of (R)-4-((1-(3-(1,1-difluoro-2-hydroxy-2-methylpropyl)-2-fluorophenyl)ethyl)amino)-2,8,8-trimethyl-6,8-dihydro-7H-pyrrolo[2,3-g]quinazolin-7-one (0.04 g, 0.085 mmol)in NMP (100.0 mL) were added 2-bromopropane (0.016 g, 0.127 mmol) and potassium carbonate (10.8 g, 78 mmol). The resulting suspension was stirred at 50 °C for 12 h. The reaction mixture was cooled to room temperature, diluted with ethyl acetate (100.0 mL) and the organic layer was washed with water (50.0 mL), brine (50.0 mL), dried over $Na_2S0_4$, concentrated, and purified by flash chromatography using eluent 50-70% ethyl acetate-n-hexane to afford the titled compound (0.015 g, 34.4 % yield).

MS(ES+) m/z = 515.42 (M+1).

$^1$H NMR (400 MHz, DMSO-$d_6$) δ 8.26 (d, *J* = 7.4 Hz, 1H), 7.90 (s, 1H), 7.62 - 7.56 (m, 2H), 7.34 - 7.29 (m, 1H), 7.24 - 7.19 (m, 1H), 5.86 - 5.79 (m, 1H), 5.34 (s, 1H), 4.62 - 4.55 (m, 1H), 2.31 (s, 3H), 1.62 (d, *J*= 7.1 *Hz,* 3H), 1.59 - 1.51 (m, 6H), 1.32 (s, 3H), 1.31 (s, 3H) , 1.24 (s, 3H), 1.21 (s, 3H)

**Example 92:** Preparation of (R)-4'-((1-(3-(1,1-difluoro-2-hydroxy-2-methylpropyl)-2-fluorophenyl)ethyl)amino)-2',6'-dimethylspiro[cyclopropane-1,8'-pyrrolo[2,3-g]quinazolin]-7'(6'H)-one **(Compound 79)**

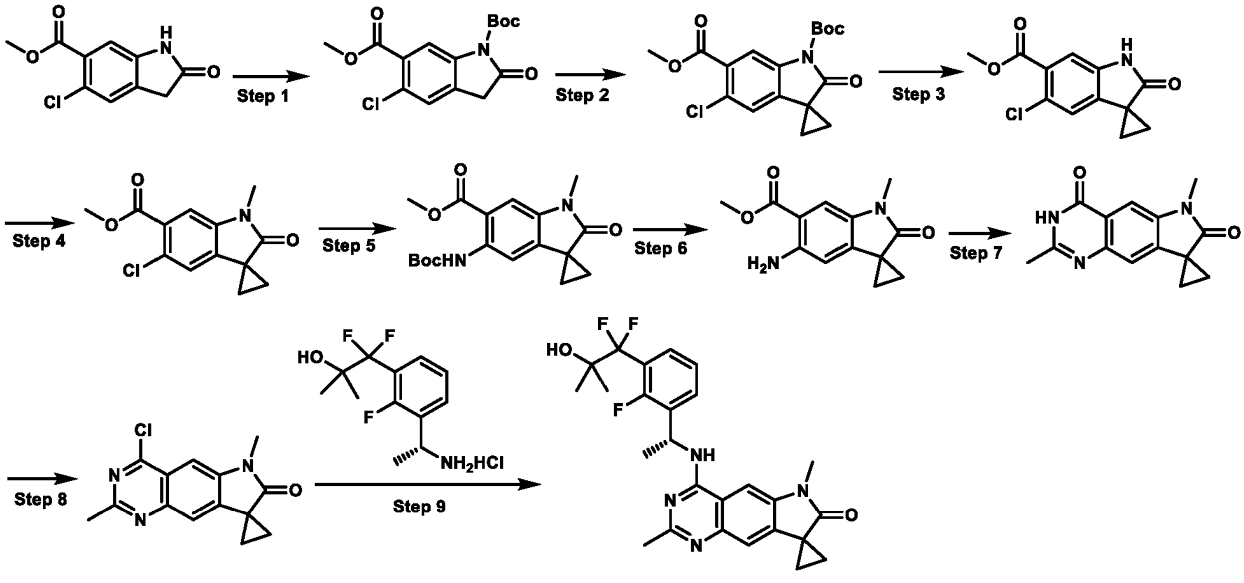


Step 1: 1-Tert-butyl 6-methyl 5-chloro-2-oxoindoline-1,6-dicarboxylate

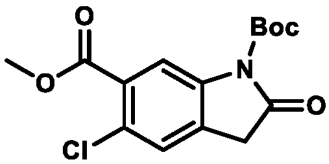

To a stirred solution of Example 76- step-4 intermediate (2.45 g, 10.86 mmol) in THF (25.0 mL), di-tert-butyldicarbonate (3.28 mL, 14.12 mmol) and $Na_2CO_3$ (1.381 g, 13.03 mmol) was added at room temperature. The reaction mass heated at 70 °C for 18 h, cooled to room temperature and diluted with water. Aqueous layer was extracted with ethyl acetate (2 x 100.0 mL). The combined organic layer was washed with brine and evaporated under reduced pressure to obtain the crude mass. The crude mass was purified through flash chromatography using 20 - 40% ethyl acetate-n-hexane as eluent to afford the titled compound (1.82 g, 51.5 % yield).

$^1$H NMR (400 MHz, Chloroform-*d*) δ 8.33 (s, 1H), 7.37 (s, 1H), 3.97 (s, 3H), 3.70 (s, 2H), 1.67 (s, 9H).

Step 2: 1'-Tert-butyl 6'-methyl 5'-chloro-2'-oxospiro[cyclopropane-1,3'-indoline]-1',6'-dicarboxylate

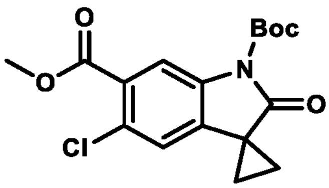

To a stirred solution of 1-(tert-butyl) 6-methyl 5-chloro-2-oxoindoline-1,6-dicarboxylate (3.4 g, 10.44 mmol) in DMSO (5.0 mL), $K_2CO_3$ (5.77 g, 41.8 mmol) and 1,2-dibromoethane (2.160 mL , 14.72 mmol) were added at -10°C for 15 min. The reaction mass was stirred at room temperature for 18 h. The reaction mass was poured in water and extracted with Ethyl acetate (2 x 100.0 mL). The combined organic layer was washed with water (100.0 mL), brine, dried over anhydrous $Na_2SO_4$ and concentrated under reduced pressure. The crude product was purified by flash chromatography in Ethyl acetate-hexane gradient to afford the titled compound. (1.8 g 49% yield).

$^1$H NMR (400 MHz, Chloroform-d) δ 8.44 (s, 1H), 6.90 (s, 1H), 3.96 (s, 3H), 1.97 - 1.91 (m, 2H)1.68 (s, 9H), 1.67 - 1.64 (m, 2H).

Step 3: Methyl 5'-chloro-2'-oxospiro[cyclopropane-1,3'-indoline]-6'-carboxylate

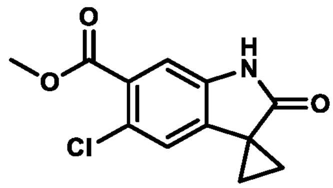

To a stirred solution of Step 2 (1.8 g, 5.12 mmol) in DCM (20.0 mL) was added HCl in 1,4-dioxane (8.53 mL, 25.6 mmol) at 0°C. The mixture was stirred at room temperature for 18 h. The reaction mass was poured in water and extracted with Ethyl acetate (2 x 20.0 mL). The combined organic layer was washed with water (20.0 mL), brine, dried over anhydrous $Na_2SO_4$ and concentrated under reduced pressure to get the crude mass. The crude product was purified by flash chromatography in Ethyl acetate-hexane gradient to afford the titled compound (1.2 g, 96 % yield)
MS(ES+) m/z = 252.17 (M+1).

Step 4: Methyl 5'-chloro-1'-methyl-2'-oxospiro[cyclopropane-1,3'-indoline]-6'-carboxylate

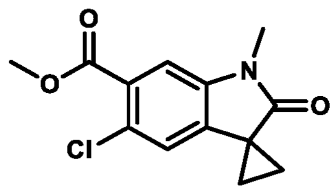

The titled compound was prepared from **Step-3** by employing analogous protocol mentioned in **Example-76 Step-5.** (0.75 g, 59.2 % yield).
MS(ES+) m/z = 265.92(M+1).

Step 5: Methyl 5'-((tert-butoxycarbonyl)amino)-1'-methyl-2'-oxospiro[cyclopropane-1,3'-indoline]-6'-carboxylate

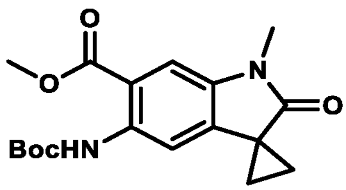

The titled compound was prepared from **Step-4** by employing analogous protocol mentioned in **Example 71 Step-6.** (0.60 g, 92 % yield)
MS(ES+) m/z = 247.11 (M-100).

Step 6: Methyl 5'-amino-1'-methyl-2'-oxospiro[cyclopropane-1,3'-indoline]-6'-carboxylate

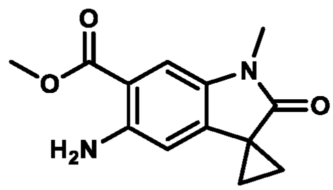

The titled compound was prepared from **Step-5** by employing analogous protocol mentioned in **Example 71 Step-7.** (0.030 g, 56.3% Yield)

MS(ES+) m/z = 247. 17(M+1).

Step 7: 2',6'-Dimethylspiro[cyclopropane-1,8'-pyrrolo[2,3-g]quinazoline]-4',7'(3'H,6'H)-dione

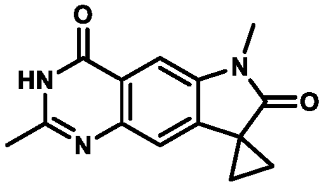

The titled compound was prepared from **Step-6** by employing analogous protocol mentioned in **Example 71 Step-8.** (0.010 g, 32.2% Yield).

MS(ES+) m/z = 256.17 (M+1).

Step 8: 4'-Chloro-2',6'-dimethylspiro[cyclopropane-1,8'-pyrrolo[2,3-g]quinazolin]-7'(6'H)-one

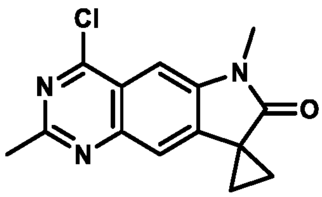

The titled compound was prepared from **Step-7** by employing analogous protocol mentioned in **Example 71 Step-9.** (0.055 g, 64.1 % yield).

MS(ES+) m/z = 273.94 (M+1).

Step 9: (R)-4'-((1-(3-(1,1-difluoro-2-hydroxy-2-methylpropyl)-2-fluorophenyl)ethyl) amino)-2',6'-dimethylspiro[cyclopropane-1,8'-pyrrolo[2,3-g]quinazolin]-7'(6'H)-one **(Compound 79)**

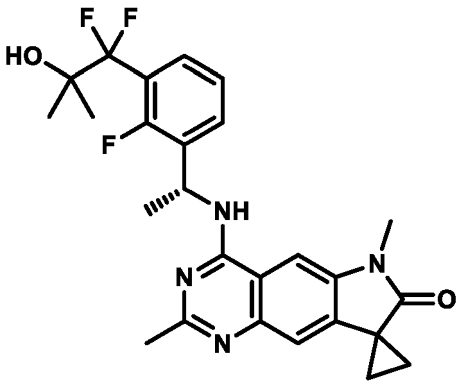

The titled compound was prepared by reaction of **Step-8** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10.** (0.025 g, 31.6% yield)

MS(ES+) m/z = 485.10 (M+1).

$^{1}H$ NMR (400 MHz, DMSO-$d_6$) δ 8.25 (s, 1H), 7.95 (s, 1H), 7.66 - 7.57 (m, 1H), 7.34 - 7.29 (m, 1H), 7.29 (s, 1H),

7.26 - 7.17 (m, 1H), 5.86 - 5.79 (m, 1H), 5.34 (s, 1H), 3.36 (s, 3H), 2.31 (s, 3H), 1.80 - 1.71 (m, 2H), 1.66 - 1.63 (m, 2H), 1.61 (d, *J* = 7.2 Hz, 3H), 1.24 (s, 3H), 1.22 (s, 3H).

**Example 93:** Preparation of (R)-4-((1-(3-(1,1-difluoro-2-hydroxyethyl)-2-fluorophenyl)ethyl)amino)-2,6,6,8-tetramethyl-6,8-dihydro-7H-pyrrolo[3,2-g]quinazolin-7-one **(Compound 80)**

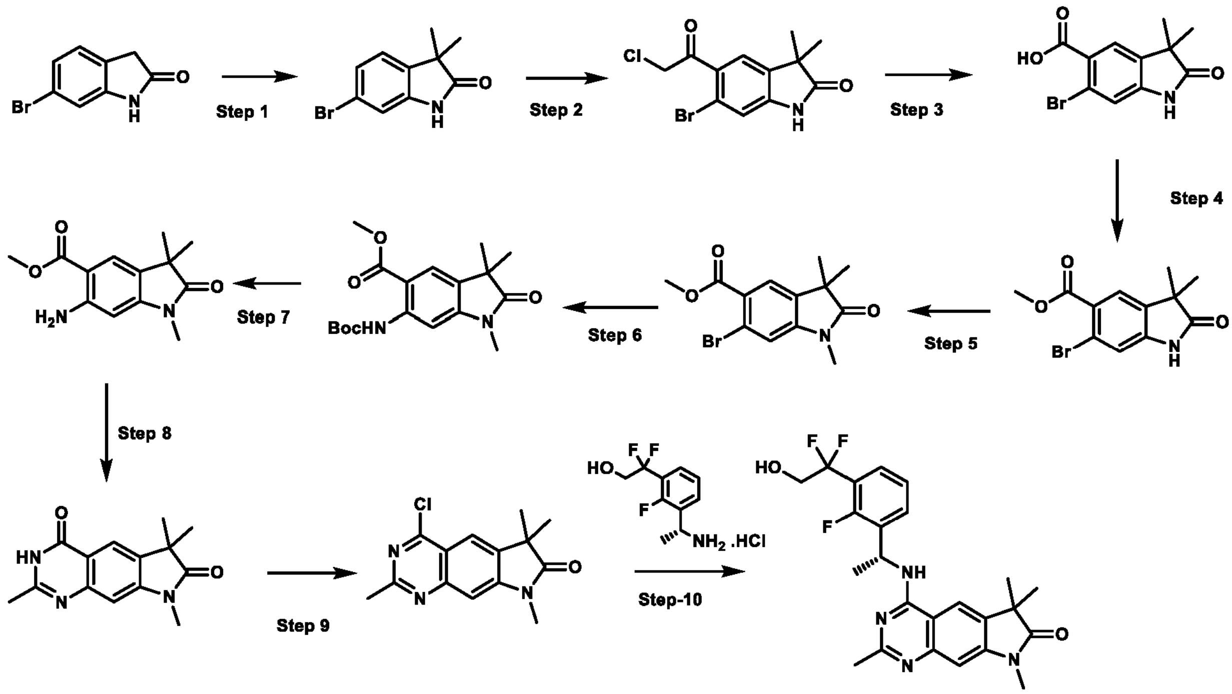


Step 1: 6-Bromo-3,3-dimethylindolin-2-one

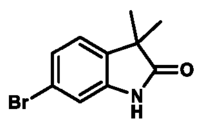

To a stirred solution of 6-bromoindolin-2-one (5.0 g, 23.58 mmol) in THF (50.0 mL), potassium tert-butoxide (12.70 g, 113 mmol) and copper(I) bromide-dimethyl sulfide complex (0.970 g, 4.72 mmol) was added slowly at 0°C. The reaction was stirred for 15 min at 0°C and methyl iodide (1.327 mL, 21.22 mmol) was added. The reaction was stirred at room temperature for 5 h. The reaction was quenched with ammonium chloride solution (20.0 mL) and extracted in Ethyl acetate (2 x 150.0 mL). The organic layer was dried over anhydrous $Na_2SO_4$. Solvent was evaporated and compound obtained was purified by flash chromatography in Ethyl acetate-hexane gradient to afford the titled compound (5.00 g, 88 % yield).

MS(ES+) m/z = 241.4 (M), 243.4 (M+2).

Step 2: 6-Bromo-5-(2-chloroacetyl)-3,3-dimethylindolin-2-one

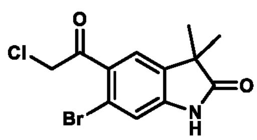

The titled compound was prepared from Step-1 intermediate by employing analogous protocol mentioned in Example **71-Step-2** (2.5 g, 86 % yield).

$^1$H NMR (400 MHz, DMSO- $d_6$) δ 10.78 (s, 1H), 7.86 (s, 1H), 7.15 (s, 1H), 5.05 (s, 2H), 1.29 (s, 6H).

Step 3: 6-Bromo-3,3-dimethyl-2-oxoindoline-5-carboxylic acid

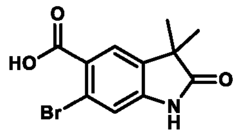

The titled compound was prepared from Step-2 intermediate by employing analogous protocol mentioned in Example **71- Step-3** (1.67 g,74.4 % yield) which was used in next reaction without purification.

MS(ES+) m/z = 284.34 (M), 286.15 (M+2).

Step 4: Methyl 6-bromo-3,3-dimethyl-2-oxoindoline-5-carboxylate

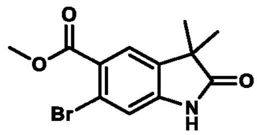

The titled compound was prepared from Step-3 intermediate by employing analogous protocol mentioned in Example **71-Step-4** (1.0 g, 79 % yield).

$^1$H NMR (400 MHz, DMSO-$d_6$) δ 10.76 (s, 1H), 7.79 (s, 1H), 7.14 (s, 1H), 3.83 (s, 3H), 1.28 (s, 6H).

Step 5: Methyl 6-bromo-1,3,3-trimethyl-2-oxoindoline-5-carboxylate

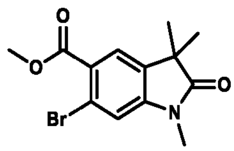

The titled compound was prepared from **Step-4** by employing analogous protocol mentioned in **Example 71-Step-5** (0.9 g, 86 % yield).

$^1$H NMR (400 MHz, DMSO-$d_6$) δ 7.82 (s, 1H), 7.44 (s, 1H), 3.84 (s, 3H), 3.17 (s, 3H), 1.29 (s, 6H).

Step 6: Methyl 6-((tert-butoxycarbonyl)amino)-1,3,3-trimethyl-2-oxoindoline-5-carboxylate

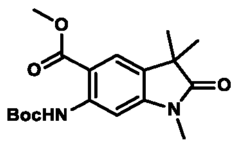

The titled compound was prepared from **Step-5** by employing analogous protocol mentioned in **Example 71 Step-6** (0.8 g, 80 % yield).

$^1$H NMR (400 MHz, DMSO-$d_6$) δ 10.65 (s, 1H), 7.93 (s, 1H), 7.90 (s, 1H), 3.85 (s, 3H), 3.15 (s, 3H), 1.50 (s, 6H), 1.37 (s, 9H).

Step 7: Methyl 6-amino-1,3,3-trimethyl-2-oxoindoline-5-carboxylate

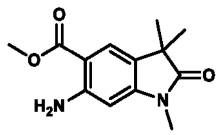

The titled compound was prepared from **Step-6** by employing analogous protocol mentioned in **Example 71 Step-7.** (0.6 g, 92 % yield).

MS(ES+) m/z = 217.5 (M+1).

Step 8: 2,6,6,8-Tetramethyl-6,8-dihydro-3H-pyrrolo[3,2-g]quinazoline-4,7-dione

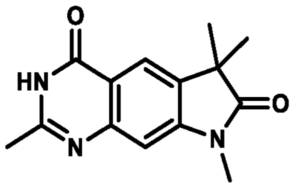

The titled compound was prepared from **Step-7** by employing analogous protocol mentioned in **Example 71 Step-8.** (0.45g, 83% yield)

MS(ES+) m/z = 257.2 (M+1).

Step 9: 4-Chloro-2,6,6,8-tetramethyl-6,8-dihydro-7H-pyrrolo[3,2-g]quinazolin-7-one (10714-168).

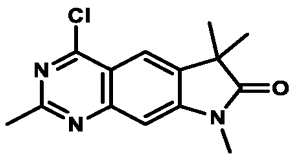

The titled compound was prepared from **Step-8** by employing analogous protocol mentioned in **Example 71 Step-9.** (0.4 g, 83 % yield)

MS(ES+) m/z = 276.08 (M+1).

Step 10: (R)-4-((1-(3-(1,1-difluoro-2-hydroxyethyl)-2-fluorophenyl)ethyl)amino)-2,6,6,8-tetramethyl-6,8-dihydro-7H-pyrrolo[3,2-g]quinazolin-7-one **(Compound 80)**

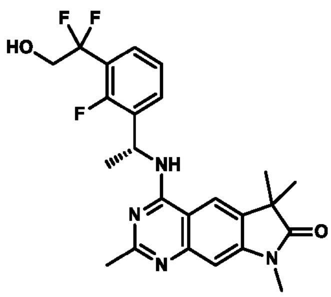

The titled compound was prepared from **Step-9** by employing analogous protocol mentioned in **Example 71 Step-10.** (0.020 g, 17.18 % yield)

MS(ES+) m/z = 459.23 (M+1).

$^1$H NMR (400 MHz, DMSO-$d_6$) δ 8.35 (s, 1H), 8.21 (s, 1H), 7.69 - 7.62 (m, 1H), 7.46 - 7.37 (m, 1H), 7.31 - 7.21 (m, 1H), 7.09 (s, 1H), 5.85 - 5.78 (m, 1H), 5.75 - 5.70 (m, 1H), 4.02 - 3.89 (m, 2H), 3.21 (s, 3H), 2.34 (s, 3H), 1.60 (d, *J* = 7.0 *Hz,* 3H), 1.39 (s, 3H), 1.38 (s, 3H)

**Examples 94-98b** in **Table-6** were synthesized by following the analogous procedure as described in Example **93** using corresponding intermediate and appropriate chiral amine/commercially available chiral amine for compound 96.

**Table-6**

| Example | Chemical structure | LCMS and $^1$HNMR data |
|---|---|---|
| 94 | 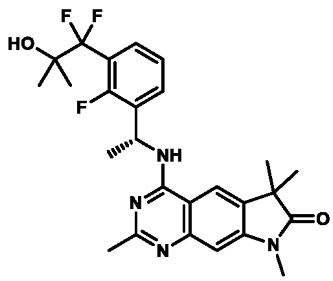 (R)-4-((1-(3-(1,1-difluoro-2-hydroxy-2-methylpropyl)-2-fluorophenyl)ethyl)amino)-2,6,6,8-tetramethyl-6,8-dihydro-7H-pyrrolo[3,2-g]quinazolin-7-one **(Compound 81)** | MS(ES+) m/z = 487.18 (M+1) $^1$H NMR (400 MHz, DMSO-$d_6$) δ 8.35 (s, 1H), 8.20 - 8.13 (m, 1H), 7.65 - 7.57 (m, 1H), 7.35 - 7.27 (m, 1H), 7.27 - 7.18 (m, 1H), 7.09 (s, 1H), 5.82 - 5.77 (m, 1H), 5.36 - 5.31 (m, 1H), 3.21 (s, 3H), 2.32 (s, 3H), 1.59 (d, *J* = 7.1 Hz, 3H), 1.39 (s, 3H), 1.38 (s, 3H), 1.23 (s, 3H), (1.22 (s, 3H). |
| 95 | 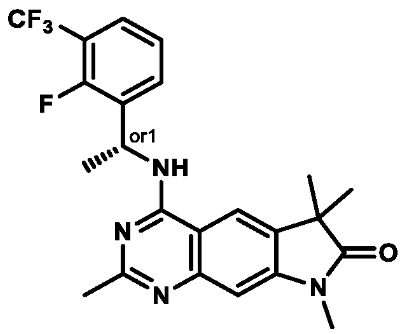 (R/S)-4-((1-(2-fluoro-3-(trifluoromethyl)phenyl)ethyl)amino)-2,6,6,8-tetramethyl-6H-pyrrolo[3,2-g]quinazolin-7(8H)-one **(Compound 82)** | MS(ES+) m/z = 447.17 (M+1) $^1$H NMR (400 MHz, DMSO-$d_6$) δ 8.34 (s, 1H), 8.27 - 8.20 (m, 1H), 7.86 - 7.77 (m, 1H), 7.68 - 7.60 (m, 1H), 7.41 - 7.32 (m, 1H), 7.09 (s, 1H), 5.81 - 5.69 (m, 1H), 3.21 (s, 3H), 2.31 (s, 3H), 1.63 (d, *J* = 7.0 Hz, 3H), 1.39 (s, 3H), 1.38 (s, 3H). |

(continued)

| Example | Chemical structure | LCMS and 1HNMR data |
|---|---|---|
| 96 | 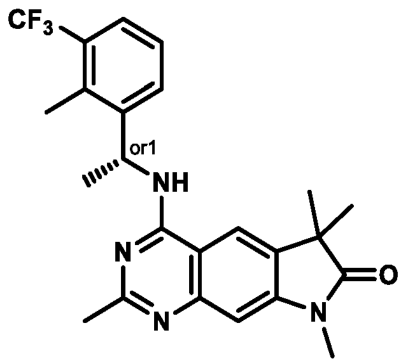 (R/S)-2,6,6,8-tetramethyl-4-((1-(2-methyl-3-(trifluoromethyl)phenyl)ethyl)amino)-6H-pyrrolo[3,2-g]quinazolin-7(8H)-one **(Compound 83)** | MS(ES+) m/z = 443.23 (M+1) $^1$H NMR (400 MHz, DMSO-$d_6$) δ 8.35 (s, 2H), 7.83 - 7.76 (m, 1H), 7.58 - 7.50 (m, 1H), 7.41 - 7.33 (m, 1H), 7.07 (s, 1H), 5.79 - 5.67 (m, 1H), 3.20 (s, 3H), 2.64 (s, 3H), 2.33 (s, 3H), 1.57 (d, *J* = 7.0 Hz, 3H), 1.39 (s, 6H). |
| 97 | 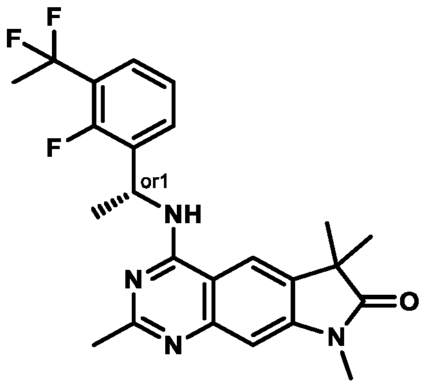 (R/S)-4-((1-(3-(1,1-difluoroethyl)-2-fluorophenyl)ethyl)amino)-2,6,6,8-tetramethyl-6H-pyrrolo[3,2-g]quinazolin-7(8H)-one **(Compound 84)** | MS(ES+) m/z = 443.23 (M+1) $^1$H NMR (400 MHz, DMSO-$d_6$) δ 8.35 (s, 1H), 8.21 - 8.14 (m, 1H), 7.69 - 7.60 (m, 1H), 7.48 - 7.39 (m, 1H), 7.30 - 7.21 (m, 1H), 7.09 (s, 1H), 5.85 - 5.73 (m, 1H), 3.21 (s, 3H), 2.33 (s, 3H), 2.04 (t, *J* = 19.1 *Hz,* 3H), 1.61 (d, *J* = 7.1 Hz, 3H), 1.39 (s, 3H), 1.38 (s, 3H). |
| 98a | 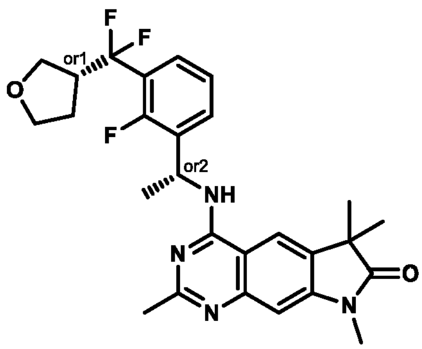 4-(((R/S)-1-(3-(difluoro((R/S)-tetrahydrofuran-3-yl)methyl)-2-fluorophenyl)ethyl) amino)-2,6,6,8-tetramethyl-6,8-dihydro-7H-pyrrolo[3,2-g]quinazolin-7-one **(Compound 85a)** | MS(ES+) m/z = 499.42 (M+1) $^1$H NMR (400 MHz, DMSO-$d_6$) δ 8.35 (s, 1H), 8.22 (s, 1H), 7.69 - 7.63 (m, 1H), 7.43 (m, 1H), 7.31 - 7.25 (m, 1H), 7.09 (s, 1H), 5.82 - 5.73 (m, 1H), 3.82 - 3.61 (m, 4H), 3.33 - 3.25 (m, 1H), 3.21 (s, 3H), 2.32 (s, 3H), 2.03 - 1.94 (m, 1H), 1.93 - 1.83 (m, 1H), 1.61 (d, *J* = 7.1 *Hz,* 3H), 1.47 - 1.31 (m, 6H). HPLC $t_{ret}$(min) = 6.99 |

(continued)

| Example | Chemical structure | LCMS and $^1$HNMR data |
|---|---|---|
| 98b | 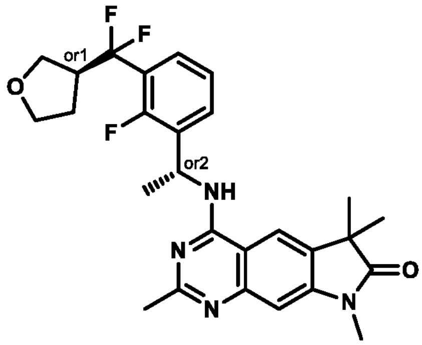 4-(((R/S)-1-(3-(difluoro((S/R)-tetrahydrofuran-3-yl)methyl)-2-fluorophenyl)ethyl) amino)-2,6,6,8-tetramethyl-6,8-dihydro-7H-pyrrolo[3,2-g]quinazolin-7-one **(Compound 85b)** | MS(ES+) m/z = 499.42 (M+1) $^1$H NMR (400 MHz, DMSO-$d_6$) δ 8.35 (s, 1H), 8.23 (s, 1H), 7.69 - 7.63 (m, 1H), 7.46 - 7.41 (m, 1H), 7.30 - 7.25 (m, 1H), 7.09 (s, 1H), 5.82 - 5.73 (m, 1H), 3.76 (m, 3H), 3.66 (m, 1H), 3.33 - 3.25 (m, 1H), 3.21 (s, 3H), 2.32 (s, 3H), 2.01 - 1.79 (m, 2H), 1.61 (d, *J* = 7.0 *Hz,* 3H), 1.45 - 1.31 (m, 6H). HPLC $t_{ret}$(min) = 8.29 |

**Example 99:** Preparation of (R)-4-((1-(3-(1,1-difluoro-2-hydroxy-2-methylpropyl)-2-fluorophenyl) ethyl)amino)-2-ethyl-6,6,8-trimethyl-6,8-dihydro-7H-pyrrolo[3,2-g]quinazolin-7-one **(Compound 86)**

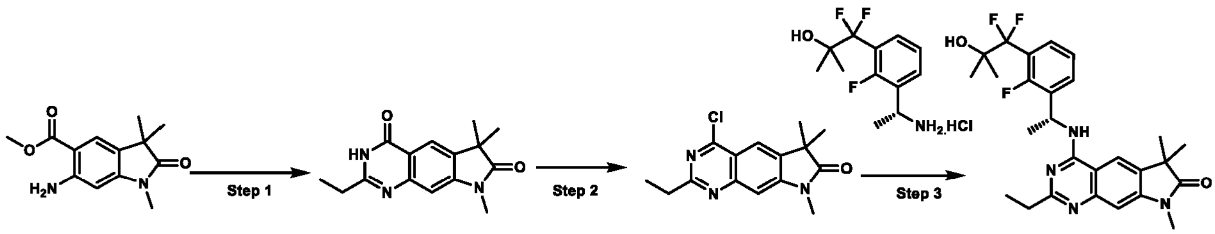


Step 1: 2-Ethyl-6,6,8-trimethyl-6,8-dihydro-3H-pyrrolo[3,2-g]quinazoline-4,7-dione

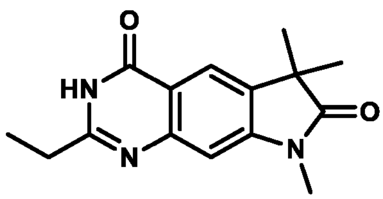

The titled compound was prepared from **Example-93-Step-7** intermediate using propionitrile by employing analogous protocol mentioned in **Example 71-Step-8** (0.45 g, 74.9 % yield).

MS(ES+) m/z = 272.0 (M+1).

Step 2: 4-Chloro-2-ethyl-6,6,8-trimethyl-6,8-dihydro-7H-pyrrolo[3,2-g]quinazolin-7-one

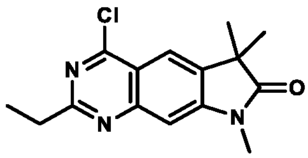

The titled compound was prepared from **Step-1** intermediate by employing analogous protocol mentioned in Example 71-Step-9 (0.40 g, 83 % yield).

MS(ES+) m/z = 290.27 (M+1).

Step-3: (R)-4-((1-(3-(1,1-difluoro-2-hydroxy-2-methylpropyl)-2-fluorophenyl)ethyl)amino)-2-ethyl-6,6,8-trimethyl-6,8-dihydro-7H-pyrrolo[3,2-g]quinazolin-7-one **(Compound 86)**

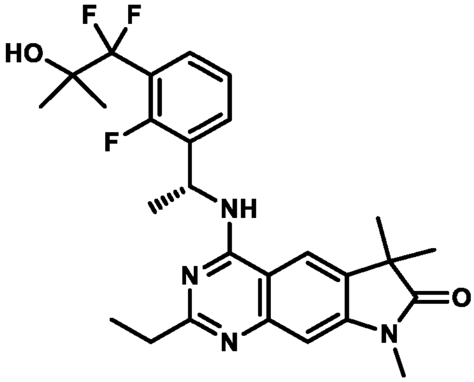

The titled compound was prepared by reaction of **Step-2** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.11 g, 42.5 % yield).

MS(ES+) m/z = 501.3 (M+1).

$^{1}$H NMR (400 MHz, DMSO-$d_6$) δ 8.36 (s, 1H), 8.17 (d, *J* = 7.1 Hz, 1H), 7.60 - 7.56 (m, 1H), 7.35 - 7.26 (m, 1H), 7.24 - 7.16 (m, 1H), 7.11 (s, 1H), 5.82 - 5.77 (m, 1H), 5.32 (s, 1H, exchangeable), 3.21 (s, 3H), 2.60 - 2.54 (m, 2H), 1.60 (d, *J* = 7.0 Hz, 3H), 1.47 - 1.36 (m, 6H), 1.24 (s, 3H), 1.20 (s, 3H) 1.10 (t, *J* = 7.5 *Hz,* 3H).

**Example 100:** Preparation of (R)-4-((1-(3-(1,1-difluoro-2-hydroxy-2-methylpropyl)-2-fluorophenyl)ethyl)amino)-2-isopropyl-6,6,8-trimethyl-6H-pyrrolo[3,2-g]quinazolin-7(8H)-one **(Compound 87)**

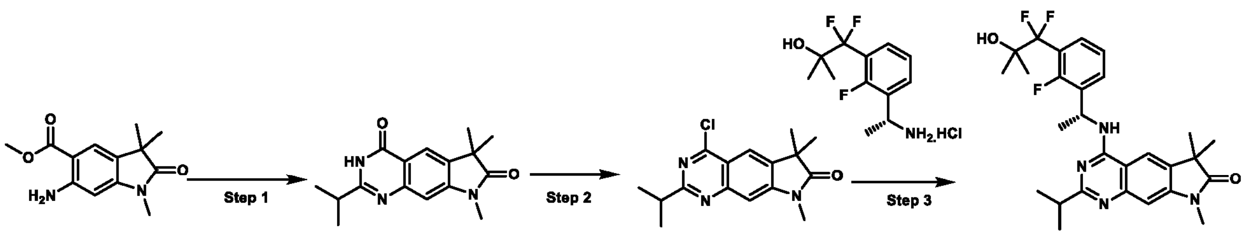


Step-1: 2-Isopropyl-6,6,8-trimethyl-3H-pyrrolo[3,2-g]quinazoline-4,7(6H,8H)-dione 10673-528

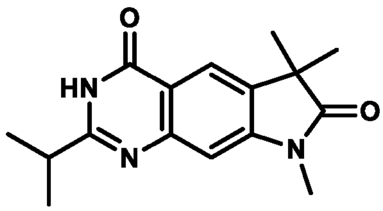

The titled compound was prepared from **Example-93 Step-7** intermediate using Isobutyronitrile by employing analogous protocol mentioned in **Example 71 Step-8** (0.39 g, 97 % yield).

MS(ES+) m/z = 286.34 (M+1).

Step 2: 4-Chloro-2-isopropyl-6,6,8-trimethyl-6H-pyrrolo[3,2-g]quinazolin-7(8H)-one 10673-530

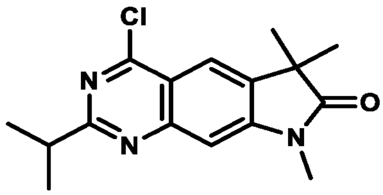

The titled compound was prepared from Step-1 intermediate by employing analogous protocol mentioned in **Example 71-Step-9** (0.40, 94 % yield).

MS(ES+) m/z = 304.40 (M+1).

**Step 3:** (R)-4-((1-(3-(1,1-difluoro-2-hydroxy-2-methylpropyl)-2-fluorophenyl)ethyl)amino)-2-isopropyl-6,6,8-trimethyl-6H-pyrrolo[3,2-g]quinazolin-7(8H)-one **(Compound 87)**

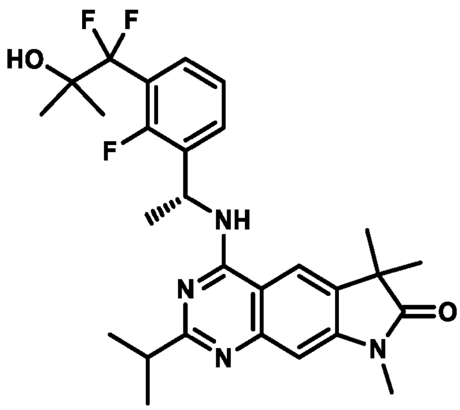

The titled compound was prepared by reaction of **Step-2** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.012 g, 14.17 % yield).

MS(ES+) m/z = 515.2 (M+1).

$^{1}$H NMR (400 MHz, DMSO-$d_6$) δ 8.36 (s, 1H), 8.17 (d, *J* = 6.7 *Hz,* 1H), 7.59 - 7.51 (m, 1H), 7.33 - 7.25 (m, 1H), 7.22 - 7.14 (m, 1H), 7.12 (s, 1H), 5.78 - 5.70 (m, 1H), 5.31 (s, 1H), 3.22 (s, 3H), 2.84 - 2.74 (m, 1H), 1.60 (d, *J* = 7.1 Hz, 3H), 1.43 - 1.36 (m, 6H) 1.24, (s, 3H), 1.20 (s, 3H) 1.12 (d, *J* = 6.9 Hz, 3H), 1.02 (d, *J* = 6.8 *Hz,* 3H).

**Example 101:** (R)-4-((1-(3-(1,1-difluoro-2-hydroxyethyl)-2-fluorophenyl)ethyl)amino)-8-ethyl-2,6,6-trimethyl-6,8-dihydro-7H-pyrrolo[3,2-g]quinazolin-7-one **(Compound 88)**

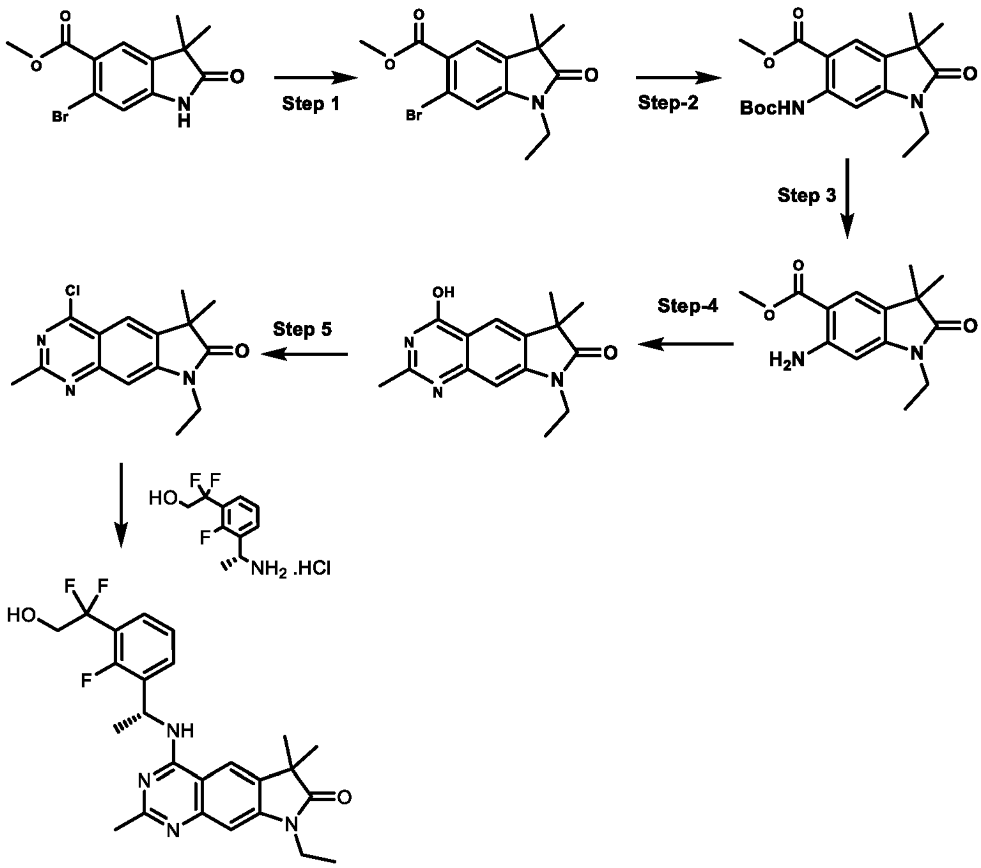


Step-1: Methyl 6-bromo-1-ethyl-3,3-dimethyl-2-oxoindoline-5-carboxylate

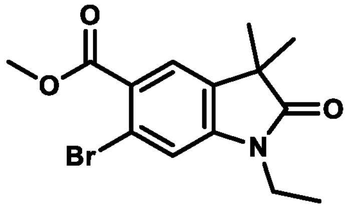

To a stirred solution of methyl 6-bromo-3,3-dimethyl-2-oxoindoline-5-carboxylate **Example 93 Step 4** (0.5 g, 1.67 mmol) in DMF (10.0 mL), $K_2CO_3$ (0.29 g, 2.09 mmol) and ethyl iodide (0.15 mL , 1.84 mmol) was added at 0°C. The reaction was stirred at room temperature for 15 h. The reaction was diluted with ice cold water and extracted with Ethyl acetate (2 x 25.0 mL). The organic layer was separated, dried over anhydrous $Na_2SO_4$ and concentrated in vacuum to get crude solid which was purified by flash chromatography by using 50% Ethyl acetate-n-hexane to afford the titled compound. (0.5 g, 91 % yield).

$^1$H NMR (400 MHz, DMSO-$d_6$) δ 7.83 (s, 1H), 7.50 (s, 1H), 3.84 (s, 3H), 3.74 (q, J = 7.1 *Hz,* 2H), 1.29 (s, 6H), 1.13 (t, J = *7.1 Hz,* 3H).

Step-2: Methyl 6-((tert-butoxycarbonyl)amino)-1-ethyl-3,3-dimethyl-2-oxoindoline-5-carboxylate

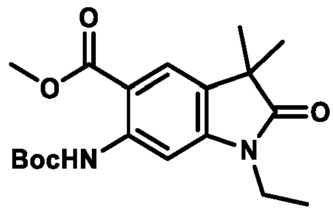

The titled compound was prepared from **Step-1** intermediate by employing analogous protocol mentioned in **Example 71-Step-6** (0.31g, 55.8% yield).
MS(ES+) m/z = 348.34 (M+1).

Step-3: Methyl 6-amino-1-ethyl-3,3-dimethyl-2-oxoindoline-5-carboxylate

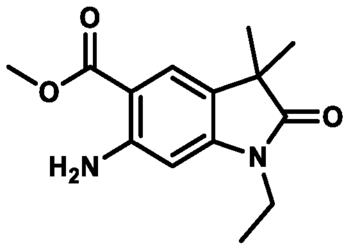

The titled compound was prepared from Step-2 intermediate by employing analogous protocol mentioned in **Example 71-Step-7** (0.22g, 86 % yield).
MS(ES+) m/z = 231.39 (M+1).

Step-4: 8-Ethyl-2,6,6-trimethyl-3H-pyrrolo[3,2-g]quinazoline-4,7(6H,8H)-dione

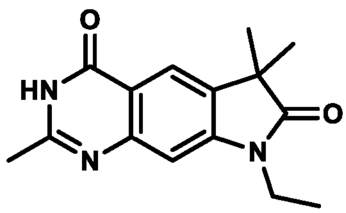

The titled compound was prepared from Step-3 intermediate by employing analogous protocol mentioned in **Example 71-Step-8** (0.20g, 73.4% yield).
MS(ES+) m/z = 272.27 (M+1).

Step-5: 4-Chloro-8-ethyl-2,6,6-trimethyl-6H-pyrrolo[3,2-g]quinazolin-7(8H)-one

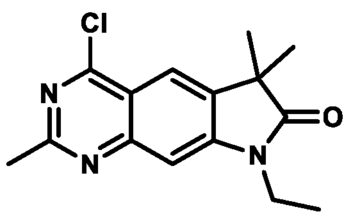

The titled compound was prepared from **Step-4** intermediate by employing analogous protocol mentioned in **Example 71-Step-9** (0.14g, 87 % yield).

MS(ES+) m/z = 289.21 (M+1).

Step-6: (R)-4-((1-(3-(1,1-difluoro-2-hydroxyethyl)-2-fluorophenyl)ethyl)amino)-8-ethyl-2,6,6-trimethyl-6,8-dihydro-7H-pyrrolo[3,2-g]quinazolin-7-one **(Compound 88)**

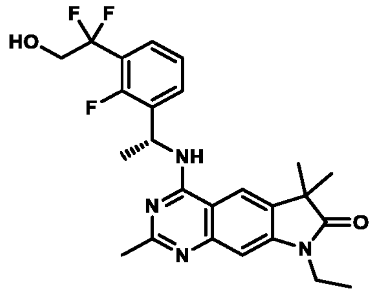

The titled compound was prepared from Step-5 intermediate by employing analogous protocol mentioned in Example-10 Step-10 (0.014 g, 13% yield)

MS(ES+) m/z = 473.23 (M+1).

$^1$H NMR (400 MHz, DMSO-$d_6$) δ 8.37 (s, 1H), 8.30 - 8.18 (m, 1H), 7.69 - 7.61 (m, 1H), 7.46 - 7.41 (m, 1H), 7.29 - 7.23 (m, 1H), 7.14 (s, 1H), 5.87 - 5.75 (m, 1H), 5.75 - 5.67 (m, 1H), 3.95 (t, *J*= 14.3 *Hz,* 2H), 3.78 (q, *J* = 7.1 *Hz,* 2H), 2.34 (s, 3H), 1.60 (d, *J* = 7.1 *Hz,* 3H), 1.40 - 1.37 (m, 6H) 1.18 (t, *J* = 7.1 *Hz,* 3H).

**Example 102:** Preparation of (R)-4-((1-(3-(1,1-difluoro-2-hydroxy-2-methylpropyl)-2-fluorophenyl) ethyl)amino)-8-ethyl-2,6,6-trimethyl-6H-pyrrolo[3,2-g]quinazolin-7(8H)-one **(Compound 89)**

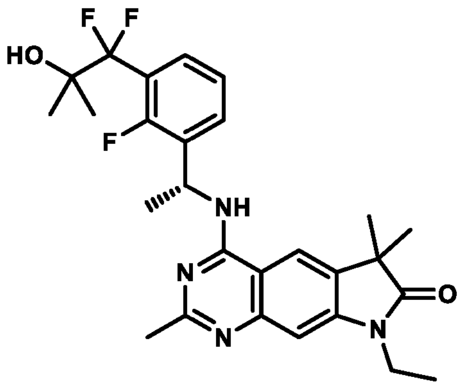

The titled compound was prepared by reaction of **Example 101-step-5** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71 Step-10** (0.007 g, 5.79 % yield)

MS(ES+) m/z = 501.19 (M+1).

$^1$H NMR (400 MHz, DMSO-$d_6$) δ 8.40 (s, 1H), 7.68 - 7.57 (m, 1H), 7.36 - 7.27 (m, 1H), 7.28 - 7.20 (m, 1H), 7.13 (s, 1H), 5.89 - 5.74 (m, 1H), 5.34 (s, 1H), 3.83 - 3.72 (m, 2H), 2.34 (s, 3H), 1.60 (d, *J* = 7.0 *Hz,* 3H), 1.40 - 1.37 (m, 3H), 1.25 (s, 3H), 1.23 (s, 3H), 1.22 - 1.18 (m, 3H).

**Example 103:** (R)-4-((1-(3-(1,1-difluoro-2-hydroxy-2-methylpropyl)-2-fluorophenyl)ethyl)amino)-2,6,6,8,9-pentamethyl-6,8-dihydro-7H-pyrrolo[3,2-g]quinazolin-7-one **(Compound 90)**

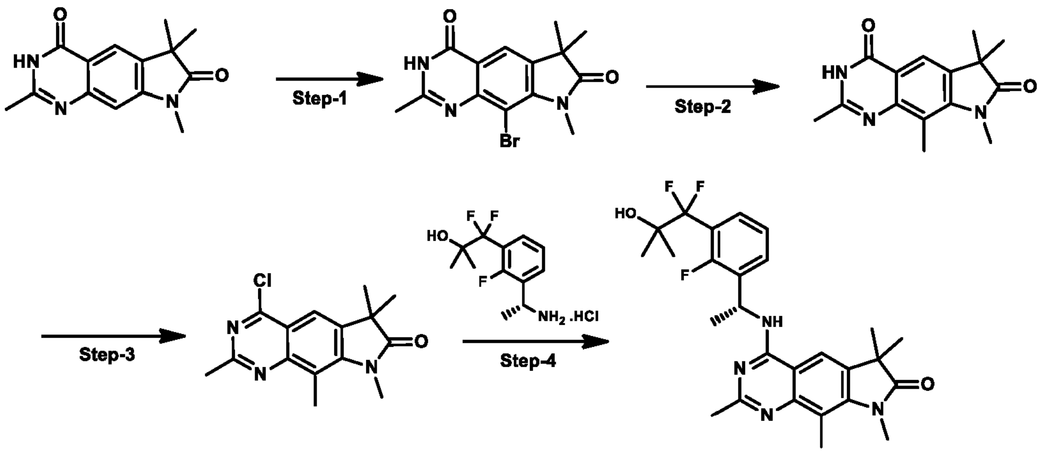


Step-1: 9-Bromo-2,6,6,8-tetramethyl-6,8-dihydro-3H-pyrrolo[3,2-g]quinazoline-4,7-dione.

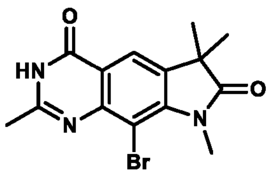

To a stirred solution of 2,6,6,8-tetramethyl-6,8-dihydro-3H-pyrrolo[3,2-g]quinazoline-4,7-dione (0.180 g, 0.700 mmol) **(Example 93 step 8)** in chloroform (10.0 mL) was added NBS (0.149 g, 0.840 mmol). The reaction mixture was stirred at 50-55°C for 4.5 h. The reaction mixture was diluted with DCM (20.0 mL) and washed with water (20.0 mL), brine (20.0 mL) and dried over anhydrous $Na_2SO_4$. The solvent was evaporated under reduced pressure and the crude product was purified by flash chromatography in DCM-MeOH gradient to afford the titled compound. (0.165 g, 70.2 %).

MS(ES+) m/z = 336.1 (M+).

Step-2: 2,6,6,8,9-Pentamethyl-6,8-dihydro-3H-pyrrolo[3,2-g]quinazoline-4,7-dione

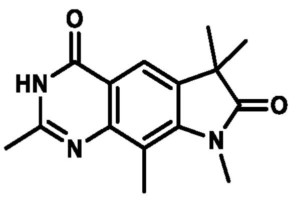

To a stirred solution of 9-bromo-2,6,6,8-tetramethyl-6,8-dihydro-3H-pyrrolo[3,2-g]quinazoline-4,7-dione (Step-1, 0.165 g, 0.491 mmol)) and methyl boronic acid (0.123 g, 2.061 mmol) in 1, 4-dioxane (10.0 mL) was degassed with $N_2$ gas for the 15 min. $PdCl_2$(dppf)-$CH_2Cl_2$ complex (0.040 g, 0.049 mmol) and $Na_2CO_3$ (0.218 g, 2.061 mmol) were added and reaction was stirred at 105°C for 30 h. The reaction mass was diluted with DCM (10.0 mL) and filtered through celite bed. Filtrate was washed with water (10.0 mL), brine (10.0 mL), dried over anhydrous $Na_2SO_4$. The solvent was evaporated, and residue was purified by flash chromatography to afford the titled compound. (0.082 g, 61.6 %).

MS(ES+) m/z = 272.2 (M+1).

Step-3: 4-Chloro-2,6,6,8,9-pentamethyl-6,8-dihydro-7H-pyrrolo[3,2-g]quinazolin-7-one

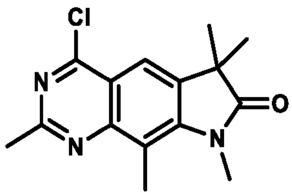

The titled compound was prepared from **Step-2** intermediate by employing analogous protocol mentioned in **Example 71-Step-9** (0.060 g, 80% yield).

MS(ES+) m/z = 289.65 $(M)^+$

Step-4: (R)-4-((1-(3-(1,1-difluoro-2-hydroxy-2-methylpropyl)-2-fluorophenyl)ethyl)amino)-2,6,6,8,9-pentamethyl-6,8-dihydro-7H-pyrrolo[3,2-g]quinazolin-7-one **(Compound 90)**

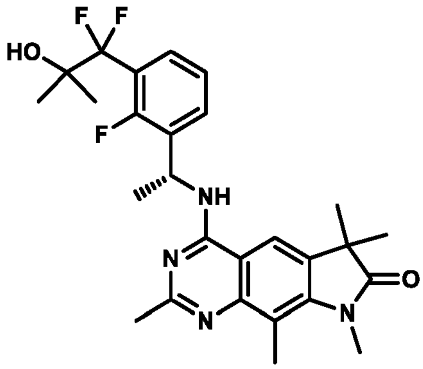

The titled compound was prepared by reaction of **Step-3** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.14 g, 47 % yield).

MS(ES+) m/z = 501.1 (M+1).

$^1H$ NMR (400 MHz, DMSO-$d_6$) δ 8.23 (s, 1H), 8.13 - 8.06 (m, 1H), 7.64 - 7.55 (m, 1H), 7.35 - 7.26 (m, 1H), 7.26 - 7.17 (m, 1H), 5.84 - 5.77 (m, 1H), 5.33 (s, 1H), 3.33 (s, 3H), 2.78 (s, 3H), 2.35 (s, 3H), 1.59 (d, *J* = 7.0 *Hz,* 3H),, 1.37 (s, 3H), 1.36 (s, 3H) 1.24 (s, 3H), 1.21 (s, 3H).

**Example 104:** (R)-4-((1-(3-(1,1-difluoro-2-hydroxy-2-methylpropyl)-2-fluorophenyl)ethyl)amino)-6,6-diethyl-2,8-dimethyl-6,8-dihydro-7H-pyrrolo[3,2-g]quinazolin-7-one **(Compound 91)**

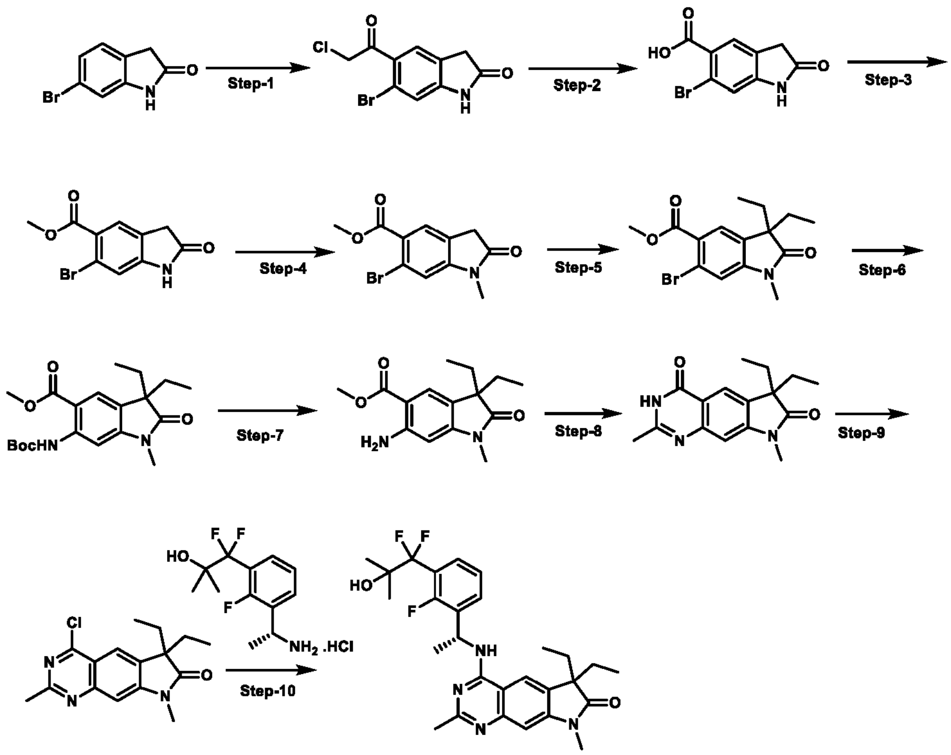


Step-1: 6-Bromo-5-(2-chloroacetyl)indolin-2-one

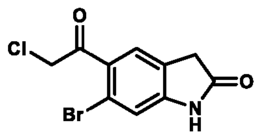

The titled compound was prepared from 6-bromoindolin-2-one by employing analogous protocol mentioned in **Example 71 Step-2** (38g, 93 % yield).

$^1$H NMR (400 MHz, DMSO- $d_6$) δ 10.80 (s, 1H), 7.71 (d, *J* = 1.4 Hz, 1H), 7.10 (s, 1H), 5.00 (s, 2H), 3.53 (s, 2H).

Step-2: 6-Bromo-2-oxoindoline-5-carboxylic acid

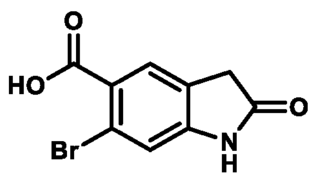

The titled compound was prepared from **Step-1** intermediate by employing analogous protocol mentioned in **Example 71 Step-3** (27g, 80% yield).

$^1$H NMR (400 MHz, DMSO- $d_6$) δ 12.93 (s, 1H), 10.74 (s, 1H), 7.68 (s, 1H), 7.07 (s, 1H), 3.51 (s, 2H). Step-3: Methyl 6-bromo-2-oxoindoline-5-carboxylate (10716-209)

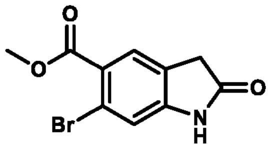

The titled compound was prepared from **Step-2** intermediate by employing analogous protocol mentioned in **Example 71 Step-4** (20g, 72% yield)

$^1H$ NMR (400 MHz, DMSO- $d_6$) δ 10.77 (s, 1H), 7.69 (d, J = 1.2 Hz, 1H), 7.09 (s, 1H), 3.81 (s, 3H), 3.52 (s, 2H).

Step-4: Methyl 6-bromo-1-methyl-2-oxoindoline-5-carboxylate

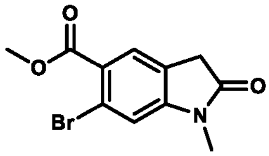

The titled compound was prepared from **Step-3** intermediate by employing analogous protocol mentioned in **Example 71 Step-5** (2.0, 76% yield).

$^1H$ NMR (400 MHz, DMSO- $d_6$) δ 7.71 (s, 1H), 7.39 (s, 1H), 3.83 (s, 3H), 3.58 (s, 2H), 3.15 (s, 3H).

Step-5: Methyl 6-bromo-3,3-diethyl-1-methyl-2-oxoindoline-5-carboxylate

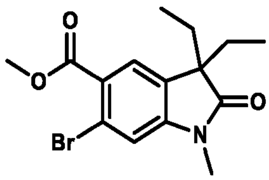

To a stirred solution of methyl 6-bromo-1-methyl-2-oxoindoline-5-carboxylate (0.54 g, 1.901 mmol) and in ethyl iodide (0.338 mL , 4.18 mmol) DMF (15.0 mL), sodium hydride (0.19 g, 4.75 mmol) was added portion wise at -10 °C. The reaction was stirred at same temperature for 1 h. and was diluted with water. Aqueous layer was extracted with Ethyl acetate (2 x 20.0 mL) and the combined organic layers were washed with brine, dried over anhydrous $Na_2SO_4$ and evaporated in vacuum to obtain crude product. The crude product was purified by flash chromatography in n-Hexane-Ethyl acetate gradient to afford the titled compound (0.55 g, 85 %).

$^1H$ NMR (400 MHz, DMSO- $d_6$) δ 7.74 (s, 1H), 7.46 (s, 1H), 3.84 (s, 3H), 3.18 (s, 3H), 1.92 - 1.69 (m, 4H), 0.52 - 0.37 (m, 6H).

Step-6: Methyl 6-((tert-butoxycarbonyl)amino)-3,3-diethyl-1-methyl-2-oxoindoline-5-carboxylate

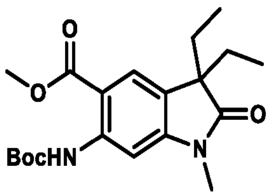

The titled compound was prepared from **Step-5** intermediate by employing analogous protocol mentioned in **Example 71 Step-6** (0.450 g, 58.1 % yield).

$^1H$ NMR (400 MHz, DMSO- $d_6$) δ 10.67 (s, 1H), 7.95 (s, 1H), 7.81 (s, 1H), 3.86 (s, 3H), 3.16 (s, 3H), 1.86 - 1.69 (m,

4H), 1.51 (s, 9H), 0.51 - 0.42 (m, 6H).

Step-7: Methyl 6-amino-3,3-diethyl-1-methyl-2-oxoindoline-5-carboxylate

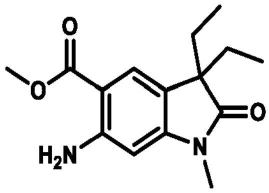

The titled compound was prepared from Step-6 intermediate by employing analogous protocol mentioned in Example 71 Step-7 (0.300 g, 91 % yield).
MS(ES+) m/z = 277.21 (M+1).

Step-8: 6,6-Diethyl-2,8-dimethyl-6,8-dihydro-3H-pyrrolo[3,2-g]quinazoline-4,7-dione

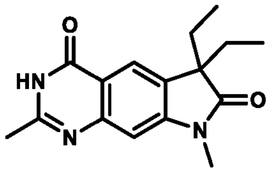

The titled compound was prepared from **Step-7** intermediate by employing analogous protocol mentioned in **Example 71 Step-8** (0.150 g, 97 % yield).
MS(ES+) m/z = 286.0 (M+1).

Step-9: 4-Chloro-6,6-diethyl-2,8-dimethyl-6,8-dihy-dro-7H-pyrrolo[3,2-g]quinazolin-7-one

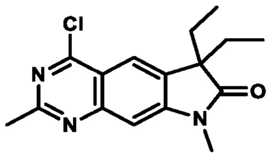

The titled compound was prepared from Step-8 intermediate by employing analogous protocol mentioned in **Example 71-Step-9** (Yield: 94 %).
MS(ES+) m/z = 304.2 (M+1).

Step-10: (R)-4-((1-(3-(1,1-difluoro-2-hydroxy-2-methylpropyl)-2-fluorophenyl)ethyl)amino)-6,6-diethyl-2,8-dimethyl-6,8-dihydro-7H-pyrrolo[3,2-g]quinazolin-7-one **(Compound 91)**

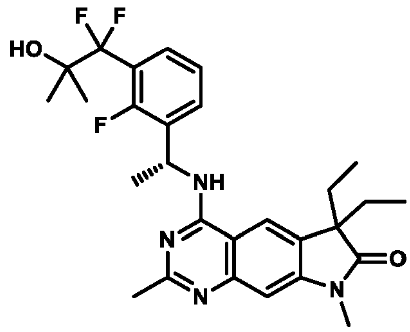

The titled compound was prepared by reaction of **Step-9** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.030 g, 11.81% yield).

MS(ES+) m/z = 514.19 (M+1).

$^1$H NMR (400 MHz, DMSO- $d_6$) δ 8.25 (s, 1H), 8.18 (d, $J$ = 7.3 Hz, 1H), 7.66 - 7.57 (m, 1H), 7.36 - 7.18 (m, 2H), 7.10 (s, 1H), 5.83 - 5.77 (m, 1H), 5.33 (s, 1H), 3.21 (s, 3H), 2.32 (s, 3H), 1.94 - 1.79 (m, 4H), 1.59 (d, $J$ = 7.0 Hz, 3H), 1.24 (s, 3H), 1.21 (s, 3H), 0.58 - 0.48 (m, 6H).

**Example 105** : Preparation of (R)-4-((1-(3-(1,1-difluoro-2-hydroxy-2-methylpropyl)-2-fluorophenyl)ethyl)amino)-6,6-bis(fluoromethyl)-2, 8-dimethyl-6,8-dihydro-7H-pyrrolo [3,2-g] quinazolin-7-one **(Compound 92)**

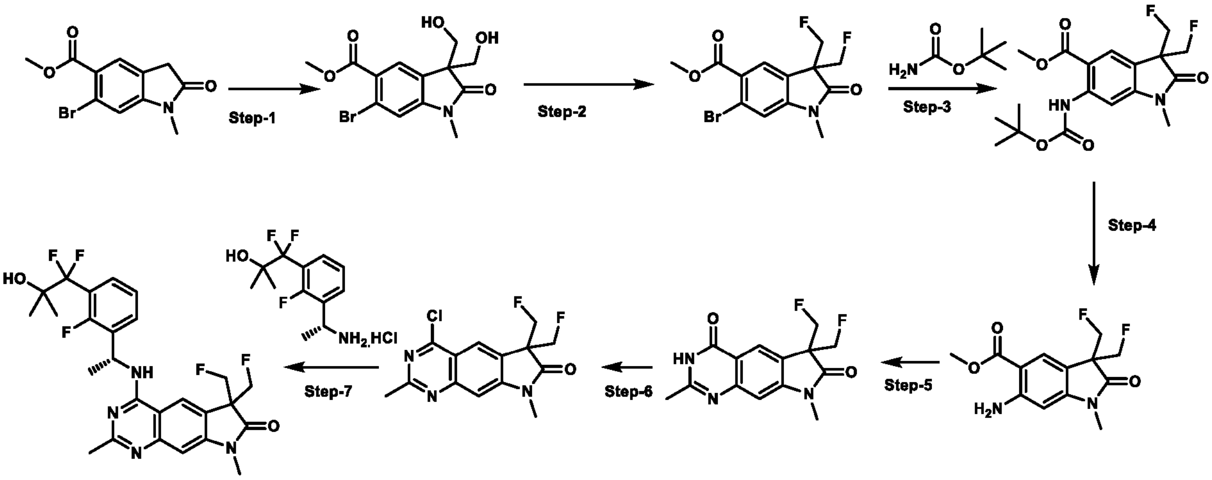


Step 1: Methyl 6-bromo-3,3-bis(hydroxymethyl)-1-methyl-2-oxoindoline-5-carboxylate

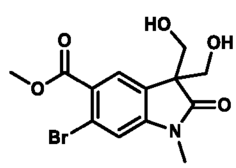

To a suspension of methyl 6-bromo-1-methyl-2-oxoindoline-5-carboxylate (Example 104, step-4, 0.30 g, 1.056 mmol) and $Na_2CO_3$ (0.022 g, 0.211 mmol) in 1,4-dioxane (5.0 mL), formaldehyde solution (aqueous 37%, 0.5 mL) was added and reaction was stirred at room temperature for 18 h and the reaction mixture was filtered. The filtrate was concentrated in vacuum, and the residue was purified by flash chromatography in hexane-Ethyl acetate gradient to afford the titled compound (0.25 g, 68.8% Yield).

MS(ES+) m/z = 346.28 (M+2).

Step 2: Methyl 6-bromo-3,3-bis(fluoromethyl)-1-methyl-2-oxoindoline-5-carboxylate.

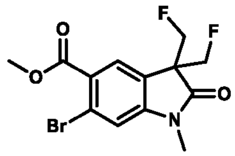

To a stirred solution of methyl 6-bromo-3,3-bis(hydroxymethyl)-1-methyl-2-oxoindoline-5-carboxylate (0.20 g, 0.581 mmol) in DCM (5.0 mL) was slowly added DAST (0.384 mL , 2.91 mmol) at -78 °C and then stirred it at room temperature for 18 h. Reaction mixture was quenched with ice cold water and it was extracted with DCM (50.0 mL x 2). Combined organic layer was washed with brine (50.0 mL), dried over anhydrous $Na_2SO_4$ and concentrated under the reduced pressure. The crude product was purified by flash chromatography to afford the titled compound (0.1 g, 49.4% yield).

MS(ES+) m/z = 348.28 (M+).

Step 3: Methyl 6-((tert-butoxycarbonyl)amino)-3,3-bis(fluoromethyl)-1-methyl-2-oxoindoline-5-carboxylate.

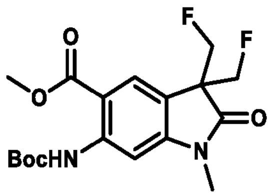

The titled compound was prepared from **Step-2** intermediate by employing analogous protocol mentioned in **Example 71-Step-6** (0.080 g, 72.5 % yield)

MS(ES+) m/z = 285.21 (M-100), 384.54 (M+).

Step 4: Methyl 6-amino-3,3-bis(fluoromethyl)-1-methyl-2-oxoindoline-5-carboxylate

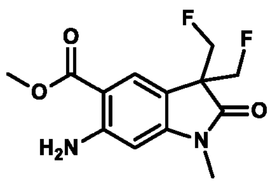

The titled compound was prepared from **Step-3** intermediate by employing analogous protocol mentioned in **Example 71-Step-7** (0.060 g, 90 % yield).

MS(ES+) m/z = 285.21 (M+1).

Step 5: 6,6-Bis(fluoromethyl)-2,8-dimethyl-6,8-dihydro-3H-pyrrolo[3,2-g]quinazoline-4,7-dione

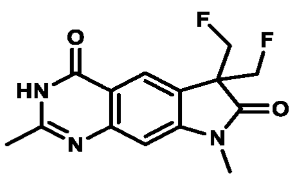

The titled compound was prepared from **Step-4** intermediate by employing analogous protocol mentioned in **Example 71-Step-8** (0.015 g, 91% yield)
MS(ES+) m/z = 294.21 (M+1)

Step 6: 4-Chloro-6,6-bis(fluoromethyl)-2,8-dimethyl-6,8-dihydro-7H-pyrrolo[3,2-g]quinazolin-7-one

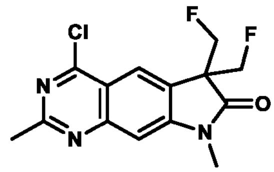

The titled compound was prepared from **Step-5** intermediate by employing analogous protocol mentioned in **Example 71-Step-9** (0.015 g, 94% yield)
MS(ES+) m/z = 312.15 (M+1).

Step 7: (R)-4-((1-(3-(1,1-difluoro-2-hydroxy-2-methylpropyl)-2-fluorophenyl)ethyl)amino)-6,6-bis(fluoromethyl)-2,8-dimethyl-6,8-dihydro-7H-pyrrolo[3,2-g]quinazolin-7-one (Compound 92)

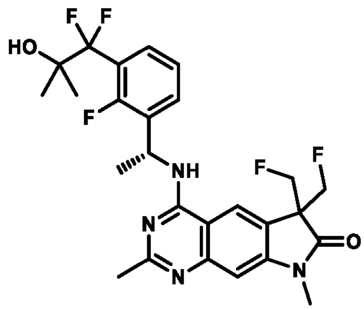

The titled compound was prepared by reaction of **Step-6** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.010 g, 11.93 % yield).
MS(ES+) m/z = 523.32 (M+1).
$^1$H NMR (400 MHz, DMSO-$d_6$) δ 8.51 (s, 1H), 8.32 (d, *J* = 7.3 *Hz,* 1H), 7.65 - 7.58 (m, 1H), 7.36 - 7.29 (m, 1H), 7.23 (m, 1H), 7.18 (s, 1H), 5.83 - 5.78 (m, 1H), 5.34 (s, 1H), 4.91 (s, 2H), 4.79 (s, 2H), 3.24 (s, 3H), 2.33 (s, 3H), 1.59 (d, *J*= 7.1 *Hz,* 3H), 1.24 (s, 3H), 1.22 (s, 3H).

**Example 106:** Preparation of (R&S)-4-(((R)-1-(3-(1,1-difluoro-2-hydroxy-2-methylpropyl)-2-fluorophenyl) ethyl)amino)-6-ethyl-2,6,8-trimethyl-6,8-dihydro-7H-pyrrolo[3,2-g]quinazolin-7-one

**(Compound 93a and Compound 93b)**

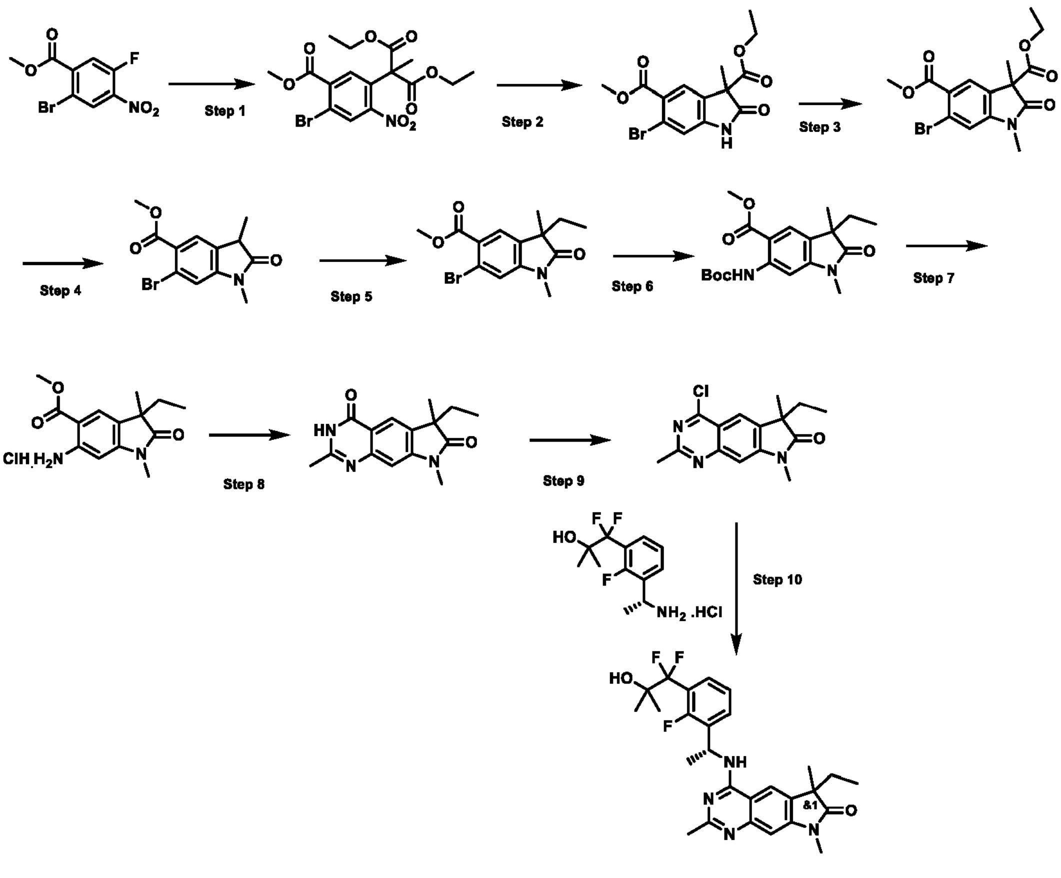


Compound 93a and 93b

Step 1: Diethyl 2-(4-bromo-5-(methoxycarbonyl)-2-nitrophenyl)-2-methylmalonate

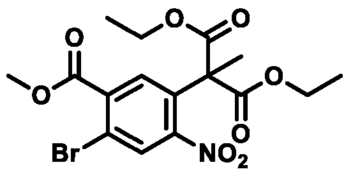

The titled compound was prepared from commercially available methyl 2-bromo-5-fluoro-4-nitrobenzoate by employing analogous protocol mentioned in Example 76-Step-1. (3.7g, 47.6 % yield).

$^1H$ NMR (400 MHz, $CDCl_3$) δ 8.31 (s, 1H), 7.82 (s, 1H), 4.31 - 4.21 (m, 4H), 4.00 (s, 3H), 2.03 (s, 3H), 1.30 - 1.20 (m, 6H)

Step 2: 3-Ethyl 5-methyl 6-bromo-3-methyl-2-oxoindoline-3,5-dicarboxylate

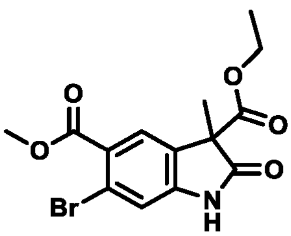

The titled compound was prepared from Step-1 intermediate by employing analogous protocol mentioned in Example 76- Step-4 (2.6 g, 85 % yield).
$^1H$ NMR (400 MHz, DMSO- $d_6$) δ 11.13 (s, 1H), 7.70 (s, 1H), 7.20 (s, 1H), 4.20 - 4.02 (m, 2H), 3.82 (s, 3H), 1.54 (s, 3H), 1.07 (t, J = 7.1 Hz, 3H)

Step 3: 3-Ethyl 5-methyl 6-bromo-1,3-dimethyl-2-oxoindoline-3,5-dicarboxylate

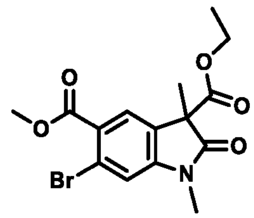

The titled compound was prepared from **Step-2** intermediate by employing analogous protocol mentioned in **Example 71-Step-5** (1.1 g, 81 % yield)
$^1H$ NMR (400 MHz, DMSO- $d_6$) δ 7.73 (s, 1H), 7.54 (s, 1H), 4.18 - 3.98 (m, 2H), 3.83 (s, 3H), 3.22 (s, 3H), 1.56 (s, 3H), 1.07 (t, J = 7.1 Hz, 3H).

Step 4: Methyl 6-bromo-1,3-dimethyl-2-oxoindoline-5-carboxylate

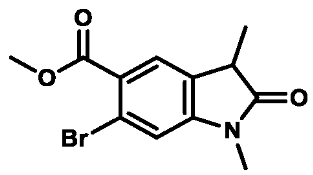

To a stirred solution of 3-ethyl 5-methyl 6-bromo-1,3-dimethyl-2-oxoindoline-3,5-dicarboxylate **(Step 3)**(3.0 g, 8.10 mmol) in TFA (6.24 mL , 81.0 mmol), $H_2SO_4$ (30% aq., 14.40 mL , 81 mmol) was added and the reaction was stirred at 60°C for 6h. The reaction mixture was poured in ice water and extracted with Ethyl acetate (2 x 150.0 mL). The combined organic layer was washed with brine (60.0 mL), dried over anhydrous $Na_2SO_4$ and concentrate in vacuum to get crude product. The crude product was purified by flash chromatography using Ethyl acetate- n- hexane gradient to afford the titled compound (2.3 g, 95 %)
$^1H$ NMR (400 MHz, DMSO-$d_6$) δ 7.76 (d, *J* = 1.2 Hz, 1H), 7.40 (s, 1H), 3.83 (s, 3H), 3.60 - 3.48 (m, 1H), 3.15 (s, 3H), 1.36 (d, *J* = 7.6 Hz, 3H).

Step 5: Methyl 6-bromo-3-ethyl-1,3-dimethyl-2-oxoindoline-5-carboxylate

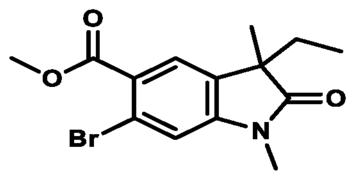

To a stirred solution of methyl 6-bromo-1,3-dimethyl-2-oxoindoline-5-carboxylate (0.40 g, 1.342 mmol) **(Step 4)** in DMF (5.0 mL), NaH (0.064 g, 1.610 mmol), followed by addition of ethyl iodide (0.130 mL , 1.610 mmol) were added at 0°C. The reaction was stirred at 0°C for 30 min. The reaction mixture was poured in ice water and extracted with Ethyl acetate (2 x 150.0 mL). The combined organic layer was washed with brine (60.0 mL), dried over anhydrous $Na_2SO_4$ and concentrate in vacuum to get crude product. The crude product was purified by flash chromatography using Ethyl acetate - n-hexane gradient to afford the titled compound (0.38 g, 87 %).

MS(ES+) m/z = 367.28 (M+41).

Step 6: Methyl 6-((tert-butoxycarbonyl)amino)-3-ethyl-1,3-dimethyl-2-oxoindoline-5-carboxylate

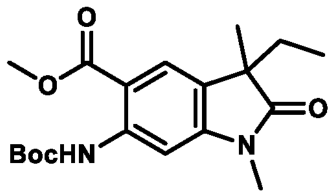

The titled compound was prepared from Step-5 intermediate by employing analogous protocol mentioned in **Example 71-Step-6** (0.35 g, 83% yield).
MS(ES+) m/z = 363.2 (M+1)

Step 7: Methyl 6-amino-3-ethyl-1,3-dimethyl-2-oxoindoline-5-carboxylate

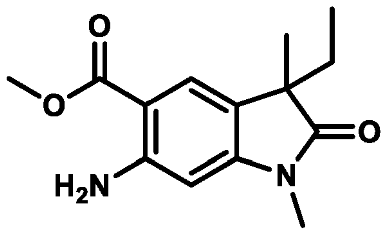

The titled compound was prepared from **Step-6** intermediate by employing analogous protocol mentioned in **Example 71-Step-7** (0.28 g, 97% yield).
$^1$H NMR (400 MHz, DMSO-$d_6$) δ 7.53 (s, 1H), 6.34 (s, 1H), 3.76 (s, 3H), 3.08 (s, 3H), 1.78 - 1.66 (m, 2H), 1.21 (s, 3H), 0.49 (t, *J*= 7.3 *Hz,* 3H).

Step 8: 6-Ethyl-2,6,8-trimethyl-6,8-dihydro-3H-pyrrolo[3,2-g]quinazoline-4,7-dione

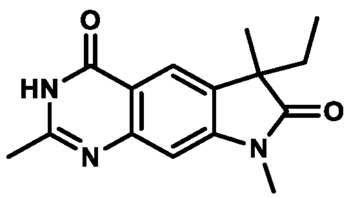

The titled compound was prepared from **Step-7** intermediate by employing analogous protocol mentioned in **Example 71-Step-8** (0.20 g, 79 % yield).
$^1$H NMR (400 MHz, DMSO-$d_6$) δ 12.12 (s, 1H), 7.94 (s, 1H), 7.12 (s, 1H), 3.21 (s, 3H), 2.34 (s, 3H), 1.92 - 1.75 (m, 2H), 1.32 (s, 3H), 0.49 (t, *J* = 7.4 *Hz,* 3H).

Step-9: 4-Chloro-6-ethyl-2,6,8-trimethyl-6,8-dihydro-7H-pyrrolo[3,2-g]quinazolin-7-one

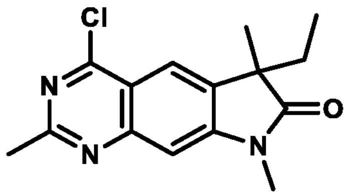

The titled compound was prepared from **Step-8** intermediate by employing analogous protocol mentioned in **Example 71-Step-9** (0.20 g, 94% yield).

MS(ES+) m/z = 290.1 (M+1).

Step-10: (R&S)-4-(((R)-1-(3-(1,1-difluoro-2-hydroxy-2-methylpropyl)-2-luorophenyl)ethyl)amino)-6-ethyl-2,6,8-trimethyl-6,8-dihydro-7H-pyrrolo[3,2-g]quinazolin-7-one

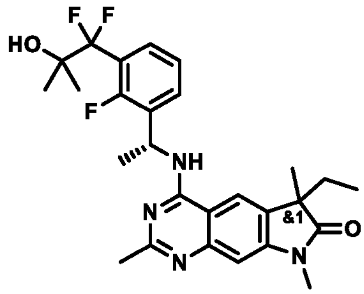

The titled compound was prepared by reaction of **Step-9** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.11 g, 31% yield)

MS(ES+) m/z = 501.31 (M+1).

This compound was further subjected to chiral preparative HPLC and two stereoisomers were separated and characterized as follows:

**Chiral séparation method** : CHIRALPAK OX-H CRL-081HEX_0.1%DEA_IPA-MEOH_80_20_B_A_1.2 ML_10MIN_264NM 1.20 mL /min

Peak-1 : (S/R)-4-(((R)-1-(3-(1,1-difluoro-2-hydroxy-2-methylpropyl)-2-fluorophenyl)ethyl)amino)-6-ethyl-2,6,8-trimethyl-6,8-dihydro-7H-pyrrolo[3,2-g]quinazolin-7-one **(Compound 93a)**

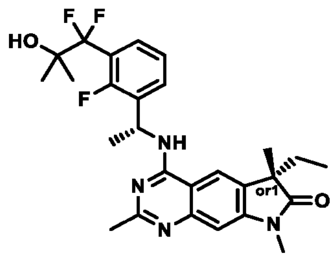

MS(ES+) m/z = 501.31 (M+1).
$t_{ret}$(min) = 6.53

$^1H$ NMR (400 MHz, DMSO-$d_6$) δ 8.30 (s, 1H), 8.18 (d, *J*= 7.3 Hz, 1H), 7.65 - 7.55 (m, 1H), 7.37 - 7.27 (m, 1H), 7.25 - 7.19 (m, 1H), 7.09 (s, 1H), 5.86 - 5.74 (m, 1H), 5.33 (s, 1H), 3.21 (s, 3H), 2.32 (s, 3H), 1.94 - 1.79 (m, 2H), 1.59 (d, *J*= 7.0 *Hz,* 3H), 1.38 (s, 3H), 1.24 (s, 3H), 1.21 (s, 3H), 0.54 (t, *J*= 7.3 *Hz,* 3H).

**Peak-2** (R/S)-4-(((R)-1-(3-(1,1-difluoro-2-hydroxy-2-methylpropyl)-2-fluorophenyl)ethyl)amino)-6-ethyl - 2,6,8-trimethyl-6,8-dihydro-7H-pyrrolo[3,2-g]quinazolin-7-one **(Compound 93b)**

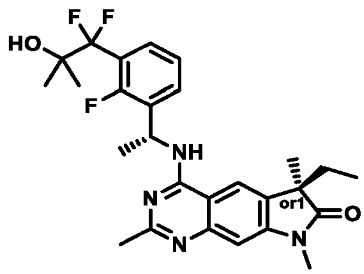

MS(ES+) m/z = 501.31 (M+1).
$t_{ret}$(min) = 7.70

$^1$H NMR (400 MHz, DMSO- $d_6$) δ 8.30 (s, 1H), 8.20 (s, 1H), 7.67 - 7.58 (m, 1H), 7.34 - 7.29 (m, 1H), 7.27 - 7.20 (m, 1H), 7.09 (s, 1H), 5.84 - 5.74 (m, 1H), 5.33 (s, 1H), 3.21 (s, 3H), 2.33 (s, 3H), 1.92 - 1.80 (m, 2H), 1.59 (d, *J* = 7.0 Hz, 3H), 1.37 (s, 3H), 1.24 (s, 3H), 1.21 (s, 3H), 0.56 (t, *J* = 7.3 *Hz,* 3H).

**Example 107:** Preparation of (R&S)- 4-(((R)-1-(3-(1,1-difluoro-2-hydroxy-2-methylpropyl)-2-fluorophenyl) ethyl)amino) -6-(methoxymethyl)-2,6,8-trimethyl-6H-pyrrolo[3,2-g]quinazolin-7(8H)-one **(Compound 94)**

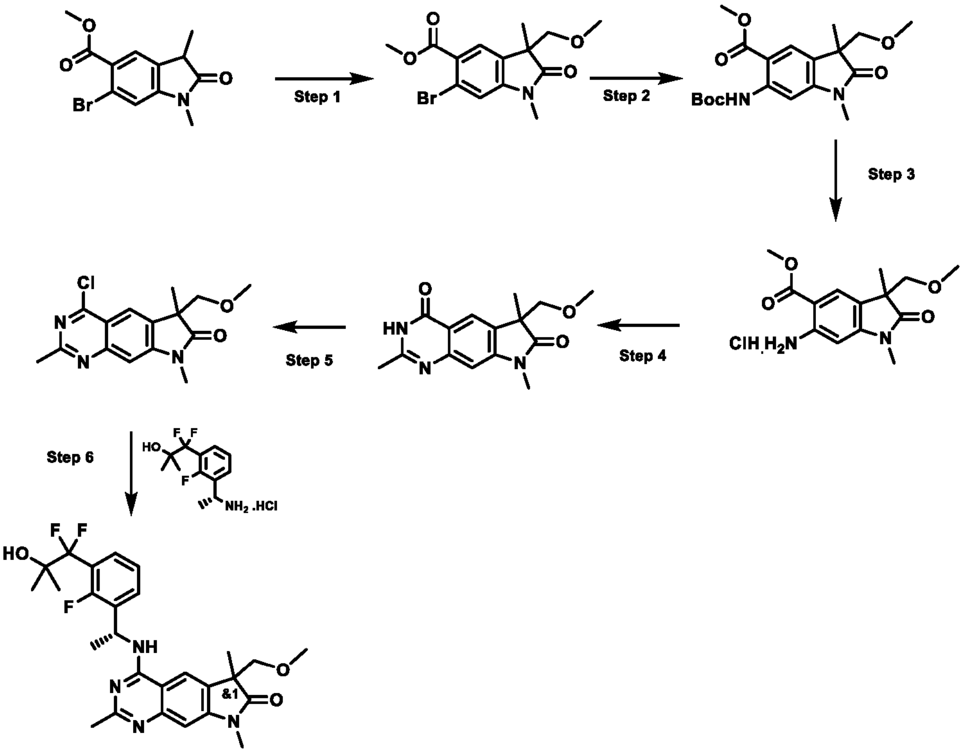


Step 1: Methyl 6-bromo-3-(methoxymethyl)-1,3-dimethyl-2-oxoindoline-5-carboxylate

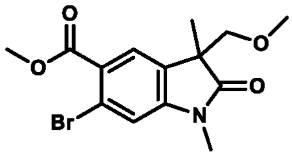

The titled compound was prepared from Example **106-Step-4** intermediate using chloro(methoxy)methane by employing analogous protocol mentioned in **Example-106-Step-7** (0.22 g, 63.9 % yield).
MS(ES+) m/z = 342.22(M+), 344.22(M+2).

Step 2: Methyl 6-((tert-butoxycarbonyl)amino)-3-(methoxymethyl)-1,3-dimethyl-2-oxoindoline-5-carboxylate

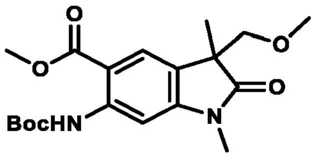

The titled compound was prepared from Step-1 intermediate by employing analogous protocol mentioned in Example 71-Step-6 (0.15 g, 61.7 % yield).
MS(ES+) m/z = 379.28 (M+1).

Step 3: Methyl 6-amino-3-(methoxymethyl)-1,3-dimethyl-2-oxoindoline-5-carboxylate

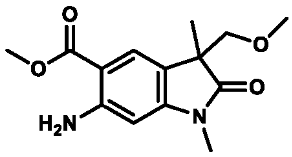

The titled compound was prepared from **Step-2** intermediate by employing analogous protocol mentioned in **Example 71-Step-7** (0.12 g, 96 % yield).
MS(ES+) m/z = 279.21 (M+1).

Step 4: 6-(Methoxymethyl)-2,6,8-trimethyl-6,8-dihydro-3H-pyrrolo[3,2-g]quinazoline-4,7-dione

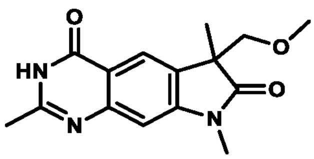

The titled compound was prepared from **Step-3** intermediate by employing analogous protocol mentioned in **Example 71-Step-8** (0.10 g, 91 % yield).
MS(ES+) m/z = 287.96 (M+1).

Step 5: 4-Chloro-6-(methoxymethyl)-2,6,8-trimethyl-6,8-dihydro-7H-pyrrolo[3,2-g]quinazolin-7-one

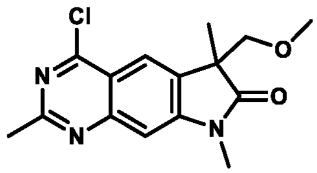

The titled compound was prepared from Step-4 intermediate by employing analogous protocol mentioned in **Example 71-Step-9** (0.10 g, 94 % yield).

MS(ES+) m/z = 306.28 (M+1).

Step 6: 4-(((R)-1-(3-(1,1-difluoro-2-hydroxy-2-methylpropyl)-2-fluorophenyl)ethyl)amino)-6-(methoxy methyl)-2,6,8-trimethyl-6,8-dihydro-7H-pyrrolo[3,2-g]quinazolin-7-one

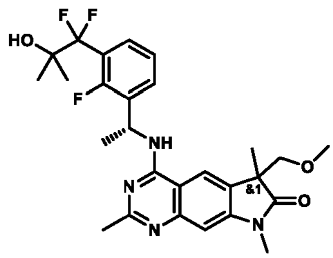

The titled compound was prepared by reaction of **Step-5** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.055 g, 45 % Yield)

MS(ES+) m/z = 517.24 (M+1).

The purified compound was subjected to chiral column séparation and two stereo isomers were isolated as by chiral préparative HPLC.

**Chiral séparation method** : CHIRALPAK OX-H CRL-081HEX_0.1%DEA_IPA-MEOH_80_20_B_A_1.2 ML_10MIN_264NM 1.20 mL /min

**Peak 1:** (R/S)-4-(((R)-1-(3-(1,1-difluoro-2-hydroxy-2-methylpropyl)-2-fluorophenyl)ethyl)amino)-6-(methoxymethyl)-2,6,8-trimethyl-6,8-dihydro-7H-pyrrolo[3,2-g]quinazolin-7-one **(Compound 94a)**

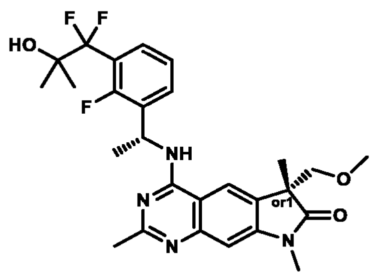

MS(ES+) m/z = 517.07 (M+1).
Peak-1, $t_{ret}$(min) = 5.42

$^1$H NMR (400 MHz, DMSO-$d_6$) δ 8.33 (s, 1H), 8.18 (d, *J* = 7.4 Hz, 1H), 7.65 - 7.56 (m, 1H), 7.35 - 7.26 (m, 1H), 7.26 - 7.17 (m, 1H), 7.06 (s, 1H), 5.84 - 5.74 (m, 1H), 5.33 (s, 1H), 3.68 (s, 2H), 3.19 (s, 3H), 3.12 (s, 3H), 2.31 (s, 3H), 1.60 (d, *J*= 7.0 *Hz,* 3H), 1.31 (s, 3H), 1.24 (s, 3H), 1.21 (s, 3H)

**Peak-2:** (S/R)-4-(((R)-1-(3-(1,1-difluoro-2-hydroxy-2-methylpropyl)-2-fluorophenyl)ethyl)amino)-6-(methoxymethyl)-2,6,8-trimethyl-6,8-dihydro-7H-pyrrolo[3,2-g]quinazolin-7-one **(Compound 94b)**

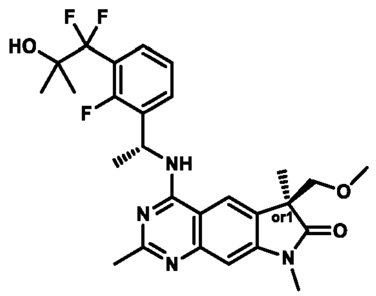

MS(ES+) m/z = 517.24 (M+1).
Peak-2, $t_{ret}$(min) = 5.92

$^1$H NMR (400 MHz, DMSO- $d_6$) δ 8.32 (s, 1H), 8.16 (d, *J*= 7.3 *Hz,* 1H), 7.65 - 7.60 (m, 1H), 7.35 - 7.29 (m, 1H), 7.26 - 7.21 (m, 1H), 7.06 (s, 1H), 5.83 - 5.77 (m, 1H), 5.33 (s, 1H), 3.69 (s, 2H), 3.20 (s, 3H), 3.12 (s, 3H), 2.32 (s, 3H), 1.59 (d, *J* = 7.0 *Hz,* 3H), 1.30 (s, 3H), 1.24 (s, 3H), 1.21(s, 3H)

**Example 108**:Preparation of (R&S)-4-(((R)-1-(3-(1,1-difluoro-2-hydroxy-2-methylpropyl)-2-fluorophenyl)ethyl) amino)-2,6,8-trimethyl-6,8-dihydro-7H-pyrrolo[3,2-g]quinazolin-7-one **(Compound 95)**

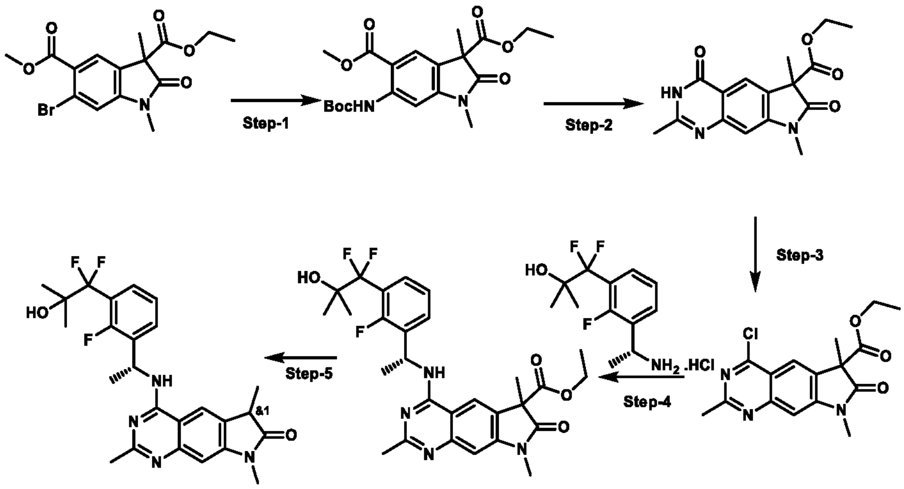


Step 1: 3-Ethyl 5-methyl 6-((tert-butoxycarbonyl)amino)-1,3-dimethyl-2-oxoindoline-3,5-dicarboxylate

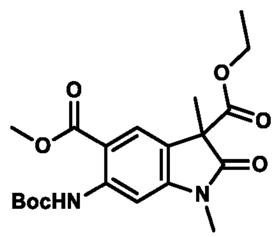

The titled compound was prepared from **Example-106-Step-3** intermediate by employing analogous protocol

mentioned in **Example 71-Step-6** (2.25 g, 91 % yield).

$^1$H NMR (400 MHz, DMSO-$d_6$) δ 10.66 (s, 1H), 7.98 (s, 1H), 7.80 (s, 1H), 4.17 - 4.00 (m, 2H), 3.85 (s, 3H), 3.20 (s, 3H), 1.51 (s, 9H),1.37 (s, 3H), 1.06 (t, J = 7.1 *Hz,* 3H).

Step 2 Ethyl 2,6,8-trimethyl-4,7-dioxo-4,6,7,8-tetrahydro-3H-pyrrolo[3,2-g]quinazoline-6-carboxylate

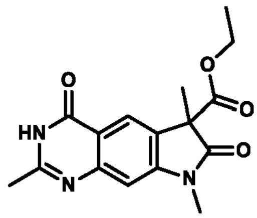

The titled compound was prepared from Step-1 intermediate by employing analogous protocol mentioned in Example 71-Step-8 (0.48 g, 30.9%).Here Boc protected compound was subjected to cyclization in acidic medium.

MS(ES+) m/z = 316.34 (M+1).

Step 3: Ethyl 4-chloro-2,6,8-trimethyl-7-oxo-7,8-dihydro-6H-pyrrolo[3,2-g]quinazoline-6-carboxylate

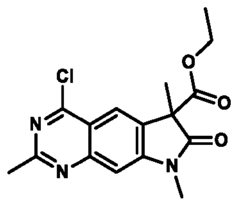

The titled compound was prepared from Step-2 intermediate by employing analogous protocol mentioned in **Example 71-Step-9** (0.172 g, 96%).

MS(ES+) m/z = 334.40 (M+1).

Step 4: Ethyl 4-(((R)-1-(3-(1,1-difluoro-2-hydroxy-2-methylpropyl)-2-fluorophenyl)ethyl)amino)-2,6,8-trimethyl-7-oxo-7,8-dihydro-6H-pyrrolo[3,2-g]quinazoline-6-carboxylate.

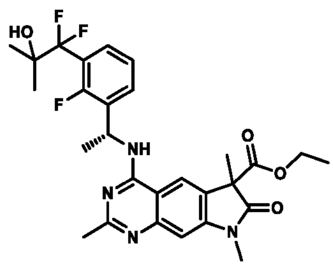

The titled compound was prepared by reaction of Step - 3 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Step-10 of Example** 71 (0.073 g, 26.0 % yield).

MS(ES+) m/z = 545.20 (M+1).

Step 5: (R&S)-4-(((R)-1-(3-(1,1-difluoro-2-hydroxy-2-methylpropyl)-2-fluorophenyl)ethyl) amino)-2,6,8-trimethyl-6,8-dihydro-7H-pyrrolo[3,2-g]quinazolin-7-one **(Compound 95)**

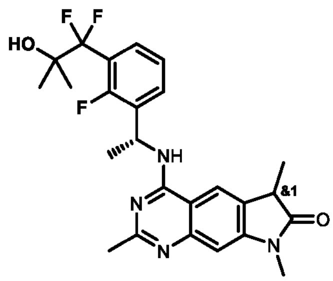

Titled compound was prepared from Step-4 intermediate by employing analogous protocol mentioned in Example **106-Step-4** (0.080 g, 92%)

MS(ES+) m/z = 473.36 (M+1).

$^1$H NMR (400 MHz, DMSO-$d_6$) δ 8.33 (s, 1H), 8.24 - 8.17 (m, 1H), 7.65 - 7.56 (m, 1H), 7.35 - 7.26 (m, 1H), 7.26 - 7.16 (m, 1H), 7.05 (s, 1H), 5.87 - 5.77 (m, 1H), 5.36 - 5.31 (m, 1H), 3.70 (q, *J* = 7.5 *Hz,* 1H), 3.19 (s, 3H), 2.32 (s, 3H), 1.58 (d, *J* = 7.0 *Hz* 3H), 1.50-1.43 (m, 3H) 1.24 (s, 3H), 1.22 (s, 3H).

**Example 109:** (R&S)-4-(((R)-1-(3-(1,1-difluoro-2-hydroxy-2-methylpropyl)-2-fluorophenyl)ethyl)amino)-6-methoxy-2,6,8-trimethyl-6,8-dihydro-7H-pyrrolo[3,2-g]quinazolin-7-one **(Compound 96)**

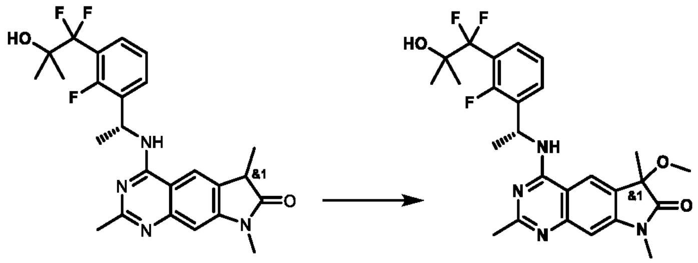

To a stirred solution of 4-(((R)-1-(3-(1,1-difluoro-2-hydroxy-2-methylpropyl)-2-fluorophenyl)ethyl) amino)-2,6,8-trimethyl-6,8-dihydro-7H-pyrrolo[3,2-g]quinazolin-7-one **Compound 95** (7.5 g, 15.87 mmol) in MeOH (150.0 mL) was added CAN (9.14 g, 34.9 mmol) at 25 °C under inert atmosphere. The reaction mixture was stirred at same temperature for 12 h. The reaction mixture was concentrated under reduced pressure to get sticky compound which was dissolved in DCM (200.0 mL) and washed with water (3 x 100.0 mL). The organic layer was separated, dried over anhydrous $Na_2SO_4$ and concentrated to get a crude product. The crude product was purified by preparative HPLC to afford the titled compound as mixture of diastereomers

Two diastereomers were separated by chiral preparative HPLC

Peak-1: (S/R)-4-(((R)-1-(3-(1,1-difluoro-2-hydroxy-2-methylpropyl)-2-fluorophenyl)ethyl)amino)-6-methoxy-2,6,8-trimethyl-6,8-dihydro-7H-pyrrolo[3,2-g]quinazolin-7-one ( 0.60 g, 7.52% yield) **(Compound 96a)**

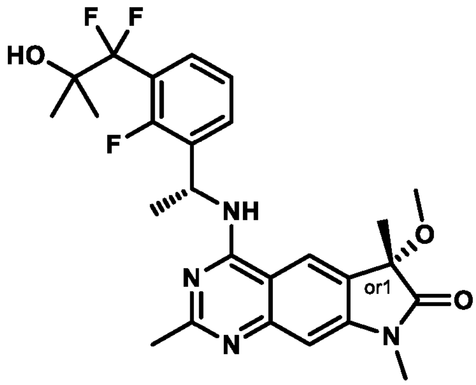

MS(ES+) m/z = 503.43 (M+1).
RT: $t_{ret}$(min) = 9.96.

$^1$H NMR (400 MHz, DMSO-$d_6$) δ 8.46 (s, 1H), 8.38 (d, *J* = 6.9 *Hz,* 1H), 7.65 - 7.56 (m, 1H), 7.34 - 7.28 (m, 1H), 7.27 - 7.19 (m, 1H), 7.13 (s, 1H), 5.82 - 5.77 (m, 1H), 5.33 (s, 1H), 3.22 (s, 3H), 2.92 (s, 3H), 2.32 (s, 3H), 1.58 (d, *J*= 7.0 *Hz,* 3H), 1.54 (s, 3H), 1.24 (s, 3H), 1.21 (s, 3H).

Peak-2: (R/S)-4-(((R)-1-(3-(1,1-difluoro-2-hydroxy-2-methylpropyl)-2-fluorophenyl)ethyl)amino)-6-methoxy-2,6,8-trimethyl-6,8-dihydro-7H-pyrrolo[3,2-g]quinazolin-7-one (0.45 g, 5.64% yield)

**(Compound 96b)**

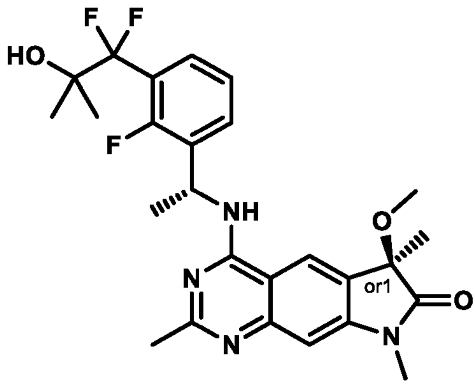

MS(ES+) m/z = 503.43 (M+1).
RT: $t_{ret}$(min) = 10.61

$^1$H NMR (400 MHz, DMSO-$d_6$) 8.44 (s, 1H), 8.37(s, 1H), 7.65 - 7.60 (m, 1H), 7.35 - 7.29 (m, 1H), 7.27 - 7.23 (m, 1H), 7.13 (s, 1H), 5.81 - 5.76 (m, 1H), 5.33 (s, 1H), 3.22 (s, 3H), 2.93 (s, 3H), 2.34 (s, 3H), 1.58 (d, *J = 7.0 Hz,* 3H), 1.53 (s, 3H) 1.24 (s, 3H), 1.23 (s, 3H)

**Compound 96a and 96b (Example 109) can also be prepared by the following method:**

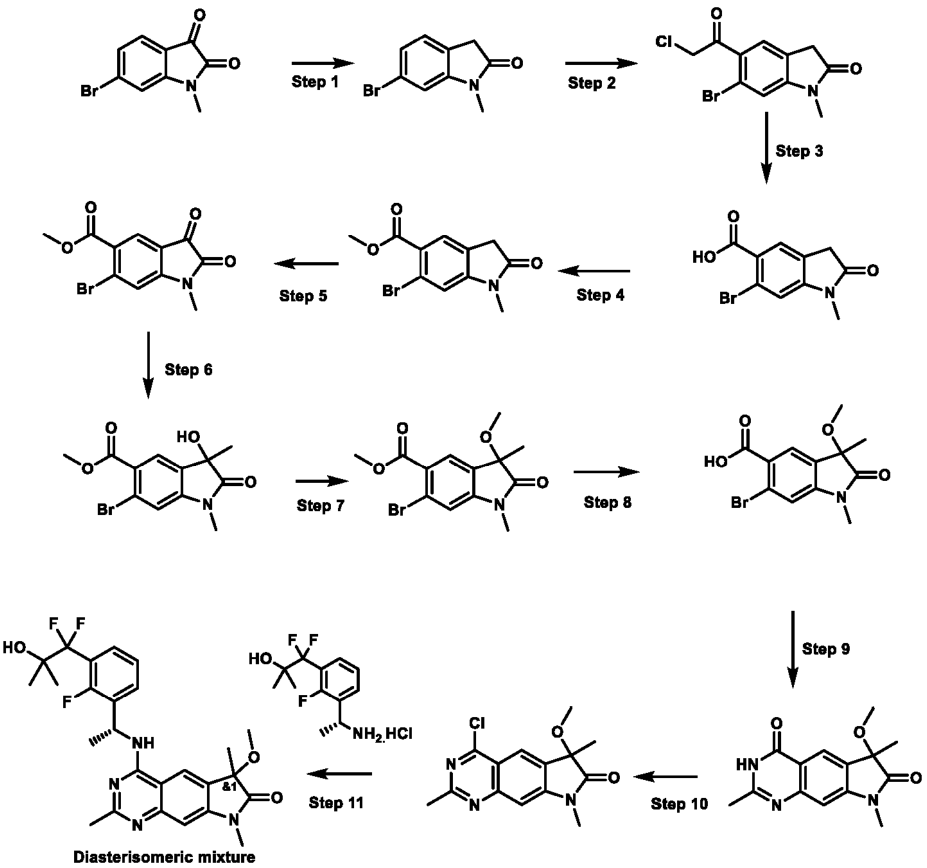


Step 1 : 6-Bromo-1-methylindolin-2-one

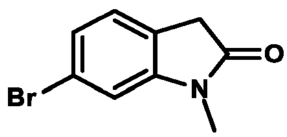

To a stirred suspension of 6-bromo-1-methylindoline-2,3-dione (4.30 g, 17.91 mmol) and hydrazine hydrate (11.54 mL, 233 mmol) was heated at 125 °C for 4 h.. Water was added to the reaction mixture and was extracted with DCM (2 x 50.0 mL). The combined organic layer was washed with water, brine, dried over anhydrous $Na_2SO_4$ and concentrated under reduced pressure to get a crude mixture. The crude mixture was purified by flash chromatography 10% Ethyl acetate-n-hexane to afford the titled compound (0.65 g, 69.0 % yield) off white solid. MS(ES+) m/z = 227.89 (M+1).

Step 2: 6-Bromo-5-(2-chloroacetyl)-1-methylindolin-2-one

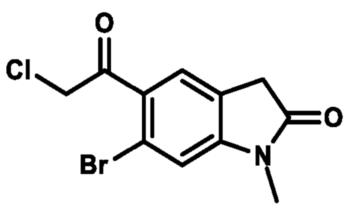

The titled compound was prepared from **Step-1** by employing analogous protocol mentioned in **Example 71-Step-2** (3.0 g, 64 % Yield).

$^1H$ NMR (400 MHz, DMSO-$d_6$) δ 7.73 (s, 1H), 7.42 (s, 1H), 5.01 (s, 2H), 3.60 (s, 2H), 3.15 (s, 3H).

Step 3 : 6-Bromo-1-methyl-2-oxoindoline-5-carboxylic acid

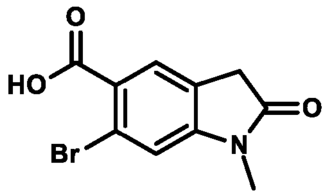

The titled compound was prepared from Step-2 by employing analogous protocol mentioned in **Example 71-Step-3** (2.0 g, 74.7 % yield).

MS(ES+) m/z = 270.08 (M+1).

Step 4: Methyl 6-bromo-1-methyl-2-oxoindoline-5-carboxylate

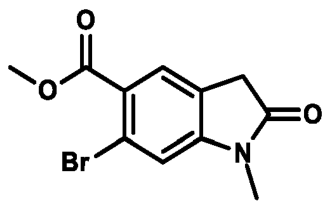

The titled compound was prepared from Step-3 by employing analogous protocol mentioned in **Example 71-Step-4** (0.27 g, 36.7 % yield).

MS(ES+) m/z = 284.21 (M+2).

Step 5: Methyl 6-bromo-1-methyl-2,3-dioxoindoline-5-carboxylate

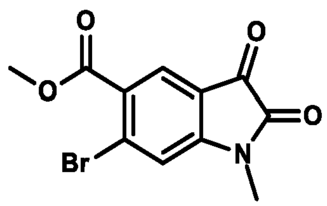

A stirred suspension of methyl 6-bromo-1-methyl-2-oxoindoline-5-carboxylate (1.0 g, 3.52 mmol) and selenium dioxide (0.98 g, 8.80 mmol) in DMSO (5.0 mL) was heated at 50 °C for 1 h. Water was added to reaction mixture and the precipitated solid was filtered, washed with water and dried under reduced pressure to afford the titled compound (0.90 g, 86 % yield) as a red solid.

$^1H$ NMR (400 MHz, DMSO-$d_6$) δ 7.88 (s, 1H), 7.64 (s, 1H), 3.85 (s, 3H), 3.18 (s, 3H).

Step 6: Methyl 6-bromo-3-hydroxy-1,3-dimethyl-2-oxoindoline-5-carboxylate

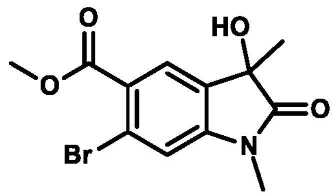

To a stirred solution of methyl 6-bromo-1-methyl-2,3-dioxoindoline-5-carboxylate (1 g, 3.35 mmol) in DCM (25 mL) was added Dimethyl Zinc (15.21 ml, 22.81 mmol, 1.5 M solution in Toluene) at 0 °C and stirred at room temperature for 2 h. Reaction monitoring was done by TLC shows completion of reaction. Reaction mixture was quenched with saturated solution of ammonium chloride and it was extracted with ethyl acetate. Combined organic layer was washed with brine, dried over sodium sulphate and concentrated under the reduced pressure to get a crude. This was purified by column chromatography to get a titled compound (0.9 g, 85 % yield)

MS(ES+) m/z = 314.28 (M+1).

Step 7: Methyl 6-bromo-3-methoxy-1,3-dimethyl-2-oxoindoline-5-carboxylate

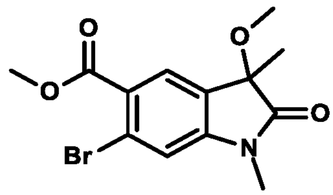

The titled compound was prepared from **Step-6** by employing analogous protocol mentioned in **Example 71-Step-5** (0.13g, 89% yield).

$^1$H NMR (400 MHz, DMSO-$d_6$) $\delta$ 7.77 (s, 1H), 7.53 (s, 1H), 3.85 (s, 3H), 3.18 (s, 3H), 2.88 (s, 3H), 1.44 (s, 3H).

Step 8: 6-Bromo-3-methoxy-1,3-dimethyl-2-oxoindoline-5-carboxylic acid

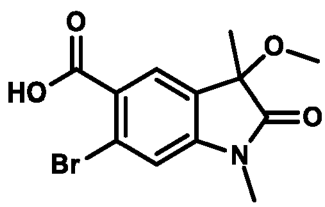

To a stirred solution of methyl 6-bromo-3-methoxy-1,3-dimethyl-2-oxoindoline-5-carboxylate (0.35 g, 1.067 mmol) in THF (0.5 mL) and water (0.5 mL) was added LiOH (0.13 g, 5.33 mmol) at 0 °C. The resulting reaction mixture was stirred at room temperature for 15 h. The reaction mixture was concentrated under reduced pressure and residue was acidified with saturated citric acid solution. The aqueous layer was extracted with Ethyl acetate (2 x 50.0 mL). The combined organic layer was dried over anhydrous $Na_2SO_4$ and evaporated under reduced pressure to give crude product which was purified by flash chromatography to afford the titled compound (0.30 g, 90 % yield).

$^1$H NMR (400 MHz, DMSO-$d_6$) $\delta$ 13.25 (s, 1H), 7.75 (s, 1H), 7.48 (s, 1H), 3.18 (s, 3H), 2.88 (s, 3H), 1.44 (s, 3H)

Step 9: 6-Methoxy-2,6,8-trimethyl-3H-pyrrolo[3,2-g]quinazoline-4,7(6H,8H)-dione

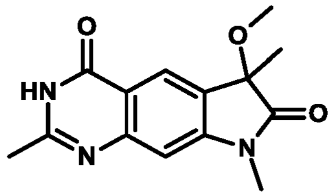

The titled compound was prepared from Step-8 intermediate by employing analogous protocol mentioned in **Example 81a- Step-3a** (0.02 g, 46.0 % yield).

MS(ES+) m/z = 274.21 (M+1).

Step 10: 4-Chloro-6-methoxy-2,6,8-trimethyl-6H-pyrrolo[3,2-g]quinazolin-7(8H)-one

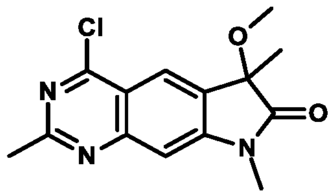

The titled compound was prepared from **Step-9** by employing analogous protocol mentioned in **Example 71-Step-9** (0.030 g, 94% yield). MS(ES+) m/z = 292.02 (M+1).

Step 11 : (R&S)-4-(((R)-1-(3-(1,1-difluoro-2-hydroxy-2-methylpropyl)-2-fluorophenyl)ethyl)amino)-6-methoxy-2,6,8-trimethyl-6,8-dihydro-7H-pyrrolo[3,2-g]quinazolin-7-one

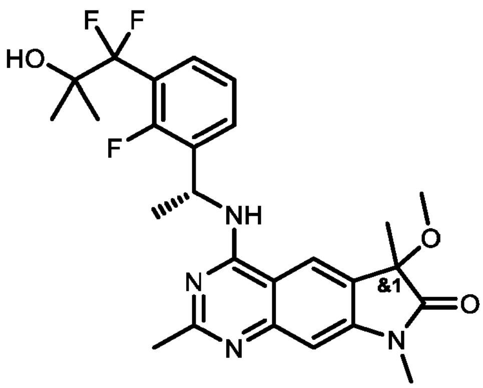

The titled compound as a diastereomeric mixture was prepared from **Step-10** by employing analogous protocol mentioned in **Example 71-Step-10** (0.030 g, 94% yield).

Analytical data matched with as reported in Example 109(Compound 96a and Compound 96 b)

**Example 110:** (R&S)-4-(((R)-1-(3-(1,1-difluoro-2-hydroxy-2-methylpropyl)-2-fluorophenyl)ethyl)amino)-6-hydroxy-2,6,8-trimethyl-6,8-dihydro-7H-pyrrolo[3,2-g]quinazolin-7-one (Compound 97)

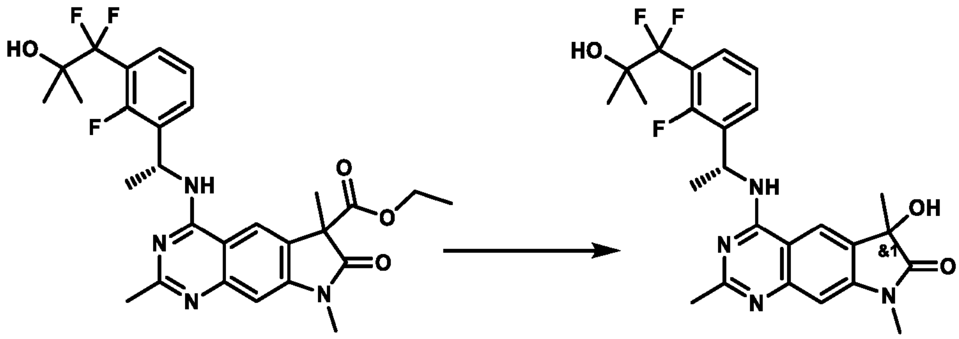

To a stirred solution of ethyl 4-(((R)-1-(3-(1,1-difluoro-2-hydroxy-2-methylpropyl)-2-fluorophenyl)ethyl) amino)-2,6,8-trimethyl-7-oxo-7,8-dihydro-6H-pyrrolo[3,2-g]quinazoline-6-carboxylate **(Example 108- Step 4,** 0.070 g, 0.129 mmol) in EtOH (0.7 mL) was added LiOH.$H_2O$ (0.006 g, 0.154 mmol) by dissolving in water (0.3 mL). The resulting reaction mixture was stirred for the 1h at room temperature. The reaction mixture was concentrated under reduced pressure and triturated with water (10.0 mL). The aqueous layer was decanted and dried under reduced pressure and the diastereomers obtained were purified by reverse phase flash chromatography to afford the titled compound. The two diastereomers were separated by reverse phase preparative HPLC.

Peak-1: (S/R)-4-(((R)-1-(3-(1,1-difluoro-2-hydroxy-2-methylpropyl)-2-fluorophenyl)ethyl)amino)-6-hydroxy-2,6,8-trimethyl-6,8-dihydro-7H-pyrrolo[3,2-g]quinazolin-7-one ( 0.012 g, 19.11% yield)

**(Compound 97a)**

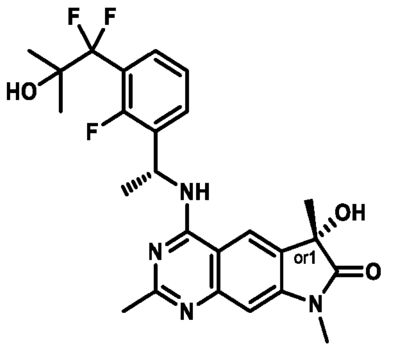

MS(ES+) m/z = 489.31 (M+1).
Reverse phase chromatography $t_{ret}$(min) = 1.26

$^1$H NMR (400 MHz, DMSO- $d_6$) d 8.46 (s, 1H), 8.36 (d, *J* = 7.4 Hz, 1H), 7.66 - 7.56 (m, 1H), 7.35 - 7.25 (m, 1H), 7.22 - 7.16 (m, 1H), 7.05 (s, 1H), 6.16 (s, 1H), 5.86 - 5.74 (m, 1H), 5.33 (s, 1H), 3.18 (s, 3H), 2.32 (s, 3H), 1.58 (d, *J*= 7.1 *Hz,* 3H), 1.51 (s, 3H), 1.24 (s, 3H), 1.22 (s, 3H).

Peak-2: (R/S)-4-(((R)-1-(3-(1,1-difluoro-2-hydroxy-2-methylpropyl)-2-fluorophenyl)ethyl)amino)-6-hydroxy-2,6,8-trimethyl-6,8-dihydro-7H-pyrrolo[3,2-g]quinazolin-7-one (0.012 g, 19.11% yield) (Compound 97b)

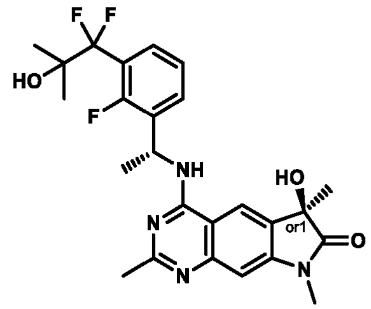

MS(ES+) m/z =489.31 (M+1).
Reverse phase chromatography $t_{ret}$(min) = 1.28

$^1H$ NMR (400 MHz, DMSO-$d_6$) δ 8.45 (s, 1H), 8.34 (d, *J* = 7.4 Hz, 1H), 7.66 - 7.56 (m, 1H), 7.35 - 7.26 (m, 1H), 7.25 - 7.18 (m, 1H), 7.06 (s, 1H), 6.15 (s, 1H), 5.86 - 5.77 (m, 1H), 5.33 (s, 1H), 3.18 (s, 3H), 2.32 (s, 3H), 1.57 (d, *J*= 7.1 *Hz,* 3H), 1.50 (s, 3H), 1.23 (s, 3H), 1.21 (s, 3H).

**Example 111:** (R&S)-4-(((R)-1-(3-(1,1-difluoro-2-hydroxy-2-methylpropyl)-2-fluorophenyl)ethyl)amino)-8-(methoxymethyl)-2,6,8-trimethyl-6,8-dihydro-7H-pyrrolo[2,3-g]quinazolin-7-one **(Compound 98)**

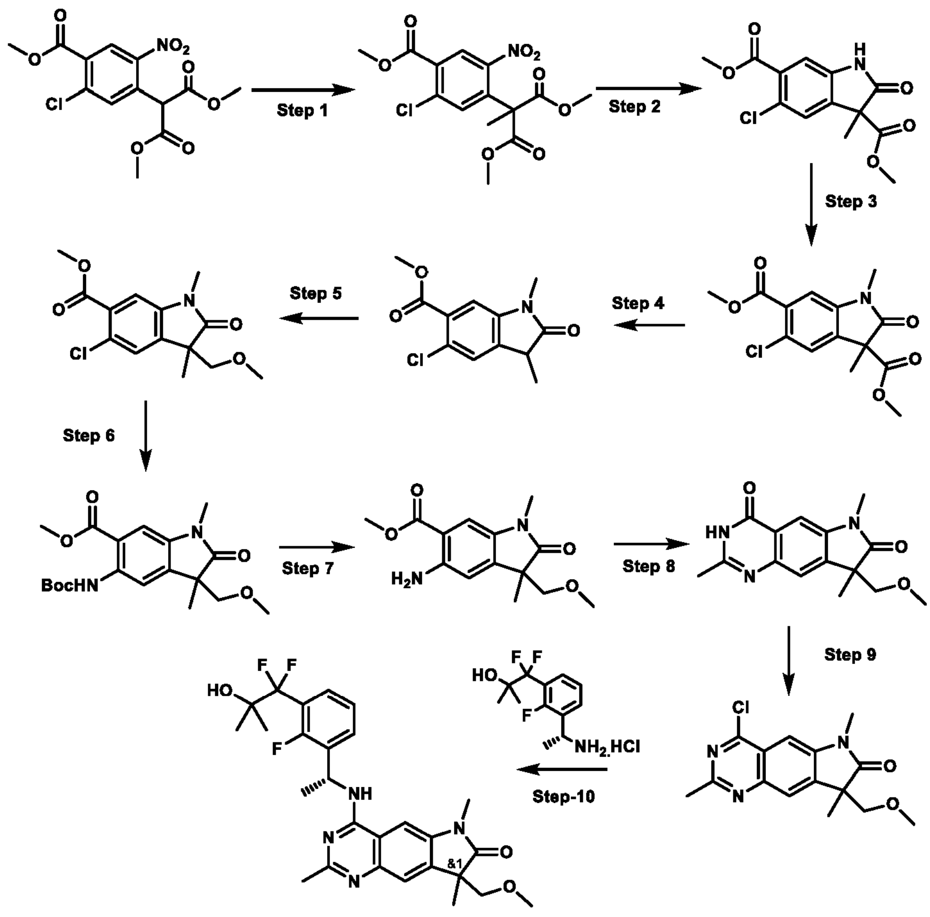


Compound 98a and 98b

Step-1: Dimethyl 2-(5-chloro-4-(methoxycarbonyl)-2-nitrophenyl)-2-methylmalonate

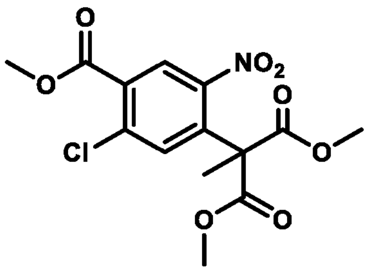

To a stirred solution of dimethyl 2-(5-chloro-4-(methoxycarbonyl)-2-nitrophenyl)malonate Example **76- Step 1** (2.6 g, 7.52 mmol) in DMF (25.0 mL), $K_2CO_3$ (1.455 g, 10.53 mmol) was added followed by methyl iodide (0.658 mL , 10.53 mmol) and the reaction was stirred at room temperature for 16 h. The reaction was diluted with Ethyl acetate (100.0 mL), washed it with water (30.0 mL x 2) and brine (30.0 mL). The separated organic layer was dried over anhydrous $Na_2SO_4$ and removal of solvent provided the crude material. This crude material was purified over flash chromatography using 10% Ethyl acetate-n-hexane as eluent to afford the titled compound (2.6 g, 96 % yield)

MS(ES+) m/z = 360.16 (M+1).

Step-2: Dimethyl-5-chloro-3-methyl-2-oxoindoline-3,6-dicarboxylate

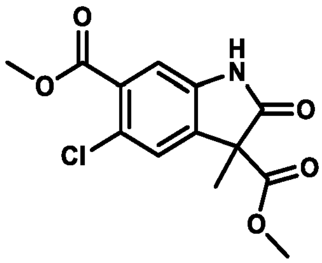

The titled compound was prepared from **Step-1** intermediate by employing analogous protocol mentioned in **Step-4 of Example 76** (2.0 g, 97 %).

$^1$H NMR (400 MHz, DMSO-$d_6$) δ 11.02 (s, 1H), 7.54 (s, 1H), 7.24 (s, 1H), 3.86 (s, 3H), 3.62 (s, 3H), 1.56 (s, 3H).

Step-3: Dimethyl 5-chloro-1,3-dimethyl-2-oxoindoline-3,6-dicarboxylate

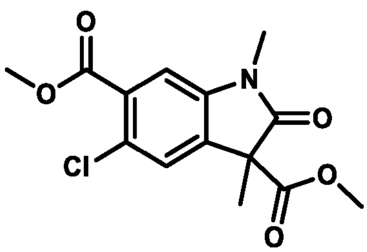

The titled compound was prepared from Step-2 intermediate by employing analogous protocol mentioned in Example 71-Step-5 (8.0 g, 76 % yield).

MS(ES+) m/z = 312.28

Step-4 : Methyl 5-chloro-1,3-dimethyl-2-oxoindoline-6-carboxylate

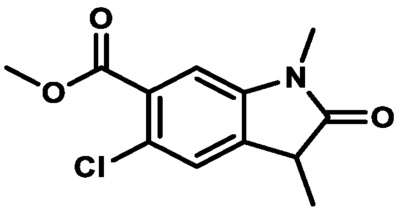

The titled compound was prepared from **Step-3** intermediate by employing analogous protocol mentioned in **Example-106-Step-4** (0.82 g, 84 % yield).

MS(ES+) m/z = 254.08 (M+1).

Step-5 : Methyl 5-chloro-3-(methoxymethyl)-1,3-dimethyl-2-oxoindoline-6-carboxylate

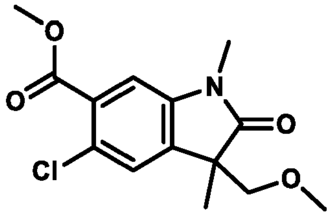

The titled compound was prepared by reaction of **Step-4** intermediate and Methoxy methyl chloride using analogous protocol mentioned in **Example-106-Step-5** (0.55 g, 78 % yield).

MS(ES+) m/z = 298.21 (M+1).

Step-6 : Methyl 5-((tert-butoxycarbonyl)amino)-3-(methoxymethyl)-1,3-dimethyl-2-oxoindoline-6-carboxylate

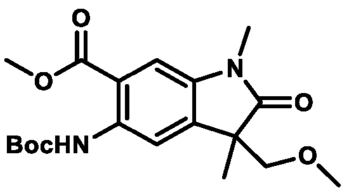

The titled compound was prepared from **Step-5** intermediate by employing analogous protocol mentioned in **Example 71-Step-6** (0.090 g, 79 % yield).

MS(ES+) m/z = 379.35 (M+1).

Step-7 : Methyl 5-amino-3-(methoxymethyl)-1,3-dimethyl-2-oxoindoline-6-carboxylate

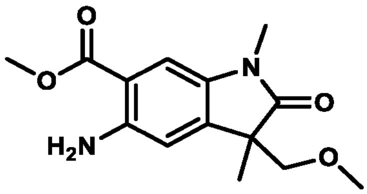

The titled compound was prepared from **Step-6** intermediate by employing analogous protocol mentioned in **Example 71-Step-7** (0.45 g, 98 % yield).

MS(ES+) m/z = 279.27 (M+1)

Step-8 : 8-(Methoxymethyl)-2,6,8-trimethyl-6,8-dihydro-3H-pyrrolo[2,3-g]quinazoline-4,7-dione

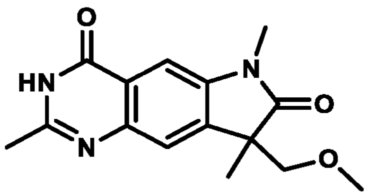

The titled compound was prepared from **Step-7** intermediate by employing analogous protocol mentioned in **Example 71-Step-8** (0.30 g, 73.0 % yield).

MS(ES+) m/z = 287.96 (M+1).

Step-9 : 4-Chloro-8-(methoxymethyl)-2,6,8-trimethyl-6,8-dihydro-7H-pyrrolo[2,3-g]quinazolin-7-one

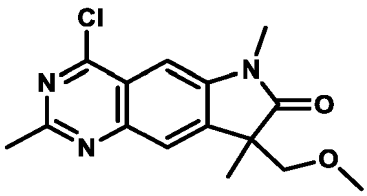

The titled compound was prepared from **Step-8** intermediate by employing analogous protocol mentioned in **Example 71-Step-9** (0.28 g, 88 % yield).

MS(ES+) m/z = 306.28 (M+1).

Step-10 : (R&S)-4-(((R)-1-(3-(1,1-difluoro-2-hydroxy-2-methylpropyl)-2-fluorophenyl)ethyl)amino)-8-(methoxymethyl)-2,6,8-trimethyl-6,8-dihydro-7H-pyrrolo[2,3-g]quinazolin-7-one (Compound 98)

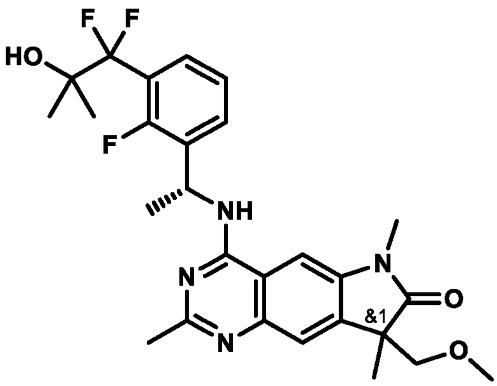

The titled compound was prepared by reaction of **Step-9** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10.**

The two stereoisomers were separated by chiral preparative HPLC

**HPLC method:** CHIRALPAK IC CRL-087 HEX_0.1%DEA_IPA_DCM_80_20_A_B_1.2 ML _15MIN_238NM_1.20 mL /min

Peak-1 :- (R/S)- 4-(((R)-1-(3-(1,1-difluoro-2-hydroxy-2-methylpropyl)-2-fluorophenyl)ethyl)amino)-8-(methoxy methyl)-2,6,8-trimethyl-6,8-dihydro-7H-pyrrolo[2,3-g]quinazolin-7-one (0.020 g, 18.18 % yield)

**(Compound 98 a)**

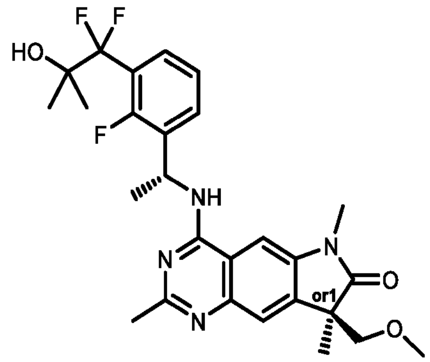

MS(ES+) m/z = 517.30. (M+1)
$t_{ret}$(min) = 7.16

$^1$H NMR (400 MHz, DMSO-$d_6$) δ 8.30 (s, 1H), 7.88 (s, 1H), 7.68 - 7.56 (m, 2H), 7.38 - 7.27 (m, 1H), 7.27 - 7.16 (m, 1H), 5.88 - 5.79 (m, 1H), 5.35 (s, 1H), 3.74 (d, *J*= 8.9 Hz, 1H), 3.64 (d, *J*= 8.9 *Hz,* 1H), 3.27 (s, 3H), 3.08 (s, 3H), 2.33 **(s,** 3H), 1.61 (d, *J* = 7.0 *Hz,* 3H), 1.30 - 1.16 (m, 9H).

Peak-2:- (S/R)-4-(((R)-1-(3-(1,1-difluoro-2-hydroxy-2-methylpropyl)-2-fluorophenyl)ethyl)amino)-8-(methoxymethyl)-2,6,8-trimethyl-6,8-dihydro-7H-pyrrolo[2,3-g]quinazolin-7-one (0.032 g, 29.1 % yield).

**(Compound 98b)**

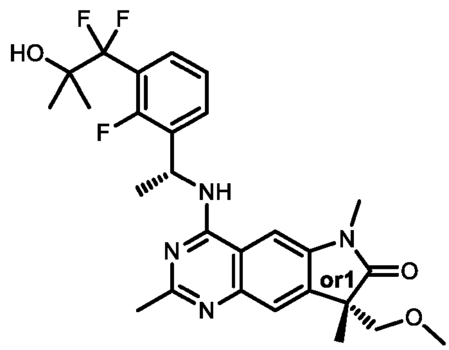

MS(ES+) m/z = 517.30 (M+1).
$t_{ret}$(min) = 9.20

$^1$H NMR (400 MHz, DMSO-$d_6$) δ 8.33 (s, 1H), 7.93 (s, 1H), 7.64 (d, *J* = 8.9 *Hz,* 2H), 7.38 - 7.27 (m, 1H), 7.28 - 7.16 (m, 1H), 5.92 - 5.79 (m, 1H), 5.35 (s, 1H), 3.74 (d, J = 8.9 *Hz,* 1H), 3.63 (d, *J* = 9.0 *Hz,* 1H), 3.28 (s, 3H), 3.06 (s, 3H), 2.36 (s, 3H), 1.62 (d, *J* = 7.0 *Hz,* 3H), 1.32 - 1.20 (m, 9H).

**Example 112:** Preparation of (R&S)-4-(((R)-1-(3-(1,1-difluoro-2-hydroxy-2-methylpropyl)-2-fluorophenyl)ethyl)amino)-2,6,8-trimethyl-6,8-dihy-dro-7H-pyrrolo[2,3-g]quinazolin-7-one **(Compound 99)**

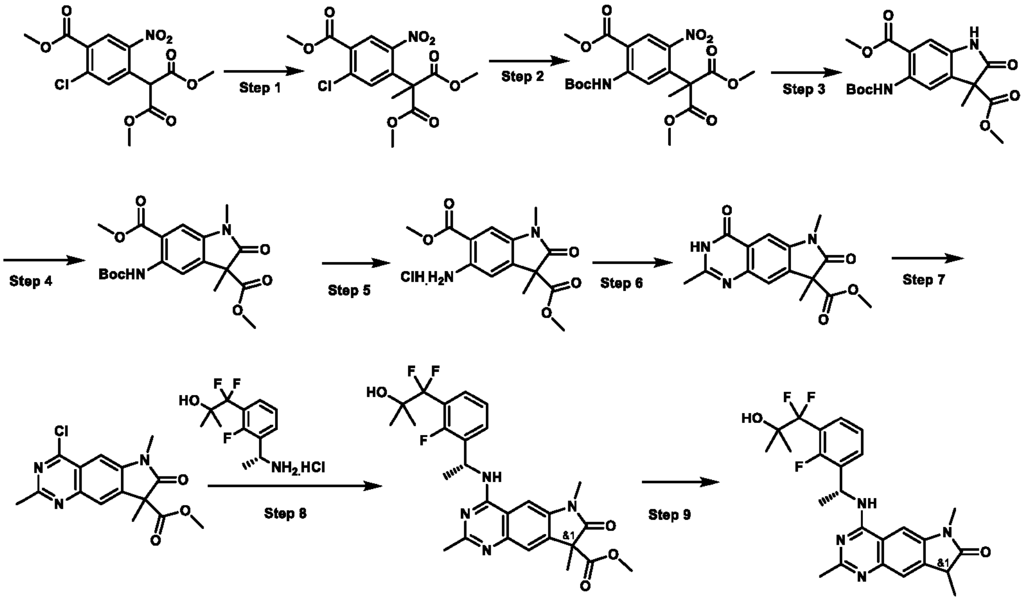


Step 1: Dimethyl 2-(5-chloro-4-(methoxycarbonyl)-2-nitrophenyl)-2-methylmalonate

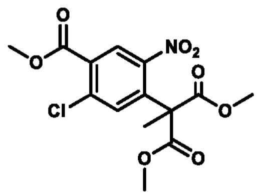

To a solution of dimethyl 2-(5-chloro-4-(methoxycarbonyl)-2-nitrophenyl)malonate **(Example 76 Step-1** , 65 g, 188 mmol) in DMF (250 mL), $K_2CO_3$ (36.4 g, 263 mmol) and methyl iodide (16.46 mL, 263 mmol) were added at 0 °C subsequently. The reaction mixture was stirred overnight at room temperature. Reaction mixture was poured into ice water and extracted with ethyl acetate (2 x 500.0 mL). Combined organic layer was washed with water (2 x 500.0 mL), brine (500.0 mL) and dried on anhydrous $Na_2SO_4$. Organic layer was evaporated on rotavapor to afford the titled compound. (60 g, 89 % yield).

MS(ES+) m/z = 360.22 (M+1).

Step 2 : Dimethyl 2-(5-((tert-butoxycarbonyl)amino)-4-(methoxycarbonyl)-2-nitrophenyl)-2-methylmalonate

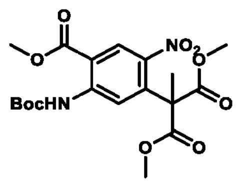

To a solution of dimethyl 2-(5-chloro-4-(methoxycarbonyl)-2-nitrophenyl)-2-methylmalonate (10 g, 27.8 mmol) in dry 1,4-Dioxane (150.0 ml), were added tert-butyl carbamate (4.89 g, 41.7 mmol), $Cs_2CO_3$ (11.78 g, 36.1 mmol). The resulting suspension was degassed with nitrogen for 10 min. Xantphos (1.930 g, 3.34 mmol) and $Pd_2(dba)_3$ (2.55 g, 2.78 mmol) were added and the reaction mixture was heated at 120 °C for 2 h. The reaction was cooled to room temperature and solvent was removed under reduced pressure. The crude product was purified by flash chromatography in ethyl acetate-n-hexane gradient to afford titled compound (10 g, 82 % yield).

MS(ES+) m/z = 441.23 (M+1).

Step 3 : Dimethyl 5-((tert-butoxy carbonyl)amino)-3-methyl-2-oxoindoline-3,6-dicarboxylate

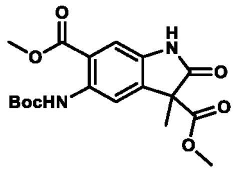

To a stirred solution of dimethyl 2-(5-((tert-butoxycarbonyl)amino)-4-(methoxycarbonyl)-2-nitrophenyl)-2-methylmalonate (10 g, 22.71 mmol) ) in Ethanol (120.0 mL) and acetic acid (20.0 mL), iron (2.54 g, 45.4 mmol) was added. The resulting reaction mixture was stirred at 100°C for 2 h. The reaction mixture was concentrated and the residue was partitioned between ethyl acetate (200.0 mL) and water (100.0 mL). Organic layer separated, dried over anhydrous $Na_2SO_4$, and concentrated under vacuum to afford titled compound (8.51 g, 99 % yield).

MS(ES+) m/z = 379.35 (M + 1).

Step 4: Dimethyl 5-((tert-butoxy carbonyl)amino)-1,3-dimethyl-2-oxoindoline-3,6-dicarboxylate

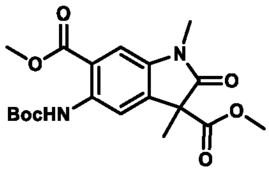

To a stirred solution of dimethyl 5-((tert-butoxycarbonyl)amino)-3-methyl-2-oxoindoline-3,6-dicarboxylate (8.5 g, 22.46 mmol) in DMF (100.0 mL), $K_2CO_3$ (4.04 g, 29.2 mmol) and methyl iodide (1.826 ml, 29.2 mmol) were added subsequently. The resulting reaction mixture was stirred at room temperature for 12 h. reaction was diluted with ethyl acetate (200.0 mL), washed it with water (2 x 300.0 mL) and brine (100.0 mL). Organic layer was dried over anhydrous $Na_2SO_4$, filtered and concentrated to get crude product. The crude product was purified by column chromatography (Silica gel, Eluent used: 0 to 30% EtOAc in Hexane) to afford dimethyl 5-((tert-butoxycarbonyl)amino)-1,3-dimethyl-2-oxoindoline-3,6-dicarboxylate (7 g, 17.84 mmol, 79 % yield) MS(ES+) m/z = 393.2 (M+1).

Step-5: Dimethyl 5-amino-1,3-dimethyl-2-oxoindoline-3,6-dicarboxylate hydrochloride

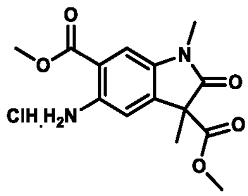

To a solution of dimethyl 5-((tert-butoxycarbonyl)amino)-1,3-dimethyl-2-oxoindoline-3,6-dicarboxylate (7.0 g, 17.84 mmol) in 1,4-dioxane, was added HCl (4M in 1,4-dioxane, 12.0 mL) at 0°C and the reaction was stirred at 70 °C for 2 h. Reaction was cooled to room temperature and solvent was evaporated under vacuum to get crude material. Crude product was triturated with diethyl ether to afford titled compound (5.6g, 95 % yield). Crude product was used as such for next step.

MS(ES+) m/z = 293.34 (M+1, salt free amine).

Step-6: Methyl 2,6,8-trimethyl-4,7-dioxo-4,6,7,8-tetrahydro-3H-pyrrolo[2,3-g] quinazoline-8-carboxylate

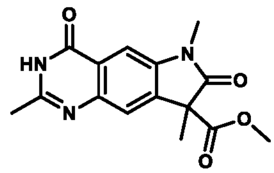

To a solution of dimethyl 5-amino-1,3-dimethyl-2-oxoindoline-3,6-dicarboxylate hydrochloride (5.5 g, 16.73 mmol) in acetonitrile (20.0 mL), methanesulfonic acid (10.86 ml, 167 mmol) was added and the reaction was stirred at 100 °C for 16 h. Solvent was evaporated and to the residue was dissolved ethyl acetate (50.0 mL) was added washed it with aq. sodium bicarbonate (2 x 15.0 mL) and water (15.0 mL). The separated organic layer was dried over anhydrous $Na_2SO_4$, filtered and concentrated to get crude methyl 2,6,8-trimethyl-4,7-dioxo-4,6,7,8-tetrahydro-3H-pyrrolo[2,3-g]quinazoline-8-carboxylate (3.2 g, 10.62 mmol, 63.5 % yield).

MS(ES+) m/z = 302.2 (M+1).

Step-7: Methyl 4-chloro-2,6,8-trimethyl-7-oxo-7,8-dihydro-6H-pyrrolo[2,3-g]quinazoline-8-carboxylate

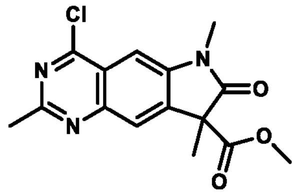

To a suspension of methyl 2,6,8-trimethyl-4,7-dioxo-4,6,7,8-tetrahydro-3H-pyrrolo[2,3-g]quinazoline-8-carboxylate (1 g, 3.32 mmol) in chlorobenzene (15.0 mL) was added DIPEA (1.739 ml, 9.96 mmol) followed by addition of $POCl_3$ (0.773 ml, 8.30 mmol) in drop wise manner at room temperature. Resulting reaction mixture was heated at 90 °C for the 2.5 h. Reaction mixture was poured in cold water and extracted with ethyl acetate (2 x 30.0 mL). Combined organic layer was washed with brine (25.0 mL), dried over $Na_2SO_4$, concentrated to dryness under vacuum to afford titled compound (1 g, 94 % yield).

MS(ES+) m/z = 319.96 (M+).

Step 8: Methyl 4-(((R)-1-(3-(1,1-difluoro-2-hydroxy-2-methylpropyl)-2-fluorophenyl) ethyl) amino)-2,6,8-trimethyl-7-oxo-7,8-dihydro-6H-pyrrolo[2,3-g]quinazoline-8-carboxylate

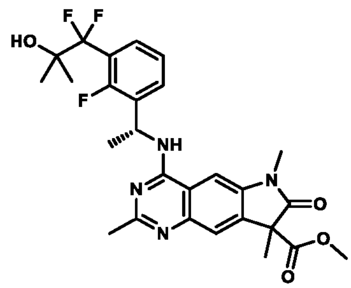

To a suspension of methyl 4-chloro-2,6,8-trimethyl-7-oxo-7,8-dihydro-6H-pyrrolo[2,3-g]quinazoline-8-carboxylate (1 g, 3.13 mmol) in dioxane (15.0 mL). were added (R)-1-(3-(1-aminoethyl)-2-fluorophenyl)-1,1-difluoro-2-methylpropan-2-ol hydrochloride (0.887 g, 3.13 mmol) and DIPEA (2.73 ml, 15.64 mmol) at room temperature. Resulting reaction mixture was heated at 120 °C for 16 h. Solvent was evaporated and crude material was purified by flash chromatography in MeOH-DCM gradient to afford titled compound (1.2 g, 72.3 % yield)

MS(ES+) m/z = 531.44 (M+1).

Step-9: (R&S)-4-(((R)-1-(3-(1,1-difluoro-2-hydroxy-2-methylpropyl)-2-fluorophenyl)ethyl)amino)-2,6,8-trimethyl-6,8-dihydro-7H-pyrrolo[2,3-g]quinazolin-7-one (Compound 99)

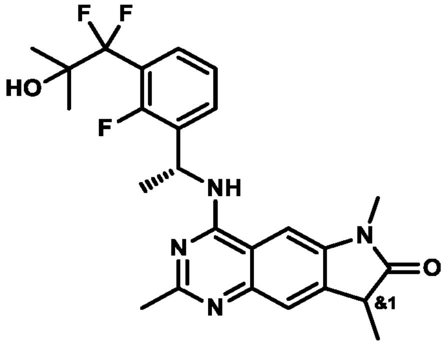

To a solution of methyl 4-(((R)-1-(3-(1,1-difluoro-2-hydroxy-2-methylpropyl)-2-fluorophenyl)ethyl)amino)-2,6,8-trimethyl-7-oxo-7,8-dihydro-6H-pyrrolo[2,3-g]quinazoline-8-carboxylate (1 g, 1.885 mmol) in TFA (1.452 ml, 18.85 mmol), $H_2SO_4$ (3.35 ml, 18.85 mmol) was added and reaction was stirred at 80 °C for 6 h. Reaction was cooled to room temperature, poured in an ice and precipitated solid was filtered. Solid was dissolved in DCM, dried over anhydrous $Na_2SO_4$ and solvent was evaporated to afford titled compound (0.8 g, 90 % yield).

MS(ES+) m/z = 473.36 (M+1).

$^1$H NMR (400 MHz, DMSO-$d_6$) δ 8.24 - 8.16 (m, 1H), 7.88 - 7.82 (m, 1H), 7.60 (s, 1H), 7.57 - 7.52 (m, 1H), 7.35 - 7.27 (m, 1H), 7.26 - 7.16 (m, 1H), 5.84 - 5.79 (m, 1H), 5.37 - 5.31 (m, 1H), 3.70 - 3.61 (m, 1H), 3.27 (s, 3H), 2.32 (d, *J* = 1.6 *Hz,* 3H), 1.60 (d, *J*= 7.0 *Hz,* 3H), 1.45 - 1.37 (m, 3H), 1.23 (s, 3H), 1.22 (s, 3H).

**Example 113:** (R&S)-4-(((R)-1-(3-(1,1-difluoro-2-hydroxy-2-methylpropyl)-2-fluorophenyl)ethyl)amino)-8-hydroxy-2,6,8-trimethyl-6,8-dihydro-7H-pyrrolo[2,3-g]quinazolin-7-one **(Compound 100)**

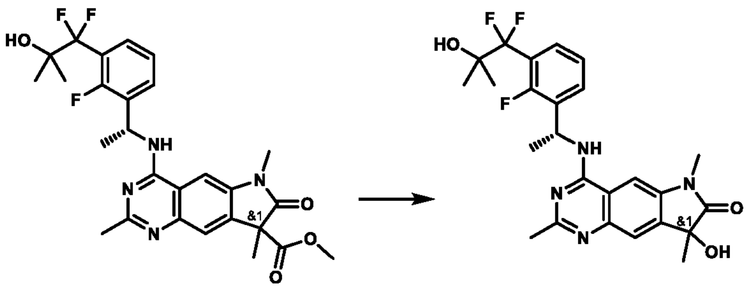

The titled compound was prepared from **Example 112- Step-8** intermediate by employing analogous protocol mentioned in **Example-110.**

Diastereoisomers formed in product were separated by reverse phase preparative HPLC

**Peak 1:** (S/R)-4-(((R)-1-(3-(1,1-difluoro-2-hydroxy-2-methylpropyl)-2-fluorophenyl) ethyl)amino)-8-hydroxy-2,6,8-trimethyl-6H-pyrrolo[2,3-g]quinazolin-7(8H)-one

**(Compound 100a)**

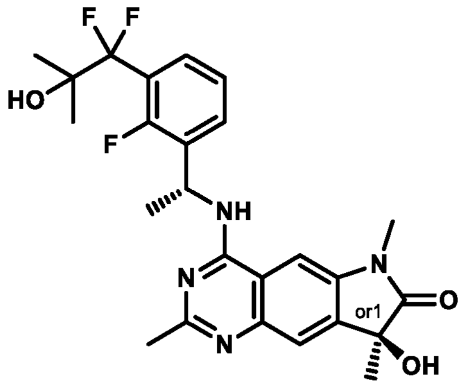

MS(ES+) m/z = 489.42 (M+1)
Reverse phase chromatography $t_{ret}$(min) = 1.81

$^{1}$H NMR (400 MHz, DMSO-$d_6$) δ 8.25 - 8.18 (m, 1H), 7.85 (s, 1H), 7.62 - 7.51 (m, 2H), 7.36 - 7.27 (m, 1H), 7.26 - 7.17 (m, 1H), 6.16 (s, 1H), 5.87 - 5.75 (m, 1H), 5.34 (s, 1H), 3.25 (s, 3H), 2.32 (s, 3H), 1.60 (d, *J* = 7.0 *Hz,* 3H), 1.46 (s, 3H), 1.23 (s, 3H), 1.21 (s, 3H).

**Peak 2:** (R/S)-4-(((R)-1-(3-(1,1-difluoro-2-hydroxy-2-methylpropyl)-2-fluorophenyl)ethyl)amino)-8-hydroxy-2,6,8-trimethyl-6H-pyrrolo[2,3-g]quinazolin-7(8H)-one **(Compound 100b)**

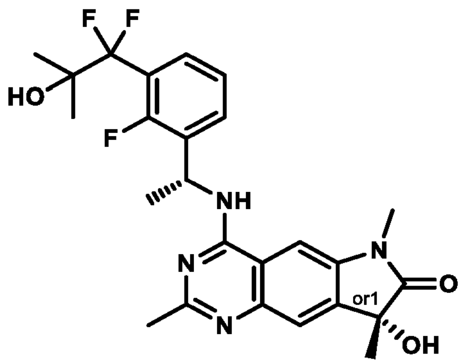

MS(ES+) m/z = 489.42 (M+1).
Reverse phase chromatography ($t_{ret}$(min) = 1.87

$^{1}$H NMR (400 MHz, DMSO-$d_6$) δ 8.36 - 8.25 (m, 1H), 7.88 (s, 1H), 7.64 - 7.52 (m, 2H), 7.36 - 7.27 (m, 1H), 7.25 - 7.16 (m, 1H), 6.20 (s, 1H), 5.85 - 5.79 (m, 1H), 5.34 (s, 1H), 3.26 (s, 3H), 2.33 (s, 3H), 1.61 (d, J = 7.0 *Hz,* 3H), 1.45 (s, 3H), 1.24 (s, 3H), 1.22 (s, 3H)
Reverse phase chromatography ($t_{ret}$(min) = 1.87

**Example 114** :- (R&S)-4-(((R)-1-(3-(1,1-difluoro-2-hydroxy-2-methylpropyl)-2-fluorophenyl) ethyl) amino)-8-methoxy-2,6,8-trimethyl-6,8-dihydro-7H-pyrrolo[2,3-g]quinazolin-7-one **(Compound 101)**

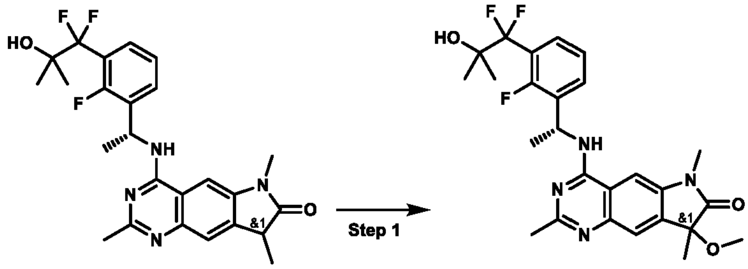


To a stirred solution of 4-(((R)-1-(3-(1,1-difluoro-2-hydroxy-2-methylpropyl)-2-fluorophenyl)ethyl)amino)-2,6,8-trimethyl-6,8-dihydro-7H-pyrrolo[2,3-g]quinazolin-7-one (0.8 g, 1.693 mmol) in methanol (15.0 mL) was added ceric ammonium nitrate (2.042 g, 3.72 mmol) at 25 °C under inert atmosphere and the resulting reaction mixture was stirred at same temperature for 20 h. Reaction mixture was concentrated under reduced pressure to get sticky compound which was dissolved in DCM (20.0 ml) and washed with water (3 x 10.0 mL). Organic layer was dried over anhydrous $Na_2SO_4$ and concentrated to get crude product. Crude product was purified by RP HPLC to afford titled compound as mixture of diastereomers (0.17 g, 20% yield).

$^1$H NMR (400 MHz, DMSO-$d_6$) δ 8.30 (d, *J* = 7.3 *Hz,* 1H), 7.95 (s, 1H), 7.65 - 7.58 (m, 1H), 7.56 (d, *J* = 1.8 *Hz,* 1H), 7.35 - 7.28 (m, 1H), 7.26 - 7.18 (m, 1H), 5.87 - 5.78 (m, 1H), 5.35 (s, 1H), 3.30 (s, 3H), 2.90 (S, 3H), 2.33 (s, 3H), 1.61 (d, *J*= 7.0 *Hz,* 3H), 1.49 (s, 3H), 1.24 (s, 3H), 1.22 (s, 3H).

(NMR spectra of Diastereomeric mixture)

MS(ES+) m/z = 503.43 (M+1).

The Diastereomers of **Example 114** were separated by preparative chiral HPLC

**HPLC method:** CHIRALPAK IC CRL-087 HEX0.1% DEA_IPA-DCM_70_30_A_B_1.2 ML _10MIN_290nm 1.2 mL /min

Peak 1: (S/R)-4-(((R)-1-(3-(1,1-difluoro-2-hydroxy-2-methylpropyl)-2-fluorophenyl)ethyl)amino)-8-methoxy-2,6,8-trimethyl-6H-pyrrolo[2,3-g]quinazolin-7(8H)-one (0.015 g, 1.763 % yield)

(Compound 101a)

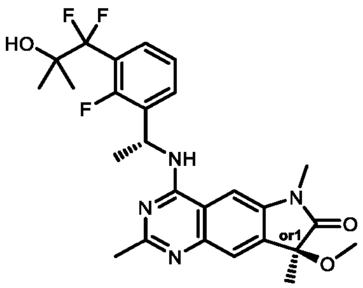

$t_{ret}$(min) = 4.58
MS(ES+) m/z = 503.43 (M+1)

$^1$H NMR (400 MHz, DMSO-$d_6$) δ 8.35 (d, *J*= 7.3 *Hz,* 1H), 7.96 (s, 1H), 7.64 - 7.58 (m, 1H), 7.57 (s, 1H), 7.35 - 7.29 (m, 1H), 7.25 - 7.19 (m, 1H), 5.87 - 5.78 (m, 1H), 5.35 (s, 1H), 3.30 (s, 3H), 2.91 (s, 3H), 2.34 (s, 3H), 1.61 (d, *J* = 7.0 *Hz,* 3H), 1.48 (s, 3H), 1.24 (s, 3H), 1.22 (s, 3H).

Peak 2: (R/S)-4-(((R)-1-(3-(1,1-difluoro-2-hydroxy-2-methylpropyl)-2-fluorophenyl)ethyl) amino)-8-methoxy-2,6,8-trimethyl-6H-pyrrolo[2,3-g]quinazolin-7(8H)-one (0.020 g, 2.3 % yield)

**(Compound 101b)**

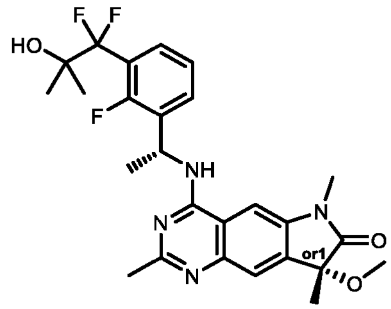

$t_{ret}$(min) = 5.39
MS(ES+) m/z = 503.44 (M+1)

$^1$H NMR (400 MHz, DMSO-$d_6$) δ 8.31 (d, *J*= 7.1 *Hz,* 1H), 7.95 (s, 1H), 7.66 - 7.58 (m, 1H), 7.56 (s, 1H), 7.35 - 7.28 (m, 1H), 7.26 - 7.19 (m, 1H), 5.87 - 5.78 (m, 1H), 5.35 (s, 1H), 3.30 (s, 3H), 2.89 (s, 3H), 2.34 (s, 3H), 1.61 (d, *J*= 7.0 *Hz,* 3H), 1.49 (s, 3H), 1.24 (s, 3H), 1.22 (s, 3H).

**Example 115** :- (S/R)-4-(((R/S)-1-(3-((S/R)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one

**(Compound 102)**

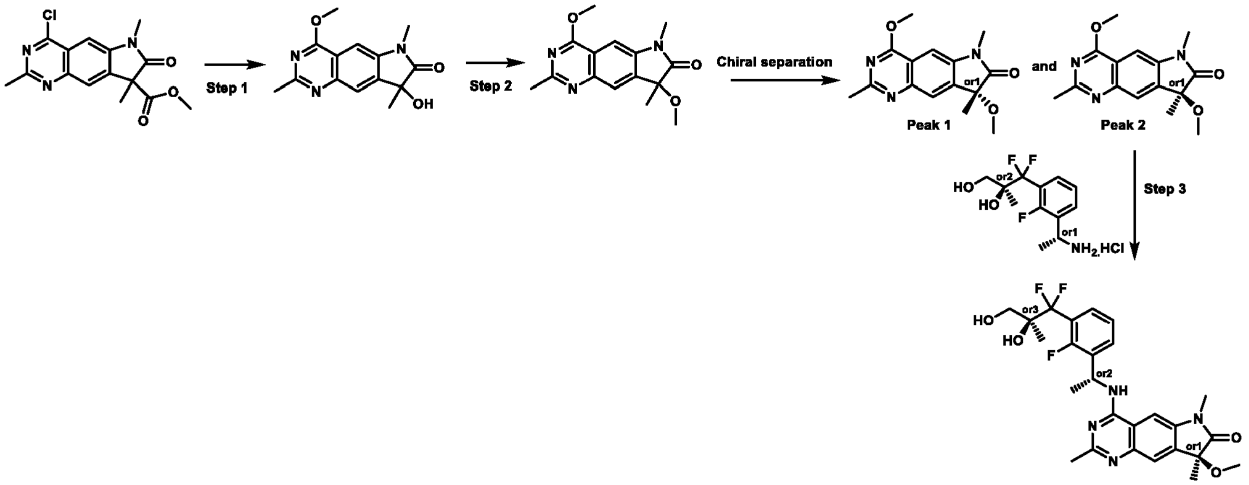


Step 1: 8-Hydroxy-4-methoxy-2,6,8-trimethyl-6H-pyrrolo[2,3-g]quinazolin-7(8H)-one

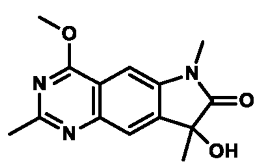

The solution of methyl 4-chloro-2,6,8-trimethyl-7-oxo-7,8-dihydro-6H-pyrrolo[2,3-g]quinazoline-8-carboxylate (2.65g, 8.29 mmol) **Example 112-Step 7** in Methanol (30 mL) was stirred at 50°C for 15 minutes and cooled to RT was added LiOH (0.595 g, 24.86 mmol) at 0 °C. Resulting reaction mixture was stirred at RT for 15 hr. Reaction mixture was concentrated in vacuo, and column purified using Methanol in Dichloromethane (0-5%) to get 8-hydroxy-4-methoxy-2,6,8-trimethyl-6,8-dihydro-7H-pyrrolo[2,3-g]quinazolin-7-one (2.02 g, 7.39 mmol, 89 % yield)
MS(ES+) m/z = 274.21(M+ 1).

Step 2: 4,8-Dimethoxy-2,6,8-trimethyl-6H-pyrrolo[2,3-g]quinazolin-7(8H)-one

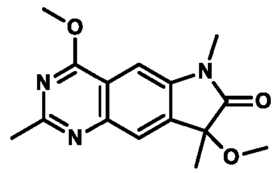

To a stirred solution of 8-hydroxy-4-methoxy-2,6,8-trimethyl-6,8-dihydro-7H-pyrrolo[2,3-g]quinazolin-7-one (2.300 g, 8.42 mmol) and MeI (1.052 mL , 16.83 mmol) in DMF (30 mL) was added NaH (0.539 g, 13.47 mmol) portion wise at 0°C. Resulting reaction mixture was stirred at same temperature for the 45 min. Reaction was quenched with Aq.$NH_4Cl$ and extracted with Ethyl acetate(50.0 mL x 2). Organic layer was separated, dried over anhydrous $Na_2SO_4$ and evaporated. The crude product obtained was purified by flash chromatography to give titled compound as racemate. The enantiomers were separated by chiral preparative

HPLC

**HPLC method:** CHIRALPAK IC CRL-087 Column ACN_0.1%DEA_100_B_1.0 ML _10MIN_254NM Flow Rate: 1.0 mL /min

Peak 1:- (R/S)-4,8-dimethoxy-2,6,8-trimethyl-6,8-dihydro-7H-pyrrolo[2,3-g]quinazolin-7-one (0. 49 g, 20.2 % yield)

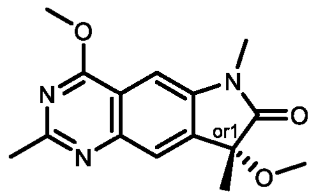

$t_{ret}$(min) = 5.22

$^1$H NMR (400 MHz, DMSO-$d_6$) δ 7.80 (s, 1H), 7.49 (s, 1H), 4.13 (s, 3H), 3.27 (s, 3H), 2.91 (s, 3H), 2.63 (s, 3H), 1.52 (s, 3H).

Peak 2:- (S/R)-4,8-dimethoxy-2,6,8-trimethyl-6,8-dihydro-7H-pyrrolo[2,3-g]quinazolin-7-one (0.49 g, 20.2 % yield) (0.35 g, 14.47%)

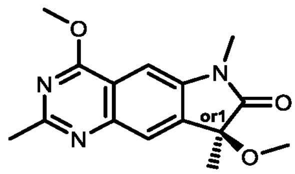

$t_{ret}$(min) = 5.98

$^1$H NMR (400 MHz, DMSO-$d_6$) δ 7.80 (s, 1H), 7.48 (s, 1H), 4.13 (s, 3H), 3.27 (s, 3H), 2.91 (s, 3H), 2.63 (s, 3H), 1.52 (s, 3H).

Stereochemistry of Peak 2 isomer is " S " , confirmed by X-ray crystallography.

**Step 2 intermediate could also be prepared by following method**

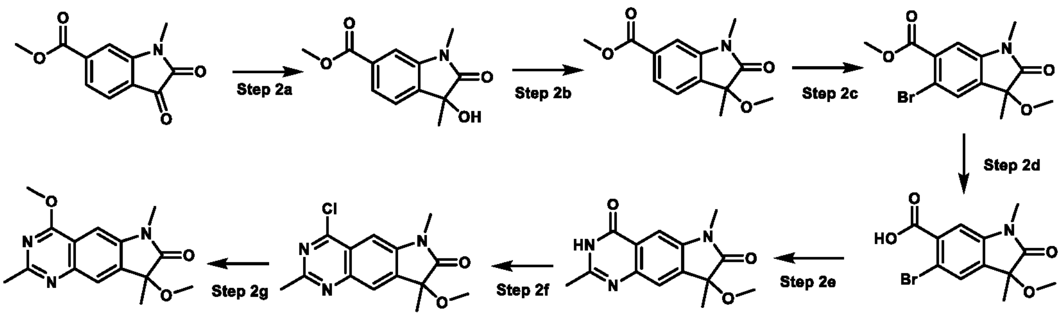


Step-2a: Methyl 3-hydroxy-1,3-dimethyl-2-oxoindoline-6-carboxylate

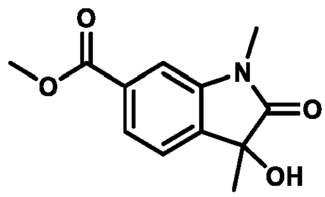

To a stirred solution of commercially available methyl 1-methyl-2,3-dioxoindoline-6-carboxylate (1g, 4.56 mmol) in DCM (25 ml) was added 1.5M solution of Dimethyl zinc (9.12 ml, 13.69 mmol) in toluene at -20 °C. Reaction mixture was gradually warmed to RT and stirred at RT for 2 hr. Reaction monitoring was done by TLC shows completion of reaction. Reaction mixture was quenched with saturated solution of citric acid and it was extracted with ethyl acetate (2*50 mL). Combined organic layer was washed with brine, dried over sodium sulfate and concentrated under the reduced pressure to get a crude compound. Crude compound was purified by column chromatography in 20 to 30 % ethyl acetate in hexane to get a titled compound (0.8 g, 74.5 % yield) as an off white solid
GCMS m/z: 235.20(M+)

Step-2b: Methyl 3-methoxy-1,3-dimethyl-2-oxoindoline-6-carboxylate

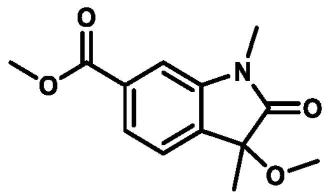

The titled compound was prepared from step-2a intermediate by following analogous protocol as mentioned in example 115-step- 2.
$^1$H NMR (400 MHz, DMSO-$d_6$) δ 7.77 (dd, J = 7.7, 1.5 Hz, 1H), 7.57 - 7.48 (m, 2H), 3.89 (s, 3H), 3.21 (s, 3H), 2.87 (s, 3H), 1.44 (s, 3H).

Step-2c: Methyl 5-bromo-3-methoxy-1,3-dimethyl-2-oxoindoline-6-carboxylate

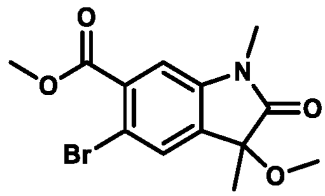

The titled compound was prepared from step-2b intermediate by following analogous protocol as mentioned in example 81-step- 2a
MS(ES+) m/z = 330.28(M+2)

Step-2d: 5-bromo-3-methoxy-1,3-dimethyl-2-oxoindoline-6-carboxylic acid

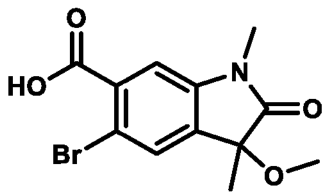

The titled compound was prepared from step-2c intermediate by following analogous protocol as mentioned in example 81-step- 4a
MS(ES+) m/z = 314.25(M+)

Step-2e: 8-methoxy-2,6,8-trimethyl-6,8-dihydro-3H-pyrrolo[2,3-g]quinazoline-4,7-dione

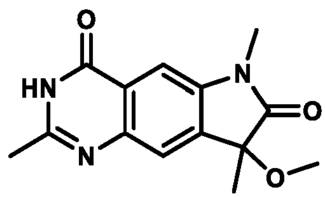

The titled compound was prepared from step-2d intermediate by following analogous protocol as mentioned in example 81-step- 5a
MS(ES+) m/z = 274.40(M+1)

Step-2f: 4-chloro-8-methoxy-2,6,8-trimethyl-6,8-dihydro-7H-pyrrolo[2,3-g]quinazolin-7-one

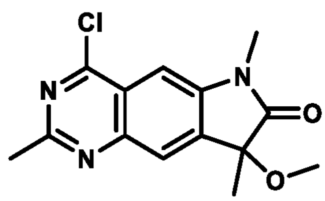

The titled compound was prepared from step-2e intermediate by following analogous protocol as mentioned in example 71-step- 9
MS(ES+) m/z = 292.25(M+1)

Step 2g: 4,8-dimethoxy-2,6,8-trimethyl-6,8-dihydro-7H-pyrrolo[2,3-g]quinazolin-7-one

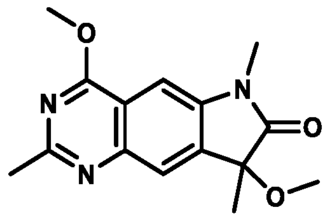

The solution of 4-chloro-8-methoxy-2,6,8-trimethyl1-6,8-dihydro-7H-pyrrolo[2,3-g]quinazolin-7-one (step 2f, 100 mg, 0.360 mmol) of 10 ml methanol stirred at 60 °C for 15 min. After completion of reaction, reaction mixture was evaporated on rotavapor and column purified using 30 % ethyl acetate in n-hexane as eluent to afford titled compound (70 mg, 67.7 % yield)
MS(ES+) m/z = 288.17(M+1)

Step 3 : (S/R)-4-(((R/S)-1-(3-((S/R)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one

**(Compound 102)**

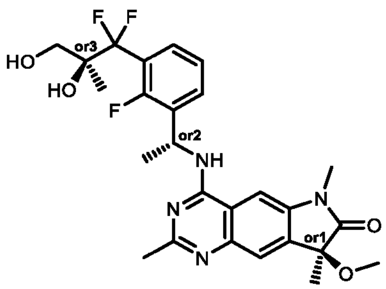

To a suspension of (R/S)-3-(3-((R/S)-1-aminoethyl)-2-fluorophenyl)-3,3-difluoro-2-methylpropane-1,2-diol hydrochloride (0.051 g, 0.171 mmol) **(Example 9-step-6A)** in DIPEA (2 mL) added (S/R)-4,8-dimethoxy-2,6,8-trimethyl-6,8-dihydro-7H-pyrrolo[2,3-g]quinazolin-7-one **(Step 2-peak 2)** (0.041 g, 0.143 mmol) was added at room temperature and resulting reaction mixture was heated at 130°C for the 12h in sealed vial. Reaction mixture was cooled to room temperature, diluted with DCM-MeOH (5.0 mL) and concentrated in vacuum to dryness. The residue was purified by preparative HPLC to furnish titled compound (0.015 g, 20.21%) **(compound 102)**

MS(ES+) m/z = 519.42 (M+1),

$^1$H NMR (400 MHz, DMSO-$d_6$) δ 8.30 (d, *J* = 7.4 *Hz,* 1H), 7.95 (s, 1H), 7.64 - 7.59 (m, 1H), 7.56 (s, 1H), 7.35 - 7.30 (m, 1H), 7.24 - 7.19 (m, 1H), 5.86 - 5.79 (m, 1H), 5.24 (s, 1H), 4.73 - 4.67 (m, 1H), 3.49 - 3.42 (m, 2H), 3.30 (s, 3H), 2.89 (s, 3H), 2.35 (s, 3H), 1.61 (d, *J* = 7.0 *Hz,* 3H), 1.49 (s, 3H), 1.21 (s, 3H).

**Example 116** :- (S/R)-4-(((R/S)-1-(3-((R/S)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one **(Compound 103)**

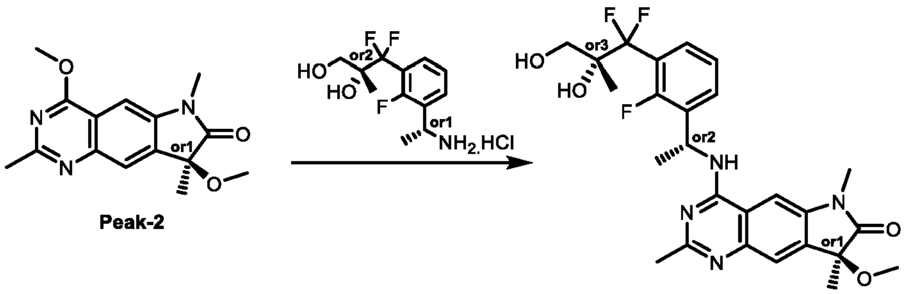


The titled compound was prepared from **Example 115-Step-2 (peak 2)** intermediate and (S/R)-3-(3-((S/R)-1-aminoethyl)-2-fluorophenyl)-3,3-difluoro-2-methylpropane-1,2-diol hydrochloride **(Example 9-step-6b)**,by employing analogous protocol mentioned in **Example 115.**
(0.025 g, 29% yield)

MS(ES+) m/z = 519.42(M+1)

$^{1}$H NMR (400 MHz, DMSO-$d_6$) δ 8.29 (d, *J* = 7.4 *Hz,* 1H), 7.95 (s, 1H), 7.64 - 7.59 (m, 1H), 7.56 (s, 1H), 7.36 - 7.27 (m, 1H), 7.26 - 7.17 (m, 1H), 5.86 - 5.79 (m, 1H), 5.27 (s, 1H), 4.73 - 4.65 (m, 1H), 3.50 - 3.43 (m, 1H), 3.41 - 3.34 (m, 1H), 3.30 (s, 3H), 2.89 (s, 3H), 2.36 (s, 3H), 1.61 (d, J = 7.1 *Hz,* 3H), 1.49 (s, 3H), 1.23 (s, 3H).

**Compounds 101a and 101b (Example 114) could also be synthesized by enantiomers [Mentioned in peak-1 and Peak-2 of step-2(example-115) respectively] using (R)-1-(3-(1-aminoethyl)-2-fluorophenyl)-1,1-difluoro-2-methylpropan-2-ol hydrochloride by employing analogous protocol mentioned in Example 115.**

**Example 117:** Preparation of (R)-8-((1-(3-(1,1-difluoro-2-hydroxy-2-methylpropyl)-2-fluorophenyl)ethyl)amino)-1-ethyl-3,6-dimethyl-1,3-dihydro-2H-imidazo[4,5-g]quinazolin-2-one **(Compound** 104)

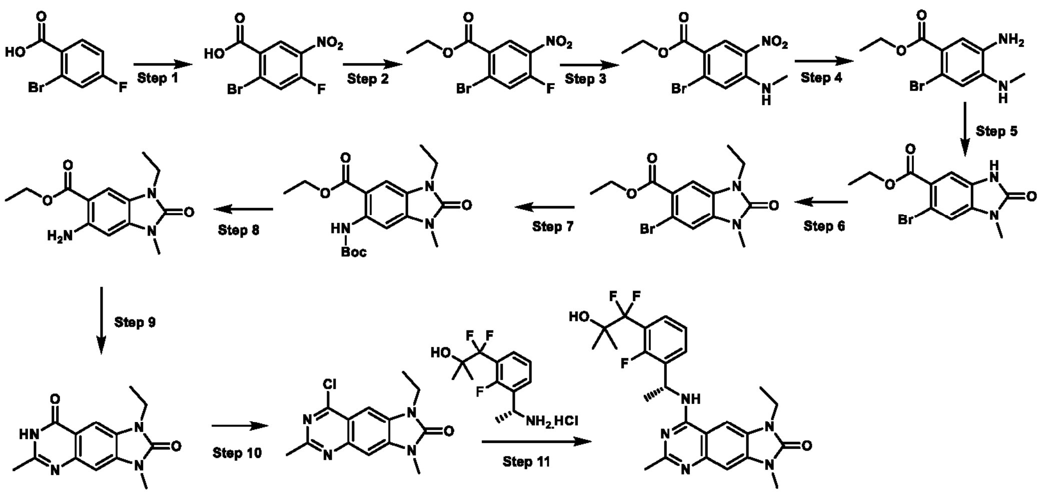


Step 1: 2-Bromo-4-fluoro-5-nitrobenzoic acid

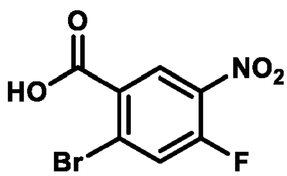

To a stirred solution of 2-bromo-4-fluorobenzoic acid (26.3 g, 120 mmol) in $H_2SO_4$ (235.0 mL) at 0 °C was added $KNO_3$ (12.75 g, 126 mmol) portion wise for over 10 mins, the reaction mixture was stirred at 0°C for 3 h. The reaction mixture was poured on ice water, filtered and dried over reduced pressure to afford the titled compound (31.0 g, 98 % yield).

$^1H$ NMR (400 MHz, DMSO-$d_6$) δ 8.50 (d, *J* = 8.0 Hz, 1H), 8.17 (d, *J*= 10.9 Hz, 1H).

Step 2: Ethyl 2-bromo-4-fluoro-5-nitrobenzoate

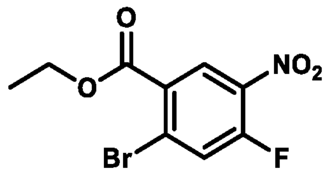

To a stirred solution of 2-bromo-4-fluoro-5-nitrobenzoic acid (25.0 g, 95 mmol) in ethanol (200.0 mL), conc. $H_2SO_4$ (10.0 mL) was added and the reaction mixture was refluxed for 20 h. The reaction was cooled to room temperature and solvent was removed under vacuum. The reaction mixture was quenched with ice cold solution of $NaHCO_3$ and extracted with Ethyl acetate. The combined organic layer was dried over anhydrous $Na_2SO_4$ and concentrated under reduced pressure to afford the titled compound (27 g, 98 % yield).

$^1H$ NMR (400 MHz, DMSO-$d_6$) δ 8.52 (d, *J* = 8.0 Hz, 1H), 8.21 (d, *J* = 10.9 Hz, 1H), 4.38 (q, 2H), 1.35 (t, *J* = 7.1 Hz, 3H).

Step 3: Ethyl 2-bromo-4-(methylamino)-5-nitrobenzoate.

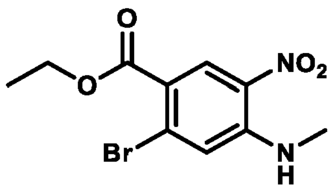

To a stirred solution of ethyl 2-bromo-4-fluoro-5-nitrobenzoate (5.0 g, 17.12 mmol) in MeOH (25.0 mL), methenamine (2.66 g, 34.2 mmol) was added dropwise at 0°C for 10 min. The resulting mixture was stirred for 1h under ice cooling. diethyl ether (50.0 mL) was added and the precipitated solid was filtered. Solid was washed with diethyl ether and dried under vacuum to afford titled compound (4.0 g, 77 % yield)

MS(ES+) m/z = 303.15 (M+1), 305.15 (M+2).

Step 4: Ethyl 5-amino-2-bromo-4-(methylamino)benzoate

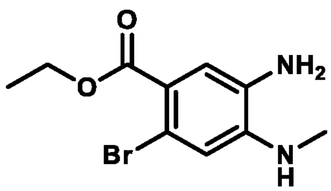

The titled compound was prepared from **Step-3** intermediate by employing analogous protocol mentioned in **Example 14-Step-3** (2.5 g, 92 % yield) as a solid.

MS(ES+) m/z = 273.08 (M+), 275.08(M+2).

Step 5: Ethyl 6-bromo-1-methyl-2-oxo-2,3-dihydro-1H-benzo[d]imidazole-5-carboxylate

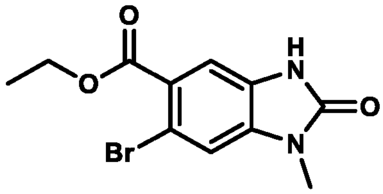

To a stirred solution of ethyl 5-amino-2-bromo-4-(methylamino)benzoate (2.5 g, 9.15 mmol) in DMF (15.0 mL) was added CDI (2.23 g, 13.73 mmol) and the réaction was stirred at 60°C for 1h in sealed tube. The reaction was cooled to room temperature and crushed ice was added, the precipitated solid was filtered and air dried to afford the titled compound (2.5 g, 91 % yield).

MS(ES+) m/z = 297.15 ($M^+$), 299.21 (M+2).

Step 6: Ethyl 6-bromo-3-ethyl-1-methyl-2-oxo-2,3-dihydro-1H-benzo[d]imidazole-5-carboxylate

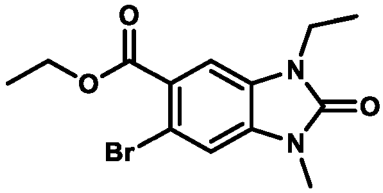

The titled compound was prepared from **Step-5** intermediate by employing analogous protocol mentioned in **Example 71-Step-5** (0.45 g, 82 % yield) as a solid.

MS(ES+) m/z = 327.15 (M), 329.15 (M+2)

Step 7: Ethyl 6-((tert-butoxycarbonyl)amino)-3-ethyl-1-methyl-2-oxo-2,3-dihydro-1H-benzo[d]imidazole-5-carboxylate

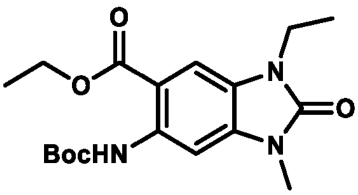

The titled compound was prepared from **Step-6** intermediate by employing analogous protocol mentioned in **Example 71-Step-6** (0.365 g, 72.7 % yield).

MS(ES+) m/z = 363.41 (M+1)

Step 8: Ethyl 6-amino-3-ethyl-1-methyl-2-oxo-2,3-dihydro-1H-benzo[d]imidazole-5-carboxylate

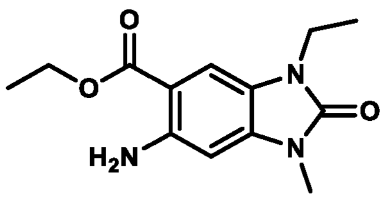

The titled compound was prepared from **Step-7** intermediate by employing analogous protocol mentioned in **Example 71-Step-7** (0.291 g, 99 % yield).

MS(ES+) m/z = 263.14

Step 9: 1-Ethyl-3,6-dimethyl-1H-imidazo[4,5-g]quinazoline-2,8(3H,7H)-dione

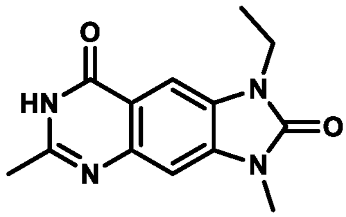

The titled compound was prepared from **Step-8** intermediate by employing analogous protocol mentioned in **Example 71-Step-8** (0.195 g, 74.4 % yield).

MS(ES+) m/z = 258.39 (M+)

Step 10: 8-Chloro-1-ethyl-3,6-dimethyl-1H-imidazo[4,5-g]quinazolin-2(3H)-one

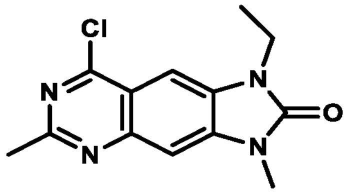

The titled compound was prepared from **Step-9** intermediate by employing analogous protocol mentioned in **Example 71-Step-9** (0.200 g, 98 % yield).

MS(ES+) m/z = 277.15 (M+1).

Step 11: (R)-8-((1-(3-(1,1-difluoro-2-hydroxy-2-methylpropyl)-2-fluorophenyl)ethyl)amino)-1-ethyl-3,6-dimethyl-1H-imidazo[4,5-g]quinazolin-2(3H)-one **(Compound 104)**

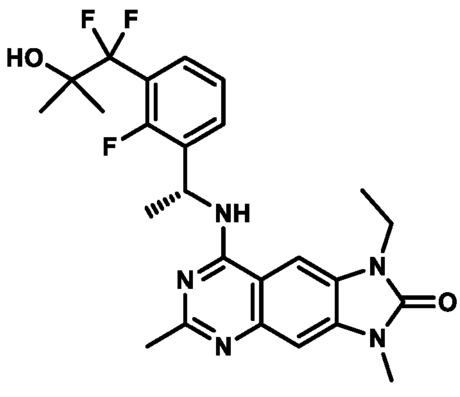

The titled compound was prepared by reaction of **Step-10** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.065 g, 36.9 % yield)

MS(ES+) m/z = 488.36 (M+1).

$^1$H NMR (400 MHz, DMSO-$d_6$) δ 8.18 (s, 1H), 8.11(s, 1H), 7.66 - 7.57 (m, 1H), 7.35 - 7.27 (m, 2H), 7.25 - 7.17 (m, 1H), 5.88 - 5.79 (m, 1H), 5.34 (s, 1H), 4.01 - 3.93 (m, 2H), 3.39 (s, 3H), 2.33 (s, 3H), 1.69 - 1.56 (m, 3H), 1.39 - 1.31 (m, 3H), 1.24 (s, 3H), 1.23 - 1.19 (m, 3H).

**Example 118:** Preparation of (R)-8-((1-(3-(1,1-difluoro-2-hydroxy-2-methylpropyl)-2-fluoro phenyl)ethyl)amino)-1-isopropyl-3,6-dimethyl-1,3-dihydro-2H-imidazo[4,5-g]quinazolin-2-one **(Compound 105)**

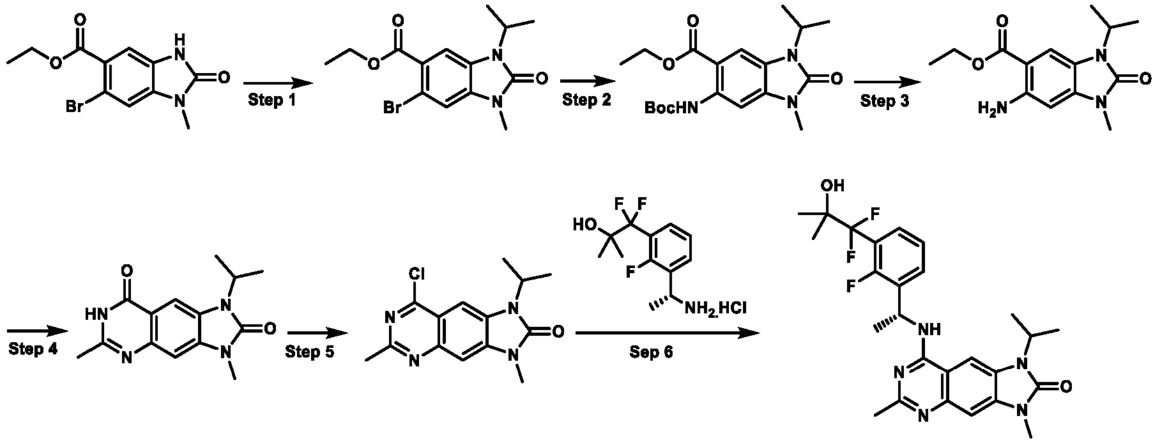


Step 1: Ethyl 6-bromo-3-isopropyl-1-methyl-2-oxo-2,3-dihydro-1H-benzo[d]imidazole-5-carboxylate

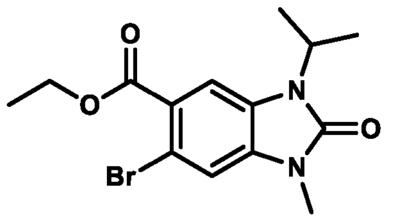

The titled compound was prepared from **Example 117 Step-5** intermediate and appropriate alkyl halide by employing analogous protocol mentioned in **Example 71-Step-5** ( 0.25 g, 54.8% yield)
MS(ES+) m/z = 343.22 (M+2).

Step 2: Ethyl 6-((tert-butoxycarbonyl)amino)-3-isopropyl-1-methyl-2-oxo-2,3-dihydro-1H-benzo[d]imidazole-5-carboxylate

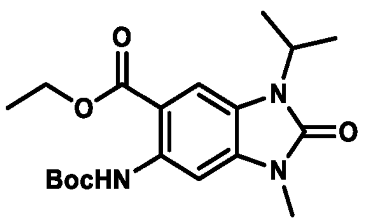

The titled compound was prepared from **Step-1** intermediate by employing analogous protocol mentioned in **Example 71-Step-6** ( 0.18 g, 86% yield)
MS(ES+) m/z = 378.28 (M+1).

Step 3: Ethyl 6-amino-3-isopropyl-1-methyl-2-oxo-2,3-dihydro-1H-benzo[d]imidazole-5-carboxylate

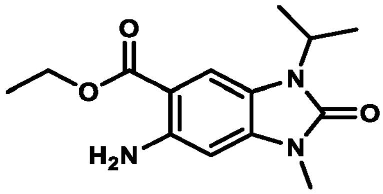

The titled compound was prepared from **Step-2** intermediate by employing analogous protocol mentioned in **Example 71-Step-7** (0.14 g, 94 % yield).

MS(ES+) m/z = 278.28 (M+1).

Step 4: 1-Isopropyl-3,6-dimethyl-1H-imidazo[4,5-g]quinazoline-2,8(3H,7H)-dione

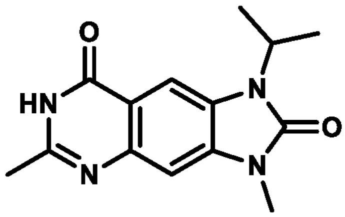

The titled compound was prepared from **Step-3** intermediate by employing analogous protocol mentioned in **Example 71-Step-8** (0.10 g, 82 % yield).

MS(ES+) m/z = 273.15 (M+1).

Step 5: 8-Chloro-1-isopropyl-3,6-dimethyl-1H-imidazo[4,5-g]quinazolin-2(3H)-one

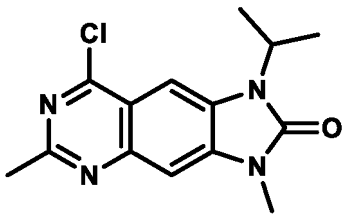

The titled compound was prepared from **Step-4** intermediate by employing analogous protocol mentioned in **Example 71-Step-9** (0.080 g, 74.9 % yield).

MS(ES+) m/z = 290.40 (M+1).

Step 6: (R)-8-((1-(3-(1,1-difluoro-2-hydroxy-2-methylpropyl)-2-fluorophenyl)ethyl)amino)-1-isopropyl-3,6-dimethyl-1H-imidazo[4,5-g]quinazolin-2(3H)-one **(Compound 105)**

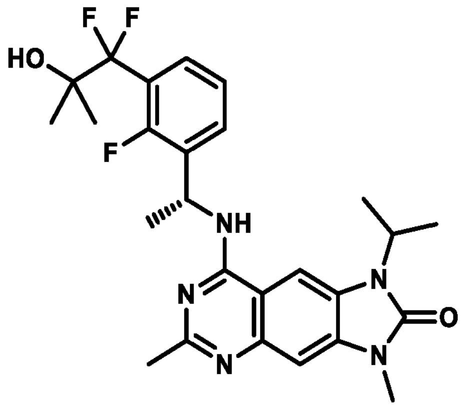

The titled compound was prepared by reaction of **Step-5** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** ( 0.010 g, 9.66 % yield)

MS(ES+) m/z = 502.43 (M+1)

$^1$H NMR (400 MHz, DMSO-$d_6$) δ 8.19 (d, *J* = 7.3 Hz, 1H), 8.10 (s, 1H), 7.66 - 7.56 (m, 1H), 7.36 - 7.26 (m, 2H), 7.26 - 7.18 (m, 1H), 5.93 - 5.81 (m, 1H), 5.34 (s, 1H), 4.74 - 4.62 (m, 1H), 3.36 (s, 3H), 2.32 (s, 3H), 1.68 - 1.52 (m, 9H), 1.24 (s, 3H), 1.22 (s, 3H).

**Example 119:** Preparation of (R)-8-((1-(3-(1,1-difluoro-2-hydroxy-2-methylpropyl)-2-fluorophenyl)ethyl)amino)-1-(2,2-difluoroethyl)-3,6-dimethyl-1H-imidazo[4,5-g]quinazolin-2(3H)-one **(Compound 106)**

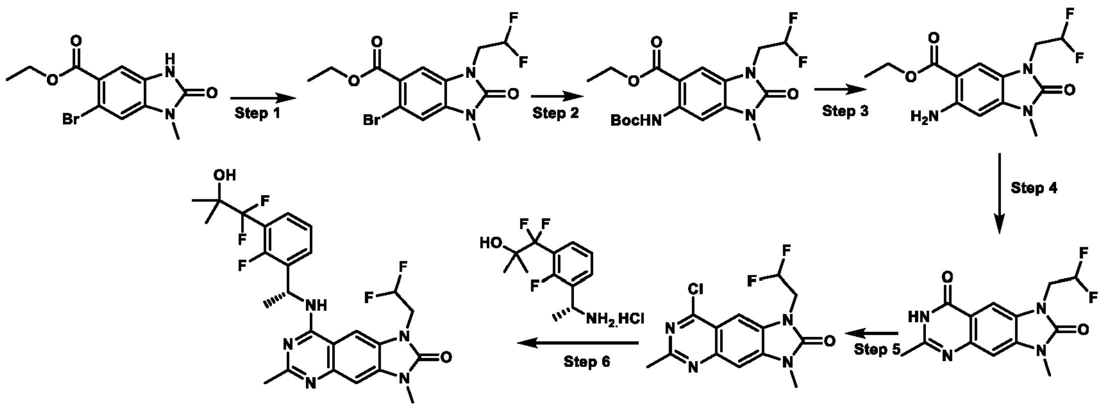


Step 1: Ethyl 6-bromo-3-(2,2-difluoroethyl)-1-methyl-2-oxo-2,3-dihydro-1H-benzo[d]imidazole-5-carboxylate

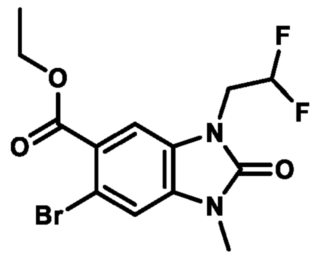

The titled compound was prepared from **Example 117-Step-5** intermediate and 1,1-difluoro-2-iodoethane using analogous reaction protocol mentioned in **Example 71-Step-5** (0.24 g, 39.5 % yield)

MS(ES+) m/z = 363.22 (M+), 365.10 (M+2).

Step 2: Ethyl 6-((tert-butoxycarbonyl)amino)-3-(2,2-difluoroethyl)-1-methyl-2-oxo-2,3-dihydro-1H-benzo[d]imidazole-5-carboxylate

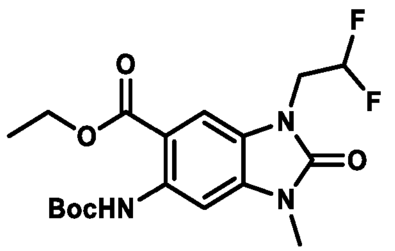

The titled compound was prepared from **Step-1** intermediate by employing analogous protocol mentioned in **Example 71-Step-6** (0.140 g, 55.3 % yield).

MS(ES+) m/z = 400.10 (M+1)

Step 3: Ethyl 6-amino-3-(2,2-difluoroethyl)-1-methyl-2-oxo-2,3-dihydro-1H-benzo[d]imidazole-5-carboxylate

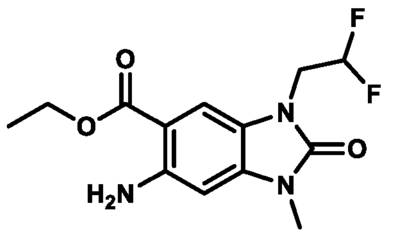

The titled compound was prepared from **Step-2** intermediate by employing analogous protocol mentioned in **Example 71-Step-7** (0.115 g, 98 % yield).

MS(ES+) m/z = 300.21(M+1)

Step 4: 1-(2,2-Difluoroethyl)-3,6-dimethyl-1H-imidazo[4,5-g]quinazoline-2,8(3H,7H)-dione10716-297

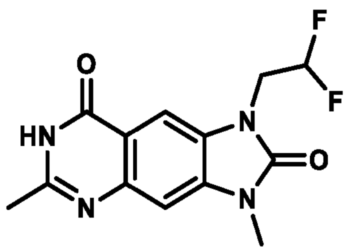

The titled compound was prepared from **Step-3** intermediate by employing analogous protocol mentioned in **Example 71-Step-8** (0.070 g, 69.4 % yield).

MS(ES+) m/z = 295.15 (M+1)

Step 5: 8-Chloro-1-(2,2-difluoroethyl)-3,6-dimethyl-1H-imidazo[4,5-g]quinazolin-2(3H)-one

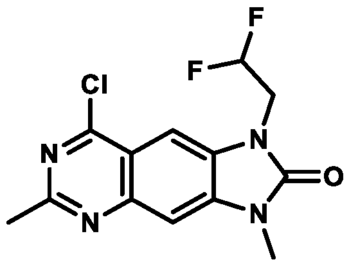

The titled compound was prepared from Step-4 intermediate by employing analogous protocol mentioned in **Example 71-Step-9** (0.065 g, 87 % yield).
MS(ES+) m/z = 313.21(M+1)

Step 6: (R)-8-((1-(3-(1,1-difluoro-2-hydroxy-2-methylpropyl)-2-fluorophenyl)ethyl)amino)-1-(2,2-difluoroethyl)-3,6-dimethyl-1H-imidazo[4,5-g]quinazolin-2(3H)-one **(Compound 106)**

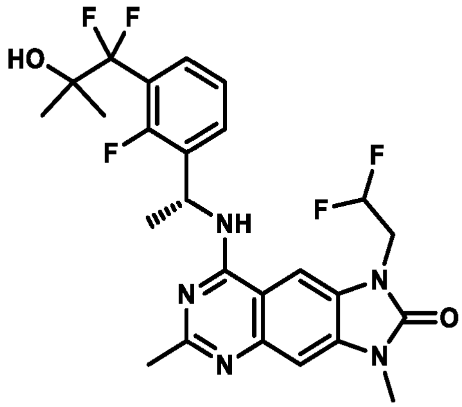

The titled compound was prepared by reaction of **Step-5** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.025 g, 22.97 % yield)
MS(ES+) m/z = 524.32 (M+1).
$^1$H NMR (400 MHz, DMSO-$d_6$) δ 8.16 (s, 1H), 8.14 - 8.08 (m, 1H), 7.64 - 7.57 (m, 1H), 7.35 - 7.28 (m, 2H), 7.25 - 7.19 (m, 1H), 6.66 - 6.31 (m, 1H), 5.88 - 5.79 (m, 1H), 5.34 (s, 1H), 4.41 - 4.28 (m, 2H), 3.41 (s, 3H), 2.33 (s, 3H), 1.61 (d, *J* = 7.0 *Hz,* 3H), 1.25 (s, 3H), 1.21 (s, 3H).
MS(ES+) m/z = 524.32 (M+1).

**Example 120:** ((R)-1,1-difluoro-1-(2-fluoro-3-(1-((1,2,2,3,6-pentamethyl-2,3-dihydro-1H-imidazo[4,5-g]quinazolin-8-yl) amino)ethyl)phenyl)-2-methylpropan-2-ol

**(Compound 107) and (Compound 108)**

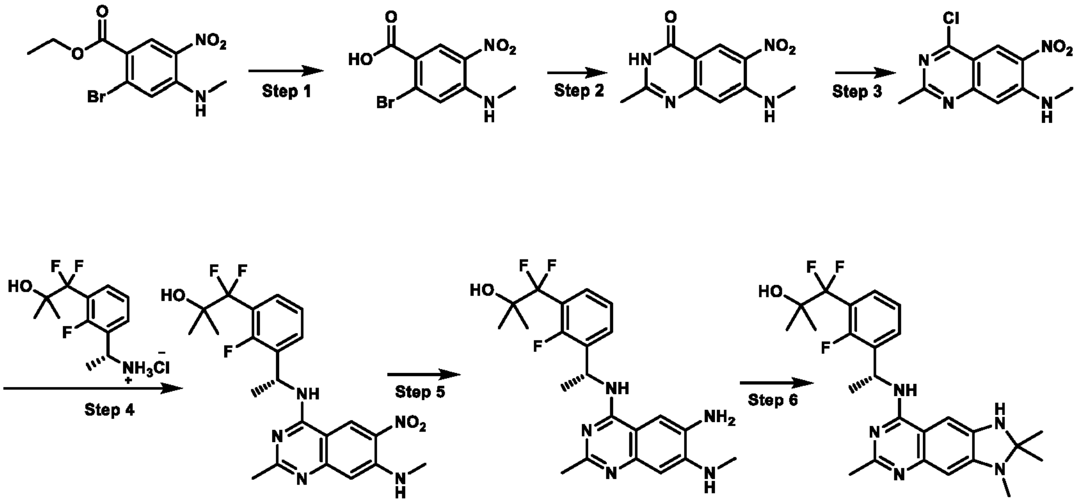


Step 1: 2-Bromo-4-(methylamino)-5-nitrobenzoic acid

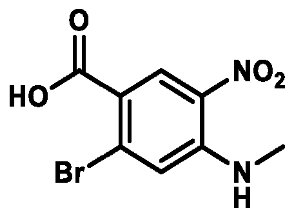

The titled compound was prepared from ethyl 2-bromo-4-(methylamino)-5-nitrobenzoate **Example 117-Step-3** by employing analogous protocol mentioned in **Example 81-step-4a.** (1.51 g, 83 % yield).

$^1$H NMR (400 MHz, DMSO-$d_6$) δ 12.62 (s, 1H), 8.62 (s, 1H), 8.54 - 8.46 (m, 1H), 7.26 (s, 1H), 3.05 - 2.95 (m, 3H).

Step 2: 2-Methyl-7-(methylamino)-6-nitroquinazolin-4(3H)-one

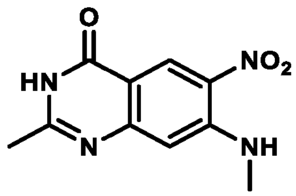

The titled compound was prepared from Step-1 intermediate by employing analogous protocol mentioned in **Example 81a-Step5a** (0.100 g, 58.7 % yield)

MS(ES+) m/z = 235.08.

Step 3: 4-Chloro-N,2-dimethyl-6-nitroquinazolin-7-amine

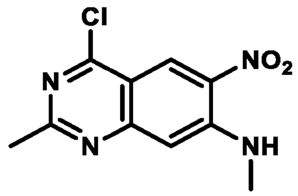

The titled compound was prepared from Step-2 intermediate by employing analogous protocol mentioned in Example **71-Step-9** (0.10 g, 54.5 % yield).
MS(ES+) m/z = 253.08 (M+1).

Step 4: (R)-1,1-difluoro-1-(2-fluoro-3-(1-((2-methyl-7-(methylamino)-6-nitroquinazolin-4-yl)amino) ethyl)phenyl)-2-methylpropan-2-ol

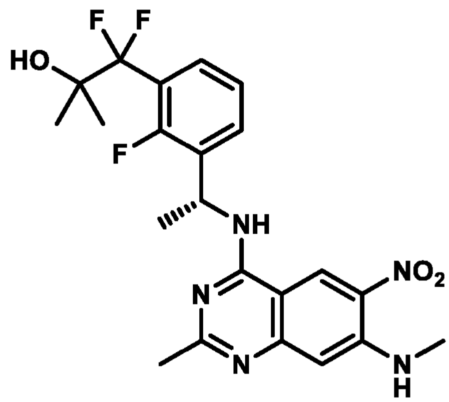

The titled compound was prepared by Step-3 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in Example **71-Step-10** (0.110 g, 60.0 % yield).
MS(ES+) m/z = 464.30 (M+1).

Step 5: (R)-1-(3-(1-((6-amino-2-methyl-7-(methylamino)quinazolin-4-yl)amino)ethyl)-2-fluorophenyl)-1,1-difluoro-2-methylpropan-2-ol

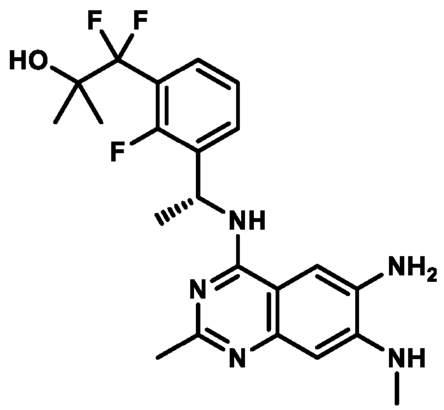

The titled compound was prepared from Step-4 intermediate by employing analogous protocol mentioned in **Example 14-Step-3** (0.58 g, 98.0 % yield).
MS(ES+) m/z = 434.23 (M+1).

Step 6: (R)-1,1-difluoro-1-(2-fluoro-3-(1-((2,2,3,6-tetramethyl-2,3-dihydro-1H-imidazo[4,5-g]quinazolin-8-yl)amino) ethyl)phenyl)-2-methylpropan-2-ol

**(Compound 107)**

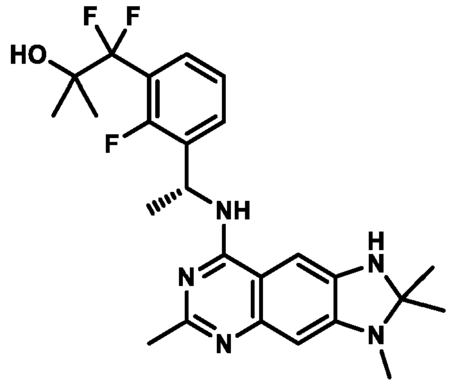

To a stirred solution of (R)-1-(3-(1-((6-amino-2-methyl-7-(methylamino)quinazolin-4-yl)amino)ethyl)-2-fluorophenyl)-1,1-difluoro-2-methylpropan-2-ol (0.20 g, 0.461 mmol) in acetone (6.0 mL), pTsOH (0.088 g, 0.461 mmol) was added. The reaction mixture was heated at 100 °C for 2 h. Reaction mixture was concentrated. Ethyl acetate (20.0 mL) was added to the residue and 6.0 mL $NaHCO_3$ aq solution resulting mixture filtered through celite bed pad. The filtrate was extracted with Ethyl acetate (3 x 10.0 mL) and the combined organic layers were dried over $Na_2SO_4$. Organic layer was concentrated in vacuum and the crude product was purified by flash chromatography using 0-40% Ethyl acetate-n-hexane as eluent to afford the titled compound (0.218 g, 92.0 % yield).

MS(ES+) m/z = 474.36 (M+1).

$^1$H NMR (400 MHz, DMSO-$d_6$) δ 7.61 - 7.51 (m, 1H), 7.41 (d, *J* = 7.8 *Hz,* 1H), 7.32 - 7.23 (m, 1H), 7.20 - 7.13 (m, 1H), 6.89 (s, 1H), 6.45 (s, 1H), 6.11 (s, 1H), 5.83 - 5.67 (m, 1H), 5.31 (s, 1H), 2.77 (s, 3H), 2.19 (s, 3H), 1.50 (d, *J* = 7.1 *Hz*, 3H), 1.40 (s, 3H), 1.39 (s, 3H), 1.24 (s, 3H), 1.21 (s, 3H).

**Example 121:** (R)-1,1-difluoro-1-(2-fluoro-3-(1-((1,2,2,3,6-pentamethyl-2,3-dihydro-1H-imidazo[4,5-g]quinazolin-8-yl) amino)ethyl)phenyl)-2-methylpropan-2-ol **(Compound 108)**

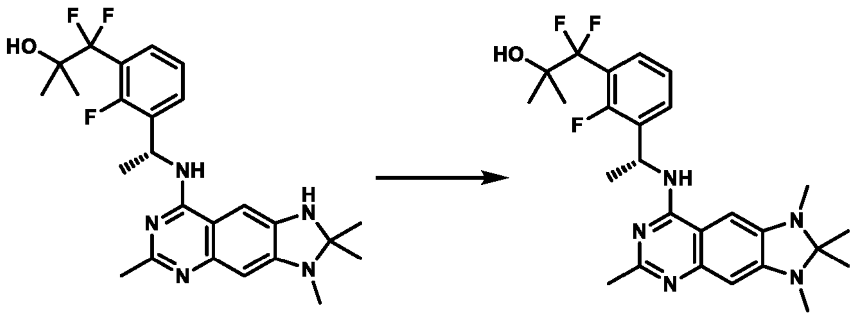

To a stirred solution of (R)-1,1-difluoro-1-(2-fluoro-3-(1-((2,2,3,6-tetramethyl-2,3-dihydro-1H-imidazo[4,5-g]quinazolin-8-yl)amino)ethyl)phenyl)-2-methylpropan-2-ol (0.030 g, 0.063 mmol) **(Example 120-compound 107)** in AcOH (1.0 mL) was added paraformaldehyde (0.019 g, 0.634 mmol) and stirred for the 1h at room temperature. sodium cyanoborohydride (0.020 g, 0.317 mmol) was added subsequently and the reaction was stirred for 1h at room temperature. Reaction was quenched with MeOH (2.0 mL) and concentrated in vacuum to afford crude product. The crude compound was purified by preparative HPLC to afford the titled compound (0.007 g, 22.66 % yield).

$^1$H NMR (400 MHz, DMSO-$d_6$) δ 8.20 (s, 1H), 7.65 (d, *J* = 7.7 *Hz,* 1H), 7.62 - 7.54 (m, 1H), 7.33 - 7.25 (m, 1H), 7.23 - 7.17 (m, 1H), 6.82 (s, 1H), 6.13 (s, 1H), 5.82 - 5.73 (m, 1H), 2.86 (s, 3H), 2.82 (s, 3H), 2.24 (s, 3H), 1.55 (d, *J*= 7.0 *Hz,* 3H), 1.38 (s, 3H), 1.36 (s, 3H) 1.24 (s, 3H), 1.21 (s, 3H).

MS(ES+) m/z = 488.36 (M+1).

**Example No-122:** (R)-8-((1-(3-(1,1-difluoro-2-hydroxy-2-methylpropyl)-2-fluorophenyl)ethyl)amino)-1,3,6-trimethyl-1,3-dihydro-2H-imidazo[4,5-g]quinazolin-2-one **(Compound 109)**

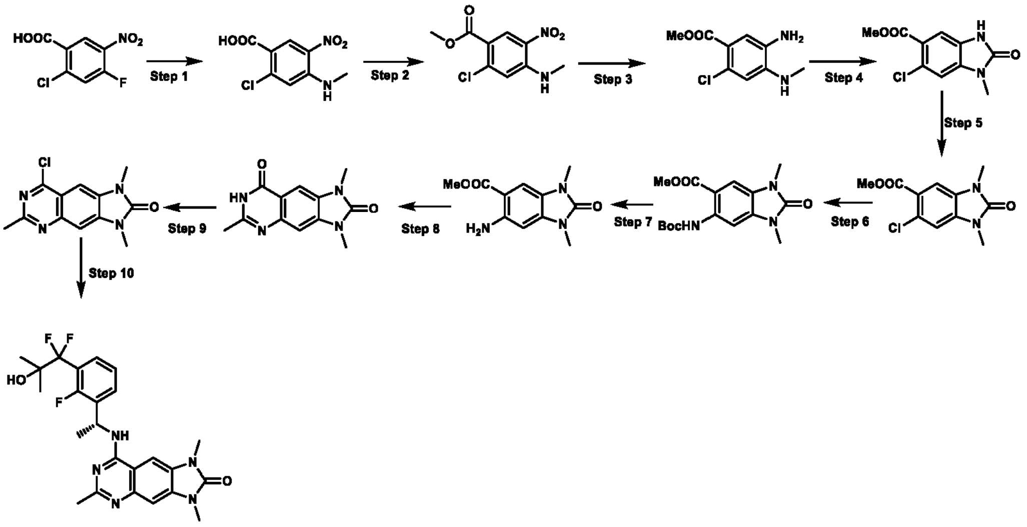


Step 1:- 2-chloro-4-(methylamino)-5-nitrobenzoic acid

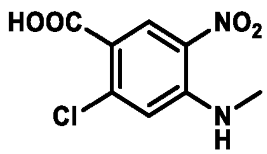

methenamine (3.54 g, 45.5 mmol) is added to an ice-cooled mixture of 2-chloro-4-fluoro-5-nitrobenzoic acid (10.0 g, 45.5 mmol) and water (150.0 mL). and the resulting mixture was stirred at room for 5h. reaction mixture was acidified with 10% HCl. The solid was filtered, washed with water and dried under vacuum to yield the titled compound (10.0 g, 95 % yield).

$^{1}H$ NMR (400 MHz, DMSO-$d_6$) δ 8.65 (s, 1H), 8.53 (q, *J*= 4.9 Hz, 1H), 7.06 (s, 1H), 2.99 (d, *J* = 4.9 Hz, 3H).

Step 2:- Methyl 2-chloro-4-(methylamino)-5-nitrobenzoate

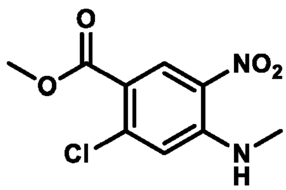

To a stirred solution of 2-chloro-4-(methylamino)-5-nitrobenzoic acid (10.5 g, 45.5 mmol) in MeOH (150 mL) was added thionyl chloride (4.98 mL , 68.3 mmol) at 0°C. The resulting mixture was stirred at 80°C for 4h. The reaction was cooled to room temperature and solvent was evaporated. The residue was suspended in cold water (100.0 mL), filtered under vacuum and dried to afford the titled compound. (11.0 g, 99 % yield) $^{1}H$ NMR (400 MHz, DMSO-$d_6$) δ 8.62 (s, 1H), 8.58 (q, *J*= 5.0 *Hz,* 1H), 7.06 (s, 1H), 3.82 (s, 3H), 2.99 (d, *J* = 5.0 Hz, 3H).

Step 3:- methyl 5-amino-2-chloro-4-(methylamino)benzoate

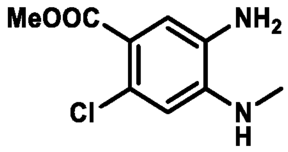

To a stirred solution of methyl 2-chloro-4-(methylamino)-5-nitrobenzoate (6.0 g, 24.53 mmol)in ethanol (60.0 mL) was added iron(6.85 g, 123 mmol), aq. $NH_4Cl$ (35.0 mL , 245 mmol) . The resulting mixture was stirred at 80 °C for 3h. Reaction was cooled to room temperature and filtered over celite bed. Celite bed was washed with 20% Ethyl acetate: MeOH. Combined filtrate was evaporated, residue thus obtained was dissolved in Ethyl acetate (2 x 100.0 mL). Combined organic layer was washed with saturated sodium bicarbonate, brine and dried over anhydrous $Na_2SO_4$. Solvent was evaporated and residue was purified by flash chromatography in hexane-Ethyl acetate gradient to afford titled compound (1.8 g, 34.2 % yield).

$^1$H NMR (400 MHz, DMSO-$d_6$) δ 7.14 (s, 1H), 6.35 (s, 1H), 5.64 (q, *J* = 4.8 *Hz,* 1H), 4.83 (s, 2H), 3.73 (s, 3H), 2.77 (d, *J* = 4.8 *Hz,* 3H).

Step 4:- Methyl 6-chloro-1-methyl-2-oxo-2,3-dihydro-1H-benzo[d]imidazole-5-carboxylate

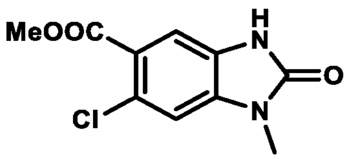

The titled compound was prepared from Step - 3 intermédiate by employing analogous protocol mentioned in **Example 117-Step-5.** (2.3 g, 93 % yield) as a solid.

$^1$H NMR (400 MHz, DMSO-$d_6$) δ 11.23 (s, 1H), 7.41 (s, 1H), 7.36 (s, 1H), 3.83 (s, 3H), 3.31 (s, 3H).

Step 5:- methyl 6-chloro-1,3-dimethyl-2-oxo-2,3-dihydro-1H-benzo[d]imidazole-5-carboxylate

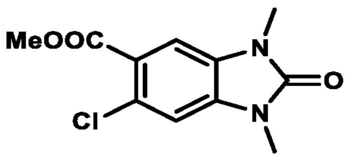

The titled compound was prepared from Step - 4 intermédiate by employing analogous protocol mentioned in **Example 71-Step-5.** (1.1 g, 80 % yield) .

$^1$H NMR (400 MHz, DMSO-$d_6$) δ 7.61 (s, 1H), 7.44 (s, 1H), 3.86 (s, 3H), 3.36 (s, 6H).

Step 6:- Methyl 6-((tert-butoxycarbonyl)amino)-1,3-dimethyl-2-oxo-2,3-dihydro-1H-benzo[d]imidazole-5-carboxylate

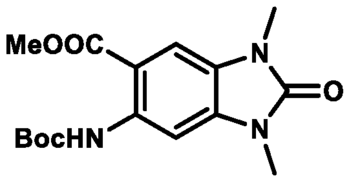

The titled compound was prepared from Step - 5 interemdiate by employing analogous protocol mentioned in **Example 71-Step-6** (0.70 g, 76 % yield).

$^1$H NMR (400 MHz, DMSO-$d_6$) δ 10.47 (s, 1H), 8.01 (s, 1H), 7.63 (s, 1H), 3.87 (s, 3H), 3.33 (d, *J* = 1.9 Hz, 6H), 1.50

(s, 9H).

Step 7:- Methyl 6-amino-1,3-dimethyl-2-oxo-2,3-dihydro-1H-benzo[d]imidazole-5-carboxylate

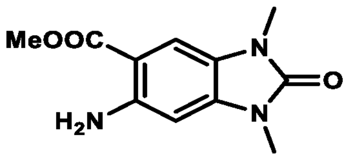

The titled compound was prepared from Step - 6 intermédiate by employing analogous protocol mentioned in **Example 71-Step-7.** (0.49 g , 99 % yield).

$^1$H NMR (400 MHz, DMSO-$d_6$) δ 7.37 (s, 1H), 6.51 (s, 1H), 3.79 (s, 3H), 3.26 (s, 3H), 3.24 (s, 3H).

Step 8:- 1,3,6-trimethyl-1,7-dihydro-2H-imidazo[4,5-g]quinazoline-2,8(3H)-dione

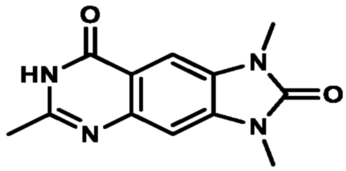

The titled compound was prepared from Step - 7 intermédiate by employing analogous protocol mentioned in **Example 71-Step-8.** (0.25 g, 60.2 % yield)

$^1$H NMR (400 MHz, DMSO-$d_6$) δ 12.03 (s, 1H), 7.67 (s, 1H), 7.28 (s, 1H), 3.40 (s, 3H), 3.39 (s, 3H), 2.34 (s, 3H).

Step 9:- 8-chloro-1,3,6-trimethyl-1,3-dihydro-2H-imidazo[4,5-g]quinazolin-2-one

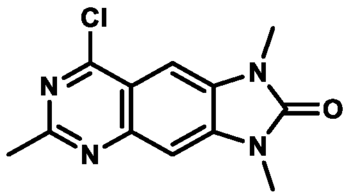

The titled compound was prepared from Step - 8 intermédiate by employing analogous protocol mentioned in **Example 71-Step-9.** (0.060 g, 46.5 % yield) .

$^1$H NMR (400 MHz, DMSO-$d_6$) δ 7.71 (s, 1H), 7.65 (s, 1H), 3.48 (s, 3H), 3.46 (s, 3H), 2.70 (s, 3H).

**Step-10** (R)-8-((1-(3-(1,1-difluoro-2-hydroxy-2-methylpropyl)-2-fluorophenyl)ethyl)amino)-1,3,6-trimethyl-1,3-dihydro-2H-imidazo[4,5-g]quinazolin-2-one **(Compound 109)**

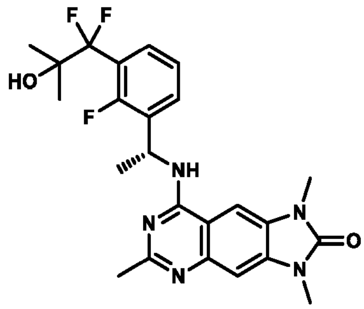

The titled compound was prepared by reaction of Step - 9 intermédiate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10.** (0.040 g, 40% yield).
MS(ES+) m/z = 474.42 (M+1)
$^1$H NMR (400 MHz, DMSO-$d_6$) δ 8.14 (d, $J$ = 7.4 Hz, 1H), 8.07 (s, 1H), 7.64 - 7.57 (m, 1H), 7.36 - 7.27 (m, 2H), 7.25 - 7.19 (m, 1H), 5.91 - 5.76 (m, 1H), 5.33 (s, 1H), 3.45 (s, 3H), 3.38 (s, 3H), 2.32 (s, 3H), 1.60 (d, $J$ = 7.0 Hz, 3H), 1.24 (s, 3H), 1.22 (s, 3H).

**Example 123:** (R)-2-cyclopropyl-4-((1-(3-(1,1-difluoro-2-hydroxy-2-methylpropyl)-2-fluorophenyl)ethyl)amino)-6,6,8-trimethyl-6,8-dihydro-7H-pyrrolo[3,2-g]quinazolin-7-one

**(Compound 110)**

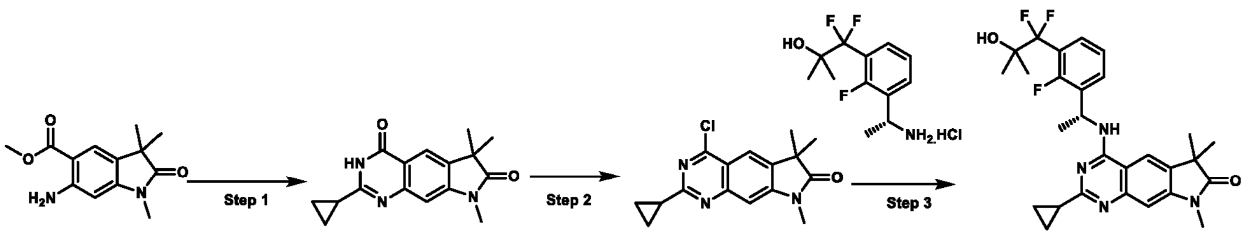


Step-1: 2-cyclopropyl-6,6,8-trimethyl-6,8-dihydro-3H-pyrrolo[3,2-g]quinazoline-4,7-dione

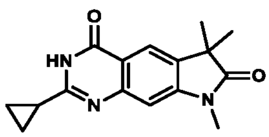

The titled compound was prepared from **Example-93-Step-7** intermediate and cyclopropyl nitrile using analogous protocol mentioned in Example 71-Step-8 ( 0.450 g, 90% yield)
MS(ES+) m/z = 284.34(M+1)

Step-2: 4-chloro-2-cyclopropyl-6,6,8-trimethyl-6,8-dihydro-7H-pyrrolo[3,2-g]quinazolin-7-one

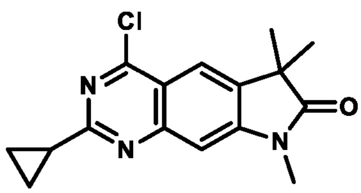

The titled compound was prepared from **Step-1** intermediate by employing analogous protocol mentioned in **Example 71-Step-9** (0.100 g, 94% yield)

MS(ES+) m/z = 302.34 (M +1)

**Step-3:** (R)-2-cyclopropyl-4-((1-(3-(1,1-difluoro-2-hydroxy-2-methylpropyl)-2-fluorophenyl)ethyl)amino)-6,6,8-trimethyl-6,8-dihydro-7H-pyrrolo[3,2-g]quinazolin-7-one **(Compound 110)**

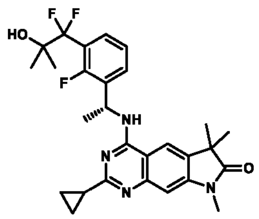

The titled compound was prepared by reaction of Step - **2** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10**

MS(ES+) m/z = 512.32 (M+1)

1H NMR (400 MHz, DMSO-$d_6$) δ 8.33 (s, 1H), 8.14 (d, *J* = 6.8 Hz, 1H), 7.55 - 7.49 (m, 1H), 7.34 - 7.27 (m, 1H), 7.23 - 7.16 (m, 1H), 7.06 (s, 1H), 5.69 - 5.58 (m, 1H), 5.32 (s, 1H), 3.21 (s, 3H), 1.90 - 1.81 (m, 1H), 1.58 (d, *J*= 7.0 Hz, 3H), 1.39 (s, 3H), 1.38 (s, 3H), 1.24 (s, 3H), 1.22 (s, 3H), 1.00 - 0.93 (m, 1H), 0.84 - 0.77 (m, 1H), 0.73 - 0.66 (m, 1H), 0.59 - 0.51 (m, 1H).

**Example 124:** (R)-8-((1-(3-(1,1-difluoro-2-hydroxy-2-methylpropyl)-2-fluorophenyl)ethyl)amino)-1,6-dimethyloxazolo [4,5-g]quinazolin-2(1H)-one **(Compound 111)**

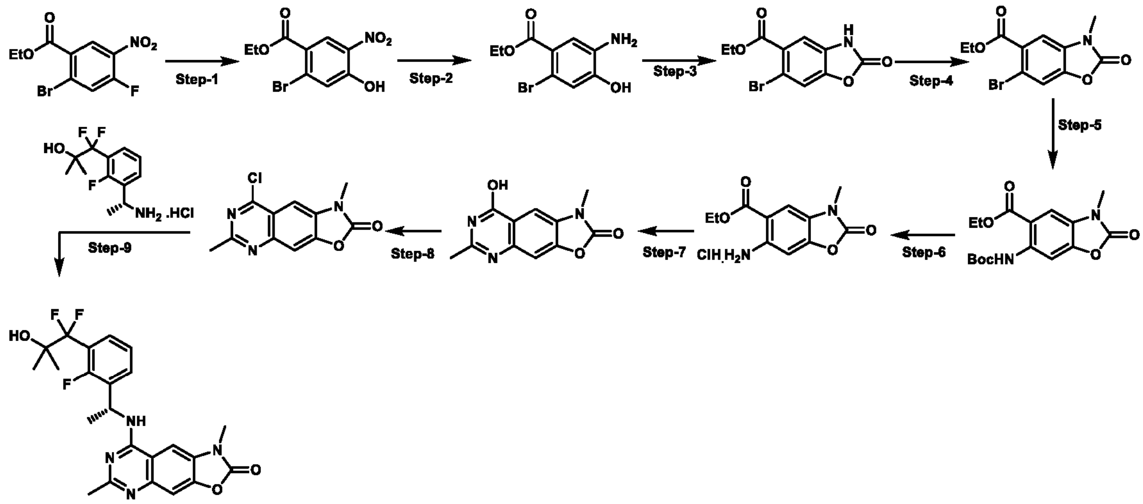


Step-1: Ethyl 2-bromo-4-hydroxy-5-nitrobenzoate

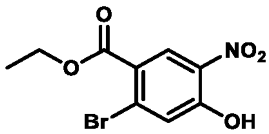

To a stirred solution of N-hydroxyacetamide (4.63 g, 61.6 mmol) in DMSO ( 40.0 mL) was added potassium

carbonate (14.20 g, 103 mmol) and ethyl 2-bromo-4-fluoro-5-nitrobenzoate (6.0 g, 20.54 mmol) in a screwcap vial. The vessel was sealed and heated at 80 °C for 2 h. The reaction mixture was cooled to room temperature, acidified with 2M hydrochloric acid (pH= 3 to 6) and extracted with Ethyl acetate (100.0 mL). Organic layer was separated, washed with brine (50.0 mL) and dried over anhydrous Na2SO4. Solvent was evaporated and residue was purified over flash chromatography to get titled compound (5.2 g, 87 % yield) as yellow solid.

MS(ES+) m/z = 290.52(M+)

Step-2: Ethyl 5-amino-2-bromo-4-hydroxybenzoate

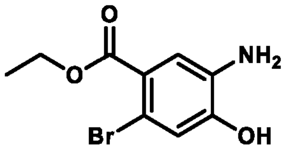

The titled compound was prepared from Step-**1** intermediate by employing analogous protocol mentioned in **Example 14-Step-3** (3.1 g, 69.1 % yield) as off white solid.

MS(ES+) m/z = 261.96 (M+2)

Step- 3: Ethyl 6-bromo-2-oxo-2,3-dihydrobenzo[d]oxazole-5-carboxylate

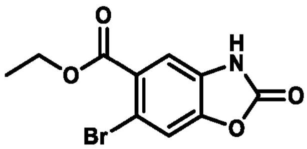

To a stirred solution of ethyl 5-amino-2-bromo-4-hydroxybenzoate (3.1 g, 11.92 mmol) in anhydrous THF (20.0 mL) at 25 °C was added CDI (2.12 g, 13.11 mmol) and the reaction was stirred at room temperature for 2h. Reaction was quenched with aqueous hydrochloric acid solution (50.0 mL) and the aqueous phase was extracted with Ethyl acetate (100.0 mL). The organic layer was separated, washed with brine (50.0 mL), dried over anhydrous $Na_2SO_4$ and concentrated to afford titled compound (1.3 g, 38.1 % yield)

$^1$H NMR (400 MHz, DMSO-$d_6$) δ 12.03 (s, 1H), 7.76 (s, 1H), 7.43 (s, 1H), 4.32 (q, *J* = 7.1 Hz, 2H), 1.34 (t, J = 7.0 Hz, 3H).

Step-4: Ethyl 6-bromo-3-methyl-2-oxo-2,3-dihydrobenzo[d]oxazole-5-carboxylate

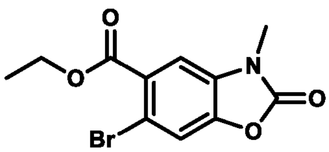

The titled compound was prepared from Step-**3** intermediate by employing analogous protocol mentioned in **Example 71-Step-5** (1.25 g, 92 % yield) as off white solid.

$^1$H NMR (400 MHz, DMSO-$d_6$) δ 7.80 (s, 1H), 7.65 (s, 1H), 4.34 (q *J* = 7.1 Hz, 2H), 3.36 (s, 3H), 1.34 (t, *J* = 7.0 Hz, 3H).

Step-5: Ethyl 6-((tert-butoxycarbonyl)amino)-3-methyl-2-oxo-2,3-dihydrobenzo[d]oxazole-5-carboxylate

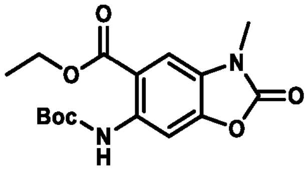

The titled compound was prepared from Step-**4** intermediate by employing analogous protocol mentioned in **Example 71-Step-6** (0.41g, 91 % yield).

MS(ES+) m/z = 337.40 (M+1)

Step-6: Ethyl 6-amino-3-methyl-2-oxo-2,3-dihydrobenzo[d]oxazole-5-carboxylate

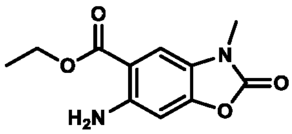

The titled compound was prepared from Step-**5** intermediate by employing analogous protocol mentioned in **Example 71-Step-7** (0.33 g, 99 % yield).

MS(ES+) m/z = 237.20 (M)

Step-7: 1,6-dimethyl-1,7-dihydrooxazolo[4,5-g]quinazoline-2,8-dione

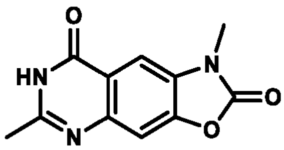

The titled compound was prepared from Step-**6** intermediate by employing analogous protocol mentioned in **Example 71-Step-8** (0.017 g, 60.8 % yield).

MS(ES+) m/z = 232.20 (M+1)

Step - 8: 8-chloro-1,6-dimethyloxazolo[4,5-g]quinazolin-2(1H)-one

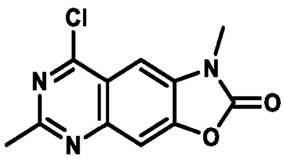

The titled compound was prepared from Step-7 intermediate by employing analogous protocol mentioned in Example **71-Step-9** (0.070 g, 38.1 % yield).

MS(ES+) m/z = 250.08 (M+1)

**Step-9::** (R)-8-((1-(3-(1,1-difluoro-2-hydroxy-2-methylpropyl)-2-fluorophenyl)ethyl)amino)-1,6-dimethyloxazolo[4,5-g]quinazolin-2(1H)-one **(Compound 111)**

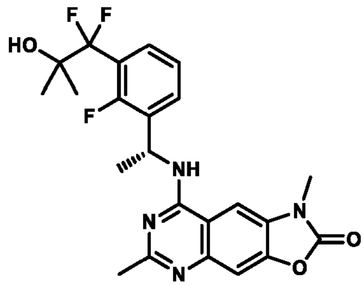

The titled compound was prepared by reaction of Step-**8** intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-Step-10** (0.035 g, 29.2 % yield).

MS(ES+) m/z = 461.30 (M+1)

$^1H$ NMR (400 MHz, DMSO-$d_6$) δ 8.33 (s, 1H), 8.19 (s, 1H), 7.64 - 7.57 (m, 1H), 7.46 (s, 1H), 7.38 - 7.27 (m, 1H), 7.26 - 7.19 (m, 1H), 5.86 - 5.78 (m, 1H), 5.34 (s, 1H), 3.45 (s, 3H), 2.34 (s, 3H), 1.61 (d, $J$ = 7.1 Hz, 3H), 1.24 (s, 3H), 1.22 (s, 3H).

**Example 125:** (R)-4-((1-(3-(1,1-difluoro-2-hydroxy-2-methylpropyl)-2-fluorophenyl)ethyl)amino)-2,6-dimethyl-6H-pyrrolo[2,3-g]quinazoline-7,8-dione **(compound 112)**

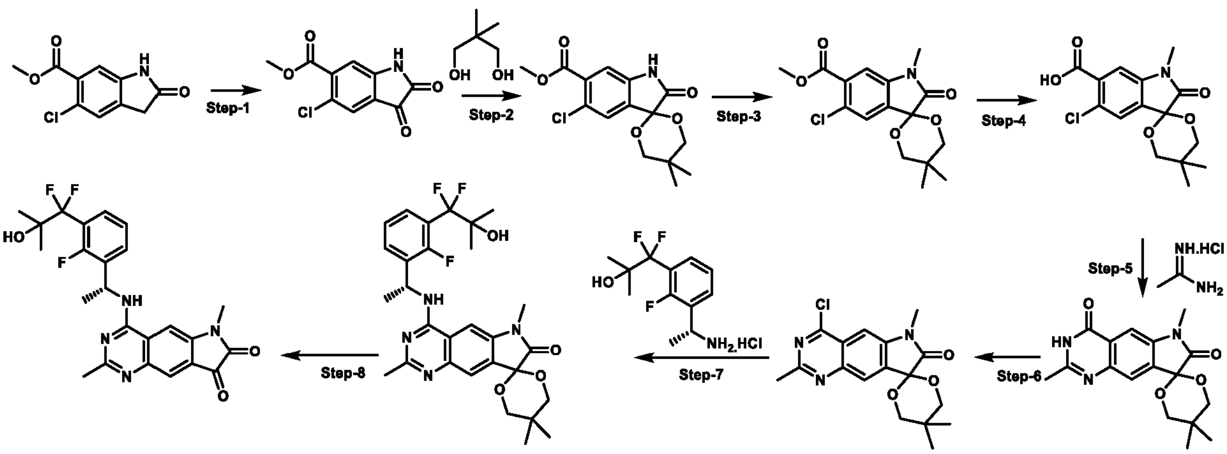


Step-1: methyl 5-chloro-2,3-dioxoindoline-6-carboxylate

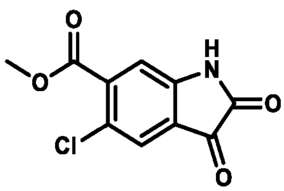

To a stirred solution of commercially available methyl 5-chloro-2-oxoindoline-6-carboxylate (7.0 g, 31.0 mmol) in DCE (100.0 mL) was added 2-hydroperoxy-2-methylpropane (8.59 mL , 62.0 mmol) at room temperature and reaction was stirred at 80 °C for 16h. Reaction mixture was cooled to room temperature and added another lot of 2-hydroperoxy-2-methylpropane (8.59 mL , 62.0 mmol) and 0.1 eq of $Cu(OAc)_2$ and stirred at same temp for 1 h. reaction was diluted with DCM (100.0 mL) and washed with water (3 x 50.0 mL).

Organic layer was separated, dried over anhydrous $Na_2SO_4$ and concentrated to get titled compound (5.0g, 67.3% yield)

MS(ES+) m/z = 241.02 (M+2)

Step-2: methyl 5-chloro-5',5'-dimethyl-2-oxospiro[indoline-3,2'-[1,3]dioxane]-6-carboxylate

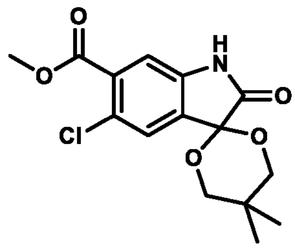

The mixture of methyl 5-chloro-2,3-dioxoindoline-6-carboxylate (0.90 g, 3.76 mmol),2,2-dimethylpropane-1,3-diol (0.430 g, 4.13 mmol) and pTsOH (0.072 g, 0.376 mmol) in cyclohexane (25.0 mL) was heated at 100 °C for 16 h. Solvent was evaporated and reaction was diluted by cold water. Aqueous layer was extracted in Ethyl acetate (2x 20.0 mL). The organic layer was separated, dried over anhydrous $Na_2SO_4$ and evaporated to obtained crude compound. The crude material was purified by flash chromatography in hexane -Ethyl acetate gradient to afford titled compound. (0.85 g, 69.1 % yield)

MS(ES+) m/z = 326.05 (M+1)

Step-3: methyl 5-chloro-1,5',5'-trimethyl-2-oxospiro[indoline-3,2'-[1,3]dioxane]-6-carboxylate

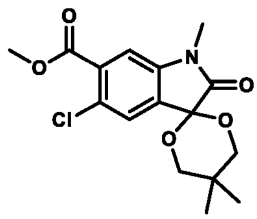

The titled compound was prepared from Step-**2** intermediate by employing analogous protocol mentioned in **Example 71-Step-5** (0.760 g, 87 % yield)

MS(ES+) m/z = 338.58

Step-4: 5-chloro-1,5',5'-trimethyl-2-oxospiro[indoline-3,2'-[1,3]dioxane]-6-carboxylic acid

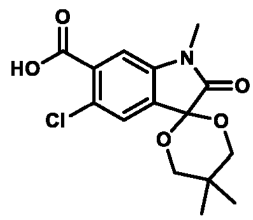

To a stirred solution of methyl 5-chloro-1,5',5'-trimethyl-2-oxospiro[indoline-3,2'-[1,3]dioxane]-6-carboxylate (0.17 g, 0.494 mmol) in MeOH-THF (4.0 mL (1: 1) and Water (1.0 mL) was added LiOH (0.024 g, 0.989 mmol) at room temperature and reaction was stirred at same temperature for 2 h. Solvent was evaporated and residue was acidified (pH: 4-5) using citric acid solution. The precipitated solid was filtered, washed with water and dried under vacuum to afford titled compound (0.154 g, 96 % yield)

$^1H$ NMR (400 MHz, DMSO-$d_6$) δ 13.72 (s, 1H), 7.55 (s, 1H), 7.37 (s, 1H), 4.48 (d, *J* = 10.8 Hz, 2H), 3.55 (d, *J*= 11.2 Hz, 2H), 3.11 (s, 3H), 1.32 (s, 3H), 0.85 (s, 3H).

Step-5: 2,5',5',6-tetramethyl-3,6-dihydrospiro[pyrrolo[2,3-g]quinazoline-8,2'-[1,3]dioxane]-4,7-dione

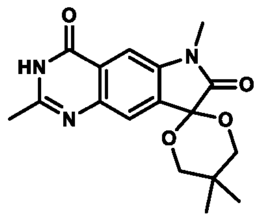

The titled compound was prepared from Step-**4** intermediate by employing analogous protocol mentioned in **Example-81 step-5a** (0.090 g, 60.1 % yield).
MS(ES+) m/z = 330.25.

Step-6: 4-chloro-2,5',5',6-tetramethylspiro[pyrrolo[2,3-g]quinazoline-8,2'-[1,3]dioxan]-7(6H)-one

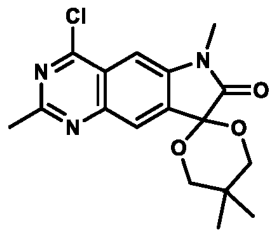

the titled compound was obtained from Step-5 intermediate by following analogous protocol used in Example **71-Step 9** (0.025 g, 23.67 % yield)
MS(ES+) m/z = 348.33 (M+1).

Step-7: (R)-4-((1-(3-(1,1-difluoro-2-hydroxy-2-methylpropyl)-2-fluorophenyl)ethyl)amino)-2,5',5',6-tetramethylspiro[pyrrolo[2,3-g]quinazoline-8,2'-[1,3]dioxan]-7(6H)-one

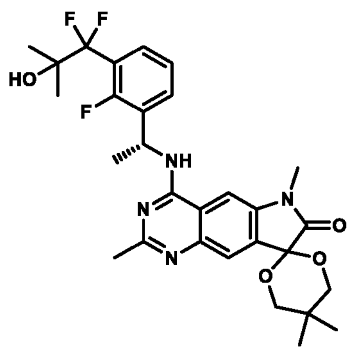

The titled compound was obtained by reaction of Step-6 intermediate and (R)-1-(3-(1-Aminoethyl)-2-fluorophenyl)-1,1-difluoro-2-methylpropan-2-ol hydrochloride (Example 4-Step 6a) by following analogous protocol used in Example **71-Step-10** (0.015 g, 62.3 % yield)
MS(ES+) m/z = 559.50 (M+1).

Step-8: (R)-4-((1-(3-(1,1-difluoro-2-hydroxy-2-methylpropyl)-2-fluorophenyl)ethyl)amino)-2,6-dimethyl-6H-pyrrolo[2,3-g]quinazoline-7,8-dione **(Compound 112)**

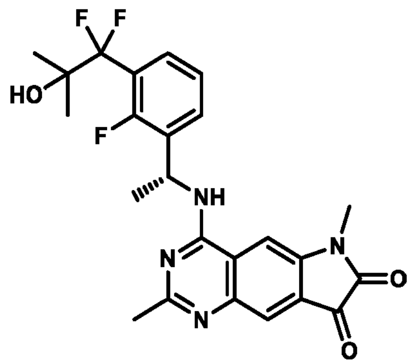

The mixture of (R)-4-((1-(3-(1,1-difluoro-2-hydroxy-2-methylpropyl)-2-fluorophenyl)ethyl)amino)-2,5',5',6-tetramethylspiro[pyrrolo[2,3-g]quinazoline-8,2'-[1,3]dioxan]-7(6H)-one (0.024 g, 0.043 mmol), concentrated HCl (0.559 mL , 6.44 mmol) and AcOH (0.492 mL , 8.59 mmol) in Dioxane (0.5 mL) were heated at 90 °C for 16 h. the reaction was diluted by cold sat. solution of $NaHCO_3$ and extracted in Ethyl acetate (2 x 20.0 mL). The organic layer was separated, dried over anhydrous $Na_2SO_4$ and evaporated to get crude compound. Crude product was purified by preparative HPLC afford titled compound (0.007 g, 34.5 % yield)

MS(ES+) m/z = 473.42 (M+1).

$^1$H NMR (400 MHz, DMSO-$d_6$) δ 8.43 (d, *J* = 7.2 Hz, 1H), 7.92 (s, 1H), 7.71 (s, 1H), 7.63 - 7.59 (m, 1H), 7.35 - 7.28 (m, 1H), 7.26 - 7.20 (m, 1H), 5.85 - 5.74 (m, 1H), 5.35 (s, 1H), 3.29 (s, 3H), 2.34 (s, 3H), 1.62 (d, *J* = 7.0 Hz, 3H), 1.24 (s, 3H), 1.21 (s, 3H).

**Example 126-** (R)-4-((1-(3-(1,1-difluoro-2-hydroxy-2-methylpropyl)-2-fluorophenyl)ethyl)amino)-2,6-dimethyl-6,8-dihydro-7H-pyrrolo[2,3-g]quinazolin-7-one **(Compound 113)**

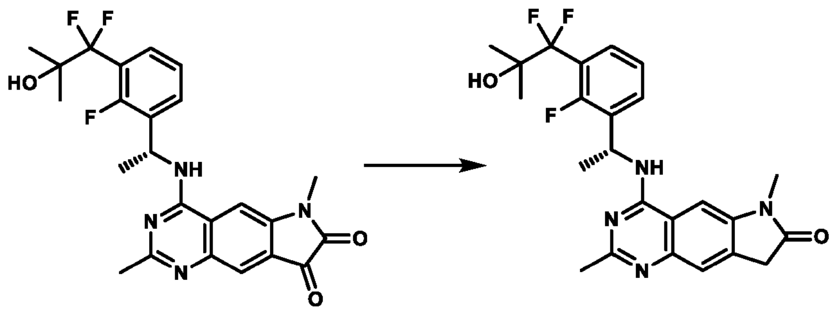

The mixture of (R)-4-((1-(3-(1,1-difluoro-2-hydroxy-2-methylpropyl)-2-fluorophenyl)ethyl)amino)-2,6-dimethyl-6H-pyrrolo[2,3-g]quinazoline-7,8-dione ( compound 55, 0.100 g, 0.212 mmol ) and hydrazine hydrate solution in water (98%) (1329 μl, 42.3 mmol) were heated at 95 °C for 4 h. Reaction was diluted by cold water( 10.0 mL) and extracted in Ethyl acetate( 2 x 10.0 mL). the organic layer was isolated, dried over anhydrous $Na_2SO_4$ and evaporated. The crude product was purified by flash chromatography in MeOH-DCM gradient to afford titled compound (0.044 g, 45.3 % yield)

MS(ES+) m/z = 459.23 (M+1) $^1$H NMR (400 MHz, DMSO-$d_6$) δ 8.20 (d, *J* = 7.4 Hz, 1H), 7.83 (s, 1H), 7.63 - 7.59 (m, 1H), 7.47 (s, 1H), 7.36 - 7.26 (m, 1H), 7.24 - 7.19 (m, 1H), 5.81 - 5.75 (m,1H), 5.34 (s, 1H), 3.71 (s, 2H), 3.26 (s, 3H), 2.32 (s, 3H), 1.60 (d, *J*= 7.0 Hz, 3H), 1.24 (s, 3H), 1.22 (s, 3H).

**Example 127-** (R)-4-((1-(3-(1,1-difluoro-2-hydroxy-2-methylpropyl)-2-fluorophenyl)ethyl)amino)-2,6,6,7-tetramethyl-6,7-dihydro-8H-pyrrolo[3,4-g]quinazolin-8-one **(Compound 114)**

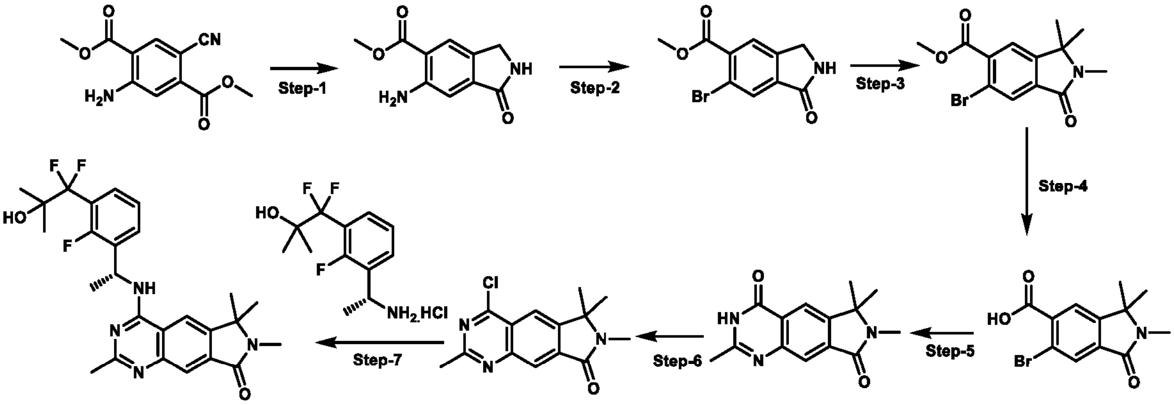


Step-1: Methyl 6-amino-1-oxoisoindoline-5-carboxylate

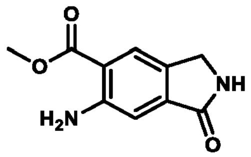

To a stirred solution of dimethyl 2-amino-5-cyanoterephthalate (2.2 g, 9.39 mmol) in MeOH (45.0 mL), TEA (11.00 mL , 79 mmol) and Dioxane (30.0 mL), Raney Nickel (0.551 g, 9.39 mmol) was added and the reaction mixture was stirred under 100 psi of $H_2$ at 70° C. for 15 h. The reaction mixture was filtered, and the filtrate was concentrated under vacuum. The crude product was purified by flash chromatography in DCM-MeOH gradient to afford titled compound (0.35 g, 18.07 % yield)

MS(ES+) m/z = 207.17 (M+1).

Step-2: Methyl 6-bromo-1-oxoisoindoline-5-carboxylate

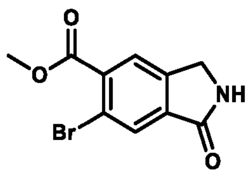

To a stirred solution of methyl 6-amino-1-oxoisoindoline-5-carboxylate (1.5g, 7.27 mmol)) in Acetonitrile ( 30.0 mL) was added tert-butyl nitrite (1.92 mL , 14.55 mmol) at 0 °C over a period of 10 min. After stirring at 0 °C for 1 h, CuBr (1.56 g, 10.91 mmol) was added portion wise To a reaction mixture for 10 min and the reaction was heated to 60 °C for 5 h. Reaction mixture was cooled to room temperature and diluted with DCM (100.0 mL) and washed with water (50.0 mL) and Aq.HCl (1N, 50.0 mL) to get crude compound which was purified by flash chromatography in Hexane Ethyl acetate gradient to afford titled compound (0.7 g, 35.6 % yield)

MS(ES+) m/z = 270.17. (M+), 272.17 (M+2).

Step-3: Methyl 6-bromo-2,3,3-trimethyl-1-oxoisoindoline-5-carboxylate

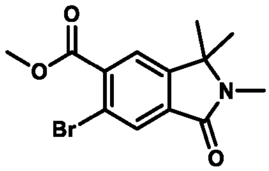

the titled compound was obtained from Step - 2 intermediate by following analogous protocol used in Example **81-Step-2a** (0.8 g, 72.9 %).

MS(ES+) m/z = 314.17 (M+2)

Step-4: 6-bromo-2,3,3-trimethyl-1-oxoisoindoline-5-carboxylic acid

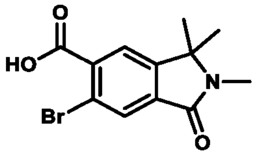

The titled compound was prepared from step - 3 intermediate by employing analogous protocol mentioned in **Example 81 step-4a** (0.025 g, 65.4 % yield).

MS(ES+) m/z = 297.17 (M+1).

Step-5: 2,6,6,7-tetramethyl-6,7-dihydro-3H-pyrrolo[3,4-g]quinazoline-4,8-dione

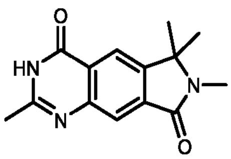

The titled compound was obtained from Step - 4 intermediate by following analogous protocol used in Example **81-Step-5a**

MS(ES+) m/z = 258.25 (M+1).

Step-6: 4-chloro-2,6,6,7-tetramethyl-6,7-dihydro-8H-pyrrolo[3,4-g]quinazolin-8-one

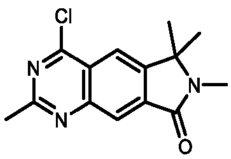

The titled compound was obtained from Step-5 intermediate by following analogous protocol used in Example **71-Step-9** (0.050 g, 78 % yield).

MS(ES+) m/z = 276.25 (M+1).

Step-7: (R)-4-((1-(3-(1,1-difluoro-2-hydroxy-2-methylpropyl)-2-fluorophenyl)ethyl)amino)-2,6,6,7-tetramethyl-6,7-dihydro-8H-pyrrolo[3,4-g]quinazolin-8-one **(Compound 114)**

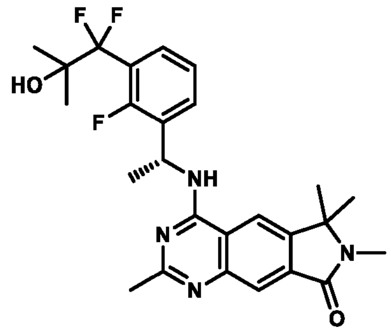

The titled compound was obtained by reaction of Step-6 intermediate and (R)-1-(3-(1-Aminoethyl)-2-fluorophenyl)-1,1-difluoro-2-methylpropan-2-ol hydrochloride (Example 4-Step 6a) by following analogous protocol used in Example **71-Step-10** (0.010 g, 16.19 % yield).

$^1$H NMR (400 MHz, DMSO-*d*$_6$) δ 8.71 (s, 1H), 8.50 (d, *J*= 7.2 Hz, 1H), 7.76 (s, 1H), 7.67 - 7.60 (m, 1H), 7.37 - 7.30 (m, 1H), 7.27 - 7.21 (m, 1H), 5.86 - 5.76 (m, 1H), 5.35 (s, 1H), 3.00 (s, 3H), 2.37 (s, 3H), 1.63 (d, J = 7.0 Hz, 3H), 1.57 - 1.52 (m, 6H), 1.24 (s, 3H), 1.21 (s, 3H).

MS(ES+) m/z = 487.42 (M+1).

**Example 128** : 4-((1-(2-fluoro-3-(1-(hydroxymethyl)cyclopropyl)phenyl)ethyl)amino)-2,6,8,8-tetramethyl-6,8-dihydro-7H-pyrrolo[2,3-g]quinazolin-7-one **(Compound 115a) and (Compound 115b)**

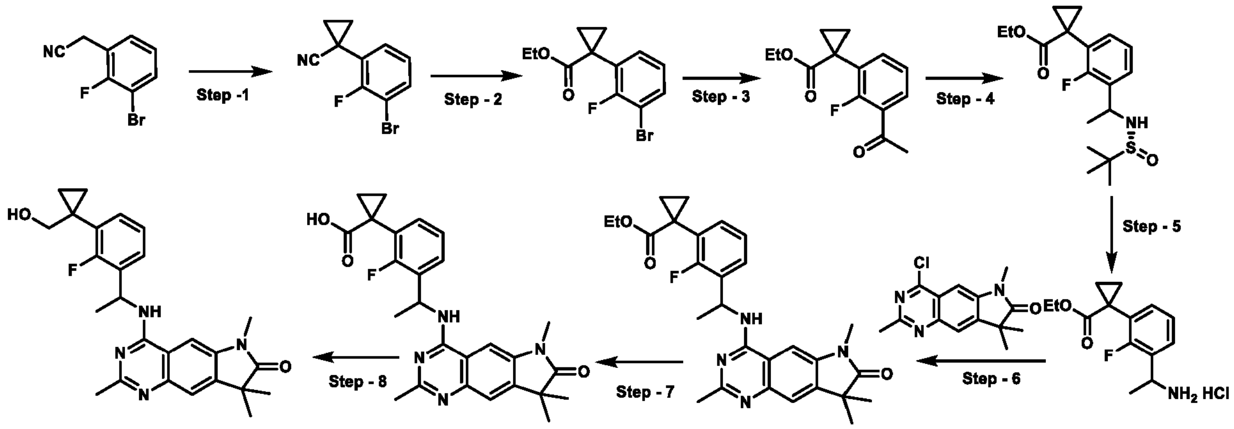


Step - 1: 1-(3-bromo-2-fluorophenyl)cyclopropane-1-carbonitrile

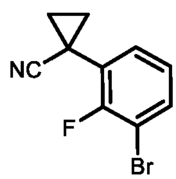

To a stirred solution of 2-(3-bromo-2-fluorophenyl)acetonitrile (commercial) (14.0 g, 65.4 mmol) and 1,2-dibromoethane (7.36 mL , 85 mmol) in THF (150.0 mL), sodium hydride (60% dispersion in mineral oil) (6.54 g, 164 mmol) was added portion wise at 0 °C. Resulting mixture was stirred at ambient temperature for 16 h under inert atm. The reaction mixture was quenched by addition of isopropyl alcohol (10.0 mL) and water (200.0 mL) and extracted with Ethyl acetate (2 x 200.0 mL). Combined organic layer was dried over anhydrous $Na_2SO_4$ and concentrated in vacuum. The residue was purified by flash chromatography using eluent 0-5% Ethyl acetate in hexane to afford titled compound (9.0 g, 57.3 % yield)

$^1$H NMR (400 MHz, Chloroform-*d*) δ 7.60-7.53 (m, 1H), 7.34-7.27 (m, 1H), 7.08-7.01 (m, 1H), 1.77 - 1.71 (m, 2H),

1.46 - 1.38 (m, 2H).

Step - 2: ethyl 1-(3-bromo-2-fluorophenyl)cyclopropane-1-carboxylate

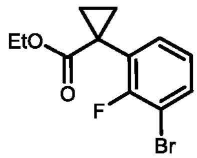

To a stirred solution of 1-(3-bromo-2-fluorophenyl)cyclopropane-1-carbonitrile (9 g, 37.5 mmol)) in Ethanol (150 mL) was added $H_2SO_4$ (15 mL) at 25-30 °C and stirred at 80 °C for 16 h. The reaction was concentrated and poured into ice cold water and extracted with Ethyl acetate (2 x 200.0 mL). The combined organic layers were washed with brine, dried over anhydrous $Na_2SO_4$, filtered and concentrated. Obtained residue was purified by flash chromatography using Ethyl acetate -hexane gradient to afford titled compound (4.5 g, 41.8 % yield) as a colorless liquid.

$^1$H NMR (400 MHz, Chloroform-*d*) δ 7.53-7.47 (m, 1H), 7.25-7.19 (m, 1H), 7.02-6.96 (m, 1H), 4.13 (q, *J* = 7.1 Hz, 2H), 1.71.1.67 (m, 2H), 1.22 - 1.16 (m, 5H).

Step - 3: ethyl 1-(3-acetyl-2-fluorophenyl)cyclopropane-1-carboxylate

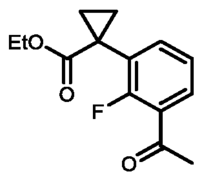

Titled compound was prepared from Step-2 intermediate by employing analogous protocol as mentioned in Example 4- Step-3 (3.8 g, 99 % yield)

$^1$H NMR (400 MHz, Chloroform-d) δ 7.85-7.78 (m, 1H), 7.50-7.43 (m, 1H), 7.21-7.15 (m, 1H), 4.14 (q, *J* = 7.1 Hz, 2H), 2.68 (s, 3H), 1.75-1.70 (m, 2H), 1.25 - 1.16 (m, 5H).

Step - 4: ethyl 1-(3-(1-)-tert-butylsulfinyl)amino)ethyl)-2-fluorophenyl)cyclopropane-1-carboxylate

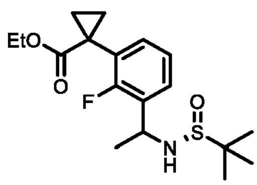

Titled compound was prepared from Step-3 intermediate by employing analogous protocol as mentioned in Example 4- Step-4 and step-5 (4 g, 70.4 % yield)

$^1$H NMR (400 MHz, DMSO-$d_6$) δ 7.43-7.37 (m, 1H), 7.25-7.17 (m, 1H), 7.15-7.08 (m, 1H), 4.73-4.62 (m, 1H), 4.08-3.95 (m, 2H), 1.54 - 1.44 (m, 3H), 1.41-1.36 (m, 2H), 1.22 - 1.14 (m, 2H), 1.12-1.04 (m, 12H).

Step - 5: ethyl 1-(3-(1-aminoethyl)-2-fluorophenyl)cyclopropane-1-carboxylate hydrochloride

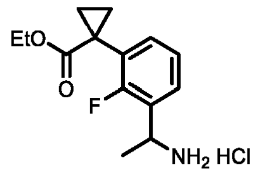

Titled compound was prepared from Step-4 intermediate by employing analogous protocol as mentioned in Example 1- Step-3 (2.5 g, 77 % yield)

$^1$H NMR (400 MHz, DMSO-$d_6$) δ 8.69 (s, 3H), 7.67 - 7.49 (m, 1H), 7.40-7.33 (m, 1H), 7.26-7.20 (m, 1H), 4.69 - 4.52 (m, 1H), 4.11 - 3.93 (m, 2H), 1.51-1.49 (m, 5H), 1.28 - 1.17 (m, 2H), 1.13 - 1.02 (m, 3H).

Step - 6: (R/S)-ethyl 1-(2-fluoro-3-(1-((2,6,8,8-tetramethyl-7-oxo-7,8-dihydro-6H-pyrrolo[2,3-g]quinazolin-4-yl)amino) ethyl)phenyl)cyclopropane-1-carboxylate

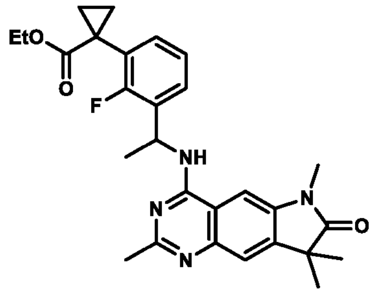

titled compound was prepared by reaction of Step-5 intermediate and chloro intermediate (Example 81-Step 6) by employing analogous protocol mentioned in Example 71- Step-10 (0.60 g, 84 % yield)

MS(ES+) m/z = 491.13 (M+).

Step - 7: 1-(2-fluoro-3-(1-((2,6,8,8-tetramethyl-7-oxo-7,8-dihydro-6H-pyrrolo[2,3-g]quinazolin-4-yl)amino)ethyl)phenyl) cyclopropane-1-carboxylic acid

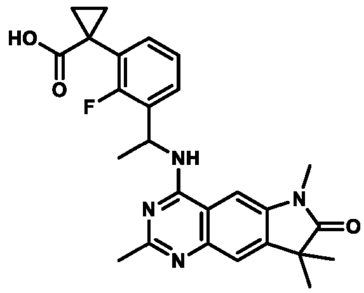

To a stirred solution of ethyl 1-(2-fluoro-3-(1-((2,6,8,8-tetramethyl-7-oxo-7,8-dihydro-6H-pyrrolo[2,3-g]quinazolin-4-yl)amino)ethyl)phenyl)cyclopropane-1-carboxylate (0.300 g, 0.612 mmol) in Ethanol (4 mL) was added a solution of NaOH (0.122 g, 3.06 mmol) in 1 mL water and stirred at room temperature for 16 h. After completion, reaction mixture was concentrated, diluted with water (10 mL) and acidified with conc. HCl (PH= 6-7). precipitated solid was then filtered and dried over vacuum to afford titled compound (0.100 g, 0.216 mmol, 35.4 % yield).

MS(ES+) m/z = 463.10 (M+1).

Step - 8: (R/S)-4-((1-(2-fluoro-3-(1-(hydroxymethyl)cyclopropyl)phenyl)ethyl)amino)-2,6,8,8-tetramethyl-6,8-dihydro-7H-pyrrolo[2,3-g]quinazolin-7-one

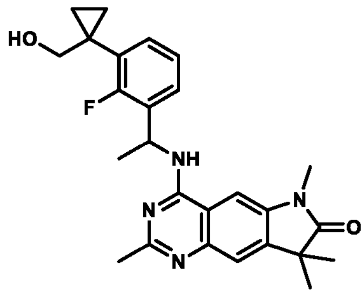

To a stirred solution of 1-(2-fluoro-3-(1-((2,6,8,8-tetramethyl-7-oxo-7,8-dihydro-6H-pyrrolo[2,3-g]quinazolin-4-yl)amino)ethyl)phenyl)cyclopropane-1-carboxylic acid (0.200 g, 0.432 mmol) in THF (1 mL) was added triethylamine (0.090 mL , 0.649 mmol) at 0 °C under inert atmosphere, followed by dropwise addition of ethyl chloroformate (0.056 g, 0.519 mmol). The reaction mixture was then stirred at 0° C for 1 h. The reaction mixture was filtered and rinsed with anhydrous THF (5 mL). The THF filtrate was stirred at 0 C under $N_2$ and MeOH (2 mL) was added, followed by portion wise addition of $NaBH_4$ (0.16 g, 0.432 mmol). The resulting mixture was stirred at 0° C for 35 minutes. After completion of the reaction. Saturated $Na_2SO_4$ was then added. The mixture was extracted with Ethyl acetate (2 x 10 mL). The organic layer was washed with brine and dried over anhydrous $Na_2SO_4$, concentrated to dryness and purified by prep-HPLC and further with chiral prep-HPLC (HEX_0.1%DEA_IPA_DCM_70_30_A_D_1.0 ML _12MIN_250nm) to afford titled compound-

**Peak-1:** (R/S)-4-((1-(2-fluoro-3-(1-(hydroxymethyl)cyclopropyl)phenyl)ethyl)amino)-2,6,8,8-tetramethyl-6,8-dihydro-7H-pyrrolo[2,3-g]quinazolin-7-one (0.010 g, 5.16 % yield)

**(Compound 115a)**

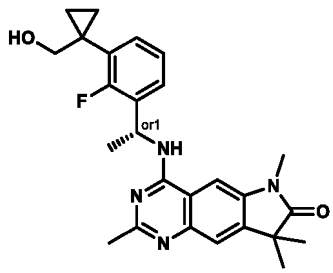

MS(ES+) m/z = 449.42 (M+1),
Chiral RT - 5.89 min.

1H NMR (400 MHz, DMSO-$d_6$) δ 8.21-8.12 (m, 1H), 7.89 (s, 1H), 7.60 (s, 1H), 7.40 - 7.33 (m, 1H), 7.22 - 7.16 (m, 1H), 7.07 - 7.01 (m, 1H), 5.89 - 5.80 (m, 1H), 4.69 - 4.64 (m, 1H), 3.53 - 3.42 (m, 2H), 3.28 (s, 3H), 2.36 (s, 3H), 1.59 (d, *J* = 7.0 Hz, 3H), 1.35 (s, 3H), 1.34 (s, 3H), 0.87 - 0.85 (m, 2H), 0.70 - 0.65 (m, 2H). **Peak-2:** (S/R)-4-((1-(2-fluoro-3-(1-(hydroxymethyl)cyclopropyl)phenyl)ethyl)amino)-2,6,8,8-tetramethyl-6,8-dihydro-7H-pyrrolo[2,3-g]quinazolin-7-one (0.006 g, 3.09 % yield) **(Compound 115b)**

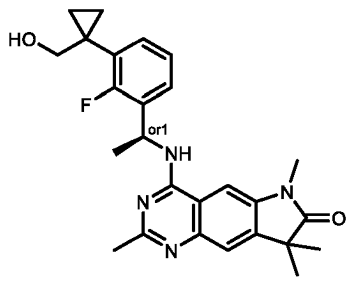

MS(ES+) m/z = 449.42 (M+1),

Chiral RT - 6.63 min.

$^1$H NMR (400 MHz, DMSO-$d_6$) δ 8.16 - 8.09 (m, 1H), 7.89 (s, 1H), 7.60 (s, 1H), 7.41 - 7.36 (m, 1H), 7.23 - 7.15 (m, 1H), 7.07 - 7.01 (m, 1H), 5.91 - 5.80 (m, 1H), 4.72 - 4.61 (m, 1H), 3.52 - 3.44 (m, 2H), 3.28 (s, 3H), 2.35 (s, 3H), 1.59 (d, *J* = 7.0 Hz, 3H), 1.35 (s, 3H), 1.33 (s, 3H), 0.90-0.81 (m, 2H), 0.73 - 0.63 (m, 2H).

**Example 129** (R&S)-4-(((R)-1-(3-(1,1-difluoro-2-hydroxy-2-methylpropyl)-2-fluorophenyl)ethyl)amino)-8-fluoro-2,6,8-trimethyl-6,8-dihydro-7H-pyrrolo[2,3-g]quinazolin-7-one **(Compound 116)**

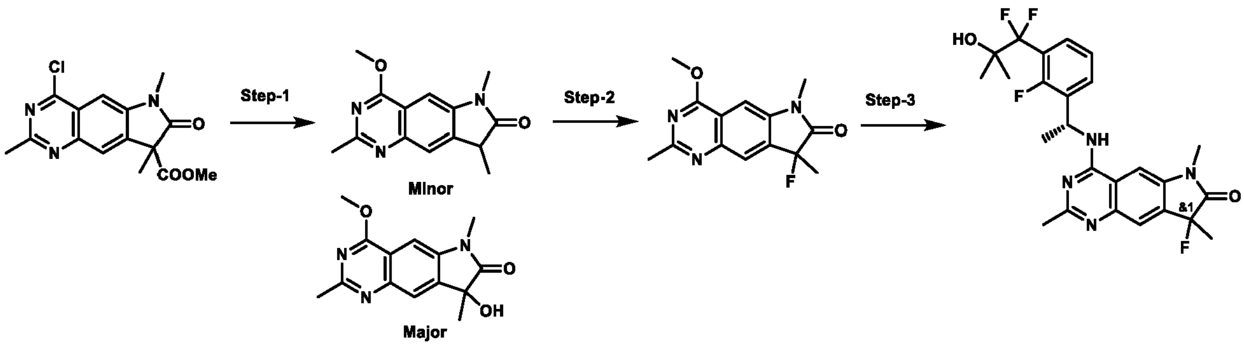


Step-1: 4-methoxy-2,6,8-trimethyl-6,8-dihydro-7H-pyrrolo[2,3-g]quinazolin-7-one

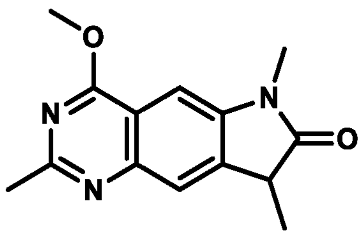

The solution of methyl 4-chloro-2,6,8-trimethyl-7-oxo-7,8-dihydro-6H-pyrrolo[2,3-g]quinazoline-8-carboxylate **(Example 112 Step-7)** 1g, 3.13 mmol) in MeOH (10.0 mL) was stirred at 50°C for 10 minutes and cooled to room temperature was added LiOH (0.187 g, 7.82 mmol) at 0 °C. Resulting reaction mixture was stirred at room temperature for 15 h. Reaction mixture was concentrated in vacuum, and flash purified using MeOH-DCM to afford titled compound as minor product.

MS(ES+) m/z = 257.17(M+1)

Step-2: 8-fluoro-4-methoxy-2,6,8-trimethyl-6,8-dihydro-7H-pyrrolo[2,3-g]quinazolin-7-one

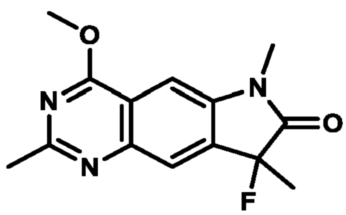

To an ice cooled solution of 4-methoxy-2,6,8-trimethyl-6,8-dihydro-7H-pyrrolo[2,3-g]quinazolin-7-one (0.100 g, 0.389 mmol) in acetonitrile ( 1 mL) and Water (1 mL) was added SELECTFLUOR (0.413 g, 1.166 mmol). The reaction was stirred at 60 °C for 2h. Reaction was diluted with ice water and extracted with Ethyl acetate. Organic layer was dried over

anhydrous $Na_2SO_4$ and evaporated. The crude material was purified by flash chromatography using MeOH - DCM gradient to afford titled compound (0.075 g, 70.1 % yield).

MS(ES+) m/z = 276.17 (M+1)

Step-3: (R&S)-4-(((R)-1-(3-(1,1-difluoro-2-hydroxy-2-methylpropyl)-2-fluorophenyl)ethyl)amino)-8-fluoro-2,6,8-trimethyl-6,8-dihydro-7H-pyrrolo[2,3-g]quinazolin-7-one (Compound 116)

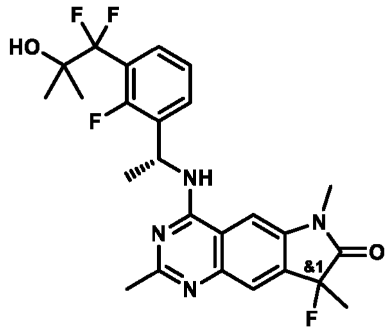

Titled compound was prepared by using Step-2 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 115.**

The two Diastereomers were separated by chiral prep HPLC.

Peak-1 : (R/S)-4-(((R)-1-(3-(1,1-difluoro-2-hydroxy-2-methylpropyl)-2-fluorophenyl)ethyl)amino)-8-fluoro-2,6,8-trimethyl-6,8-dihydro-7H-pyrrolo[2,3-g]quinazolin-7-one **(Compound 116a)**

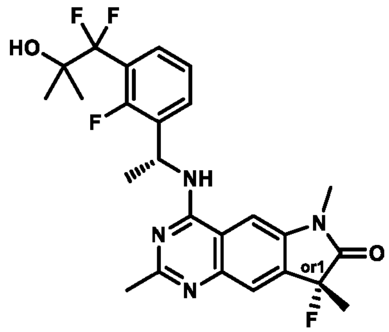

MS(ES+) m/z = 491.42 (M+1)
Chiral RT: 4.35min

$^1$H NMR (400 MHz, DMSO-$d_6$) δ 8.34 (d, *J*= 7.3 Hz, 1H), 7.96 (s, 1H), 7.88 - 7.81 (m, 1H), 7.62 - 7.54 (m, 1H), 7.36 - 7.27 (m, 1H), 7.26 - 7.17 (m, 1H), 5.86 - 5.74 (m, 1H), 5.35 (s, 1H), 3.29 (s, 3H), 2.34 (s, 3H), 1.77 (d, *J* = 23.0 Hz, 3H), 1.61 (d, *J*= 7.0 Hz, 3H), 1.25 (s, 3H), 1.21 (s, 3H)

Peak-2 (S/R)-4-(((R)-1-(3-(1,1-difluoro-2-hydroxy-2-methylpropyl)-2-fluorophenyl)ethyl)amino)-8-fluoro-2,6,8-trimethyl-6,8-dihydro-7H-pyrrolo[2,3-g]quinazolin-7-one **(Compound 116b)**

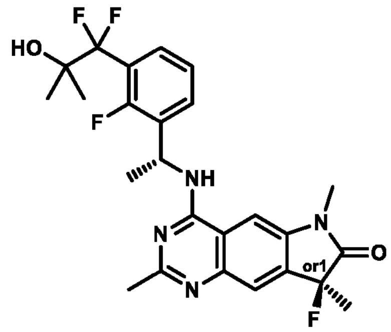

MS(ES+) m/z = 491.39 (M+1)
Chiral RT: 4.98min

$^{1}$H NMR (400 MHz, DMSO-$d_6$) δ 8.35 (d, *J*= 7.3 Hz, 1H), 7.96 (s, 1H), 7.86 - 7.80 (m, 1H), 7.65 - 7.56 (m, 1H), 7.37 - 7.27 (m, 1H), 7.27 - 7.18 (m, 1H), 5.86 - 5.74 (m, 1H), 5.35 (s, 1H), 3.29 (s, 3H), 2.34 (s, 3H), 1.79 (d, *J* = 23.0 Hz, 3H), 1.61 (d, J = 7.0 Hz, 3H), 1.25 (s, 3H), 1.21 (s, 3H)

**Example 130:** (R)-4-((1-(3-(1,1-difluoro-2-hydroxy-2-methylpropyl)-2-fluorophenyl)ethyl)amino)-8,8-difluoro-2,6-dimethyl-6,8-dihydro-7H-pyrrolo[2,3-g]quinazolin-7-one **(Compound 117** )

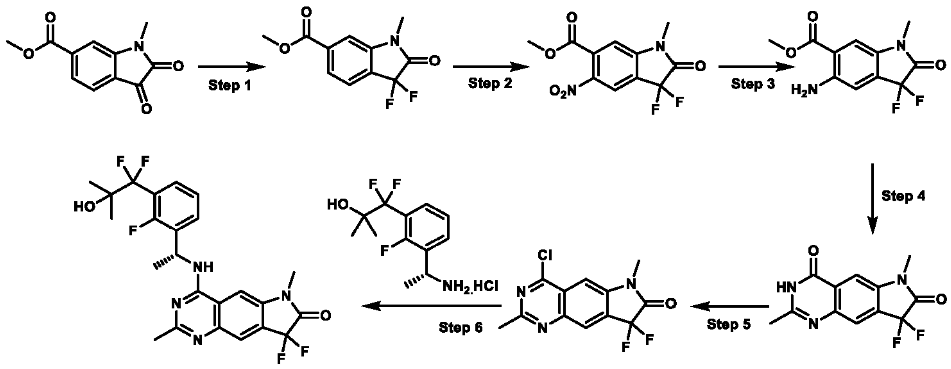


Step-1: Methyl 3,3-difluoro-1-methyl-2-oxoindoline-6-carboxylate

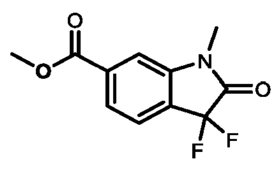

To a stirred solution of methyl 1-methyl-2,3-dioxoindoline-6-carboxylate (0.200 g, 0.912 mmol) in DCM (10 mL) was added DAST (0.603 mL, 4.56 mmol) at -78 °C. the reaction was warmed to room temperature and stirred for 12h. The réaction mass was diluted with water (20 mL) and extracted with DCM (3 x 20 mL). The combined organic layer was washed with brine (50 mL). dried over anh. $Na_2SO_4$ and concentrated to give crude residue. It was purified by flash chromatography in Ethyl acetate-hexane gradient to afford the titled compound (0.170 g, 77 % yield)
MS(ES+) m/z = 241.25 (M+)

Step-2: Methyl 3,3-difluoro-1-methyl-5-nitro-2-oxoindoline-6-carboxylate

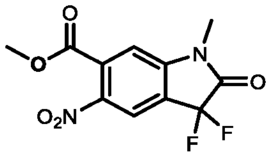

A solution of methyl 3,3-difluoro-1-methyl-2-oxoindoline-6-carboxylate (0.050 g, 0.207 mmol) in sulphuric acid (1.0 mL) was cooled to -5 °C and potassium nitrate (0.023 g, 0.228 mmol) was added in portions. The resulting mixture was stirred at room temperature for 10min. The reaction mass was poured in ice water (5 mL) extracted with Ethyl acetate (2×5 mL). The combined organic layer was washed with brine (10 mL), dried over anhydrous $Na_2SO_4$ and evaporated to afford the titled compound (0.060 g, 0.210 mmol, 101 % yield) as a crude solid.
MS(ES+) m/z = 286.21 (M)

Step-3: methyl 5-amino-3,3-difluoro-1-methyl-2-oxoindoline-6-carboxylate

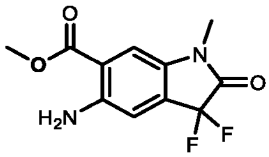

To a stirred solution of methyl 3,3-difluoro-1-methyl-5-nitro-2-oxoindoline-6-carboxylate (0.500 g, 1.747 mmol) in MeOH (30 mL) and THF (15.00 mL), Pd-C (1.673 g, 1.572 mmol) was added under nitrogen and reaction mixture was hydrogenated on parr shaker at 70 psi for 5h. Reaction mass was filtered and filtrate was evaporated. The residue was purified by flash chromatography in Hexane- Ethyl acetate to afford titled compound. (0.40 g, 89 % yield)
MS(ES+) m/z = 256.46 (M+)

Step-4: 8,8-difluoro-2,6-dimethyl-6,8-dihydro-3H-pyrrolo[2,3-g]quinazoline-4,7-dione

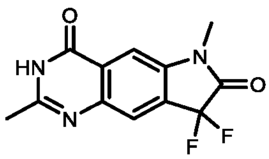

Titled compound was prepared by using Step-3 intermediate by employing analogous protocol mentioned in **Example 71-Step-8** ( 0.250 g, 60%yield)
MS(ES+) m/z = 266.17 (M+1)

Step-5: 4-chloro-8,8-difluoro-2,6-dimethyl-6,8-dihydro-7H-pyrrolo[2,3-g]quinazolin-7-one

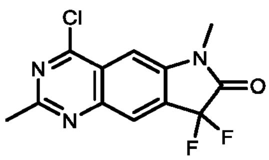

Titled compound was prepared by using Step-4 intermediate by employing analogous protocol mentioned in **Example 71-Step-9** (0.15 g, 70% yield)
MS(ES+) m/z = 284.25 (M+1)

Step-6: (R)-4-((1-(3-(1,1-difluoro-2-hydroxy-2-methylpropyl)-2-fluorophenyl)ethyl)amino)-8,8-difluoro-2,6-dimethyl-6,8-dihydro-7H-pyrrolo[2,3-g]quinazolin-7-one **(Compound 117)**

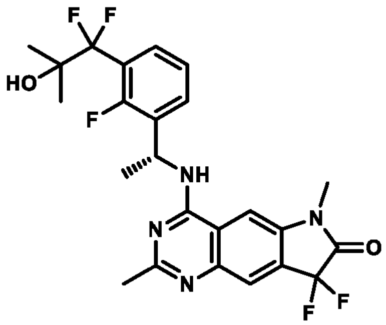

Titled compound was prepared by using Step-5 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71 - Step 10** (0.045 g, 32.3% yield)

MS(ES+) m/z = 495.42 (M+1)

1H NMR (400 MHz, DMSO-$d_6$) δ 8.47 (d, *J*= 7.2 Hz, 1H), 8.09 (s, 1H), 7.99 - 7.95 (m, 1H), 7.64 - 7.57 (m, 1H), 7.37 - 7.28 (m, 1H), 7.25 - 7.20 (m, 1H), 5.85 - 5.75 (m, 1H), 5.35 (s, 1H), 3.32 (s, 3H), 2.35 (s, 3H), 1.62 (d, *J*= 7.0 Hz, 3H), 1.24 (s, 3H), 1.22 (s, 3H)

**Example 131:** (R)-4-((1-(3-(1,1-difluoro-2-hydroxy-2-methylpropyl)-2-fluorophenyl)ethyl)amino)-2,7,8,8-tetramethyl-7,8-dihydro-6H-pyrrolo[3,4-g]quinazolin-6-one **(Compound 118)**

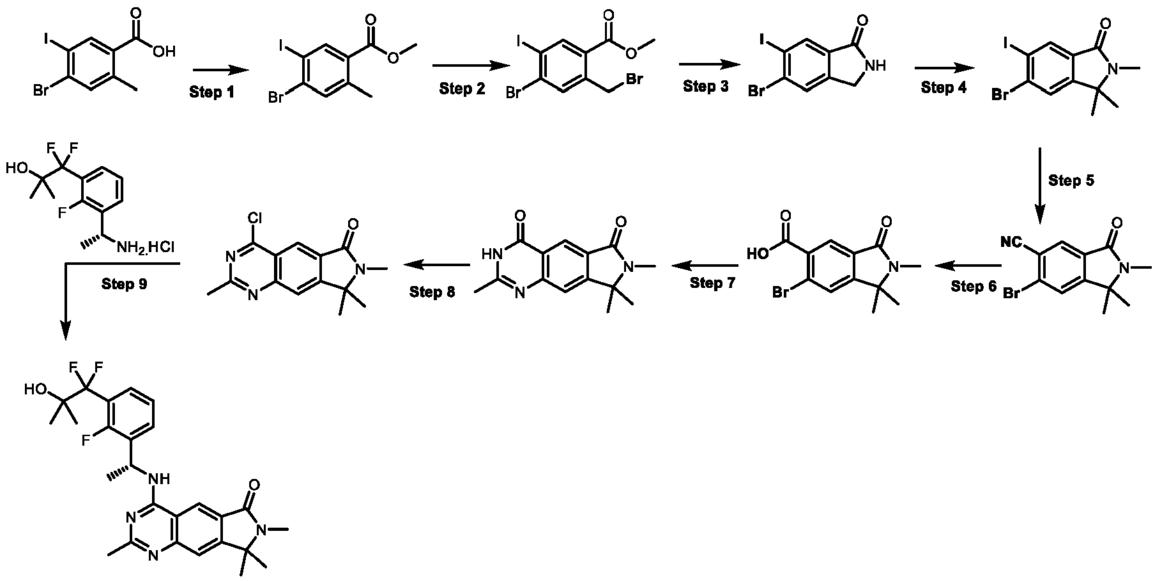


Step-1: Methyl 4-bromo-5-iodo-2-methylbenzoate

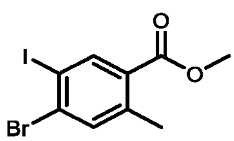

To a stirred solution of 4-bromo-5-iodo-2-methylbenzoic acid (28.0g , 82 mmol) in MeOH (300.0 mL) was added $H_2SO_4$ (17.51 mL, 329 mmol) dropwise, the reaction was stirred at 70°C for 16h. Reaction was cooled to room temperature & solvent was evaporated, poured the reaction mixture into water (50.0 mL) and extracted with DCM (2 x 500.0 mL), organic layer was washed with water (100.0 mL), brine (100.0 mL), dried over anhydrous $Na_2SO_4$ and concentrated under reduced pressure The crude material obtained was purified by flash chromatography in Ethyl acetate -hexane gradient to afford titled compound (26.5 g, 91 % yield).

GC/MS m/z = 354.08 (M+) 356.08 (M+2)

Step-2: Methyl 4-bromo-2-(bromomethyl)-5-iodobenzoate

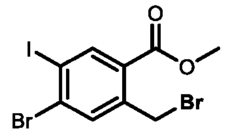

To a stirred solution of methyl 4-bromo-5-iodo-2-methylbenzoate (8 g, 22.54 mmol) in Carbon tetrachloride (80 mL), AIBN (0.740 g, 4.51 mmol) & NBS (4.41 g, 24.79 mmol) were added and the reaction was stirred at 85°C for 16h. Reaction was cooled to room temperature & solvent was evaporated. Residue was partitioned between water (50.0 mL) and DCM (50.0 mL). Organic layer was washed with water (50.0 mL), brine (50.0 mL) and dried over anhydrous $Na_2SO_4$. Solvent was evaporated and residue was purified by flash chromatography in Ethyl acetate- hexane gradient to afford titled compound (3.8 g, 38.9 % yield) as an off white solid.

$^1$H NMR (400 MHz, Chloroform-d) $\delta$ 8.44 (s, 1H), 7.74 (s, 1H), 4.86 (s, 2H), 3.97 (s, 3H).

Step-3: 5-bromo-6-iodoisoindolin-1-one

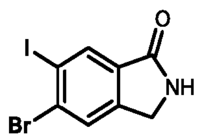

methyl 4-bromo-2-(bromomethyl)-5-iodobenzoate (3.6 g, 8.30 mmol) was dissolved in MeOH (30 mL) and $NH_4OH$ (12.92 mL, 83 mmol) was added & reaction was stirred at room temperature for 16h. Solvent was evaporated and residue partitioned between water and DCM . Organic layer was separated, dried over anhydrous $Na_2SO_4$ and solvent was evaporated completely to get crude product. It was purified by flash chromatography in MeOH -DCM gradient to afford titled compound (1.5 g, 53.5 % yield)

MS(ES+) m/z = 340.08 (M+2)

Step-4: 5-bromo-6-iodo-2,3,3-trimethylisoindolin-1-one

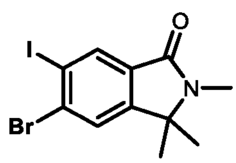

the titled compound was obtained from Step-3 intermediate by following analogous protocol used in Example 81-Step- 2 (1.6 g, 95 % yield)

MS(ES+) m/z = 380.20/382.17 (M+), (M+2)

Step-5: 6-bromo-1,1,2-trimethyl-3-oxoisoindoline-5-carbonitrile

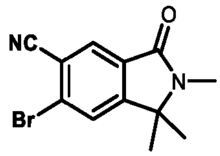

To a stirred solution of 5-bromo-6-iodo-2,3,3-trimethylisoindolin-1-one (1.6 g, 4.21 mmol) in DMF (20 mL) was added Cu(I)CN (0.377 g, 4.21 mmol), the reaction temperature was raised to 140°C & stirred for 16h. Reaction was cooled to room temperature, diluted with ice water and extracted with DCM (25 mL x 3). Organic layer was washed brine and dried over anhydrous $Na_2SO_4$. Solvent was evaporated and crude product obtained was purified by flash chromatography in ethyl acetate in hexane as gradient to afford titled compound. (1.1 g, 94 % yield)
MS(ES+) m/z = 280.85 (M+2)

Step-6: 6-bromo-1,1,2-trimethyl-3-oxoisoindoline-5-carboxylic acid

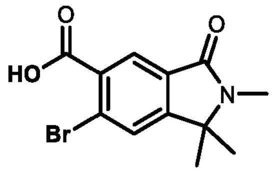

To a stirred solution of 6-bromo-1,1,2-trimethyl-3-oxoisoindoline-5-carbonitrile (1.1 g, 3.94 mmol) in Water (5.0 ml) was added sulfuric acid (4.20 ml, 79 mmol) and the reaction was stirred at 140°C for 36h. Reaction was cooled to room temperature & poured in to cold water. Aqueous layer was extracted with ethyl acetate (3 x 25.0 mL). Combined organic layer was dried over anhydrous Na2SO4 and concentrated under reduced pressure to afford titled compound (1.0 g, 85 % yield).
MS(ES+) m/z = 300.09 (M+2)

Step-7: 2,7, 8,8-tetramethyl-7,8-dihydro-3H-pyrrolo[3,4-g]quinazoline-4,6-dione

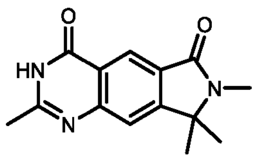

Titled compound was prepared by using Step-6 intermediate by employing analogous protocol mentioned in **Example 81- Step-5a** (0.61 g, 2.371 mmol, 70.7 % yield)
MS(ES+) m/z = 258.03 (M+2)

Step-8: 4-chloro-2,7,8,8-tetramethyl-7,8-dihydro-6H-pyrrolo[3,4-g]quinazolin-6-one

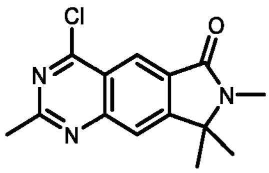

Titled compound was prepared by using Step-7 intermediate by employing analogous protocol mentioned in Example **71- Step-9** (0.32g 66% yield)

MS(ES+) m/z = 276.25

Step-9: (R)-4-((1-(3-(1,1-difluoro-2-hydroxy-2-methylpropyl)-2-fluorophenyl)ethyl)amino)-2,7,8,8-tetramethyl-7,8-dihydro-6H-pyrrolo[3,4-g]quinazolin-6-one **(Compound 118)**

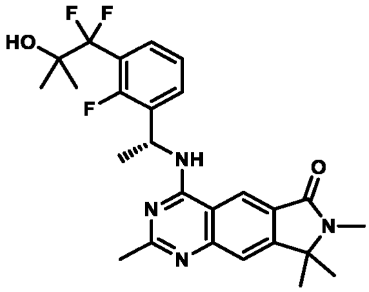

Titled compound was prepared by using Step-8 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71- Step-10** (0.077 g, 295 yield)

MS(ES+) m/z = 487.42 (M+1)

$^1$H NMR (400 MHz, DMSO-$d_6$) δ 8.91 (s, 1H), 8.84 (d, *J* = 7.4 Hz, 1H), 7.85 (s, 1H), 7.65 - 7.57 (m, 1H), 7.33 - 7.27 (m, 1H), 7.24 - 7.18 (m, 1H), 5.86 - 5.78 (m, 1H), 5.34 (s, 1H), 2.98 (s, 3H), 2.36 (s, 3H), 1.59 (d, *J*= 7.0 Hz, 3H), 1.50 (s, 3H), 1.49 (s, 3H), 1.24 (s, 3H), 1.21 (s, 3H).

**Example 132** : (R)-8-((1-(3-(1,1-difluoro-2-hydroxy-2-methylpropyl)-2-fluorophenyl)ethyl)amino)-3,6-dimethyl-1,3-dihydro-2H-imidazo[4,5-g]quinazolin-2-one **(Compound 119)**

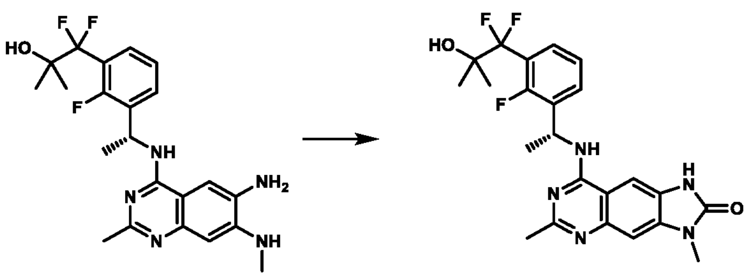

To a stirred solution of (R)-1-(3-(1-((6-amino-2-methyl-7-(methylamino)quinazolin-4-yl)amino)ethyl)-2-fluorophenyl)-1,1-difluoro-2-methylpropan-2-ol ( **Example 120- Step-5)** 0.077 g, 0.178 mmol) in DMF (2.0 mL) was added CDI (0.043 g, 0.266 mmol). The resulting mixture was stirred at 60°C for 1h. Reaction was cooled to room temperature and diluted with ice cold water. The precipitated solid was filtered, dried and purified by RP HPLC to afford titled compound. (0.010 g, 12.25 % yield) as a white solid.

$^1$H NMR (400 MHz, DMSO-$d_6$) δ 11.36 (s, 1H), 8.21 (d, J = 7.5 Hz, 1H), 7.95 (s, 1H), 7.65 - 7.56 (m, 1H), 7.34 - 7.25 (m, 1H), 7.24 - 7.15 (m, 2H), 5.87 - 5.74 (m, 1H), 5.32 (s, 1H), 3.34 (s, 3H), 2.31 (s, 3H), 1.57 (d, J = 7.0 Hz, 3H), 1.24 (s, 3H), 1.21 (s, 3H)..

MS(ES+) m/z = 460.30 (M+1)

**Example 133:** (S)-4-(((S)-1-(3-(1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one **(Compound 120)**

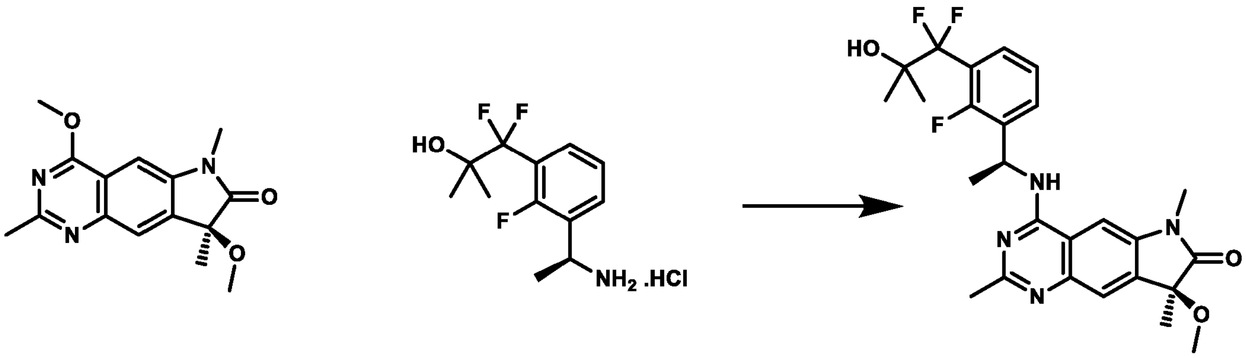

Titled compound was prepared by using **Example 114-Step-2a-Peak** 2 intermediate and (S)-1-(3-(1-Aminoethyl)-2-fluorophenyl)-1,1-difluoro-2-methylpropan-2-ol hydrochloride (Example 4-Step 6b) by employing analogous protocol mentioned in **Example 115** (0.015g, 17% yield)
MS(ES+) m/z = 503.42 (M+1)

$^1$H NMR (400 MHz, DMSO-$d_6$) δ 8.30 (d, *J*= 7.5 Hz, 1H), 7.95 (s, 1H), 7.64-7.57 (m, 1H), 7.56 (s, 1H), 7.34 - 7.29 (m, 1H), 7.24 - 7.18 (m, 1H), 5.87 - 5.77 (m, 1H), 5.35 (s, 1H), 3.30 (s, 3H), 2.91 (s, 3H), 2.33 (s, 3H), 1.61 (d, *J*= 7.0 Hz, 3H), 1.48 (s, 3H), 1.24 (s, 3H), 1.22 (s, 3H).

**Example 134:** (R&S)-4-(((R)-1-(3-(1,1-difluoro-2-hydroxy-2-methylpropyl)-2-fluorophenyl)ethyl)amino)-8-ethoxy-2,6,8-trimethyl-6,8-dihydro-7H-pyrrolo[2,3-g]quinazolin-7-one **(Compound 121a and Compound 121b)**

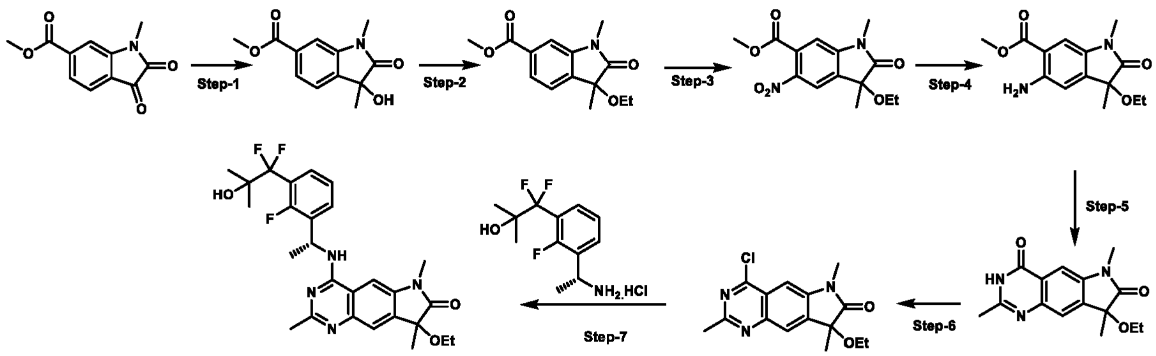


Step 1: Methyl 3-hydroxy-1,3-dimethyl-2-oxoindoline-6-carboxylate

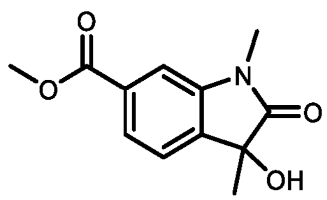

To a stirred solution of methyl 1-methyl-2,3-dioxoindoline-6-carboxylate (7 g, 31.9 mmol) in THF (70 ml) was added Methyl magnesium bromide (15.97 mL, 47.9 mmol) at -60 °C and stirred for 1 hr. Reaction was monitored by TLC, which showed complete consumption of starting material. The reaction mixture was poured in an ice-cold water and extracted with ethyl acetate (2×100 mL). The combined organic layers were washed with brine (200 mL). Separated

organic layer was dried over anh.$Na_2SO_4$ and filtered. The filtrate was rotary evaporated and the obtained crude residue was purified by column chromatography (silica gel, 30-80 % ethyl acetate-hexane mixture as eluent) to afford the titled compound (4.2 g, 17.85 mmol, 55.9 % yield) as a red solid.

Step-2: methyl 3-ethoxy-1,3-dimethyl-2-oxoindoline-6-carboxylate

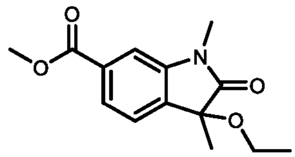

To a stirred solution of methyl 3-hydroxy-1,3-dimethyl-2-oxoindoline-6-carboxylate (4.2 g, 17.85 mmol) in DMF 15 ml was added ethyl iodide (1.731 ml, 21.43 mmol) followed by portion wise addition of NaH (1.285 g, 21.43 mmol) at -10 °C. The reaction mixture was stirred at -10 °C for 1 hr. Reaction was monitored by TLC, which showed complete consumption of starting material. The reaction mass was diluted with water (100 mL) and ethyl acetate (100 mL), the layers were separated, and the aqueous layer was extracted with ethyl acetate (2×50 mL). The combined organic layers were washed with brine (200 mL). Separated organic layer was dried over anh.$Na_2SO_4$ and filtered. The filtrate was rotary evaporated and the obtained crude residue was purified by column chromatography (silica gel, 30-80 % ethyl acetate-hexane mixture as eluent) to afford the titled compound (3.1 g, 11.77 mmol, 65.9 % yield)

$^1H$ NMR (400 MHz, DMSO-$d_6$) δ 7.79 - 7.72 (m, 1H), 7.55 - 7.49 (m, 2H), 3.88 (s, 3H), 3.20 (s, 3H), 3.08 - 2.91 (m, 2H), 1.44 (s, 3H), 1.01 (t, J = 7.0 Hz, 3H).

Step-3: Methyl 3-ethoxy-1,3-dimethyl-5-nitro-2-oxoindoline-6-carboxylate

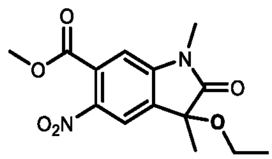

Titled compound was prepared by using step-2 intermediate by employing analogous protocol mentioned in Example **81- Step-6a.**

$^1H$ NMR (400 MHz, DMSO-$d_6$) δ 8.17 (s, 1H), 7.49 (s, 1H), 3.89 (s, 3H), 3.22 (s, 3H), 3.11 - 2.99 (m, 2H), 1.50 (s, 3H), 1.04 (t, J = 7.0 Hz, 3H).

Step-4: methyl 5-amino-3-ethoxy-1,3-dimethyl-2-oxoindoline-6-carboxylate

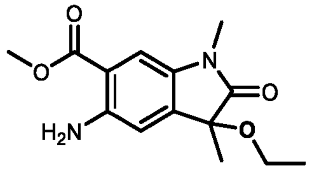

Titled compound was prepared by using step-3 intermediate by employing analogous protocol mentioned in **Example 14- Step-3.**

$^1H$ NMR (400 MHz, DMSO-$d_6$) δ 7.19 (s, 1H), 6.87 (s, 1H), 6.67 (s, 2H), 3.81 (s, 3H), 3.09 (s, 3H), 3.06 - 2.92 (m, 2H), 1.38 (s, 3H), 1.02 (t, J = 7.0 Hz, 3H).

Step-5: 8-ethoxy-2,6,8-trimethyl-6,8-dihydro-3H-pyrrolo[2,3-g]quinazoline-4,7-dione

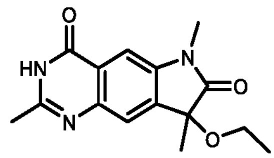

Titled compound was prepared by using Step-3 intermediate by employing analogous protocol mentioned in Example **71-Step-8** .

MS(ES+) m/z = 288.09 (M+1).

Step-6: 4-chloro-8-ethoxy-2,6,8-trimethyl-6,8-dihydro-7H-pyrrolo[2,3-g]quinazolin-7-one

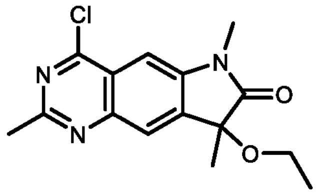

Titled compound was prepared by using Step-5 intermediate by employing analogous protocol mentioned in Example **71-Step-9** .

MS(ES+) m/z = 306.4 (M+1).

Step-7: (R & S)-4-(((R)-1-(3-(1,1-difluoro-2-hydroxy-2-methylpropyl)-2-fluorophenyl)ethyl)amino)-8-ethoxy-2,6,8-trimethyl-6,8-dihydro-7H-pyrrolo[2,3-g]quinazolin-7-one

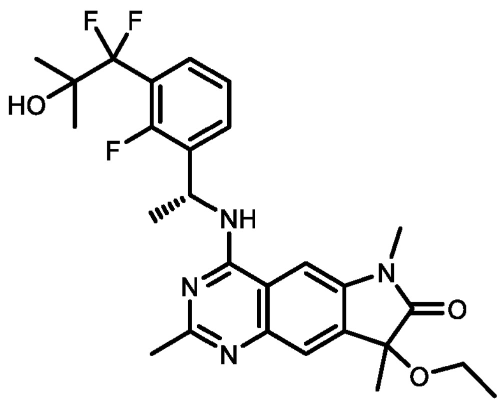

Titled compound was prepared by reaction of Step-6 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in Example **71-Step-10**

The two stereoisomers were separated by chiral preparative HPLC.

**Chiral HPLC Method:** HEX_0.1%DEA_IPA_DCM_70_30_B_C_1.0 ML _10MIN_/1.0 mL /min

Peak-1 : (R/S)-4-(((R)-1-(3-(1,1-difluoro-2-hydroxy-2-methylpropyl)-2-fluorophenyl)ethyl)amino)-8-ethoxy-2,6,8-trimethyl-6,8-dihydro-7H-pyrrolo[2,3-g]quinazolin-7-one (80 mg, 23.68 % yield) **(Compound 121a)**

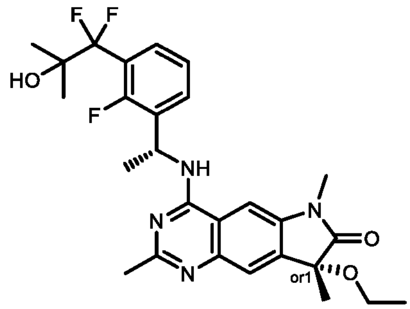

MS(ES+) m/z = 517.24 (M+1)
RT- 4.53 min

$^1$H NMR (400 MHz, DMSO-$d_6$) δ 8.36 - 8.22 (m, 1H), 7.94 (s, 1H), 7.63 - 7.57 (m, 1H), 7.55 (s, 1H), 7.35 - 7.29 (m, 1H), 7.25 - 7.18 (m, 1H), 5.87 - 5.78 (m, 1H), 5.35 (s, 1H), 3.29 (s, 3H), 3.14 - 2.94 (m, 2H), 2.33 (s, 3H), 1.61 (d, *J*= 7.1 Hz, 3H), 1.48 (s, 3H), 1.24 (s, 3H), 1.22 (s, 3H), 1.05 - 0.99 (m, 3H).

Peak-2 : (S/R)-4-(((R)-1-(3-(1,1-difluoro-2-hydroxy-2-methylpropyl)-2-fluorophenyl)ethyl)amino)-8-ethoxy-2,6,8-trimethyl-6,8-dihydro-7H-pyrrolo[2,3-g]quinazolin-7-one. (80 mg, 23.68 % yield) **(Compound 121b)**

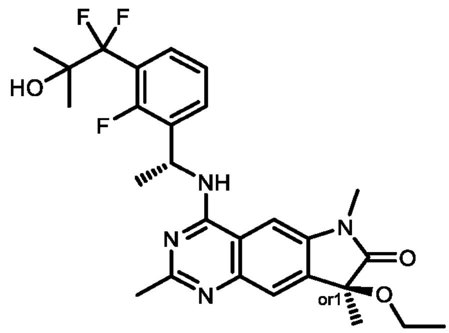

MS(ES+) m/z = 517.24 (M+1)
RT- 5.13 min

$^1$H NMR (400 MHz, DMSO-$d_6$) δ 8.33 - 8.26 (m, 1H), 7.94 (s, 1H), 7.65 - 7.57 (m, 1H), 7.55 (s, 1H), 7.36 - 7.27 (m, 1H), 7.26 - 7.14 (m, 1H), 5.86 - 5.78 (m, 1H), 5.35 (s, 1H), 3.29 (s, 3H), 3.12 - 2.91 (m, 2H), 2.33 (s, 3H), 1.61 (d, *J* = 7.0 Hz, 3H), 1.49 (s, 3H), 1.24 (s, 3H), 1.22 (s, 3H), 1.05 - 0.98 (m, 3H).

**Example 135:** (R)-4'-((1-(3-(1,1-difluoro-2-hydroxy-2-methylpropyl)-2-fluorophenyl)ethyl)amino)-2',6'-dimethyl-2,3,5,6-tetrahydrospiro[pyran-4,8'-pyrrolo[2,3-g]quinazolin]-7'(6'H)-one **(compound 122)**

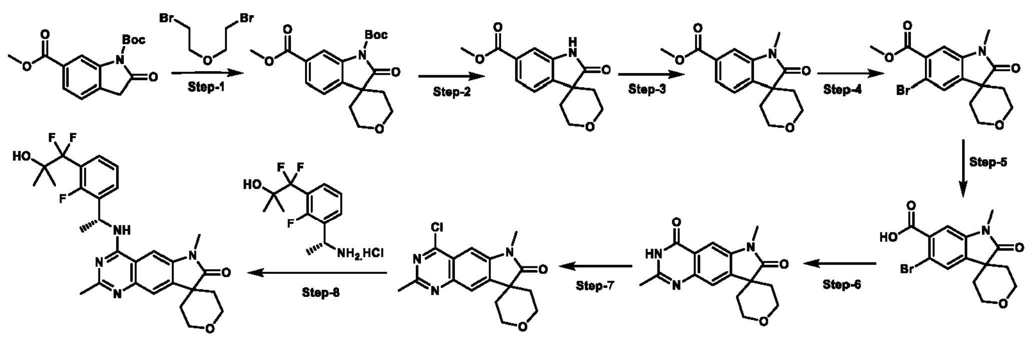

Step-1: 1-(tert-butyl) 6-methyl 2-oxo-2',3',5',6'-tetrahydrospiro[indoline-3,4'-pyran]-1,6-dicarboxylate

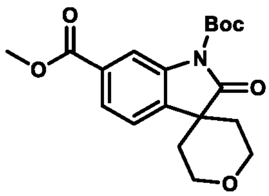

Titled compound was prepared by using **Example 92-Step-1** intermediate and appropriate dibromo compound by employing analogous protocol mentioned in Example **92-Step-2.**

MS(ES+) m/z = 261.96 (M-100), 303.21 (M-55)

Step-2: Methyl 2-oxo-2',3',5',6'-tetrahydrospiro[indoline-3,4'-pyran]-6-carboxylate

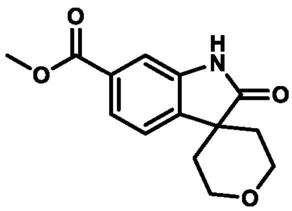

Titled compound was prepared from Step-1 intermediate by employing analogous protocol as mentioned in Example **71-Step-7**

MS(ES+) m/z = 262.2 (M+1)

Step-3: methyl 1-methyl-2-oxo-2',3',5',6'-tetrahydrospiro[indoline-3,4'-pyran]-6-carboxylate

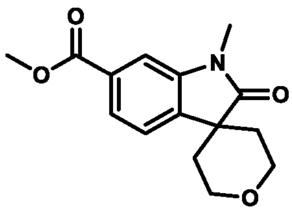

Titled compound was prepared from Step-2 intermediate by employing analogous protocol as mentioned in Example **71-Step-5**

MS(ES+) m/z = 276.40 (M+1)

Step-4 methyl 5-bromo-1-methyl-2-oxo-2',3',5',6'-tetrahydrospiro[indoline-3,4'-pyran]-6-carboxylate

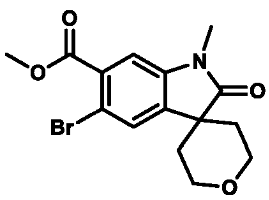

Titled compound was prepared from Step-3 intermediate by employing analogous protocol as mentioned in Example **81-Step-2a**

MS(ES+) m/z = 354.33(M+), 356.17 (M+2)

Step-5: 5-bromo-1-methyl-2-oxo-2',3',5',6'-tetrahydrospiro[indoline-3,4'-pyran]-6-carboxylic acid

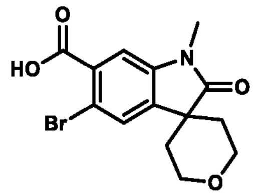

Titled compound was prepared from Step-4 intermediate by employing analogous protocol as mentioned in Example **81 Step-4a**

MS(ES+) m/z = 342.25 (M+2)

Step-6: 2',6'-dimethyl-2,3,3',5,6,6'-hexahydrospiro[pyran-4,8'-pyrrolo[2,3-g]quinazoline]-4',7'-dione

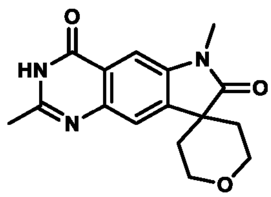

Titled compound was prepared from Step-5 intermediate by employing analogous protocol as mentioned in **Example 81- Step-5a**

MS(ES+) m/z = 300.25 (M+1)

Step-7: 4'-chloro-2',6'-dimethyl-2,3,5,6-tetrahydrospiro[pyran-4,8'-pyrrolo[2,3-g]quinazolin]-7'(6'H)-one

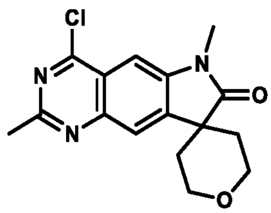

Titled compound was prepared from Step-6 intermediate by employing analogous protocol as mentioned in Example **71-Step-9**

MS(ES+) m/z = 318.46 (M+1)

Step-8 (R)-4'-((1-(3-(1,1-difluoro-2-hydroxy-2-methylpropyl)-2-fluorophenyl)ethyl)amino)-2',6'-dimethyl-2,3,5,6-tetrahydrospiro[pyran-4,8'-pyrrolo[2,3-g]quinazolin]-7'(6'H)-one **(compound 122)**

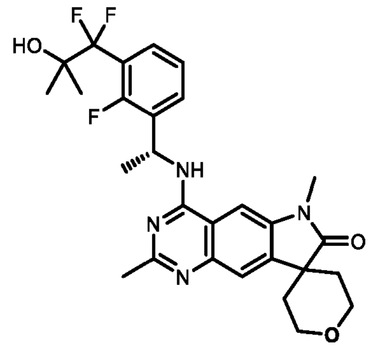

Titled compound was prepared by reaction of Step-7 intermediate and (R)-1-(3-(1-Aminoethyl)-2-fluorophenyl)-1,1-difluoro-2-methylpropan-2-ol hydrochloride (Example 4-Step 6a) by employing analogous protocol as mentioned in Example **71-Step-10**

MS(ES+) m/z = 529.37 (M+1)

$^1$H NMR (400 MHz, DMSO-$d_6$) δ 8.24 (d, $J$ = 7.4 Hz, 1H), 7.90 (s, 1H), 7.73 (s, 1H), 7.63 - 7.56 (m, 1H), 7.36 - 7.26 (m, 1H), 7.25 - 7.16 (m, 1H), 5.88 - 5.77 (m, 1H), 5.35 (s, 1H), 4.13 - 4.07 (m, 2H), 3.90 - 3.80 (m, 2H), 3.28 (s, 3H), 3.20 - 3.14 (m, 2H), 2.33 (s, 3H), 1.84 - 1.80 (m, 2H), 1.60 (d, $J$= 7.0 Hz, 3H), 1.23 (s, 3H) 1.21 (s, 3H).

**Example 136 :** (R&S)-4-(((R)-1-(3-(1,1-difluoro-2-hydroxy-2-methylpropyl)-2-fluorophenyl)ethyl)amino)-8-(2-methoxyethyl)-2,6,8-trimethyl-6,8-dihydro-7H-pyrrolo[2,3-g]quinazolin-7-one **(Compound 123a and Compound 123b)**

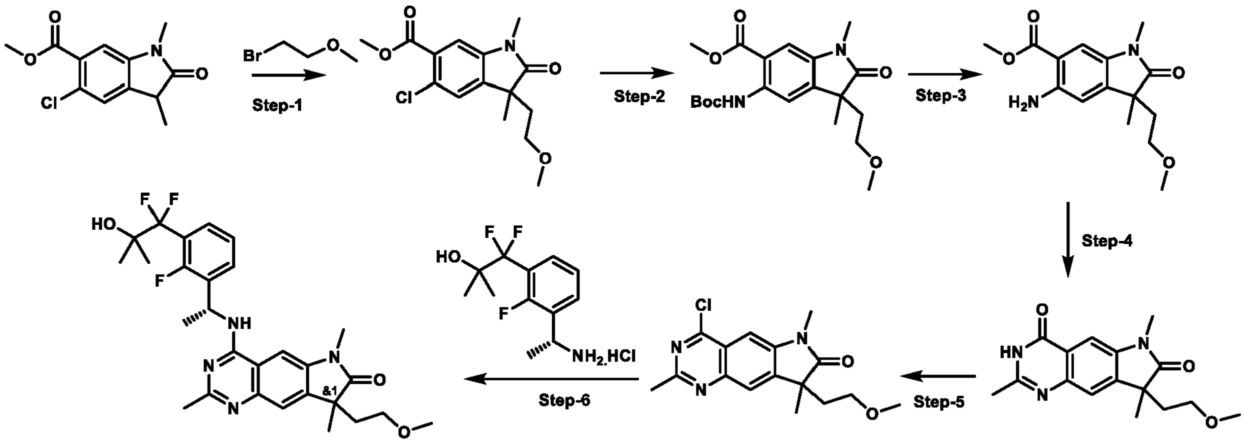


Step-1: Methyl 5-chloro-3-(2-methoxyethyl)-1,3-dimethyl-2-oxoindoline-6-carboxylate (10716-456)

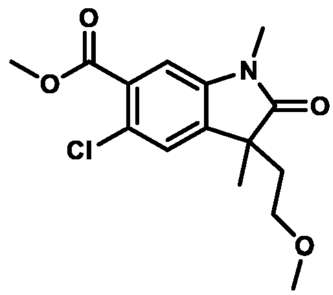

To solution of methyl 5-chloro-1,3-dimethyl-2-oxoindoline-6-carboxylate (0.500 g, 1.971 mmol) **Example 111-step 4** in DMF (10.0 mL) under nitrogen atmosphere was $Cs_2CO_3$ (1.284 g, 3.94 mmol) followed by addition of 1-bromo-2-methoxyethane (329 mg, 2.365 mmol). The reaction was stirred at room temperature for 12 h and quenched with addition

of water (10.0 mL) and extracted with Ethyl acetate (2 x 30.0 mL). The organic layer was separated, dried over anhydrous $Na_2SO_4$ and concentrated in vacuum. The crude residue was purified by flash column chromatography to afford titled compound (0.55 g, 90 % yield).

MS(ES+) m/z = 312.40 (M+1)

Step-2: methyl 5-((tert-butoxycarbonyl)amino)-3-(2-methoxyethyl)-1,3-dimethyl-2-oxoindoline-6-carboxylate

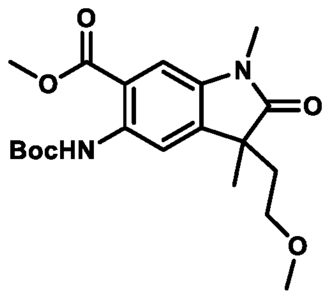

To a stirred solution of methyl 5-chloro-3-(2-methoxyethyl)-1,3-dimethyl-2-oxoindoline-6-carboxylate (0. 45 g, 1.443 mmol) in Dioxane (10.0 mL), tert-butyl carbamate (254 mg, 2.165 mmol), $Cs_2CO_3$ (1.035 g, 3.18 mmol) & the suspension was degassed with nitrogen for 10 min. $Pd(OAc)_2$ (32.4 mg, 0.144 mmol) and Chloro(2-dicyclohexylphosphino-2',4',6'-triisopropyl-1,1'-biphenyl)[2-(2'-amino-1,1'-biphenyl)]palladium(II), (103 mg, 0.217 mmol) were added and reaction was heated at 120 °C for 3 hr. Solvent was evaporated and residue was purified by flash column chromatography to afford titled compound (0.50 g, 88 % yield).

MS(ES+) m/z = 393.29 (M+1)

Step-3: Methyl 5-amino-3-(2-methoxyethyl)-1,3-dimethyl-2-oxoindoline-6-carboxylate

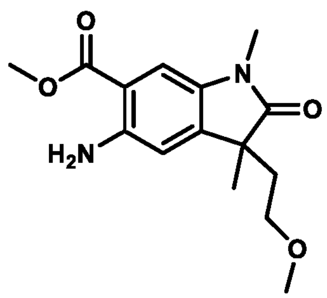

Titled compound was prepared from step-2 intermediate by employing analogous protocol mentioned in **Example 71-step-7** (0.40 g, 95 % yield),

MS(ES+) m/z = 293.34 (M+1)

Step-4: 8-(2-methoxyethyl)-2,6,8-trimethyl-6,8-dihydro-3H-pyrrolo[2,3-g]quinazoline-4,7-dione

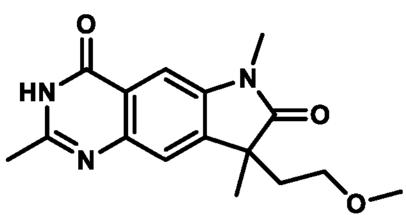

Titled compound was prepared from step-3 intermediate by employing analogous protocol mentioned in **Example 71-step-8** (0.250 g, 68.2 % yield).
MS(ES+) m/z = 302.46 (M+1)

Step-5: 4-chloro-8-(2-methoxyethyl)-2,6,8-trimethyl-6,8-dihydro-7H-pyrrolo[2,3-g]quinazolin-7-one

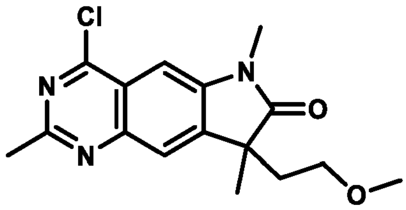

Titled compound was prepared from step-4 intermediate by employing analogous protocol mentioned in **Example 71-step-9** (0.250 g, 94 % yield).
MS(ES+) m/z = 320.03 (M+1)

Step-6: (R&S)-4-(((R)-1-(3-(1,1-difluoro-2-hydroxy-2-methylpropyl)-2-fluorophenyl)ethyl)amino)-8-(2-methoxyethyl)-2,6,8-trimethyl-6,8-dihydro-7H-pyrrolo[2,3-g]quinazolin-7-one **(compound 123)**

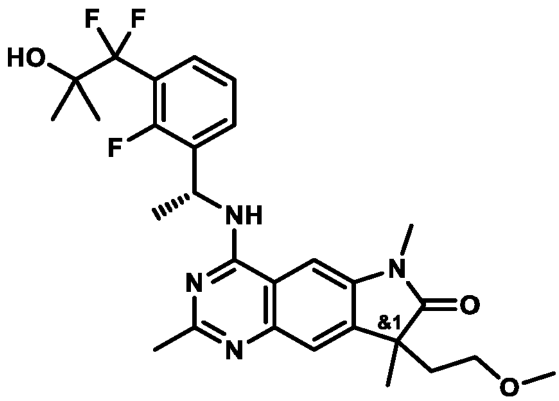

Titled compound was prepared by reaction of step-5 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in **Example 71-step-10.** The purification was done using preparative HPLC.
The two stereoisomers were separated by chiral preparative HPLC.

Peak-1 : (S/R)-4-(((R)-1-(3-(1,1-difluoro-2-hydroxy-2-methylpropyl)-2-fluorophenyl)ethyl)amino)-8-(2-methoxyethyl)-2,6,8-trimethyl-6,8-dihydro-7H-pyrrolo[2,3-g]quinazolin-7-one **(Compound 123a** )

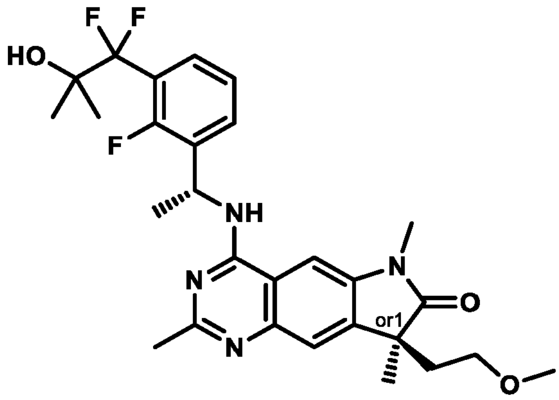

MS(ES+) m/z = 531.43 (M+1)

$^1$H NMR (400 MHz, DMSO-$d_6$) δ 8.32 - 8.24 (m, 1H), 7.89 (s, 1H), 7.62 - 7.58 (m, 2H), 7.34 - 7.30 (m, 1H), 7.24 - 7.19 (m, 1H), 5.84 - 5.79 (m, 1H), 5.35 (s, 1H), 3.26 (s, 3H), 3.04 -2.93 (m, 5H), 2.33 (s, 3H), 2.19 - 2.10 (m, 2H), 1.61 (d, *J*= 7.0 Hz, 3H), 1.33 (s, 3H), 1.25 (s, 3H), 1.22 (s, 3H).

Peak-2 : (R/S)-4-(((R)-1-(3-(1,1-difluoro-2-hydroxy-2-methylpropyl)-2-fluorophenyl)ethyl)amino)-8-(2-methoxyethyl)-2,6,8-trimethyl-6,8-dihydro-7H-pyrrolo[2,3-g]quinazolin-7-one **(Compound 123b)**

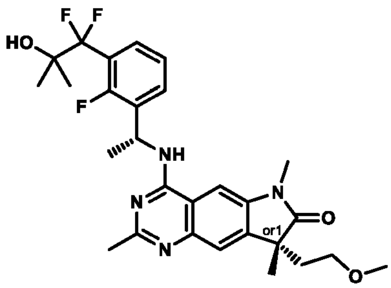

MS(ES+) m/z = 531.43 (M+1)

$^1$H NMR (400 MHz, DMSO-$d_6$) δ 8.41 - 8.28 (m, 1H), 7.93 - 7.87 (m, 1H), 7.68 - 7.58 (m, 2H), 7.36 - 7.27 (m, 1H), 7.27 - 7.18 (m, 1H), 5.89 - 5.78 (m, 1H), 5.34 (s, 1H), 3.26 (s, 3H), 3.05 - 2.89 (m, 5H), 2.34 (s, 3H), 2.21 - 2.07 (m, 2H), 1.61 (d, *J*= 7.0 Hz, 3H), 1.34 (s, 3H), 1.24 (s, 3H), 1.22 (s, 3H).

**Example 137:** (R&S)-4'-(((R)-1-(3-(1,1-difluoro-2-hydroxy-2-methylpropyl)-2-fluorophenyl)ethyl)amino)-2',3,6'-trimethylspiro[oxazolidine-5,8'-pyrrolo[2,3-g]quinazoline]-2,7'(6'H)-dione (Compound 124)

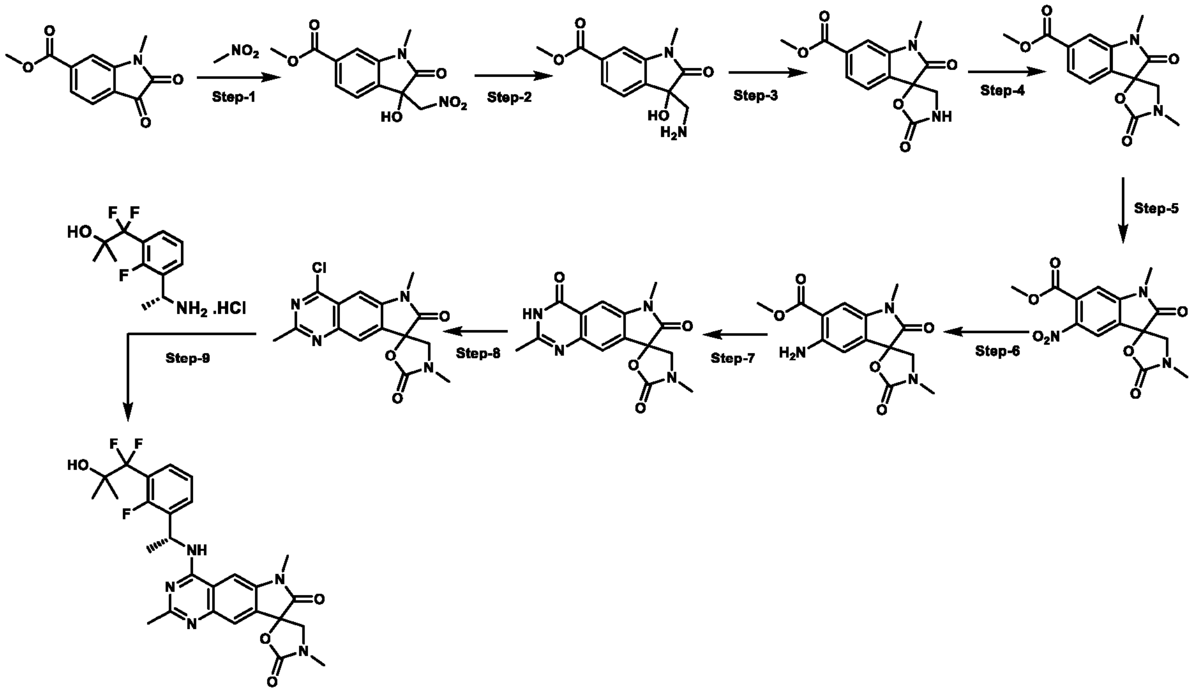


Step-1: methyl 3-hydroxy-1-methyl-3-(nitromethyl)-2-oxoindoline-6-carboxylate

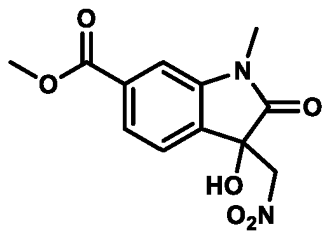

To a solution of methyl 1-methyl-2,3-dioxoindoline-6-carboxylate (500 mg, 2.281 mmol) in nitromethane (2785 mg, 45.6 mmol) was added DABCO (25.6 mg, 0.228 mmol) and the reaction was stirred at room temperature for 15 min. the mixture was diluted with ethyl acetate (20.0 mL) and water (20.0 mL). Organic layer was separated, washed with brine (10.0 mL), dried over anhydrous $Na_2SO_4$, and concentrated. The crude product was purified by flash chromatography in 50 % EA-Hexane to afford titled compound (0.520 g, 81% yield).
MS(ES+) m/z = 280.08 (M+)

Step-2: Methyl 3-(aminomethyl)-3-hydroxy-1-methyl-2-oxoindoline-6-carboxylate

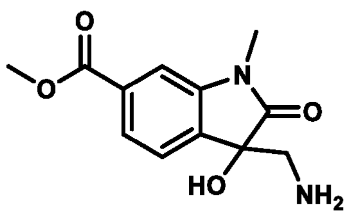

To a stirred solution of methyl 3-hydroxy-1-methyl-3-(nitromethyl)-2-oxoindoline-6-carboxylate (0.5 g, 1.784 mmol) in glacial acetic acid (15.0 mL), activated zinc (2.33 g, 35.7 mmol) dust was added portion wise over 45 min under inert atmosphere. The reaction mixture was stirred at room temperature 4 h and filtered through Celite bed. Celite bed was

washed with AcOH. Filtrate was concentrated under vacuum to give the title compound (520 mg crude, 100 yield).

MS(ES+) m/z = 251.17(M+1)

Step-3: methyl 1-methyl-2,2'-dioxospiro[indoline-3,5'-oxazolidine]-6-carboxylate

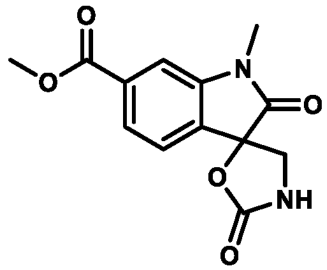

To a stirred solution of methyl 3-(aminomethyl)-3-hydroxy-1-methyl-2-oxoindoline-6-carboxylate (500 mg, 1.998 mmol) in THF (25.0 mL), CDI (389 mg, 2.398 mmol) was added at room temperature and reaction was stirred at 50°C for 16 h. The reaction was filtered through celite bed and washed celite bed with THF (30.0 mL) . Filtrate was concentrated under vacuum and the residue was purified by flash chromatography in MeOH-DCM gradient to afford titled compound. (0.31 g, 56.2% yield).

Step-4: methyl 1,3'-dimethyl-2,2'-dioxospiro[indoline-3,5'-oxazolidine]-6-carboxylate

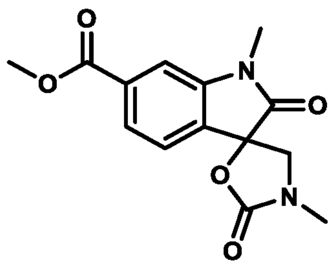

To a stirred solution of methyl 1-methyl-2,2'-dioxospiro[indoline-3,5'-oxazolidine]-6-carboxylate (100 mg, 0.362 mmol) in THF (Volume: 5 ml)under N2 was added with Methyl iodide(77 mg, 0.543 mmol) at RT Reaction mixture was cooled to 0°C and added Sodium Hydride (17.37 mg, 0.724 mmol) After stirring the mixture at 0°C for 1 hr. , the mixture was quenched with 20 ml of Aq.NH4Cl .Aqueous reaction mixture was extracted with Ethyl acetate(2* 15 ml).Combined organic layer was washed with water and brine. Organic layer was dried and concentrated to get crude compound which was purified in 80 to 90 % EA-Hexane to get as a 0.06 g brownish solid.

MS(ES+) m/z = 291.17 (M+1)

Step-5: Methyl 1,3'-dimethyl-5-nitro-2,2'-dioxospiro[indoline-3,5'-oxazolidine]-6-carboxylate

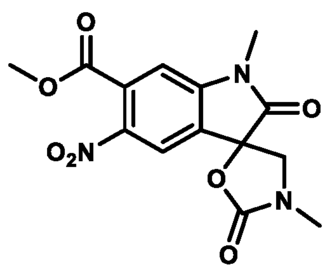

Titled compound was prepared from step-4 intermediate by employing analogous protocol mentioned in **Example 81-step-6a** (0.82 g 89% yield)
MS(ES+) m/z = 336.25 (M+1)

Step-6: methyl 5-amino-1,3'-dimethyl-2,2'-dioxospiro[indoline-3,5'-oxazolidine]-6-carboxylate

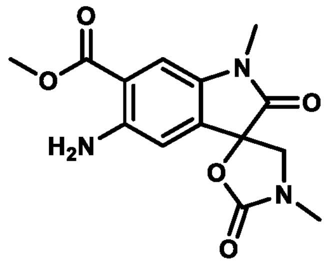

Titled compound was prepared from step-7 intermediate by employing analogous protocol mentioned in **Example 81-step-7a**
MS(ES+) m/z = 306.25 (M+1)

Step-7: 2',3,6'-trimethyl-3',6'-dihydrospiro[oxazolidine-5,8'-pyrrolo[2,3-g]quinazoline]-2,4',7'-trione

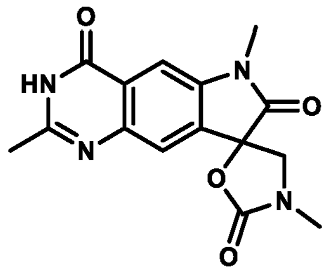

Titled compound was prepared from step-6 intermediate by employing analogous protocol mentioned in **Example 71-step-8**
MS(ES+) m/z = 315.25 (M+1)

Step-8: 4'-chloro-2',3,6'-trimethylspiro[oxazolidine-5,8'-pyrrolo[2,3-g]quinazoline]-2,7'(6'H)-dione

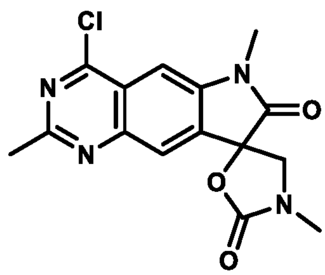

Titled compound was prepared from step-7 intermediate by employing analogous protocol mentioned in **Example 71-step-9**
MS(ES+) m/z = 333.40 (M+1)

Step-9: (R&S)-4'-(((R)-1-(3-(1,1-difluoro-2-hydroxy-2-methylpropyl)-2-fluorophenyl)ethyl)amino)-2',3,6'-trimethylspiro [oxazolidine-5,8'-pyrrolo[2,3-g]quinazoline]-2,7'(6'H)-dione

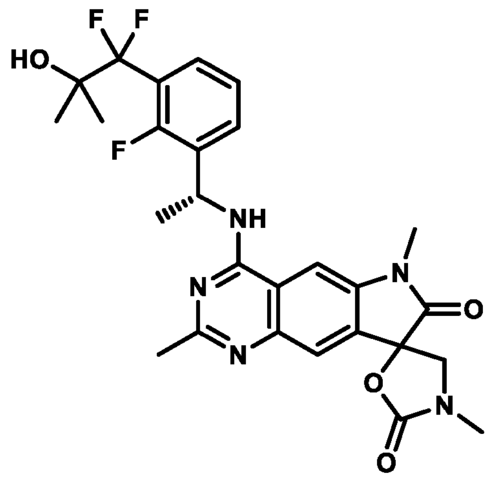

Titled compound was prepared by reaction of step-8 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in Example 71-step-10.

The two stereoisomers were separated by chiral preparative HPLC as

Chiral HPLC Method: CHIRALPAK IC CRL-087_MEOH_0.1%DEA_A_1.0ML_8MIN

Peak-1 : (S/R)-4'-(((R)-1-(3-(1,1-difluoro-2-hydroxy-2-methylpropyl)-2-fluorophenyl)ethyl)amino)-2',3,6'-trimethylspiro [oxazolidine-5,8'-pyrrolo[2,3-g]quinazoline]-2,7'(6'H)-dione **(Compound 124a)**

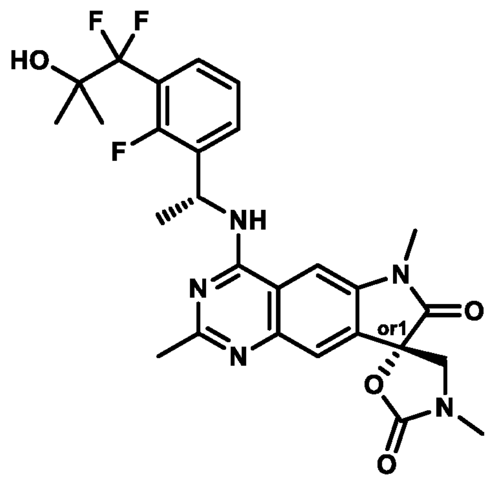

HPLC Rt: 4.02 min.
MS(ES+) m/z = 543.87 (M+1)

$^1H$ NMR (400 MHz, DMSO-$d_6$) δ 8.34 (d, *J* = 7.3 Hz, 1H), 7.95 (s, 1H), 7.88 (s, 1H), 7.61 - 7.53 (m, 1H), 7.35 - 7.28 (m, 1H), 7.25 - 7.18 (m, 1H), 5.85 - 5.77 (m, 1H), 5.35 (s, 1H), 3.85 (s, 2H), 3.28 (s, 3H), 2.90 (s, 3H), 2.34 (s, 3H), 1.62 (d, *J* = 7.0 Hz, 3H), 1.24 (s, 3H), 1.21 (s, 3H).

Peak-2: (R/S)-4'-(((R)-1-(3-(1,1-difluoro-2-hydroxy-2-methylpropyl)-2-fluorophenyl)ethyl)amino)-2',3,6'-trimethylspiro [oxazolidine-5,8'-pyrrolo[2,3-g]quinazoline]-2,7'(6'H)-dione **(Compound 124b)**

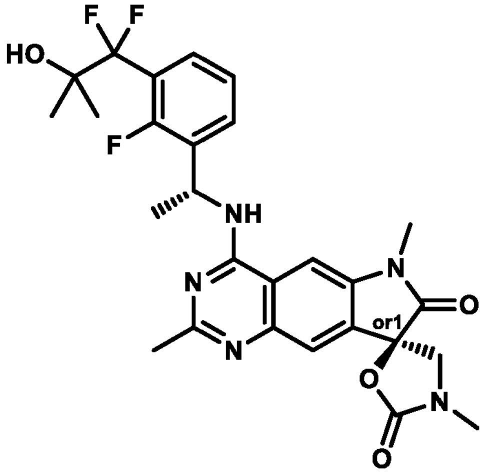

HPLC RT: 4.45 min.
MS(ES+) m/z = 543.87 (M+1)

$^1$H NMR (400 MHz, DMSO-$d_6$) δ 8.36 (d, *J* = 7.3 Hz, 1H), 7.95 (s, 1H), 7.88 (s, 1H), 7.63 - 7.56 (m, 1H), 7.36 - 7.28 (m, 1H), 7.26 - 7.19 (m, 1H), 5.85 - 5.77 (m, 1H), 5.35 (s, 1H), 3.87 (s, 2H), 3.28 (s, 3H), 2.91 (s, 3H), 2.34 (s, 3H), 1.61 (d, *J* = 7.0 Hz, 3H), 1.24 (s, 3H), 1.22 (s, 3H).

**Example 138** : (R&S)- 4-(((R)-1-(3-(1,1-difluoro-2-hydroxy-2-methylpropyl)-2-fluorophenyl)ethyl)amino)-8-methoxy-2,6-dimethyl-8-(trifluoromethyl)-6,8-dihydro-7H-pyrrolo[2,3-g]quinazolin-7-one **(Compound 125a and Compound 125b)**

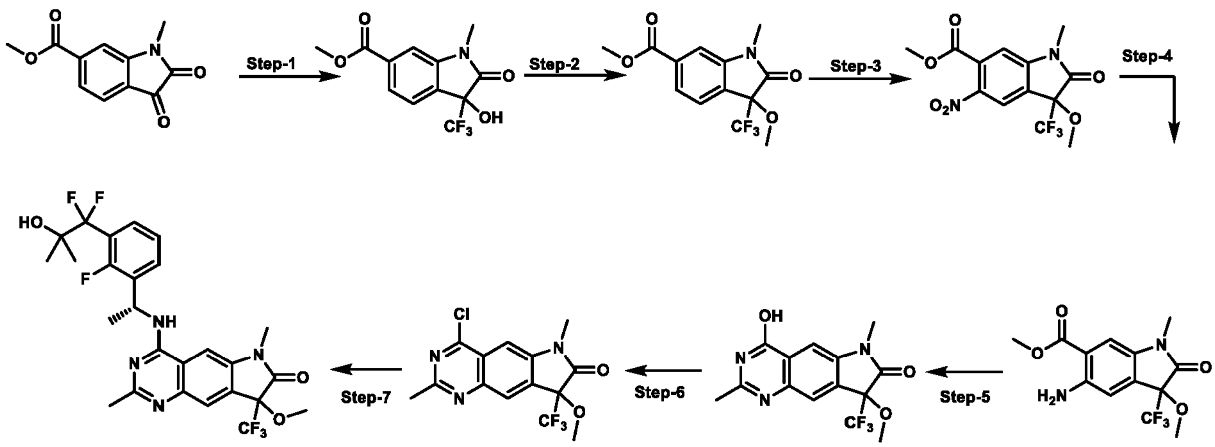


Step 1 : Methyl 3-hydroxy-1-methyl-2-oxo-3-(trifluoromethyl)indoline-6-carboxylate

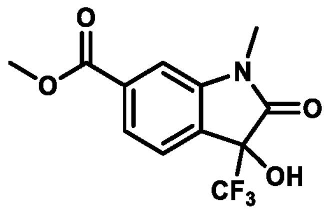

To a stirred solution of methyl 1-methyl-2,3-dioxoindoline-6-carboxylate (0.5 g, 2.281 mmol) in dry THF (10.0 mL), Trifluoromethyl triethylsilane (0.515 ml, 2.74 mmol) was added dropwise at room temperature. To this TBAF 1.0 M in THF (0.456 ml, 0.456 mmol) was added and then the reaction mixture was stirred at room temperature for 3h and then heated at 60 °C for 16h. The reaction mixture was treated with aqueous $NaHCO_3$ and was extracted with ethyl acetate (100 ml). The organic layers were collected and dried with anhydrous sodium sulphate, followed by solvent removal under reduced pressure to get crude (0.6 g). This crude residue was purified by Flash chromatography using eluent (20-30% ethyl acetate in hexane) to afford methyl 3-hydroxy-1-methyl-2-oxo-3-(trifluoromethyl)indoline-6-carboxylate (0.251 g, 0.868 mmol, 38.0 % yield) as a pale yellow solid.

MS (ES+) m/z = 290.17 (M+1).

1H NMR (400 MHz, DMSO-$d_6$) δ 7.94 (s, 1H), 7.80 (dd, J = 7.7, 1.5 Hz, 1H), 7.65 - 7.60 (m, 2H), 3.90 (s, 3H), 3.24 (s, 3H).

Step 2 : Methyl 3-methoxy-1-methyl-2-oxo-3-(trifluoromethyl)indoline-6-carboxylate

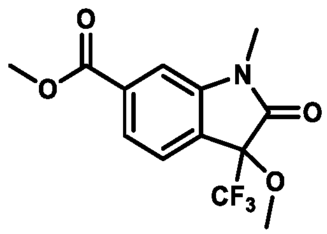

To a stirring solution of methyl 3-hydroxy-1-methyl-2-oxo-3-(trifluoromethyl)indoline-6-carboxylate (2.2 g, 7.61 mmol) **(Step-1)** in dry DMF (Volume: 5 ml) was added NaH (0.365 g, 9.13 mmol) at 0 °C stirred for 15 min and followed by addition of MeI (0.713 ml, 11.41 mmol) at 0 °C and stirred at room temperature for 2hr.After completion checked by TLC, reaction mixture was quenched with ice cooled water and extracted with ethyl acetate (200 ml x2) . The organic layers were collected and dried with anhydrous sodium sulphate, concentrated under reduced pressure to get crude (3.0g). This crude residue was purified by Flash chromatography column chromatography using eluent (30% ethyl acetate in hexane) to afford methyl 3-methoxy-1-methyl-2-oxo-3-(trifluoromethyl)indoline-6-carboxylate (2.155 g, 7.11 mmol, 93 % yield) as an off white solid.

MS (ES+) m/z = 304.42 (M+1).

Step 3 : Methyl 3-methoxy-1-methyl-5-nitro-2-oxo-3-(trifluoromethyl)indoline-6-carboxylate

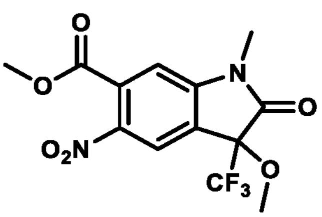

potassium nitroperoxous acid ($KNO_3$)(0.092 g, 0.907 mmol) was added portion wise to a solution of methyl 3-methoxy-1-methyl-2-oxo-3-(trifluoromethyl)indoline-6-carboxylate (0.25 g, 0.824 mmol) **(Step-2 product)** in $H_2SO_4$ (5 mL) at 0 °C over 10 min. The reaction mixture was stirred for 1hr at 0 °C. Reaction mixture was poured on ice cooled water , solid precipitates out which was filtered and concentrated in vacuo to get methyl 3-methoxy-1-methyl-5-nitro-2-oxo-3-(trifluoromethyl)indoline-6-carboxylate (0.28 g, 96 % yield) as a light yellow solid.

$^1$H NMR (400 MHz, DMSO-$d_6$) $\delta$ 8.20 (s, 1H), 7.70 (s, 1H), 3.91 (s, 3H), 3.31 (s, 3H), 3.16 (s, 3H).

Step 4 : Methyl 5-amino-3-methoxy-1-methyl-2-oxo-3-(trifluoromethyl)indoline-6-carboxylate

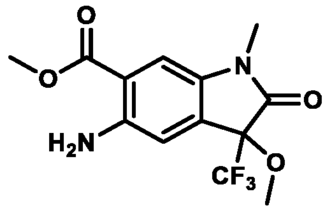

To a stirred solution of methyl 3-methoxy-1-methyl-5-nitro-2-oxo-3-(trifluoromethyl)indoline-6-carboxylate (2.0 g, 5.74 mmol) (Step-3 product) in MeOH (30 mL) ,Tetrahydrofuran ( 10 mL) & Water(4 mL) were added Ammonium chloride (3.07 g, 57.4 mmol) at room temperature. The resulting reaction mixture was heated to 90 °C, then to it added portion wise IRON (0.962 g, 17.23 mmol) at 90 °C and continued the heating for 2h. After completion reaction mixture was filtered through pad of celite bed and concentrated in vacuo. Residue was partitioned between ethyl acetate (100 mL) and water (50 mL), then extracted with Ethyl acetate (100 mL x 2). The combined organic extracts were washed with brine, dried over sodium sulphate, and concentrated to get crude (3.0 g). This crude residue was purified by flash column chromatography with gradient elution (50%) of ethyl acetate in hexane to afford titled compound (955 mg, 3.00 mmol, 52.2 % yield) as light brown solid. MS (ES+) m/z = 319.08 (M+1).

Step 5 : 8-methoxy-2,6-dimethyl-8-(trifluoromethyl)-6,8-dihydro-3H-pyrrolo[2,3-g]quinazoline-4,7-dione

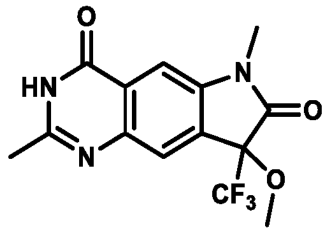

The titled compound was prepared from step-4 intermediate by employing analogous protocol mentioned in **example 71-step-8** .

MS (ES+) m/z = 328.17 (M+1).

Step 6 : 4-chloro-8-methoxy-2,6-dimethyl-8-(trifluoromethyl)-6,8-dihydro-7H-pyrrolo[2,3-g]quinazolin-7-one

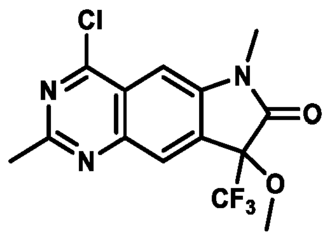

The titled compound was prepared from step-5 intermediate by employing analogous protocol mentioned in **example 71-step-9** (0.305 g, 64.2 % yield).

MS (ES+) m/z = 346.34 (M+1).

Step 7 : (R&S)- 4-(((R)-1-(3-(1,1-difluoro-2-hydroxy-2-methylpropyl)-2-fluorophenyl)ethyl)amino)-8-methoxy-2,6-dimethyl-8-(trifluoromethyl)-6,8-dihydro-7H-pyrrolo[2,3-g]quinazolin-7-one

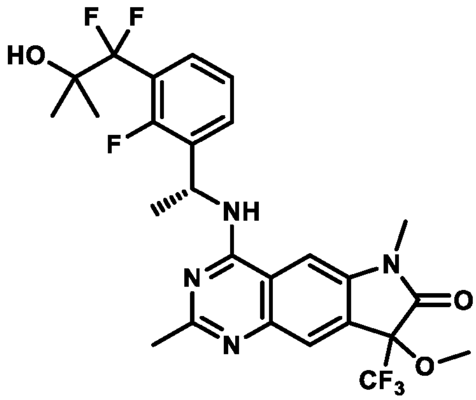

Titled compound was prepared by reaction of step-6 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in Example 71-step-10.

The two stereoisomers were separated by chiral preparative HPLC.

Chiral prep method (HEX_0.1%DEA_IPA_DCM_70_30_B_C_1.0ML_10MIN_250nm)

Peak-1 : (R/S)-4-(((R)-1-(3-(1,1-difluoro-2-hydroxy-2-methylpropyl)-2-fluorophenyl)ethyl)amino)-8-methoxy-2,6-dimethyl-8-(trifluoromethyl)-6,8-dihydro-7H-pyrrolo[2,3-g]quinazolin-7-one **(Compound 125a** )

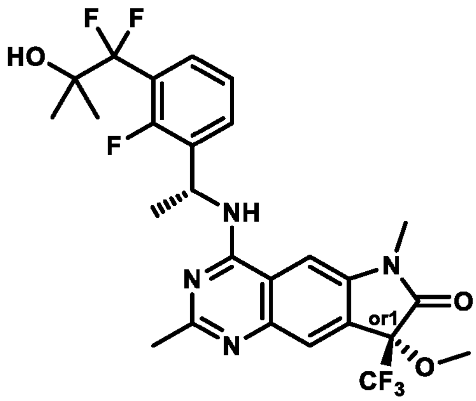

MS (ES+) m/z = 557.32 (M+1)
Peak-1: Retention time (min) - 4.17

1H NMR (400 MHz, DMSO-$d_6$) δ 8.48 (d, J = 7.0 Hz, 1H), 8.13 (s, 1H), 7.67 (s, 1H), 7.65 - 7.58 (m, 1H), 7.35 - 7.30 (m, 1H), 7.25 - 7.19 (m, 1H), 5.87 - 5.76 (m, 1H), 5.35 (s, 1H), 3.37 (s, 3H), 3.13 (s, 3H), 2.36 (s, 3H), 1.62 (d, J = 7.0 Hz, 3H), 1.24 (s, 3H), 1.22 (s, 3H).

Peak-2 : (S/R)-4-(((R)-1-(3-(1,1-difluoro-2-hydroxy-2-methylpropyl)-2-fluorophenyl)ethyl)amino)-8-methoxy-2,6-dimethyl-8-(trifluoromethyl)-6,8-dihydro-7H-pyrrolo[2,3-g]quinazolin-7-one **(Compound 125b )**

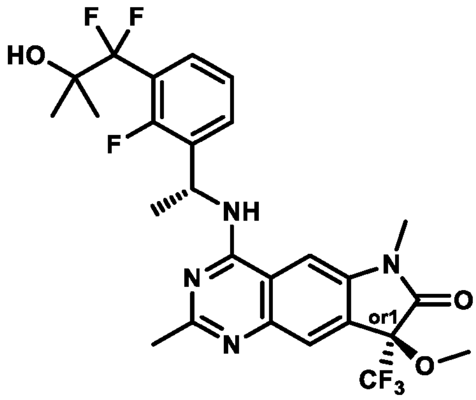

MS (ES+) m/z = 557.32 (M+1)
Peak-2: Retention time (min) - 4.69.

$^1H$ NMR (400 MHz, DMSO-$d_6$) δ 8.54 - 8.46 (m, 1H), 8.13 (s, 1H), 7.67 (s, 1H), 7.65 - 7.59 (m, 1H), 7.36 - 7.30 (m, 1H), 7.25 - 7.20 (m, 1H), 5.87 - 5.77 (m, 1H), 5.35 (s, 1H), 3.37 (s, 3H), 3.11 (s, 3H), 2.36 (s, 3H), 1.62 (d, $J$ = 7.1 Hz, 3H), 1.24 (s, 3H), 1.22 (s, 3H).

**Example 139 :** (R&S)-4-(((R)-1-(3-(1,1-difluoro-2-hydroxy-2-methylpropyl)-2-fluorophenyl)ethyl)amino)-8-ethyl-8-methoxy-2,6-dimethyl-6,8-dihydro-7H-pyrrolo[2,3-g]quinazolin-7-one **(Compound 126a) and (Compound 126b)**

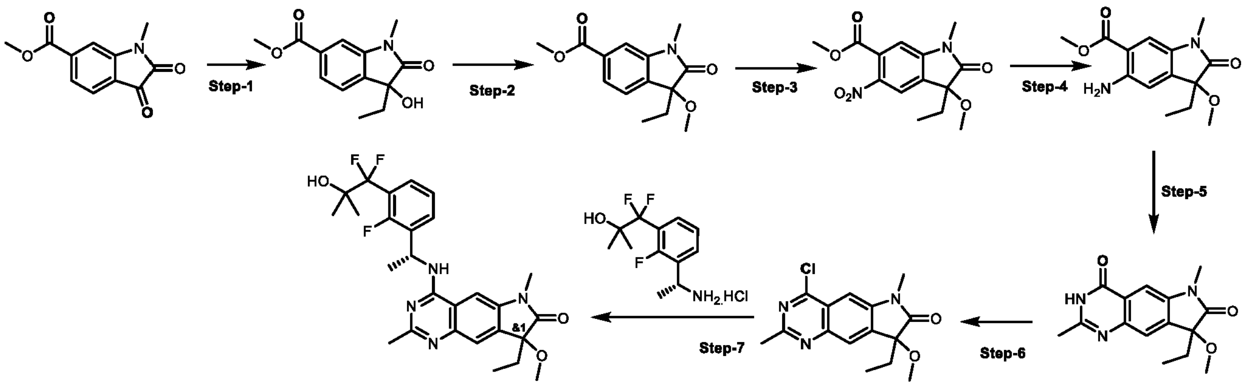


Step-1: methyl 3-ethyl-3-hydroxy-1-methyl-2-oxoindoline-6-carboxylate

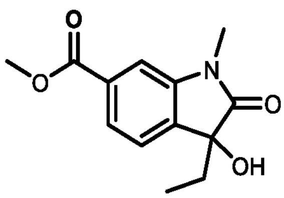

Titled compound was prepared from methyl 1-methyl-2,3-dioxoindoline-6-carboxylate and ethyl magnesium bromide by employing analogous protocol mentioned in **example-134-step 1**

MS (ES+) m/z= 273.40 (M+23)

Step-2: Methyl 3-ethyl-3-methoxy-1-methyl-2-oxoindoline-6-carboxylate

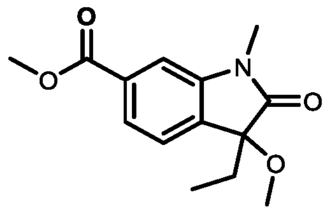

Titled compound was prepared from step-1 intermediate by employing analogous protocol mentioned in **example 134-step 2**

$^1H$ NMR (400 MHz, DMSO-$d_6$) δ 7.78 (dd, $J$ = 7.6, 1.5 Hz, 1H), 7.55 (d, $J$ = 1.4 Hz, 1H), 7.49 (d, $J$ = 7.6 Hz, 1H), 3.89 (s, 3H), 3.21 (s, 3H), 2.88 (s, 3H), 1.95 - 1.77 (m, 2H), 0.62 (t, $J$ = 7.5 Hz, 3H).

Step-3: Methyl 3-ethyl-3-methoxy-1-methyl-5-nitro-2-oxoindoline-6-carboxylate

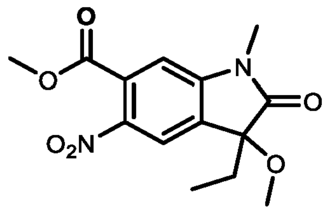

Titled compound was prepared from step-2 intermediate by employing analogous protocol mentioned in **example 81-step 6a.**

$^1H$ NMR (400 MHz, DMSO-$d_6$) δ 8.12 (s, 1H), 7.52 (s, 1H), 3.89 (s, 3H), 3.24 (s, 3H), 2.94 (s, 3H), 1.98 - 1.80 (m, 2H), 0.67 (t, $J$ = 7.5 Hz, 3H).

Step-4: methyl 5-amino-3-ethyl-3-methoxy-1-methyl-2-oxoindoline-6-carboxylate

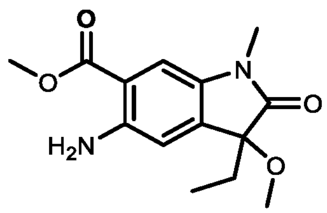

Titled compound was prepared from step-3 intermediate by employing analogous protocol mentioned in example 14-step 3

MS(ES+) m/z = 279.46 (M+1)

Step-5: 8-ethyl-8-methoxy-2,6-dimethyl-6,8-dihydro-3H-pyrrolo[2,3-g]quinazoline-4,7-dione

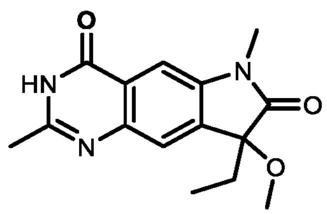

Titled compound was prepared from step-4 intermediate by employing analogous protocol mentioned in

**example 71-step-8**

MS(ES+) m/z = 288.09 (M+1)

Step-6: 4-chloro-8-ethyl-8-methoxy-2,6-dimethyl-6,8-dihydro-7H-pyrrolo[2,3-g]quinazolin-7-one

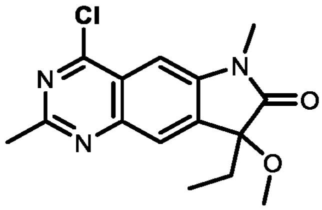

Titled compound was prepared from step-5 intermediate by employing analogous protocol mentioned in **example 71-step-9.**

MS(ES+) m/z = 306.46 (M+1)

Step-7: (R&S)-4-(((R)-1-(3-(1,1-difluoro-2-hydroxy-2-methylpropyl)-2-fluorophenyl)ethyl)amino)-8-ethyl-8-methoxy-2,6-dimethyl-6,8-dihydro-7H-pyrrolo[2,3-g]quinazolin-7-one

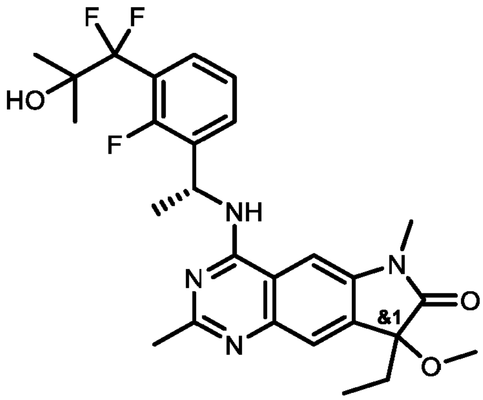

Titled compound was prepared by reaction of step-6 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in example 71-step-10

The two stereoisomers were separated by chiral preparative HPLC.

Chiral HPLC method HEX 0.1% DEA_IPA_MEOH_90_10_A_C_1.5ML_16MIN_294nM

Peak 1 : (R/S)-4-(((R)-1-(3-(1,1-difluoro-2-hydroxy-2-methylpropyl)-2-fluorophenyl)ethyl)amino)-8-ethyl-8-methoxy-2,6-dimethyl-6,8-dihydro-7H-pyrrolo[2,3-g]quinazolin-7-one **(Compound 126a)**

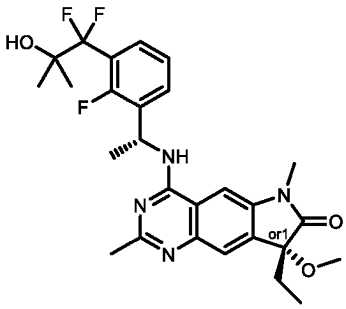

MS(ES+) m/z = 517.50
Chiral HPLC RT: 7.38 min

$^1$HNMR (400 MHz, DMSO-$d_6$) δ 8.31 (d, *J* = 7.3 Hz, 1H), 7.96 (s, 1H), 7.70 - 7.60 (m, 1H), 7.52 (s, 1H), 7.38 - 7.27 (m, 1H), 7.25 - 7.16 (m, 1H), 5.86 - 5.75 (m, 1H), 5.35 (s, 1H), 3.31 (s, 3H), 2.90 (s, 3H), 2.34 (s, 3H), 1.98 - 1.88 (m, 2H), 1.61 (d, *J* = 7.1 Hz, 3H), 1.24 (s, 3H), 1.22 (s, 3H), 0.64 (t, *J* = 7.4 Hz, 3H).

Peak 2 : (S/R)-4-(((R)-1-(3-(1,1-difluoro-2-hydroxy-2-methylpropyl)-2-fluorophenyl)ethyl)amino)-8-ethyl-8-methoxy-2,6-dimethyl-6,8-dihydro-7H-pyrrolo[2,3-g]quinazolin-7-one **(Compound 126b)**

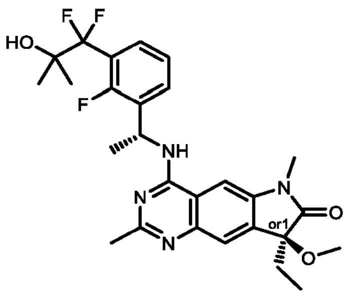

MS(ES+) m/z = 517.50
Chiral HPLC Rt: 8.60 min

$^1$H NMR (400 MHz, DMSO-$d_6$) δ 8.31 (d, *J* = 7.4 Hz, 1H), 7.96 (s, 1H), 7.66 - 7.60 (m, 1H), 7.52 (s, 1H), 7.36 - 7.28 (m, 1H), 7.26 - 7.17 (m, 1H), 5.91 - 5.73 (m, 1H), 5.34 (s, 1H), 3.30 (s, 3H), 2.91 (s, 3H), 2.34 (s, 3H), 1.97 - 1.87 (m, 2H), 1.61 (d, *J* = 7.1 Hz, 3H), 1.24 (s, 3H), 1.22 (s, 3H), 0.64 (t, *J* = 7.5 Hz, 3H).

**Example 140** : 4-(((R)-1-(3-((R & S)-1,1-difluoro-2-hydroxy-3-methoxy-2-methylpropyl)-2-fluorophenyl)ethyl)amino)-2,6,8,8-tetramethyl-6,8-dihydro-7H-pyrrolo[2,3-g]quinazolin-7-one **(Compound 127)**

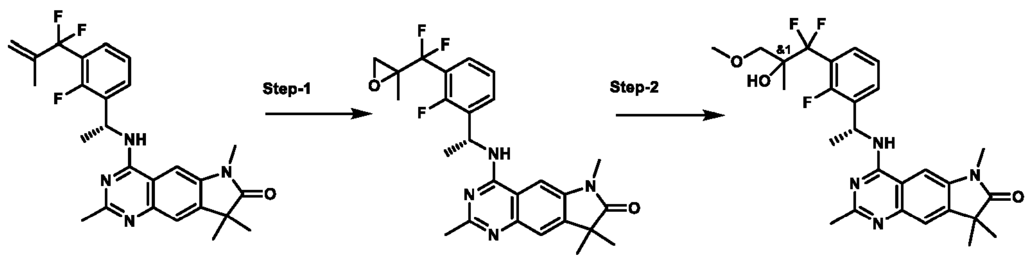

Step-1: 4-(((1R)-1-(3-(difluoro(2-methyloxiran-2-yl)methyl)-2-fluorophenyl)ethyl)amino)-2,6,8,8-tetramethyl-6,8-dihydro-7H-pyrrolo[2,3-g]quinazolin-7-one

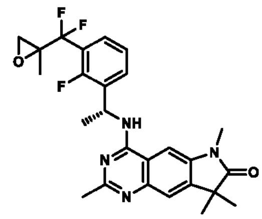

To a stirred solution of (R)-4-((1-(3-(1,1-difluoro-2-methylallyl)-2-fluorophenyl)ethyl)amino)-2,6,8,8-tetramethyl-6,8-dihydro-7H-pyrrolo[2,3-g]quinazolin-7-one (Example 89- step-1 , 2.0 gm, 4.27 mmol) in formic acid (10.0 mL) 50 % $H_2O_2$ (5.23 mL, 85 mmol) was added and reaction was stirred at 25 °C for 18 h. Reaction was concentrated in vacuum, residue was diluted with water (10.0 mL) and extracted with DCM ( 2 x 20 mL), Combined organic layer was dried over anhydrous $Na_2SO_4$ and evaporated to give titled compound (1.8 gm, 87 % yield). The crude product was used as such for next step.

MS(ES+) m/z = 435.3 (M+1)

Step-2 : 4-(((1R)-1-(3-(R&S)-(1,1-difluoro-2-hydroxy-3-methoxy-2-methylpropyl)-2-fluorophenyl)ethyl)amino)-2,6,8,8-tetramethyl-6,8-dihydro-7H-pyrrolo[2,3-g]quinazolin-7-one

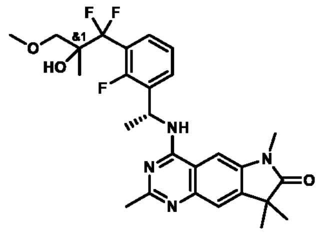

To a stirred solution of 4-(((1R)-1-(3-(difluoro(2-methyloxiran-2-yl)methyl)-2-fluorophenyl)ethyl)amino)-2,6,8,8-tetramethyl-6,8-dihydro-7H-pyrrolo[2,3-g]quinazolin-7-one ( Step-1, 0.50 gm, 1.032 mmol) in methanol (5.0 mL) sodium methoxide (0. 929 gm, 5.16 mmol) was added and reaction was stirred at 80 °C for 16 h. The reaction mixture was evaporated on rotary evaporator, quenched with ice water and extracted twice with ethyl acetate (2 x 20.0 mL). Combined organic layer was washed with brine (10.0 mL), dried over and the combined organic layer was washed with brine (15.0 mL) dried over anhydrous $Na_2SO_4$ and evaporated. Crude product was purified by flash chromatography in DCM-MeOH gradient to afford titled compound (0.16 gm, 30% yield) as diastereomeric mixture. The two diastereomers were separated by chiral preparative HPLC to provide titled compound.

Chiral separation method: CHIRALPAK IC CRL-087 HEX_0.1%DEA_IPA _75_25_B_D_1.2ML_12MIN_250nm, 1.2 ml/min

Peak-2 : 4-(((R)-1-(3-((R/S)-1,1-difluoro-2-hydroxy-3-methoxy-2-methylpropyl)-2-fluorophenyl)ethyl)amino)-2,6,8,8-tetramethyl-6,8-dihydro-7H-pyrrolo[2,3-g]quinazolin-7-one **(Compound 127a)**

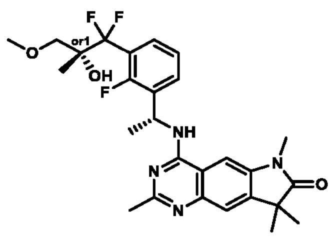

$^{1}H$ NMR (400 MHz, DMSO-$d_6$) δ 8.21 (d, *J* = 7.5 Hz, 1H), 7.90 (s, 1H), 7.64 - 7.58 (m, 2H), 7.35 - 7.27 (m, 1H), 7.25 - 7.14 (m, 1H), 5.88 - 5.72 (m, 1H), 5.50 (s, 1H), 3.47 - 3.40 (m, 1H), 3.32 - 3.27 (m, 4H), 3.25 (s, 3H), 2.34 (s, 3H), 1.60 (d, *J* = 7.1 Hz, 3H), 1.35 (s, 3H), 1.34 (s, 3H), 1.24 (s, 3H).

MS (ES+) m/z = 517.36 (M+1)
Chiral HPLC Rt: 6.51 min

Peak 1: 4-(((R)-1-(3-((S/R)-1,1-difluoro-2-hydroxy-3-methoxy-2-methylpropyl)-2-fluorophenyl)ethyl)amino)-2,6,8,8-tetramethyl-6,8-dihydro-7H-pyrrolo[2,3-g]quinazolin-7-one **(Compound 127b)**

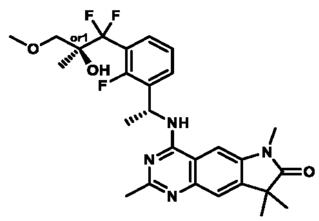

$^{1}H$ NMR (400 MHz, DMSO-$d_6$) δ 8.21 (d, J = 7.5 Hz, 1H), 7.90 (s, 1H), 7.64 - 7.58 (m, 2H), 7.35 - 7.27 (m, 1H), 7.25 - 7.14 (m, 1H), 5.88 - 5.72 (m, 1H), 5.48 (s, 1H), 3.49 - 3.43 (m, 1H), 3.32 - 3.30 (m, 1H), 3.28 (s, 3H), 3.25 (s, 3H), 2.32 (s, 3H), 1.61 (d, *J* = 7.0 Hz, 3H), 1.35 (s, 3H), 1.34 (s, 3H), 1.22 (s, 3H).

MS (ES+) m/z = 517.3 (M+1)
Chiral HPLC Rt: 5.46 min

**Example 141 :** (R & S)-4'-(((R)-1-(3-(1,1-difluoro-2-hydroxy-2-methylpropyl)-2-fluorophenyl)ethyl)amino)-2',6'-dimethyl-4,5-dihydro-2H-spiro[furan-3,8'-pyrrolo[2,3-g]quinazolin]-7'(6'H)-one **(Compound 128)**

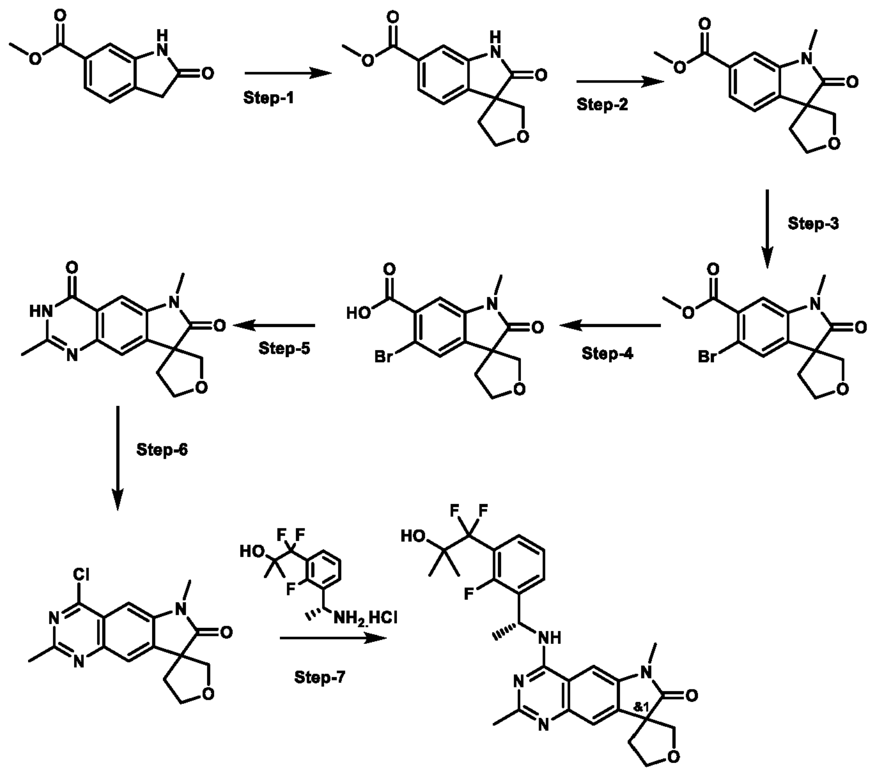

Step-1: Methyl 2'-oxo-4,5-dihydro-2H-spiro[furan-3,3'-indoline]-6'-carboxylate

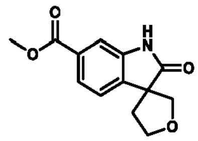

To a solution of sodium hydride (2.301 g, 57.5 mmol), a mixture of 1-chloro-2-(chloromethoxy)ethane (4.72 g, 36.6 mmol) and methyl 2-oxoindoline-6-carboxylate (5.0 g, 26.2 mmol) in DMF (50.0 mL) at -10 °C. The resulting mixture was slowly warmed to room temperature over 1h and reaction was quenched with sat. $NH_4Cl$ solution. Resulting reaction mass was extracted with ethyl acetate (2 × 50.0 mL). The combined organic layers were washed with brine (30.0 mL), dried over anhydrous $Na_2SO_4$ and concentrated in vacuum. The crude residue was purified by flash chromatography ethyl acetate-hexane to afford the titled compound (5.0 gm, 77% yield)

MS (ES+) m/z = 248.33 (M+1)

Step-2: Methyl 1'-methyl-2'-oxo-4,5-dihydro-2H-spiro[furan-3,3'-indoline]-6'-carboxylate

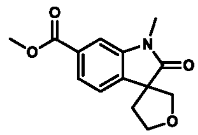

Titled compound was prepared from Step-1 intermediate by employing analogous protocol mentioned in example 81- step-2.

MS (ES+) m/z = 262.21 (M+1)

Step-3: Methyl 5'-bromo-1'-methyl-2'-oxo-4,5-dihydro-2H-spiro[furan-3,3'-indoline]-6'-carboxylate

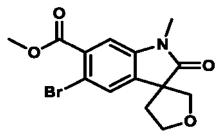

Titled compound was prepared from Step-2 intermediate by employing analogous protocol mentioned in example 81- step 2a.

MS (ES+) m/z = 340.34 (M+), 342.34 (M+2)

Step-4: 5'-Bromo-1'-methyl-2'-oxo-4,5-dihydro-2H-spiro[furan-3,3'-indoline]-6'-carboxylic acid

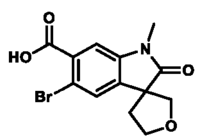

Titled compound was prepared from Step-3 intermediate by employing analogous protocol mentioned in example 81- step 4a.

MS (ES+) m/z = 326.28 (M+), 328.34 (M+2)

Step-5: 2',6'-Dimethyl-3',4,5,6'-tetrahydro-2H-spiro[furan-3,8'-pyrrolo[2,3-g]quinazoline]-4',7'-dione

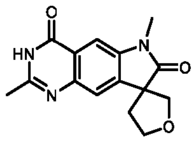

Titled compound was prepared from Step-4 intermediate by employing analogous protocol mentioned in example 81- step 5a.

MS (ES+) m/z = 286.46 (M+1)

Step-6: 4'-Chloro-2', 6'-dimethyl-4,5-dihydro-2H-spiro[furan-3,8'-pyrrolo[2,3-g]quinazolin]-7'(6'H)-one

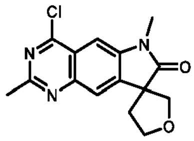

Titled compound was prepared from Step-5 intermediate by employing analogous protocol mentioned in example 71- step 9.

MS (ES+) m/z = 304.46 (M+1)

Step-7 : (R&S)-4'-(((R)-1-(3-(1,1-difluoro-2-hydroxy-2-methylpropyl)-2-fluorophenyl)ethyl)amino)-2',6'-dimethyl-4,5-dihydro-2H-spiro[furan-3,8'-pyrrolo[2,3-g]quinazolin]-7'(6'H)-one **(Compound 128)**

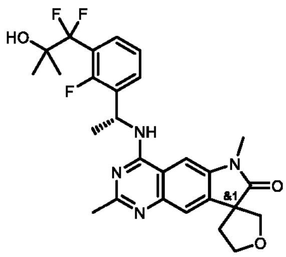

Titled compound was prepared by reaction of step-6 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in example 71- step-10

The Two stereoisomers were separated by chiral preparative HPLC to provide titled compound.

Chiral HPLC method: CHIRALPAK IC CRL-087 HEX-0.1%DEA_IPA_DCM _65_35_A_B_1.2ML_15MIN_243nm/ 1.2 ml/min

Peak-1 : (S/R)-4'-(((R)-1-(3-(1,1-difluoro-2-hydroxy-2-methylpropyl)-2-fluorophenyl)ethyl)amino)-2',6'-dimethyl-4,5-dihydro-2H-spiro[furan-3,8'-pyrrolo[2,3-g]quinazolin]-7'(6'H)-one **(Compound 128a)**

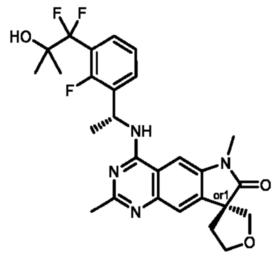

$^1$H NMR (400 MHz, DMSO-$d_6$) δ 8.28 (s, 1H), 7.91 (s, 1H), 7.63 - 7.56 (m, 1H),7.53 (s, 1H), 7.38 - 7.27 (m, 1H), 7.21 (t, $J$ = 7.7 Hz, 1H), 5.92 - 5.76 (m, 1H), 5.35 (s, 1H), 4.24 - 4.14 (m, 1H), 4.14 - 4.04 (m, 1H), 3.90 (q, $J$ = 8.6 Hz, 2H), 3.30 (s, 3H), 2.44 - 2.35 (m, 1H), 2.32 (s, 3H), 2.21 - 2.07 (m, 1H), 1.61 (d, $J$ = 7.0 Hz, 3H), 1.24 (s, 3H), 1.22 (s, 3H).

MS (ES+) m/z = 514.93 (M+1)
Chiral HPLC Rt: 6.28 min

Peak-2: (R/S)-4'-(((R)-1-(3-(1,1-difluoro-2-hydroxy-2-methylpropyl)-2-fluorophenyl)ethyl)amino)-2',6'-dimethyl-4,5-dihydro-2H-spiro[furan-3,8'-pyrrolo[2,3-g]quinazolin]-7'(6'H)-one **(Compound 128b)**

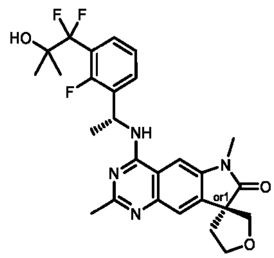

$^1$H NMR (400 MHz, DMSO-$d_6$) δ 8.24 (d, $J$ = 7.4 Hz, 1H), 7.91 (s, 1H), 7.64 - 7.55 (m, 1H), 7.52 (s, 1H), 7.35 - 7.26 (m, 1H), 7.21 (t, $J$ = 7.7 Hz, 1H), 5.87 - 5.75 (m, 1H), 5.34 (s, 1H), 4.25 - 4.15 (m, 1H), 4.15 - 4.07 (m, 1H), 3.94 - 3.81 (m, 2H), 3.30 (s, 3H), 2.44 - 2.34 (m, 1H), 2.32 (s, 3H), 2.22 - 2.14 (m, 1H), 1.61 (d, $J$ = 7.0 Hz, 3H), 1.24 (s, 3H), 1.22 (s, 3H).

MS (ES+) m/z = 514.93 (M+1)
Chiral HPLC Rt: 7.07 min

**Example 142 a:** 4-(((R)-1-(3-((R/S)-3-(dimethylamino)-1,1-difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one **(Compound 129a)**

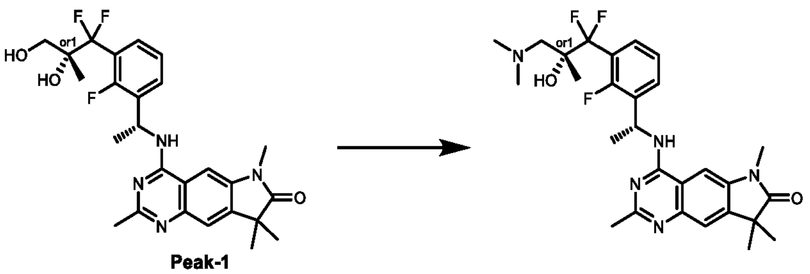

To a stirred solution of 4-(((R)-1-(3-((S/R)-1,1-difluoro-2,3-dihydroxy-2-methylpropyl)-2-fluorophenyl)ethyl)amino)-2,6,8,8-tetramethyl-6,8-dihydro-7H-pyrrolo[2,3-g]quinazolin-7-one (Compound 76a, 0.100 g, 0.199 mmol) in pyridine (3.0 mL) at 0°C, Mesyl chloride (0.019 ml, 0.239 mmol) was added dropwise at 0°C & reaction was stirred at 0°C for 1h. Reaction was concentrated to dryness in vacuum to give 0.21 gm crude Mesyl intermediate. This intermediate was dissolved in 2-propanol (3.0 mL) and dimethylamine (8.97 mg, 0.199 mmol) was added. The reaction was stirred at 100°C for 16 h. Reaction mixture was diluted with cold water (5.0 mL) and extracted with DCM (3 x 10.0 mL). Organic layer was

separated, dried over anhydrous $Na_2SO_4$ and concentrated in vacuum. The crude residue was purified using preparative HPLC to afford titled compound. (0.041 gm, 38.9 % yield).

$^1H$ NMR (400 MHz, DMSO-$d_6$) δ 8.22 (d, J = 7.4 Hz, 1H), 7.90 (s, 1H), 7.66 - 7.55 (m, 2H), 7.33 - 7.28 (m, 1H), 7.25 - 7.16 (m, 1H), 5.84 - 5.78 (m, 1H), 5.30 (s, 1H), 3.28 (s, 3H), 2.32 (s, 3H), 2.32 - 2.26 (m, 2H), 2.21 (s, 6H), 1.61 (d, J = 7.0 Hz, 3H), 1.35 (s, 3H), 1.33 (s, 3H), 1.25 (s, 3H)

MS (ES+) m/z = 530.49 (M+1)

**Example 142b:** 4-(((R)-1-(3-((S/R)-3-(dimethylamino)-1,1-difluoro-2-hydroxy-2-methylpropyl)-2-fluorophenyl)ethyl) amino)-2,6,8,8-tetramethyl-6,8-dihydro-7H-pyrrolo[2,3-g]quinazolin-7-one **(Compound 129b)**

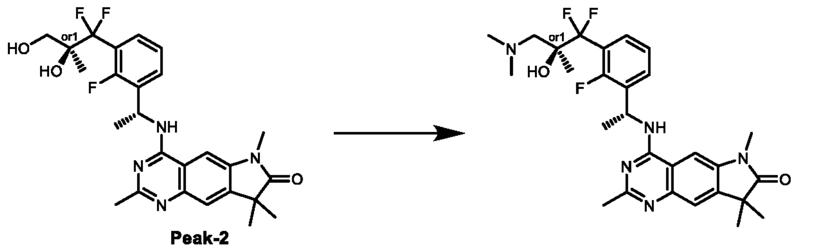

Peak-2

Titled compound was prepared from compound 76b by using analogous protocol as mentioned in example 142. $^1H$ NMR (400 MHz, DMSO-$d_6$) δ 8.21 (d, J = 7.4 Hz, 1H), 7.90 (s, 1H), 7.61 (s, 1H), 7.60 - 7.55 (m, 1H), 7.34 - 7.28 (m, 1H), 7.21 (t, J = 7.7 Hz, 1H), 5.86 - 5.78 (m, 1H), 3.28 (s, 3H), 2.34 (s, 3H), 2.29 - 2.22 (m, 2H), 2.21 (s, 6H), 1.60 (d, J = 7.0 Hz, 3H), 1.35 (s, 3H), 1.34 (s, 3H), 1.27 (s, 3H).

MS (ES+) m/z = 530.42 (M+1)

**Example 143a:** 4-(((R)-1-(3-((R/S)-1,1-difluoro-2-hydroxy-2-methyl-3-(methylamino)propyl)-2-fluorophenyl)ethyl)amino)-2,6,8,8-tetramethyl-6,8-dihydro-7H-pyrrolo[2,3-g]quinazolin-7-one **(Compound 130a)**

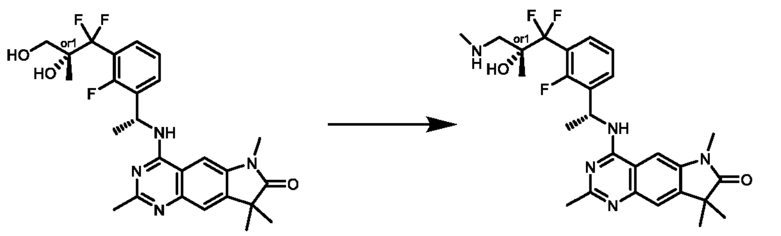

Titled compound was prepared from compound 76a by using analogous protocol as mentioned in example 142. $^1H$ NMR (400 MHz, DMSO-$d_6$) δ 8.22 (d, J = 7.4 Hz, 1H), 7.90 (s, 1H), 7.69 - 7.56 (m, 2H), 7.34 - 7.28 (m, 1H), 7.27 - 7.18 (m, 1H), 5.85 - 5.77 (m, 1H), 5.31 (s, 1H), 3.28 (s, 3H), 2.69- 2.62 (m, 1H), 2.55 - 2.53 (m, 1H), 2.32 (s, 3H), 2.29 (s, 3H), 1.61 (d, J = 7.0 Hz, 3H), 1.35 (s, 3H), 1.33 (s, 3H), 1.24 (s, 3H) MS (ES+) m/z = 516.18 (M+1

**Example 143b** : 4-(((R)-1-(3-((S/R)-1,1-difluoro-2-hydroxy-2-methyl-3-(methylamino)propyl)-2-fluorophenyl)ethyl)amino)-2,6,8,8-tetramethyl-6,8-dihydro-7H-pyrrolo[2,3-g]quinazolin-7-one **(Compound 130b)**

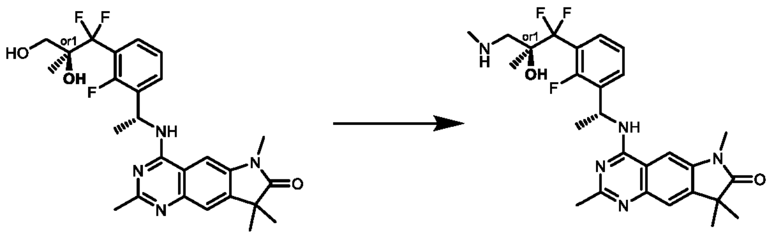

Titled compound was prepared from compound 76b by using analogous protocol as mentioned in example 142.

$^1$H NMR (400 MHz, DMSO-$d_6$) δ 8.21 (d, J = 7.5 Hz, 1H), 7.90 (s, 1H), 7.66 - 7.55 (m, 2H), 7.39 - 7.28 (m, 1H), 7.25 - 7.16 (m, 1H), 5.92 - 5.79 (m, 1H), 5.33 (s, 1H), 3.28 (s, 3H), 2.63 - 2.55 (m, 1H), 2.54 - 2.52 (m, 1H), 2.33 (s, 3H), 2.29 (s, 3H), 1.60 (d, J = 7.0 Hz, 3H), 1.35 (s, 3H), 1.34 (s, 3H), 1.26 (s, 3H). MS (ES+) m/z = 516.18 (M+1)

**Example 144:** (S)-4-(((R)-1-(3-amino-5-(trifluoromethyl) phenyl) ethyl)amino)-8-methoxy-2,6,8-trimethyl-6,8-dihydro-7H-pyrrolo[2,3-g]quinazolin-7-one **(Compound 131)**

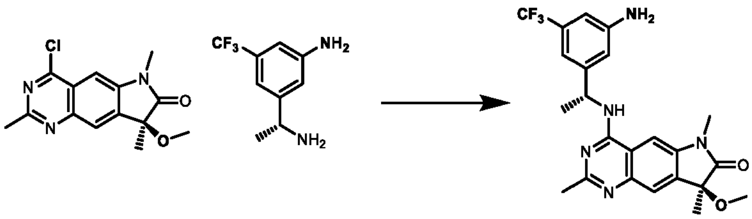

Titled compound was prepared from example 115-step-2f intermediate and (R)-3-(1-Aminoethyl)-5-(trifluoromethyl)aniline (example 1-step-4) by using analogous protocol as mentioned in example 71-step-10. $^1$H NMR (400 MHz, DMSO-$d_6$) δ 8.19 (d, *J* = 7.9 Hz, 1H), 7.91 (s, 1H), 7.56 (s, 1H), 6.91 (s, 1H), 6.89 - 6.85 (m, 1H), 6.84 - 6.62 (m, 1H), 5.72 - 5.47 (m, 3H), 3.27 (s, 3H), 2.90 (s, 3H), 2.40 (s, 3H), 1.57 (d, *J* = 7.1 Hz, 3H), 1.49 (s, 3H).

MS (ES+) m/z = 460.43 (M+1)

**Example 145:** (R&S)- 4-(((R)-1-(3-(1,1-difluoro-2-hydroxy-2-methylpropyl)-2-fluorophenyl)ethyl)amino)-8-methoxy-8-(2-methoxyethyl)-2,6-dimethyl-6,8-dihydro-7H-pyrrolo[2,3-g]quinazolin-7-one **(Compound 132)**

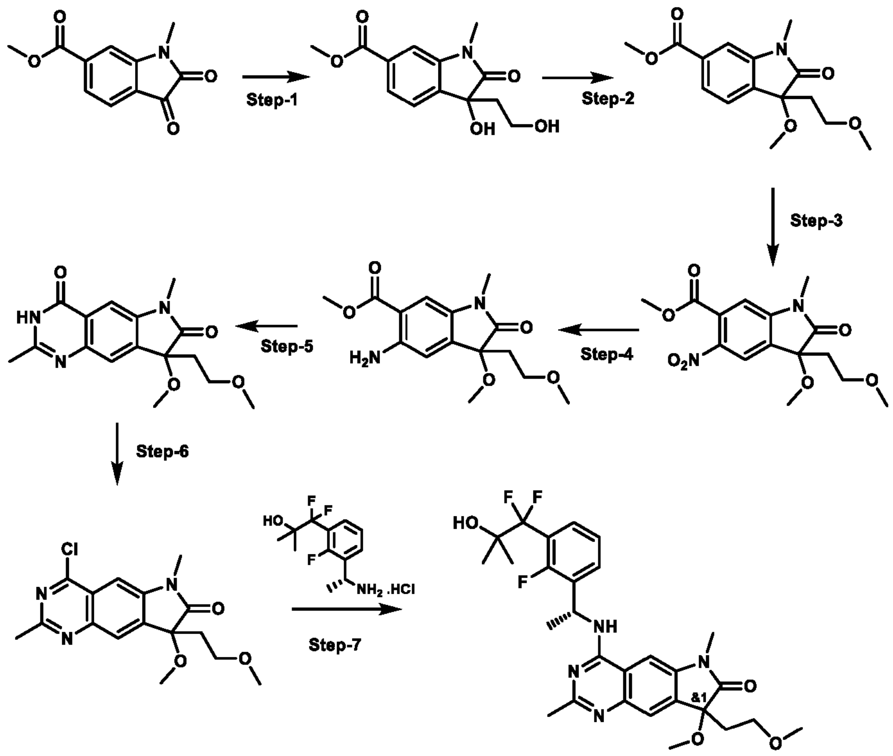


Step-1: Methyl 3-hydroxy-3-(2-hydroxyethyl)-1-methyl-2-oxoindoline-6-carboxylate

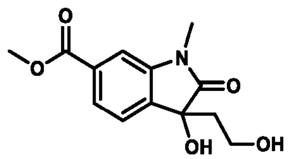

To a solution of methyl 1-methyl-2,3-dioxoindoline-6-carboxylate (3.0 g, 13.69 mmol) in acetaldehyde (60.3 g, 1369 mmol) , diethylamine (0.715 ml, 6.84 mmol) was added at room temperature and the reaction was stirred for 10 min. excess of acetaldehyde was removed by rotary evaporation under reduced pressure and residue was extracted with DCM (2 x 20.0 mL). Organic layer was separated, dried over anhydrous $Na_2SO_4$ and concentrated in vacuum to yield crude intermediate aldehyde. This intermediate was dissolved in methanol (50.0 mL) and $NaBH_4$ (0.570 g, 15.06 mmol) was added at 0 °C. The reaction was stirred at same temperature for 10 min and quenched with water. Aqueous layer was extracted with ethyl acetate. Organic layer was separated, dried over anhydrous $Na_2SO_4$ and evaporated. Crude product was purified by flash chromatography to afford titled compound (2.3 g, 63.4 % yield).

MS (ES+) m/z = 266.33 (M+1)

Step-2: Methyl 3-methoxy-3-(2-methoxyethyl)-1-methyl-2-oxoindoline-6-carboxylate

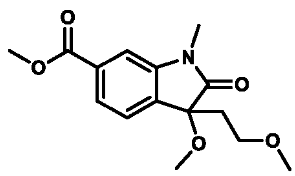

Titled compound was prepared from step-1 intermediate by employing analogous protocol mentioned in example 115-step- 2

MS (ES+) m/z = 294.40 (M+1)

Step-3: Methyl 3-methoxy-3-(2-methoxyethyl)-1-methyl-5-nitro-2-oxoindoline-6-carboxylate

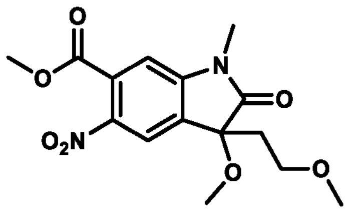

Titled compound was prepared from step-2 intermediate by employing analogous protocol mentioned in example 81-step- 6a

MS (ES+) m/z = 309.53 (M+1)

Step-4: Methyl 5-amino-3-methoxy-3-(2-methoxyethyl)-1-methyl-2-oxoindoline-6-carboxylate

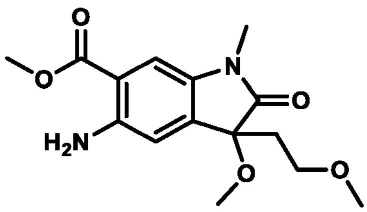

Titled compound was prepared from step-3 intermediate by employing analogous protocol mentioned in example 81-step- 7a.

MS (ES+) m/z = 309.53 (M+1)

Step-5: 8-methoxy-8-(2-methoxyethyl)-2,6-dimethyl-6,8-dihydro-3H-pyrrolo[2,3-g]quinazoline-4,7-dione

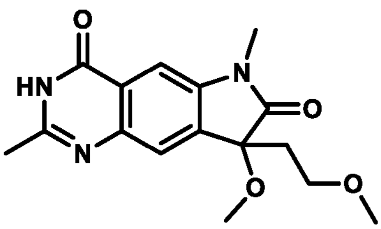

Titled compound was prepared from step-4 intermediate by employing analogous protocol mentioned in example 71-step-8. It was used as such for next step.

MS (ES+) m/z = 318.46 (M+1)

Step-6: 4-chloro-8-methoxy-8-(2-methoxyethyl)-2,6-dimethyl-6,8-dihydro-7H-pyrrolo[2,3-g]quinazolin-7-one

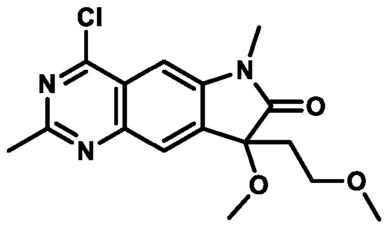

Titled compound was prepared from step-5 intermediate by employing analogous protocol mentioned in example 71-step-9.

MS (ES+) m/z = 336.47 (M+1)

Step-7 : (R &S)- 4-(((R)-1-(3-(1,1-difluoro-2-hydroxy-2-methylpropyl)-2-fluorophenyl)ethyl)amino)-8-methoxy-8-(2-methoxyethyl)-2,6-dimethyl-6,8-dihydro-7H-pyrrolo[2,3-g]quinazolin-7-one

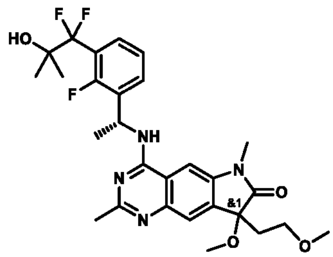

Titled compound was prepared by reaction of step-6 intermediate and (R)-1-(3-(1-Aminoethyl)-2-fluorophenyl)-1,1-difluoro-2-methylpropan-2-ol hydrochloride (Example 4-Step 6a) by employing analogous protocol mentioned in example 71-step-10.

The two stereoisomers are separated by chiral preparative HPLC.

Chiral method HEX0.1%DEA_IPA 60_40_A_D_1.2ML_12MIN_290NM CHIRALPAK IC-087

Peak 1 : (R/S)-4-(((R)-1-(3-(1,1-difluoro-2-hydroxy-2-methylpropyl)-2-fluorophenyl)ethyl)amino)-8-methoxy-8-(2-methoxyethyl)-2,6-dimethyl-6,8-dihydro-7H-pyrrolo[2,3-g]quinazolin-7-one

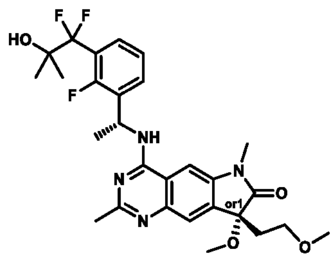

$^{1}$H NMR (400 MHz, DMSO-$d_6$) δ 8.31 (d, $J$ = 7.3 Hz, 1H), 7.93 (s, 1H), 7.68 - 7.59 (m, 1H), 7.56 (s, 1H), 7.40 - 7.28 (m, 1H), 7.28 - 7.14 (m, 1H), 5.88 - 5.74 (m, 1H), 5.35 (s, 1H), 3.28 (s, 3H), 3.26 - 3.18 (m, 1H), 3.16 - 3.05 (m, 1H), 3.00 (s, 3H), 2.89 (s, 3H), 2.34 (s, 3H), 2.29 - 2.12 (m, 2H), 1.61 (d, $J$ = 7.0 Hz, 3H), 1.24 (s, 3H), 1.22 (s, 3H).

MS (ES+) m/z = 546.93 (M+)
Chiral HPLC Rt: 4.21 min

Peak 2: (S/R)-4-(((R)-1-(3-(1,1-difluoro-2-hydroxy-2-methylpropyl)-2-fluorophenyl)ethyl)amino)-8-methoxy-8-(2-methoxyethyl)-2,6-dimethyl-6,8-dihydro-7H-pyrrolo[2,3-g]quinazolin-7-one

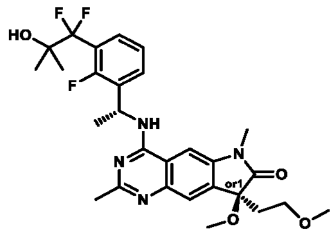

$^1H$ NMR (400 MHz, DMSO-$d_6$) δ 8.31 (d, $J$ = 7.3 Hz, 1H), 7.93 (s, 1H), 7.67 - 7.58 (m, 1H), 7.56 (s, 1H), 7.36 - 7.27 (m, 1H), 7.27 - 7.18 (m, 1H), 5.88 - 5.78 (m, 1H), 5.35 (s, 1H), 3.28 (s, 3H), 3.26 - 3.21 (m, 1H), 3.14 - 3.03 (m, 1H), 2.99 (s, 3H), 2.88 (s, 3H), 2.34 (s, 3H), 2.29 - 2.08 (m, 2H), 1.61 (d, $J$ = 7.0 Hz, 3H), 1.25 (s, 3H), 1.22 (s, 3H).

MS (ES+) m/z = 546.93 (M+)
Chiral HPLC Rt: 5.64 min

Example 146: (R & S)-4-(((R)-1-(2-fluoro-3-((R & S)-piperidin-3-yl)phenyl)ethyl)amino)-2,6,8,8-tetramethyl-6,8-dihydro-7H-pyrrolo[2,3-g]quinazolin-7-one (Compound 133)

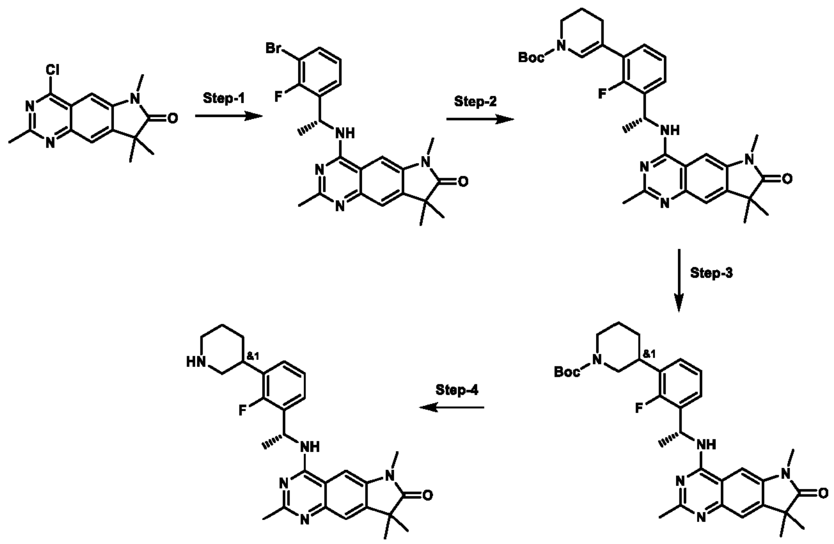


Step-1: (R)-4-((1-(3-bromo-2-fluorophenyl)ethyl)amino)-2,6,8,8-tetramethyl-6,8-dihydro-7H-pyrrolo[2,3-g]quinazolin-7-one

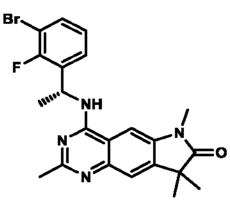

The titled compound was prepared from Example 81-Step 6 intermediate and commercially available (R)-1-(3-bromo-2-fluorophenyl)ethan-1-amine by employing similar protocol mentioned in Example71- Step 10 MS (ES+) m/z = 457.29 (M+)

Step-2: tert-butyl (R)-5-(2-fluoro-3-(1-((2,6,8,8-tetramethyl-7-oxo-7,8-dihydro-6H-pyrrolo[2,3-g]quinazolin-4-yl)amino)ethyl)phenyl)-3,4-dihydropyridine-1(2H)-carboxylate

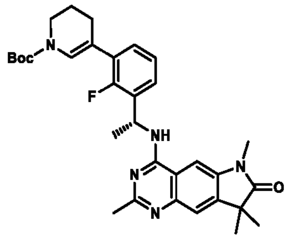

To a stirred solution of (R)-4-((1-(3-bromo-2-fluorophenyl)ethyl)amino)-2,6,8,8-tetramethyl-6,8-dihydro-7H-pyrrolo[2,3-g]quinazolin-7-one (step-1, 350 mg, 0.765 mmol), 1,4-Dioxane(10. 0 mL) and water (2.0 mL), tert-butyl 5-(4,4,5,5-tetramethyl-1,3,2-dioxaborolan-2-yl)-3,4-dihydropyridine-1(2H)-carboxylate (355 mg, 1.148 mmol), potassium carbonate (317 mg, 2.296 mmol) and $PdCl_2$(dppf).DCM (62.5 mg, 0.077 mmol) were added in inert atmosphere and reaction was stirred at 120 °C for 16 h in a sealed vial. Reaction was diluted with ethyl acetate (30.0 ml) and water (20.0 ml). The organic layer was separated, washed with brine, dried over anhydrous $Na_2SO_4$ and evaporated. The residue was purified by flash chromatography using ethyl acetate-hexane gradient to afford titled compound.

MS (ES+) m/z = 560.43 (M+1).

Step-3: (R & S) - tert-butyl 3-(2-fluoro-3-((R)-1-((2,6,8,8-tetramethyl-7-oxo-7,8-dihydro-6H-pyrrolo[2,3-g]quinazolin-4-yl)amino)ethyl)phenyl)piperidine-1-carboxylate

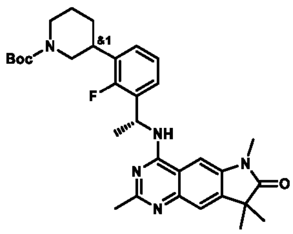

A mixture of tert-butyl (R)-5-(2-fluoro-3-(1-((2,6,8,8-tetramethyl-7-oxo-7,8-dihydro-6H-pyrrolo[2,3-g]quinazolin-4-yl)amino)ethyl)phenyl)-3,4-dihydropyridine-1(2H)-carboxylate (step-2, 0.3 g, 0.536 mmol), ammonium formate (0.237 g, 3.75 mmol) and $Pd(OH)_2$ on Carbon (0.075 g, 0.536 mmol) in EtOH (10.0 ml) and Water (2.0 ml) was stirred at 80 °C for 4 h. Reaction mixture was cooled to room temperature and filtered through celite bed. Filtrate was concentrated and obtained residue was partitioned between ethyl acetate (30.0 ml) and water (20.0 ml). Organic layer was separated, anhydrous $Na_2SO_4$ and evaporated. The residue was purified by flash chromatography using MeOH-DCM gradient and further t purified by preparative HPLC to afford titled compound (280 mg, 93 % yield)

MS (ES+) m/z = 562.58 (M+1).

Step-4: (R & S)- 4-(((1R)-1-(2-fluoro-3-(piperidin-3-yl)phenyl)ethyl)amino)-2,6,8,8-tetramethyl-6,8-dihydro-7H-pyrrolo[2,3-g]quinazolin-7-one **(compound 133)**

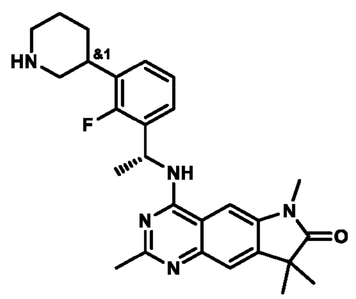

To a stirred solution of tert-butyl 3-(2-fluoro-3-((R)-1-((2,6,8,8-tetramethyl-7-oxo-7,8-dihydro-6H-pyrrolo[2,3-g] quinazolin-4-yl)amino)ethyl)phenyl)piperidine-1-carboxylate (step-3, 0.1 gm, 0.178 mmol) in DCM (10.0 ml) was added 4M HCl in Dioxane (0.890 ml, 3.56 mmol) at room temperature and stirred for 16 h. Reaction mixture was concentrated and dried using azeotropic co-evaporation with toluene and further purified by preparative HPLC in basic medium to afford titled compound.

$^1$H NMR (400 MHz, DMSO-$d_6$) δ 8.93 (s, 1H), 8.39 (s, 1H), 7.74 - 7.68 (m, 1H), 7.61 - 7.52 (m, 1H), 7.37 - 7.28 (m, 1H), 7.28 - 7.18 (m, 1H), 6.08 - 5.94 (m, 1H), 3.31 (s, 3H), 3.16 - 2.99 (m, 1H), 2.99 - 2.83 (m, 1H), 2.62 - 2.54 (m, 5H), 1.97 - 1.74 (m, 5H), 1.70 (d, J = 7.0 Hz, 3H), 1.39 (s, 3H), 1.38 (s, 3H).

MS (ES+) m/z = 462.48 (M+1).

Example 147: (R/S)-4-((1-(2-fluoro-3-(trifluoromethyl)phenyl)ethyl)amino)-2,6,8,8-tetramethyl-6,8-dihydro-7H-pyrrolo [2,3-g]quinazolin-7-one **(Compound 134)**

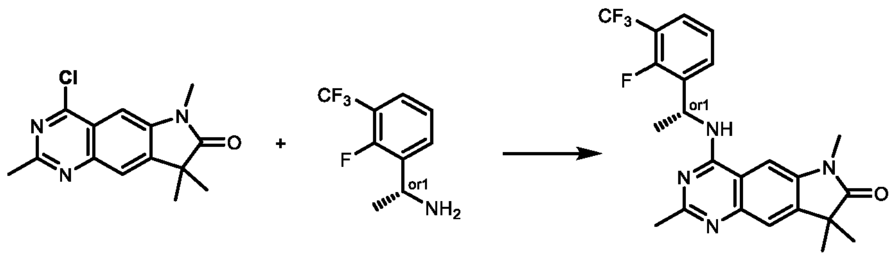

Titled compound was prepared from 4-Chloro-2,6,8,8-tetramethyl-6,8-dihydro-7H-pyrrolo[2,3-g]quinazolin-7-one (example 81- step-6 intermediate) and (R/S)-1-(2-fluoro-3-(trifluoromethyl)phenyl)ethan-1-amine ( example 2-step-3) by employing analogous protocol mentioned in example 71-step-10.

$^1$H NMR (400 MHz, DMSO-$d_6$) d 8.28 (d, J = 7.0 Hz, 1H), 7.87 (s, 1H), 7.83 - 7.78 (m, 1H), 7.68 - 7.62 (m, 1H), 7.61 (s, 1H), 7.39 - 7.32 (m, 1H), 5.82 - 5.70 (m, 1H), 3.28 (s, 3H), 2.31 (s, 3H), 1.65 (d, J = 7.1 Hz, 3H), 1.35 (s, 3H), 1.33 (s, 3H).

MS (ES+) m/z = 447.36 (M+1).

Example 148: (8R/S)-4-(((1R)-1-(3-(3-(dimethylamino)-1,1-difluoro-2-hydroxy-2-methylpropyl)-2-fluorophenyl)ethyl) amino)-8-methoxy-2,6,8-trimethyl-6,8-dihydro-7H-pyrrolo[2,3-g]quinazolin-7-one

**(Compound 135)**

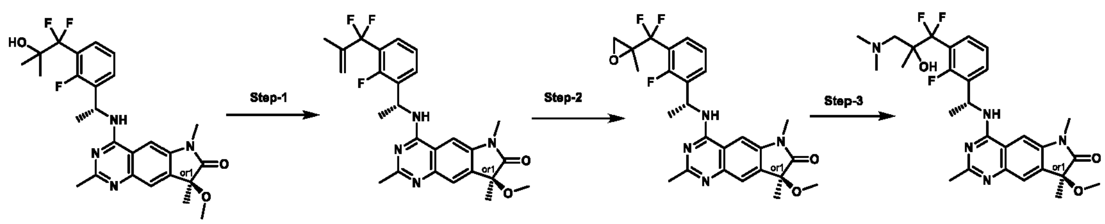

Step - 1: (R/S)-4-(((R)-1-(3-(1,1-difluoro-2-methylallyl)-2-fluorophenyl)ethyl)amino)-8-methoxy-2,6,8-trimethyl-6,8-dihydro-7H-pyrrolo[2,3-g]quinazolin-7-one

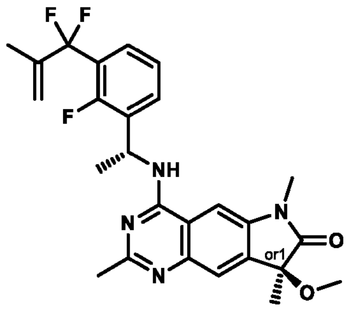

Titled compound was prepared from compound 101b by employing analogous protocol mentioned in example 89- step-1.
MS (ES+) m/z = 485.30 (M+1)

Step - 2: (8R/S)-4-(((1R)-1-(3-(difluoro(2-methyloxiran-2-yl)methyl)-2-fluorophenyl)ethyl)amino)-8-methoxy-2,6,8-trimethyl-6,8-dihydro-7H-pyrrolo[2,3-g]quinazolin-7-one

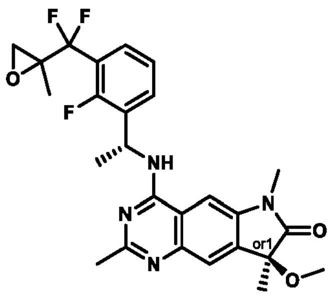

Titled compound was prepared from step-1 intermediate by employing analogous protocol mentioned in example 140-step-1
MS (ES+) m/z = 501.44 (M+1)

Step - 3: (8R/S)-4-(((1R)-1-(3-(3-(dimethylamino)-1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one **(Compound 135)**

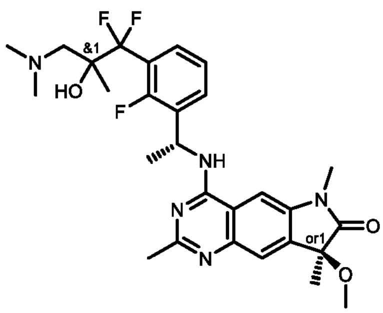

To a stirred solution of the (8R&S)-4-(((1R)-1-(3-(difluoro(2-methyloxiran-2-yl)methyl)-2-fluorophenyl)ethyl) amino)-8-methoxy-2,6,8-trimethyl-6,8-dihydro-7H-pyrrolo[2,3-g]quinazolin-7-one (0.8 g, 1.598 mmol) in 2-propanol (3.5 mL) and Water (0.5 mL) were added the dimethylamine (1.802 g, 15.98 mmol) and TEA (0.446 ml, 3.20 mmol) at

room temperature. The reaction mixture was stirred at 80 °C for 16 h. The solvent was removed under vacuo and crude (0.360 g) was purified by flash column chromatography by using 0 to 10% MeOH in DCM to afford titled compound (0.2 g, 22.93 % yield)

MS (ES+) m/z = 546.42 (M+1)

$^1$H NMR (400 MHz, *DMSO-$d_6$*) δ 8.44 - 8.31 (m, 1H), 8.00 (s, 1H), 7.71 - 7.60 (m, 1H), 7.59 - 7.54 (m, 1H), 7.38 - 7.28 (m, 1H), 7.26 - 7.18 (m, 1H), 5.89 - 5.77 (m, 1H), 3.35 - 3.31 (m, 2H), 3.30 (s, 3H), 3.15 - 2.98 (m, 2H), 2.90 - 2.87 (m, 3H), 2.42 - 2.18 (m, 3H), 1.66 - 1.58 (m, 3H), 1.49 (s, 3H), 1.32 - 1.22 (m, 4H), 1.22 - 1.14 (m, 3H), 1.12 - 1.06 (m, 1H). **(NMR of Diastereomeric mixture)**

The two stereoisomers are separated by chiral preparative HPLC.

Chiral method HEX_0.1%DEA_IPA_MEOH_90_10 _1.5ML_20Min_225NM CHIRALPAK OX-H CRL-061

Peak - 1 : (S/R)-4-(((R)-1-(3-((S/R)-3-(dimethylamino)-1,1-difluoro-2-hydroxy-2-methylpropyl)-2-fluorophenyl)ethyl) amino)-8-methoxy-2,6,8-trimethyl-6,8-dihydro-7H-pyrrolo[2,3-g]quinazolin-7-one **(Compound 135a)**

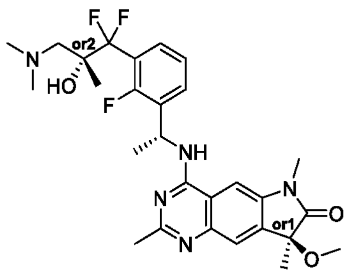

1H NMR (400 MHz, DMSO-d6) δ 8.31 (d, J = 7.3 Hz, 1H), 7.95 (s, 1H), 7.65 - 7.58 (m, 1H), 7.56 (s, 1H), 7.38 - 7.26 (m, 1H), 7.26 - 7.18 (m, 1H), 5.89 - 5.67 (m, 1H), 5.25 (bs, 1H), 3.30 (s, 3H), 2.89 (s, 3H), 2.34 (s, 3H), 1.96 - 1.85 (m, 1H), 1.84 - 1.74 (m, 1H), 2.21 (s, 6H), 1.61 (d, J = 7.1 Hz, 3H), 1.49 (s, 3H), 1.24 (s, 3H).

MS (ES+) m/z = 546.42 (M+1), Chiral HPLC RT: 9.14 min

Peak - 2: (S/R)-4-(((R)-1-(3-((R/S)-3-(dimethylamino)-1,1-difluoro-2-hydroxy-2-methylpropyl)-2-fluorophenyl)ethyl) amino)-8-methoxy-2,6,8-trimethyl-6,8-dihydro-7H-pyrrolo[2,3-g]quinazolin-7-one **(Compound 135b)**

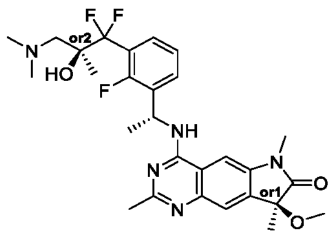

$^1$H NMR (400 MHz, DMSO-$d_6$) δ 8.31 (d, *J* = 7.4 Hz, 1H), 7.96 (s, 1H), 7.67 - 7.58 (m, 1H), 7.56 (s, 1H), 7.38 - 7.27 (m, 1H), 7.26 - 7.17 (m, 1H), 5.88 - 5.77 (m, 1H), 5.29 (bs, 1H), 3.30 (s, 3H), 2.89 (s, 3H), 2.36 (s, 3H), 2.21 (s, 6H), 1.98 - 1.85 (m, 1H), 1.85 - 1.71 (m, 1H), 1.61 (d, *J* = 7.1 Hz, 3H), 1.49 (s, 3H), 1.27 (s, 3H), MS (ES+) m/z = 546.42 (M+1), Chiral HPLC RT: 10.07 min

**Example 149:** (R/S)-2,6,8,8-tetramethyl-4-((1-(3-(trifluoromethyl)phenyl)ethyl)amino)-6,8-dihydro-7H-pyrrolo[2,3-g] quinazolin-7-one **(compound 136)**

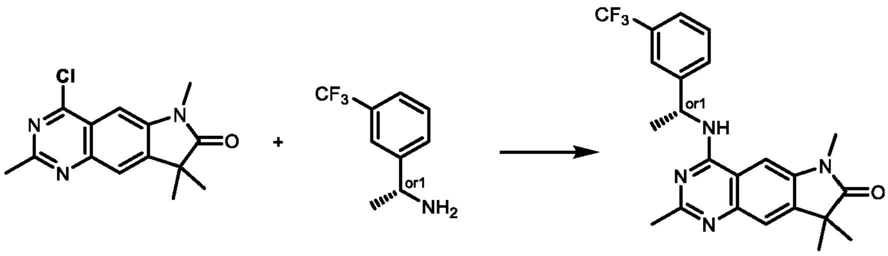

Titled compound was prepared from 4-Chloro-2,6,8,8-tetramethyl-6,8-dihydro-7H-pyrrolo[2,3-g]quinazolin-7-one (example 81- step-6 intermediate) and (R/S)-1-(3-(trifluoromethyl)phenyl)ethan-1-amine by employing analogous protocol mentioned in example 71-step-10.

$^1$H NMR (400 MHz, DMSO-$d_6$) δ 8.22 (d, J = 7.7 Hz, 1H), 7.85 - 7.81 (m, 2H), 7.80 - 7.74 (m, 1H), 7.62 - 7.55 (m, 3H), 5.73 - 5.63 (m, 1H), 3.27 (s, 3H), 2.36 (s, 3H), 1.64 (d, J = 7.1 Hz, 3H), 1.35 (s, 3H), 1.34 (s, 3H).

MS (ES+) m/z = 429.42 (M+1).

**Example 150:** 4-(((R)-1-(3-amino-5-(trifluoromethyl)phenyl)ethyl)amino)-6-methoxy-2,6,8-trimethyl-6,8-dihydro-7H-pyrrolo[3,2-g]quinazolin-7-one **(Compound 137)**

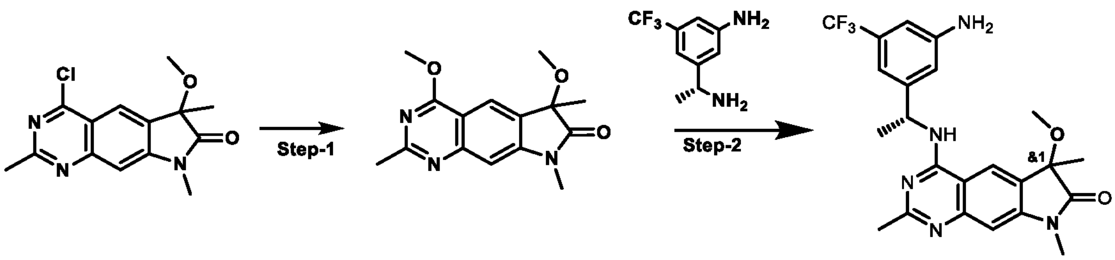


Step-1: 4,6-dimethoxy-2,6,8-trimethyl-6,8-dihydro-7H-pyrrolo[3,2-g]quinazolin-7-one

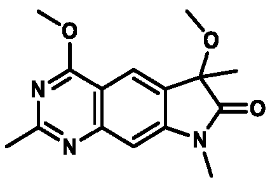

Titled compound was prepared from 4-Chloro-6-methoxy-2,6,8-trimethyl-6H-pyrrolo[3,2-g]quinazolin-7(8H)-one (Example 109-step-10) by employing analogous protocol mentioned in example 71-step-10 by employing analogous protocol mentioned in example 71-step-10

MS (ES+) m/z = 288.27 (M+1).

Step-2 : (R & S)-4-(((R)-1-(3-amino-5-(trifluoromethyl)phenyl)ethyl)amino)-6-methoxy-2,6,8-trimethyl-6,8-dihydro-7H-pyrrolo[3,2-g]quinazolin-7-one **(Compound 137)**

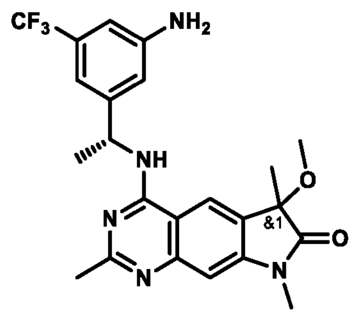

Titled compound was prepared from step-1 intermediate and (R)-3-(1-aminoethyl)-5-(trifluoromethyl) aniline (Example 1-step- 4) by employing analogous protocol mentioned in example 71-step-10 by employing analogous protocol mentioned in example 71-step-10

$^1$H NMR (400 MHz, DMSO-*d6*) δ 8.45 - 8.37 (m, 1H), 8.30 - 8.22 (m, 1H), 7.13 (s, 1H), 6.94 - 6.84 (m, 2H), 6.75 - 6.68 (m, 1H), 5.59 - 5.50 (m, 3H), 3.22 (s, 3H), 2.96 - 2.88 (m, 3H), 2.42 - 2.38 (m, 3H), 1.59 - 1.53 (m, 3H), 1.52 (s, 3H).

MS (ES+) m/z = 460.36 (M+1).

**Example 151:** (R)-4'-((1-(3-amino-5-(trifluoromethyl)phenyl)ethyl)amino)-2',6'-dimethyl-2,3,5,6-tetrahydrospiro[pyran-4,8'-pyrrolo[2,3-g]quinazolin]-7'(6'H)-one **(Compound 138)**

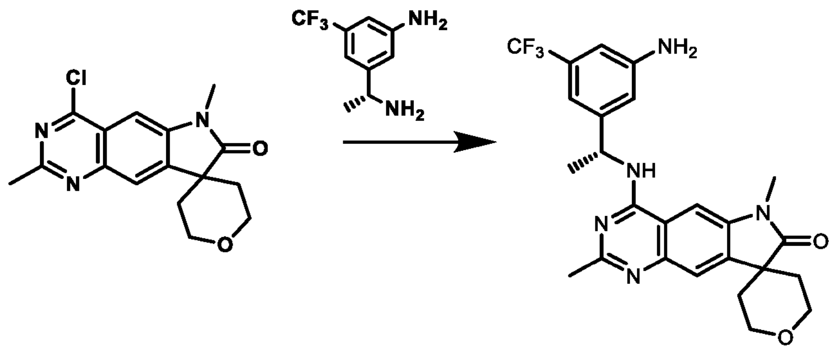

Titled compound was prepared from 4'-chloro-2',6'-dimethyl-2,3,5,6-tetrahydrospiro[pyran-4,8'-pyrrolo[2,3-g] quinazolin]-7'(6'H)-one (Example-135-step-7) and (R)-3-(1-aminoethyl)-5-(trifluoromethyl) aniline (Example 1-step- 4) by employing analogous protocol mentioned in example 71-step-10

$^1$H NMR (400 MHz, DMSO-$d_6$) δ 8.14 (d, J = 7.9 Hz, 1H), 7.87 (s, 1H), 7.73 (s, 1H), 6.92 - 6.88 (m, 1H), 6.87 - 6.84 (m, 1H), 6.74 - 6.66 (m, 1H), 5.62 - 5.51 (m, 3H), 4.17 - 3.99 (m, 2H), 3.96 - 3.77 (m, 2H), 3.26 (s, 3H), 2.39 (s, 3H), 1.90 - 1.73 (m, 4H), 1.57 (d, J = 7.0 Hz, 3H).

MS (ES+) m/z = 486.42 (M+1)

**Example 152:** (R & S)-4-(((R)-1-(3-amino-5-(trifluoromethyl)phenyl)ethyl)amino)-8-ethyl-8-methoxy-2,6-dimethyl-6,8-dihydro-7H-pyrrolo[2,3-g]quinazolin-7-one **(Compound 139)**

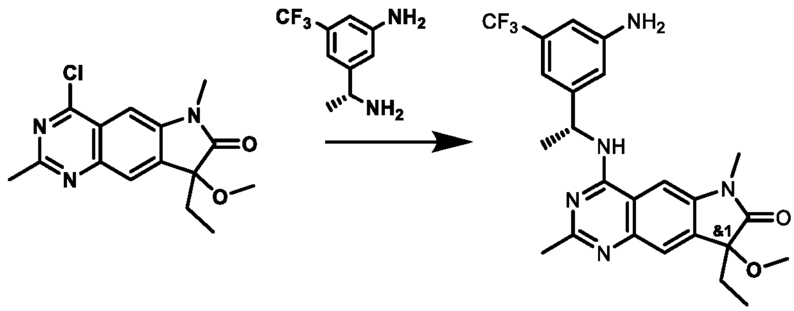

Titled compound was prepared from 4-chloro-8-ethyl-8-methoxy-2,6-dimethyl-6,8-dihydro-7H-pyrrolo[2,3-g]quinazolin-7-one (Example-139-step-6) and (R)-3-(1-aminoethyl)-5-(trifluoromethyl) aniline (Example 1-step- 4) by employing analogous protocol mentioned in example 71-step-10

$^1$H NMR (400 MHz, DMSO-*d*6) δ 8.21 (d, *J* = 7.9 Hz, 1H), 7.92 (s, 1H), 7.52 (s, 1H), 6.93 - 6.86 (m, 2H), 6.76 - 6.65 (m, 1H), 5.61 - 5.52 (m, 3H), 3.28 (s, 3H), 2.91 (s, 3H), 2.41 (s, 3H), 1.97 - 1.79 (m, 2H), 1.57 (d, *J* = 7.0 Hz, 3H), 0.69 - 0.58 (m, 3H).

MS (ES+) m/z = 474.55 (M+1).

**Example -153: KRAS-SOS1 Interaction Assay**

Interaction assay was conducted to evaluate the ability of the compounds to disrupt the protein-protein interactions between hSOS1 and hKRASG12C. Inhibitory effect of compounds on GST-hSOS1, 564-1049aa expressed and purified in-house and HIS-hKRASG12C (R06-32DH, SignalChem) interaction was assessed using HTRF detection technology in biochemical assay. Compounds were pre-incubated with 10-50 nM working solution of hSOS1 per well of a 384 well plate (Perkin Elmer, Cat #6007290) for 10 min at 20°C in the assay buffer containing 5 mM HEPES, pH 7.4, 150 mM NaCl, 10 mM EDTA, 1 mM DTT, 0.05% BSA fraction V and 0.0025% Igepal. Reaction was initiated by adding 100-300 nM working solution of hKRASG12C in assay buffer. Total assay volume was 15 μL. Reaction was continued for 30-60 min at 20$^0$C. Then detection solution containing MAb Anti-GST-d2 (# 61GSTDLA, Cisbio BioAssays) and MAb Anti-HIS-Tb (# 61HISTLA, Cisbio BioAssays), all prepared in HTRF detection buffer (#61DB10RDF, Cisbio BioAssays) was added and further incubated for 30-60 min at 20$^0$C. HTRF signal was recorded in PHERAStar microplate reader. Ratio of signal obtained at 665 nm and 620 nm was used to compute the percent inhibition of compound utilizing the following formula:

$$\%\ \text{Inhibition} = 100 - \left[ \frac{(\text{Test Ratio - Negative Control Ratio})}{(\text{Positive Control Ratio - Negative Control Ratio})} \right] \text{x } 100$$

Where.

$$\text{Positive control} = \text{hSOS1} + \text{hKRASG12C} + \text{MAb Anti-GST-d2} + \text{Mab Anti-HIS-Tb}$$

$$\text{Negative control} = \text{hKRASG12C} + \text{MAb Anti-GST-d2} + \text{MAb Anti-HIS-Tb}$$

KRAS-SOS1 Interaction inhibition $IC_{50}$ values of the compounds in accordance with embodiments of the invention are provided in **Table-7** below: Compounds with $IC_{50}$ 1 nM to 10 nM are grouped under group A, compounds with $IC_{50}$ between 11 nM and 25 nM are grouped under group B.

**Table-7**

| Group | Compound Nos. |
|---|---|
| A | 1, 2, 10, 13, 18, 23a, 23b, 25, 27a, 27b, 28, 29, 30a, 30b, 31a, 31b, 32b, 34, 35, 36a, 36b, 37, 38, 39, 42, 45, 53a, 54, 55, 58, 62, 63, 64, 66, 68, 70, 72, 73, 76a, 80, 81, 86, 88, 90, 92, 93a, 93b, 94a, 96, 98a, 99, 101, 101a, 101b, 104, 105, 106, 109, 113, 121a, 121b, 122, 123a, 127b, 128a, 128b, 129a, 130a, 132b, 135, 135a, 135b |
| B | 47, 53b, 75, 91, 95, 96a, 98, 98b, 110, 131, 138, 139 |

**Example -154: Guanine nucleotide exchange assay (GNEA)**

GNEA assay was conducted to evaluate the effects of compounds on the nucleotide exchange on hKRASG12C mediated by hSOS1. Inhibitory effect of compounds on HIS-hSOS1, 564-1049aa mediated GTP loading onto GST-hKRASG12C, 1-169aa both expressed and purified in-house was assessed using HTRF detection technology in biochemical assay. Compounds were pre-incubated with hSOS1 working solution containing 10-50 nM hSOS1 and 200 nM EDA-GTP-DY-647P1 (Custom manufactured, Jena Biosciences) per well of a 384 well plate (Perkin Elmer, Cat #6007290) for 10 min at 20°C in the assay buffer containing 10 mM HEPES, pH 7.4, 150 mM NaCl, 5 mM MgCl2, 1 mM DTT, 0.05% BSA fraction V and 0.0025% Igepal. Reaction was initiated by adding working solution of hKRASG12C containing

25-125 nM hKRASG12C and MAb Anti-GST-Tb (#61GSTTLA, Cisbio BioAssays) in assay buffer. Total assay volume was 15 μL. Reaction was continued for 30-60 min at 20$^0$C. HTRF signal was recorded in PHERAStar microplate reader. Ratio of signal obtained at 665 nm and 620 nm was used to compute the percent inhibition of compound as follows.

$$\% \text{ Inhibition} = 100 - \left[ \frac{(\text{Test Ratio - Negative Control Ratio})}{(\text{Positive Control Ratio - Negative Control Ratio})} \right] \times 100$$

Where,

$$\text{Positive control} = \text{hSOS1} + \text{EDA-GTP-DY-647P1} + \text{hKRASG12C} + \text{MAb Anti-GST-Tb}$$

$$\text{Negative control} = \text{EDA-GTP-DY-647P1} + \text{hKRASG12C} + \text{MAb Anti-GST-Tb}$$

Guanine nucleotide exchange inhibition $IC_{50}$ values of the compounds of invention are provided in **Table- 8** below: Compounds with $IC_{50}$ 1 nM to 30 nM are grouped under group A, compounds with $IC_{50}$ between 31 nM and 130 nM are grouped under group B.

**Table-8**

| Group | Compound Nos. |
|---|---|
| A | 2, 13, 18, 23a, 23b, 25, 27a, 27b, 28, 29, 30a, 30b, 31a, 31b, 32b, 35, 36a, 36b, 37, 38, 39, 42, 53a, 53b, 54, 55, 58, 62, 63, 64, 66, 68, 72, 73, 76a, 80, 81, 88, 94a, 96, 98, 98a, 99, 101, 101b, 104, 105, 106, 109, 121b, 122, 123a, 127b, 128a, 129a, 130a,132b, 135, 135a |
| B | 1, 10, 34, 45, 47, 70, 75, 86, 90, 91, 92, 93a, 93b, 95, 96a, 98b, 101a, 110, 113, 121a, 128b, 131, 135b, 138, 139 |

**Example -155: ERK phosphorylation assay protocol**

Compounds were tested for their ability to inhibit pERK after EGF stimulation in DLD1 cells. Cells were seeded at 0.2 million cells per well in 96 well cell binding tissue culture plate in 100μL complete media and were allowed to settle overnight (16 to 20 h). On the following day, cells were treated with various concentrations of test compounds for 2 h followed by stimulation with 200 ng/ml hEGF (diluted in PBS) for 5 minutes. Cells were then washed once with PBS and lysed in freshly prepared lysis buffer (provided with alphascreen surefire ERK1/2 p-T202/ Y204 assay kit, cat no: TGRESB 10K, Perkin Elmer). Lysis was carried out in plate shaker for 10-15 mins. Lysate was transferred to 386 white opti-plate (Perkin Elmer) and alphascreen for pERK was carried out as per manufacturer's instructions. Plate was read after 2 h incubation in plate shaker at h at 300 RPM, 25-28°C. The difference between the unstimulated and stimulated Vehicle control is considered as 100% ERK phosphorylation, and % inhibition of compounds is calculated accordingly.

$$\% \text{ Inhibition} = \left[ \frac{(\text{Average Stimulated - Average Unstimulated}) - (\text{Average Test - Average Unstimulated})}{(\text{Average Stimulated - Average Unstimulated}} \right] \times 100$$

ERK phosphorylation inhibition $IC_{50}$ values of the compounds of the invention are provided in Table-9 below: Compounds with $IC_{50}$ 1 nM to 200 nM are grouped under group A, compounds with $IC_{50}$ between 201 nM and 500 nM are grouped under group B.

**Table-9**

| Group | Compound Nos. |
|---|---|
| A | 1, 2, 5, 13, 14, 18, 23a, 23b, 25, 27a, 27b, 28, 29, 30a, 30b, 31a, 31b, 32a, 32b, 33, 34, 35, 36a, 36b, 37, 38, 39, 42, 43, 45, 47, 53a, 53b, 54, 55, 57, 58, 62, 63, 64, 65, 66, 68, 69, 70, 73, 75, 76a, 78, 79, 80, 81, 86, 88, 90, 91, 93a, 93b, 94a, 95, 96, 96a, 96b, 98, 98a, 98b, 99, 101, 101a, 101b, 104, 105, 106, 109, 114, 116b, 121a, 121b, 122, 123a, 123b, 125b, 126a, 127b, 128a, 128b, 129a, 129b, 130a, 130b, 131, 132a,132b, 135,135a, 135b, 137, 138 |
| B | 3, 22, 22b, 26, 40, 41, 76b, 92, 103, 108, 110, 111, 116a, 117, 125a, 126b, 127a, 133, 134, 139 |

**Example -156: Colony Formation assay protocol (Single agent activity)**

DLD-1 / MIA PaCa-2 cells were seeded at a density of 300 / 500 cells per well respectively in 48 well tissue culture plate and cells were allowed to settle overnight (16 to 20 h). On the following day, cells were treated with different concentrations of test or reference compound and the assay plates were incubated under standard cell culture conditions (37°C with 5% $CO_2$). After 7 days of drug treatment, media was removed from each well and plates were washed with PBS. Colonies were stained with crystal violet solution for 2-5 min. Plate was then washed carefully under tap water and air dried. For quantitation, 200μL destaining solution containing 10% Glacial acetic acid was added to each well and crystal violet from the colonies was allowed to solubilize for 20-30 min on plate shaker. After solubilization, absorbance of the extracted stain was recorded in BioTek Synergy Neo II plate reader at 590 nm. Absorbance values were directly proportional to colony growth.

$$\% \text{ Inhibition} = \left[\frac{(\text{Average Vehicle abs - Average Blank abs}) - (\text{Average Test abs - Average Blank abs})}{(\text{Average Vehicle abs - Average Blank abs})}\right] \text{x } 100$$

Note: Blank contains only destaining solution

**Example -157: Anticancer activity assay in 3D spheroids**

MIA PaCa-2 cells were seeded at 2000 cell per well in 96 well ultralow binding tissue culture plate in 100μL complete media and cells were allowed to settle overnight (16 to 20 h). On the following day, cells were treated with various concentrations of test or reference compound and the assay plate was incubated under standard cell culture conditions as mentioned above. On 5$^{th}$ day of treatment, plate was centrifuged at 1000 rpm for 5 min, and media was replenished with fresh treatments. Plate was incubated under standard cell culture conditions for 5 more days. After 10 days of drug treatment, 100μL media was removed from each well followed by addition of 100 μL/well of Cell Titer Glo (CTG) reagent per well. Plate was wrapped in aluminium foil and kept on a shaker (700 RPM) for 3 minutes followed by additional incubation for 10 minutes in the dark. 100μL of solution from each well was transferred to white opaque 96 well tissue culture plate and read on BioTek Synergy Neo II plate reader. RLUs (Relative Light Units) were directly proportional to the actively proliferating cell number.

$$\% \text{ Inhibition} = \left[\frac{(\text{Average Vehicle RLU - Average Blank ALU}) - (\text{Average Test RLU - Average Blank RLU})}{(\text{Average Vehicle RLU - Average Blank RLU})}\right] \text{x } 100$$

Note: Blank contains only CTG and media (no cells)

**In vivo experiments:**

In vivo efficacy of compounds 68, 101b at a dose of 30 mg/kg, b.i.d., p.o. and compound 81 at a dose of 50 mg/kg, b.i.d., p.o., was evaluated in MIA PaCa-2 human pancreatic cancer xenograft model in nude mice.

MIA PaCa-2 tumor fragments were implanted subcutaneously in the right flank region of nude mice. Tumor-bearing mice were randomized once the tumors reached an average volume of ~141-142 $mm^3$ (tumor volume range 72-242 $mm^3$). The mice were divided into the following groups (n=10/group): Vehicle control, compounds 68 and 101b at a dose of 30 mg/kg, b.i.d., p.o. The tumor growth inhibition for compounds 68 and 101b at 30 mg/kg, b.i.d., p.o., was found to be 63.82% and 65.71% respectively.

To assess the efficacy of compound 81, the tumor-bearing mice were randomized once the tumors reached an

average volume of ~181-183 mm$^3$ (tumor volume range 73-251 mm$^3$). The mice were divided into the following groups (n=8/group): Vehicle control and compound 81 (50 mg/kg, b.i.d., p.o.). The tumor growth inhibition at 50 mg/kg dose was found to be 67.68%.

## Claims

1. A compound of formula (I) and a pharmaceutically acceptable salt thereof,

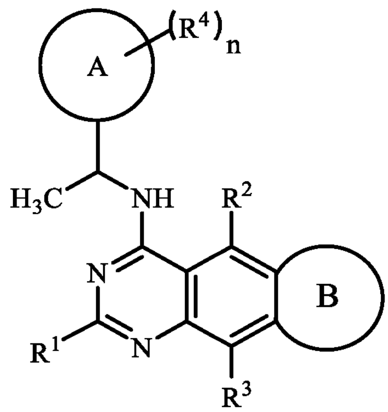

(I)

wherein,

Ring A is selected from phenyl and

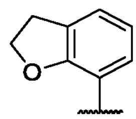
;

Ring B is selected from

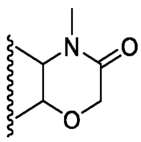
,
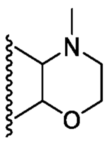
,
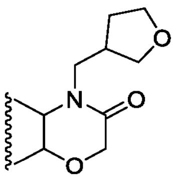
,
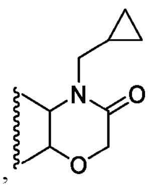
,
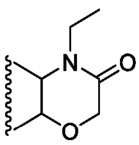
,
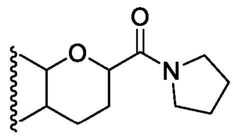
,
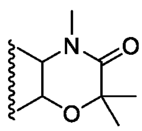
,
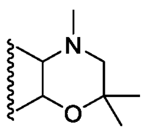
,
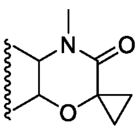
,
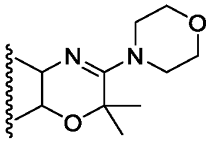
,
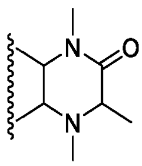
,
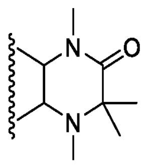
,
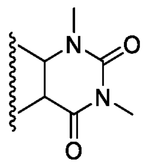
,
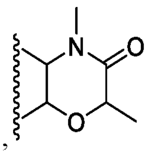
,
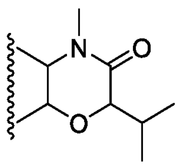
,
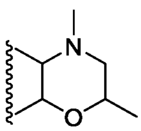
,
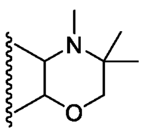
,
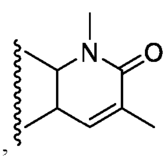
,
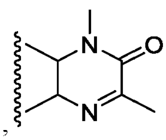
,
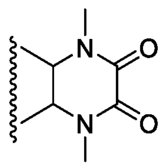
,

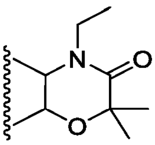, 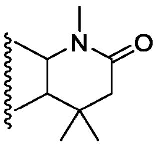, 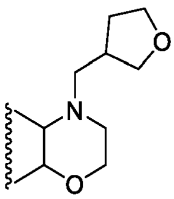, 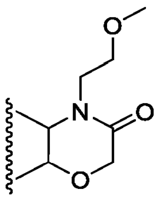, 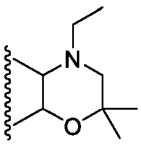, 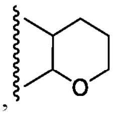, 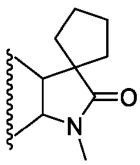,

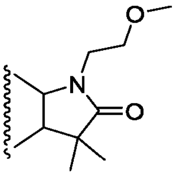, 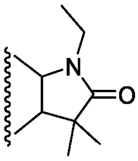, 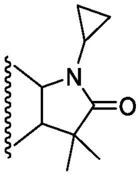, 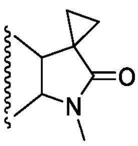, 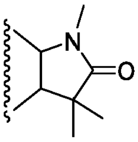, 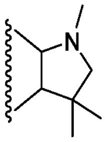, 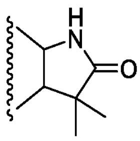,

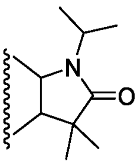, 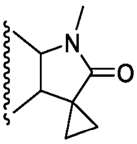, 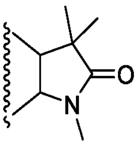, 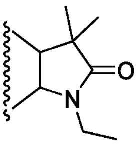, 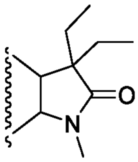, 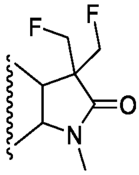, 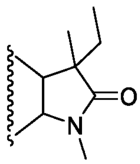,

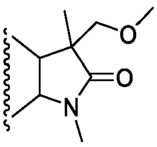, 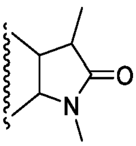, 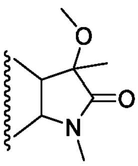, 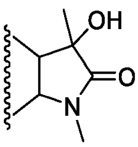, 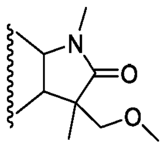, 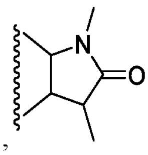, 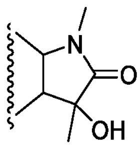,

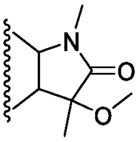, 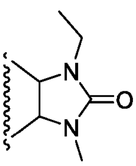, 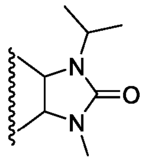, 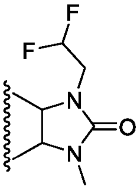, 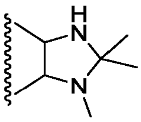, 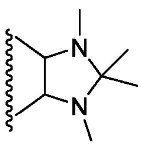, 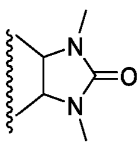,

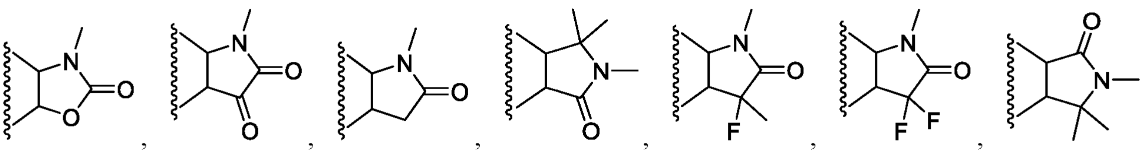, , , , , , ,

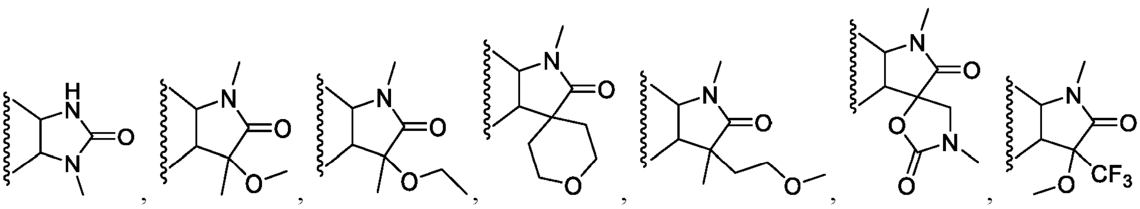, , , , , , ,

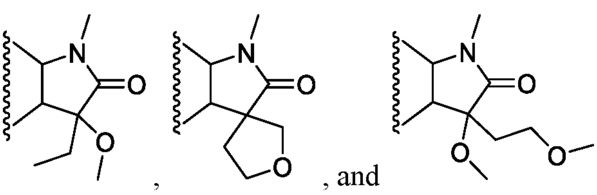, , and .

$R^1$ is selected from methyl, ethyl, isopropyl, and cyclopropyl:
$R^2$ and $R^3$ are independently selected from hydrogen, fluorine, and methyl;
$R^4$ is selected from fluorine, $-NH_2$, $-CH_3$, $-CF_3$, $-CHF_2$,

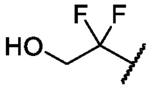 , 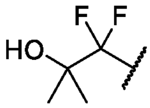 , 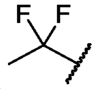 , 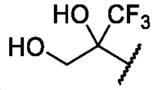 ,

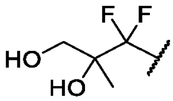 , 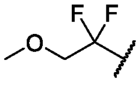 , 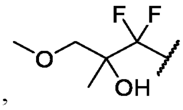 , 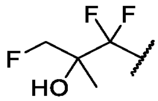 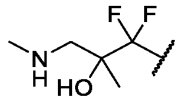 , 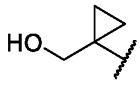 ,

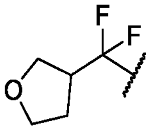 , 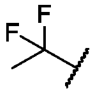 , 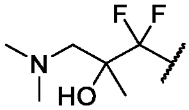 , and 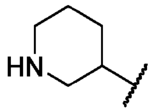 ;

and
'n' is an integer selected from 1, 2, and 3.

2. The compound of formula (I) and a pharmaceutically acceptable salt thereof, as claimed in claim 1, wherein the compound is selected from:

(R)-4-((1-(3-amino-5-(trifluoromethyl)phenyl)ethyl)amino)-2,6-dimethyl-6H-[1,4]oxazino[3,2-g]quinazolin-7(8H)-one (Compound 1);
(R)-4-((1-(3-(1,1-difluoro-2-hydroxyethyl)-2-fluorophenyl)ethyl)amino)-2,6-dimethyl-6H-[1,4]oxazino[3,2-g]quinazolin-7(8H)-one (Compound 2);
(R)-4-((1-(3-(1,1-difluoro-2-hydroxy-2-methylpropyl)-2-fluorophenyl)ethyl)amino)-2,6-dimethyl-6 H-[1,4]oxazino[3,2-g]quinazolin-7(8H)-one (Compound 3);
4-((1-(3-(1,1-difluoroethyl)-2-fluorophenyl)ethyl)amino)-2,6-dimethyl-6H-[1,4]oxazino[3,2-g]quinazolin-7(8H)-one (Compound 4);
4-((1-(3-amino-5-(difluoromethyl)phenyl)ethyl)amino)-2,6-dimethyl-6H-[1,4]oxazino[3,2-g]quinazolin-7(8H)-one (Compound 5);
4-((1-(3-(1,1-difluoro-2-hydroxyethyl)-2-methylphenyl)ethyl)amino)-2,6-dimethyl-6H-[1,4]oxazino[3,2-g]quinazolin-7(8H)-one (Compound 6);
2,6-dimethyl-4-((1-(3-(trifluoromethyl)phenyl)ethyl)amino)-6H-[1,4]oxazino[3,2-g]quinazolin-7(8H)-one (Compound 7);
4-((1-(2-fluoro-3-(trifluoromethyl)phenyl)ethyl)amino)-2,6-dimethyl-6H-[1,4]oxazino[3,2-g]quinazolin-7(8H)-one (Compound 8);
4-((1-(3,3-difluoro-2,3-dihydrobenzofuran-7-yl)ethyl)amino)-2,6-dimethyl-6H-[1,4]oxazino[3,2-g]quinazolin-7(8H)-one (Compound 9);
(R)-N-(1-(3-amino-5-(trifluoromethyl)phenyl)ethyl)-2,6-dimethyl-7,8-dihydro-6H-[1,4]oxazino[3,2-g]quinazolin-4-amine (Compound 10);
4-(((R)-1-(3-amino-5-(trifluoromethyl)phenyl)ethyl)amino)-2-methyl-6-((tetrahydrofuran-3-yl)methyl)-6H-[1,4]oxazino[3,2-g]quinazolin-7(8H)-one (Compound 11);
(R)-4-((1-(3-amino-5-(trifluoromethyl)phenyl)ethyl)amino)-6-(cyclopropylmethyl)-2-methyl-6H-[1,4]oxazino[3,2-g]quinazolin-7(8H)-one (Compound 12);
(R)-4-((1-(3-(1,1-difluoro-2-hydroxyethyl)-2-fluorophenyl)ethyl)amino)-6-ethyl-2-methyl-6H-[1,4]oxazino[3,2-g]quinazolin-7(8H)-one (Compound 13);
(R)-4-((1-(3-amino-5-(trifluoromethyl)phenyl)ethyl)amino)-2-ethyl-6-methyl-6H-[1,4]oxazino[3,2-g]quinazolin-7(8H)-one. (Compound 14);
(R)-4-((1-(3-(1,1-difluoro-2-hydroxyethyl)-2-fluorophenyl)ethyl)amino)-2-ethyl-6-methyl-6H-[1,4]oxazino[3,2-g]quinazolin-7(8H)-one (Compound 15);
(R)-2-cyclopropyl-4-((1-(3-(1,1-difluoro-2-hydroxyethyl)-2-fluorophenyl)ethyl)amino)-6-methyl-6H-[1,4]oxazino[3,2-g]quinazolin-7(8H)-one (Compound 16);

(4-(((R)-1-(3-amino-5-(trifluoromethyl)phenyl)ethyl)amino)-2-methyl-8,9-dihydro-7H-pyrano[2,3-g]quinazolin-7-yl)(pyrrolidin-1-yl)methanone (Compound 17);
(R)-4-((1-(3-(1,1-difluoro-2-hydroxyethyl)-2-fluorophenyl)ethyl)amino)-2,6,8,8-tetramethyl-6H-[1,4]oxazino[3,2-g]quinazolin-7(8H)-one (Compound 18);
2,6,8,8-tetramethyl-4-((1-(3-(trifluoromethyl)phenyl)ethyl)amino)-6H-[1,4]oxazino[3,2-g]quinazolin-7(8H)-one (Compound 19);
4-((1-(2-fluoro-3-(trifluoromethyl)phenyl)ethyl)amino)-2,6,8,8-tetramethyl-6H-[1,4]oxazino[3,2-g]quinazolin-7(8H)-one (Compound 20);
(R)-4-((1-(3-amino-5-(trifluoromethyl)phenyl)ethyl)amino)-2,6,8,8-tetramethyl-6H-[1,4]oxazino[3,2-g]quinazolin-7(8H)-one (Compound 21);
4-((1-(2-fluoro-3-(1,1,1-trifluoro-2,3-dihydroxypropan-2-yl)phenyl)ethyl)amino)-2,6,8, 8-tetramethyl-6H-[1,4]oxazino[3,2-g]quinazolin-7(8H)-one (Compound 22);
4-((1-(3-(1,1-difluoro-2,3-dihydroxy-2-methylpropyl)-2-fluorophenyl)ethyl)amino)-2,6,8,8-tetramethyl-6H-[1,4]oxazino[3,2-g]quinazolin-7(8H)-one (Compound 23);
(R)-N-(1-(3-amino-5-(trifluoromethyl)phenyl)ethyl)-2,6,8,8-tetramethyl-7,8-dihydro-6H-[1,4]oxazino[3,2-g]quinazolin-4-amine (Compound 24);
(R)-4'-((1-(3-(1,1-difluoro-2-hydroxyethyl)-2-fluorophenyl)ethyl)amino)-2',6'-dimethylspiro[cyclopropane-1,8'-[1,4]oxazino[3,2-g]quinazolin]-7'(6'H)-one (Compound 25);
(R)-1,1-Difluoro-1-(2-fluoro-3-(1-((2,8, 8-trimethyl-7-morpholino-8H[1,4]oxazino[3,2-g]quinazolin-4-yl)amino)ethyl)phenyl)-2-methylpropan-2-ol (Compound 26);
4-(((R)-1-(3-(1,1-difluoro-2-hydroxy-2-methylpropyl)-2-fluorophenyl)ethyl)amino)-2,6,8,9-tetramethyl-8,9-dihydropyrazino[2,3-g]quinazolin-7(6H)-one (Compound 27);
(R)-4-((1-(3-(1,1-difluoro-2-hydroxy-2-methylpropyl)-2-fluorophenyl)ethyl)amino)-2,6,8,8,9-pentamethyl-8,9-dihydropyrazino[2,3-g]quinazolin-7(6H)-one (Compound 28);
(R)-9-((1-(3-(1,1-difluoro-2-hydroxy-2-methylpropyl)-2-fluorophenyl)ethyl)amino)-1,3,7-trimethylpyrimido[4,5-g]quinazoline-2,4(1H,3H)-dione (Compound 29);
4-(((R)-1-(3-(1,1-difluoro-2-hydroxyethyl)-2-fluorophenyl)ethyl)amino)-2,6,8-trimethyl-6H-[1,4]oxazino[3,2-g]quinazolin-7(8H)-one (Compound 30);
4-(((R)-1-(3-(1,1-difluoro-2-hydroxy-2-methylpropyl)-2-fluorophenyl)ethyl)amino)-2,6, 8-trimethyl-6H-[1,4]oxazino[3,2-g]quinazolin-7(8H)-one (Compound 31);
4-(((R)-1-(3-(1,1-difluoro-2-hydroxy-2-methylpropyl)-2-fluorophenyl)ethyl)amino)-8-isopropyl-2,6-dimethyl-6H-[1,4]oxazino[3,2-g]quinazolin-7(8H)-one (Compound 32);
(R)-4-((1-(3-(1,1-Difluoro-2-hydroxy-2-methylpropyl)-2-fluorophenyl)ethyl) amino)-2,6,8,8-tetramethyl-6H-[1,4]oxazino[3,2-g]quinazolin-7(8H)-one (Compound 33);
(R)-1,1-Difluoro-1-(2-fluoro-3-(1-((2,6,8,8-tetramethyl-7,8-dihydro-6H-[1,4]oxazino [3,2-g]quinazolin-4-yl)amino)ethyl)phenyl)-2-methylpropan-2-ol (Compound 34);
(R)-2,2-Difluoro-2-(2-fluoro-3-(1-((2,6,8,8-tetramethyl-7,8-dihydro-6H-[1,4]oxazino[3,2-g]quinazolin-4-yl)amino)ethyl)phenyl)ethan-1-ol (Compound 35);
2,2-difluoro-2-(2-fluoro-3-((1R)-1-((2,6,8-trimethyl-7,8-dihydro-6H-[1,4]oxazino[3,2-g]quinazolin-4-yl)amino)ethyl)phenyl)ethan-1-ol (Compound 36);
(R)-1,1-Difluoro-1-(2-fluoro-3-(1-((2,6,7,7-tetramethyl-7,8-dihydro-6H-[1,4]oxazino[3,2-g]quinazolin-4-yl)amino)ethyl)phenyl)-2-methylpropan-2-ol (Compound 37);
(R)-4-((1-(3-(1,1-Difluoro-2-hydroxy-2-methylpropyl)-2-fluorophenyl)ethyl)amino)-2,6,8-trimethylpyrido[2,3-g]quinazolin-7(6H)-one (Compound 38);
(R)-4-((1-(3-(1,1-Difluoro-2-hydroxy-2-methylpropyl)-2-fluorophenyl)ethyl)amino)-2,6,8-trimethylpyrazino[2,3-g]quinazolin-7(6H)-one (Compound 39);
(R)-4-((1-(3-(1,1-Difluoro-2-hydroxy-2-methylpropyl)-2-fluorophenyl)ethyl) amino)-2,6,9-trimethyl-6,9-dihydropyrazino[2,3-g]quinazoline-7,8-dione (Compound 40);
(R)-4-((1-(3-(1,1-difluoro-2-hydroxy-2-methylpropyl)-2-fluorophenyl)ethyl)amino)-6-ethyl-2,8,8-trimethyl-6H-[1,4]oxazino[3,2-g]quinazolin-7(8H)-one (Compound 41);
(R)-4-((1-(3-(1,1-difluoro-2-hydroxy-2-methylpropyl)-2-fluorophenyl)ethyl)amino)-2,6,9,9-tetramethyl-8,9-dihydropyrido[2,3-g]quinazolin-7(6H)-one (Compound 42);
N-((R)-1-(3-amino-5-(trifluoromethyl)phenyl)ethyl)-2-methyl-6-(((R/S)-tetrahydrofuran-3-yl)methyl)-7,8-dihydro-6H-[1,4]oxazino[3,2-g]quinazolin-4-amine (Compound 43);
N-((R)-1-(3-amino-5-(trifluoromethyl)phenyl)ethyl)-2-methyl-6-(((S/R)-tetrahydrofuran-3-yl)methyl)-7,8-dihydro-6H-[1,4]oxazino[3,2-g]quinazolin-4-amine (Compound 44);
(R)-1-(3-(1-((2,6-dimethyl-7,8-dihydro-6H-[1,4]oxazino[3,2-g]quinazolin-4-yl)amino)ethyl)-2-fluorophenyl)-1,1-difluoro-2-methylpropan-2-ol (Compound 45);

4-(((R)-1-(3-amino-5-(trifluoromethyl)phenyl)ethyl)amino)-2,6,8-trimethyl-6H-[1,4]oxazino[3,2-g]quinazolin-7(8H)-one (Compound 46);
(R)-4-((1-(3-(1,1-difluoro-2-hydroxy-2-methylpropyl)-2-fluorophenyl)ethyl)amino)-10-fluoro-2,6-dimethyl-6H-[1,4]oxazino[3,2-g]quinazolin-7(8H)-one (Compound 47);
(R)-4-((1-(3-(1,1-difluoro-2-hydroxyethyl)-2-fluorophenyl)ethyl)amino)-6-(2-methoxyethyl)-2-methyl-6H-[1,4]oxazino[3,2-g]quinazolin-7(8H)-one (Compound 48);
(R)-4-((1-(3-amino-5-(trifluoromethyl)phenyl)ethyl)amino)-6-ethyl-2-methyl-6H-[1,4]oxazino[3,2-g]quinazolin-7(8H)-one (Compound 49);
(R)-1-(3-(1-((6-ethyl-2,8,8-trimethyl-7,8-dihydro-6H-[1,4]oxazino[3,2-g]quinazolin-4-yl)amino)ethyl)-2-fluorophenyl)-1,1-difluoro-2-methylpropan-2-ol (Compound 50);
(R)-4-((1-(3-(1,1-difluoro-2-methoxyethyl)-2-fluorophenyl)ethyl)amino)-2,6,8,8-tetramethyl-6H-[1,4]oxazino[3,2-g]quinazolin-7(8H)-one (Compound 51);
(R)-N-(1-(3-(1,1-difluoro-2-methoxyethyl)-2-fluorophenyl)ethyl)-2,6,8,8-tetramethyl-7,8-dihydro-6H-[1,4]oxazino[3,2-g]quinazolin-4-amine (Compound 52);
4-(((1R)-1-(3-(1,1-difluoro-2-hydroxy-3-methoxy-2-methylpropyl)-2-fluorophenyl)ethyl)amino)-2,6,8,8-tetramethyl-6H-[1,4]oxazino[3,2-g]quinazolin-7(8H)-one (Compound 53);
4-(((1R)-1-(2-fluoro-3-(1,1,3-trifluoro-2-hydroxy-2-methylpropyl)phenyl)ethyl)amino)-2,6,8,8-tetramethyl-6H-[1,4]oxazino[3,2-g]quinazolin-7(8H)-one (Compound 54);
4-(((1R)-1-(3-(1,1-difluoro-2-hydroxy-2-methyl-3-(methylamino)propyl)-2-fluorophenyl)ethyl)amino)-2,6,8,8-tetramethyl-6H-[1,4]oxazino[3,2-g]quinazolin-7(8H)-one (Compound 55);
(R)-2,2-difluoro-2-(2-fluoro-3-(1-((2-methyl-7,8-dihydro-6H-pyrano[3,2-g]quinazolin-4-yl)amino)ethyl)phenyl)ethan-1-ol (Compound 56);
(R)-4-((1-(3-(1,1-difluoro-2-hydroxy-2-methylpropyl)phenyl)ethyl)amino)-2,6,8,8-tetramethyl-6H-[1,4]oxazino[3,2-g]quinazolin-7(8H)-one (Compound 57);
(R)-4'-((1-(3-(1,1-difluoro-2-hydroxy-2-methylpropyl)-2-fluorophenyl)ethyl)amino)-2',8'-dimethylspiro[cyclopentane-1,6'-pyrrolo[3,2-g]quinazolin]-7'(8'H)-one (Compound 58);
4'-((1-(2-fluoro-3-(trifluoromethyl)phenyl)ethyl)amino)-2',8'-dimethylspiro[cyclopentane-1,6'-pyrrolo[3,2-g]quinazolin]-7'(8'H)-one (Compound 59);
2',8'-dimethyl-4'-((1-(2-methyl-3-(trifluoromethyl)phenyl)ethyl)amino)spiro[cyclopentane-1,6'-pyrrolo[3,2-g]quinazolin]-7'(8'H)-one (Compound 60);
4'-((1-(3-(difluoromethyl)-2-methylphenyl)ethyl)amino)-2',8'-dimethylspiro[cyclopentane-1,6'-pyrrolo[3,2-g]quinazolin]-7'(8'H)-one (Compound 61);
(R)-4'-((1-(3-(1,1-difluoro-2-hydroxyethyl)-2-fluorophenyl)ethyl)amino)-2',8'-dimethylspiro[cyclopentane-1,6'-pyrrolo[3,2-g]quinazolin]-7'(8'H)-one (Compound 62);
(R)-4-((1-(3-(1,1-difluoro-2-hydroxy-2-methylpropyl)-2-fluorophenyl) ethyl)amino)-6-(2-methoxyethyl)-2,8,8-trimethyl-6,8-dihydro-7H-pyrrolo[2,3-g]quinazolin-7-one (Compound 63);
(R)-4-((1-(3-(1,1-difluoro-2-hydroxy-2-methylpropyl)-2-fluorophenyl)ethyl)amino)-6-ethyl-2,8,8-trimethyl-6,8-dihydro-7H-pyrrolo[2,3-g]quinazolin-7-one (Compound 64);
(R)-6-cyclopropyl-4-((1-(3-(1,1-difluoro-2-hydroxy-2-methylpropyl)-2-fluoro phenyl)ethyl)amino)-2,8,8-trimethyl-6,8-dihydro-7H-pyrrolo[2,3-g]quinazolin-7-one (Compound 65);
(R)-4'-((1-(3-(1,1-difluoro-2-hydroxyethyl)-2-fluorophenyl)ethyl)amino)-2',8'-dimethylspiro [cyclopropane-1,6'-pyrrolo[3,2-g]quinazolin]-7'(8'H)-one (Compound 66);
(R)-4'-((1-(3-(1,1-difluoro-2-hydroxy-2-methylpropyl)-2-fluorophenyl)ethyl)amino)-2',8'-dimethylspiro[cyclopropane-1,6'-pyrrolo[3,2-g]quinazolin]-7'(8'H)-one (Compound 67);
(R)-4-((1-(3-(1,1-Difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one (Compound 68);
4-((1-(3-amino-5-(difluoromethyl)phenyl)ethyl)amino)-2,6,8,8-tetramethyl-6,8-dihydro-7H-pyrrolo[2,3-g]quinazolin-7-one (Compound 69);
(R)-4-((1-(3-amino-5-(trifluoromethyl)phenyl)ethyl)amino)-2,6,8,8-tetramethyl-6H-pyrrolo[2,3-g]quinazolin-7(8H)-one (Compound 70);
(S)-4-((1-(3-(1,1-difluoro-2-hydroxy-2-methylpropyl)-2-fluorophenyl)ethyl)amino)-2,6,8,8-tetramethyl-6H-pyrrolo[2,3-g]quinazolin-7(8H)-one (Compound 71);
(R)-4-((1-(3-(1,1-difluoro-2-hydroxyethyl)-2-fluorophenyl)ethyl)amino)-2,6,8,8-tetramethyl-6,8-dihydro-7H-pyrrolo[2,3-g]quinazolin-7-one (Compound 72);
4-((1-(3-(1,1-difluoro-2-hydroxy-2-methylpropyl)phenyl)ethyl)amino)-2,6,8,8-tetramethyl-6,8-dihydro-7H-pyrrolo[2,3-g]quinazolin-7-one (Compound 73);
4-((1-(2-fluoro-3-(1,1,1-trifluoro-2,3-dihydroxypropan-2-yl)phenyl)ethyl)amino)-2,6,8,8-tetramethyl-6,8-dihydro-7H-pyrrolo[2,3-g]quinazolin-7-one (Compound 74);

(R)-1,1-difluoro-1-(2-fluoro-3-(1-((2,6,8,8-tetramethyl-7,8-dihydro-6H-pyrrolo[2,3-g]quinazolin-4-yl)amino) ethyl)phenyl)-2-methylpropan-2-ol (Compound 75);
4-(((1R)-1-(3-(1,1-difluoro-2,3-dihydroxy-2-methylpropyl)-2-fluorophenyl)ethyl)amino)-2,6,8,8-tetramethyl-6,8-dihydro-7H-pyrrolo[2,3-g]quinazolin-7-one (Compound 76);
(R)-4-((1-(3-(1,1-difluoro-2-hydroxy-2-methylpropyl)-2-fluorophenyl)ethyl)amino)-2,8,8-trimethyl-6H-pyrrolo [2,3-g]quinazolin-7(8H)-one (Compound 77);
(R)-4-((1-(3-(1,1-difluoro-2-hydroxy-2-methylpropyl)-2-fluorophenyl)ethyl)amino)-6-isopropyl-2,8,8-trimethyl-6H-pyrrolo[2,3-g]quinazolin-7(8H)-one (Compound 78);
(R)-4'-((1-(3-(1,1-difluoro-2-hydroxy-2-methylpropyl)-2-fluorophenyl)ethyl)amino)-2',6'-dimethylspiro[cyclopropane-1,8'-pyrrolo[2,3-g]quinazolin]-7'(6'H)-one (Compound 79);
(R)-4-((1-(3-(1,1-difluoro-2-hydroxyethyl)-2-fluorophenyl)ethyl)amino)-2,6,6,8-tetramethyl-6,8-dihydro-7H-pyrrolo[3,2-g]quinazolin-7-one (Compound 80);
(R)-4-((1-(3-(1,1-difluoro-2-hydroxy-2-methylpropyl)-2-fluorophenyl)ethyl)amino)-2,6,6,8-tetramethyl-6,8-dihydro-7H-pyrrolo[3,2-g]quinazolin-7-one (Compound 81);
4-((1-(2-fluoro-3-(trifluoromethyl)phenyl)ethyl)amino)-2,6,6,8-tetramethyl-6,8-dihydro-7H-pyrrolo[3,2-g]quinazolin-7-one (Compound 82);
2,6,6,8-tetramethyl-4-((1-(2-methyl-3-(trifluoromethyl)phenyl)ethyl)amino)-6,8-dihydro-7H-pyrrolo[3,2-g]quinazolin-7-one (Compound 83);
4-((1-(3-(1,1-difluoroethyl)-2-fluorophenyl)ethyl)amino)-2,6,6,8-tetramethyl-6,8-dihydro-7H-pyrrolo[3,2-g]quinazolin-7-one (Compound 84);
4-((1-(3-(difluoro(tetrahydrofuran-3-yl)methyl)-2-fluorophenyl)ethyl)amino)-2,6,6,8-tetramethyl-6,8-dihydro-7H-pyrrolo[3,2-g]quinazolin-7-one (Compound 85);
(R)-4-((1-(3-(1,1-difluoro-2-hydroxy-2-methylpropyl)-2-fluorophenyl)ethyl)amino)-2-ethyl-6,6,8-trimethyl-6,8-dihydro-7H-pyrrolo[3,2-g]quinazolin-7-one (Compound 86);
(R)-4-((1-(3-(1,1-difluoro-2-hydroxy-2-methylpropyl)-2-fluorophenyl)ethyl)amino)-2-isopropyl-6,6,8-trimethyl-6H-pyrrolo[3,2-g]quinazolin-7(8H)-one (Compound 87);
(R)-4-((1-(3-(1,1-difluoro-2-hydroxyethyl)-2-fluorophenyl)ethyl)amino)-8-ethyl-2,6,6-trimethyl-6,8-dihydro-7H-pyrrolo[3,2-g]quinazolin-7-one (Compound 88);
(R)-4-((1-(3-(1,1-difluoro-2-hydroxy-2-methylpropyl)-2-fluorophenyl)ethyl)amino)-8-ethyl-2,6,6-trimethyl-6H-pyrrolo[3,2-g]quinazolin-7(8H)-one (Compound 89);
(R)-4-((1-(3-(1,1-difluoro-2-hydroxy-2-methylpropyl)-2-fluorophenyl)ethyl)amino)-2,6,6,8,9-pentamethyl-6,8-dihydro-7H-pyrrolo[3,2-g]quinazolin-7-one (Compound 90);
(R)-4-((1-(3-(1,1-difluoro-2-hydroxy-2-methylpropyl)-2-fluorophenyl)ethyl)amino)-6,6-diethyl-2,8-dimethyl-6,8-dihydro-7H-pyrrolo[3,2-g]quinazolin-7-one (Compound 91);
(R)-4-((1-(3-(1,1-difluoro-2-hydroxy-2-methylpropyl)-2-fluorophenyl)ethyl)amino)-6,6-bis(fluoromethyl)-2,8-dimethyl-6,8-dihydro-7H-pyrrolo[3,2-g]quinazolin-7-one (Compound 92);
4-(((R)-1-(3-(1,1-difluoro-2-hydroxy-2-methylpropyl)-2-fluorophenyl)ethyl)amino)-6-ethyl-2,6,8-trimethyl-6,8-dihydro-7H-pyrrolo[3,2-g]quinazolin-7-one (Compound 93);
4-(((R)-1-(3-(1,1-difluoro-2-hydroxy-2-methylpropyl)-2-fluorophenyl)ethyl)amino)-6-(methoxymethyl)-2,6,8-trimethyl-6,8-dihydro-7H-pyrrolo[3,2-g]quinazolin-7-one (Compound 94);
4-(((R)-1-(3-(1,1-difluoro-2-hydroxy-2-methylpropyl)-2-fluorophenyl)ethyl)amino)-2,6,8-trimethyl-6,8-dihydro-7H-pyrrolo[3,2-g]quinazolin-7-one (Compound 95);
4-(((R)-1-(3-(1,1-difluoro-2-hydroxy-2-methylpropyl)-2-fluorophenyl)ethyl)amino)-6-methoxy-2,6,8-trimethyl-6,8-dihydro-7H-pyrrolo[3,2-g]quinazolin-7-one (Compound 96);
4-(((R)-1-(3-(1,1-difluoro-2-hydroxy-2-methylpropyl)-2-fluorophenyl)ethyl)amino)-6-hydroxy-2,6,8-trimethyl-6,8-dihydro-7H-pyrrolo[3,2-g]quinazolin-7-one (Compound 97);
4-(((R)-1-(3-(1,1-difluoro-2-hydroxy-2-methylpropyl)-2-fluorophenyl)ethyl)amino)-8-(methoxymethyl)-2,6,8-trimethyl-6,8-dihydro-7H-pyrrolo[2,3-g]quinazolin-7-one (Compound 98);
4-(((R)-1-(3-(1,1-difluoro-2-hydroxy-2-methylpropyl)-2-fluorophenyl)ethyl)amino)-2,6,8-trimethyl-6,8-dihydro-7H-pyrrolo[2,3-g]quinazolin-7-one (Compound 99);
4-(((R)-1-(3-(1,1-difluoro-2-hydroxy-2-methylpropyl)-2-fluorophenyl)ethyl)amino)-8-hydroxy-2,6,8-trimethyl-6,8-dihydro-7H-pyrrolo[2,3-g]quinazolin-7-one (Compound 100);
4-(((R)-1-(3-(1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 101);
(S/R)-4-(((R/S)-1-(3-((S/R)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 102);
(S/R)-4-(((R/S)-1-(3-((R/S)-1,1-difluoro-2,3-dihydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 103);

(R)-8-((1-(3-(1,1-difluoro-2-hydroxy-2-methylpropyl)-2-fluorophenyl)ethyl)amino)-1-ethyl-3,6-dimethyl-1,3-dihydro-2H-imidazo[4,5-g]quinazolin-2-one (Compound 104);
(R)-8-((1-(3-(1,1-difluoro-2-hydroxy-2-methylpropyl)-2-fluorophenyl)ethyl)amino)-1-isopropyl-3,6-dimethyl-1,3-dihydro-2H-imidazo[4,5-g]quinazolin-2-one (Compound 105);
(R)-8-((1-(3-(1,1-difluoro-2-hydroxy-2-methylpropyl)-2-fluorophenyl)ethyl)amino)-1-(2,2-difluoroethyl)-3,6-dimethyl-1H-imidazo[4,5-g]quinazolin-2(3H)-one (Compound 106);
(R)-1,1-difluoro-1-(2-fluoro-3-(1-((2,2,3,6-tetramethyl-2,3-dihydro-1H-imidazo [4,5-g]quinazolin-8-yl)amino) ethyl)phenyl)-2-methylpropan-2-ol (Compound 107);
(R)-1,1-difluoro-1-(2-fluoro-3-(1-((1,2,2,3,6-pentamethyl-2,3-dihydro-1H-imidazo[4,5-g]quinazolin-8-yl)amino) ethyl)phenyl)-2-methylpropan-2-ol (Compound 108);
(R)-8-((1-(3-(1,1-difluoro-2-hydroxy-2-methylpropyl)-2-fluorophenyl)ethyl)amino)-1,3,6-trimethyl-1,3-dihydro-2H-imidazo[4,5-g]quinazolin-2-one (Compound 109);
(R)-2-cyclopropyl-4-((1-(3-(1,1-difluoro-2-hydroxy-2-methylpropyl)-2-fluorophenyl)ethyl)amino)-6,6,8-trimethyl-6,8-dihydro-7H-pyrrolo[3,2-g]quinazolin-7-one (Compound 110);
(R)-8-((1-(3-(1,1-difluoro-2-hydroxy-2-methylpropyl)-2-fluorophenyl)ethyl)amino)-1,6-dimethyloxazolo[4,5-g] quinazolin-2(1H)-one (Compound 111);
(R)-4-((1-(3-(1,1-difluoro-2-hydroxy-2-methylpropyl)-2-fluorophenyl)ethyl)amino)-2,6-dimethyl-6H-pyrrolo[2,3-g]quinazoline-7,8-dione (compound 112);
(R)-4-((1-(3-(1,1-difluoro-2-hydroxy-2-methylpropyl)-2-fluorophenyl)ethyl)amino)-2,6-dimethyl-6,8-dihydro-7H-pyrrolo[2,3-g]quinazolin-7-one (Compound 113);
(R)-4-((1-(3-(1,1-difluoro-2-hydroxy-2-methylpropyl)-2-fluorophenyl)ethyl)amino)-2,6,6,7-tetramethyl-6,7-dihydro-8H-pyrrolo[3,4-g]quinazolin-8-one (Compound 114);
4-((1-(2-fluoro-3-(1-(hydroxymethyl)cyclopropyl)phenyl)ethyl)amino)-2,6,8,8-tetramethyl-6,8-dihydro-7H-pyrrolo[2,3-g]quinazolin-7-one (Compound 115);
4-(((R)-1-(3-(1,1-difluoro-2-hydroxy-2-methylpropyl)-2-fluorophenyl)ethyl)amino)-8-fluoro-2,6,8-trimethyl-6,8-dihydro-7H-pyrrolo[2,3-g]quinazolin-7-one (Compound 116);
(R)-4-((1-(3-(1,1-difluoro-2-hydroxy-2-methylpropyl)-2-fluorophenyl)ethyl)amino)-8,8-difluoro-2,6-dimethyl-6,8-dihydro-7H-pyrrolo[2,3-g]quinazolin-7-one (Compound 117);
(R)-4-((1-(3-(1,1-difluoro-2-hydroxy-2-methylpropyl)-2-fluorophenyl)ethyl)amino)-2,7,8,8-tetramethyl-7,8-dihydro-6H-pyrrolo[3,4-g]quinazolin-6-one (Compound 118);
(R)-8-((1-(3-(1,1-difluoro-2-hydroxy-2-methylpropyl)-2-fluorophenyl)ethyl)amino)-3,6-dimethyl-1,3-dihydro-2H-imidazo[4,5-g]quinazolin-2-one (Compound 119);
(S)-4-(((S)-1-(3-(1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 120);
4-(((R)-1-(3-(1,1-difluoro-2-hydroxy-2-methylpropyl)-2-fluorophenyl)ethyl)amino)-8-ethoxy-2,6,8-trimethyl-6,8-dihydro-7H-pyrrolo[2,3-g]quinazolin-7-one (Compound 121);
(R)-4'-((1-(3-(1,1-difluoro-2-hydroxy-2-methylpropyl)-2-fluorophenyl)ethyl)amino)-2',6'-dimethyl-2,3,5,6-tetrahydrospiro[pyran-4,8'-pyrrolo[2,3-g]quinazolin]-7'(6'H)-one (Compound 122);
4-(((R)-1-(3-(1,1-difluoro-2-hydroxy-2-methylpropyl)-2-fluorophenyl)ethyl)amino)-8-(2-methoxyethyl)-2,6,8-trimethyl-6,8-dihydro-7H-pyrrolo[2,3-g]quinazolin-7-one (Compound 123);
4'-(((R)-1-(3-(1,1-difluoro-2-hydroxy-2-methylpropyl)-2-fluorophenyl)ethyl)amino)-2',3,6'-trimethylspiro[oxazolidine-5,8'-pyrrolo[2,3-g]quinazoline]-2,7'(6'H)-dione (Compound 124);
4-(((R)-1-(3-(1,1-difluoro-2-hydroxy-2-methylpropyl)-2-fluorophenyl)ethyl)amino)-8-methoxy-2,6-dimethyl-8-(trifluoromethyl)-6,8-dihydro-7H-pyrrolo[2,3-g]quinazolin-7-one (Compound 125);
4-(((R)-1-(3-(1,1-difluoro-2-hydroxy-2-methylpropyl)-2-fluorophenyl)ethyl)amino)-8-ethyl-8-methoxy-2,6-dimethyl-6,8-dihydro-7H-pyrrolo[2,3-g]quinazolin-7-one (compound 126);
4-(((1R)-1-(3-(1,1-difluoro-2-hydroxy-3-methoxy-2-methylpropyl)-2-fluorophenyl)ethyl)amino)-2,6,8,8-tetramethyl-6,8-dihydro-7H-pyrrolo[2,3-g]quinazolin-7-one (Compound 127);
4'-(((R)-1-(3-(1,1-difluoro-2-hydroxy-2-methylpropyl)-2-fluorophenyl)ethyl)amino)-2',6'-dimethyl-4,5-dihydro-2H-spiro[furan-3,8'-pyrrolo[2,3-g]quinazolin]-7'(6'H)-one (Compound 128);
4-(((1R)-1-(3-(3-(dimethylamino)-1,1-difluoro-2-hydroxy-2-methylpropyl)-2-fluorophenyl)ethyl)amino)-2,6,8,8-tetramethyl-6,8-dihydro-7H-pyrrolo[2,3-g]quinazolin-7-one (Compound 129);
4-(((1R)-1-(3-(1,1-difluoro-2-hydroxy-2-methyl-3-(methylamino)propyl)-2-fluorophenyl)ethyl)amino)-2,6,8,8-tetramethyl-6,8-dihydro-7H-pyrrolo[2,3-g]quinazolin-7-one (Compound 130);
4-(((R)-1-(3-amino-5-(trifluoromethyl)phenyl)ethyl)amino)-8-methoxy-2,6,8-trimethyl-6,8-dihydro-7H-pyrrolo [2,3-g]quinazolin-7-one (Compound 131);
4-(((R)-1-(3-(1,1-difluoro-2-hydroxy-2-methylpropyl)-2-fluorophenyl)ethyl)amino)-8-methoxy-8-(2-methoxyethyl)-2,6-dimethyl-6,8-dihydro-7H-pyrrolo[2,3-g]quinazolin-7-one (Compound 132);

4-(((1R)-1-(2-fluoro-3-(piperidin-3-yl)phenyl)ethyl)amino)-2,6,8,8-tetramethyl-6,8-dihydro-7H-pyrrolo[2,3-g]quinazolin-7-one (Compound 133);
(R)-4-((1-(2-fluoro-3-(trifluoromethyl)phenyl)ethyl)amino)-2,6,8,8-tetramethyl-6,8-dihydro-7H-pyrrolo[2,3-g]quinazolin-7-one (Compound 134);
4-(((1R)-1-(3-(3-(dimethylamino)-1,1-difluoro-2-hydroxy-2-methylpropyl)-2-fluorophenyl)ethyl)amino)-8-methoxy-2,6,8-trimethyl-6,8-dihydro-7H-pyrrolo[2,3-g]quinazolin-7-one (Compound 135);
2,6,8,8-tetramethyl-4-((1-(3-(trifluoromethyl)phenyl)ethyl)amino)-6,8-dihydro-7H-pyrrolo[2,3-g]quinazolin-7-one (Compound 136);
4-(((R)-1-(3-amino-5-(trifluoromethyl)phenyl)ethyl)amino)-6-methoxy-2,6,8-trimethyl-6,8-dihydro-7H-pyrrolo[3,2-g]quinazolin-7-one (Compound 137);
(R)-4'-((1-(3-amino-5-(trifluoromethyl)phenyl)ethyl)amino)-2',6'-dimethyl-2,3,5,6-tetrahydrospiro[pyran-4,8'-pyrrolo[2,3-g]quinazolin]-7'(6'H)-one (Compound 138); and
4-(((R)-1-(3-amino-5-(trifluoromethyl)phenyl)ethyl)amino)-8-ethyl-8-methoxy-2,6-dimethyl-6,8-dihydro-7H-pyrrolo[2,3-g]quinazolin-7-one (Compound 139).

**3.** A pharmaceutical composition comprising a compound of formula (I) or a pharmaceutically acceptable salt thereof, as claimed in claims 1 and 2, and a pharmaceutically acceptable carrier.

**4.** A compound of formula (I) and a pharmaceutically acceptable salt thereof as claimed in claims 1 and 2, for use in the treatment and/or prevention of a disease by inhibiting SOS1 in a subject, comprising administering to the subject a therapeutically effective amount of a said compound, wherein the disease is selected from cancer, Acute Staphylococcus aureus infection (Pediatric Patients), Acute Respiratory Distress syndrome/Acute Lung injury, Sepsis, and RASopathy.

**5.** A compound of formula (I) and a pharmaceutically acceptable salt thereof as claimed in claims 1 and 2, for use in the treatment and/or prevention of a disease by inhibiting the interaction of SOS 1 and RAS family protein in a subject, comprising administering to the subject a therapeutically effective amount of a said compound, wherein the disease is selected from cancer, Acute Staphylococcus aureus infection (Pediatric Patients), Acute Respiratory Distress syndrome/Acute Lung injury, Sepsis, and RASopathy.

**6.** The compound for use in the treatment and/or prevention of a cancer as claimed in claim 4 or 5, wherein the cancer is selected from the group consisting of pancreatic cancer, lung cancer, colorectal cancer, class 3 BRAF-mutant cancers, hematological cancer, cholangiocarcinoma, multiple myeloma, melanoma, uterine cancer, endometrial cancer, thyroid cancer, acute myeloid leukaemia, bladder cancer, urothelial cancer, gastric cancer, cervical cancer, head and neck squamous cell carcinoma, diffuse large B cell lymphoma, esophageal cancer, chronic lymphocytic leukaemia, hepatocellular cancer, breast cancer, ovarian cancer, prostate cancer, glioblastoma, renal cancer, Pure mucosal neuroma syndrome, Fibrous Epulis, and sarcomas.

**7.** The compound for use in the treatment and/or prevention of a disease as claimed in claim 4 or 5, wherein the RASopathy is selected from the group consisting of Neurofibromatosis type 1 (NF 1), Noonan Syndrome (NS), Noonan Syndrome with Multiple Lentigines (NSML), Capillary Malformation-Arteriovenous Malformation Syndrome (CM-AVM), Costello Syndrome (CS), Cardio-Facio-Cutaneous Syndrome (CFC), Legius Syndrome, Noonan-like/-multiple giant cell lesion syndrome and Hereditary gingival fibromatosis (HGF).

**8.** The compound of formula (I) and a pharmaceutically acceptable salt thereof, as claimed in claims 1 and 2, for use in the treatment and/or prevention of cancer, wherein said compound is administered in combination with at least one more pharmacologically active substance.

**9.** The compound of formula (I) and a pharmaceutically acceptable salt thereof, as claimed in claims 1 and 2, for use in the treatment and/or prevention of cancer, wherein the compound is administered before, after, or together with at least one other pharmacologically active substance.

## Patentansprüche

**1.** Eine Verbindung der Formel (I) und ein pharmakologisch akzeptables Salz davon,

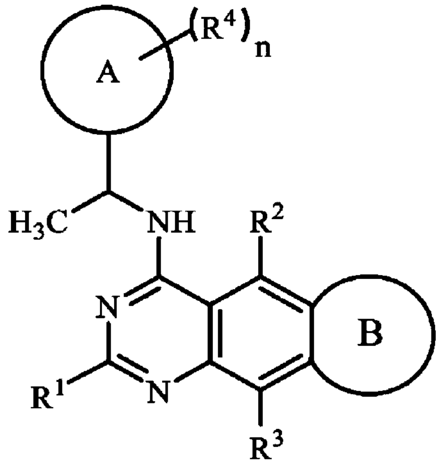

(I)

wobei:

der Ring A ausgewählt ist aus Phenyl und

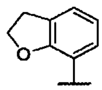
;

der Ring B ausgewählt ist aus

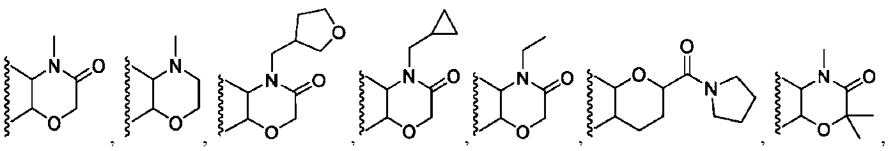
, , , , , , ,

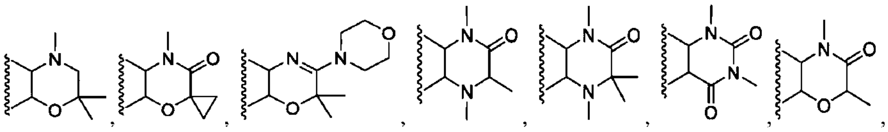
, , , , , , ,

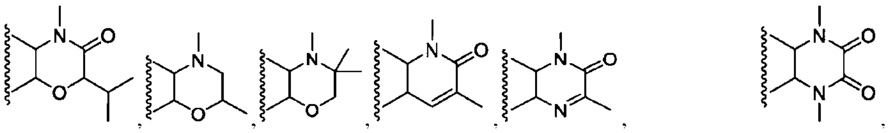
, , , , , ,

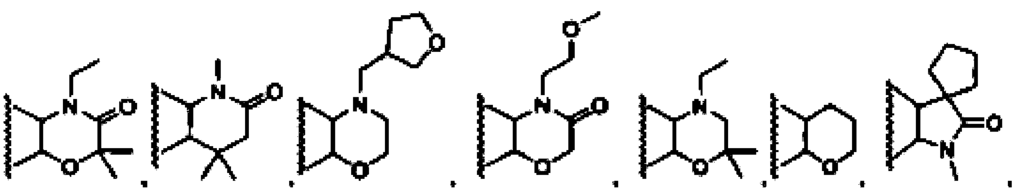
. . . . . . .

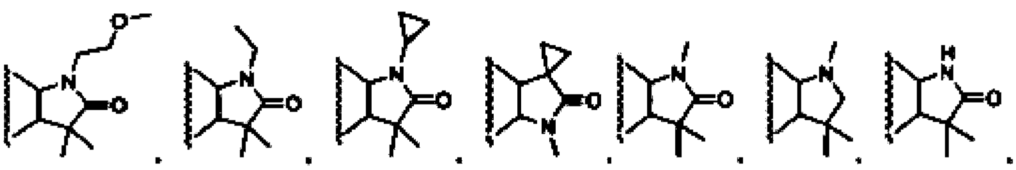
. . . . . . .

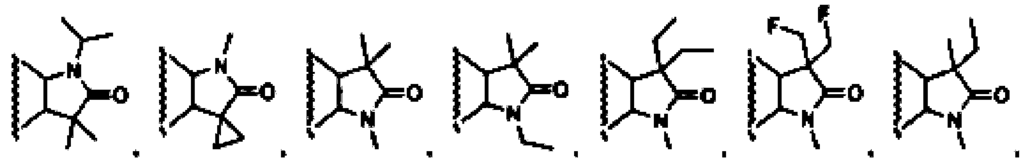

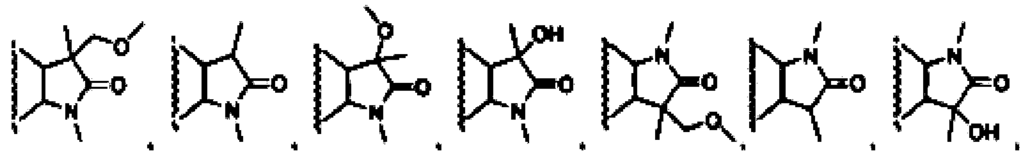

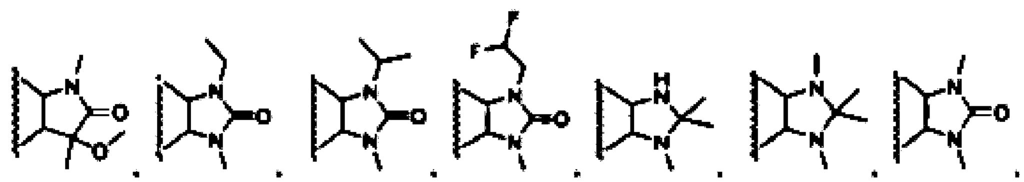

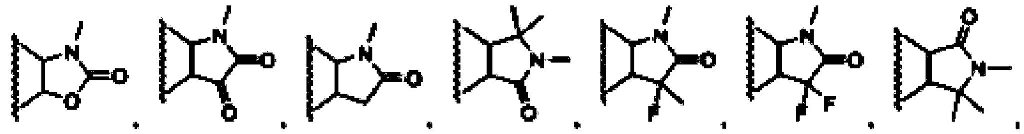

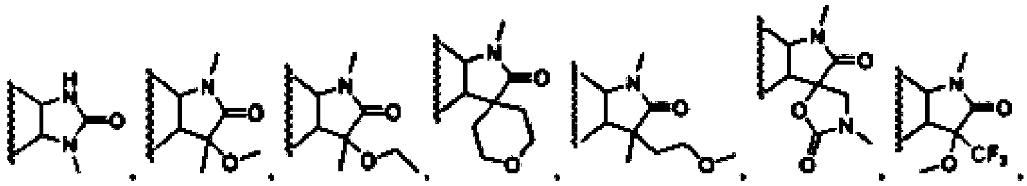

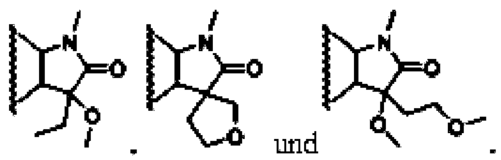

R1 ist ausgewählt aus Methyl, Ethyl, Isopropyl und Cyclopropyl:
R2 und R3 sind unabhängig voneinander ausgewählt aus Wasserstoff, Fluor und Methyl;
R4 est choisi parmi le fluor, $-NH_2$, $-CH_3$, $-CF_3$, $-CHF_2$

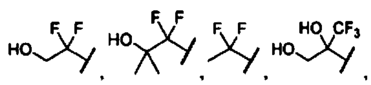

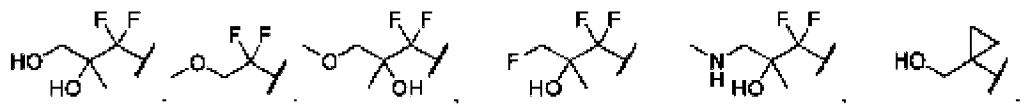

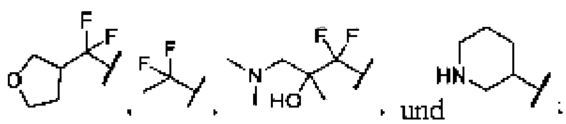

und
"n" ist eine ganze Zahl, ausgewählt aus 1, 2 und 3.

2. Die Verbindung der Formel (I) und ein pharmakologisch akzeptables Salz davon, wie in Anspruch 1 beansprucht, wobei die Verbindung ausgewählt ist aus:

(R)-4-((1-(3-amino-5-(trifluormethyl)phenyl)ethyl)amino)-2,6-dimethyl-6H-[1,4]oxazino[3,2-g]chinazolin-7(8H)-on (Verbindung 1);
(R)-4-((1-(3-(1,1-difluor-2-hydroxyethyl)-2-fluorphenyl)ethyl)amino)-2,6-dimethyl-6H-[1,4]oxazino[3,2-g]chinazolin-7(8H)-on (Verbindung 2);
(R)-4-((1-(3-(1,1-difluor-2-hydroxy-2-methylpropyl)-2-fluorphenyl)ethyl)amino)-2,6-dimethyl-6H-[1,4]oxazino[3,2-g]chinazolin-7(8H)-on (Verbindung 3);
4-((1-(3-(1,1-difluorethyl)-2-fluorphenyl)ethyl)amino)-2,6-dimethyl-6H-[1,4]oxazino[3,2-g]chinazolin-7(8H)-on (Verbindung 4);
4-((1-(3-amino-5-(difluormethyl)phenyl)ethyl)amino)-2,6-dimethyl-6H-[1,4]oxazino[3,2-g]chinazolin-7(8H)-on (Verbindung 5);
4-((1-(3-(1,1-difluor-2-hydroxyethyl)-2-methylphenyl)ethyl)amino)-2,6-dimethyl-6H-[1,4]oxazino[3,2-g]chinazolin-7(8H)-on (Verbindung 6);
2,6-dimethyl-4-((1-(3-(trifluormethyl)phenyl)ethyl)amino)-6H-[1,4]oxazino[3,2-g]chinazolin-7(8H)-on (Verbindung 7);
4-((1-(2-fluor-3-(trifluormethyl)phenyl)ethyl)amino)-2,6-dimethyl-6H-[1,4]oxazino[3,2-g]chinazolin-7(8H)-on (Verbindung 8);
4-((1-(3,3-difluor-2,3-dihydrobenzofuran-7-yl)ethyl)amino)-2,6-dimethyl-6H-[1,4]oxazino[3,2-g]chinazolin-7(8H)-on (Verbindung 9);
(R)-N-(1-(3-amino-5-(trifluormethyl)phenyl)ethyl)-2,6-dimethyl-7,8-dihydro-6H-[1,4]oxazino[3,2-g]chinazolin-4-amin (Verbindung 10);
4-(((R)-1-(3-amino-5-(trifluormethyl)phenyl)ethyl)amino)-2-methyl-6-((tetrahydrofuran-3-yl)methyl)-6H-[1,4]oxazino[3,2-g]chinazolin-7(8H)-on (Verbindung 11);
(R)-4-((1-(3-amino-5-(trifluormethyl)phenyl)ethyl)amino)-6-(cyclopropylmethyl)-2-methyl-6H-[1,4]oxazino[3,2-g]chinazolin-7(8H)-on (Verbindung 12);
(R)-4-((1-(3-(1,1-difluor-2-hydroxyethyl)-2-fluorphenyl)ethyl)amino)-6-ethyl-2-methyl-6H-[1,4]oxazino[3,2-g]chinazolin-7(8H)-on (Verbindung 13);
(R)-4-((1-(3-amino-5-(trifluormethyl)phenyl)ethyl)amino)-2-ethyl-6-methyl-6H-[1,4]oxazino[3,2-g]chinazolin-7(8H)-on (Verbindung 14);
(R)-4-((1-(3-(1,1-difluor-2-hydroxyethyl)-2-fluorphenyl)ethyl)amino)-2-ethyl-6-methyl-6H-[1,4]oxazino[3,2-g]chinazolin-7(8H)-on (Verbindung 15);
(R)-2-cyclopropyl-4-((1-(3-(1,1-difluor-2-hydroxyethyl)-2-fluorphenyl)ethyl)amino)-6-methyl-6H-[1,4]oxazino[3,2-g]chinazolin-7(8H)-on (Verbindung 16);
(4-(((R)-1-(3-amino-5-(trifluormethyl)phenyl)ethyl)amino)-2-methyl-8,9-dihydro-7H-pyrano[2,3-g]chinazolin-7-yl)(pyrrolidin-1-yl)methanon (Verbindung 17);
(R)-4-((1-(3-(1,1-difluor-2-hydroxyethyl)-2-fluorphenyl)ethyl)amino)-2,6,8,8-tetramethyl-6H-[1,4]oxazino[3,2-g]chinazolin-7(8H)-on (Verbindung 18);
2,6,8,8-Tetramethyl-4-((1-(3-(trifluormethyl)phenyl)ethyl)amino)-6H-[1,4]oxazino[3,2-g]chinazolin-7(8H)-on (Verbindung 19);
4-((1-(2-fluor-3-(trifluormethyl)phenyl)ethyl)amino)-2,6,8,8-tetramethyl-6H-[1,4]oxazino[3,2-g]chinazolin-7(8H)-on (Verbindung 20);
(R)-4-((1-(3-amino-5-(trifluormethyl)phenyl)ethyl)amino)-2,6,8,8-tetramethyl-6H-[1,4]oxazino[3,2-g]chinazolin-7(8H)-on (Verbindung 21);
4-((1-(2-fluor-3-(1,1,1-trifluor-2,3-dihydroxypropan-2-yl)phenyl)ethyl)amino)-2,6,8,8-tetramethyl-6H-[1,4]oxazino[3,2-g]chinazolin-7(8H)-on (Verbindung 22);
4-((1-(3-(1,1-difluor-2,3-dihydroxy-2-methylpropyl)-2-fluorphenyl)ethyl)amino)-2,6,8,8-tetramethyl-6H-[1,4]oxazino[3,2-g]chinazolin-7(8H)-on (Verbindung 23);
(R)-N-(1-(3-amino-5-(trifluormethyl)phenyl)ethyl)-2,6,8,8-tetramethyl-7,8-dihydro-6H-[1,4]oxazino[3,2-g]chinazolin-4-amin (Verbindung 24);
(R)-4'-((1-(3-(1,1-difluor-2-hydroxyethyl)-2-fluorphenyl)ethyl)amino)-2',6'-dimethylspiro[cyclopropan-1,8'-[1,4]oxazino[3,2-g]chinazolin]-7'(6'H)-on (Verbindung 25);
(R)-1,1-Difluor-1-(2-fluor-3-(1-((2,8,8-trimethyl-7-morpholino-8H-[1,4]oxazino[3,2-g]chinazolin-4-yl)amino)ethyl)phenyl)-2-methylpropan-2-ol (Verbindung 26);
4-(((R)-1-(3-(1,1-difluor-2-hydroxy-2-methylpropyl)-2-fluorphenyl)ethyl)amino)-2,6,8,9-tetramethyl-8,9-dihydropyrazino[2,3-g]chinazolin-7(6H)-on (Verbindung 27);
(R)-4-((1-(3-(1,1-difluor-2-hydroxy-2-methylpropyl)-2-fluorphenyl)ethyl)amino)-2,6,8,8,9-pentamethyl-8,9-dihydropyrazino[2,3-g]chinazolin-7(6H)-on (Verbindung 28);
(R)-9-((1-(3-(1,1-difluor-2-hydroxy-2-methylpropyl)-2-fluorphenyl)ethyl)amino)-1,3,7-trimethylpyrimido[4,5-g]chinazolin-2,4(1H,3H)-dion (Verbindung 29);
4-(((R)-1-(3-(1,1-difluor-2-hydroxyethyl)-2-fluorphenyl)ethyl)amino)-2,6,8-trimethyl-6H-[1,4]oxazino[3,2-g]chinazolin-7(8H)-on (Verbindung 30);
4-(((R)-1-(3-(1,1-Difluor-2-hydroxy-2-methylpropyl)-2-fluorphenyl)ethyl)amino)-2,6,8-trimethyl-6H-[1,4]oxazino[3,2-g]chinazolin-7(8H)-on (Verbindung 31);
4-(((R)-1-(3-(1,1-Difluor-2-hydroxy-2-methylpropyl)-2-fluorphenyl)ethyl)amino)-8-isopropyl-2,6-dimethyl-6H-[1,4]oxazino[3,2-g]chinazolin-7(8H)-on (Verbindung 32);
(R)-4-((1-(3-(1,1-Difluor-2-hydroxy-2-methylpropyl)-2-fluorphenyl)ethyl)amino)-2,6,8,8-tetramethyl-6H-[1,4]oxazino[3,2-g]chinazolin-7(8H)-on (Verbindung 33);
(R)-1,1-Difluor-1-(2-fluor-3-(1-((2,6,8,8-tetramethyl-7,8-dihydro-6H-[1,4]oxazino[3,2-g]chinazolin-4-yl)amino)ethyl)phenyl)-2-methylpropan-2-ol (Verbindung 34);
(R)-2,2-Difluor-2-(2-fluor-3-(1-((2,6,8,8-tetramethyl-7,8-dihydro-6H-[1,4]oxazino[3,2-g]chinazolin-4-yl)amino)ethyl)phenyl)ethan-1-ol (Verbindung 35);
2,2-Difluor-2-(2-fluor-3-((1R)-1-((2,6,8-trimethyl-7,8-dihydro-6H-[1,4]oxazino[3,2-g]chinazolin-4-yl)amino)ethyl)phenyl)ethan-1-ol (Verbindung 36);
(R)-1,1-Difluor-1-(2-fluor-3-(1-((2,6,7,7-tetramethyl-7,8-dihydro-6H-[1,4]oxazino[3,2-g]chinazolin-4-yl)amino)ethyl)phenyl)-2-methylpropan-2-ol (Verbindung 37);
(R)-4-((1-(3-(1,1-Difluor-2-hydroxy-2-methylpropyl)-2-fluorphenyl)ethyl)amino)-2,6,8-trimethylpyrido[2,3-g]chinazolin-7(6H)-on (Verbindung 38);
(R)-4-((1-(3-(1,1-Difluor-2-hydroxy-2-methylpropyl)-2-fluorphenyl)ethyl)amino)-2,6,8-trimethylpyrazino[2,3-g]chinazolin-7(6H)-on (Verbindung 39);
(R)-4-((1-(3-(1,1-Difluor-2-hydroxy-2-methylpropyl)-2-fluorphenyl)ethyl)amino)-2,6,9-trimethyl-6,9-dihydropyrazino[2,3-g]chinazolin-7,8-dion (Verbindung 40);
(R)-4-((1-(3-(1,1-Difluor-2-hydroxy-2-methylpropyl)-2-fluorphenyl)ethyl)amino)-6-ethyl-2,8,8 -trimethyl-6H-[1,4]oxazino[3,2-g]chinazolin-7(8H)-on (Verbindung 41);
(R)-4-((1-(3-(1,1-Difluor-2-hydroxy-2-methylpropyl)-2-fluorphenyl)ethyl)amino)-2,6,9,9-tetra methyl-8,9-dihydropyrido[2,3-g]chinazolin-7(6H)-on (Verbindung 42);
N-((R)-1-(3-amino-5-(trifluormethyl)phenyl)ethyl)-2-methyl-6-(((R/S)-tetrahydrofuran-3-yl)methy 1)-7,8-dihydro-6H-[1,4]oxazino[3,2-g]chinazolin-4-amin (Verbindung 43);
N-((R)-1-(3-amino-5-(trifluormethyl)phenyl)ethyl)-2-methyl-6-(((S/R)-tetrahydrofuran-3-yl)methy l)-7,8-dihydro-6H-[1,4]oxazino[3,2-g]chinazolin-4-amin (Verbindung 44);
(R)-1-(3-(1-((2,6-Dimethyl-7,8-dihydro-6H-[1,4]oxazino[3,2-g]chinazolin-4-yl)amino)ethyl)-2-fluorphenyl)-1,1-difluor-2-methylpropan-2-ol (Verbindung 45);
4-((R)-1-(3-amino-5-(trifluormethyl)phenyl)ethyl)amino)-2,6,8-trimethyl-6H-[1,4] oxazino[3,2-g]chinazolin-7(8H)-on (Verbindung 46);
(R)-4-((1-(3-(1,1-Difluor-2-hydroxy-2-methylpropyl)-2-fluorphenyl)ethyl)amino)-10-fluor-2,6-dimethyl-6H-[1,4]oxazino[3,2-g]chinazolin-7(8H)-on (Verbindung 47);
(R)-4-((1-(3-(1,1-Difluor-2-hydroxyethyl)-2-fluorphenyl)ethyl)amino)-6-(2-methoxyethyl)-2-methyl-6H-[1,4]oxazino[3,2-g]chinazolin-7(8H)-on (Verbindung 48);
(R)-4-((1-(3-amino-5-(trifluormethyl)phenyl)ethyl)amino)-6-ethyl-2-methyl-6H-[1,4]oxazino[3 ,2-g]chinazolin-7(8H)-on (Verbindung 49);
(R)-1-(3-(1-((6-ethyl-2,8,8-trimethyl-7,8-dihydro-6H-[1,4]oxazino[3,2-g]chinazolin-4-yl)amin o)ethyl)-2-fluorphenyl)-1,1-difluor-2-methylpropan-2-ol (Verbindung 50);
(R)-4-((1-(3-(1,1-Difluor-2-methoxyethyl)-2-fluorphenyl)ethyl)amino)-2,6,8,8-tetramethyl-6H-[1,4]oxazino[3,2-g]chinazolin-7(8H)-on (Verbindung 51);
(R)-N-(1-(3-(1,1-Difluor-2-methoxyethyl)-2-fluorphenyl)ethyl)-2,6,8,8-tetramethyl-7,8-dihydro-6H-[1,4]oxazino[3,2-g]chinazolin-4-amin (Verbindung 52);
4-(((1R)-1-(3-(1,1-Difluor-2-hydroxy-3-methoxy-2-methylpropyl)-2-fluorphenyl)ethyl)amino)-2,6,8,8-tetramethyl-6H-[1,4]oxazino[3,2-g]chinazolin-7(8H)-on (Verbindung 53);
4-(((1R)-1-(2-fluor-3-(1,1,3-Trifluor-2-hydroxy-2-methylpropyl)phenyl)ethyl)amino)-2,6,8,8-tetramethyl-6H-[1,4]oxazino[3,2-g]chinazolin-7(8H)-on (Verbindung 54);
4-(((1R)-1-(3-(1,1-Difluor-2-hydroxy-2-methyl-3-(methylamino)propyl)-2-fluorphenyl)ethyl)amino)-2,6,8,8-tetramethyl-6H-[1,4]oxazino[3,2-g]chinazolin-7(8H)-on (Verbindung 55);
(R)-2,2-Difluor-2-(2-fluor-3-(1-((2-methyl-7,8-dihydro-6H-pyrano[3,2-g]chinazolin-4-yl)amino)ethyl)phenyl)ethan-1-ol (Verbindung 56);
(R)-4-((1-(3-(1,1-Difluor-2-hydroxy-2-methylpropyl)phenyl)ethyl)amino)-2,6,8,8-tetramethyl-6H-[1,4]oxazino[3,2-g]chinazolin-7(8H)-on (Verbindung 57);
(R)-4'-((1-(3-(1,1-Difluor-2-hydroxy-2-methylpropyl)-2-fluorphenyl)ethyl)amino)-2',8'-dimethylspiro[cyclopentan-1,6'-pyrrolo[3,2-g]chinazolin]-7'(8'H)-on (Verbindung 58);
4'-((1-(2-fluor-3-(trifluormethyl)phenyl)ethyl)amino)-2',8'-dimethylspiro[cyclopentan-1,6'-pyrrolo[3,2-g]chinazolin]-7'(8'H)-on (Verbindung 59);
2',8'-Dimethyl-4'-((1-(2-methyl-3-(trifluormethyl)phenyl)ethyl)amino)spiro[cyclopentan-1,6'-pyrrolo[3,2-g]chinazolin]-7'(8'H)-on (Verbindung 60);
4'-((1-(3-(Difluormethyl)-2-methylphenyl)ethyl)amino)-2',8'-dimethylspiro[cyclopentan-1,6'-pyrrolo[3,2-g]chinazolin]-7'(8'H)-on (Verbindung 61);
(R)-4'-((1-(3-(1,1-Difluor-2-hydroxyethyl)-2-fluorphenyl)ethyl)amino)-2',8'-dimethylspiro[cyclopentan-1,6'-pyrrolo[3,2-g]chinazolin]-7'(8'H)-on (Verbindung 62);
(R)-4-((1-(3-(1,1-Difluor-2-hydroxy-2-methylpropyl)-2-fluorphenyl)ethyl)amino)-6-(2-methoxyethyl)-2,8,8-trimethyl-6,8-dihydro-7H-pyrrolo[2,3-g]chinazolin-7-on (Verbindung 63);
(R)-4-((1-(3-(1,1-Difluor-2-hydroxy-2-methylpropyl)-2-fluorphenyl)ethyl)amino)-6-ethyl-2,8,8-trimethyl-6,8-dihydro-7H-pyrrolo[2,3-g]chinazolin-7-on (Verbindung 64);
(R)-6-Cyclopropyl-4-((1-(3-(1,1-Difluor-2-hydroxy-2-methylpropyl)-2-fluorphenyl)ethyl)amino)-2,8,8-trimethyl-6,8-dihydro-7H-pyrrolo[2,3-g]chinazolin-7-on (Verbindung 65);
(R)-4'-((1-(3-(1,1-Difluor-2-hydroxyethyl)-2-fluorphenyl)ethyl)amino)-2',8'-dimethylspiro[cyclopropan-1,6'-pyrrolo[3,2-g]chinazolin]-7'(8'H)-on (Verbindung 66);
(R)-4'-((1-(3-(1,1-Difluor-2-hydroxy-2-methylpropyl)-2-fluorphenyl)ethyl)amino)-2',8'-dimethylspiro[cyclopropan-1,6'-pyrrolo[3,2-g]chinazolin]-7'(8'H)-on (Verbindung 67);
(R)-4-((1-(3-(1,1-Difluor-2-hydroxy-2-methylpropyl)-2-fluorphenyl)ethyl)amino)-2,6,8,8-tetramethyl-6,8-dihydro-7H-pyrrolo[2,3-g]chinazolin-7-on (Verbindung 68);
4-((1-(3-Amino-5-(difluormethyl)phenyl)ethyl)amino)-2,6,8,8-tetramethyl-6,8-dihydro-7H-pyrrolo[2,3-g]chinazolin-7-on (Verbindung 69);
(R)-4-((1-(3-Amino-5-(trifluormethyl)phenyl)ethyl)amino)-2,6,8,8-tetramethyl-6H-pyrrolo[2,3-g]chinazolin-7(8H)-on (Verbindung 70);
(S)-4-((1-(3-(1,1-Difluor-2-hydroxy-2-methylpropyl)-2-fluorphenyl)ethyl)amino)-2,6,8,8-tetramethyl-6H-pyrrolo [2,3-g]chinazolin-7(8H)-on (Verbindung 71);
(R)-4-((1-(3-(1,1-Difluor-2-hydroxyethyl)-2-fluorphenyl)ethyl)amino)-2,6,8,8-tetramethyl-6,8-dihydro-7H-pyrrolo[2,3-g]chinazolin-7-on (Verbindung 72);
4-((1-(3-(1,1-Difluor-2-hydroxy-2-methylpropyl)phenyl)ethyl)amino)-2,6,8,8-tetramethyl-6,8-dihydro-7H-pyrrolo[2,3-g]chinazolin-7-on (Verbindung 73);
4-((1-(2-Fluor-3-(1,1,1-trifluor-2,3-dihydroxypropan-2-yl)phenyl)ethyl)amino)-2,6,8,8-tetramethyl-6,8-dihydro-7H-pyrrolo[2,3-g]chinazolin-7-on (Verbindung 74);
(R)-1,1-Difluor-1-(2-Fluor-3-(1-((2,6,8,8-tetramethyl-7,8-dihydro-6H-pyrrolo[2,3-g]chinazolin-4-yl)amino)ethyl) phenyl)-2-methylpropan-2-ol (Verbindung 75);
4-(((1R)-1-(3-(1,1-Difluor-2,3-dihydroxy-2-methylpropyl)-2-fluorphenyl)ethyl)amino)-2,6,8,8-tetramethyl-6,8-dihydro-7H-pyrrolo[2,3-g]chinazolin-7-on (Verbindung 76);
(R)-4-((1-(3-(1,1-Difluor-2-hydroxy-2-methylpropyl)-2-fluorphenyl)ethyl)amino)-2,8,8-trimethyl-6H-pyrrolo[2,3-g]chinazolin-7(8H)-on (Verbindung 77);
(R)-4-((1-(3-(1,1-Difluor-2-hydroxy-2-methylpropyl)-2-fluorphenyl)ethyl)amino)-6-isopropyl-2,8,8-trimethyl-6H-pyrrolo[2,3-g]chinazolin-7(8H)-on (Verbindung 78);
(R)-4'-((1-(3-(1,1-Difluor-2-hydroxy-2-methylpropyl)-2-fluorphenyl)ethyl)amino)-2',6'-dimethylspiro[cyclopropan-1,8'-pyrrolo[2,3-g]chinazolin]-7'(6'H)-on (Verbindung 79);
(R)-4-((1-(3-(1,1-Difluor-2-hydroxyethyl)-2-fluorphenyl)ethyl)amino)-2,6,6,8-tetramethyl-6,8-dihydro-7H-pyrrolo[3,2-g]chinazolin-7-on (Verbindung 80);
(R)-4-((1-(3-(1,1-Difluor-2-hydroxy-2-methylpropyl)-2-fluorphenyl)ethyl)amino)-2,6,6,8-tetramethyl-6,8-dihydro-7H-pyrrolo[3,2-g]chinazolin-7-on (Verbindung 81);
4-((1-(2-Fluor-3-(trifluormethyl)phenyl)ethyl)amino)-2,6,6,8-tetramethyl-6,8-dihydro-7H-pyrrolo[3,2-g]chinazolin-7-on (Verbindung 82);
2,6,6,8-Tetramethyl-4-((1-(2-methyl-3-(trifluormethyl)phenyl)ethyl)amino)-6,8-dihydro-7H-pyrrolo[3,2-g]chinazolin-7-on (Verbindung 83);
4-((1-(3-(1,1-Difluorethyl)-2-fluorphenyl)ethyl)amino)-2,6,6,8-tetramethyl-6,8-dihydro-7H-pyrrolo[3,2-g]chinazolin-7-on (Verbindung 84);
4-((1-(3-(difluor(tetrahydrofuran-3-yl)methyl)-2-fluorphenyl)ethyl)amino)-2,6,6,8-tetramethyl-6,8-dihydro-7H-pyrrolo[3,2-g]chinazolin-7-on (Verbindung 85);
(R)-4-((1-(3-(1,1-Difluor-2-hydroxy-2-methylpropyl)-2-fluorphenyl)ethyl)amino)-2-ethyl-6,6,8-trimethyl-6,8-dihydro-7H-pyrrolo[3,2-g]chinazolin-7-on (Verbindung 86);
(R)-4-((1-(3-(1,1-Difluor-2-hydroxy-2-methylpropyl)-2-fluorphenyl)ethyl)amino)-2-isopropyl-6,6,8-trimethyl-6H-pyrrolo[3,2-g]chinazolin-7(8H)-on (Verbindung 87);
(R)-4-((1-(3-(1,1-Difluor-2-hydroxyethyl)-2-fluorphenyl)ethyl)amino)-8-ethyl-2,6,6-trimethyl-6,8-dihydro-7H-pyrrolo[3,2-g]chinazolin-7-on (Verbindung 88);
(R)-4-((1-(3-(1,1-Difluor-2-hydroxy-2-methylpropyl)-2-fluorphenyl)ethyl)amino)-8-ethyl-2,6,6-trimethyl-6H-pyrrolo[3,2-g]chinazolin-7(8H)-on (Verbindung 89);
(R)-4-((1-(3-(1,1-Difluor-2-hydroxy-2-methylpropyl)-2-fluorphenyl)ethyl)amino)-2,6,6,8,9-pentamethyl-6,8-dihydro-7H-pyrrolo[3,2-g]chinazolin-7-on (Verbindung 90);
(R)-4-((1-(3-(1,1-Difluor-2-hydroxy-2-methylpropyl)-2-fluorphenyl)ethyl)amino)-6,6-Diethyl-2,8-dimethyl-6,8-dihydro-7H-pyrrolo[3,2-g]chinazolin-7-on (Verbindung 91);
(R)-4-((1-(3-(1,1-Difluor-2-hydroxy-2-methylpropyl)-2-fluorphenyl)ethyl)amino)-6,6-Bis(fluormethyl)-2,8-dimethyl-6,8-dihydro-7H-pyrrolo[3,2-g]chinazolin-7-on (Verbindung 92);
4-(((R)-1-(3-(1,1-Difluor-2-hydroxy-2-methylpropyl)-2-fluorphenyl)ethyl)amino)-6-ethyl-2,6,8-trimethyl-6,8-dihydro-7H-pyrrolo[3,2-g]chinazolin-7-on (Verbindung 93);
4-(((R)-1-(3-(1,1-Difluor-2-hydroxy-2-methylpropyl)-2-fluorphenyl)ethyl)amino)-6-(methoxymethyl)-2,6,8-trimethyl-6,8-dihydro-7H-pyrrolo[3,2-g]chinazolin-7-on (Verbindung 94);
4-(((R)-1-(3-(1,1-Difluor-2-hydroxy-2-methylpropyl)-2-fluorphenyl)ethyl)amino)-2,6,8-trimethyl-6,8-dihydro-7H-pyrrolo[3,2-g]chinazolin-7-on (Verbindung 95);
4-(((R)-1-(3-(1,1-Difluor-2-hydroxy-2-methylpropyl)-2-fluorphenyl)ethyl)amino)-6-methoxy-2,6,8-trimethyl-6,8-dihydro-7H-pyrrolo[3,2-g]chinazolin-7-on (Verbindung 96);
4-(((R)-1-(3-(1,1-Difluor-2-hydroxy-2-methylpropyl)-2-fluorphenyl)ethyl)amino)-6-hydroxy-2,6,8-trimethyl-6,8-dihydro-7H-pyrrolo[3,2-g]chinazolin-7-on (Verbindung 97);
4-(((R)-1-(3-(1,1-Difluor-2-hydroxy-2-methylpropyl)-2-fluorphenyl)ethyl)amino)-8-(methoxymethyl)-2,6,8-trimethyl-6,8-dihydro-7H-pyrrolo[2,3-g]chinazolin-7-on (Verbindung 98);
4-(((R)-1-(3-(1,1-Difluor-2-hydroxy-2-methylpropyl)-2-fluorphenyl)ethyl)amino)-2,6,8-trimethyl-6,8-dihydro-7H-pyrrolo[2,3-g]chinazolin-7-on (Verbindung 99);
4-(((R)-1-(3-(1,1-Difluor-2-hydroxy-2-methylpropyl)-2-fluorphenyl)ethyl)amino)-8-hydroxy-2,6,8-trimethyl-6,8-dihydro-7H-pyrrolo[2,3-g]chinazolin-7-on (Verbindung 100);
4-(((R)-1-(3-(1,1-Difluor-2-hydroxy-2-methylpropyl)-2-fluorphenyl)ethyl)amino)-8-methoxy-2,6,8-trimethyl-6,8-dihydro-7H-pyrrolo[2,3-g]chinazolin-7-on (Verbindung 101);
(S/R)-4-((R/S)-1-(3-((S/R)-1,1-Difluor-2,3-dihydroxy-2-methylpropyl)-2-fluorphenyl)ethyl)amino)-8-methoxy-2,6,8-trimethyl-6,8-dihydro-7H-pyrrolo[2,3-g]chinazolin-7-on (Verbindung 102);
(S/R)-4-((R/S)-1-(3-((R/S)-1,1-Difluor-2,3-dihydroxy-2-methylpropyl)-2-fluorphenyl)ethyl)amino)-8-methoxy-2,6,8-trimethyl-6,8-dihydro-7H-pyrrolo[2,3-g]chinazolin-7-on (Verbindung 103);
(R)-8-((1-(3-(1,1-Difluor-2-hydroxy-2-methylpropyl)-2-fluorphenyl)ethyl)amino)-1-ethyl-3,6-dimethyl-1,3-dihydro-2H-imidazo[4,5-g]chinazolin-2-on (Verbindung 104);
(R)-8-((1-(3-(1,1-Difluor-2-hydroxy-2-methylpropyl)-2-fluorphenyl)ethyl)amino)-1-isopropyl-3,6-dimethyl-1,3-dihydro-2H-imidazo[4,5-g]chinazolin-2-on (Verbindung 105);
(R)-8-((1-(3-(1,1-Difluor-2-hydroxy-2-methylpropyl)-2-fluorphenyl)ethyl)amino)-1-(2,2-Difluorethyl)-3,6-dimethyl-1H-imidazo[4,5-g]chinazolin-2(3H)-on (Verbindung 106);
(R)-1,1-Difluor-1-(2-fluor-3-(1-((2,2,3,6-tetramethyl-2,3-dihydro-1H-imidazo[4,5-g]chinazolin-8-yl)amino)ethyl)phenyl)-2-methylpropan-2-ol (Verbindung 107);
(R)-1,1-Difluor-1-(2-fluor-3-(1-((1,2,2,3,6-pentamethyl-2,3-dihydro-1H-imidazo[4,5-g]chinazolin-8-yl)amino)ethyl)phenyl)-2-methylpropan-2-ol (Verbindung 108);
(R)-8-((1-(3-(1,1-Difluor-2-hydroxy-2-methylpropyl)-2-fluorphenyl)ethyl)amino)-1,3,6-trimethyl-1,3-dihydro-2H-imidazo[4,5-g]chinazolin-2-on (Verbindung 109);
(R)-2-Cyclopropyl-4-((1-(3-(1,1-Difluor-2-hydroxy-2-methylpropyl)-2-fluorphenyl)ethyl)amino)-6,6,8-trimethyl-6,8-dihydro-7H-pyrrolo[3,2-g]chinazolin-7-on (Verbindung 110);
(R)-8-((1-(3-(1,1-Difluor-2-hydroxy-2-methylpropyl)-2-fluorphenyl)ethyl)amino)-1,6-dimethyloxazolo[4,5-g]chinazolin-2(1H)-on (Verbindung 111);
(R)-4-((1-(3-(1,1-Difluor-2-hydroxy-2-methylpropyl)-2-fluorphenyl)ethyl)amino)-2,6-dimethyl-6H-pyrrolo[2,3-g]chinazolin-7,8-dion (Verbindung 112);
(R)-4-((1-(3-(1,1-Difluor-2-hydroxy-2-methylpropyl)-2-fluorphenyl)ethyl)amino)-2,6-dimethyl-6,8-dihydro-7H-pyrrolo[2,3-g]chinazolin-7-on (Verbindung 113);
(R)-4-((1-(3-(1,1-Difluor-2-hydroxy-2-methylpropyl)-2-fluorphenyl)ethyl)amino)-2,6,6,7-Tetramethyl-6,7-dihydro-8H-pyrrolo[3,4-g]chinazolin-8-on (Verbindung 114);
4-((1-(2-Fluor-3-(1-(hydroxymethyl)cyclopropyl)phenyl)ethyl)amino)-2,6,8,8-Tetramethyl-6,8-dihydro-7H-pyrrolo[2,3-g]chinazolin-7-on (Verbindung 115);
4-(((R)-1-(3-(1,1-Difluor-2-hydroxy-2-methylpropyl)-2-fluorphenyl)ethyl)amino)-8-Fluor-2,6,8-trimethyl-6,8-dihydro-7H-pyrrolo[2,3-g]chinazolin-7-on (Verbindung 116);
(R)-4-((1-(3-(1,1-Difluor-2-hydroxy-2-methylpropyl)-2-fluorphenyl)ethyl)amino)-8,8-Difluor-2,6-dimethyl-6,8-dihydro-7H-pyrrolo[2,3-g]chinazolin-7-on (Verbindung 117);
(R)-4-((1-(3-(1,1-Difluor-2-hydroxy-2-methylpropyl)-2-fluorphenyl)ethyl)amino)-2,7,8,8-Tetramethyl-7,8-dihydro-6H-pyrrolo[3,4-g]chinazolin-6-on (Verbindung 118);
(R)-8-((1-(3-(1,1-Difluor-2-hydroxy-2-methylpropyl)-2-fluorphenyl)ethyl)amino)-3,6-Dimethyl-1,3-dihydro-2H-imidazo[4,5-g]chinazolin-2-on (Verbindung 119);
(S)-4-(((S)-1-(3-(1,1-Difluor-2-hydroxy-2-methylpropyl)-2-fluorphenyl)ethyl)amino)-8-Methoxy-2,6,8-trimethyl-6,8-dihydro-7H-pyrrolo[2,3-g]chinazolin-7-on (Verbindung 120);
4-(((R)-1-(3-(1,1-Difluor-2-hydroxy-2-methylpropyl)-2-fluorphenyl)ethyl)amino)-8-Ethoxy-2,6,8-trimethyl-6,8-dihydro-7H-pyrrolo[2,3-g]chinazolin-7-on (Verbindung 121);
(R)-4'-((1-(3-(1,1-Difluor-2-hydroxy-2-methylpropyl)-2-fluorphenyl)ethyl)amino)-2',6'-Dimethyl-2,3,5,6-tetrahydrospiro[pyran-4,8'-pyrrolo[2,3-g]chinazolin]-7'(6'H)-on (Verbindung 122);
4-(((R)-1-(3-(1,1-Difluor-2-hydroxy-2-methylpropyl)-2-fluorphenyl)ethyl)amino)-8-(2-Methoxyethyl)-2,6,8-trimethyl-6,8-dihydro-7H-pyrrolo[2,3-g]chinazolin-7-on (Verbindung 123);
4'-(((R)-1-(3-(1,1-Difluor-2-hydroxy-2-methylpropyl)-2-fluorphenyl)ethyl)amino)-2',3,6'-Trimethylspiro[oxazolidin-5,8'-pyrrolo[2,3-g]chinazolin]-2,7'(6'H)-dion (Verbindung 124);
4-(((R)-1-(3-(1,1-Difluor-2-hydroxy-2-methylpropyl)-2-fluorphenyl)ethyl)amino)-8-Methoxy-2,6-dimethyl-8-(trifluormethyl)-6,8-dihydro-7H-pyrrolo[2,3-g]chinazolin-7-on (Verbindung 125);
4-(((R)-1-(3-(1,1-Difluor-2-hydroxy-2-methylpropyl)-2-fluorphenyl)ethyl)amino)-8-Ethyl-8-methoxy-2,6-dimethyl-6,8-dihydro-7H-pyrrolo[2,3-g]chinazolin-7-on (Verbindung 126);
4-(((1R)-1-(3-(1,1-Difluor-2-hydroxy-3-methoxy-2-methylpropyl)-2-fluorphenyl)ethyl)amino)-2,6,8,8-Tetramethyl-6,8-dihydro-7H-pyrrolo[2,3-g]chinazolin-7-on (Verbindung 127);
4'-(((R)-1-(3-(1,1-Difluor-2-hydroxy-2-methylpropyl)-2-fluorphenyl)ethyl)amino)-2',6'-Dimethyl-4,5-dihydro-2H-spiro[phuran-3,8'-pyrrolo[2,3-g]chinazolin]-7'(6'H)-on (Verbindung 128);
4-(((1R)-1-(3-(3-(Dimethylamino)-1,1-difluor-2-hydroxy-2-methylpropyl)-2-fluorphenyl)ethyl)amino)-2,6,8,8-Tetramethyl-6,8-dihydro-7H-pyrrolo[2,3-g]chinazolin-7-on (Verbindung 129);
4-(((1R)-1-(3-(1,1-Difluor-2-hydroxy-2-methyl-3-(methylamino)propyl)-2-fluorphenyl)ethyl)amino)-2,6,8,8-Tetramethyl-6,8-dihydro-7H-pyrrolo[2,3-g]chinazolin-7-on (Verbindung 130);
4-(((R)-1-(3-Amino-5-(trifluormethyl)phenyl)ethyl)amino)-8-Methoxy-2,6,8-trimethyl-6,8-dihydro-7H-pyrrolo [2,3-g]chinazolin-7-on (Verbindung 131);
4-(((R)-1-(3-(1,1-Difluor-2-hydroxy-2-methylpropyl)-2-fluorphenyl)ethyl)amino)-8-Methoxy-8-(2-Methoxyethyl)-2,6-dimethyl-6,8-dihydro-7H-pyrrolo[2,3-g]chinazolin-7-on (Verbindung 132);
4-(((1R)-1-(2-Fluor-3-(piperidin-3-yl)phenyl)ethyl)amino)-2,6,8,8-Tetramethyl-6,8-dihydro-7H-pyrrolo[2,3-g] chinazolin-7-on (Verbindung 133);
(R)-4-((1-(2-Fluor-3-(trifluormethyl)phenyl)ethyl)amino)-2,6,8,8-Tetramethyl-6,8-dihydro-7H-pyrrolo[2,3-g]chinazolin-7-on (Verbindung 134);
4-(((1R)-1-(3-(3-(Dimethylamino)-1,1-difluor-2-hydroxy-2-methylpropyl)-2-fluorphenyl)ethyl)amino)-8-Methoxy-2,6,8-trimethyl-6,8-dihydro-7H-pyrrolo[2,3-g]chinazolin-7-on (Verbindung 135);
2,6,8,8-Tetramethyl-4-((1-(3-(trifluormethyl)phenyl)ethyl)amino)-6,8-dihydro-7H-pyrrolo[2,3-g]chinazolin-7-on (Verbindung 136);
4-(((R)-1-(3-Amino-5-(trifluormethyl)phenyl)ethyl)amino)-6-Methoxy-2,6,8-trimethyl-6,8-dihydro-7H-pyrrolo [3,2-g]chinazolin-7-on (Verbindung 137);
(R)-4'-((1-(3-Amino-5-(trifluormethyl)phenyl)ethyl)amino)-2',6'-Dimethyl-2,3,5,6-tetrahydrospiro[pyran-4,8'-pyrrolo[2,3-g]chinazolin]-7'(6'H)-on (Verbindung 138);
4-(((R)-1-(3-Amino-5-(trifluormethyl)phenyl)ethyl)amino)-8-Ethyl-8-methoxy-2,6-dimethyl-6,8-dihydro-7H-pyrrolo[2,3-g]chinazolin-7-on (Verbindung 139).

**3.** Eine pharmazeutische Zusammensetzung, umfassend eine Verbindung der Formel (I) oder ein pharmakologisch akzeptables Salz davon, wie in den Ansprüchen 1 und 2 beansprucht, und einen pharmakologisch akzeptablen Träger.

**4.** Eine Verbindung der Formel (I) und ein pharmakologisch akzeptables Salz davon, wie in den Ansprüchen 1 und 2 beansprucht, zur Verwendung bei der Behandlung und/oder Vorbeugung einer Krankheit durch Hemmung von SOS1 in einem Subjekt, umfassend die Verabreichung einer therapeutisch wirksamen Menge der genannten Verbindung an das Subjekt, wobei die Krankheit ausgewählt ist aus Krebs, akuter Staphylococcus-aureus-Infektion (pädiatrische

Patienten), akutem Atemnotsyndrom / akuter Lungenschädigung, Sepsis und RASopathie.

**5.** Eine Verbindung der Formel (I) und ein pharmakologisch akzeptables Salz davon, wie in den Ansprüchen 1 und 2 beansprucht, zur Verwendung bei der Behandlung und/oder Vorbeugung einer Krankheit durch Hemmung der Interaktion von SOS1 und einem Protein der RAS-Familie in einem Subjekt, umfassend die Verabreichung einer therapeutisch wirksamen Menge der genannten Verbindung an das Subjekt, wobei die Krankheit ausgewählt ist aus Krebs, akuter Staphylococcus-aureus-Infektion (pädiatrische Patienten), akutem Atemnotsyndrom / akuter Lungenschädigung, Sepsis und RASopathie.

**6.** Die Verbindung zur Verwendung bei der Behandlung und/oder Vorbeugung von Krebs wie in Anspruch 4 oder 5 beansprucht, wobei der Krebs ausgewählt ist aus der Gruppe bestehend aus Bauchspeicheldrüsenkrebs, Lungenkrebs, kolorektalem Krebs, Klasse-3-BRAF-Mutanten-Krebsarten, hämatologischem Krebs, Cholangiokarzinom, multiplem Myelom, Melanom, Gebärmutterkrebs, Endometriumkarzinom, Schilddrüsenkrebs, akuter myeloischer Leukämie, Blasenkrebs, Urothelkarzinom, Magenkrebs, Gebärmutterhalskrebs, Plattenepithelkarzinom des Kopf-Hals-Bereichs, diffus großzelligem B-Zell-Lymphom, Speiseröhrenkrebs, chronischer lymphatischer Leukämie, hepatozellulärem Krebs, Brustkrebs, Eierstockkrebs, Prostatakrebs, Glioblastom, Nierenkrebs, rein mukosales Neurinomsyndrom, fibrösem Epulis und Sarkomen.

**7.** Die Verbindung zur Verwendung bei der Behandlung und/oder Vorbeugung einer Krankheit wie in Anspruch 4 oder 5 beansprucht, wobei die RASopathie ausgewählt ist aus der Gruppe bestehend aus Neurofibromatose Typ 1 (NF1), Noonan-Syndrom (NS), Noonan-Syndrom mit multiplen Lentigines (NSML), Kapillarmalformation-Arteriovenöse Malformation Syndrom (CM-AVM), Costello-Syndrom (CS), Cardio-Facio-Cutaneous-Syndrom (CFC), Legius-Syndrom, Noonan-ähnlichem / mit multiplen Riesenzellläsionen Syndrom und hereditärer gingivaler Fibromatose (HGF).

**8.** Die Verbindung der Formel (I) und ein pharmakologisch akzeptables Salz davon, wie in den Ansprüchen 1 und 2 beansprucht, zur Verwendung bei der Behandlung und/oder Vorbeugung von Krebs, wobei die genannte Verbindung zusammen mit mindestens einer weiteren pharmakologisch aktiven Substanz verabreicht wird.

**9.** Die Verbindung der Formel (I) und ein pharmakologisch akzeptables Salz davon, wie in den Ansprüchen 1 und 2 beansprucht, zur Verwendung bei der Behandlung und/oder Vorbeugung von Krebs, wobei die Verbindung vor, nach oder gleichzeitig mit mindestens einer weiteren pharmakologisch aktiven Substanz verabreicht wird.

## Revendications

**1.** Un composé de formule (I) et un sel pharmaceutiquement acceptable de celui-ci,

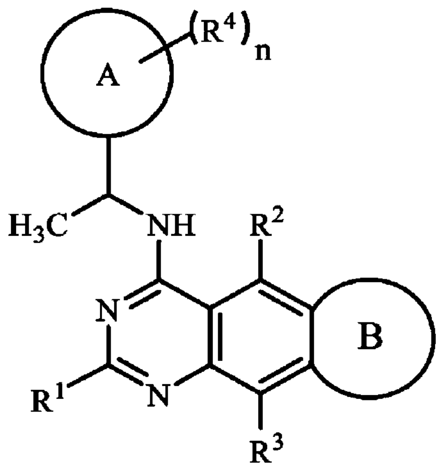

(I)

où :

le cycle A est choisi parmi le phényl et

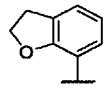 ;

le cycle B est choisi parmi

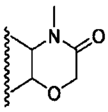, 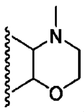, 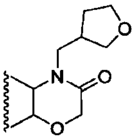, 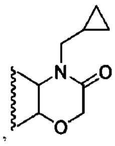, 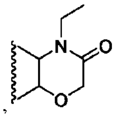, 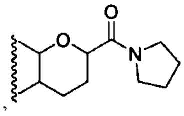, 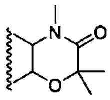,

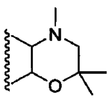, 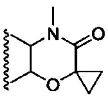, 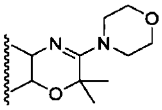, 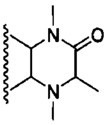, 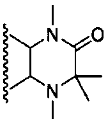, 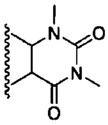, 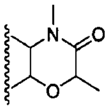,

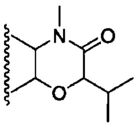, 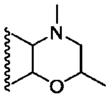, 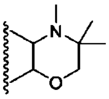, 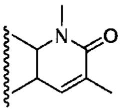, 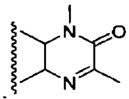, 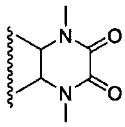,

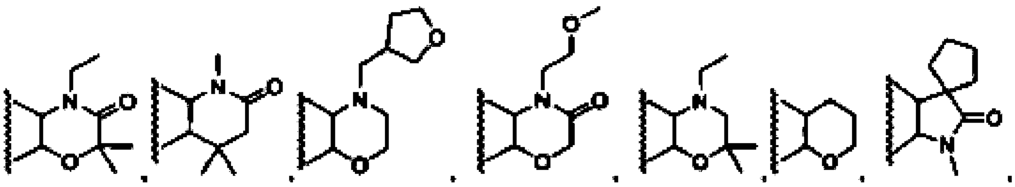

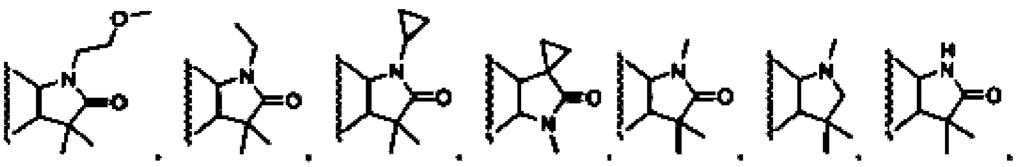

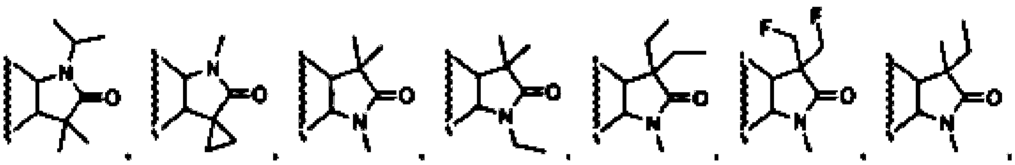

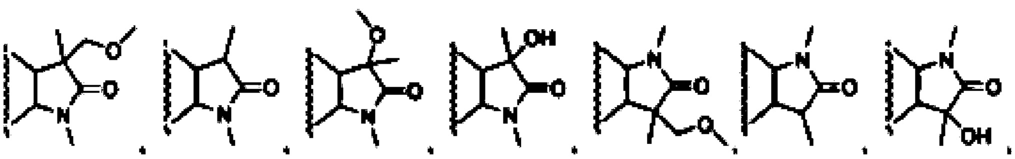

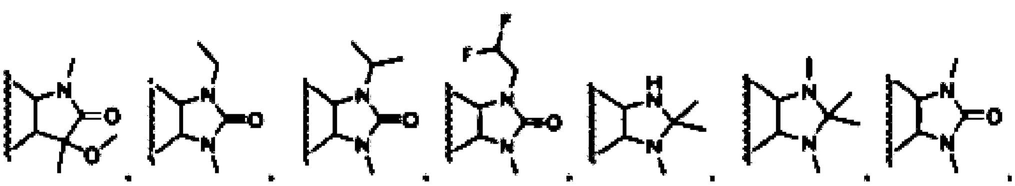

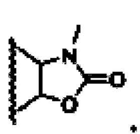, 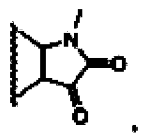, 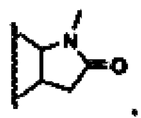, 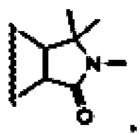, 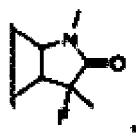, 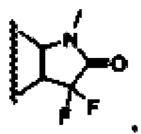, 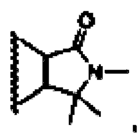,

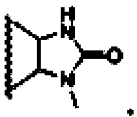, 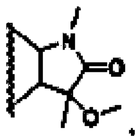, 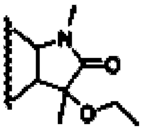, 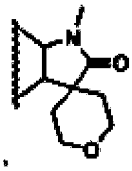, 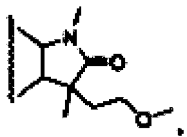, 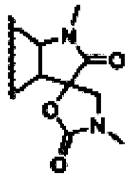, 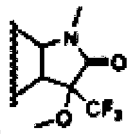,

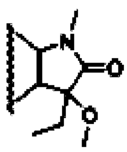, 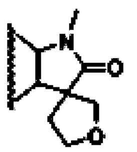 e 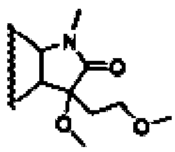.

$R^1$ est choisi parmi le méthyle, l'éthyle, l'isopropyle et le cyclopropyle :
$R^2$ et $R^3$ sont indépendamment choisis parmi l'hydrogène, le fluor et le méthyle ;
$R^4$ est choisi parmi le fluor, $-NH_2$, $-CH_3$, $-CF_3$, $-CHF_2$

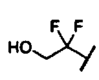, 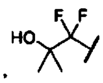, 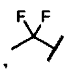, 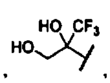,

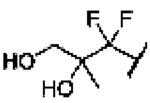, 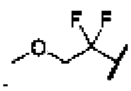, 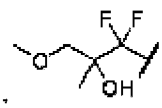, 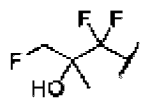, 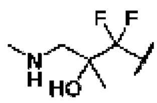, 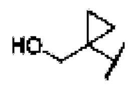,

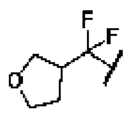, 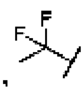, 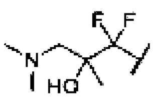, e 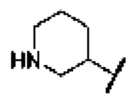 ; e

« n » est un entier choisi parmi 1, 2 et 3.

**2.** Le composé de formule (I) et un sel pharmaceutiquement acceptable de celui-ci, tel que revendiqué dans la revendication 1, dans lequel le composé est choisi parmi :

(R)-4-((1-(3-amino-5-(trifluorométhyl)phényl)éthyl)amino)-2,6-diméthyl-6H-[1,4]oxazino[3,2-g]quinazolin-7(8H)-one (Composé 1) ;
(R)-4-((1-(3-(1,1-difluoro-2-hydroxyéthyl)-2-fluorophényl)éthyl)amino)-2,6-diméthyl-6H-[1,4]oxazino[3,2-g]quinazolin-7(8H)-one (Composé 2) ;
(R)-4-((1-(3-(1,1-difluoro-2-hydroxy-2-méthylpropyl)-2-fluorophényl)éthyl)amino)-2,6-diméthyl-6H-[1,4]oxazino[3,2-g]quinazolin-7(8H)-one (Composé 3) ;
4-((1-(3-(1,1-difluoroéthyl)-2-fluorophényl)éthyl)amino)-2,6-diméthyl-6H-[1,4]oxazino[3,2-g]quinazolin-7(8H)-one (Composé 4) ;
4-((1-(3-amino-5-(difluorométhyl)phényl)éthyl)amino)-2,6-diméthyl-6H-[1,4]oxazino[3,2-g]quinazolin-7(8H)-one (Composé 5) ;
4-((1-(3-(1,1-difluoro-2-hydroxyéthyl)-2-méthylphényl)éthyl)amino)-2,6-diméthyl-6H-[1,4]oxazino[3,2-g]quinazolin-7(8H)-one (Composé 6) ;
2,6-diméthyl-4-((1-(3-(trifluorométhyl)phényl)éthyl)amino)-6H-[1,4]oxazino[3,2-g]quinazolin-7(8H)-one (Composé 7) ;
4-((1-(2-fluoro-3-(trifluorométhyl)phényl)éthyl)amino)-2,6-diméthyl-6H-[1,4]oxazino[3,2-g]quinazolin-7(8H)-one (Composé 8) ;
4-((1-(3,3-difluoro-2,3-dihydrobenzofuranne-7-yl)éthyl)amino)-2,6-diméthyl-6H-[1,4]oxazino[3,2-g]quinazolin-7(8H)-one (Composé 9) ;
(R)-N-(1-(3-amino-5-(trifluorométhyl)phényl)éthyl)-2,6-diméthyl-7,8-dihydro-6H-[1,4]oxazino[3,2-g]quinazolin-4-amine (Composé 10) ;
4-(((R)-1-(3-amino-5-(trifluorométhyl)phényl)éthyl)amino)-2-méthyl-6-((tétrahydrofuranne-3-yl)méthyl)-6H-[1,4]oxazino[3,2-g]quinazolin-7(8H)-one (Composé 11) ;
(R)-4-((1-(3-amino-5-(trifluorométhyl)phényl)éthyl)amino)-6-(cyclopropylméthyl)-2-méthyl-6H-[1,4]oxazino[3,2-g]quinazolin-7(8H)-one (Composé 12) ;
(R)-4-((1-(3-(1,1-difluoro-2-hydroxyéthyl)-2-fluorophényl)éthyl)amino)-6-éthyl-2-méthyl-6H-[1,4]oxazino[3,2-g]quinazolin-7(8H)-one (Composé 13) ;
(R)-4-((1-(3-amino-5-(trifluorométhyl)phényl)éthyl)amino)-2-éthyl-6-méthyl-6H-[1,4]oxazino[3,2-g]quinazolin-7(8H)-one (Composé 14) ;
(R)-4-((1-(3-(1,1-difluoro-2-hydroxyéthyl)-2-fluorophényl)éthyl)amino)-2-éthyl-6-méthyl-6H-[1,4]oxazino[3,2-g]quinazolin-7(8H)-one (Composé 15) ;
(R)-2-cyclopropyl-4-((1-(3-(1,1-difluoro-2-hydroxyéthyl)-2-fluorophényl)éthyl)amino)-6-méthyl-6H-[1,4]oxazino[3,2-g]quinazolin-7(8H)-one (Composé 16) ;
(4-(((R)-1-(3-amino-5-(trifluorométhyl)phényl)éthyl)amino)-2-méthyl-8,9-dihydro-7H-pyrano[2,3-g]quinazolin-7-yl)(pyrrolidin-1-yl)méthanone (Composé 17) ;
(R)-4-((1-(3-(1,1-difluoro-2-hydroxyéthyl)-2-fluorophényl)éthyl)amino)-2,6,8,8-tétraméthyl-6H-[1,4]oxazino[3,2-g]quinazolin-7(8H)-one (Composé 18) ;
2,6,8,8-tétraméthyl-4-((1-(3-(trifluorométhyl)phényl)éthyl)amino)-6H-[1,4]oxazino[3,2-g]quinazolin-7(8H)-one (Composé 19) ;
4-((1-(2-fluoro-3-(trifluorométhyl)phényl)éthyl)amino)-2,6,8,8-tétraméthyl-6H-[1,4]oxazino[3,2-g]quinazolin-7(8H)-one (Composé 20) ;
(R)-4-((1-(3-amino-5-(trifluorométhyl)phényl)éthyl)amino)-2,6,8,8-tétraméthyl-6H-[1,4]oxazino[3,2-g]quinazolin-7(8H)-one (Composé 21) ;
4-((1-(2-fluoro-3-(1,1,1-trifluoro-2,3-dihydroxypropan-2-yl)phényl)éthyl)amino)-2,6,8,8-tétraméthyl-6H-[1,4]oxazino[3,2-g]quinazolin-7(8H)-one (Composé 22) ;
4-((1-(3-(1,1-difluoro-2,3-dihydroxy-2-méthylpropyl)-2-fluorophényl)éthyl)amino)-2,6,8,8-tétraméthyl-6H-[1,4]oxazino[3,2-g]quinazolin-7(8H)-one (Composé 23) ;
(R)-N-(1-(3-amino-5-(trifluorométhyl)phényl)éthyl)-2,6,8,8-tétraméthyl-7,8-dihydro-6H-[1,4]oxazino[3,2-g]quinazolin-4-amine (Composé 24) ;
(R)-4'-((1-(3-(1,1-difluoro-2-hydroxyéthyl)-2-fluorophényl)éthyl)amino)-2',6'-diméthylspiro[cyclopropane-1,8'-[1,4]oxazino[3,2-g]quinazolin]-7'(6'H)-one (Composé 25);
(R)-1,1-difluoro-1-(2-fluoro-3-(1-((2,8,8-triméthyl-7-morpholino-8H-[1,4]oxazino[3,2-g]quinazolin-4-yl)amino)éthyl)phényl)-2-méthylpropan-2-ol (Composé 26) ;
4-(((R)-1-(3-(1,1-difluoro-2-hydroxy-2-méthylpropyl)-2-fluorophényl)éthyl)amino)-2,6,8,9-tétraméthyl-8,9-dihydropyrazino[2,3-g]quinazolin-7(6H)-one (Composé 27) ;
(R)-4-((1-(3-(1,1-difluoro-2-hydroxy-2-méthylpropyl)-2-fluorophényl)éthyl)amino)-2,6,8,8,9-pentaméthyl-8,9-dihydropyrazino[2,3-g]quinazolin-7(6H)-one (Composé 28) ;
(R)-9-((1-(3-(1,1-difluoro-2-hydroxy-2-méthylpropyl)-2-fluorophényl)éthyl)amino)-1,3,7-triméthylpyrimido[4,5-g]quinazoline-2,4(1H,3H)-dione (Composé 29) ;
4-(((R)-1-(3-(1,1-difluoro-2-hydroxyéthyl)-2-fluorophényl)éthyl)amino)-2,6,8-triméthyl-6H-[1,4]oxazino[3,2-g]quinazolin-7(8H)-one (Composé 30) ;
4-(((R)-1-(3-(1,1-difluoro-2-hydroxy-2-méthylpropyl)-2-fluorophényl)éthyl)amino)-2,6,8-triméthyl-6H-[1,4]oxazino[3,2-g]quinazolin-7(8H)-one (Composé 31) ;
4-(((R)-1-(3-(1,1-difluoro-2-hydroxy-2-méthylpropyl)-2-fluorophényl)éthyl)amino)-8-isopropyl-2,6-diméthyl-6H-[1,4]oxazino[3,2-g]quinazolin-7(8H)-one (Composé 32) ;
(R)-4-((1-(3-(1,1-difluoro-2-hydroxy-2-méthylpropyl)-2-fluorophényl)éthyl)amino)-2,6,8,8-tétraméthyl-6H-[1,4]oxazino[3,2-g]quinazolin-7(8H)-one (Composé 33) ;
(R)-1,1-difluoro-1-(2-fluoro-3-(1-((2,6,8,8-tétraméthyl-7,8-dihydro-6H-[1,4]oxazino[3,2-g]quinazolin-4-yl)amino)éthyl)phényl)-2-méthylpropan-2-ol (Composé 34) ;
(R)-2,2-difluoro-2-(2-fluoro-3-(1-((2,6,8,8-tétraméthyl-7,8-dihydro-6H-[1,4]oxazino[3,2-g]quinazolin-4-yl)amino)éthyl)phényl)éthan-1-ol (Composé 35) ;
2,2-difluoro-2-(2-fluoro-3-((1R)-1-((2,6,8-triméthyl-7,8-dihydro-6H-[1,4]oxazino[3,2-g]quinazolin-4-yl)amino)éthyl)phényl)éthan-1-ol (Composé 36) ;
(R)-1,1-difluoro-1-(2-fluoro-3-(1-((2,6,7,7-tétraméthyl-7,8-dihydro-6H-[1,4]oxazino[3,2-g]quinazolin-4-yl)amino)éthyl)phényl)-2-méthylpropan-2-ol (Composé 37) ;
(R)-4-((1-(3-(1,1-difluoro-2-hydroxy-2-méthylpropyl)-2-fluorophényl)éthyl)amino)-2,6,8-triméthylpyrido[2,3-g]quinazolin-7(6H)-one (Composé 38) ;
(R)-4-((1-(3-(1,1-difluoro-2-hydroxy-2-méthylpropyl)-2-fluorophényl)éthyl)amino)-2,6,8-triméthylpyrazino[2,3-g]quinazolin-7(6H)-one (Composé 39) ;
(R)-4-((1-(3-(1,1-difluoro-2-hydroxy-2-méthylpropyl)-2-fluorophényl)éthyl)amino)-2,6,9-triméthyl-6,9-dihydropyrazino[2,3-g]quinazoline-7,8-dione (Composé 40) ;
(R)-4-((1-(3-(1,1-difluoro-2-hydroxy-2-méthylpropyl)-2-fluorophényl)éthyl)amino)-6-éthyl-2,8,8-triméthyl-6H-[1,4]oxazino[3,2-g]quinazolin-7(8H)-one (Composé 41) ;
(R)-4-((1-(3-(1,1-difluoro-2-hydroxy-2-méthylpropyl)-2-fluorophényl)éthyl)amino)-2,6,9,9-tétraméthyl-8,9-dihydropyrido[2,3-g]quinazolin-7(6H)-one (Composé 42) ;
N-((R)-1-(3-amino-5-(trifluorométhyl)phényl)éthyl)-2-méthyl-6-(((R/S)-tétrahydrofuranne-3-yl)méthyl)-7,8-dihydro-6H-[1,4]oxazino[3,2-g]quinazolin-4-amine (Composé 43) ;
N-((R)-1-(3-amino-5-(trifluorométhyl)phényl)éthyl)-2-méthyl-6-(((S/R)-tétrahydrofuranne-3-yl)méthyl)-7,8-dihydro-6H-[1,4]oxazino[3,2-g]quinazolin-4-amine (Composé 44) ;
(R)-1-(3-(1-((2,6-diméthyl-7,8-dihydro-6H-[1,4]oxazino[3,2-g]quinazolin-4-yl)amino)éthyl)-2-fluorophényl)-1,1-difluoro-2-méthylpropan-2-ol (Composé 45) ;
4-(((R)-1-(3-amino-5-(trifluorométhyl)phényl)éthyl)amino)-2,6,8-triméthyl-6H-[1,4]oxazino[3,2-g]quinazolin-7(8H)-one (Composé 46) ;
(R)-4-((1-(3-(1,1-difluoro-2-hydroxy-2-méthylpropyl)-2-fluorophényl)éthyl)amino)-10-fluoro-2,6-diméthyl-6H-[1,4]oxazino[3,2-g]quinazolin-7(8H)-one (Composé 47) ;
(R)-4-((1-(3-(1,1-difluoro-2-hydroxyéthyl)-2-fluorophényl)éthyl)amino)-6-(2-méthoxyéthyl)-2-méthyl-6H-[1,4]oxazino[3,2-g]quinazolin-7(8H)-one (Composé 48) ;
(R)-4-((1-(3-amino-5-(trifluorométhyl)phényl)éthyl)amino)-6-éthyl-2-méthyl-6H-[1,4]oxazino[3,2-g]quinazolin-7(8H)-one (Composé 49) ;
(R)-1-(3-(1-((6-éthyl-2,8,8-triméthyl-7,8-dihydro-6H-[1,4]oxazino[3,2-g]quinazolin-4-yl)amino)éthyl)-2-fluorophényl)-1,1-difluoro-2-méthylpropan-2-ol (Composé 50) ;
(R)-4-((1-(3-(1,1-difluoro-2-méthoxyéthyl)-2-fluorophényl)éthyl)amino)-2,6,8,8-tétraméthyl-6H-[1,4]oxazino[3,2-g]quinazolin-7(8H)-one (Composé 51) ;
(R)-N-(1-(3-(1,1-difluoro-2-méthoxyéthyl)-2-fluorophényl)éthyl)-2,6,8,8-tétraméthyl-7,8-dihydro-6H-[1,4]oxazino[3,2-g]quinazolin-4-amine (Composé 52) ;
4-(((1R)-1-(3-(1,1-difluoro-2-hydroxy-3-méthoxy-2-méthylpropyl)-2-fluorophényl)éthyl)amino)-2,6,8,8-tétraméthyl-6H-[1,4]oxazino[3,2-g]quinazolin-7(8H)-one (Composé 53) ;
4-(((1R)-1-(2-fluoro-3-(1,1,3-trifluoro-2-hydroxy-2-méthylpropyl)phényl)éthyl)amino)-2,6,8,8-tétraméthyl-6H-[1,4]oxazino[3,2-g]quinazolin-7(8H)-one (Composé 54) ;
4-(((1R)-1-(3-(1,1-difluoro-2-hydroxy-2-méthyl-3-(méthylamino)propyl)-2-fluorophényl)éthyl)amino)-2,6,8,8-tétraméthyl-6H-[1,4]oxazino[3,2-g]quinazolin-7(8H)-one (Composé 55) ;
(R)-2,2-difluoro-2-(2-fluoro-3-(1-((2-méthyl-7,8-dihydro-6H-pyrano[3,2-g]quinazolin-4-yl)amino)éthyl)phényl)éthan-1-ol (Composé 56) ;
(R)-4-((1-(3-(1,1-difluoro-2-hydroxy-2-méthylpropyl)phényl)éthyl)amino)-2,6,8,8-tétraméthyl-6H-[1,4]oxazino[3,2-g]quinazolin-7(8H)-one (Composé 57) ;
(R)-4'-((1-(3-(1,1-difluoro-2-hydroxy-2-méthylpropyl)-2-fluorophényl)éthyl)amino)-2',8'-diméthylspiro[cyclopentane-1,6'-pyrrolo[3,2-g]quinazolin]-7'(8'H)-one (Composé 58) ;
4'-((1-(2-fluoro-3-(trifluorométhyl)phényl)éthyl)amino)-2',8'-diméthylspiro[cyclopentane-1,6'-pyrrolo[3,2-g]quinazolin]-7'(8'H)-one (Composé 59) ;
2',8'-diméthyl-4'-((1-(2-méthyl-3-(trifluorométhyl)phényl)éthyl)amino)spiro[cyclopentane-1,6'-pyrrolo[3,2-g]quinazolin]-7'(8'H)-one (Composé 60) ;
4'-((1-(3-(difluorométhyl)-2-méthylphényl)éthyl)amino)-2',8'-diméthylspiro[cyclopentane-1,6'-pyrrolo[3,2-g]quinazolin]-7'(8'H)-one (Composé 61) ;
(R)-4'-((1-(3-(1,1-difluoro-2-hydroxyéthyl)-2-fluorophényl)éthyl)amino)-2',8'-diméthylspiro[cyclopentane-1,6'-pyrrolo[3,2-g]quinazolin]-7'(8'H)-one (Composé 62) ;
(R)-4-((1-(3-(1,1-difluoro-2-hydroxy-2-méthylpropyl)-2-fluorophényl)éthyl)amino)-6-(2-méthoxyéthyl)-2,8,8-triméthyl-6,8-dihydro-7H-pyrrolo[2,3-g]quinazolin-7-one (Composé 63) ;
(R)-4-((1-(3-(1,1-difluoro-2-hydroxy-2-méthylpropyl)-2-fluorophényl)éthyl)amino)-6-éthyl-2,8,8-triméthyl-6,8-dihydro-7H-pyrrolo[2,3-g]quinazolin-7-one (Composé 64) ;
(R)-6-cyclopropyl-4-((1-(3-(1,1-difluoro-2-hydroxy-2-méthylpropyl)-2-fluorophényl)éthyl)amino)-2,8,8-triméthyl-6,8-dihydro-7H-pyrrolo[2,3-g]quinazolin-7-one (Composé 65) ;
(R)-4'-((1-(3-(1,1-difluoro-2-hydroxyéthyl)-2-fluorophényl)éthyl)amino)-2',8'-diméthylspiro[cyclopropane-1,6'-pyrrolo[3,2-g]quinazolin]-7'(8'H)-one (Composé 66) ;
(R)-4'-((1-(3-(1,1-difluoro-2-hydroxy-2-méthylpropyl)-2-fluorophényl)éthyl)amino)-2',8'-diméthylspiro[cyclopropane-1,6'-pyrrolo[3,2-g]quinazolin]-7'(8'H)-one (Composé 67) ;
(R)-4-((1-(3-(1,1-difluoro-2-hydroxy-2-méthylpropyl)-2-fluorophényl)éthyl)amino)-2,6,8,8-tétraméthyl-6,8-dihydro-7H-pyrrolo[2,3-g]quinazolin-7-one (Composé 68) ;
4-((1-(3-amino-5-(difluorométhyl)phényl)éthyl)amino)-2,6,8,8-tétraméthyl-6,8-dihydro-7H-pyrrolo[2,3-g]quinazolin-7-one (Composé 69) ;
(R)-4-((1-(3-amino-5-(trifluorométhyl)phényl)éthyl)amino)-2,6,8,8-tétraméthyl-6H-pyrrolo[2,3-g]quinazolin-7(8H)-one (Composé 70) ;
(S)-4-((1-(3-(1,1-difluoro-2-hydroxy-2-méthylpropyl)-2-fluorophényl)éthyl)amino)-2,6,8,8-tétraméthyl-6H-pyrrolo[2,3-g]quinazolin-7(8H)-one (Composé 71) ;
(R)-4-((1-(3-(1,1-difluoro-2-hydroxyéthyl)-2-fluorophényl)éthyl)amino)-2,6,8,8-tétraméthyl-6,8-dihydro-7H-pyrrolo[2,3-g]quinazolin-7-one (Composé 72) ;
4-((1-(3-(1,1-difluoro-2-hydroxy-2-méthylpropyl)phényl)éthyl)amino)-2,6,8,8-tétraméthyl-6,8-dihydro-7H-pyrrolo[2,3-g]quinazolin-7-one (Composé 73) ;
4-((1-(2-fluoro-3-(1,1,1-trifluoro-2,3-dihydroxypropan-2-yl)phényl)éthyl)amino)-2,6,8,8-tétraméthyl-6,8-dihydro-7H-pyrrolo[2,3-g]quinazolin-7-one (Composé 74) ;
(R)-1,1-difluoro-1-(2-fluoro-3-(1-((2,6,8,8-tétraméthyl-7,8-dihydro-6H-pyrrolo[2,3-g]quinazolin-4-yl)amino) éthyl)phényl)-2-méthylpropan-2-ol (Composé 75) ;
4-(((1R)-1-(3-(1,1-difluoro-2,3-dihydroxy-2-méthylpropyl)-2-fluorophényl)éthyl)amino)-2,6,8,8-tétraméthyl-6,8-dihydro-7H-pyrrolo[2,3-g]quinazolin-7-one (Composé 76) ;
(R)-4-((1-(3-(1,1-difluoro-2-hydroxy-2-méthylpropyl)-2-fluorophényl)éthyl)amino)-2,8,8-triméthyl-6H-pyrrolo [2,3-g]quinazolin-7(8H)-one (Composé 77) ;
(R)-4-((1-(3-(1,1-difluoro-2-hydroxy-2-méthylpropyl)-2-fluorophényl)éthyl)amino)-6-isopropyl-2,8,8-triméthyl-6H-pyrrolo[2,3-g]quinazolin-7(8H)-one (Composé 78) ;
(R)-4'-((1-(3-(1,1-difluoro-2-hydroxy-2-méthylpropyl)-2-fluorophényl)éthyl)amino)-2',6'-diméthylspiro[cyclopropane-1,8'-pyrrolo[2,3-g]quinazolin]-7'(6'H)-one (Composé 79) ;
(R)-4-((1-(3-(1,1-difluoro-2-hydroxyéthyl)-2-fluorophényl)éthyl)amino)-2,6,6,8-tétraméthyl-6,8-dihydro-7H-pyrrolo[3,2-g]quinazolin-7-one (Composé 80) ;
(R)-4-((1-(3-(1,1-difluoro-2-hydroxy-2-méthylpropyl)-2-fluorophényl)éthyl)amino)-2,6,6,8-tétraméthyl-6,8-dihydro-7H-pyrrolo[3,2-g]quinazolin-7-one (Composé 81) ;
4-((1-(2-fluoro-3-(trifluorométhyl)phényl)éthyl)amino)-2,6,6,8-tétraméthyl-6,8-dihydro-7H-pyrrolo[3,2-g]quinazolin-7-one (Composé 82) ;
2,6,6,8-tétraméthyl-4-((1-(2-méthyl-3-(trifluorométhyl)phényl)éthyl)amino)-6,8-dihydro-7H-pyrrolo[3,2-g]quinazolin-7-one (Composé 83) ;
4-((1-(3-(1,1-difluoroéthyl)-2-fluorophényl)éthyl)amino)-2,6,6,8-tétraméthyl-6,8-dihydro-7H-pyrrolo[3,2-g]quinazolin-7-one (Composé 84) ;
4-((1-(3-(difluoro(tétrahydrofuranne-3-yl)méthyl)-2-fluorophényl)éthyl)amino)-2,6,6,8-tétraméthyl-6,8-dihydro-7H-pyrrolo[3,2-g]quinazolin-7-one (Composé 85) ;
(R)-4-((1-(3-(1,1-difluoro-2-hydroxy-2-méthylpropyl)-2-fluorophényl)éthyl)amino)-2-éthyl-6,6,8-triméthyl-6,8-dihydro-7H-pyrrolo[3,2-g]quinazolin-7-one (Composé 86) ;
(R)-4-((1-(3-(1,1-difluoro-2-hydroxy-2-méthylpropyl)-2-fluorophényl)éthyl)amino)-2-isopropyl-6,6,8-triméthyl-6H-pyrrolo[3,2-g]quinazolin-7(8H)-one (Composé 87) ;
(R)-4-((1-(3-(1,1-difluoro-2-hydroxyéthyl)-2-fluorophényl)éthyl)amino)-8-éthyl-2,6,6-triméthyl-6,8-dihydro-7H-pyrrolo[3,2-g]quinazolin-7-one (Composé 88) ;
(R)-4-((1-(3-(1,1-difluoro-2-hydroxy-2-méthylpropyl)-2-fluorophényl)éthyl)amino)-8-éthyl-2,6,6-triméthyl-6H-pyrrolo[3,2-g]quinazolin-7(8H)-one (Composé 89) ;
(R)-4-((1-(3-(1,1-difluoro-2-hydroxy-2-méthylpropyl)-2-fluorophényl)éthyl)amino)-2,6,6,8,9-pentaméthyl-6,8-dihydro-7H-pyrrolo[3,2-g]quinazolin-7-one (Composé 90) ;
(R)-4-((1-(3-(1,1-difluoro-2-hydroxy-2-méthylpropyl)-2-fluorophényl)éthyl)amino)-6,6-diéthyl-2,8-diméthyl-6,8-dihydro-7H-pyrrolo[3,2-g]quinazolin-7-one (Composé 91) ;
(R)-4-((1-(3-(1,1-difluoro-2-hydroxy-2-méthylpropyl)-2-fluorophényl)éthyl)amino)-6,6-bis(fluorométhyl)-2,8-diméthyl-6,8-dihydro-7H-pyrrolo[3,2-g]quinazolin-7-one (Composé 92) ;
4-(((R)-1-(3-(1,1-difluoro-2-hydroxy-2-méthylpropyl)-2-fluorophényl)éthyl)amino)-6-éthyl-2,6,8-triméthyl-6,8-dihydro-7H-pyrrolo[3,2-g]quinazolin-7-one (Composé 93) ;
4-(((R)-1-(3-(1,1-difluoro-2-hydroxy-2-méthylpropyl)-2-fluorophényl)éthyl)amino)-6-(méthoxyméthyl)-2,6,8-triméthyl-6,8-dihydro-7H-pyrrolo[3,2-g]quinazolin-7-one (Composé 94) ;
4-(((R)-1-(3-(1,1-difluoro-2-hydroxy-2-méthylpropyl)-2-fluorophényl)éthyl)amino)-2,6,8-triméthyl-6,8-dihydro-7H-pyrrolo[3,2-g]quinazolin-7-one (Composé 95) ;
4-(((R)-1-(3-(1,1-difluoro-2-hydroxy-2-méthylpropyl)-2-fluorophényl)éthyl)amino)-6-méthoxy-2,6,8-triméthyl-6,8-dihydro-7H-pyrrolo[3,2-g]quinazolin-7-one (Composé 96) ;
4-(((R)-1-(3-(1,1-difluoro-2-hydroxy-2-méthylpropyl)-2-fluorophényl)éthyl)amino)-6-hydroxy-2,6,8-triméthyl-6,8-dihydro-7H-pyrrolo[3,2-g]quinazolin-7-one (Composé 97) ;
4-(((R)-1-(3-(1,1-difluoro-2-hydroxy-2-méthylpropyl)-2-fluorophényl)éthyl)amino)-8-(méthoxyméthyl)-2,6,8-triméthyl-6,8-dihydro-7H-pyrrolo[2,3-g]quinazolin-7-one (Composé 98) ;
4-(((R)-1-(3-(1,1-difluoro-2-hydroxy-2-méthylpropyl)-2-fluorophényl)éthyl)amino)-2,6,8-triméthyl-6,8-dihydro-7H-pyrrolo[2,3-g]quinazolin-7-one (Composé 99) ;
4-(((R)-1-(3-(1,1-difluoro-2-hydroxy-2-méthylpropyl)-2-fluorophényl)éthyl)amino)-8-hydroxy-2,6,8-triméthyl-6,8-dihydro-7H-pyrrolo[2,3-g]quinazolin-7-one (Composé 100) ;
4-(((R)-1-(3-(1,1-difluoro-2-hydroxy-2-méthylpropyl)-2-fluorophényl)éthyl)amino)-8-méthoxy-2,6,8-triméthyl-6,8-dihydro-7H-pyrrolo[2,3-g]quinazolin-7-one (Composé 101) ;
(S/R)-4-(((R/S)-1-(3-((S/R)-1,1-difluoro-2,3-dihydroxy-2-méthylpropyl)-2-fluorophényl)éthyl)amino)-8-méthoxy-2,6,8-triméthyl-6,8-dihydro-7H-pyrrolo[2,3-g]quinazolin-7-one (Composé 102) ;
(S/R)-4-(((R/S)-1-(3-((R/S)-1,1-difluoro-2,3-dihydroxy-2-méthylpropyl)-2-fluorophényl)éthyl)amino)-8-méthoxy-2,6,8-triméthyl-6,8-dihydro-7H-pyrrolo[2,3-g]quinazolin-7-one (Composé 103) ;
(R)-8-((1-(3-(1,1-difluoro-2-hydroxy-2-méthylpropyl)-2-fluorophényl)éthyl)amino)-1-éthyl-3,6-diméthyl-1,3-dihydro-2H-imidazo[4,5-g]quinazolin-2-one (Composé 104) ;
(R)-8-((1-(3-(1,1-difluoro-2-hydroxy-2-méthylpropyl)-2-fluorophényl)éthyl)amino)-1-isopropyl-3,6-diméthyl-1,3-dihydro-2H-imidazo[4,5-g]quinazolin-2-one (Composé 105) ;
(R)-8-((1-(3-(1,1-difluoro-2-hydroxy-2-méthylpropyl)-2-fluorophényl)éthyl)amino)-1-(2,2-difluoroéthyl)-3,6-diméthyl-1H-imidazo[4,5-g]quinazolin-2(3H)-one (Composé 106) ;
(R)-1,1-difluoro-1-(2-fluoro-3-(1-((2,2,3,6-tétraméthyl-2,3-dihydro-1H-imidazo[4,5-g]quinazolin-8-yl)amino) éthyl)phényl)-2-méthylpropan-2-ol (Composé 107) ;
(R)-1,1-difluoro-1-(2-fluoro-3-(1-((1,2,2,3,6-pentaméthyl-2,3-dihydro-1H-imidazo[4,5-g]quinazolin-8-yl)amino) éthyl)phényl)-2-méthylpropan-2-ol (Composé 108) ;
(R)-8-((1-(3-(1,1-difluoro-2-hydroxy-2-méthylpropyl)-2-fluorophényl)éthyl)amino)-1,3,6-triméthyl-1,3-dihydro-2H-imidazo[4,5-g]quinazolin-2-one (Composé 109) ;
(R)-2-cyclopropyl-4-((1-(3-(1,1-difluoro-2-hydroxy-2-méthylpropyl)-2-fluorophényl)éthyl)amino)-6,6,8-triméthyl-6,8-dihydro-7H-pyrrolo[3,2-g]quinazolin-7-one (Composé 110) ;
(R)-8-((1-(3-(1,1-difluoro-2-hydroxy-2-méthylpropyl)-2-fluorophényl)éthyl)amino)-1,6-diméthyloxazolo[4,5-g] quinazolin-2(1H)-one (Composé 111) ;
(R)-4-((1-(3-(1,1-difluoro-2-hydroxy-2-méthylpropyl)-2-fluorophényl)éthyl)amino)-2,6-diméthyl-6H-pyrrolo[2,3-g]quinazoline-7,8-dione (Composé 112) ;
(R)-4-((1-(3-(1,1-difluoro-2-hydroxy-2-méthylpropyl)-2-fluorophényl)éthyl)amino)-2,6-diméthyl-6,8-dihydro-7H-pyrrolo[2,3-g]quinazolin-7-one (Composé 113) ;
(R)-4-((1-(3-(1,1-difluoro-2-hydroxy-2-méthylpropyl)-2-fluorophényl)éthyl)amino)-2,6,6,7-tétraméthyl-6,7-dihydro-8H-pyrrolo[3,4-g]quinazolin-8-one (Composé 114) ;
4-((1-(2-fluoro-3-(1-(hydroxyméthyl)cyclopropyl)phényl)éthyl)amino)-2,6,8,8-tétraméthyl-6,8-dihydro-7H-pyrrolo[2,3-g]quinazolin-7-one (Composé 115) ;
4-(((R)-1-(3-(1,1-difluoro-2-hydroxy-2-méthylpropyl)-2-fluorophényl)éthyl)amino)-8-fluoro-2,6,8-triméthyl-6,8-dihydro-7H-pyrrolo[2,3-g]quinazolin-7-one (Composé 116) ;
(R)-4-((1-(3-(1,1-difluoro-2-hydroxy-2-méthylpropyl)-2-fluorophényl)éthyl)amino)-8,8-difluoro-2,6-diméthyl-6,8-dihydro-7H-pyrrolo[2,3-g]quinazolin-7-one (Composé 117) ;
(R)-4-((1-(3-(1,1-difluoro-2-hydroxy-2-méthylpropyl)-2-fluorophényl)éthyl)amino)-2,7,8,8-tétraméthyl-7,8-dihydro-6H-pyrrolo[3,4-g]quinazolin-6-one (Composé 118) ;
(R)-8-((1-(3-(1,1-difluoro-2-hydroxy-2-méthylpropyl)-2-fluorophényl)éthyl)amino)-3,6-diméthyl-1,3-dihydro-2H-imidazo[4,5-g]quinazolin-2-one (Composé 119) ;
(S)-4-(((S)-1-(3-(1,1-difluoro-2-hydroxy-2-méthylpropyl)-2-fluorophényl)éthyl)amino)-8-méthoxy-2,6,8-triméthyl-6,8-dihydro-7H-pyrrolo[2,3-g]quinazolin-7-one (Composé 120) ;
4-(((R)-1-(3-(1,1-difluoro-2-hydroxy-2-méthylpropyl)-2-fluorophényl)éthyl)amino)-8-éthoxy-2,6,8-triméthyl-6,8-dihydro-7H-pyrrolo[2,3-g]quinazolin-7-one (Composé 121) ;
(R)-4'-((1-(3-(1,1-difluoro-2-hydroxy-2-méthylpropyl)-2-fluorophényl)éthyl)amino)-2',6'-diméthyl-2,3,5,6-tétrahydrospiro[pyrane-4,8'-pyrrolo[2,3-g]quinazolin]-7'(6'H)-one (Composé 122) ;
4-(((R)-1-(3-(1,1-difluoro-2-hydroxy-2-méthylpropyl)-2-fluorophényl)éthyl)amino)-8-(2-méthoxyéthyl)-2,6,8-triméthyl-6,8-dihydro-7H-pyrrolo[2,3-g]quinazolin-7-one (Composé 123) ;
4'-(((R)-1-(3-(1,1-difluoro-2-hydroxy-2-méthylpropyl)-2-fluorophényl)éthyl)amino)-2',3,6'-triméthylspiro[oxazolidine-5,8'-pyrrolo[2,3-g]quinazoline]-2,7'(6'H)-dione (Composé 124) ;
4-(((R)-1-(3-(1,1-difluoro-2-hydroxy-2-méthylpropyl)-2-fluorophényl)éthyl)amino)-8-méthoxy-2,6-diméthyl-8-(trifluorométhyl)-6,8-dihydro-7H-pyrrolo[2,3-g]quinazolin-7-one (Composé 125) ;
4-(((R)-1-(3-(1,1-difluoro-2-hydroxy-2-méthylpropyl)-2-fluorophényl)éthyl)amino)-8-éthyl-8-méthoxy-2,6-diméthyl-6,8-dihydro-7H-pyrrolo[2,3-g]quinazolin-7-one (Composé 126) ;
4-(((1R)-1-(3-(1,1-difluoro-2-hydroxy-3-méthoxy-2-méthylpropyl)-2-fluorophényl)éthyl)amino)-2,6,8,8-tétraméthyl-6,8-dihydro-7H-pyrrolo[2,3-g]quinazolin-7-one (Composé 127) ;
4'-(((R)-1-(3-(1,1-difluoro-2-hydroxy-2-méthylpropyl)-2-fluorophényl)éthyl)amino)-2',6'-diméthyl-4,5-dihydro-2H-spiro[furane-3,8'-pyrrolo[2,3-g]quinazolin]-7'(6'H)-one (Composé 128) ;
4-(((1R)-1-(3-(3-(diméthylamino)-1,1-difluoro-2-hydroxy-2-méthylpropyl)-2-fluorophényl)éthyl)amino)-2,6,8,8-tétraméthyl-6,8-dihydro-7H-pyrrolo[2,3-g]quinazolin-7-one (Composé 129) ;
4-(((1R)-1-(3-(1,1-difluoro-2-hydroxy-2-méthyl-3-(méthylamino)propyl)-2-fluorophényl)éthyl)amino)-2,6,8,8-tétraméthyl-6,8-dihydro-7H-pyrrolo[2,3-g]quinazolin-7-one (Composé 130) ;
4-(((R)-1-(3-amino-5-(trifluorométhyl)phényl)éthyl)amino)-8-méthoxy-2,6,8-triméthyl-6,8-dihydro-7H-pyrrolo[2,3-g]quinazolin-7-one (Composé 131) ;
4-(((R)-1-(3-(1,1-difluoro-2-hydroxy-2-méthylpropyl)-2-fluorophényl)éthyl)amino)-8-méthoxy-8-(2-méthoxyéthyl)-2,6-diméthyl-6,8-dihydro-7H-pyrrolo[2,3-g]quinazolin-7-one (Composé 132) ;
4-(((1R)-1-(2-fluoro-3-(pipéridin-3-yl)phényl)éthyl)amino)-2,6,8,8-tétraméthyl-6,8-dihydro-7H-pyrrolo[2,3-g]quinazolin-7-one (Composé 133) ;
(R)-4-((1-(2-fluoro-3-(trifluorométhyl)phényl)éthyl)amino)-2,6,8,8-tétraméthyl-6,8-dihydro-7H-pyrrolo[2,3-g]quinazolin-7-one (Composé 134) ;
4-(((1R)-1-(3-(3-(diméthylamino)-1,1-difluoro-2-hydroxy-2-méthylpropyl)-2-fluorophényl)éthyl)amino)-8-méthoxy-2,6,8-triméthyl-6,8-dihydro-7H-pyrrolo[2,3-g]quinazolin-7-one (Composé 135) ;
2,6,8,8-tétraméthyl-4-((1-(3-(trifluorométhyl)phényl)éthyl)amino)-6,8-dihydro-7H-pyrrolo[2,3-g]quinazolin-7-one (Composé 136) ;
4-(((R)-1-(3-amino-5-(trifluorométhyl)phényl)éthyl)amino)-6-méthoxy-2,6,8-triméthyl-6,8-dihydro-7H-pyrrolo[3,2-g]quinazolin-7-one (Composé 137) ;
(R)-4'-((1-(3-amino-5-(trifluorométhyl)phényl)éthyl)amino)-2',6'-diméthyl-2,3,5,6-tétrahydrospiro[pyrane-4,8'-pyrrolo[2,3-g]quinazolin]-7'(6'H)-one (Composé 138) ;
4-(((R)-1-(3-amino-5-(trifluorométhyl)phényl)éthyl)amino)-8-éthyl-8-méthoxy-2,6-diméthyl-6,8-dihydro-7H-pyrrolo[2,3-g]quinazolin-7-one (Composé 139).

**3.** Une composition pharmaceutique comprenant un composé de formule (I) ou un sel pharmaceutiquement acceptable de celui-ci, tel que revendiqué dans les revendications 1 et 2, et un excipient pharmaceutiquement acceptable.

**4.** Un composé de formule (I) et un sel pharmaceutiquement acceptable de celui-ci tel que revendiqué dans les revendications 1 et 2, pour une utilisation dans le traitement et/ou la prévention d'une maladie par inhibition de SOS1 chez un sujet, comprenant l'administration au sujet d'une quantité thérapeutiquement efficace dudit composé, la maladie étant sélectionnée parmi le cancer, l'infection aiguë à Staphylococcus aureus (patients pédiatriques), le syndrome de détresse respiratoire aiguë/lésions pulmonaires aiguës, la septicémie, et la RASopathie.

**5.** Un composé de formule (I) et un sel pharmaceutiquement acceptable de celui-ci tel que revendiqué dans les revendications 1 et 2, pour une utilisation dans le traitement et/ou la prévention d'une maladie par inhibition de l'interaction de SOS1 et de la protéine de la famille RAS chez un sujet, comprenant l'administration au sujet d'une quantité thérapeutiquement efficace dudit composé, la maladie étant sélectionnée parmi le cancer, l'infection aiguë à Staphylococcus aureus (patients pédiatriques), le syndrome de détresse respiratoire aiguë/lésions pulmonaires aiguës, la septicémie, et la RASopathie.

**6.** Le composé pour une utilisation dans le traitement et/ou la prévention d'un cancer tel que revendiqué dans la revendication 4 ou 5, le cancer étant sélectionné parmi le groupe constitué du cancer du pancréas, du cancer du poumon, du cancer colorectal, des cancers mutants BRAF de classe 3, du cancer hématologique, du cholangiocarcinome, du myélome multiple, du mélanome, du cancer de l'utérus, du cancer de l'endomètre, du cancer de la thyroïde, de la leucémie myéloïde aiguë, du cancer de la vessie, du cancer urothélial, du cancer gastrique, du cancer du col de l'utérus, du carcinome épidermoïde de la tête et du cou, du lymphome diffus à grandes cellules B, du cancer de l'œsophage, de la leucémie lymphoïde chronique, du cancer hépatocellulaire, du cancer du sein, du cancer de l'ovaire, du cancer de la prostate, du glioblastome, du cancer du rein, du syndrome de la neurome muqueuse pure, de l'épulis fibreuse, et des sarcomes.

**7.** Le composé pour une utilisation dans le traitement et/ou la prévention d'une maladie tel que revendiqué dans la revendication 4 ou 5, la RASopathie étant sélectionnée parmi le groupe constitué de la neurofibromatose de type 1

(NF1), du syndrome de Noonan (NS), du syndrome de Noonan avec lentigines multiples (NSML), du syndrome de malformation capillaire-malformation artérioveineuse (CM-AVM), du syndrome de Costello (CS), du syndrome cardio-facio-cutané (CFC), du syndrome de Legius, du syndrome de Noonan-like/avec lésions géantes multiples, et de la fibromatose gingivale héréditaire (HGF).

**8.** Le composé de formule (I) et un sel pharmaceutiquement acceptable de celui-ci, tel que revendiqué dans les revendications 1 et 2, pour une utilisation dans le traitement et/ou la prévention du cancer, ledit composé étant administré en association avec au moins une autre substance pharmacologiquement active.

**9.** Le composé de formule (I) et un sel pharmaceutiquement acceptable de celui-ci, tel que revendiqué dans les revendications 1 et 2, pour une utilisation dans le traitement et/ou la prévention du cancer, le composé étant administré avant, après ou conjointement avec au moins une autre substance pharmacologiquement active.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004003152 A
- WO 2014144148 A
- WO 2016077793 A
- WO 2018115380 A
- WO 2018172250 A
- WO 2019122129 A
- WO 2019201848 A
- WO 2020180768 A
- WO 2020180770 A
- EP 2243779 A
- WO 2015164480 A
- WO 200879759 A
- WO 243823 A
- US 4235871 A
- US 4501728 A
- US 4837028 A
- US 5019369 A
- WO 2009106599 A
- US 5597820 A
- WO 201740963 A
- WO 40963 A
- WO 2019243823 A
- US 2018291002 A


### Non-patent literature cited in the description


- **FRANCISCO SANCHEZ-VEGA et al.** *Cell*, 2018, vol. 173 (2), 321-337.e10
- **SIQI LI et al.** *Nat. Rev. Cancer*, 2018, vol. 18 (12), 767-777
- **JOHANNES L. BOS et al.** *Cell*, 2007, vol. 129 (5), 865-77
- **SUZANNE SCHUBBERT et al.** *Nat. Rev. Cancer*, 2007, vol. 7 (4), 295-308
- **YOUSEF AHMED FOUAD et al.** *Am. J. Cancer Res.*, 2017, vol. 7 (5), 1016-1036
- **DOUGLAS HANAHAN et al.** *Cell*, 2011, vol. 144 (5), 646-74
- **IAN A. PRIOR et al.** *Cancer Res.*, 2012, vol. 72 (10), 2457-2467
- **ADRIENNE D. COX et al.** *Nat. Rev. Drug. Discov.*, 2014, vol. 13 (11), 828-51
- **PRADEEP BANDARU et al.** *Cold Spring Harb Perspect Med.*, 2019, vol. 9 (2), a031534
- **S. MARIANA MARGARIT et al.** *Cell*, 2003, vol. 112 (5), 685-95
- **HAO-HSUAN JENG et al.** *Nat. Commun.*, 2012, vol. 3, 1168
- **YOU X et al.** *Blood.*, 2018, vol. 132 (24), 2575-2579
- **ERIN SHEFFELS et al.** *Sci Signal.*, 2018, vol. 11 (546), eaar8371
- **FRANK MCCORMICK et al.** *Nature*, 1993, vol. 363 (6424), 45-51
- **STEPHANE PIERRE et al.** *Biochem Pharmacol.*, 2011, vol. 82 (9), 1049-56
- **MATEUSZ POLTORAK et al.** *Eur J Immunol.*, 2014, vol. 44 (5), 1535-40
- **STEPHEN R. BROOKS et al.** *J Immunol.*, 2000, vol. 164 (6), 3123-31
- **MARIO N. LIOUBIN et al.** *Mol Cell Biol.*, 1994, vol. 14 (9), 5682-91
- **ROMAN C. HILLIG et al.** *Proc. Natl. Acad. Sci. U S A.*, 2019, vol. 116 (7), 2551-2560
- **CHRIS R. EVELYN et al.** *J Biol Chem.*, 2015, vol. 290 (20), 12879-98
- **PIERRE**. *Biochem. Pharmacol.*, 2011, vol. 82 (9), 1049-56
- **F.C. BALTANÁS et al.** *BBA - Reviews on Cancer*, 2020, vol. 1874, 188445
- *Chemistry - A European Journal*, 2015, vol. 21 (4), 1482-1487
- *ACS Medicinal Chemistry Letters*, 2018, vol. 9 (8), 827-831
- **BERGE S. M. et al.** *Pharmaceutical Salts, a review article in Journal of Pharmaceutical sciences volume*, 1977, vol. 66, 1-19
- **P. HEINRICH STAHLAND** ; **CAMILLE G. WERMUTH**. Handbook of Pharmaceutical Salts - Properties, Selection, and Use. Wiley- VCH, 2002
- Remington's Pharmaceutical Sciences,. Mack Publishing Company, 1990, 1445
- *Journal of Pharmaceutical Science*, 1977, vol. 66, 2-19
- Pharmaceutics and Pharmacy Practice. J.B. Lippincott Company, 1982, 238-250
- ASHP Handbook on Injectable Drugs. *Toissel*, 1986, 622-630
- Remington's Pharmaceutical Science. Mack Publishing Company, 1985

- **SZOKA et al.** *Ann. Rev. Biophys. Bioeng.*, 1980, vol. 9, 467
- *Physicians' Desk Reference*, 2004
- **WASSERMAN et al.** *Cancer*, 1975, vol. 36, 1258-1268
- Physicians' Desk Reference. *Thomson PDR*, 2004
- *Bulletin of the Chemical Society of Japan*, 1987, vol. 60 (2), 767-768
- *Bioorganic and Medicinal Chemistry Letters*, 2016, vol. 26 (6), 1571-1575
- *Tetrahedron*, 2008, vol. 64 (4), 688-695
- *European Journal of Medicinal Chemistry*, 2015, vol. 92, 818-838